# EUROPÄISCHE PATENTSCHRIFT

(11) **EP 0 984 944 B1**
(45) Veröffentlichungstag und Bekanntmachung des Hinweises auf die Patenterteilung: **04.08.2004**
(21) Anmeldenummer: 98924195.5
(22) Anmeldetag: 05.05.1998
(51) Int. Cl.: C07D 231/20, A01N 43/56

(54) **SUBSTITUIERTE 4-(3-ALKENYL-BENZOYL)-PYRAZOLE**
SUBSTITUTED 4-(3-ALKENYL-BENZOYL)-PYRAZOLES
4-(3-ALKENYLE-BENZOYLE)-PYRAZOLS

(30) Priorität: 07.05.1997 DE 19726710
(43) Veröffentlichungstag der Anmeldung: 15.03.2000
(73) Patentinhaber: BASF AKTIENGESELLSCHAFT, 67056 Ludwigshafen (DE)
(72) Erfinder: BAUMANN, Ernst, D-67373 Dudenhofen (DE); VON DEYN, Wolfgang, D-67435 Neustadt (DE); ENGEL, Stefan, D-65510 Idstein (DE); HILL, Regina, Luise, D-67346 Speyer (DE); KARDORFF, Uwe, D-68159 Mannheim (DE); MAYER, Guido, D-67433 Neustadt (DE); OTTEN, Martina, D-67069 Ludwigshafen (DE); RACK, Michael, D-69123 Heidelberg (DE); RHEINHEIMER, Joachim, D-67063 Ludwigshafen (DE); WITSCHEL, Matthias, D-67061 Ludwigshafen (DE); WESTPHALEN, Karl-Otto, D-67346 Speyer (DE); MISSBLITZ, Ulf, D-67433 Neustadt (DE); WALTER, Helmut, D-67283 Obrigheim (DE)
(86) Internationale Anmeldenummer: PCT/EP1998/002433
(87) Internationale Veröffentlichungsnummer: WO 1998/050366

(56) Entgegenhaltungen:
- EP-A- 0 240 001
- EP-A- 0 282 944
- US-A- 4 643 757

## Beschreibung

Die vorliegende Erfindung betrifft 4-(3-Alkenyl-benzoyl)-pyrazole der Formel I 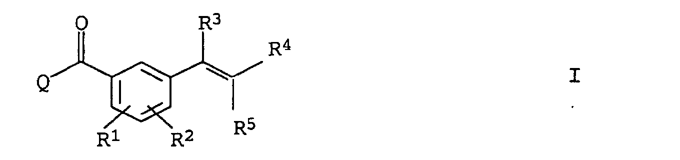 in der die Variablen folgende Bedeutung haben:
- R¹, R²: Wasserstoff, Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁶, -OCOR⁷, -OSO₂R⁷, -SH, -S(O)ₙR⁸, -SO₂OR⁶, -SO₂NR⁶R⁹, -NR⁹SO₂R⁷ oder -NR⁹COR⁷;
- R³: Wasserstoff, Halogen, C₁-C₆-Alkyl, C₁-C₆-Halogenalkyl, C₁-C₆-Alkoxy, C₂-C₆-Alkenyl oder C₂-C₆-Alkinyl;
- R⁴: Wasserstoff, Nitro, Halogen, Cyano, Rhodano, C₁-C₆-Alkyl, C₁-C₆-Halogenalkyl, C₃-C₆-Cycloalkyl, C₂-C₆-Alkenyl, C₄-C₆-Cycloalkenyl, C₂-C₆-Alkinyl, -COR¹⁰, -CO₂R¹⁰, -COSR¹⁰, -CONR¹⁰R¹¹, -C(R¹²)=NR¹³, -PO(OR¹⁰)(OR¹¹), C₁-C₄-Alkyl, das einen Rest aus folgender Gruppe trägt: -COR¹⁰, -CO₂R¹⁰, -COSR¹⁰, -CONR¹⁰R¹¹ oder -C(R¹²)=NR¹³; Heterocyclyl, Heterocyclyl-C₂-C₄-alkyl (wobei der Heterocyclyl-Rest der beiden letztgenannten Reste drei- bis siebengliedrig, gesättigt oder partiell ungesättigt, mono- oder polycyclisch ist, und ein- bis drei Heteroatome, ausgewählt aus der Gruppe bestehend aus Sauerstoff, Stickstoff und Schwefel, enthält), Phenyl, Phenyl-C₁-C₄-alkyl, Hetaryl oder Hetaryl-C₁-C₄-alkyl (wobei der Hetaryl-Rest der beiden letztgenannten Reste aromatisch, mono- oder polycyclisch ist und neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten kann), wobei die sechs letztgenannten Reste substituiert sein können;
- R⁵: Wasserstoff, Halogen, Cyano, Rhodano, C₁-C₆-Alkyl, C₁-C₆-Halogenalkyl, -COR¹⁰, -CO₂R¹⁰, -COSR¹⁰, -CONR¹⁰R¹¹ oder -PO(OR¹⁰)(OR¹¹) ;
- n: 0, 1 oder 2;
- R⁶: Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
- R⁷: C₁-C₆-Alkyl oder C₁-C₆-Halogenalkyl;
- R⁸: C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
- R⁹: Wasserstoff oder C₁-C₆-Alkyl;
- R¹⁰: Wasserstoff, C₁-C₆-Alkyl, C₃-C₆-Cycloalkyl, C₁-C₆-Halogenalkyl, C₃-C₆-Alkenyl, C₃-C₆-Alkinyl, Phenyl oder Benzyl, wobei die beiden letztgenannten Reste partiell oder vollständig halogeniert sein können und/oder einen bis drei Reste aus der folgenden Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl oder C₁-C₄-Alkoxycarbonyl;
- R¹¹: Wasserstoff, C₁-C₆-Alkyl, C₃-C₆-Alkenyl oder C₃-C₆-Alkinyl;
oder
- R¹⁰ und R¹¹: bilden gemeinsam eine C₂-C₆-Alkandiylkette, die einbis vierfach durch C₁-C₄-Alkyl substituiert sein kann und/oder durch Sauerstoff oder Schwefel oder einen gegebenenfalls C₁-C₄-Alkyl substituierten Stickstoff unterbrochen sein kann;
- R¹²: Wasserstoff, C₁-C₆-Alkyl, C₁-C₆-Halogenalkyl, C₁-C₆-Alkoxy, C₁-C₆-Alkoxycarbonyl, C₃-C₆-Cycloalkyl, C₂-C₆-Alkenyl, C₂-C₆-Alkinyl, Phenyl oder Benzyl, wobei die beiden letztgenannten Reste partiell oder vollständig halogeniert sein können und/oder einen bis drei Reste aus der folgenden Gruppe tragen können: Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl oder C₁-C₄-Alkoxycarbonyl;
- R¹³: C₁-C₆-Alkyl, C₁-C₆-Halogenalkyl, C₃-C₆-Cycloalkyl, C₃-C₆-Alkenyl, C₃-C₆-Alkinyl, C₁-C₆-Alkoxy, C₁-C₆-Halogenalkoxy, C₃-C₆-Cycloalkoxy, C₃-C₆-Alkenyloxy, C₃-C₆-Alkinyloxy, Phenyl, Benzyl oder Phenyl-C₁-C₄-alkoxy, wobei die drei letztgenannten Reste partiell oder vollständig halogeniert sein können und/oder einen bis drei Reste aus der folgenden Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl oder C₁-C₄-Alkoxycarbonyl;
- Q: ein in 4-Stellung verknüpftes Pyrazol der Formel II 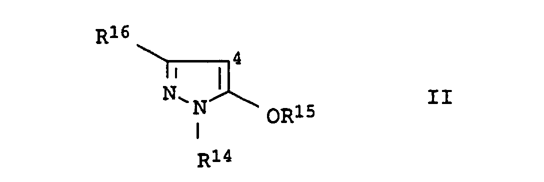 wobei
R¹⁴ für C₁-C₆-Alkyl, C₁-C₆-Halogenalkyl, Phenyl oder Phenyl das partiell oder vollständig halogeniert ist und/oder einen bis drei der folgenden Reste trägt:
Nitro, Cyano, C₁-C₄-Alkyl, C₁-C₄-Halogenalkyl, C₁-C₄-Alkoxy, C₁-C₄-Halogenalkoxy;
R¹⁵ für Wasserstoff, C₁-C₆-Alkyl, C₁-C₆-Halogenalkyl, C₁-C₆-Alkylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, Phenyl-C₁-C₄-alkyl, Phenylcarbonyl, Phenylcarbonylmethyl, Phenoxycarbonyl oder Phenylsulfonyl, wobei die fünf letztgenannten Substituenten unsubstituiert sind oder der Phenylring jeweils partiell oder vollständig halogeniert ist und/oder einen bis drei der folgenden Reste trägt:
Nitro, Cyano, C₁-C₄-Alkyl, C₁-C₄-Halogenalkyl, C₁-C₄-Alkoxy, C₁-C₄-Halogenalkoxy;
R¹⁶ für Wasserstoff, C₁-C₆-Alkyl oder C₁-C₆-Halogenalkyl; stehen;
sowie deren landwirtschaftlich brauchbaren Salze.

Außerdem betrifft die Erfindung Verfahren zur Herstellung von Verbindungen der Formel I, Mittel welche diese enthalten, sowie die Verwendung der Verbindungen der Formel I und diese enthaltende Mittel zur Schadpflanzenbekämpfung.

Aus EP-A 282 944 sind 4-[3-(2-Alkoxy-1-ethenyl)-benzoyl]-pyrazole bekannt. Die herbiziden Eigenschaften dieser Verbindungen sowie die Verträglichkeiten gegenüber Kulturpflanzen können jedoch nur bedingt befriedigen. Es lag daher dieser Erfindung die Aufgabe zugrunde insbesondere herbizid wirksame Verbindungen mit verbesserten Eigenschaften zu finden.

Demgemäß wurden die 4-(3-Alkenyl-benzoyl)-pyrazole der Formel I sowie deren herbizide Wirkung gefunden.

Ferner wurden sehr gut wirksame herbizide Mittel gefunden, die die Verbindungen I enthalten. Außerdem wurden Verfahren zur Herstellung dieser Mittel und Verfahren zur Bekämpfung von unerwünschtem Pflanzenwuchs mit den Verbindungen I gefunden.

Gegenstand der vorliegenden Erfindung sind auch Stereoisomere der Verbindungen der Formel I. Es werden sowohl reine Stereoisomere als auch Gemische hiervon erfaßt.

Die Verbindungen der Formel I enthalten eine Kohlenstoff-Kohlenstoff-Doppelbindung und liegen daher als E-Isomere oder Z-Isomere oder als E/Z-Isomerengemische vor. Weiterhin können die Verbindungen der Formel I weitere Kohlenstoff- bzw. Kohlenstoff-Stickstoff-Doppelbindungen enthalten. Gegenstand der Erfindung sind sowohl die reinen geometrischen Isomere als auch Gemische hiervon.

Die Verbindungen der Formel I können ebenso je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)-eth-1-ylammonium, Di-(2-hydroxyeth-1-yl)ammonium, Trimethylbenzylammonium, des weiteren Phosphoniumionen, Sulfoniumionen, vorzugsweise Tri(C₁-C₄-alkyl)-sulfonium und Sulfoxoniumionen, vorzugsweise Tri(C₁-C₄-alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R¹⁶ oder als Reste an Phenyl-, Hetaryl- und Heterocyclylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cycloalkyl-, Alkoxyalkyl-, Alkoxy-, Halogenalkoxy-, Cycloalkoxy, Alkylthio, Alkylsulfonyl-, Halogenalkylsulfonyl, Alkylcarbonyl-, Halogenalkylcarbonyl, Alkoxycarbonyl-, Alkenyl-, Cycloalkenyl-, Alkinyl-, Alkenyloxy-, Alkinyloxy-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₂-C₄-Alkyl: Ethyl, n-Propyl, 1-Methylethyl, Butyl, 1-Methylpropyl, 2-Methylpropyl oder 1,1-Dimethylethyl;
- C₁-C₄-Alkyl, sowie die Alkylteile von C₁-C₄-Alkoxy-C₁-C₄-alkyl, Hydroxy-C₁-C₄-alkoxy-C₁-C₄-alkyl, C₁-C₄-Alkylcarbonyl Heterocyclyl-C₁-C₄-alkyl, Phenyl-C₁-C₄-alkyl und Hetaryl-C₁-C₄-alkyl: C₂-C₄-Alkyl, wie voranstehend genannt sowie Methyl;
- C₂-C₆-Alkyl, sowie die Alkylteile von C₁-C₆-Alkoxy-C₂-C₆-alkyl: C₂-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl oder 1-Ethyl-3-methylpropyl;
- C₁-C₆-Alkyl, sowie die Alkylteile von C₁-C₆-Alkoxy-C₁-C₆-alkyl und C₁-C₆-Alkylcarbonyl: C₂-C₆-Alkyl, wie voranstehend genannt, sowie Methyl;
- C₁-C₄-Halogenalkyl: einen C₁-C₄-Alkylrest, wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₆-Halogenalkyl, sowie die Halogenalkylteile von C₁-C₆-Halogenalkylcarbonyl: C₁-C₄-Halogenalkyl wie voranstehend genannt, sowie 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl oder Dodecafluorhexyl;
- C₁-C₄-Alkoxy, sowie die Alkoxyteile von C₁-C₄-Alkoxy-C₁-C₄-alkyl, Hydroxy-C₁-C₄-alkoxy-C₁-C₄-alkyl, C₁-C₄-Alkoxycarbonyl: Methoxy, Ethoxy, Propoxy, 1-Methylethoxy, Butoxy, 1-Methylpropoxy, 2-Methylpropoxy oder 1,1-Dimethylethoxy;
- C₁-C₆-Alkoxy, sowie die Alkoxyteile von C₁-C₆-Alkoxy-C₁-C₆-alkyl, C₁-C₆-Alkoxy-C₂-C₆-alkyl und C₁-C₆-Alkoxycarbonyl: C₁-C₄-Alkoxy wie voranstehend genannt, sowie Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy;
- C₁-C₆-Halogenalkoxy: C₁-C₄-Halogenalkoxy, wie voranstehend genannt, sowie z.B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy oder Dodecafluorhexoxy;
- C₁-C₆-Alkylthio: z.B. Methylthio, Ethylthio, Propylthio, 1-Methylethylthio, Butylthio, 1-Methylpropylthio, 2-Methylpropylthio, 1,1-Dimethylethylthio Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio oder 1-Ethyl-2-methylpropylthio;
- C₁-C₆-Alkylsulfonyl (C₁-C₆-Alkyl-S(=O)₂-): z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl oder 1,1-Dimethylethylsulfonyl, Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, 1,1-Dimethylpropylsulfonyl, 1,2-Dimethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl oder 1-Ethyl-2-methylpropylsulfonyl;
- C₁-C₆-Halogenalkylsulfonyl: einen C₁-C₆-Alkylsulfonylrest, wie voranstehend genannt, der partiell oder vollständig durch Fluor, Chlor, Brom und/oder Iod substituiert ist, also Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2,2,2-Trichlorethylsulfonyl, 2-Chlor-2-fluorethylsulfonyl, 2-Chlor-2,2-difluorethylsulfonyl, 2,2-Dichlor-2-fluorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl oder Dodecafluorhexylsulfonyl;
- C₃-C₆-Alkenyl: Prop-1-en-1-yl, Prop-2-en-1-yl, 1-Methylethenyl, Buten-1-yl, Buten-2-yl, Buten-3-yl, 1-Methylprop-1-en-1-yl, 2-Methyl-prop-1-en-1-yl, 1-Methyl-prop-2-en-1-yl, 2-Methyl-prop-2-en-1-yl, Penten-1-yl, Penten-2-yl, Penten-3-yl, Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethylbut-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methylprop-1-en-1-yl oder 1-Ethyl-2-methyl-prop-2-en-1-yl;
- C₂-C₆-Alkenyl: C₃-C₆-Alkenyl wie voranstehend genannt, sowie Ethenyl;
- C₃-C₆-Alkenyloxy: z.B. Prop-1-en-1-yloxy, Prop-2-en-1-yloxy, 1-Methylethenyloxy, Buten-1-yloxy, Buten-2-yloxy, Buten-3-yloxy, 1-Methyl-prop-1-en-1-yloxy, 2-Methyl-prop-1-en-1-yloxy, 1-Methyl-prop-2-en-1-yloxy, 2-Methyl-prop-2-en-1-yloxy, Penten-1-yloxy, Penten-2-yloxy, Penten-3-yloxy, Penten-4-yloxy, 1-Methyl-but-1-en-1-yloxy, 2-Methyl-but-1-en-1-yloxy, 3-Methyl-but-1-en-1-yloxy, 1-Methyl-but-2-en-1-yloxy, 2-Methyl-but-2-en-1-yloxy, 3-Methyl-but-2-en-1-yloxy, 1-Methyl-but-3-en-1-yloxy, 2-Methyl-but-3-en-1-yloxy, 3-Methyl-but-3-en-1-yloxy, 1,1-Dimethyl-prop-2-en-1-yloxy, 1,2-Dimethyl-prop-1-en-1-yloxy, 1,2-Dimethyl-prop-2-en-1-yloxy, 1-Ethyl-prop-1-en-2-yloxy, 1-Ethyl-prop-2-en-1-yloxy, Hex-1-en-1-yloxy, Hex-2-en-1-yloxy, Hex-3-en-1-yloxy, Hex-4-en-1-yloxy, Hex-5-en-1-yloxy, 1-Methyl-pent-1-en-1-yloxy, 2-Methyl-pent-1-en-1-yloxy, 3-Methyl-pent-1-en-1-yloxy, 4-Methyl-pent-1-en-1-yloxy, 1-Methyl-pent-2-en-2-yloxy, 2-Methyl-pent-2-en-1-yloxy, 3-Methyl-pent-2-en-1-yloxy, 4-Methylpent-2-en-1-yloxy, 1-Methyl-pent-3-en-1-yloxy, 2-Methylpent-3-en-1-ylaxy, 3-Methyl-pent-3-en-1-yloxy, 4-Methylpent-3-en-1-yloxy, 1-Methyl-pent-4-en-1-yloxy, 2-Methylpent-4-en-1-yloxy, 3-Methyl-pent-4-en-1-yloxy, 4-Methylpent-4-en-1-yloxy, 1,1-Dimethyl-but-2-en-1-yloxy, 1,1-Dimethyl-but-3-en-1-yloxy, 1,2-Dimethyl-but-1-en-1-yloxy, 1,2-Dimethyl-but-2-en-1-yloxy, 1,2-Dimethyl-but-3-en-1-yloxy, 1,3-Dimethyl-but-1-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-1-en-1-yloxy, 2,3-Dimethyl-but-2-en-1-yloxy, 2,3-Dimethyl-but-3-en-1-yloxy, 3,3-Dimethyl-but-1-en-1-yloxy, 3,3-Dimethyl-but-2-en-1-yloxy, 1-Ethyl-but-1-en-1-yloxy, 1-Ethyl-but-2-en-1-yloxy, 1-Ethyl-but-3-en-1-yloxy, 2-Ethylbut-1-en-1-yloxy, 2-Ethyl-but-2-en-1-yloxy, 2-Ethylbut-3-en-1-yloxy, 1,1,2-Trimethyl-prop-2-en-1-yloxy, 1-Ethyl-1-methyl-prop-2-en-1-yloxy, 1-Ethyl-2-methylprop-1-en-1-yloxy oder 1-Ethyl-2-methyl-prop-2-en-1-yloxy;
- C₃-C₆-Alkinyl: Prop-1-in-1-yl, Prop-2-in-1-yl, But-1-in-1-yl, But-1-in-3-yl, But-1-in-4-yl, But-2-in-1-yl, Pent-1-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-1-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl;
- C₂-C₆-Alkinyl: C₃-C₆-Alkinyl, wie voranstehend genannt, sowie Ethinyl:

- C₃-C₆-Alkinyloxy: z.B. Prop-1-in-1-yloxy, Prop-2-in-1-yloxy, But-1-in-1-yloxy, But-1-in-3-yloxy, But-1-in-4-yloxy, But-2-in-1-yloxy, Pent-1-in-1-yloxy, Pent-1-in-3-yloxy, Pent-1-in-4-yloxy, Pent-1-in-5-yloxy, Pent-2-in-1-yloxy, Pent-2-in-4-yloxy, Pent-2-in-5-yloxy, 3-Methyl-but-1-in-3-yloxy, 3-Methyl-but-1-in-4-yloxy, Hex-1-in-1-yloxy, Hex-1-in-3-yloxy, Hex-1-in-4-yloxy, Hex-1-in-5-yloxy, Hex-1-in-6-yloxy, Hex-2-in-1-yloxy, Hex-2-in-4-yloxy, Hex-2-in-5-yloxy, Hex-2-in-6-yloxy, Hex-3-in-1-yloxy, Hex-3-in-2-yloxy, 3-Methylpent-1-in-1-yloxy, 3-Methylpent-1-in-3-yloxy, 3-Methyl-pent-1-in-4-yloxy, 3-Methylpent-1-in-5-yloxy, 4-Methyl-pent-1-in-1-yloxy, 4-Methylpent-2-in-4-yloxy oder 4-Methylpent-2-in-5-yloxy;
- C₃-C₆-Cycloalkyl: Cyclopropyl, Cyclobutyl, Cyclopentyl oder Cyclohexyl;
- C₃-C₆-Cycloalkoxy: Cyclopropoxy, Cyclobutoxy, Cyclopentoxy oder Cyclohexoxy;
- C₄-C₆-Cycloalkenyl: Cyclobuten-1-yl, Cyclobuten-3-yl, Cyclopenten-1-yl, Cyclopenten-3-yl, Cyclohexen-1-yl, Cyclohexen-3-yl oder Cyclohexen-4-yl;
- Heterocyclyl, sowie die Heterocyclylreste in Heterocyclyloxy und Heterocyclyl-C₁-C₄-alkyl: drei- bis siebengliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, wie Oxiranyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofuran-2-yl, 2,3-Dihydrofuran-3-yl, 2,3-Dihydrofuran-4-yl, 2,3-Dihydrofuran-5-yl, 2,5-Dihydrofuran-2-yl, 2,5-Dihydrofuran-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisothiazol-3-yl, 2,3-Dihydroisothiazol-4-yl, 2,3-Dihydroisothiazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydropyrazol-3-yl, 2,3-Dihydropyrazol-4-yl, 2,3-Dihydropyrazol-5-yl, 4,5-Dihydropyrazol-3-yl, 4,5-Dihydropyrazol-4-yl, 4,5-Dihydropyrazol-5-yl, 2,5-Dihydropyrazol-3-yl, 2,5-Dihydropyrazol-4-yl, 2,5-Dihydropyrazol-5-yl, 2,3-Dihydrooxazol-2-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 2-Tetrahydropyranyl, 3-Tetrahydropyranyl, 4-Tetrahydropyranyl, 2-Tetrahydrothiopyranyl, 3-Tetrahydrothiopyranyl, 4-Tetrahydrothiopyranyl, 1,3-Dioxolan-2-yl, 1,3-Dioxolan-4-yl, 1,3-Dithiolan-2-yl, 1,3-Dithiolan-4-yl, 1,3-Dioxan-2-yl, 1,3-Dioxan-4-yl, 1,3-Dioxan-5-yl, 1,3-Dithian-2-yl, 1,3-Dithian-4-yl, 1,3-Dithian-5-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl oder 1,3-Dihydrooxazin-2-yl;
- Hetaryl, sowie die Hetarylreste in Hetaryloxy und Hetaryl-C₁-C₄-alkyl: aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4,5-Tetrazin-3-yl, sowie die entsprechenden benzokondensierten Derivate;
- C₂-C₆-Alkandiyl: z.B. Ethan-1,2-diyl, Propan-1,3-diyl, Butan-1,4-diyl, Pentan-1,5-diyl oder Hexan-1,6-diyl.

Alle Phenyl-, Hetaryl- und Heterocyclylringe sind vorzugsweise unsubstituiert oder tragen ein bis drei Halogenatome und/oder einen oder zwei Reste aus folgender Gruppe: Nitro, Cyano, Methyl, Trifluormethyl, Methoxy, Trifluormethoxy oder Methoxycarbonyl.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- R¹: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁶ oder -S(O)ₙR⁸; besonders bevorzugt Nitro, Halogen wie z.B. Fluor, Chlor oder Brom, C₁-C₆-Halogenalkyl, -OR⁶ oder -SO₂R⁸;
- R²: Wasserstoff, Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁶ oder -S(O)ₙR⁸;
besonders bevorzugt Wasserstoff, Nitro, Halogen, wie z.B. Fluor, Chlor oder Brom, C₁-C₆-Alkyl, C₁-C₆-Halogenalkyl, -OR⁶ oder -SO₂R⁸;
insbesondere bevorzugt Nitro, Halogen wie z.B. Fluor, Chlor oder Brom, C₁-C₆-Alkyl wie z.B. Methyl, Ethyl, C₁-C₆-Halogenalkyl, wie z.B. Difluormethyl, Trifluormethyl, -OR⁶ oder SO₂R⁸;
- R³: Wasserstoff, Halogen, C₁-C₆-Alkyl, C₁-C₆-Halogenalkyl, C₁-C₆-Alkoxy, C₂-C₆-Alkenyl oder C₂-C₆-Alkinyl; besonders bevorzugt Wasserstoff, Halogen wie z.B. Fluor, Chlor oder Brom, C₁-C₄-Alkyl wie z.B. Methyl oder Ethyl, C₁-C₄-Halogenalkyl wie z.B. Trifluormethyl, C₁-C₄-Alkoxy wie z.B. Methoxy oder Ethoxy, Allyl oder Propargyl;
insbesondere bevorzugt Wasserstoff oder Methyl;
- R⁴: Wasserstoff, Nitro, Halogen, Cyano, Rhodano, C₁-C₆-Alkyl, C₁-C₆-Halogenalkyl, C₃-C₆-Cycloalkyl, C₂-C₆-Alkenyl, C₄-C₆-Cycloalkenyl, C₂-C₆-Alkinyl, -COR¹⁰, -CO₂R¹⁰, -COSR¹⁰, -CONR¹⁰R¹¹, -C(R¹²)=NR¹³, -PO(OR¹⁰)(OR¹¹), C₁-C₄-Alkyl, das einen Rest aus folgender Gruppe trägt: -COR¹⁰, -CO₂R¹⁰, -COSR¹⁰, -CONR¹⁰R¹¹ oder -C(R¹²)=NR¹³; Heterocyclyl, Heterocyclyl-C₁-C₄-alkyl (wobei der Heterocyclyl-Rest der beiden letztgenannten Reste ein- bis siebengliedrig, gesättigt oder partiell ungesättigt, mono- oder polycyclisch ist, und ein- bis drei Heteroatome, ausgewählt aus der Gruppe bestehend aus Sauerstoff, Stickstoff und Schwefel, enthält), Phenyl, Phenyl-C₁-C₄-alkyl, Hetaryl oder Hetaryl-C₁-C₄-alkyl (wobei der Hetaryl-Rest der beiden letztgenannten Reste aromatisch, mono- oder polycyclisch ist und neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten kann), wobei die sechs letztgenannten Reste ihrerseits substituiert sein können durch ein bis drei Halogenatome und/oder einen bis drei Reste aus folgender Gruppe tragen können: Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl oder C₁-C₄-Alkoxycarbonyl;
- R⁵: Wasserstoff, Halogen, Cyano, Rhodano, C₁-C₆-Alkyl, C₁-C₆-Halogenalkyl, -COR¹⁰, -CO₂R¹⁰, -COSR¹⁰, -CONR¹⁰R¹¹ oder -PO(OR¹⁰)(OR¹¹);
- n: 0 oder 2;
- R⁶: Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
besonders bevorzugt C₁-C₄-Alkyl, wie z.B. Methyl oder Ethyl, C₁-C₄-Halogenalkyl wie z.B. Trifluormethyl oder Difluormethyl, C₁-C₄-Alkoxy-C₂-C₄-alkyl wie z.B. Methoxyethyl, Allyl oder Propargyl;
- R⁸: C₁-C₆-Alkyl, C₁-C₆-Halogenalkyl, C₁-C₆-Alkoxy-C₂-C₆-alkyl, C₃-C₆-Alkenyl oder C₃-C₆-Alkinyl; besonders bevorzugt C₁-C₄-Alkyl, wie z.B. Methyl oder Ethyl, C₁-C₄-Halogenalkyl wie z.B. Trifluormethyl oder Difluormethyl, C₁-C₄-Alkoxy-C₂-C₄-alkyl wie z.B. Methoxyethyl, Allyl oder Propargyl;
- R¹⁰: Wasserstoff, C₁-C₆-Alkyl, C₃-C₆-Cycloalkyl, C₁-C₆-Halogenalkyl, C₃-C₆-Alkenyl, C₃-C₆-Alkinyl, Phenyl oder Benzyl;
besonders bevorzugt Wasserstoff, C₁-C₄-Alkyl wie z.B. Methyl oder Ethyl, C₁-C₄-Halogenalkyl wie z.B. Trifluormethyl, Allyl, Propargyl und Benzyl;
- R¹¹: Wasserstoff, C₁-C₆-Alkyl, C₃-C₆-Alkenyl oder C₃-C₆-Alkinyl
oder
- R¹⁰ und R¹¹: bilden gemeinsam eine C₂-C₆-Alkandiylkette, die einbis vierfach durch C₁-C₄-Alkyl substituiert sein kann;
- R¹²: Wasserstoff, C₁-C₆-Alkyl, C₁-C₆-Halogenalkyl, C₁-C₆-Alkoxy, C₁-C₆-Alkoxycarbonyl, C₃-C₆-Cycloalkyl, C₂-C₆-Alkenyl, C₂-C₆-Alkinyl, Phenyl oder Benzyl; besonders bevorzugt Wasserstoff, C₁-C₄-Alkyl wie z.B. Methyl oder Ethyl, C₁-C₄-Alkoxycarbonyl wie z.B. Methoxycarbonyl oder Ethoxycarbonyl, C₁-C₄-Halogenalkyl wie z.B. Trifluormethyl, C₁-C₄-Alkoxy wie z.B. Methoxy oder Ethoxy;
- R¹³: C₁-C₆-Alkyl, C₁-C₆-Halogenalkyl, C₃-C₆-Cycloalkyl, C₃-C₆-Alkenyl, C₃-C₆-Alkinyl, C₁-C₆-Alkoxy, C₁-C₆-Halogenalkoxy, C₃-C₆-Cycloalkoxy, C₃-C₆-Alkenyloxy, C₃-C₆-Alkinyloxy, Phenyl, Benzyl oder Benzyloxy; besonders bevorzugt C₁-C₄-Alkyl wie z.B. Methyl oder Ethyl, C₁-C₄-Alkoxy wie z.B. Methoxy oder Ethoxy, Allyloxy, Propargyloxy, Benzyl oder Benzyloxy;
- R¹⁴: C₁-C₆-Alkyl oder C₁-C₆-Halogenalkyl; besonders bevorzugt C₁-C₄-Alkyl wie z.B. Methyl, Ethyl, Propyl, Isopropyl, Butyl oder Isobutyl;
- R¹⁵: Wasserstoff, C₁-C₆-Alkyl, C₁-C₆-Alkylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, Phenylcarbonylmethyl oder Phenylsulfonyl, wobei der Phenylring der zwei letztgenannten Substituenten partiell oder vollständig halogeniert sein kann und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy;
- R¹⁶: Wasserstoff, C₁-C₆-Alkyl oder C₁-C₆-Halogenalkyl; besonders bevorzugt Wasserstoff, C₁-C₄-Alkyl wie z.B. Methyl oder Ethyl oder C₁-C₄-Halogenalkyl wie z.B. Trifluormethyl;
insbesondere bevorzugt Wasserstoff oder Methyl.

Außerordentlich bevorzugt sind die Verbindungen der Formel I, wobei
- R¹: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁶ oder -S(O)ₙR⁸ bedeutet;
- R²: für Wasserstoff oder einen wie voranstehend unter R¹ genannten Rest steht.

Besonders außerordentlich bevorzugt sind die Verbindungen der formel I, wobei
- R¹: Nitro, Halogen wie z.B. Fluor, Chlor oder Brom, C₁-C₆-Halogenalkyl, -OR⁶ oder -SO₂R⁸;
- R²: Nitro, Halogen wie z.B. Fluor, Chlor oder Brom, C₁-C₆-Alkyl, wie z.B. Methyl oder Ethyl, C₁-C₆-Halogenalkyl, -OR⁶ oder -SO₂R⁸, wie z.B. Methylsulfonyl oder Ethylsulfonyl;
bedeuten.

Besonderst außerordentlichst bevorzugt sind die Verbindungen der Formel I, wobei
- R⁴: Wasserstoff, Cyano, C₁-C₆-Alkyl, C₁-C₆-Halogenalkyl, C₃-C₆-Cycloalkyl, C₂-C₆-Alkenyl, C₄-C₆-Cycloalkenyl, C₂-C₆-Alkinyl, -COR¹⁰, -CO₂R¹⁰, -COSR¹⁰, -CONR¹⁰R¹¹, - C(R¹²)=NR¹³, C₁-C₄-Alkyl, das einen Rest aus folgender Gruppe trägt: -COR¹⁰, CO₂R¹⁰, -COSR¹⁰, -CONR¹⁰R¹¹, -C(R¹²)=NR¹³; Heterocyclyl, Heterocyclyl-C₁-C₄-alkyl, (wobei der Heterocyclyl-Rest der beiden letztgenannten Reste ein- bis siebengliedrig, gesättigt oder partiell ungesättigt, mono- oder polycyclisch ist, und ein- bis drei Heteroatome, ausgewählt aus der Gruppe bestehend aus Sauerstoff, Stickstoff und Schwefel, enthält), Phenyl, Phenyl-C₁-C₄-alkyl, Hetaryl oder Hetaryl-C₁-C₄-alkyl (wobei der Hetaryl-Rest der beiden letztgenannten Reste aromatisch, mono- oder polycyclisch ist und neben Kohlenstoffringgliedern zusätzlich ein bis vier Stickstoffatome oder ein bis drei Stickstoffatome und ein Sauerstoffoder ein Schwefelatom oder ein Sauerstoff- oder ein Schwefelatom enthalten kann), wobei die sechs letztgenannten Reste substituiert sein können;
- R⁵: Wasserstoff, Cyano, C₁-C₆-Alkyl, C₁-C₆-Halogenalkyl, -COR¹⁰, -CO₂R¹⁰, -COSR¹⁰, -CONR¹⁰R¹¹ oder -PO(OR¹⁰)(OR¹¹);
bedeuten.

Ebenso außerordentlich bevorzugt sind Verbindungen der Formel I, wobei
- R¹: Nitro, Halogen, C₁-C₆-Halogenalkyl oder C₁-C₆-Alkylsulfonyl; insbesondere Nitro, Chlor, Trifluormethyl, Methylsulfonyl oder Ethylsulfonyl;
- R²: Nitro, Halogen, C₁-C₆-Alkyl, C₁-C₆-Halogenalkyl, C₁-C₆-Alkoxy oder C₁-C₆-Alkylsulfonyl;
insbesondere Nitro, Chlor, Methyl, Trifluormethyl, Methoxy oder Methylsulfonyl;
- R³: Wasserstoff oder C₁-C₆-Alkyl;
insbesondere Wasserstoff oder Methyl, bevorzugt Wasserstoff;
- R⁴: Wasserstoff, Halogen, Cyano, C₁-C₆-Alkyl, C₁-C₄-Alkylcarbonyl, C₁-C₄-Alkoxycarbonyl, Formyl, Hydroxycarbonyl oder -C(R¹²)=NR¹³;
insbesondere Wasserstoff, Chlor, Brom, Cyano, Methyl, Ethyl, Isopropyl, Methylcarbonyl, Ethylcarbonyl, Methoxycarbonyl, Ethoxycarbonyl oder -C(R¹²)=NR¹³;
- R⁵: Wasserstoff, Halogen oder C₁-C₄-Alkyl;
insbesondere Wasserstoff, Chlor, Methyl oder Ethyl;
- R¹²: Wasserstoff, C₁-C₄-Alkyl oder C₁-C₄-Alkoxy;
insbesondere Wasserstoff oder Methyl;
- R¹³: C₁-C₆-Alkyl oder C₁-C₆-Alkoxy;
insbesondere Methoxy oder Ethoxy;
- R¹⁴: C₁-C₆-Alkyl;
insbesondere Methyl oder Ethyl;
- R¹⁵: Wasserstoff, C₁-C₆-Alkylsulfonyl oder Phenyl-C₁-C₄-alkyl, wobei der Phenylring des letztgenannten Restes partiell oder vollständig halogeniert ist und/oder einen bis drei der folgenden Reste trägt: Nitro, Cyano, C₁-C₄-Alkyl, C₁-C₄-Halogenalkyl, C₁-C₄-Alkoxy, C₁-C₄-Halogenalkoxy;
insbesondere Wasserstoff, Methylsulfonyl, Ethylsulfonyl oder Benzyl;
- R¹⁶: Wasserstoff oder C₁-C₄-Alkyl;
insbesondere Wasserstoff oder Methyl;
bedeuten, und wobei die Restedefinitionen R¹ bis R¹⁶ nicht nur in Kombination miteinander, sondern auch jeweils für sich allein betrachtet für die erfindungsgemäßen Verbindungen der Formel I eine besondere Bedeutung haben.

Insbesondere außerordentlich bevorzugt sind Verbindungen der Formel Ia (≙ I wobei R¹ in Position 4 des Phenylringes und R² in Position 2 des Phenylringes gebunden sind).

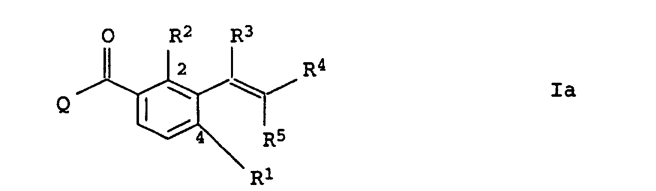

Außergewöhnlich bevorzugt sind die Verbindungen Ia1 (≙ I mit R¹ = C1, R¹⁴ = CH₃ und R¹⁵ und R¹⁶ = H, wobei R¹ in 4-Position des Phenylringes und R² in 2-Position des Phenylringes gebunden sind), insbesondere die Verbindungen der Tabelle 1, wobei die Restedefinitionen R² bis R⁵ nicht nur in Kombination miteinander, sondern auch jeweils allein betrachtet für die erfindungsgemäßen Verbindungen eine besondere Bedeutung haben.

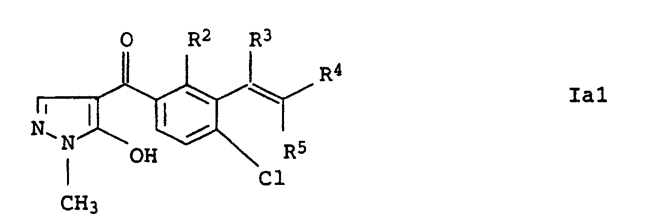

**Tabelle 1**

| Nr. | R² | R³ | R⁴ | R⁵ |
|---|---|---|---|---|
| Ia1.001 | Cl | H | H | H |
| Ia1.002 | Cl | H | CH₃ | H |
| Ia1.003 | Cl | H | CH₃ | CH₃ |
| Ia1.004 | Cl | H | C₂H₅ | H |
| Ia1.005 | Cl | H | n-C₃H₇ | H |
| Ia1.006 | Cl | H | n-C₄H₉ | H |
| Ia1.007 | Cl | H | CH(CH₃)₂ | H |
| Ia1.008 | Cl | H | cyclo-C₃H₅ | H |
| Ia1.009 | Cl | H | cyclo-C₄H₇ | H |
| Ia1.010 | Cl | H | cyclo-C₅H₉ | H |
| Ia1.011 | Cl | H | cyclo-C₆H₁₁ | H |
| Ia1.012 | Cl | H | C₆H₅ | H |
| Ia1.013 | Cl | H | CH₂-C₆H₅ | H |
| Ia1.014 | Cl | H | 2-Furyl | H |
| Ia1.015 | Cl | H | 3-Furyl | H |
| Ia1.016 | Cl | H | 2-Thienyl | H |
| Ia1.017 | Cl | H | 3-Thienyl | H |
| Ia1.018 | Cl | H | 1,3-Dioxan-2-yl | H |
| Ia1.019 | Cl | H | CHO | H |
| Ia1.020 | Cl | H | COCH₃ | H |
| Ia1.021 | Cl | H | COOCH₃ | H |
| Ia1.022 | Cl | H | COOC₂H₅ | H |
| Ia1.023 | Cl | H | COO-n-C₃H₇ | H |
| Ia1.024 | Cl | H | CN | H |
| Ia1.026 | Cl | H | COCF₃ | H |
| Ia1.027 | Cl | H | COC₆H₅ | H |
| Ia1.028 | Cl | H | CH=NOCH₃ | H |
| Ia1.029 | Cl | H | CH=NOC₂H₅ | H |
| Ia1.030 | Cl | H | C(CH₃)=NOCH₃ | H |
| Ia1.031 | CH₃ | H | H | H |
| Ia1.032 | CH₃ | H | CH₃ | H |
| Ia1.033 | CH₃ | H | CH₃ | CH₃ |
| Ia1.034 | CH₃ | H | C₂H₅ | H |
| Ia1.035 | CH₃ | H | n-C₃H₇ | H |
| Ia1.036 | CH₃ | H | n-C₄H₉ | H |
| Ia1.037 | CH₃ | H | CHO | H |
| Ia1.038 | CH₃ | H | COCH₃ | H |
| Ia1.039 | CH₃ | H | COOCH₃ | H |
| Ia1.040 | CH₃ | H | COOC₂H₅ | H |
| Ia1.041 | CH₃ | H | C₆H₅ | H |
| Ia1.042 | CH₃ | H | C₆H₅ | CH₃ |
| Ia1.043 | CH₃ | H | C₆H₅ | C₂H₅ |
| Ia1.044 | CH₃ | H | CH₂-CHO | H |
| Ia1.045 | CH₃ | H | COOCH₂C₆H₅ | H |
| Ia1.046 | CH₃ | Cl | CH₃ | H |
| Ia1.047 | CH₃ | CH₃ | CH₃ | H |
| Ia1.048 | CH₃ | C₂H₅ | CH₃ | H |
| Ia1.049 | CH₃ | CF₃ | CH₃ | H |
| Ia1.050 | CH₃ | OCH₃ | CH₃ | H |
| Ia1.051 | CH₃ | OC₂H₅ | CH₃ | H |
| Ia1.052 | CH₃ | CH₂-C≡CH | CH₃ | H |
| Ia1.053 | CH₃ | CH₂-CH=CH₂ | CH₃ | H |
| Ia1.054 | CH₃ | Cl | C₂H₅ | H |
| Ia1.055 | CH₃ | CH₃ | C₂H₅ | H |
| Ia1.056 | CH₃ | CF₃ | C₂H₅ | H |
| Ia1.057 | CH₃ | OCH₃ | C₂H₅ | H |
| Ia1.058 | CH₃ | OC₂H₅ | C₂H₅ | H |
| Ia1.059 | CH₃ | CH₂-C≡CH | C₂H₅ | H |
| Ia1.060 | CH₃ | CH₂-CH=CH₂ | C₂H₅ | H |
| Ia1.061 | OCH₃ | H | H | H |
| Ia1.062 | OCH₃ | H | CH₃ | H |
| Ia1.063 | OCH₃ | H | C₂H₅ | H |
| Ia1.064 | OCH₃ | H | n-C₃H₇ | H |
| Ia1.065 | OCH₃ | H | n-C₄H₉ | H |
| Ia1.066 | OCH₃ | H | CHO | H |
| Ia1.067 | OCH₃ | H | COCH₃ | H |
| Ia1.068 | OCH₃ | H | COOCH₃ | H |
| Ia1.069 | OCH₃ | H | COOC₂H₅ | H |
| Ia1.070 | OCH₃ | H | C₆H₅ | H |
| Ia1.071 | OCH₃ | H | CH=NOCH₃ | H |
| Ia1.072 | OCH₃ | H | C (CH₃)=NOCH₃ | H |
| Ia1.073 | OCH₃ | CH₃ | 2-Cl-C₆H₄ | H |
| Ia1.074 | OCH₃ | CH₃ | 3-Br-C₆H₄ | H |
| Ia1.075 | OCH₃ | CH₃ | 4-F-C₆H₄ | H |
| Ia1.076 | OCH₃ | CH₃ | 2,4-Cl₂-C₆H₃ | H |
| Ia1.077 | OCH₃ | CH₃ | 2-NO₂-C₆H₄ | H |
| Ia1.078 | OCH₃ | CH₃ | 3-CN-C₆H₄ | H |
| Ia1.079 | OCH₃ | CH₃ | 4-CH₃-C₆H₄ | H |
| Ia1.080 | OCH₃ | CH₃ | 2-OCH₃-C₆H₄ | H |
| Ia1.081 | OCH₃ | CH₃ | 3-CF₃-C₆H₄ | H |
| Ia1.082 | OCH₃ | CH₃ | 4-OCF₃-C₆H₄ | H |
| Ia1.083 | OCH₃ | CH₃ | 2-CH₃-C₆H₄ | H |
| Ia1.084 | OCH₃ | CH₃ | 3-CH₃-C₆H₄ | H |
| Ia1.085 | OCH₃ | CH₃ | 2-COCH₃-C₆H₄ | H |
| Ia1.086 | OCH₃ | CH₃ | 3-COOMe-C₆H₄ | H |
| Ia1.087 | OCH₃ | CH₃ | 4-CF₃-C₆H₄ | H |
| Ia1.088 | OCH₃ | CH₃ | 2-CF₃-C₆H₄ | H |
| Ia1.089 | OCH₃ | CH₃ | 3-OCH₃-C₆H₄ | H |
| Ia1.090 | OCH₃ | CH₃ | 4-OCH₃-C₆H₄ | H |
| Ia1.091 | CF₃ | H | H | H |
| Ia1.092 | CF₃ | H | CH₃ | H |
| Ia1.093 | CF₃ | H | C₂H₅ | H |
| Ia1.094 | CF₃ | H | n-C₃H₇ | H |
| Ia1.095 | CF₃ | H | n-C₄H₉ | H |
| Ia1.096 | CF₃ | H | CHO | H |
| Ia1.097 | CF₃ | H | COCH₃ | H |
| Ia1.098 | CF₃ | H | COOCH₃ | H |
| Ia1.099 | CF₃ | H | COOC₂H₅ | H |
| Ia1.100 | CF₃ | H | C₆H₅ | H |
| Ia1.101 | CF₃ | H | CH=NOCH₃ | H |
| Ial.102 | CF₃ | H | C(CH₃)=NOCH₃ | H |
| Ia1.103 | CF₃ | H | 2-Furyl | CH₃ |
| Ia1.104 | CF₃ | H | 3-Furyl | CH₃ |
| Ia1.105 | CF₃ | H | 2-Thienyl | CH₃ |
| Ia1.106 | CF₃ | H | 3-Thienyl | CH₃ |
| Ia1.107 | CF₃ | H | 2-Pyridyl | CH₃ |
| Ia1.108 | CF₃ | H | 3-Pyridyl | CH₃ |
| Ia1.109 | CF₃ | H | 4-Pyridyl | CH₃ |
| Ia1.110 | CF₃ | H | 2-Thiazolyl | CH₃ |
| Ia1.111 | CF₃ | H | 4-Thiazolyl | CH₃ |
| Ia1.112 | CF₃ | H | 5-Thiazolyl | CH₃ |
| Ia1.113 | CF₃ | H | 2-Pyrrolyl | CH₃ |
| Ia1.114 | CF₃ | H | 3-Pyrrolyl | CH₃ |
| Ia1.115 | CF₃ | H | 4-Pyrrolyl | CH₃ |
| Ia1.116 | CF₃ | H | 3-Isoxazolyl | CH₃ |
| Ia1.117 | CF₃ | H | 4-Isoxazolyl | CH₃ |
| Ia1.118 | CF₃ | H | 5-Isoxazolyl | CH₃ |
| Ia1.119 | CF₃ | H | 2-Oxazolyl | CH₃ |
| Ia1.120 | CF₃ | H | 4-Oxazolyl | CH₃ |
| Ia1.121 | SO₂CH₃ | H | H | H |
| Ia1.122 | SO₂CH₃ | H | CH₃ | H |
| Ia1.123 | SO₂CH₃ | H | C₂H₅ | H |
| Ia1.124 | SO₂CH₃ | H | n-C₃H₇ | H |
| Ia1.125 | SO₂CH₃ | H | n-C₄H₉ | H |
| Ia1.126 | SO₂CH₃ | H | CHO | H |
| Ia1.127 | SO₂CH₃ | H | COCH₃ | H |
| Ia1.128 | SO₂CH₃ | H | COOCH₃ | H |
| Ia1.129 | SO₂CH₃ | H | CO₂C₂H₅ | H |
| Ia1.130 | SO₂CH₃ | H | C₆H₅ | H |
| Ia1.131 | SO₂CH₃ | H | CH=NOCH₃ | H |
| Ia1.132 | SO₂CH₃ | H | C(CH₃)=NOCH₃ | H |
| Ia1.133 | SO₂CH₃ | C₂H₅ | 5-Oxazolyl | H |
| Ia1.134 | SO₂CH₃ | C₂H₅ | 3-Pyrazolyl | H |
| Ia1.135 | SO₂CH₃ | C₂H₅ | 4-Pyrazolyl | H |
| Ia1.136 | SO₂CH₃ | C₂H₅ | 5-Pyrazolyl | H |
| Ia1.137 | SO₂CH₃ | C₂H₅ | 2-Imidazolyl | H |
| Ia1.138 | SO₂CH₃ | C₂H₅ | 4-Imidazolyl | H |
| Ia1.139 | SO₂CH₃ | C₂H₅ | 5-Imidazolyl | H |
| Ia1.140 | SO₂CH₃ | C₂H₅ | 2-Pyrimidinyl | H |
| Ia1.141 | SO₂CH₃ | C₂H₅ | 4-Pyrimidinyl | H |
| Ia1.142 | SO₂CH₃ | C₂H₅ | 5-Pyrimidinyl | H |
| Ia1.143 | SO₂CH₃ | C₂H₅ | 1,3-Dioxolan-2-yl | H |
| Ial.144 | SO₂CH₃ | C₂H₅ | 1,3-Dioxolan-4-yl | H |
| Ia1.145 | SO₂CH₃ | C₂H₅ | 1,3-Dioxan-2-yl | H |
| Ia1.146 | SO₂CH₃ | C₂H₅ | 3-Pyridazinyl | H |
| Ia1.147 | SO₂CH₃ | C₂H₅ | 4-Pyridazinyl | H |
| Ia1.148 | SO₂CH₃ | C₂H₅ | 2-Pyrazinyl | H |
| Ia1.149 | SO₂CH₃ | C₂H₅ | 2-Pyridyl | H |
| Ia1.150 | SO₂CH₃ | C₂H₅ | 2-N-Methylpyrrolyl | H |
| Ia1.151 | NO₂ | H | H | H |
| Ia1.152 | NO₂ | H | CH₃ | H |
| Ia1.153 | NO₂ | H | C₂H₅ | H |
| Ia1.154 | NO₂ | H | n-C₃H₇ | H |
| Ia1.155 | NO₂ | H | n-C₄H₉ | H |
| Ia1.156 | NO₂ | H | CHO | H |
| Ia1.157 | NO₂ | H | COCH₃ | H |
| Ia1.158 | NO₂ | H | COOCH₃ | H |
| Ia1.159 | NO₂ | H | CO₂C₂H₅ | H |
| Ia1.160 | NO₂ | H | C₆H₅ | H |
| Ia1.161 | NO₂ | H | CH=NOCH₃ | H |
| Ia1.162 | NO₂ | H | C(CH₃)=NOCH₃ | H |
| Ia1.163 | NO₂ | H | COOH | C₂H₅ |
| Ia1.164 | NO₂ | H | COOMe | C₂H₅ |
| Ia1.165 | NO₂ | H | COOC₂H₅ | C₂H₅ |
| Ia1.166 | NO₂ | H | COOCH₂C₆H₅ | C₂H₅ |
| Ia1.167 | NO₂ | H | COOC (CH₃)₃ | C₂H₅ |
| Ia1.168 | NO₂ | H | CH=NOCH₃ | C₂H₅ |
| Ia1.169 | NO₂ | H | CH=NOC₂H₅ | C₂H₅ |
| Ia1.170 | NO₂ | H | CH=NOCH₂C₆H₅ | C₂H₅ |
| Ia1.171 | NO₂ | H | CH=NOCH(CH₃)₂ | C₂H₅ |
| Ia1.172 | NO₂ | H | C (CH₃)=NOCH) | C₂H₅ |
| Ia1.173 | NO₂ | H | C(CH₃)=NOC₂H₅ | C₂H₅ |
| Ia1.174 | NO₂ | H | C(CH₃)=NOCH(CH₃)₂ | C₂H₅ |
| Ia1.175 | NO₂ | H | C(CH₃)=NOCH₂C₆H₅ | C₂H₅ |
| Ia1.176 | NO₂ | H | CH=NOCH₂-CH=CH₂ | C₂H₅ |
| Ia1.177 | NO₂ | H | CH=NOCH₂-C≡CH | C₂H₅ |
| Ia1.178 | NO₂ | H | CH₂-CHO | C₂H₅ |
| Ia1.179 | NO₂ | H | CH₂-CH=NOCH₃ | C₂H₅ |
| Ia1.180 | NO₂ | H | CH₂-CH=NOC₂H₅ | C₂H₅ |

- Ebenso außergewöhnlich bevorzugt sind die Verbindungen Ia2, insbesondere die Verbindungen Ia2.001-Ia2.180, die sich von den entsprechenden Verbindungen Ia1.001-Ia1.180 dadurch unterscheiden, daß R¹⁴ für Ethyl steht: 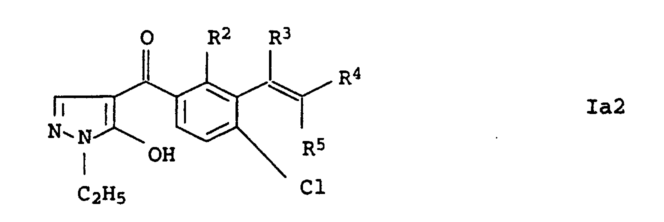
- Ebenso außergewöhnlich bevorzugt sind die Verbindungen Ia3, insbesondere die Verbindungen Ia3.001-Ia3.180, die sich von den entsprechenden Verbindungen Ia1.001-Ia1.180 dadurch unterscheiden, daß R¹⁴ für n-Propyl steht: 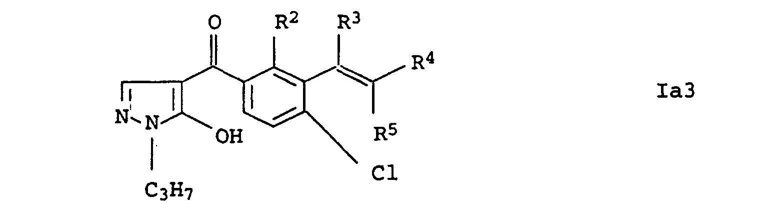
- Ebenso außergewöhnlich bevorzugt sind die Verbindungen Ia4, insbesondere die Verbindungen Ia4.001-Ia4.180, die sich von den entsprechenden Verbindungen Ia1.001-Ia1.180 dadurch unterscheiden, daß R¹⁴ für n-Butyl stehen: 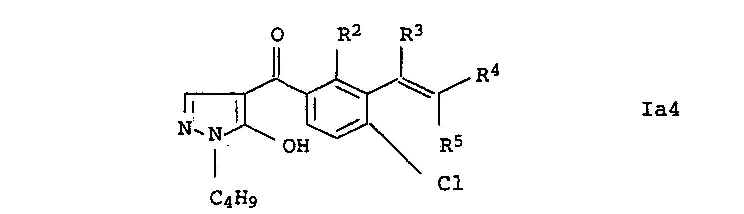
- Ebenso außergewöhnlich bevorzugt sind die Verbindungen Ia5, insbesondere die Verbindungen Ia5.001-Ia5.180, die sich von den entsprechenden Verbindungen Ia1.001-Ia1.180 dadurch unterscheiden, daß R¹⁴ für iso-Butyl steht: 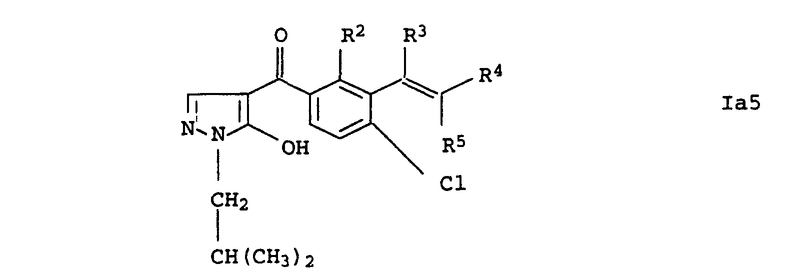
- Ebenso außergewöhnlich bevorzugt sind die Verbindungen Ia6, insbesondere die Verbindungen Ia6.001-Ia6.180, die sich von den entsprechenden Verbindungen Ia1.001-Ia1.180 dadurch unterscheiden, daß R¹⁵ für Methyl steht: 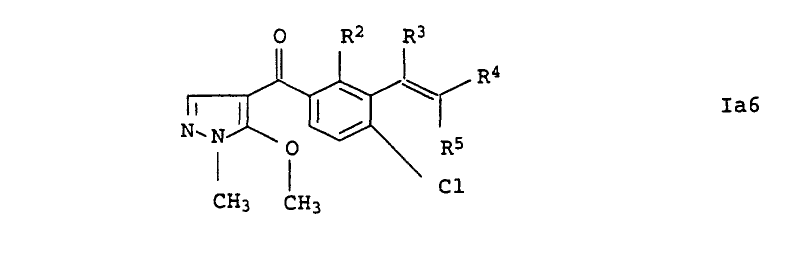
- Ebenso außergewöhnlich bevorzugt sind die Verbindungen Ia7, insbesondere die Verbindungen Ia7.001-Ia7.180, die sich von den entsprechenden Verbindungen Ia1.001-Ia1.180 dadurch unterscheiden, daß R¹⁴ für Ethyl und R¹⁵ für Methyl stehen: 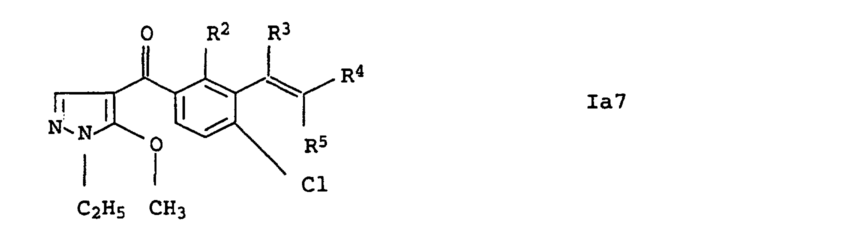
- Ebenso außergewöhnlich bevorzugt sind die Verbindungen Ia8, insbesondere die Verbindungen Ia8.001-Ia8.180, die sich von den entsprechenden Verbindungen Ia1.001-Ia1.180 dadurch unterscheiden, daß R¹⁴ für n-Propyl und R¹⁵ für Methyl stehen: 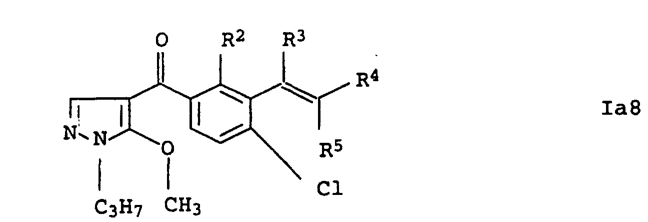
- Ebenso außergewöhnlich bevorzugt sind die Verbindungen Ia9, insbesondere die Verbindungen Ia9.001-Ia9.180, die sich von den entsprechenden Verbindungen Ia1.001-Ia1.180 dadurch unterscheiden, daß R¹⁴ für n-Butyl und R¹⁵ für Methyl stehen: 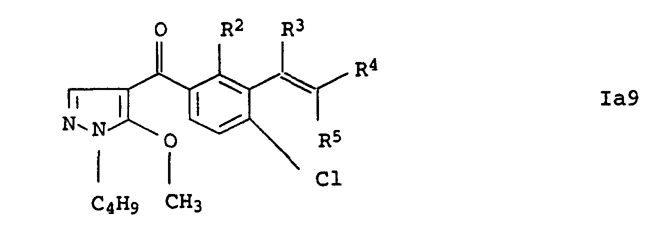
- Ebenso außergewöhnlich bevorzugt sind die Verbindungen Ia10, insbesondere die Verbindungen Ia10.001-Ia10.180, die sich von den entsprechenden Verbindungen Ia1.001-Ia1.180 dadurch unterscheiden, daß R¹⁴ für iso-Butyl und R¹⁵ für Methyl stehen: 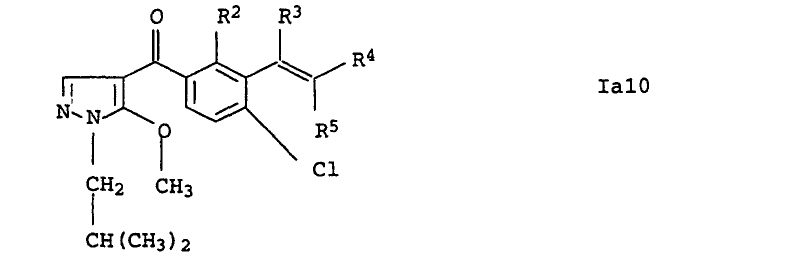
- Ebenso außergewöhnlich bevorzugt sind die Verbindungen Ia11, insbesondere die Verbindungen Ia11.001-Ia11.180, die sich von den entsprechenden Verbindungen Ia1.001-Ia1.180 dadurch unterscheiden, daß R¹⁵ für Ethyl steht: 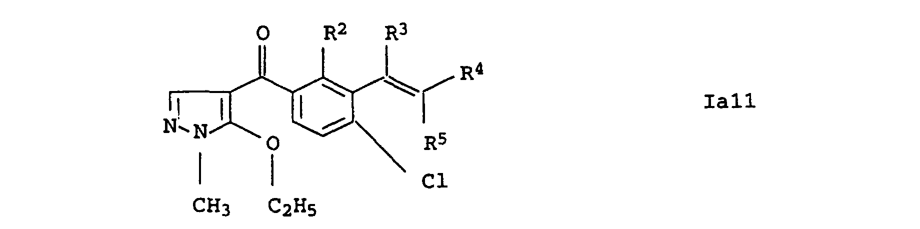
- Ebenso außergewöhnlich bevorzugt sind die Verbindungen Ia12, insbesondere die Verbindungen Ia12.001-Ia12.180, die sich von den entsprechenden Verbindungen Ia1.001-Ia1.180 dadurch unterscheiden, daß R¹⁴ und R¹⁵ für Ethyl stehen: 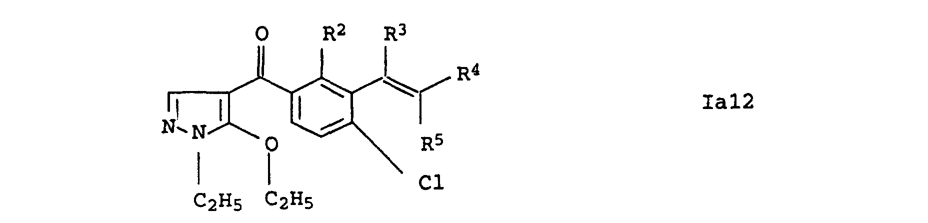
- Ebenso außergewöhnlich bevorzugt sind die Verbindungen Ia13, insbesondere die Verbindungen Ia13.001-Ia13.180, die sich von den entsprechenden Verbindungen Ia1.001-Ia1.180 dadurch unterscheiden, daß R¹⁴ für Propyl und R¹⁵ für Ethyl stehen: 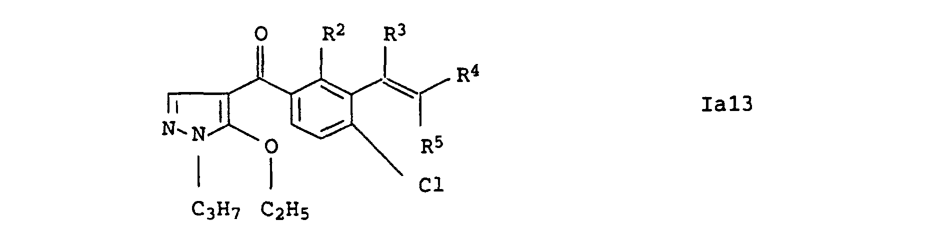
- Ebenso außergewöhnlich bevorzugt sind die Verbindungen Ia14, insbesondere die Verbindungen Ta14.001-Ia14.180, die sich von den entsprechenden Verbindungen Ia1.001-Ia1.180 dadurch unterscheiden, daß R¹⁴ für n-Butyl und R¹⁵ für Ethyl stehen: 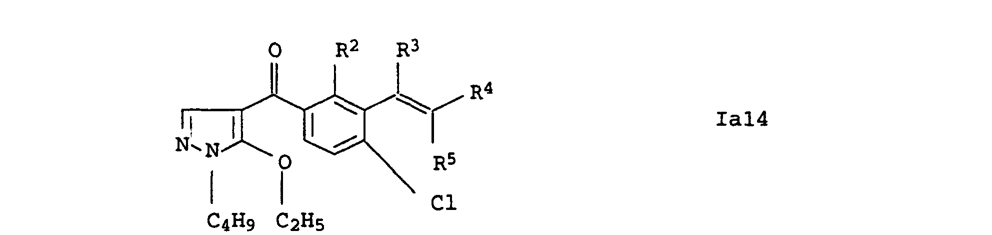
- Ebenso außergewöhnlich bevorzugt sind die Verbindungen Ia15, insbesondere die Verbindungen Ia15.001-Ia15.180, die sich von den entsprechenden Verbindungen Ia1.001-Ia1.180 dadurch unterscheiden, daß R¹⁴ für iso-Butyl und R¹⁵ für Ethyl stehen: 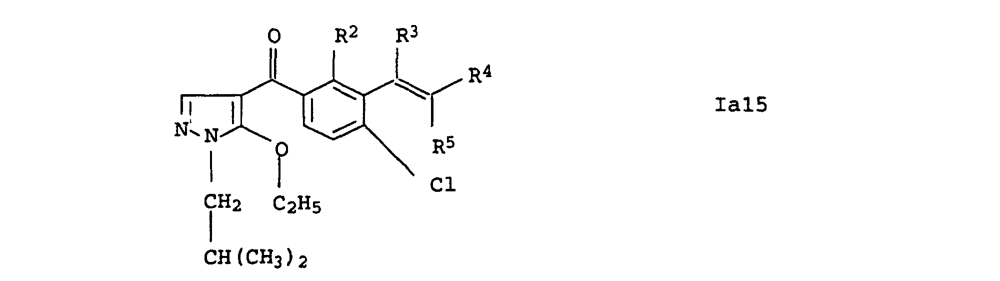
- Ebenso außergewöhnlich bevorzugt sind die Verbindungen Ia16, insbesondere die Verbindungen Ia16.001-Ia16.180, die sich von den entsprechenden Verbindungen Ia1.001-Ia1.180 dadurch unterscheiden, daß R¹⁵ für n-Propyl steht: 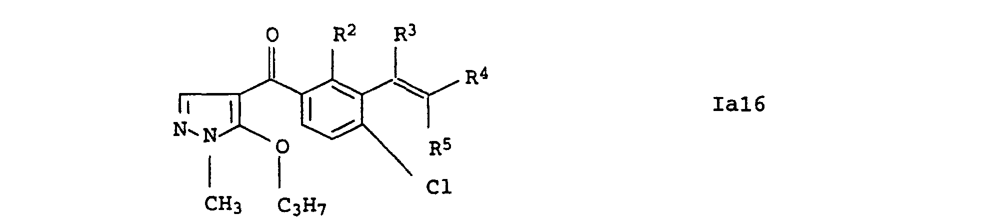
- Ebenso außergewöhnlich bevorzugt sind die Verbindungen Ia17, insbesondere die Verbindungen Ia17.001-Ia17.180, die sich von den entsprechenden Verbindungen Ia1.001-Ia1.180 dadurch unterscheiden, daß R¹⁴ für Ethyl und R¹⁵ für n-Propyl stehen: 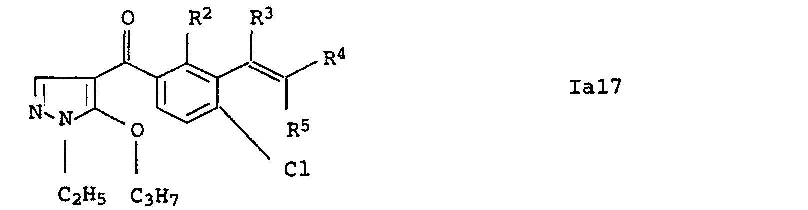
- Ebenso außergewöhnlich bevorzugt sind die Verbindungen Ia18, insbesondere die Verbindungen Ia18.001-Ia18.180, die sich von den entsprechenden Verbindungen Ia1.001-Ia1.180 dadurch unterscheiden, daß R¹⁴ und R¹⁵ für n-Propyl stehen: 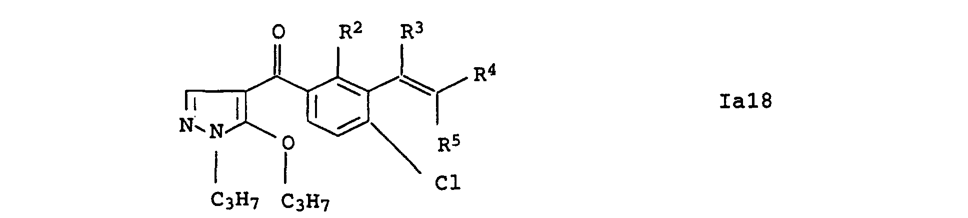
- Ebenso außergewöhnlich bevorzugt sind die Verbindungen Ia19, insbesondere die Verbindungen Ia19.001-Ia19.180, die sich von den entsprechenden Verbindungen Ia1.001-Ia1.180 dadurch unterscheiden, daß R¹⁴ für n-Butyl und R¹⁵ für n-Propyl stehen: 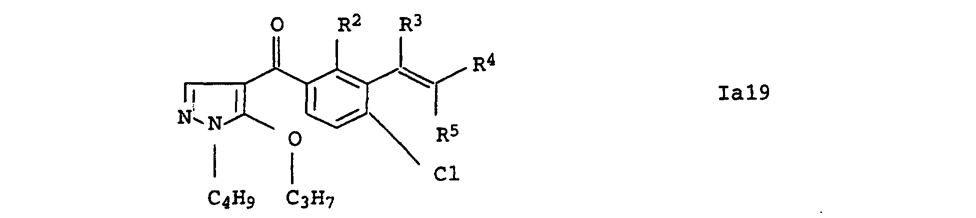
- Ebenso außergewöhnlich bevorzugt sind die Verbindungen Ia20, insbesondere die Verbindungen Ia20.001-Ia20.180, die sich von den entsprechenden Verbindungen Ia1.001-Ia1.180 dadurch unterscheiden, daß R¹⁴ für iso-Butyl und R¹⁵ für n-Propyl stehen: 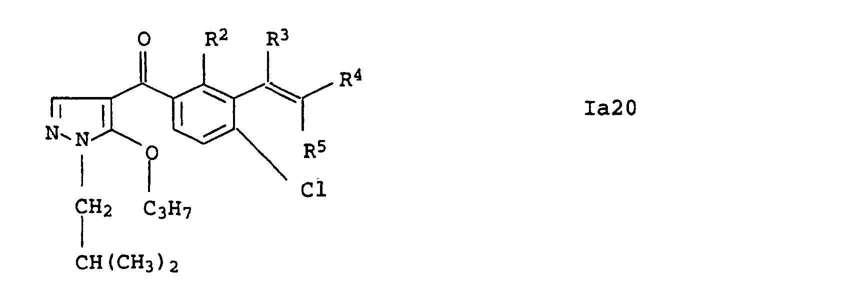
- Ebenso außergewöhnlich bevorzugt sind die Verbindungen Ia21, insbesondere die Verbindungen Ia21.001-Ia21.180, die sich von den entsprechenden Verbindungen Ia1.001-Ia1.180 dadurch unterscheiden, daß R¹⁵ für iso-Propyl steht: 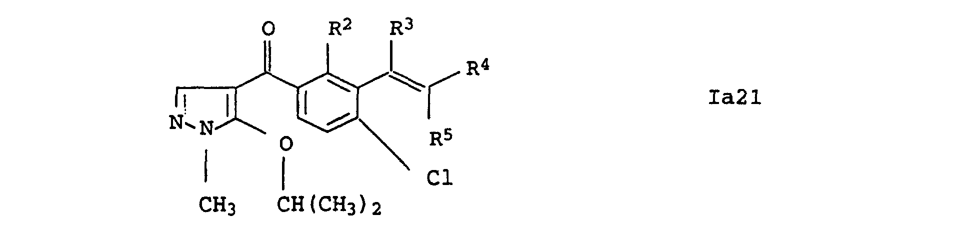
- Ebenso außergewöhnlich bevorzugt sind die Verbindungen Ia22, insbesondere die Verbindungen Ia22.001-Ia22.180, die sich von den entsprechenden Verbindungen Ia1.001-Ia1.180 dadurch unterscheiden, daß R¹⁴ für Ethyl und R¹⁵ für iso-Propyl stehen: 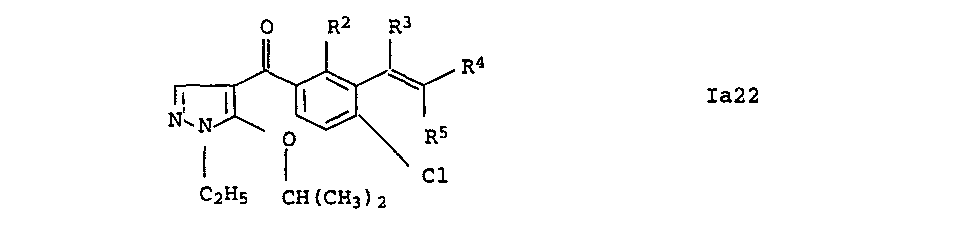
- Ebenso außergewöhnlich bevorzugt sind die Verbindungen Ia23, insbesondere die Verbindungen Ia23.001-Ia23.180, die sich von den entsprechenden Verbindungen Ia1.001-Ia1.180 dadurch unterscheiden, daß R¹⁴ für n-Propyl und R¹⁵ für iso-Propyl stehen: 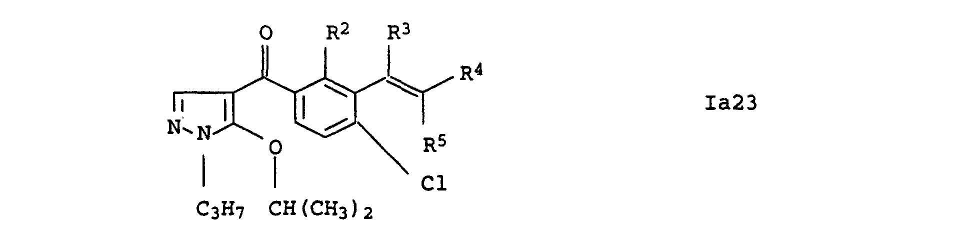
- Ebenso außergewöhnlich bevorzugt sind die Verbindungen Ia24, insbesondere die Verbindungen Ia24.001-Ia24.180, die sich von den entsprechenden Verbindungen Ia1.001-Ia1.180 dadurch unterscheiden, daß R¹⁴ für n-Butyl und R¹⁵ für iso-Propyl stehen: 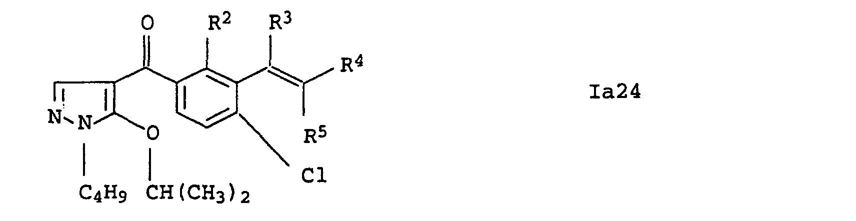
- Ebenso außergewöhnlich bevorzugt sind die Verbindungen Ia25, insbesondere die Verbindungen Ia25.001-Ia25.180, die sich von den entsprechenden Verbindungen Ia1.001-Ia1.180 dadurch unterscheiden, daß R¹⁴ für iso-Butyl und R¹⁵ für iso-Propyl stehen: 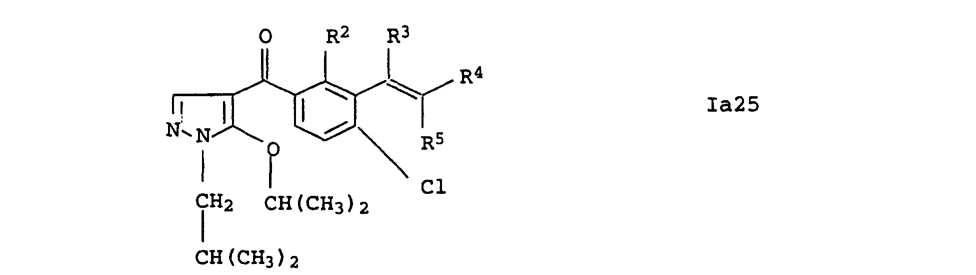
- Ebenso außergewöhnlich bevorzugt sind die Verbindungen Ia26, insbesondere die Verbindungen Ia26.001-Ia26.180, die sich von den entsprechenden Verbindungen Ia1.001-Ia1.180 dadurch unterscheiden, daß R¹⁵ für n-Butyl steht: 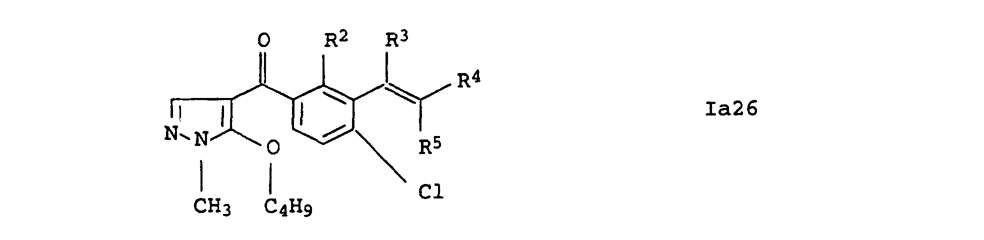
- Ebenso außergewöhnlich bevorzugt sind die Verbindungen Ia27, insbesondere die Verbindungen Ia27.001-Ia27.180, die sich von den entsprechenden Verbindungen Ia1.001-Ia1.180 dadurch unterscheiden, daß R¹⁴ für Ethyl und R¹⁵ für n-Butyl stehen: 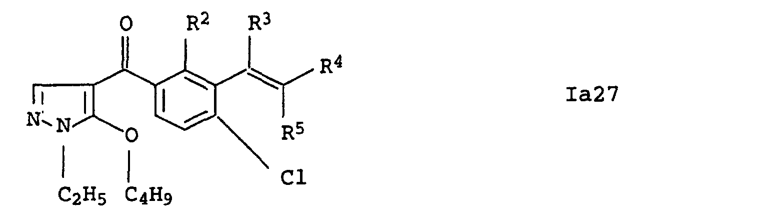
- Ebenso außergewöhnlich bevorzugt sind die Verbindungen Ia28, insbesondere die Verbindungen Ia28.001-Ia28.180, die sich von den entsprechenden Verbindungen Ia1.001-Ia1.180 dadurch unterscheiden, daß R¹⁴ für n-Propyl und R¹⁵ für n-Butyl stehen: 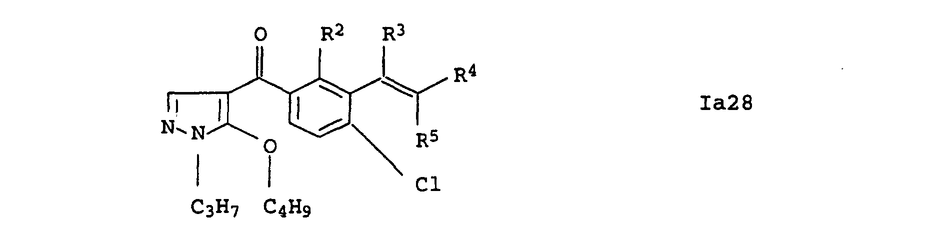
- Ebenso außergewöhnlich bevorzugt sind die Verbindungen Ia29, insbesondere die Verbindungen Ia29.001-Ia29.180, die sich von den entsprechenden Verbindungen Ia1.001-Ia1.180 dadurch unterscheiden, daß R¹⁴ und R¹⁵ für n-Butyl stehen: 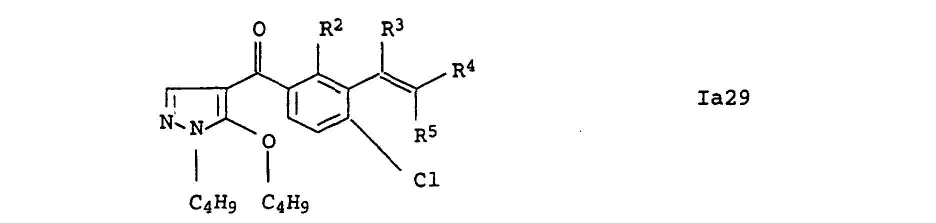
- Ebenso außergewöhnlich bevorzugt sind die Verbindungen Ia30, insbesondere die Verbindungen Ia30.001-Ia30.180, die sich von den entsprechenden Verbindungen Ia1.001-Ia1.180 dadurch unterscheiden, daß R¹⁴ für iso-Butyl und R¹⁵ für n-Butyl stehen: 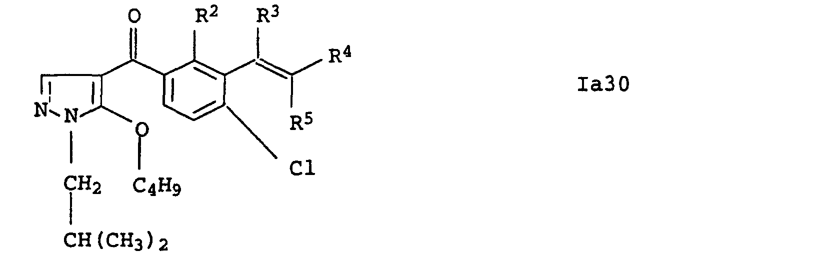
- Ebenso außergewöhnlich bevorzugt sind die Verbindungen Ia31, insbesondere die Verbindungen Ia31.001-Ia31.180, die sich von den entsprechenden Verbindungen Ia1.001-Ia1.180 dadurch unterscheiden, daß R¹⁵ für sec-Butyl steht: 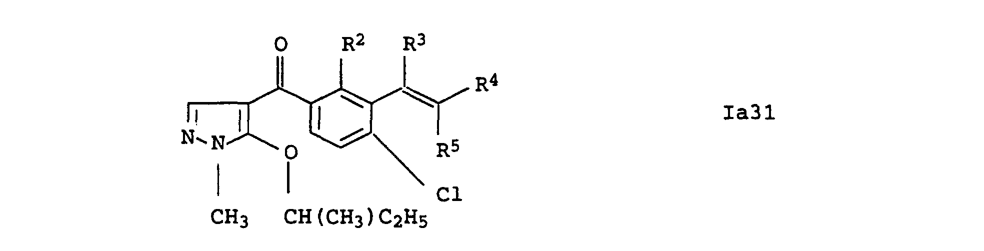
- Ebenso außergewöhnlich bevorzugt sind die Verbindungen Ia32, insbesondere die Verbindungen Ia32.001-Ia32.180, die sich von den entsprechenden Verbindungen Ia1.001-Ia1.180 dadurch unterscheiden, daß R¹⁴ für Ethyl und R¹⁵ sec-Butyl für stehen: 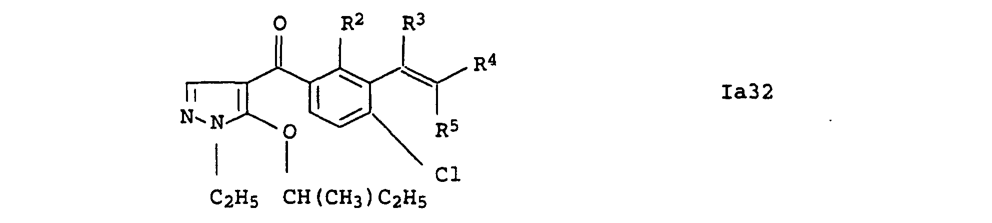
- Ebenso außergewöhnlich bevorzugt sind die Verbindungen Ia33, insbesondere die Verbindungen Ia33.001-Ia33.180, die sich von den entsprechenden Verbindungen Ia1.001-Ia1.180 dadurch unterscheiden, daß R¹⁴ für n-Propyl und R¹⁵ für sec-Butyl stehen: 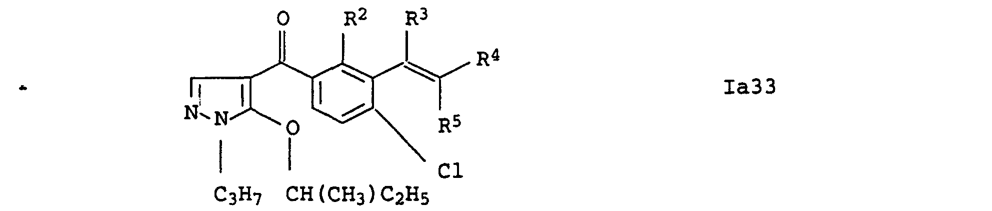
- Ebenso außergewöhnlich bevorzugt sind die Verbindungen Ia34, insbesondere die Verbindungen Ia34.001-Ia34.180, die sich von den entsprechenden Verbindungen Ia1.001-Ia1.180 dadurch unterscheiden, daß R¹⁴ für n-Butyl und R¹⁵ für sec-Butyl stehen: 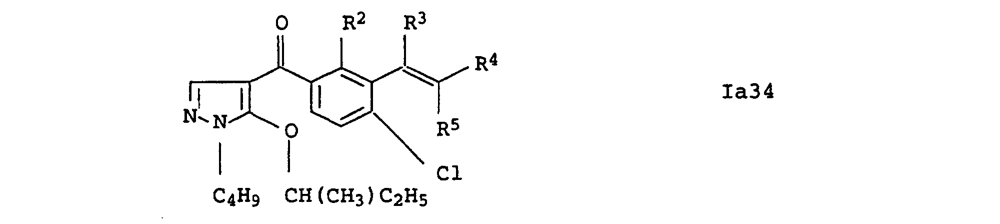
- Ebenso außergewöhnlich bevorzugt sind die Verbindungen Ia35, insbesondere die Verbindungen Ia35.001-Ia35.180, die sich von den entsprechenden Verbindungen Ia1.001-Ia1.180 dadurch unterscheiden, daß R¹⁴ für iso-Butyl und R¹⁵ für sec-Butyl stehen: 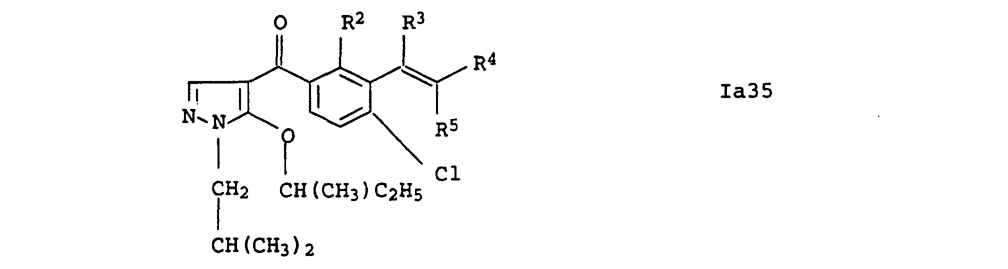
- Ebenso außergewöhnlich bevorzugt sind die Verbindungen Ia36, insbesondere die Verbindungen Ia36.001-Ia36.180, die sich von den entsprechenden Verbindungen Ia1.001-Ia1.180 dadurch unterscheiden, daß R¹⁵ für iso-Butyl steht: 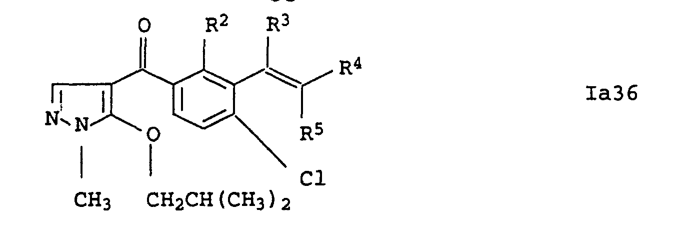
- Ebenso außergewöhnlich bevorzugt sind die Verbindungen Ia37, insbesondere die Verbindungen Ia37.001-Ia37.180, die sich von den entsprechenden Verbindungen Ia1.001-Ia1.180 dadurch unterscheiden, daß R¹⁵ für Ethyl und R¹⁶ für iso-Butyl stehen: 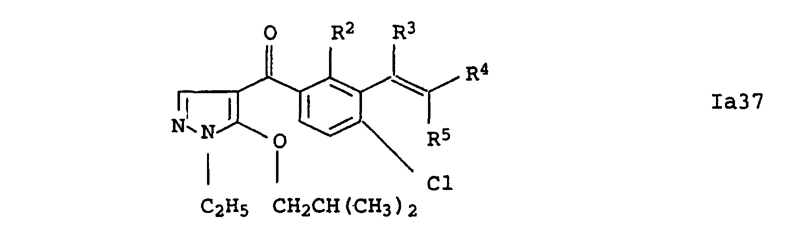
- Ebenso außergewöhnlich bevorzugt sind die Verbindungen Ia38, insbesondere die Verbindungen Ia38.001-Ia38.180, die sich von den entsprechenden Verbindungen Ia1.001-Ia1.180 dadurch unterscheiden, daß R¹⁴ für n-Propyl und R¹⁵ für iso-Butyl stehen: 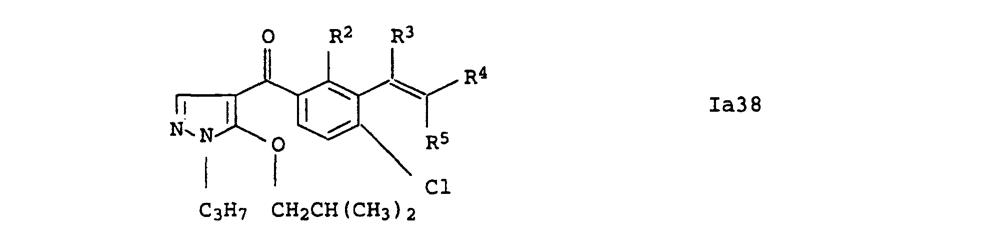
- Ebenso außergewöhnlich bevorzugt sind die Verbindungen Ia39, insbesondere die Verbindungen Ia39.001-Ia39.180, die sich von den entsprechenden Verbindungen Ia1.001-Ia1.180 dadurch unterscheiden, daß R¹⁴ für n-Butyl und R¹⁵ für iso-Butyl stehen: 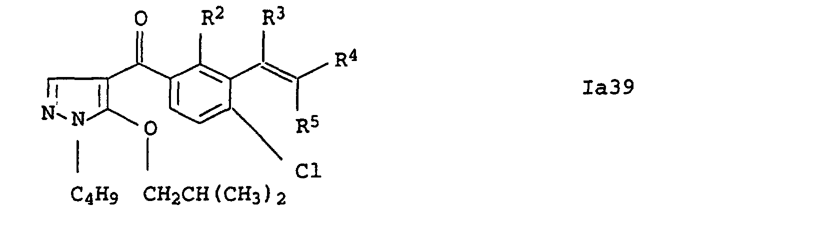
- Ebenso außergewöhnlich bevorzugt sind die Verbindungen Ia40, insbesondere die Verbindungen Ia40.001-Ia40.180, die sich von den entsprechenden Verbindungen Ia1.001-Ia1.180 dadurch unterscheiden, daß R¹⁴ und R¹⁵ für iso-Butyl stehen: 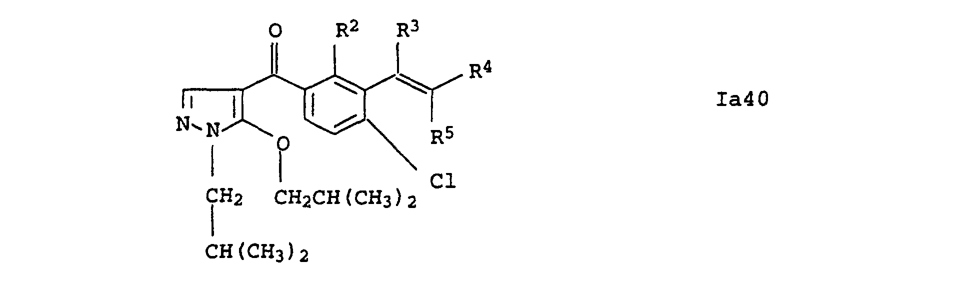
- Ebenso außergewöhnlich bevorzugt sind die Verbindungen Ia41, insbesondere die Verbindungen Ia41.001-Ia41.180, die sich von den entsprechenden Verbindungen Ia1.001-Ia1.180 dadurch unterscheiden, daß R¹⁵ für Methylcarbonyl steht: 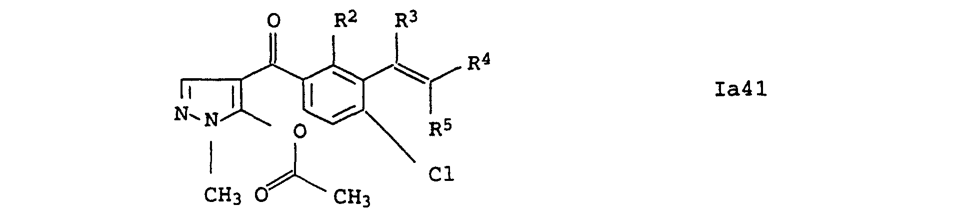
- Ebenso außergewöhnlich bevorzugt sind die Verbindungen Ia42, insbesondere die Verbindungen Ia42.001-Ia42.180, die sich von den entsprechenden Verbindungen Ia1.001-Ia1.180 dadurch unterscheiden, daß R¹⁴ für Ethyl und R¹⁵ für Methylcarbonyl stehen: 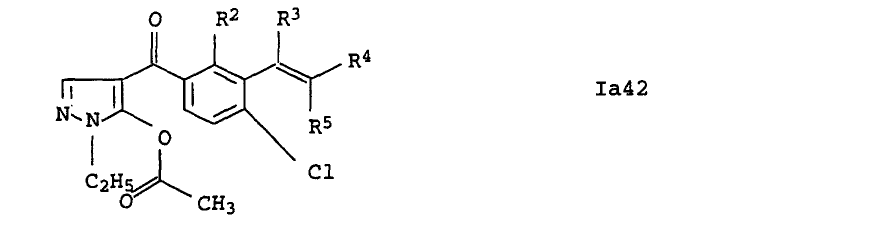
- Ebenso außergewöhnlich bevorzugt sind die Verbindungen Ia43, insbesondere die Verbindungen Ia43.001-Ia43.180, die sich von den entsprechenden Verbindungen Ia1.001-Ia1.180 dadurch unterscheiden, daß R¹⁴ für Propyl und R¹⁵ für Methylcarbonyl stehen: 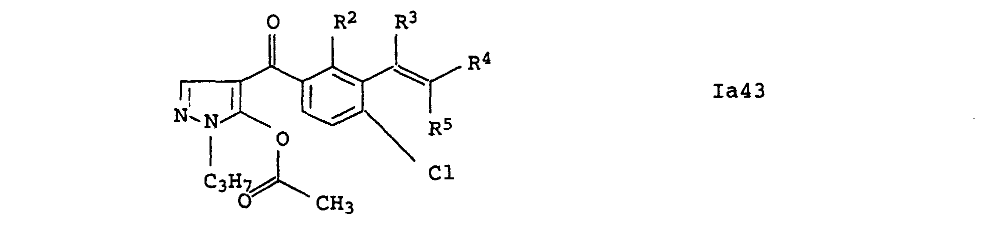
- Ebenso außergewöhnlich bevorzugt sind die Verbindungen Ia44, insbesondere die Verbindungen Ia44.001-Ia44.180, die sich von den entsprechenden Verbindungen Ia1.001-Ia1.180 dadurch unterscheiden, daß R¹⁴ für n-Butyl und R¹⁵ für Methylcarbonyl stehen: 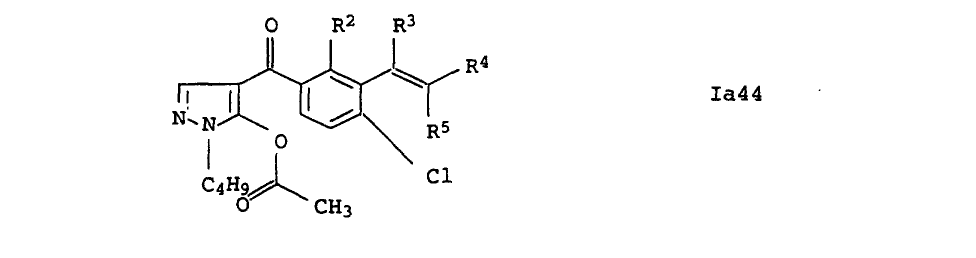
- Ebenso außergewöhnlich bevorzugt sind die Verbindungen Ia45, insbesondere die Verbindungen Ia45.001-Ia45.180, die sich von den entsprechenden Verbindungen Ia1.001-Ia1.180 dadurch unterscheiden, daß R¹⁴ für iso-Butyl und R¹⁵ für Methylcarbonyl stehen: 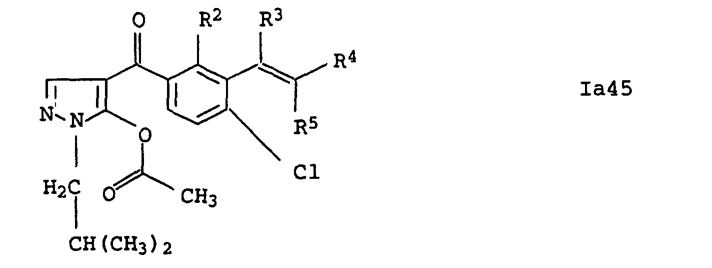
- Ebenso außergewöhnlich bevorzugt sind die Verbindungen Ia46, insbesondere die Verbindungen Ia46.001-Ia46.180, die sich von den entsprechenden Verbindungen Ia1.001-Ia1.180 dadurch unterscheiden, daß R¹⁵ für Ethylcarbonyl steht: 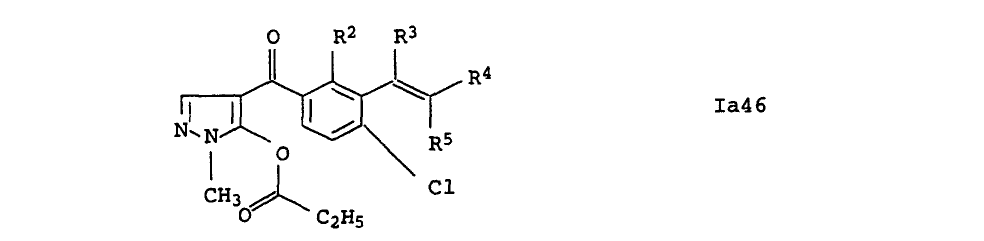
- Ebenso außergewöhnlich bevorzugt sind die Verbindungen Ia47, insbesondere die Verbindungen Ia47.001-Ia47.180, die sich von den entsprechenden Verbindungen Ia1.001-Ia1.180 dadurch unterscheiden, daß R¹⁴ für Ethyl und R¹⁵ für Ethylcarbonyl stehen: 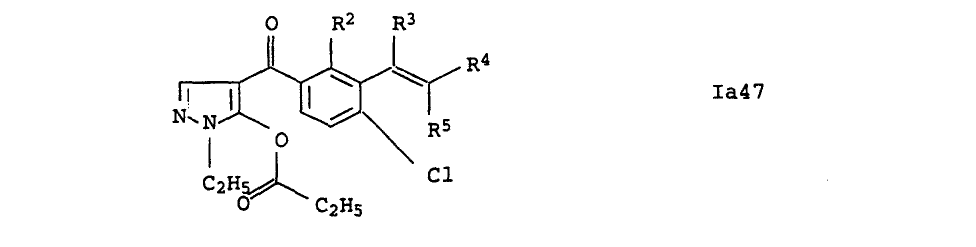
- Ebenso außergewöhnlich bevorzugt sind die Verbindungen Ia48, insbesondere die Verbindungen Ia48.001-Ia48.180, die sich von den entsprechenden Verbindungen Ia1.001-Ia1.180 dadurch unterscheiden, daß R¹⁴ für Propyl und R¹⁵ für Ethylcarbonyl stehen: 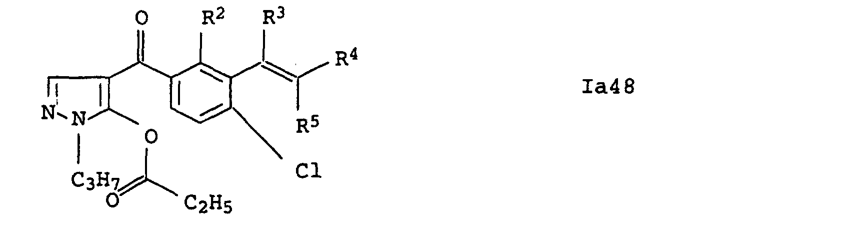
- Ebenso außergewöhnlich bevorzugt sind die Verbindungen Ia49, insbesondere die Verbindungen Ia49.001-Ia49.180, die sich von den entsprechenden Verbindungen Ia1.001-Ia1.180 dadurch unterscheiden, daß R¹⁴ für n-Butyl und R¹⁵ für Ethylcarbonyl stehen: 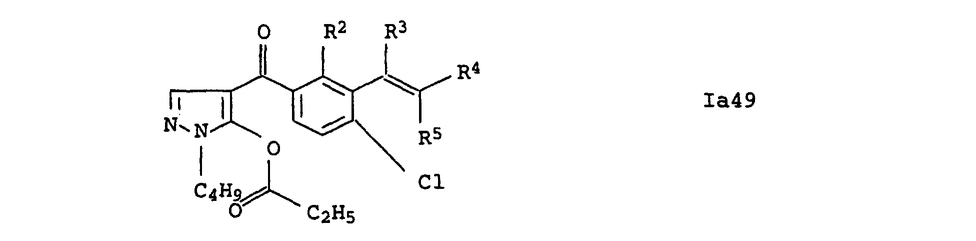
- Ebenso außergewöhnlich bevorzugt sind die Verbindungen Ia50, insbesondere die Verbindungen Ia50.001-Ia50.180, die sich von den entsprechenden Verbindungen Ia1.001-Ia1.180 dadurch unterscheiden, daß R¹⁴ für iso-Butyl und R¹⁵ für Ethylcarbonyl stehen: 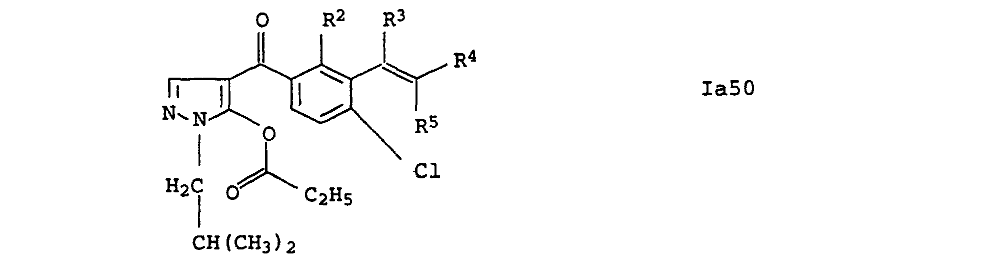
- Ebenso außergewöhnlich bevorzugt sind die Verbindungen Ia51, insbesondere die Verbindungen Ia51.001-Ia51.180, die sich von den entsprechenden Verbindungen Ia1.001-Ia1.180 dadurch unterscheiden, daß R¹⁵ für n-Propylcarbonyl steht: 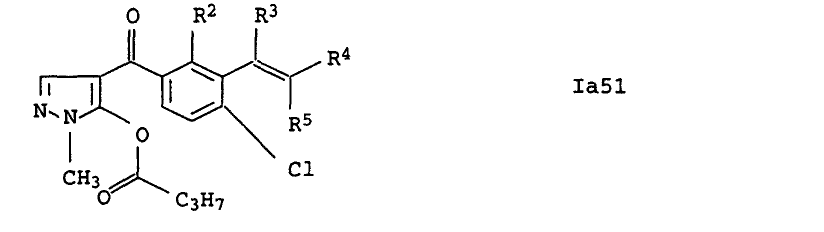
- Ebenso außergewöhnlich bevorzugt sind die Verbindungen Ia52, insbesondere die Verbindungen Ia52.001-Ia52.180, die sich von den entsprechenden Verbindungen Ia1.001-Ia1.180 dadurch unterscheiden, daß R¹⁴ für Ethyl und R¹⁵ für n-Propylcarbonyl stehen: 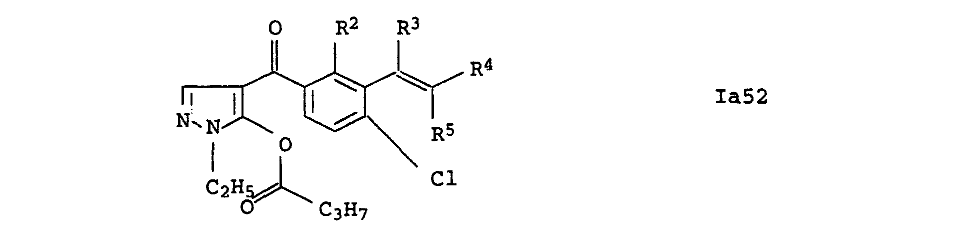
- Ebenso außergewöhnlich bevorzugt sind die Verbindungen Ia53, insbesondere die Verbindungen Ia53.001-Ia53.180, die sich von den entsprechenden Verbindungen Ia1.001-Ia1.180 dadurch unterscheiden, daß R¹⁴ für Propyl und R¹⁵ für n-Propylcarbonyl stehen: 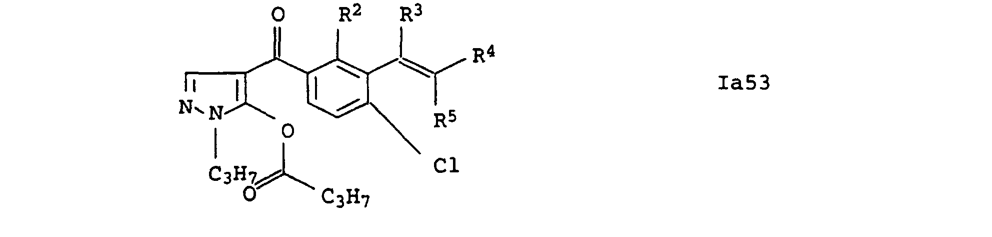
- Ebenso außergewöhnlich bevorzugt sind die Verbindungen Ia54, insbesondere die Verbindungen Ia54.001-Ia54.180, die sich von den entsprechenden Verbindungen Ia1.001-Ia1.180 dadurch unterscheiden, daß R¹⁴ für n-Butyl und R¹⁵ für n-Propylcarbonyl stehen: 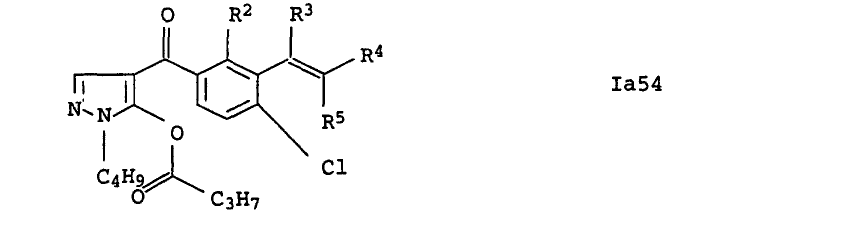
- Ebenso außergewöhnlich bevorzugt sind die Verbindungen Ia55, insbesondere die Verbindungen Ia55.001-Ia55.180, die sich von den entsprechenden Verbindungen Ia1.001-Ia1.180 dadurch unterscheiden, daß R¹⁴ für iso-Butyl und R¹⁵ für n-Propylcarbonyl stehen: 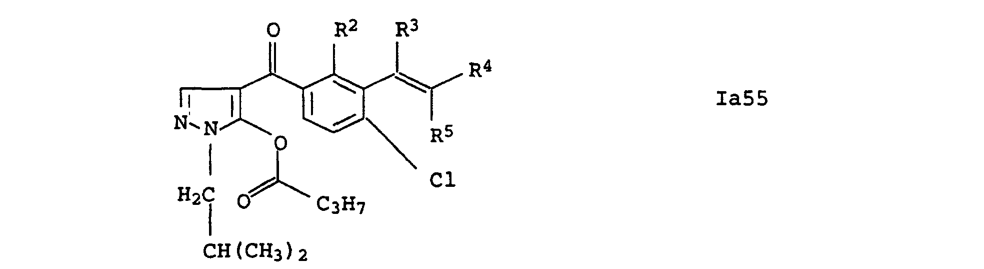
- Ebenso außergewöhnlich bevorzugt sind die Verbindungen Ia56, insbesondere die Verbindungen Ia56.001-Ia56.180, die sich von den entsprechenden Verbindungen Ia1.001-Ia1.180 dadurch unterscheiden, daß R¹⁵ für Trifluormethylcarbonyl steht: 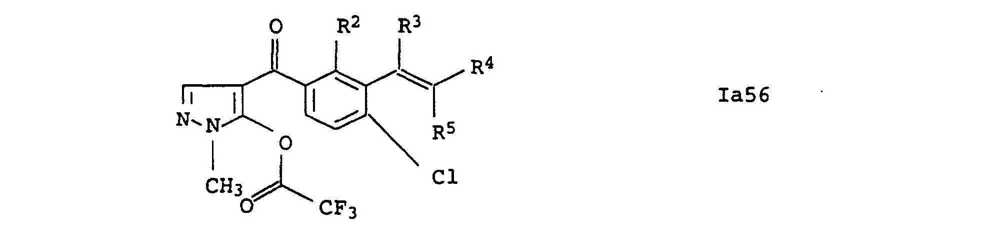
- Ebenso außergewöhnlich bevorzugt sind die Verbindungen Ia57, insbesondere die Verbindungen Ia57.001-Ia57.180, die sich von den entsprechenden Verbindungen Ia1.001-Ia1.180 dadurch unterscheiden, daß R¹⁴ für Ethyl und für R¹⁵ Trifluormethylcarbonyl stehen: 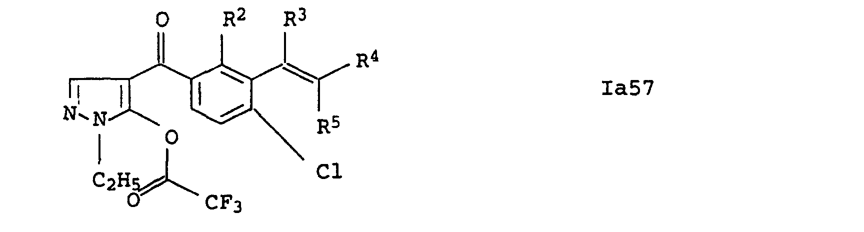
- Ebenso außergewöhnlich bevorzugt sind die Verbindungen Ia58, insbesondere die Verbindungen Ia58.001-Ia58.180, die sich von den entsprechenden Verbindungen Ia1.001-Ia1.180 dadurch unterscheiden, daß R¹⁴ für n-Propyl und R¹⁵ für Trifluormethylcarbonyl stehen: 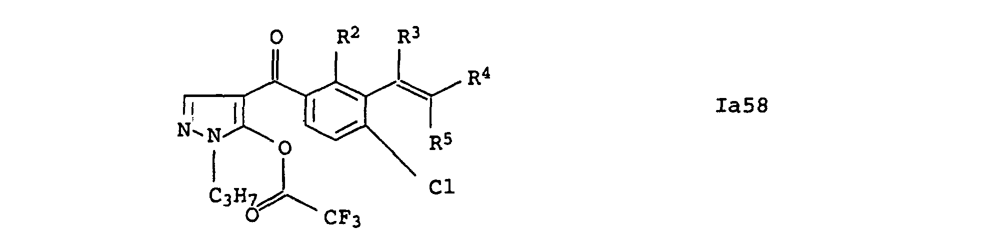
- Ebenso außergewöhnlich bevorzugt sind die Verbindungen Ia59, insbesondere die Verbindungen Ia59.001-Ia59.180, die sich von den entsprechenden Verbindungen Ia1.001-Ia1.180 dadurch unterscheiden, daß R¹⁴ für n-Butyl und R¹⁵ für Trifluormethylcarbonyl stehen: 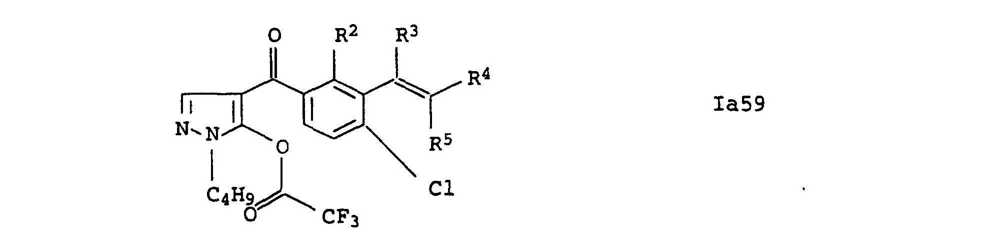
- Ebenso außergewöhnlich bevorzugt sind die Verbindungen Ia60, insbesondere die Verbindungen Ia60.001-Ia60.180, die sich von den entsprechenden Verbindungen Ia1.001-Ia1.180 dadurch unterscheiden, daß R¹⁴ für iso-Butyl und R¹⁵ für Trifluormethylcarbonyl stehen: 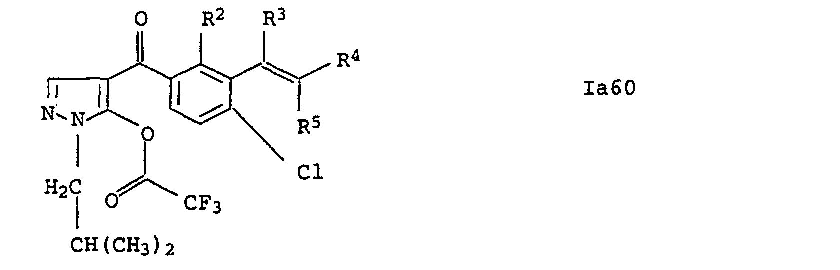
- Ebenso außergewöhnlich bevorzugt sind die Verbindungen Ia61, insbesondere die Verbindungen Ia61.001-Ia61.180, die sich von den entsprechenden Verbindungen Ia1.001-Ia1.180 dadurch unterscheiden, daß R¹⁵ für Methylsulfonyl steht: 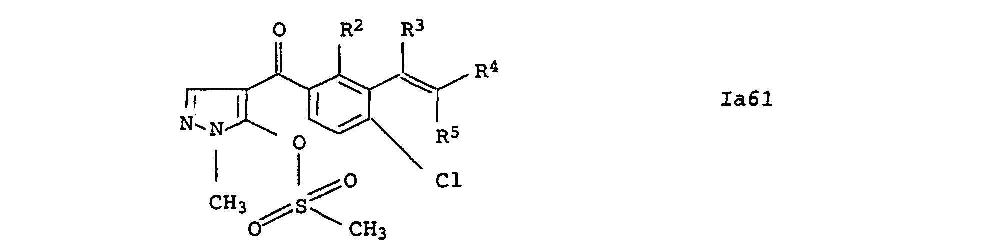
- Ebenso außergewöhnlich bevorzugt sind die Verbindungen Ia62, insbesondere die Verbindungen Ia62.001-Ia62.180, die sich von den entsprechenden Verbindungen Ia1.001-Ia1.180 dadurch unterscheiden, daß R¹⁴ für Ethyl und für R¹⁵ Methylsulfonyl stehen: 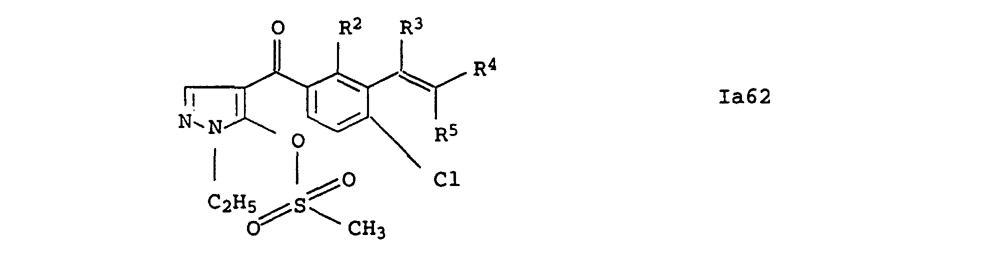
- Ebenso außergewöhnlich bevorzugt sind die Verbindungen Ia63, insbesondere die Verbindungen Ia63.001-Ia63.180, die sich von den entsprechenden Verbindungen Ia1.001-Ia1.180 dadurch unterscheiden, daß R¹⁴ für n-Propyl und R¹⁵ für Methylsulfonyl stehen: 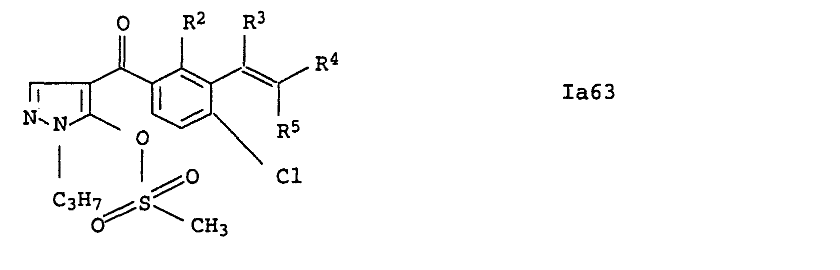
- Ebenso außergewöhnlich bevorzugt sind die Verbindungen Ia64, insbesondere die Verbindungen Ia64.001-Ia64.180, die sich von den entsprechenden Verbindungen Ia1.001-Ia2.180 dadurch unterscheiden, daß R¹⁴ für n-Butyl und R¹⁵ für Methylsulfonyl stehen: 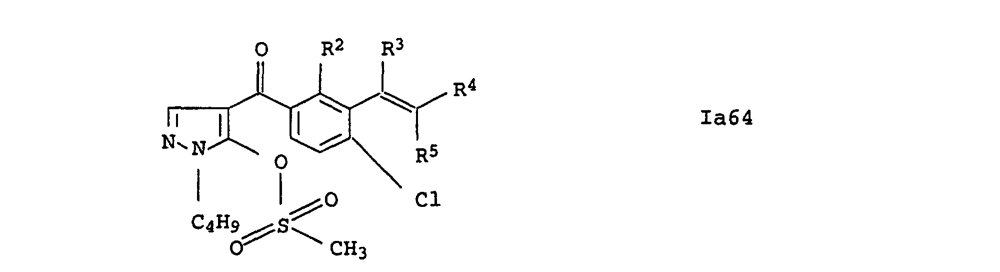
- Ebenso außergewöhnlich bevorzugt sind die Verbindungen Ia65, insbesondere die Verbindungen Ia65.001-Ia65.180, die sich von den entsprechenden Verbindungen Ia1.001-Ia1.180 dadurch unterscheiden, daß R¹⁴ für iso-Butyl und R¹⁵ für Methylsulfonyl stehen: 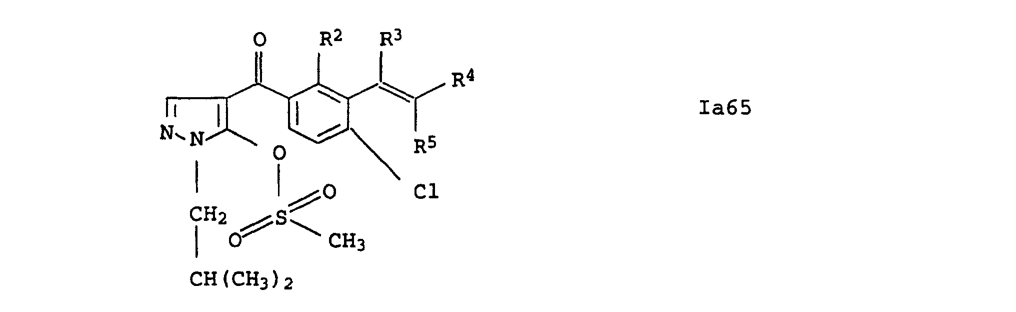
- Ebenso außergewöhnlich bevorzugt sind die Verbindungen Ia66, insbesondere die Verbindungen Ia66.001-Ia66.180, die sich von den entsprechenden Verbindungen Ia1.001-Ia1.180 dadurch unterscheiden, daß R¹⁵ für Ethylsulfonyl steht: 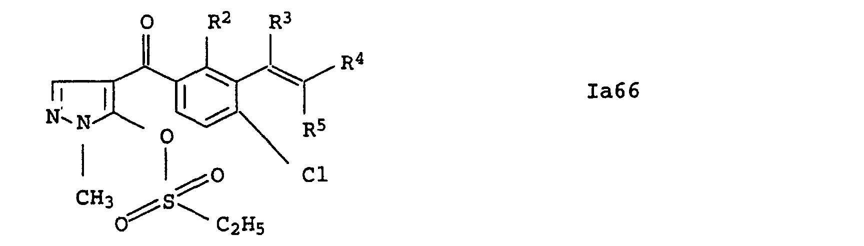
- Ebenso außergewöhnlich bevorzugt sind die Verbindungen Ia67, insbesondere die Verbindungen Ia67.001-Ia67.180, die sich von den entsprechenden Verbindungen Ia1.001-Ia1.180 dadurch unterscheiden, daß R¹⁴ für Ethyl und für R¹⁵ Ethylsulfonyl stehen: 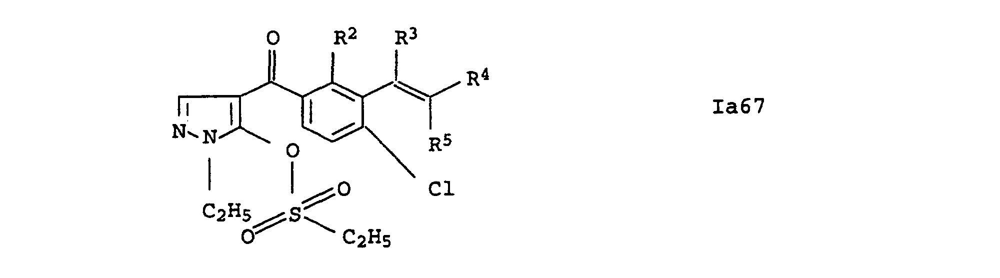
- Ebenso außergewöhnlich bevorzugt sind die Verbindungen Ia68, insbesondere die Verbindungen Ia68.001-Ia68.180, die sich von den entsprechenden Verbindungen Ia1.001-IaI.180 dadurch unterscheiden, daß R¹⁴ für Propyl und R¹⁵ für Ethylsulfonyl stehen: 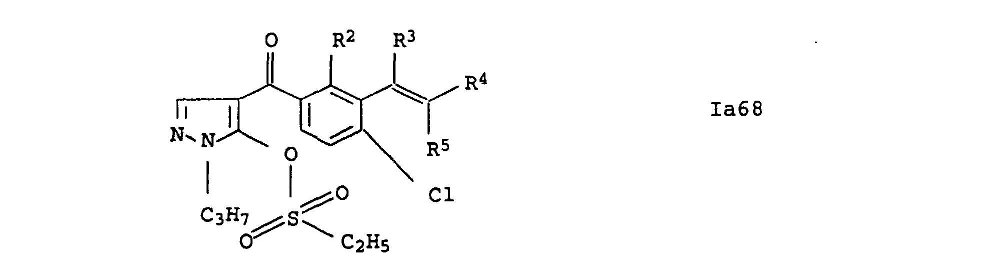
- Ebenso außergewöhnlich bevorzugt sind die Verbindungen Ia69, insbesondere die Verbindungen Ia69.001-Ia69.180, die sich von den entsprechenden Verbindungen Ia1.001-Ia1.180 dadurch unterscheiden, daß R¹⁴ für n-Butyl und R¹⁵ für Ethylsulfonyl stehen: 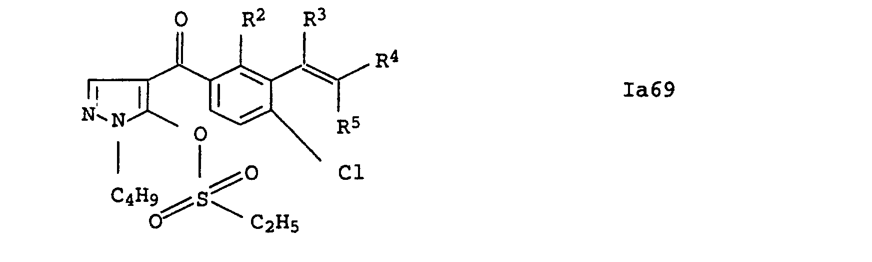
- Ebenso außergewöhnlich bevorzugt sind die Verbindungen Ia70, insbesondere die Verbindungen Ia70.001-Ia70.180, die sich von den entsprechenden Verbindungen Ia1.001-Ia1.180 dadurch unterscheiden, daß R¹⁴ für iso-Butyl steht und R¹⁵ für Ethylsulfonyl stehen: 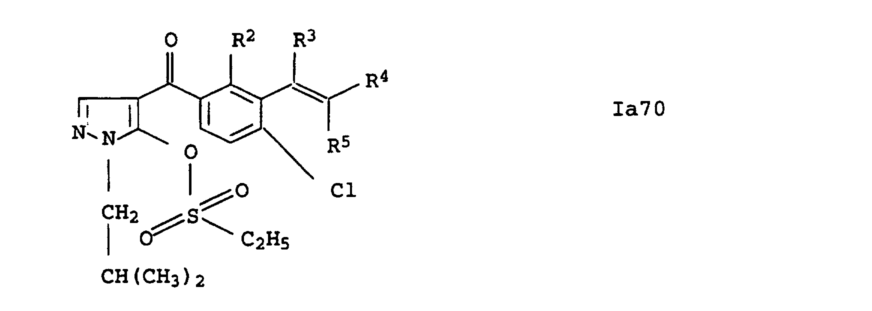
- Ebenso außergewöhnlich bevorzugt sind die Verbindungen Ia71, insbesondere die Verbindungen Ia71.001-Ia71.180, die sich von den entsprechenden Verbindungen Ia1.001-Ia1.180 dadurch unterscheiden, daß R¹⁵ für n-Propylsulfonyl steht: 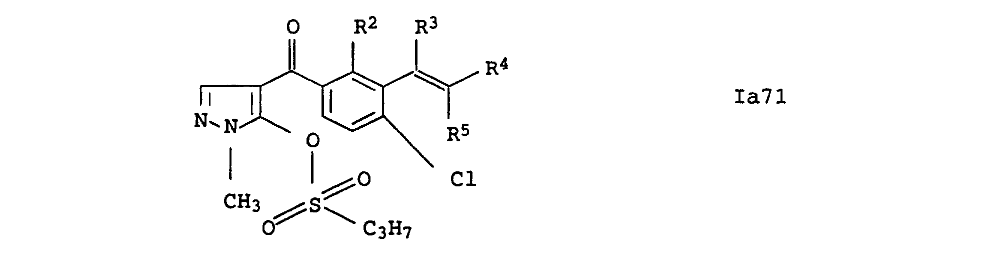
- Ebenso außergewöhnlich bevorzugt sind die Verbindungen Ia72, insbesondere die Verbindungen Ia72.001-Ia72.180, die sich von den entsprechenden Verbindungen Ia1.001-Ia1.180 dadurch unterscheiden, daß R¹⁴ für Ethyl und für R¹⁵ für n-Propylsulfonyl stehen: 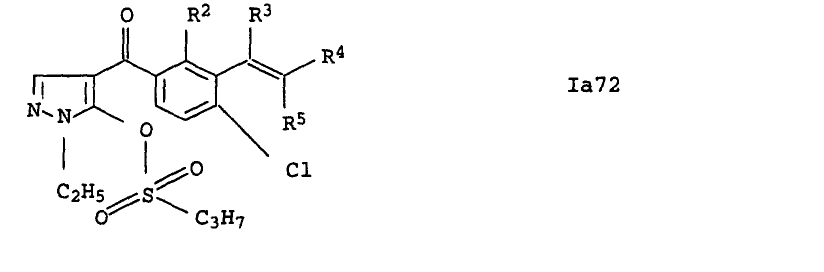
- Ebenso außergewöhnlich bevorzugt sind die Verbindungen Ia73, insbesondere die Verbindungen Ia73.001-Ia73.180, die sich von den entsprechenden Verbindungen Ia1.001-Ia1.180 dadurch unterscheiden, daß R¹⁴ für n-Propyl und R¹⁵ für n-Propylsulfonyl stehen: 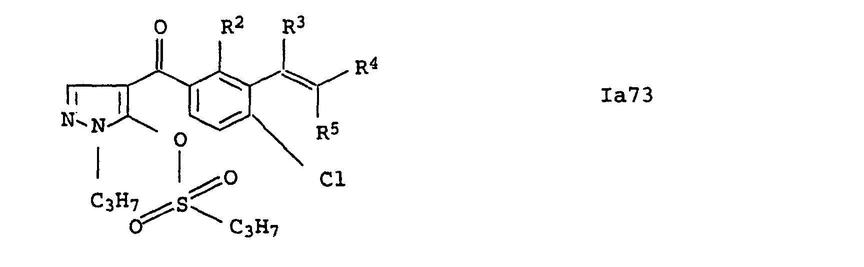
- Ebenso außergewöhnlich bevorzugt sind die Verbindungen Ia74, insbesondere die Verbindungen Ia74.001-Ia74.180, die sich von den entsprechenden Verbindungen Ia1.001-Ia1.180 dadurch unterscheiden, daß R¹⁴ für n-Butyl und R¹⁵ für n-Propylsulfonyl stehen: 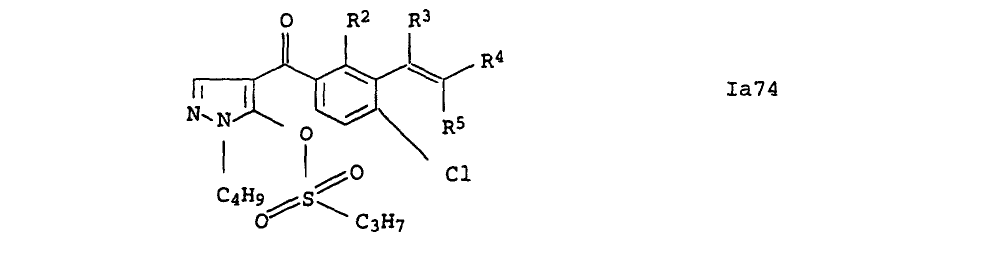
- Ebenso außergewöhnlich bevorzugt sind die Verbindungen Ia75, insbesondere die Verbindungen Ia75.001-Ia75.180, die sich von den entsprechenden Verbindungen Ia1.001-Ia1.180 dadurch unterscheiden, daß R¹⁴ für iso-Butyl und R¹⁵ für n-Propylsulfonyl stehen: 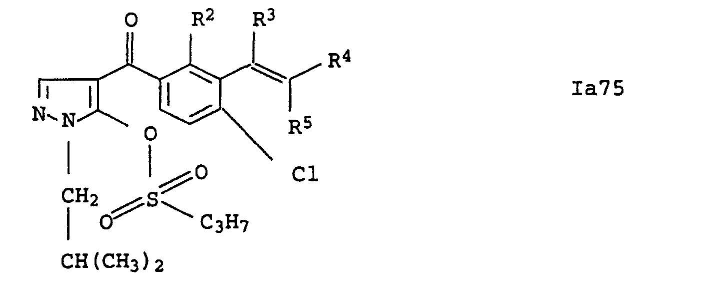
- Ebenso außergewöhnlich bevorzugt sind die Verbindungen Ia76, insbesondere die Verbindungen Ia76.001-Ia76.180, die sich von den entsprechenden Verbindungen Ia1.001-Ia1.180 dadurch unterscheiden, daß R¹⁵ für iso-Propylsulfonyl steht: 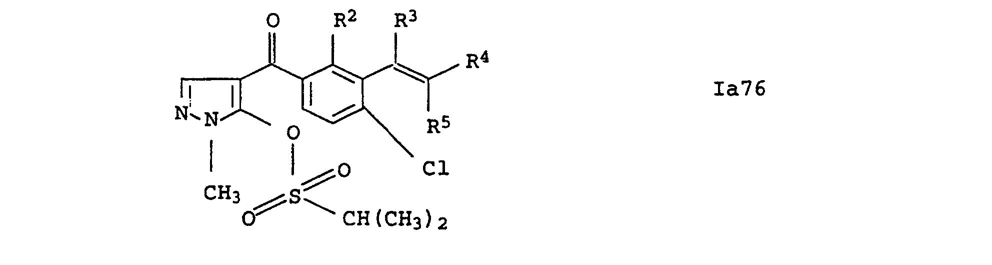
- Ebenso außergewöhnlich bevorzugt sind die Verbindungen Ia77, insbesondere die Verbindungen Ia77.001-Ia77.180, die sich von den entsprechenden Verbindungen Ia1.001-Ia1.180 dadurch unterscheiden, daß R¹⁴ für Ethyl und R¹⁵ für iso-Propylsulfonyl stehen: 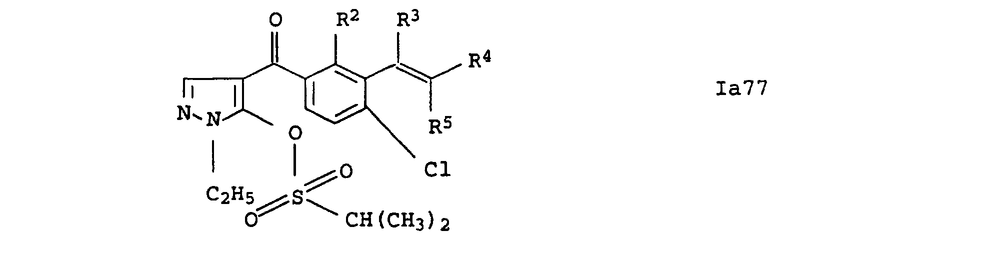
- Ebenso außergewöhnlich bevorzugt sind die Verbindungen Ia78, insbesondere die Verbindungen Ia78.001-Ia78.180, die sich von den entsprechenden Verbindungen Ia1.001-Ia1.180 dadurch unterscheiden, daß R¹⁴ für Propyl und R¹⁵ für iso-Propylsulfonyl stehen: 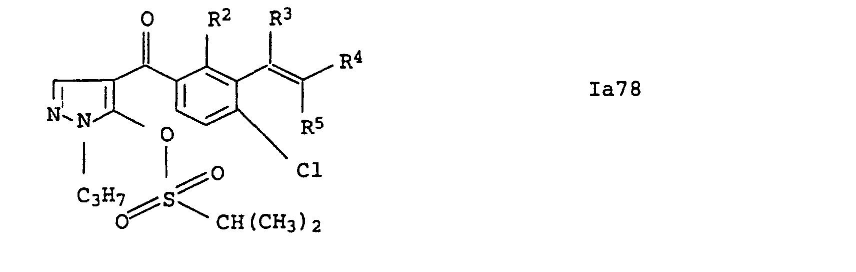
- Ebenso außergewöhnlich bevorzugt sind die Verbindungen Ia79, insbesondere die Verbindungen Ia79.001-Ia79.180, die sich von den entsprechenden Verbindungen Ia1.001-Ia1.180 dadurch unterscheiden, daß R¹⁴ für n-Butyl und R¹⁵ für iso-Propylsulfonyl stehen: 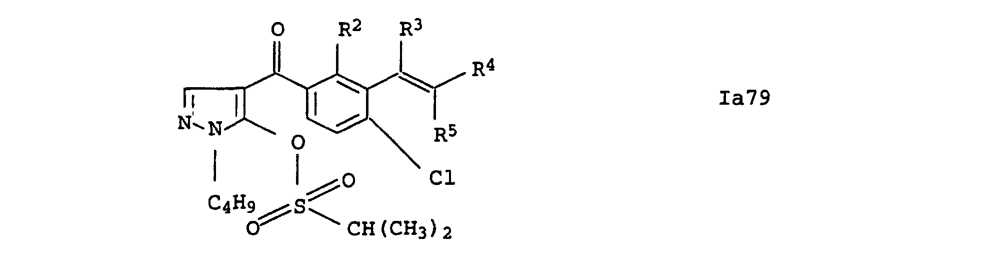
- Ebenso außergewöhnlich bevorzugt sind die Verbindungen Ia80, insbesondere die Verbindungen Ia80.001-Ia80.180, die sich von den entsprechenden Verbindungen Ia1.001-Ia1.180 dadurch unterscheiden, daß R¹⁴ für iso-Butyl und R¹⁵ für iso-Propylsulfonyl stehen: 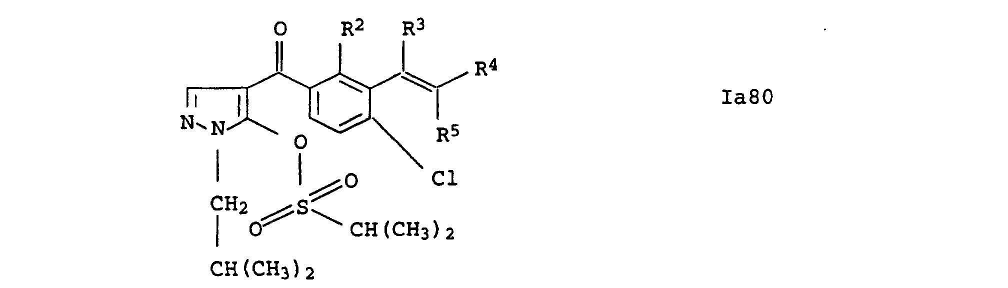
- Ebenso außergewöhnlich bevorzugt sind die Verbindungen Ia81, insbesondere die Verbindungen Ia81.001-Ia81.180, die sich von den entsprechenden Verbindungen Ia1.001-Ia1.180 dadurch unterscheiden, daß R¹⁵ für n-Butylsulfonyl steht: 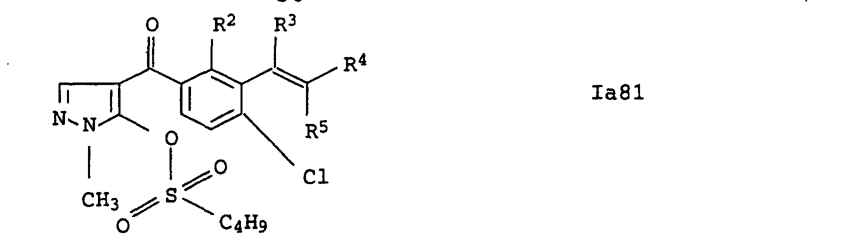
- Ebenso außergewöhnlich bevorzugt sind die Verbindungen Ia82, insbesondere die Verbindungen Ia82.001-Ia82.180, die sich von den entsprechenden Verbindungen Ia1.001-Ia1.180 dadurch unterscheiden, daß R¹⁴ für Ethyl und R¹⁵ für n-Butylsulfonyl stehen: 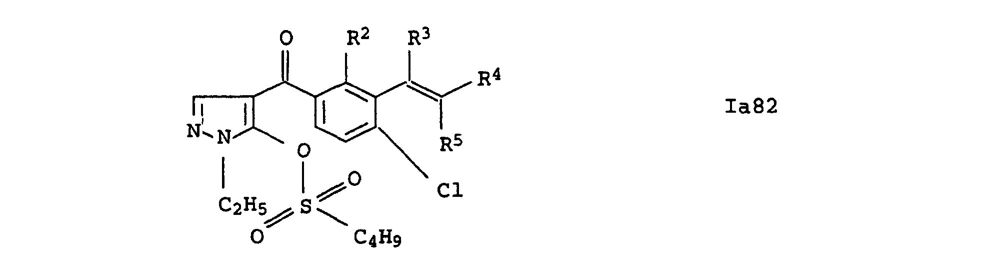
- Ebenso außergewöhnlich bevorzugt sind die Verbindungen Ia83, insbesondere die Verbindungen Ia83.001-Ia83.180, die sich von den entsprechenden Verbindungen Ia1.001-Ia1.180 dadurch unterscheiden, daß R¹⁴ für n-Propyl und R¹⁵ für n-Butylsulfonyl stehen: 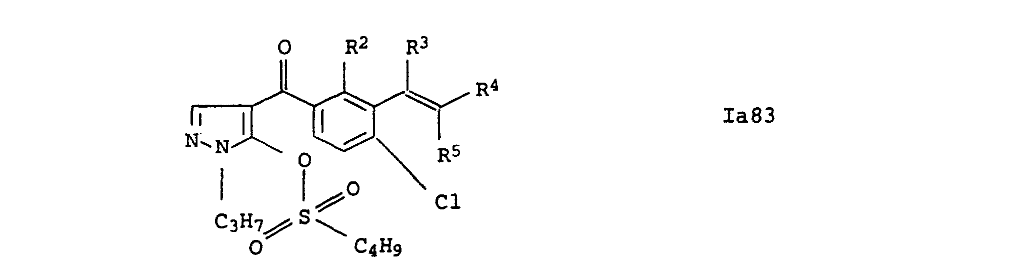
- Ebenso außergewöhnlich bevorzugt sind die Verbindungen Ia84, insbesondere die Verbindungen Ia84.001-Ia84.180, die sich von den entsprechenden Verbindungen Ia1.001-Ia1.180 dadurch unterscheiden, daß R¹⁴ für n-Butyl und R¹⁵ für n-Butylsulfonyl stehen: 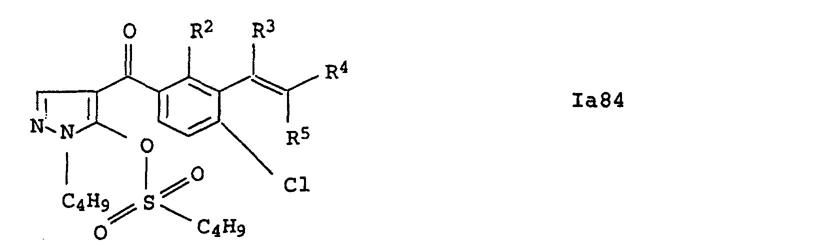
- Ebenso außergewöhnlich bevorzugt sind die Verbindungen Ia85, insbesondere die Verbindungen Ia85.001-Ia85.180, die sich von den entsprechenden Verbindungen Ia1.001-Ta1.180 dadurch unterscheiden, daß R¹⁴ für iso-Butyl und R¹⁵ für n-Butylsulfonyl stehen: 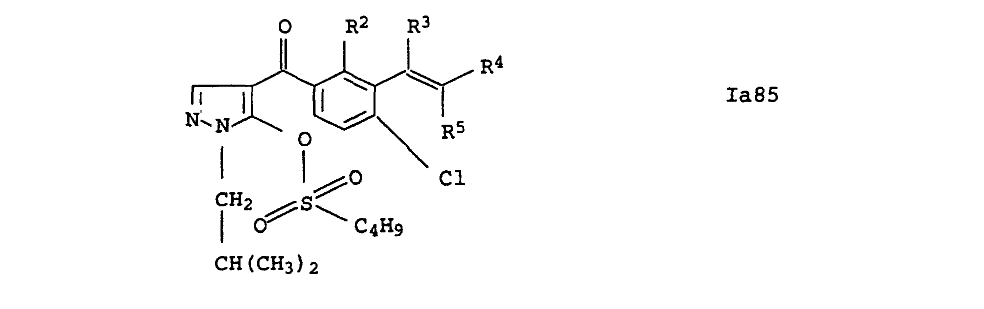
- Ebenso außergewöhnlich bevorzugt sind die Verbindungen Ia86, insbesondere die Verbindungen Ia86.001-Ia86.180, die sich von den entsprechenden Verbindungen Ia1.001-Ia1.180 dadurch unterscheiden, daß R¹⁵ für iso-Butylsulfonyl steht: 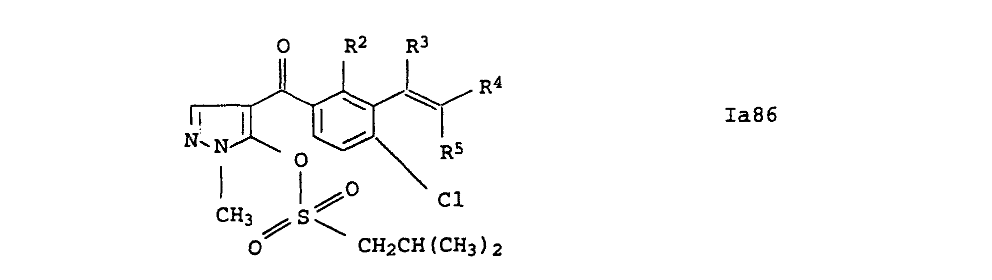
- Ebenso außergewöhnlich bevorzugt sind die Verbindungen Ia87, insbesondere die Verbindungen Ia87.001-Ia87.180, die sich von den entsprechenden Verbindungen Ia1.001-Ia1.180 dadurch unterscheiden, daß R¹⁴ für Ethyl und R¹⁵ für iso-Butylsulfonyl stehen: 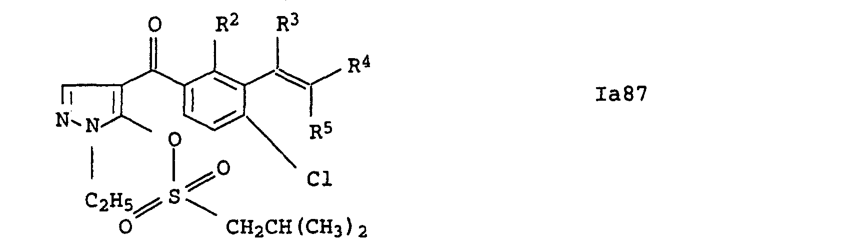
- Ebenso außergewöhnlich bevorzugt sind die Verbindungen Ia88, insbesondere die Verbindungen Ia88.001-Ia88.180, die sich von den entsprechenden Verbindungen Ia1.001-Ia1.180 dadurch unterscheiden, daß R¹⁴ für n-Propyl und R¹⁵ für iso-Butylsulfonyl stehen: 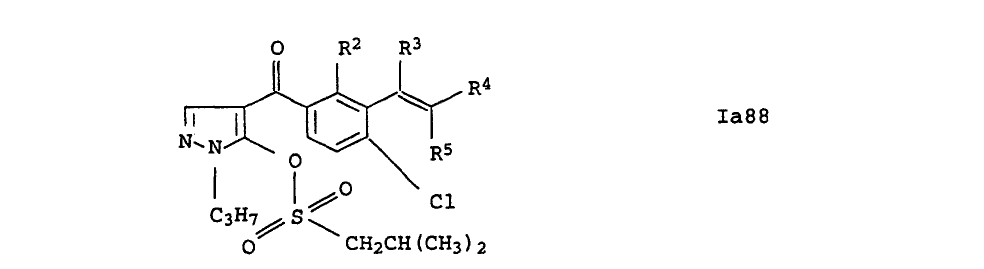
- Ebenso außergewöhnlich bevorzugt sind die Verbindungen Ia89, insbesondere die Verbindungen Ia89.001-Ia89.180, die sich von den entsprechenden Verbindungen Ia1.001-Ia1.180 dadurch unterscheiden, daß R¹⁴ für n-Butyl und R¹⁵ für iso-Butylsulfonyl stehen: 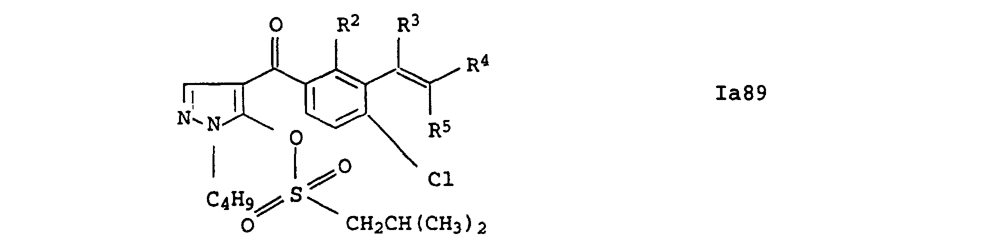
- Ebenso außergewöhnlich bevorzugt sind die Verbindungen Ia90, insbesondere die Verbindungen Ia90.001-Ia90.180, die sich von den entsprechenden Verbindungen Ia1.001-Ia1.180 dadurch unterscheiden, daß R¹⁴ für iso-Butyl und R¹⁵ für iso-Butylsulfonyl stehen: 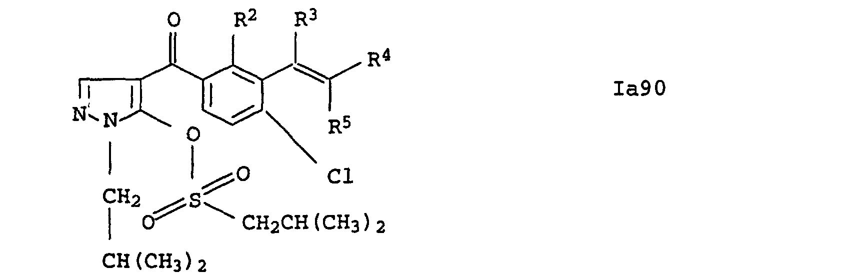
- Ebenso außergewöhnlich bevorzugt sind die Verbindungen Ia91, insbesondere die Verbindungen Ia91.001-Ia91.180, die sich von den entsprechenden Verbindungen Ia1.001-Ia1.180 dadurch unterscheiden, daß R¹⁵ für sec-Butylsulfonyl steht: 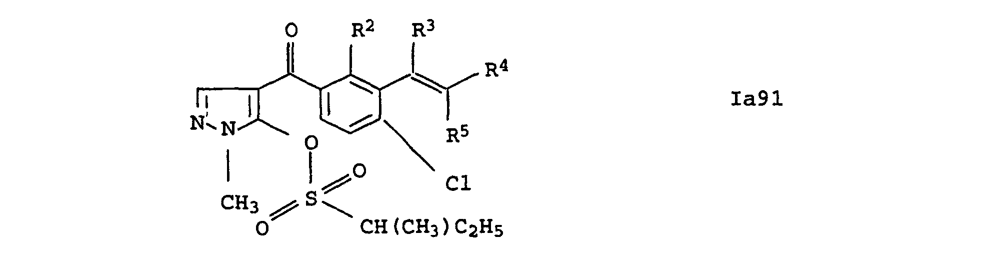
- Ebenso außergewöhnlich bevorzugt sind die Verbindungen Ia92, insbesondere die Verbindungen Ia92.001-Ia92.180, die sich von den entsprechenden Verbindungen Ia1.001-Ia1.180 dadurch unterscheiden, daß R¹⁴ für Ethyl und R¹⁵ für sec-Butylsulfonyl stehen: 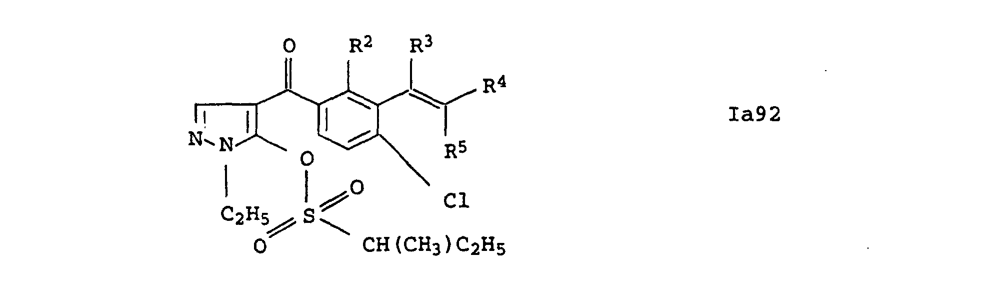
- Ebenso außergewöhnlich bevorzugt sind die Verbindungen Ia93, insbesondere die Verbindungen Ia93.001-Ia93.180, die sich von den entsprechenden Verbindungen Ia1.001-Ia1.180 dadurch unterscheiden, daß R¹⁴ für n-Propyl und R¹⁵ für sec-Butylsulfonyl stehen: 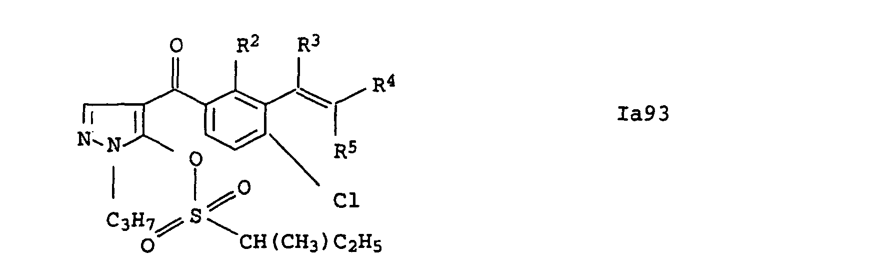
- Ebenso außergewöhnlich bevorzugt sind die Verbindungen Ia94, insbesondere die Verbindungen Ia94.001-Ia94.180, die sich von den entsprechenden Verbindungen Ia1.001-Ia1.180 dadurch unterscheiden, daß R¹⁴ für n-Butyl und R¹⁵ für sec-Butylsulfonyl stehen: 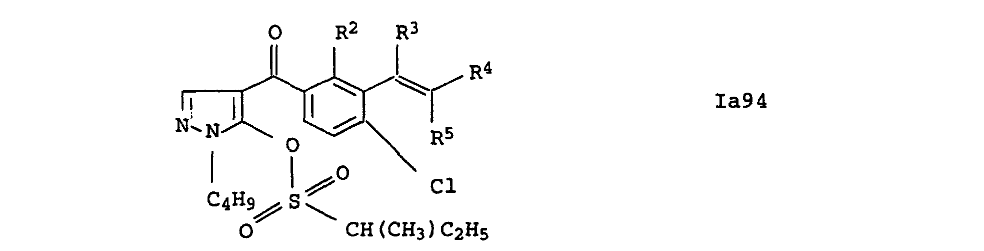
- Ebenso außergewöhnlich bevorzugt sind die Verbindungen Ia95, insbesondere die Verbindungen Ia95.001-Ia95.180, die sich von den entsprechenden Verbindungen Ia1.001-Ia1.180 dadurch unterscheiden, daß R¹⁴ für iso-Butyl und R¹⁵ für sec-Butylsulfonyl stehen: 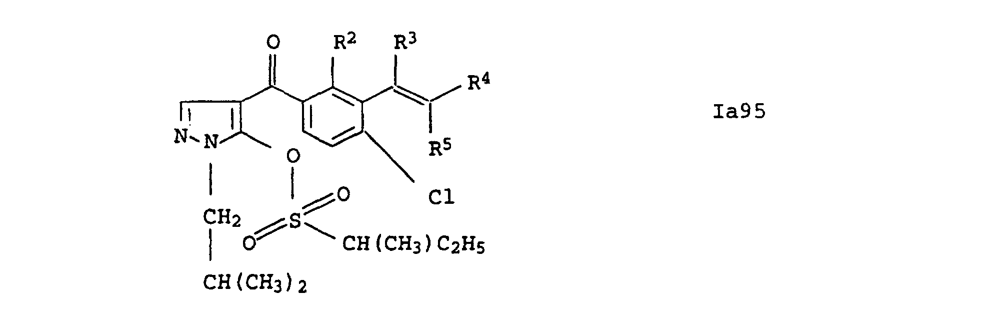
- Ebenso außergewöhnlich bevorzugt sind die Verbindungen Ia96, insbesondere die Verbindungen Ia96.001-Ia96.180, die sich von den entsprechenden Verbindungen Ia1.001-Ia1.180 dadurch unterscheiden, daß R¹⁵ Trifluormethylsulfonyl stehen: 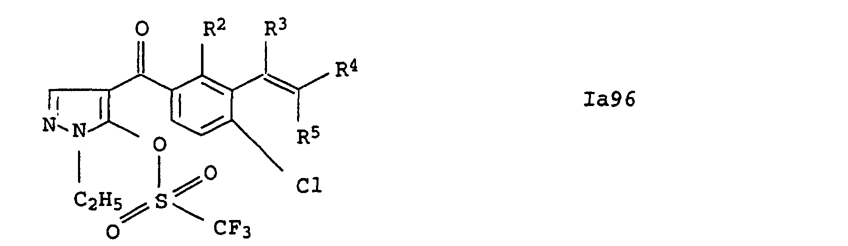
- Ebenso außergewöhnlich bevorzugt sind die Verbindungen Ia97, insbesondere die Verbindungen Ia97.001-Ia97.180, die sich von den entsprechenden Verbindungen Ia1.001-Ia1.180 dadurch unterscheiden, daß R¹⁴ für Ethyl und R¹⁵ für Trifluormethylsulfonyl stehen: 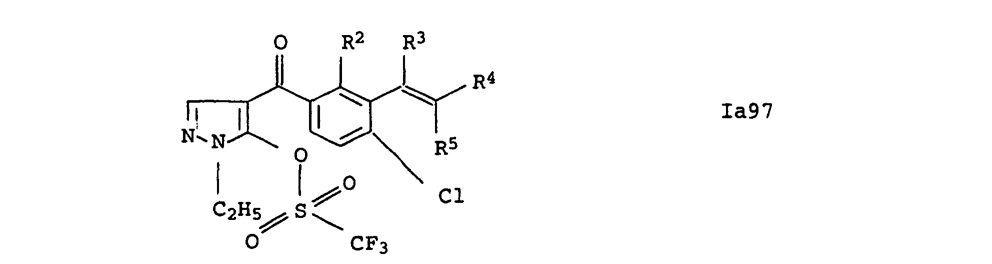
- Ebenso außergewöhnlich bevorzugt sind die Verbindungen Ia98, insbesondere die Verbindungen Ia98.001-Ia98.180, die sich von den entsprechenden Verbindungen Ia1.001-Ia1.180 dadurch unterscheiden, daß R¹⁴ für n-Propyl und R¹⁵ für Trifluormethylsulfonyl stehen: 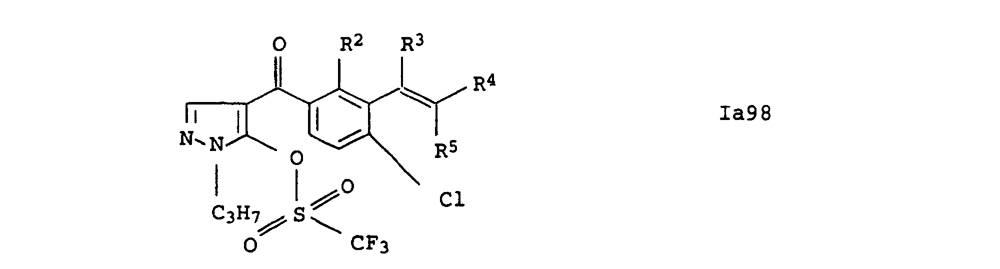
- Ebenso außergewöhnlich bevorzugt sind die Verbindungen Ia99, insbesondere die Verbindungen Ia99.001-Ia99.180, die sich von den entsprechenden Verbindungen Ia1.001-Ia1.180 dadurch unterscheiden, daß R¹⁴ für n-Butyl und R¹⁵ für Trifluormethylsulfonyl stehen: 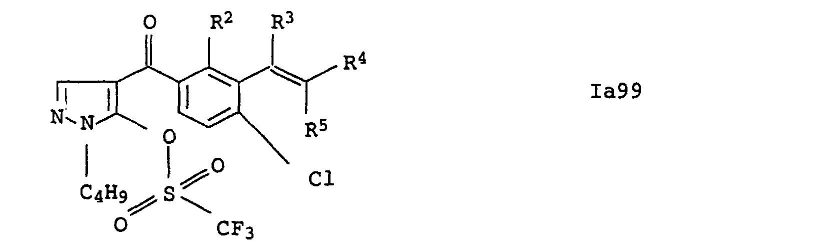
- Ebenso außergewöhnlich bevorzugt sind die Verbindungen Ia100, insbesondere die Verbindungen Ia100.001-Ia100.180, die sich von den entsprechenden Verbindungen Ia1.001-Ia1.180 dadurch unterscheiden, daß R¹⁴ für iso-Butyl und R¹⁵ für Trifluormethylsulfonyl stehen: 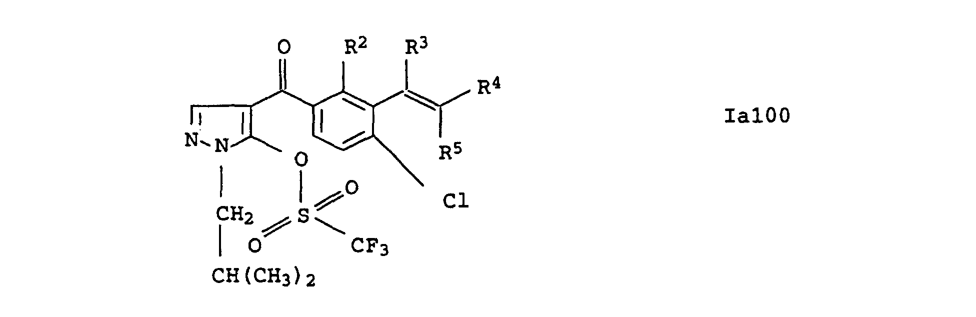
- Ebenso außergewöhnlich bevorzugt sind die Verbindungen Ia101, insbesondere die Verbindungen Ia101.001-Ia101.180, die sich von den entsprechenden Verbindungen Ia1.001-Ia1.180 dadurch unterscheiden, daß R¹⁵ für Phenylcarbonylmethyl steht: 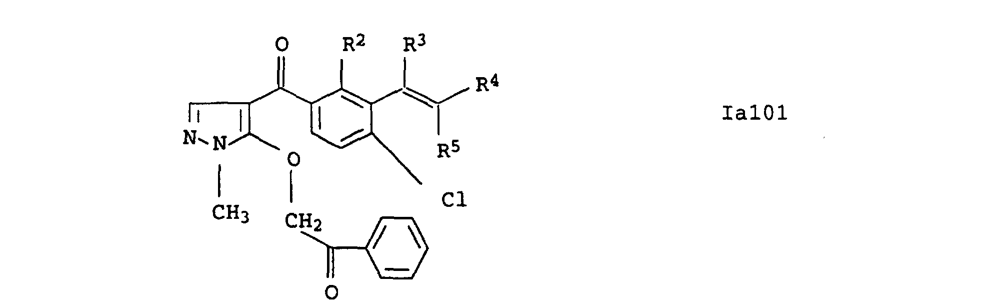
- Ebenso außergewöhnlich bevorzugt sind die Verbindungen Ia102, insbesondere die Verbindungen Ia102.001-Ia102.180, die sich von den entsprechenden Verbindungen Ia1.001-Ia1.180 dadurch unterscheiden, daß R¹⁴ für Ethyl und R¹⁵ für Phenylcarbonylmethyl stehen: 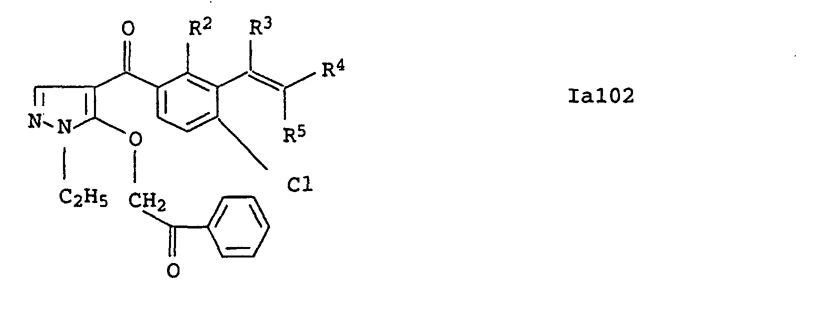
- Ebenso außergewöhnlich bevorzugt sind die Verbindungen Ia103, insbesondere die Verbindungen Ia103.001-Ia103.180, die sich von den entsprechenden Verbindungen Ta1.001-Ia1.180 dadurch unterscheiden, daß R¹⁴ für n-Propyl und R¹⁵ für Phenylcarbonylmethyl stehen: 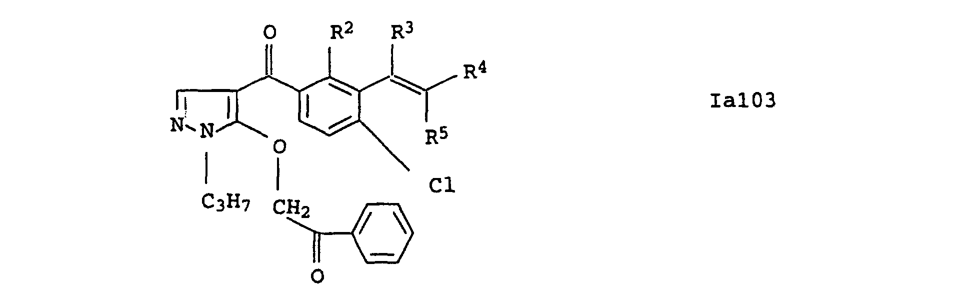
- Ebenso außergewöhnlich bevorzugt sind die Verbindungen Ia104, insbesondere die Verbindungen Ia104.001-Ia104.180, die sich von den entsprechenden Verbindungen Ia1.001-Ia1.180 dadurch unterscheiden, daß R¹⁴ für n-Butyl und R¹⁵ für Phenylcarbonylmethyl stehen: 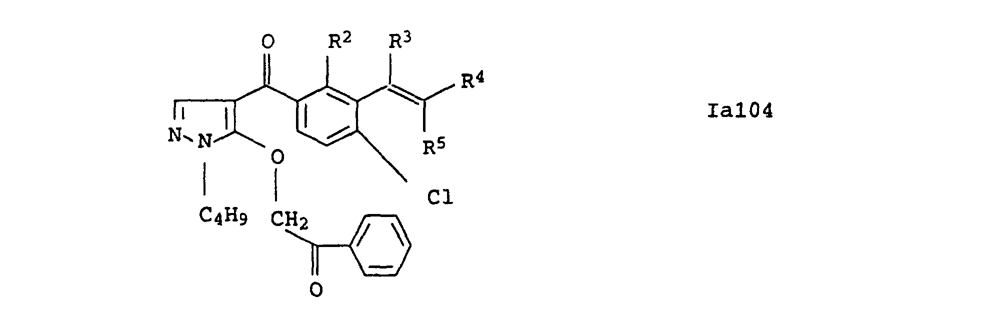
- Ebenso außergewöhnlich bevorzugt sind die Verbindungen Ia105, insbesondere die Verbindungen Ia105.001-Ia105.180, die sich von den entsprechenden Verbindungen Ia1.001-Ia1.180 dadurch unterscheiden, daß R¹⁴ für iso-Butyl und R¹⁵ für Phenylcarbonylmethyl stehen: 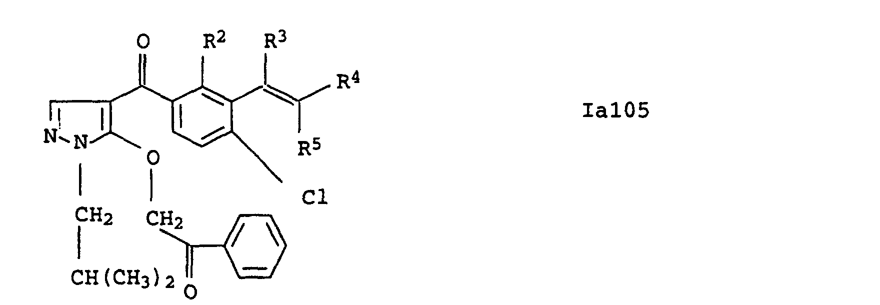
- Ebenso außergewöhnlich bevorzugt sind die Verbindungen Ia106, insbesondere die Verbindungen Ia106.001-Ia106.180, die sich von den entsprechenden Verbindungen Ta1.001-Ia1.180 dadurch unterscheiden, daß R¹⁵ für Phenylsulfonyl steht: 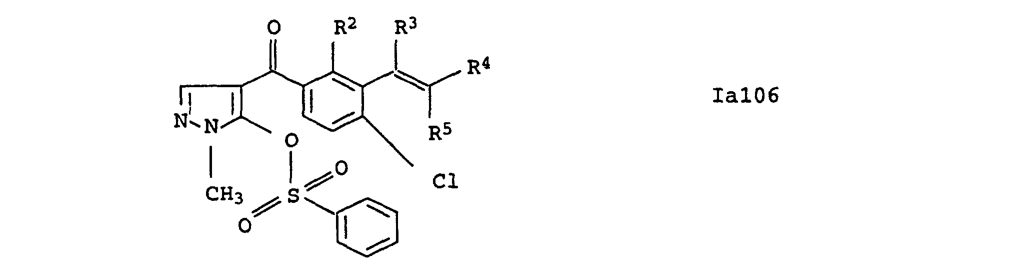
- Ebenso außergewöhnlich bevorzugt sind die Verbindungen Ia107, insbesondere die Verbindungen Ia107.001-Ia107.180, die sich von den entsprechenden Verbindungen Ia1.001-Ia1.180 dadurch unterscheiden, daß R¹⁴ für Ethyl und R¹⁵ für Phenylsulfonyl stehen: 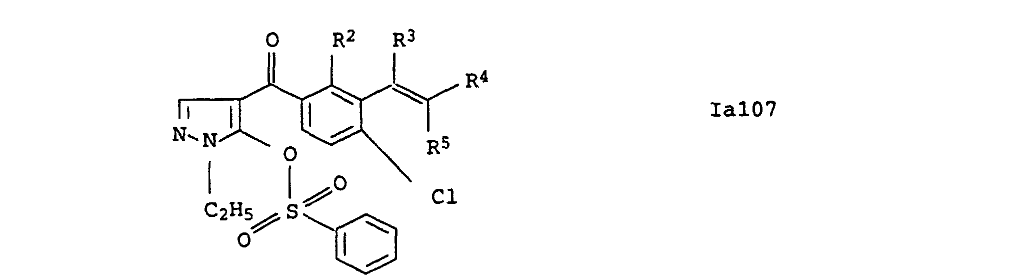
- Ebenso außergewöhnlich bevorzugt sind die Verbindungen Ia108, insbesondere die Verbindungen Ia108.001-Ia108.180, die sich von den entsprechenden Verbindungen Ia1.001-Ia1.180 dadurch unterscheiden, daß R¹⁴ für n-Propyl und R¹⁵ für Phenylsulfonyl stehen: 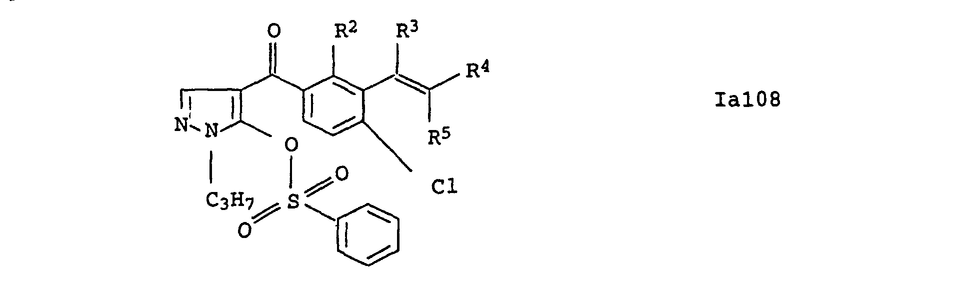
- Ebenso außergewöhnlich bevorzugt sind die Verbindungen Ia109, insbesondere die Verbindungen Ia109.001-Ia109.180, die sich von den entsprechenden Verbindungen Ia1.001-Ia1.180 dadurch unterscheiden, daß R¹⁴ für n-Butyl und R¹⁵ für Phenylsulfonyl stehen: 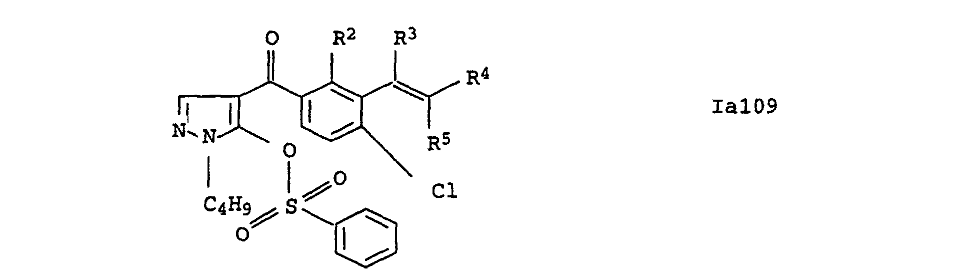
- Ebenso außergewöhnlich bevorzugt sind die Verbindungen Ia110, insbesondere die Verbindungen Ia110.001-Ia110.180, die sich von den entsprechenden Verbindungen Ia1.001-Ia1.180 dadurch unterscheiden, daß R¹⁴ für iso-Butyl und R¹⁵ für Phenylsulfonyl stehen: 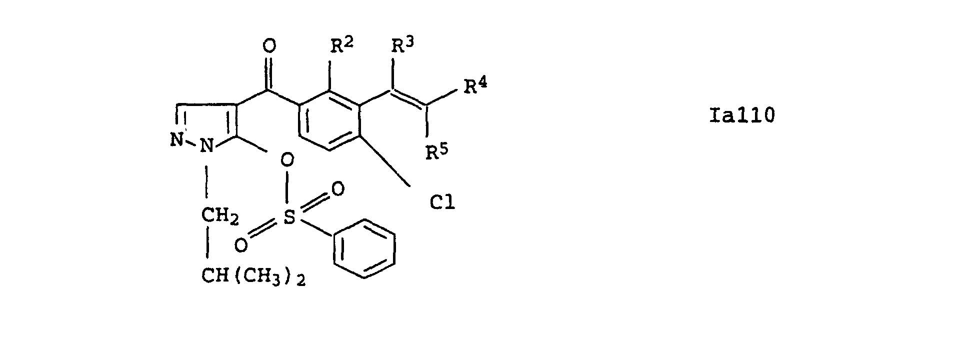
- Ebenso außergewöhnlich bevorzugt sind die Verbindungen Ia111, insbesondere die Verbindungen Ia111.001-Ia111.180, die sich von den entsprechenden Verbindungen Ia1.001-Ia1.180 dadurch unterscheiden, daß R¹⁵ für 4-Methylphenylsulfonyl steht: 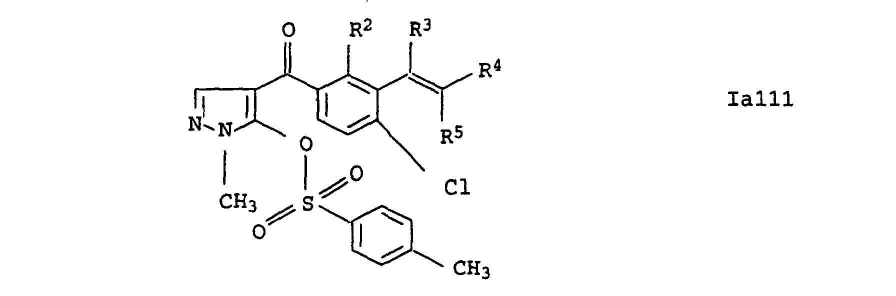
- Ebenso außergewöhnlich bevorzugt sind die Verbindungen Ia112, insbesondere die Verbindungen Ia112.001-Ia112.180, die sich von den entsprechenden Verbindungen Ia1.001-Ia1.180 dadurch unterscheiden, daß R¹⁴ für Ethyl und R¹⁵ für 4-Methylphenylsulfonyl stehen: 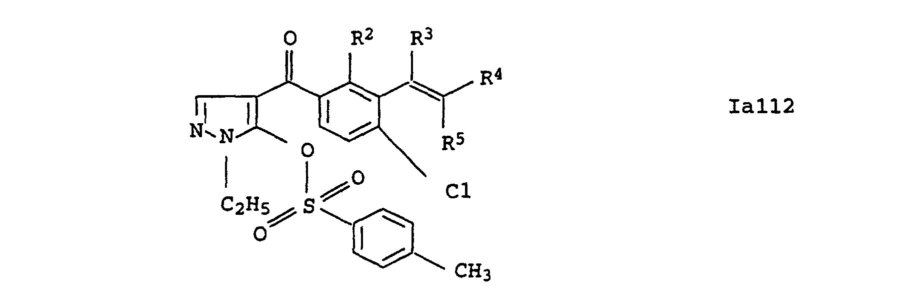
- Ebenso außergewöhnlich bevorzugt sind die Verbindungen Ia113, insbesondere die Verbindungen Ia113.001-Ia113.180, die sich von den entsprechenden Verbindungen Ia1.001-Ia1.180 dadurch unterscheiden, daß R¹⁴ für n-Propyl und R¹⁵ für 4-Methylphenylsulfonyl stehen: 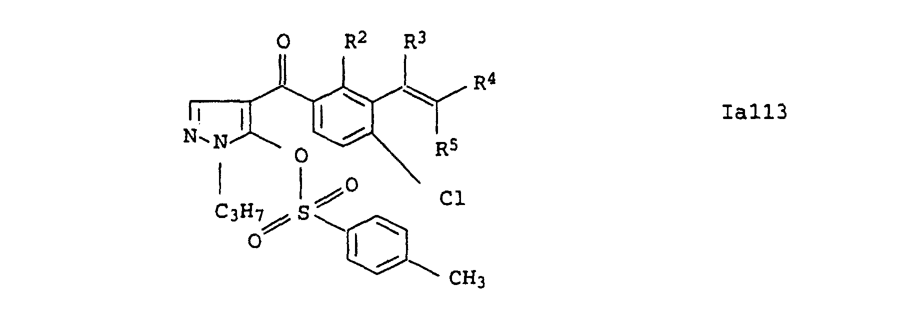
- Ebenso außergewöhnlich bevorzugt sind die Verbindungen Ia114, insbesondere die Verbindungen Ia114.001-Ia114.180, die sich von den entsprechenden Verbindungen Ia1.001-Ia1.180 dadurch unterscheiden, daß R¹⁴ für n-Butyl und R¹⁵ für 4-Methylphenylsulfonyl stehen: 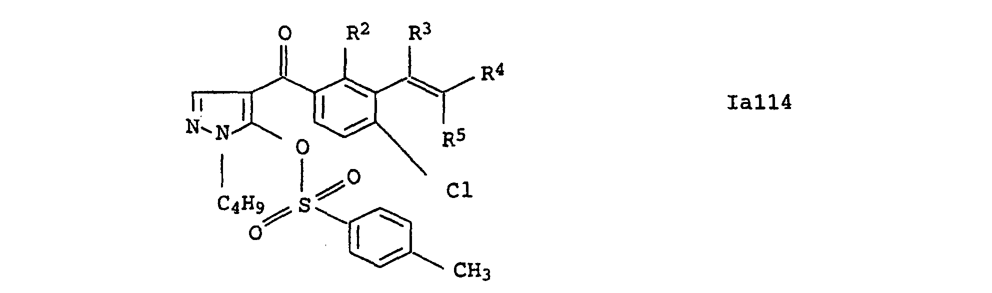
- Ebenso außergewöhnlich bevorzugt sind die Verbindungen Ia115, insbesondere die Verbindungen Ia115.001-Ia115.180, die sich von den entsprechenden Verbindungen Ia1.001-Ia1.180 dadurch unterscheiden, daß R¹⁴ für iso-Butyl und R¹⁵ für 4-Methylphenylsulfonyl stehen: 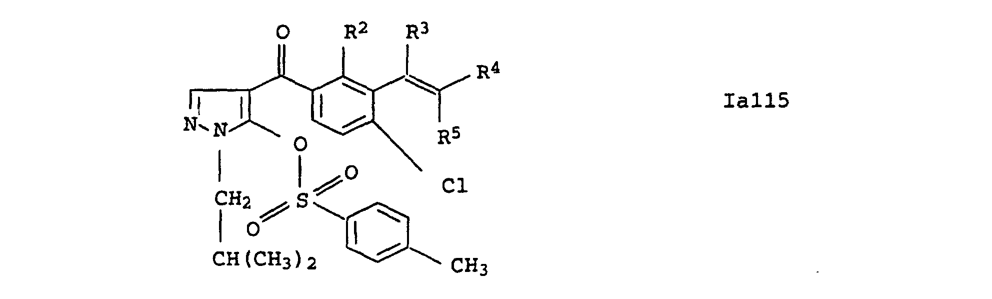
- Ebenso außergewöhnlich bevorzugt sind die Verbindungen Ia116, insbesondere die Verbindungen Ia116.001-Ia116.180, die sich von den entsprechenden Verbindungen Ia1.001-Ia1.180 dadurch unterscheiden, daß R¹ für Nitro steht: 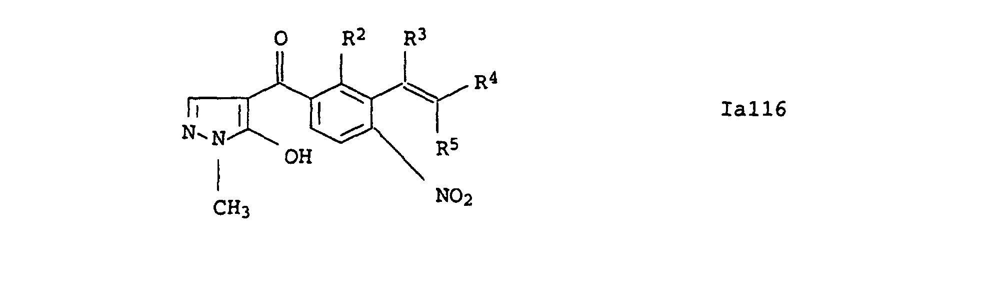
- Ebenso außergewöhnlich bevorzugt sind die Verbindungen Ia117, insbesondere die Verbindungen Ia117.001-Ia117.180, die sich von den entsprechenden Verbindungen Ia1.001-Ia1.180 dadurch unterscheiden, daß R¹ für Nitro und R¹⁴ für Ethyl stehen: 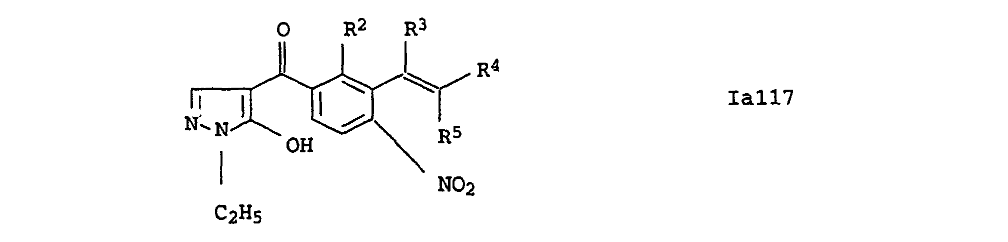
- Ebenso außergewöhnlich bevorzugt sind die Verbindungen Ia118, insbesondere die Verbindungen Ia118.001-Ia118.180, die sich von den entsprechenden Verbindungen Ia1.001-Ia1.180 dadurch unterscheiden, daß R¹ für Nitro und R¹⁴ für Propyl stehen: 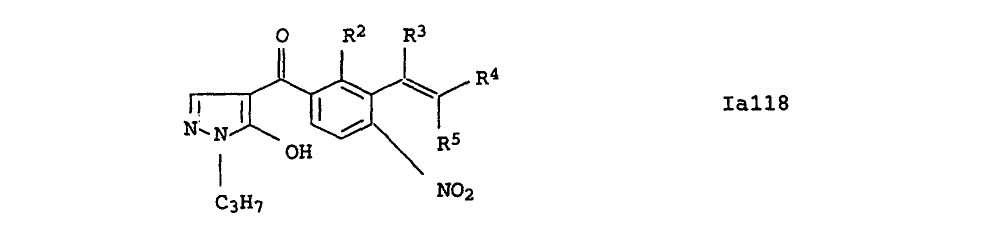
- Ebenso außergewöhnlich bevorzugt sind die Verbindungen Ia119, insbesondere die Verbindungen Ia119.001-Ia119.180, die sich von den entsprechenden Verbindungen Ia1.001-Ia1.180 dadurch unterscheiden, daß R¹ für Nitro und R¹⁴ für n-Butyl stehen: 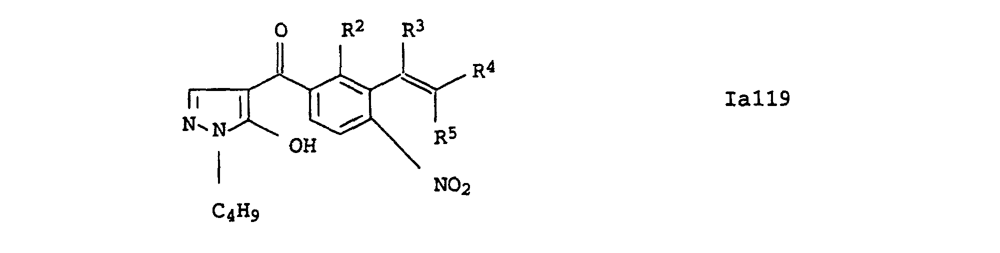
- Ebenso außergewöhnlich bevorzugt sind die Verbindungen Ia120, insbesondere die Verbindungen Ia120.001-Ia120.180, die sich von den entsprechenden Verbindungen Ia1.001-Ia1.180 dadurch unterscheiden, daß R¹ für Nitro und R¹⁴ für iso-Butyl stehen: 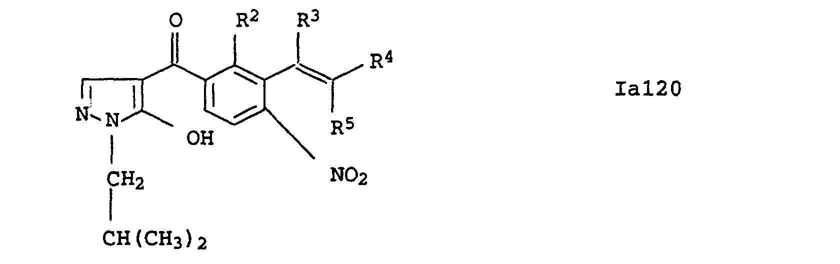
- Ebenso außergewöhnlich bevorzugt sind die Verbindungen Ia121, insbesondere die Verbindungen Ia121.001-Ia121.180, die sich von den entsprechenden Verbindungen Ia1.001-Ia1.180 dadurch unterscheiden, daß R¹ für Nitro und R¹⁵ für Methyl stehen: 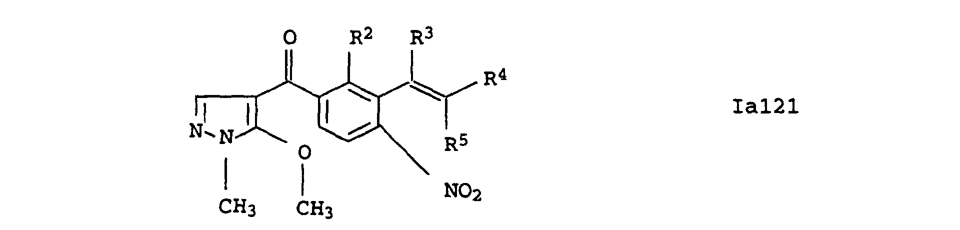
- Ebenso außergewöhnlich bevorzugt sind die Verbindungen Ia122, insbesondere die Verbindungen Ia122.001-Ia122.180, die sich von den entsprechenden Verbindungen Ia1.001-Ia1.180 dadurch unterscheiden, daß R¹ für Nitro, R¹⁴ für Ethyl und R15 für Methyl stehen: 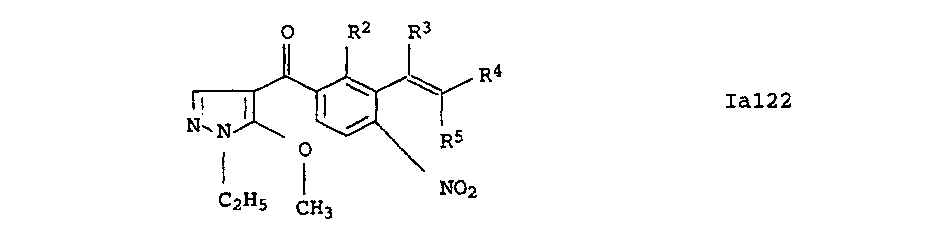
- Ebenso außergewöhnlich bevorzugt sind die Verbindungen Ia123, insbesondere die Verbindungen Ia123.001-Ia123.180, die sich von den entsprechenden Verbindungen Ia1.001-Ia1.180 dadurch unterscheiden, daß R¹ für Nitro, R¹⁴ für n-Propyl und R¹⁵ für Methyl stehen: 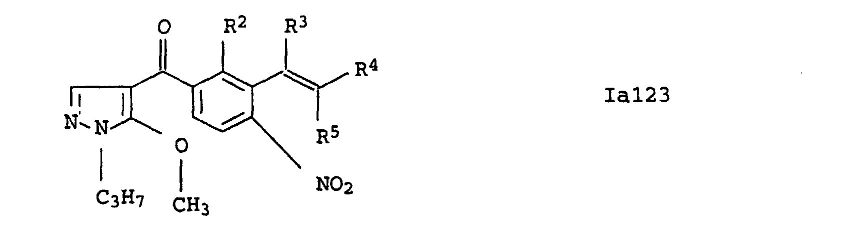
- Ebenso außergewöhnlich bevorzugt sind die Verbindungen Ia124, insbesondere die Verbindungen Ia124.001-Ia124.180, die sich von den entsprechenden Verbindungen Ia1.041-Ia1.180 dadurch unterscheiden, daß R¹ für Nitro, R¹⁴ für n-Butyl und R¹⁵ für Methyl stehen: 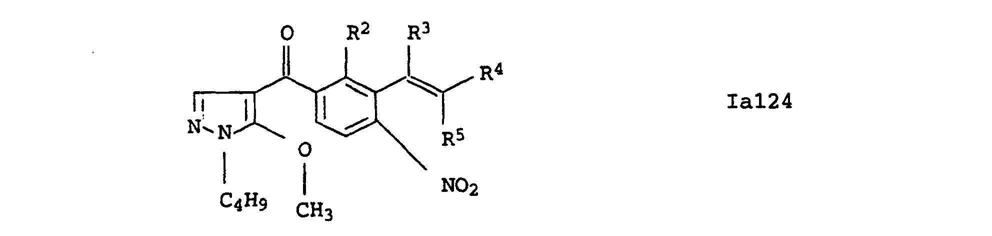
- Ebenso außergewöhnlich bevorzugt sind die Verbindungen Ia125, insbesondere die Verbindungen Ia125.001-Ia125.180, die sich von den entsprechenden Verbindungen Ia1.001-Ia1.180 dadurch unterscheiden, daß R¹ für Nitro, R¹⁴ für iso-Butyl und R¹⁵ für Methyl stehen: 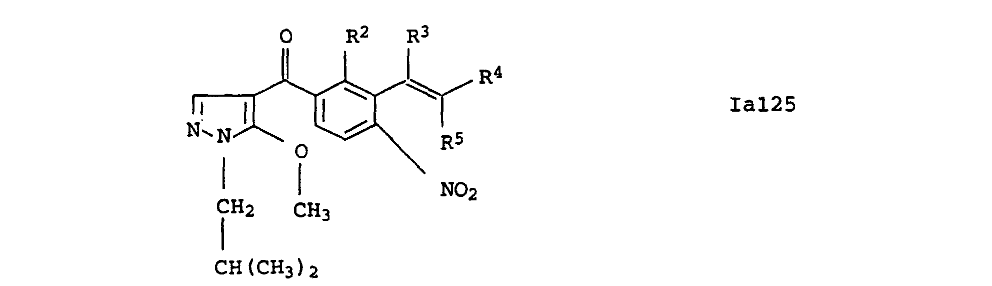
- Ebenso außergewöhnlich bevorzugt sind die Verbindungen Ia126, insbesondere die Verbindungen Ia126.001-Ia126.180, die sich von den entsprechenden Verbindungen Ia1.001-Ia1.180 dadurch unterscheiden, daß R¹ für Nitro und R¹⁵ für Ethyl stehen: 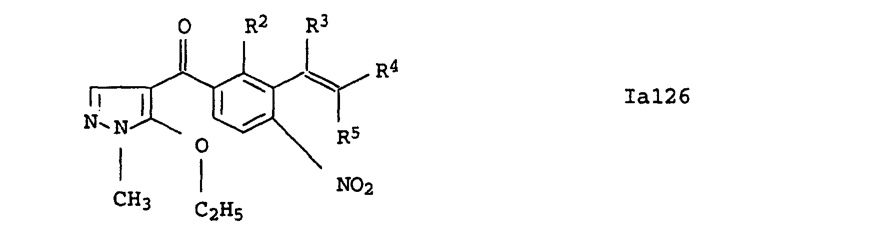
- Ebenso außergewöhnlich bevorzugt sind die Verbindungen Ia127, insbesondere die Verbindungen Ia127.00I-Ia127.180, die sich von den entsprechenden Verbindungen Ia1.001-Ia1.180 dadurch unterscheiden, daß R¹ für Nitro und R¹⁴ und R¹⁵ für Ethyl stehen: 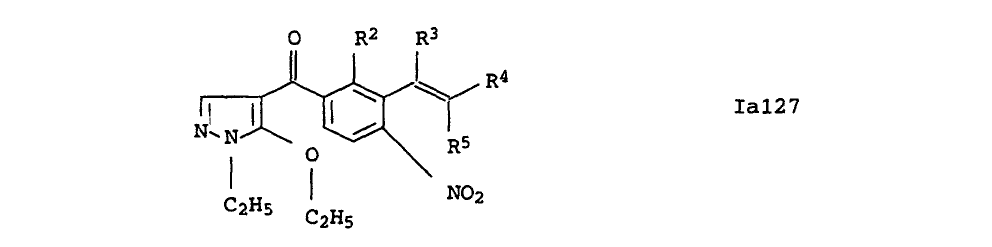
- Ebenso außergewöhnlich bevorzugt sind die Verbindungen Ia128, insbesondere die Verbindungen Ia128.001-Ia128.180, die sich von den entsprechenden Verbindungen Ia1.001-Ia1.180 dadurch unterscheiden, daß R¹ für Nitro, R¹⁴ für n-Propyl und R¹⁵ für Ethyl stehen: 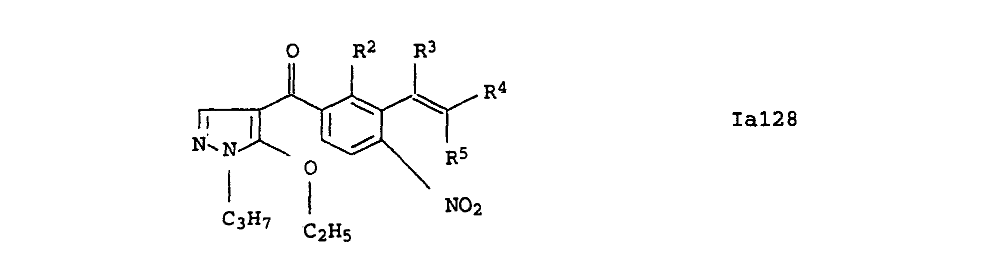
- Ebenso außergewöhnlich bevorzugt sind die Verbindungen Ia129, insbesondere die Verbindungen Ia129.001-Ia129.180, die sich von den entsprechenden Verbindungen Ia1.001-Ia1.180 dadurch unterscheiden, daß R¹ für Nitro, R¹⁴ für n-Butyl und R¹⁵ für Ethyl stehen: 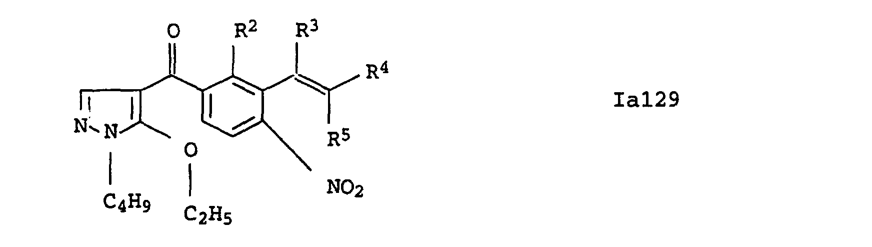
- Ebenso außergewöhnlich bevorzugt sind die Verbindungen Ia130, insbesondere die Verbindungen Ia130.001-Ia130.180, die sich von den entsprechenden Verbindungen Ia1.001-Ia1.180 dadurch unterscheiden, daß R¹ Nitro, R¹⁴ für iso-Butyl und R¹⁵ für Ethyl stehen: 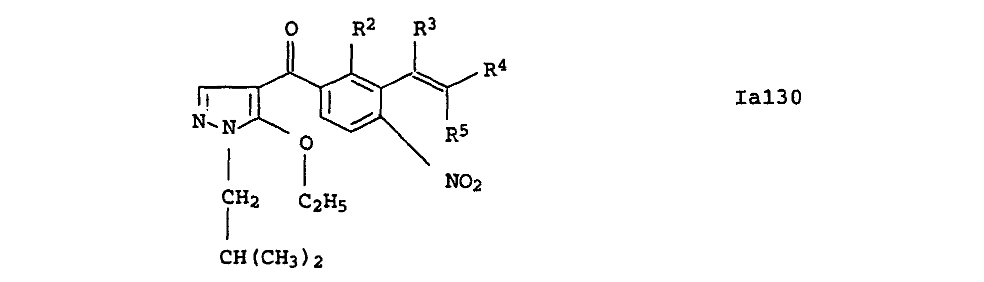
- Ebenso außergewöhnlich bevorzugt sind die Verbindungen Ia131, insbesondere die Verbindungen Ia131.001-Ia131.180, die sich von den entsprechenden Verbindungen Ia1.001-Ia1.180 dadurch unterscheiden, daß R¹ für Nitro und R¹⁵ für n-Propyl stehen: 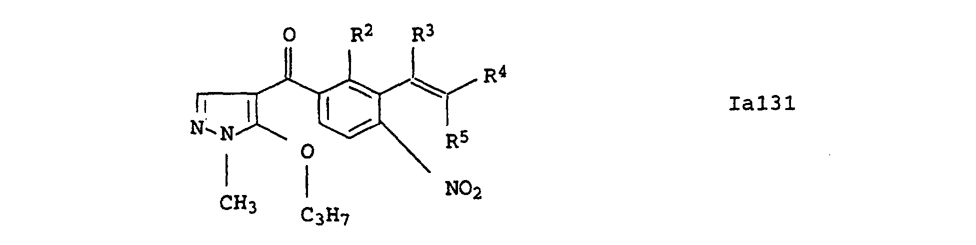
- Ebenso außergewöhnlich bevorzugt sind die Verbindungen Ia132, insbesondere die Verbindungen Ia132.001-Ia132.180, die sich von den entsprechenden Verbindungen Ia1.001-Ia1.180 dadurch unterscheiden, daß R¹ für Nitro, R¹⁴ für Ethyl und R¹⁵ für n-Propyl stehen: 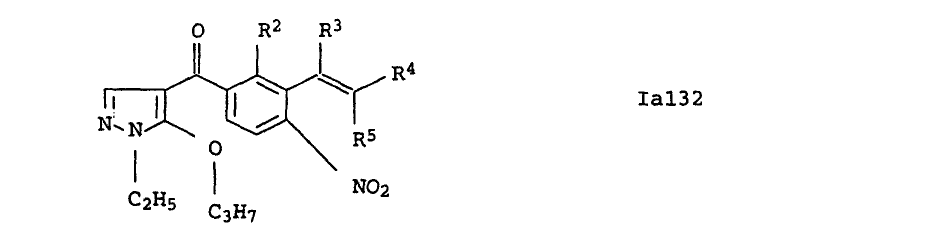
- Ebenso außergewöhnlich bevorzugt sind die Verbindungen Ia133, insbesondere die Verbindungen Ia133.001-Ia133.180, die sich von den entsprechenden Verbindungen Ia1.001-Ia1.180 dadurch unterscheiden, daß R¹ für Nitro, R¹⁴ und R¹⁵ für n-Propyl stehen: 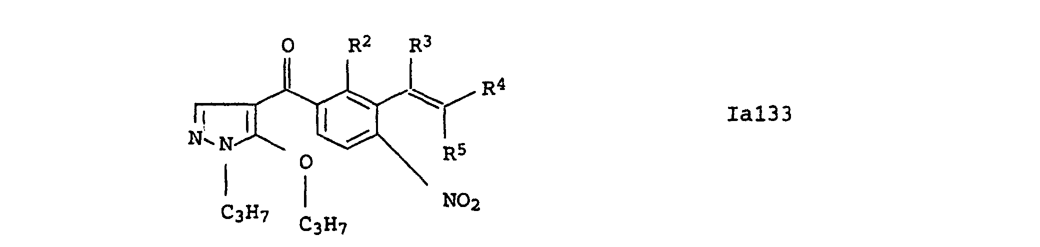
- Ebenso außergewöhnlich bevorzugt sind die Verbindungen Ia134, insbesondere die Verbindungen Ia134.001-Ia134.180, die sich von den entsprechenden Verbindungen Ia1.001-Ia1.180 dadurch unterscheiden, daß R¹ Nitro, R¹⁴ für n-Butyl und R¹⁵ für n-Propyl stehen: 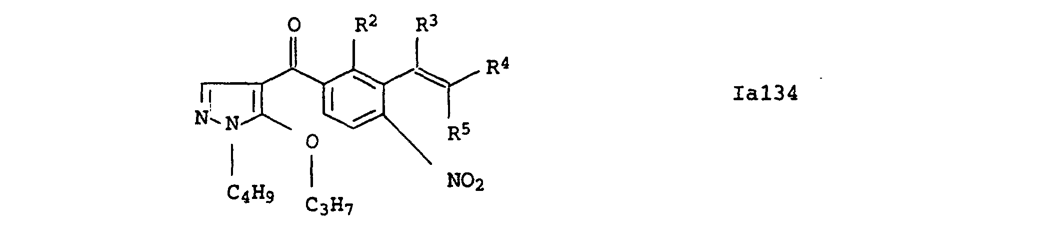
- Ebenso außergewöhnlich bevorzugt sind die Verbindungen Ia135, insbesondere die Verbindungen Ia135.001-Ia135.180, die sich von den entsprechenden Verbindungen Ia1.001-Ia1.180 dadurch unterscheiden, daß R¹ Nitro, R¹⁴ für iso-Butyl und R¹⁵ für n-Propyl stehen: 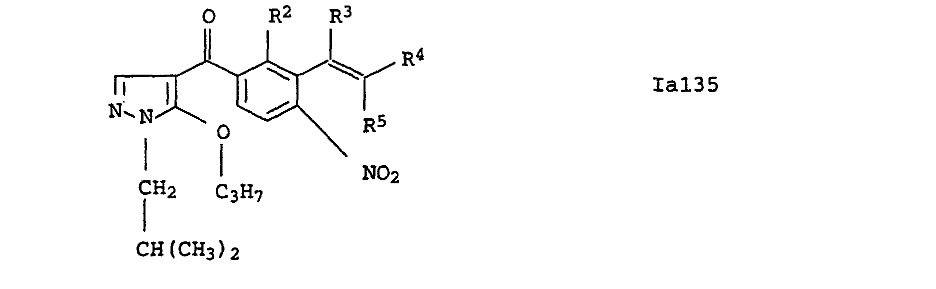
- Ebenso außergewöhnlich bevorzugt sind die Verbindungen Ia136, insbesondere die Verbindungen Ia136.001-Ia136.180, die sich von den entsprechenden Verbindungen Ia1.001-Ia1.180 dadurch unterscheiden, daß R¹ für Nitro und R¹⁵ für iso-Propyl stehen: 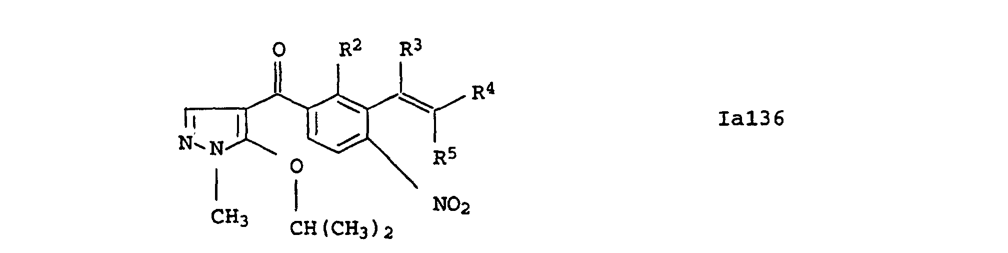
- Ebenso außergewöhnlich bevorzugt sind die Verbindungen Ia137, insbesondere die Verbindungen Ia137.001-Ia137.180, die sich von den entsprechenden Verbindungen Ia1.001-Ia1.180 dadurch unterscheiden, daß R¹ für Nitro, R¹⁴ für Ethyl und R¹⁵ für iso-Propyl stehen: 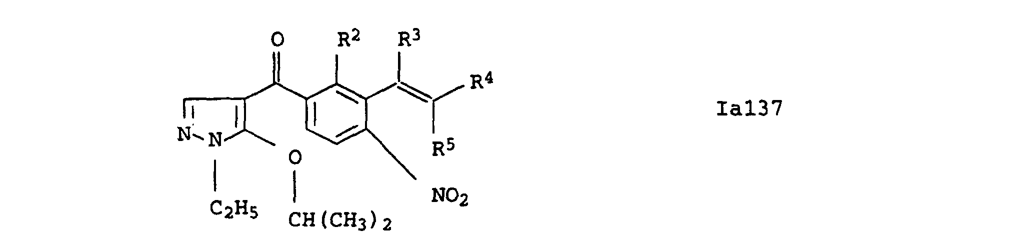
- Ebenso außergewöhnlich bevorzugt sind die Verbindungen Ia138, insbesondere die Verbindungen Ia138.001-Ia138.180, die sich von den entsprechenden Verbindungen Ia1.001-Ia1.180 dadurch unterscheiden, daß R¹ für Nitro, R¹⁴ für n-Propyl und R¹⁵ für iso-Propyl stehen: 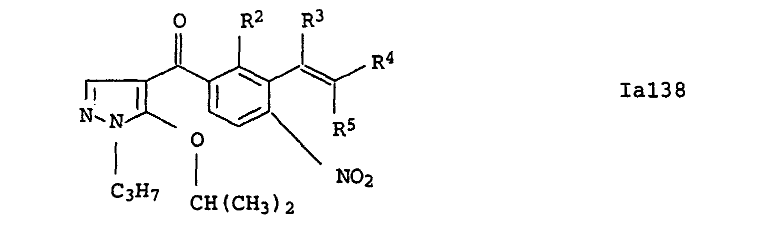
- Ebenso außergewöhnlich bevorzugt sind die Verbindungen Ia139, insbesondere die Verbindungen Ia139.001-Ia139.180, die sich von den entsprechenden Verbindungen Ia1.001-Ia1.180 dadurch unterscheiden, daß R¹ Nitro, R¹⁴ für n-Butyl und R¹⁵ für iso-Propyl stehen: 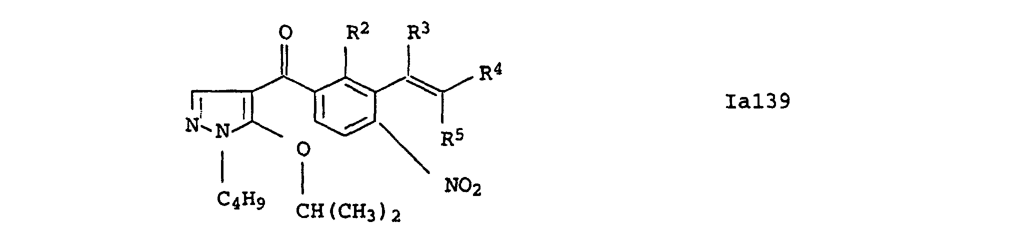
- Ebenso außergewöhnlich bevorzugt sind die Verbindungen Ia140, insbesondere die Verbindungen Ia140.001-Ia140.180, die sich von den entsprechenden Verbindungen Ia1.001-Ia1.180 dadurch unterscheiden, daß R¹ für Nitro, R¹⁴ für iso-Butyl und R¹⁵ für iso-Propyl stehen: 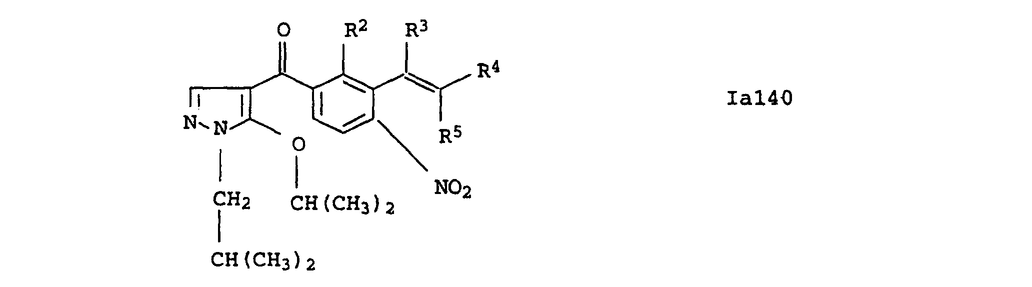
- Ebenso außergewöhnlich bevorzugt sind die Verbindungen Ia141, insbesondere die Verbindungen Ia141.001-Ia141.180, die sich von den entsprechenden Verbindungen Ia1.001-Ia1.180 dadurch unterscheiden, daß R¹ für Nitro und R¹⁵ für n-Butyl stehen: 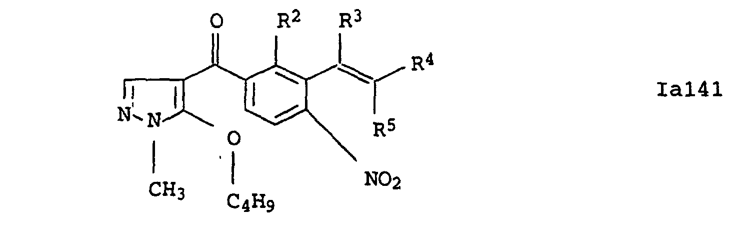
- Ebenso außergewöhnlich bevorzugt sind die Verbindungen Ia142, insbesondere die Verbindungen Ia142.001-Ia142.180, die sich von den entsprechenden Verbindungen Ia1.001-Ia1.180 dadurch unterscheiden, daß R¹ für Nitro, R¹⁴ für Ethyl und R¹⁵ für n-Butyl stehen: 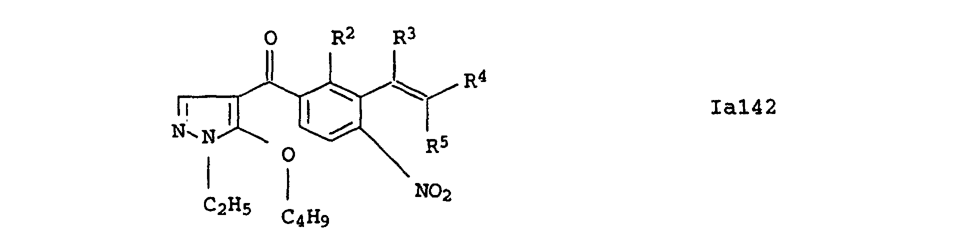
- Ebenso außergewöhnlich bevorzugt sind die Verbindungen Ia143, insbesondere die Verbindungen Ia143.001-Ia143,180, die sich von den entsprechenden Verbindungen Ia1.001-Ia1.180 dadurch unterscheiden, daß R¹ für Nitro, R¹⁴ für n-Propyl und R¹⁵ für n-Butyl stehen: 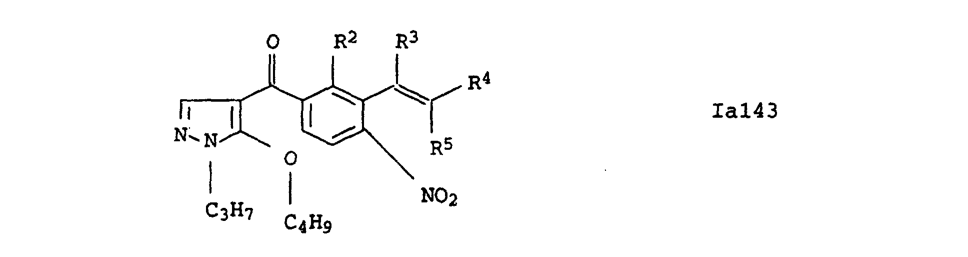
- Ebenso außergewöhnlich bevorzugt sind die Verbindungen Ia144, insbesondere die Verbindungen Ia144.001-Ia144.180, die sich von den entsprechenden Verbindungen Ia1.001-Ia1.180 dadurch unterscheiden, daß R¹ für Nitro, R¹⁴ und R¹⁵ für n-Butyl stehen: 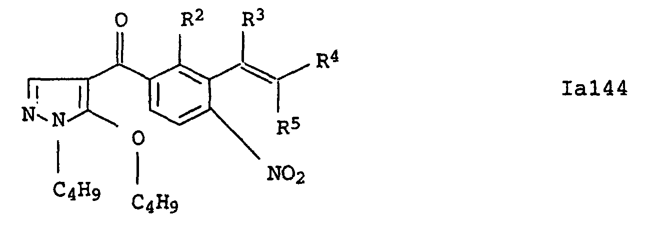
- Ebenso außergewöhnlich bevorzugt sind die Verbindungen Ia145, insbesondere die Verbindungen Ia145.001-Ia145.180, die sich von den entsprechenden Verbindungen Ia1.001-Ia1.180 dadurch unterscheiden, daß R¹ für Nitro, R¹⁴ für iso-Butyl und R¹⁵ für n-Butyl stehen: 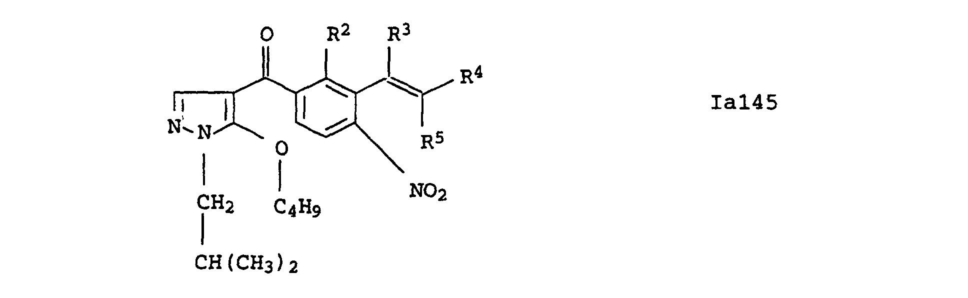
- Ebenso außergewöhnlich bevorzugt sind die Verbindungen Ia146, insbesondere die Verbindungen Ia146.001-Ia146.180, die sich von den entsprechenden Verbindungen Ia1.001-Ia1.180 dadurch unterscheiden, daß R¹ für Nitro und R¹⁵ für sec-Butyl stehen: 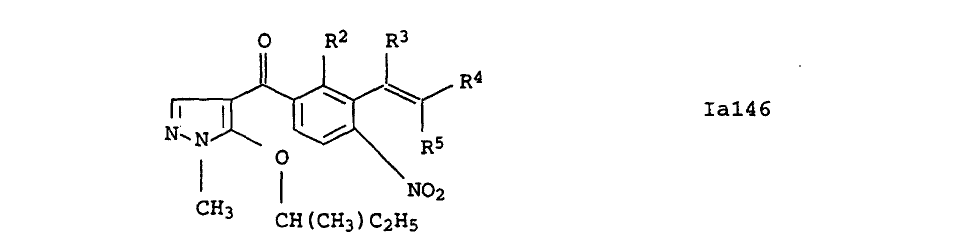
- Ebenso außergewöhnlich bevorzugt sind die Verbindungen Ia147, insbesondere die Verbindungen Ia147.001-Ia147.180, die sich von den entsprechenden Verbindungen Ia1.001-Ia1.180 dadurch unterscheiden, daß R¹ für Nitro, R¹⁴ für Ethyl und R¹⁵ für sec-Butyl stehen: 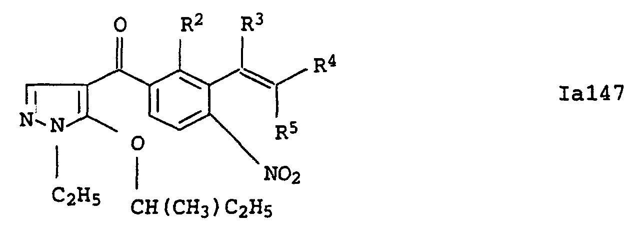
- Ebenso außergewöhnlich bevorzugt sind die Verbindungen Ia148, insbesondere die Verbindungen Ia148.001-Ia148.180, die sich von den entsprechenden Verbindungen Ia1.001-Ia1.180 dadurch unterscheiden, daß R¹ für Nitro, R¹⁴ für n-Propyl und R¹⁵ für sec-Butyl stehen: 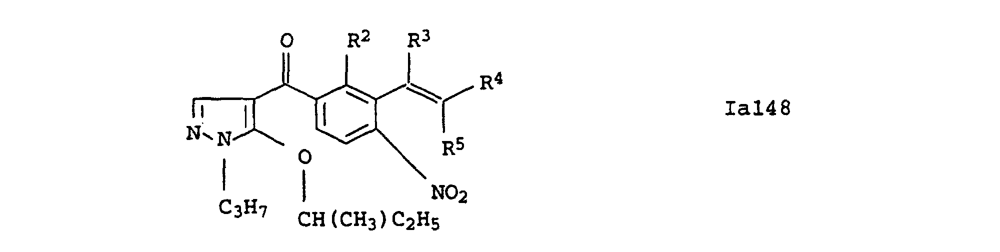
- Ebenso außergewöhnlich bevorzugt sind die Verbindungen Ia149, insbesondere die Verbindungen Ia149.001-Ia149.180, die sich von den entsprechenden Verbindungen Ia1.001-Ia1.180 dadurch unterscheiden, daß R¹ für Nitro, R¹⁴ für n-Butyl und R¹⁵ für sec-Butyl stehen: 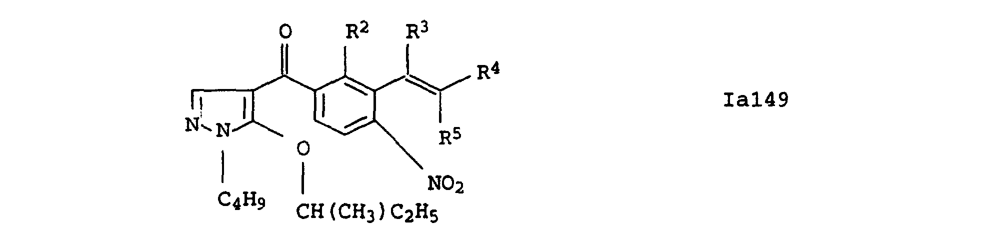
- Ebenso außergewöhnlich bevorzugt sind die Verbindungen Ia150, insbesondere die Verbindungen Ia150.001-Ia150.180, die sich von den entsprechenden Verbindungen Ia1.001-Ia1.180 dadurch unterscheiden, daß R¹ für Nitro, R¹⁴ für iso-Butyl und R¹⁵ für sec-Butyl stehen: 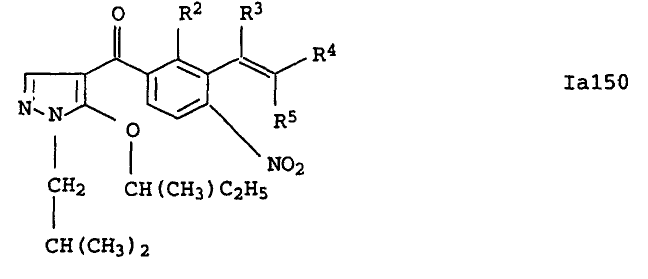
- Ebenso außergewöhnlich bevorzugt sind die Verbindungen Ia151, insbesondere die Verbindungen Ia151.001-Ia151.180, die sich von den entsprechenden Verbindungen Ia1.001-Ia1.180 dadurch unterscheiden, daß R¹ für Nitro und R¹⁵ für iso-Butyl steht: 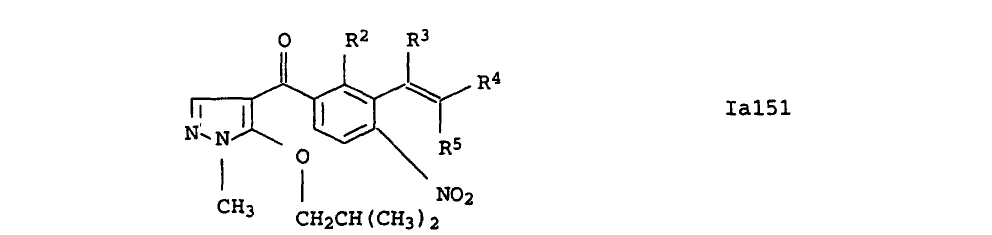
- Ebenso außergewöhnlich bevorzugt sind die Verbindungen Ia152, insbesondere die Verbindungen Ia152.001-Ia152.180, die sich von den entsprechenden Verbindungen Ia1.001-Ia1.180 dadurch unterscheiden, daß R¹ für Nitro, R¹⁴ für Ethyl und R¹⁵ für iso-Butyl stehen: 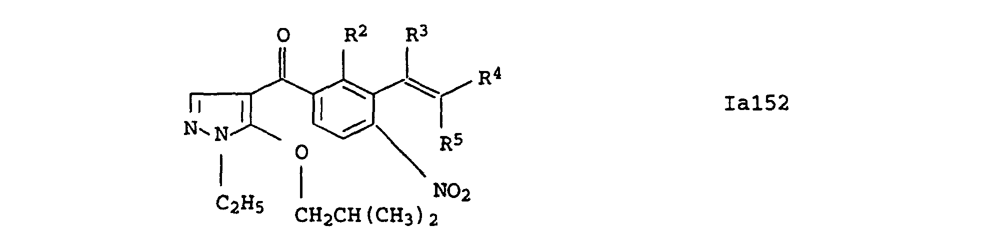
- Ebenso außergewöhnlich bevorzugt sind die Verbindungen Ia153, insbesondere die Verbindungen Ia153.001-Ia153.180, die sich von den entsprechenden Verbindungen Ia1.001-Ia1.180 dadurch unterscheiden, daß R¹ für Nitro, R¹⁴ für n-Propyl und R¹⁵ für iso-Butyl stehen: 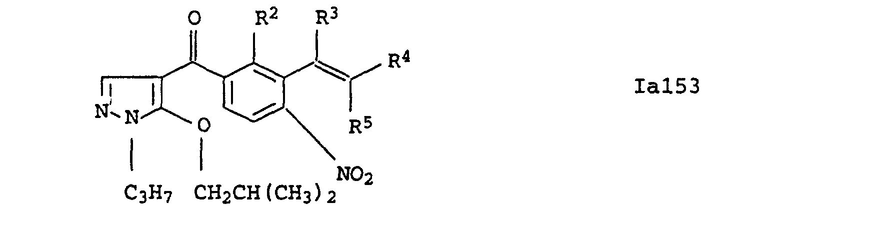
- Ebenso außergewöhnlich bevorzugt sind die Verbindungen Ia154, insbesondere die Verbindungen Ia154.001-Ia154.180, die sich von den entsprechenden Verbindungen Ia1.001-Ia1.180 dadurch unterscheiden, daß R¹ für Nitro, R¹⁴ für n-Butyl und R¹⁵ für iso-Butyl stehen: 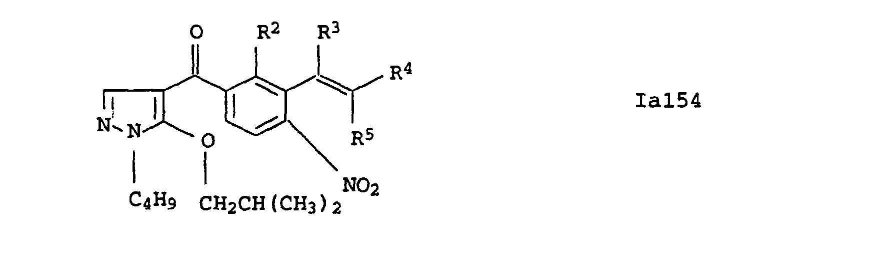
- Ebenso außergewöhnlich bevorzugt sind die Verbindungen Ia155, insbesondere die Verbindungen Ia155.001-Ia154.180, die sich von den entsprechenden Verbindungen Ia1.001-Ia1.180 dadurch unterscheiden, daß R¹ für Nitro, R¹⁴ und R¹⁵ für iso-Butyl stehen: 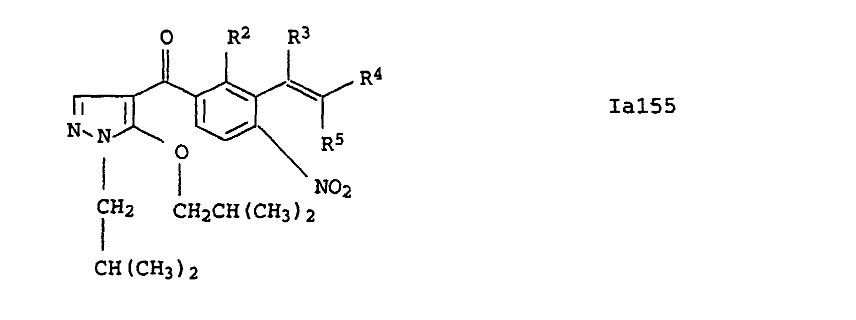
- Ebenso außergewöhnlich bevorzugt sind die Verbindungen Ia156, insbesondere die Verbindungen Ia156.001-Ia156.180, die sich von den entsprechenden Verbindungen Ia1.001-Ia1.180 dadurch unterscheiden, daß R¹ für Nitro und R¹⁵ für Methylcarbonyl stehen: 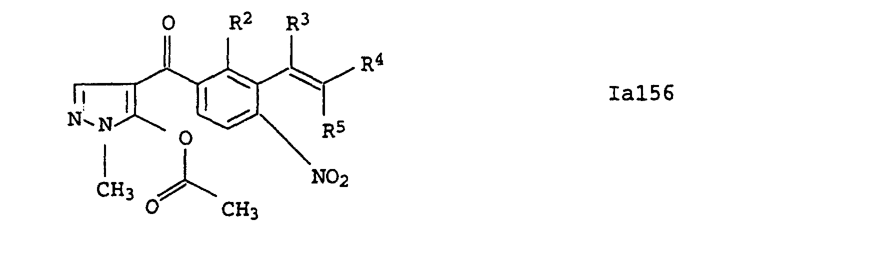
- Ebenso außergewöhnlich bevorzugt sind die Verbindungen Ia157, insbesondere die Verbindungen Ia157.001-Ia157.180, die sich von den entsprechenden Verbindungen Ia1.001-Ia1.180 dadurch unterscheiden, daß R¹ für Nitro, R¹⁴ für Ethyl und R¹⁵ für Methylcarbonyl stehen: 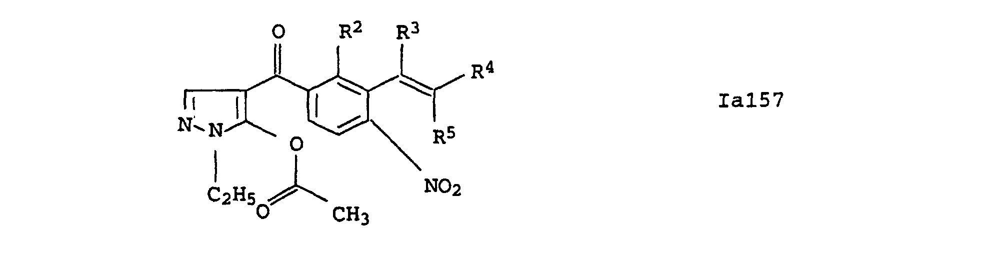
- Ebenso außergewöhnlich bevorzugt sind die Verbindungen Ia158, insbesondere die Verbindungen Ia158.001-Ia158.180, die sich von den entsprechenden Verbindungen Ia1.001-Ia1.180 dadurch unterscheiden, daß R¹ für Nitro, R¹⁴ für n-Propyl und R¹⁵ für Methylcarbonyl stehen: 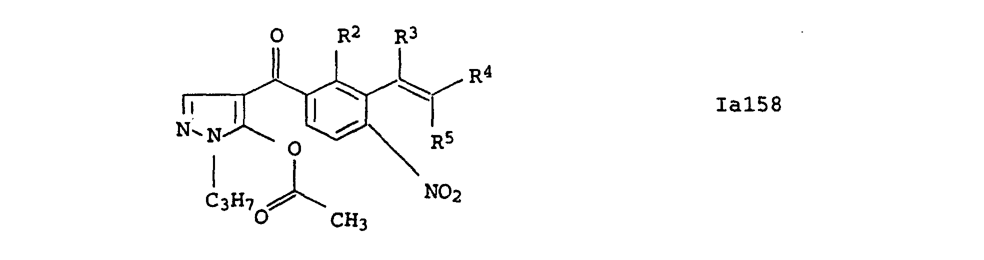
- Ebenso außergewöhnlich bevorzugt sind die Verbindungen Ia159, insbesondere die Verbindungen Ia159.001-Ia159.180, die sich von den entsprechenden Verbindungen Ia1.0a1-Ia1.180 dadurch unterscheiden, daß R¹ für Nitro, R¹⁴ für n-Butyl und R¹⁵ für Methylcarbonyl stehen: 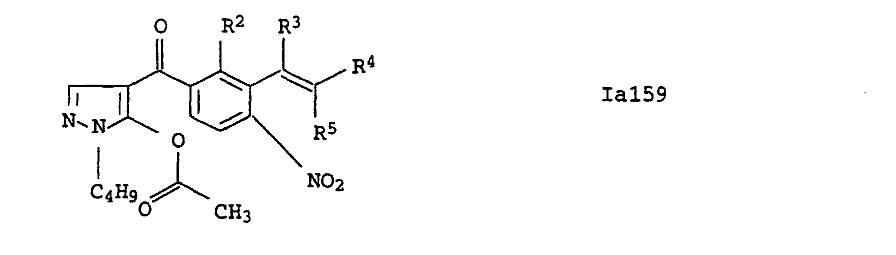
- Ebenso außergewöhnlich bevorzugt sind die Verbindungen Ia160, insbesondere die Verbindungen Ia160.001-Ia160.180, die sich von den entsprechenden Verbindungen Ia1.001-Ia1.180 dadurch unterscheiden, daß R¹ für Nitro, R¹⁴ für iso-Butyl und R¹⁵ für Methylcarbonyl stehen: 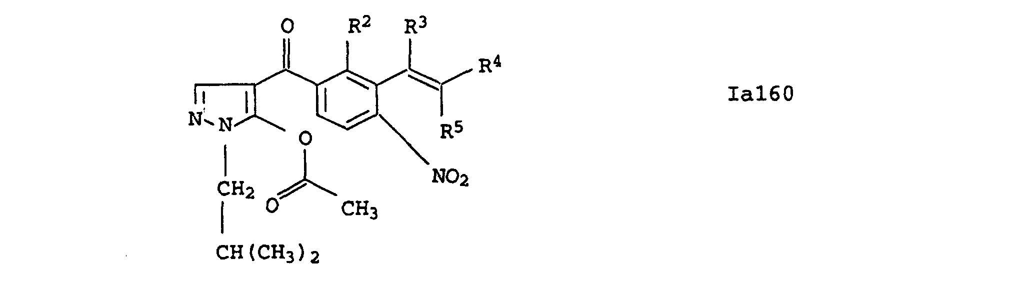
- Ebenso außergewöhnlich bevorzugt sind die Verbindungen Ia161, insbesondere die Verbindungen Ia161.001-Ia161.180, die sich von den entsprechenden Verbindungen Ia1.001-Ia1.180 dadurch unterscheiden, daß R¹ für Nitro und R¹⁵ für Ethylcarbonyl stehen: 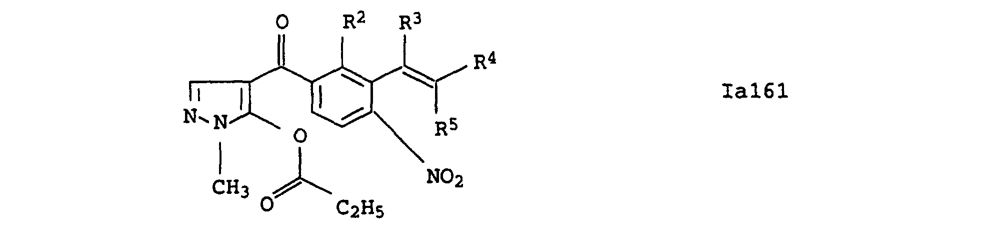
- Ebenso außergewöhnlich bevorzugt sind die Verbindungen Ia162, insbesondere die Verbindungen Ia162.001-Ia162.180, die sich von den entsprechenden Verbindungen Ia1.001-Ia1.180 dadurch unterscheiden, daß R¹ Nitro, R¹⁴ für Ethyl und R¹⁵ für Ethylcarbonyl stehen: 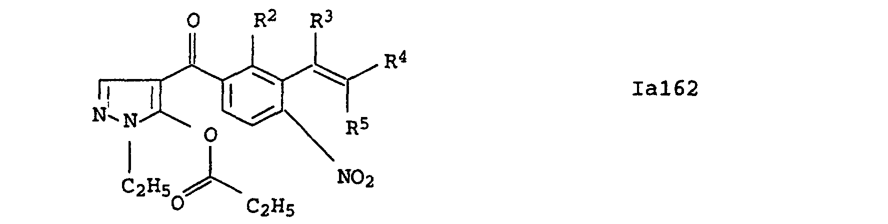
- Ebenso außergewöhnlich bevorzugt sind die Verbindungen Ia163, insbesondere die Verbindungen Ia163.001-Ia163.180, die sich von den entsprechenden Verbindungen Ia1.001-Ia1.180 dadurch unterscheiden, daß R¹ für Nitro, R¹⁴ für n-Propyl und R¹⁵ für Ethylcarbonyl stehen: 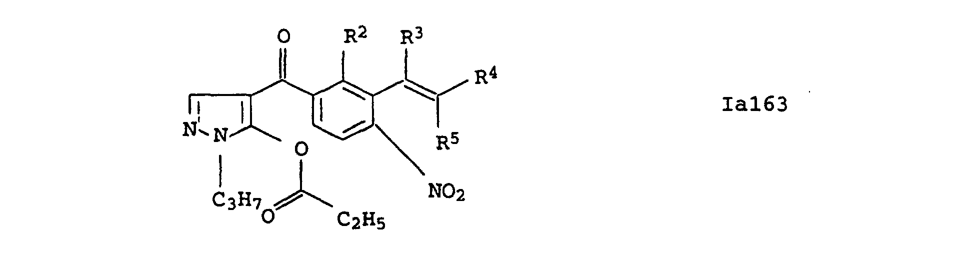
- Ebenso außergewöhnlich bevorzugt sind die Verbindungen Ia164, insbesondere die Verbindungen Ia164.001-Ia164.180, die sich von den entsprechenden Verbindungen Ia1.001-Ia1.180 dadurch unterscheiden, daß R¹ Nitro, R¹⁴ für n-Butyl und R¹⁵ für Ethylcarbonyl stehen: 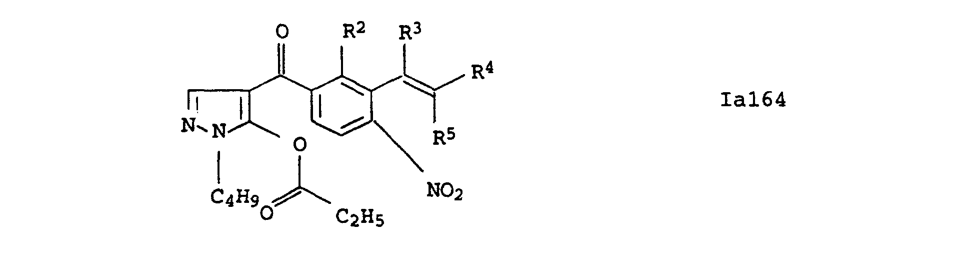
- Ebenso außergewöhnlich bevorzugt sind die Verbindungen Ia165, insbesondere die Verbindungen Ia165.001-Ia165.180, die sich von den entsprechenden Verbindungen Ia1.001-Ia1.180 dadurch unterscheiden, daß R¹ Nitro, R¹⁴ für iso-Butyl und R¹⁵ für Ethylcarbonyl stehen: 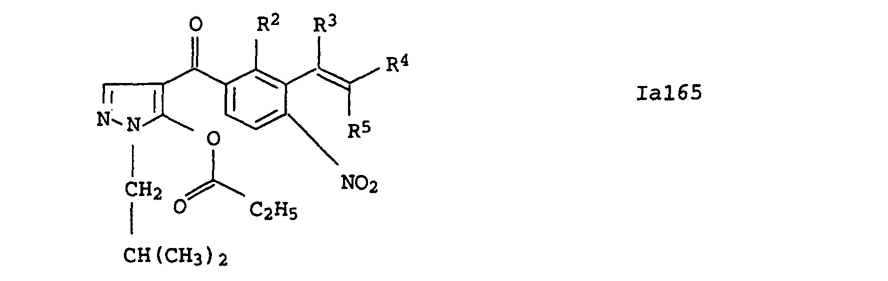
- Ebenso außergewöhnlich bevorzugt sind die Verbindungen Ia166, insbesondere die Verbindungen Ia166.001-Ia166.180, die sich von den entsprechenden Verbindungen Ia1.001-Ia1.180 dadurch unterscheiden, daß R¹ für Nitro und R¹⁵ für n-Propylcarbonyl stehen: 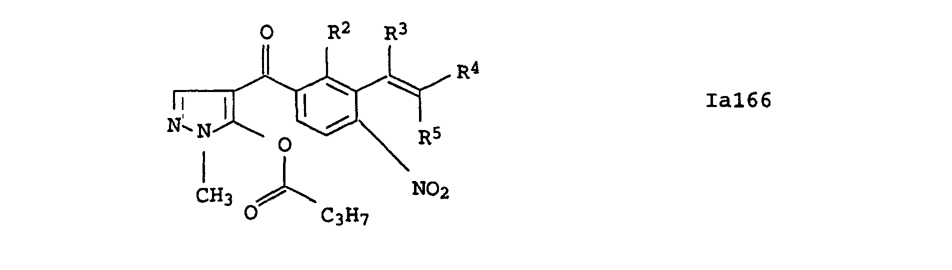
- Ebenso außergewöhnlich bevorzugt sind die Verbindungen Ia167, insbesondere die Verbindungen Ia167.001-Ia167.180, die sich von den entsprechenden Verbindungen Ia1.001-Ia1.180 dadurch unterscheiden, daß R¹ für Nitro, R¹⁴ für Ethyl und R¹⁵ für n-Propylcarbonyl stehen: 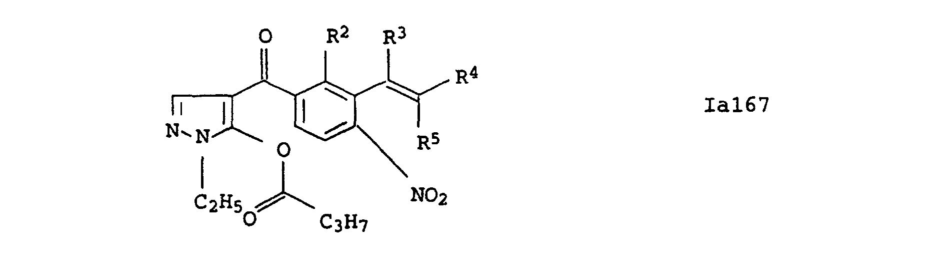
- Ebenso außergewöhnlich bevorzugt sind die Verbindungen Ia168, insbesondere die Verbindungen Ia168.001-Ia168.180, die sich von den entsprechenden Verbindungen Ia1.001-Ia1.180 dadurch unterscheiden, daß R¹ für Nitro, R¹⁴ für n-Propyl und R¹⁵ für n-Propylcarbonyl stehen: 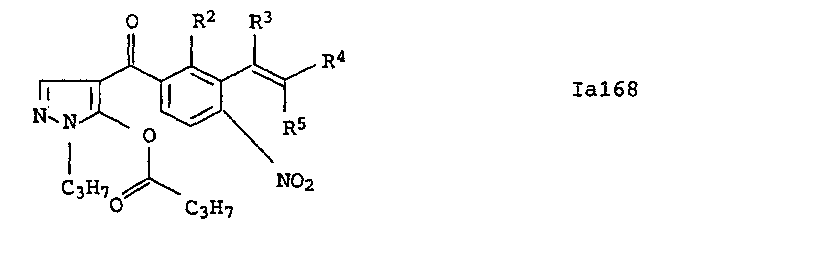
- Ebenso außergewöhnlich bevorzugt sind die Verbindungen Ia169, insbesondere die Verbindungen Ia169.001-Ia169.180, die sich von den entsprechenden Verbindungen Ia1.001-Ia1.180 dadurch unterscheiden, daß R¹ Nitro, R¹⁴ für n-Butyl und R¹⁵ für n-Propylcarbonyl stehen: 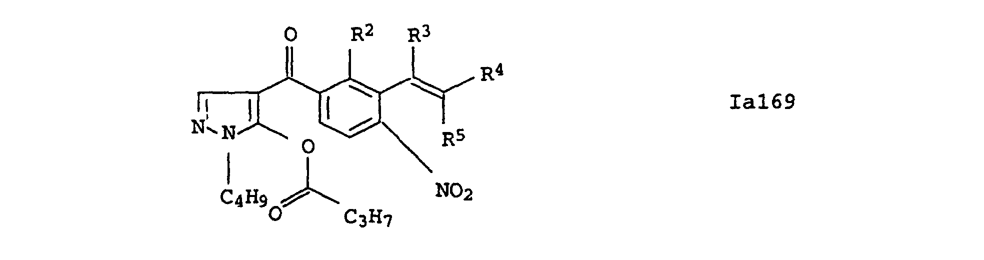
- Ebenso außergewöhnlich bevorzugt sind die Verbindungen Ia170, insbesondere die Verbindungen Ia170.001-Ia170.180, die sich von den entsprechenden Verbindungen Ia1.001-Ia1.180 dadurch unterscheiden, daß R¹ für Nitro, R¹⁴ für iso-Butyl und R¹⁵ für n-Propylcarbonyl stehen: 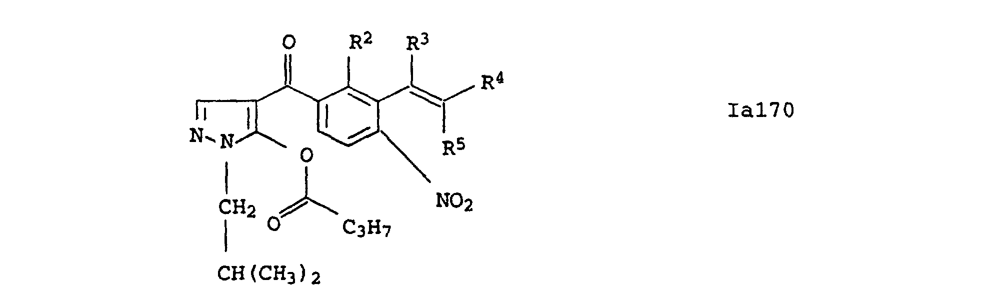
- Ebenso außergewöhnlich bevorzugt sind die Verbindungen Ia171, insbesondere die Verbindungen Ia171.001-Ia171.180, die sich von den entsprechenden Verbindungen Ia1.001-Ia1.180 dadurch unterscheiden, daß R¹ für Nitro und R¹⁵ für Trifluormethylcarbonyl stehen: 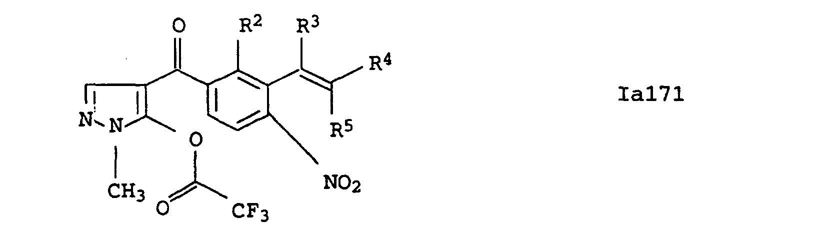
- Ebenso außergewöhnlich bevorzugt sind die Verbindungen Ia172, insbesondere die Verbindungen Ia172.001-Ia172.180, die sich von den entsprechenden Verbindungen Ia1.001-Ia1.180 dadurch unterscheiden, daß R¹ für Nitro, R¹⁴ für Ethyl und R¹⁵ für Trifluormethylcarbonyl stehen: 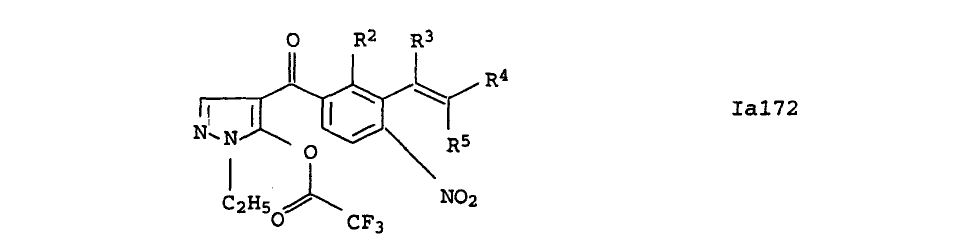
- Ebenso außergewöhnlich bevorzugt sind die Verbindungen Ia173, insbesondere die Verbindungen Ia173.001-Ia173.180, die sich von den entsprechenden Verbindungen Ia1.001-Ia1.180 dadurch unterscheiden, daß R¹ für Nitro, R¹⁴ für n-Propyl und R¹⁵ für Trifluormethylcarbonyl stehen: 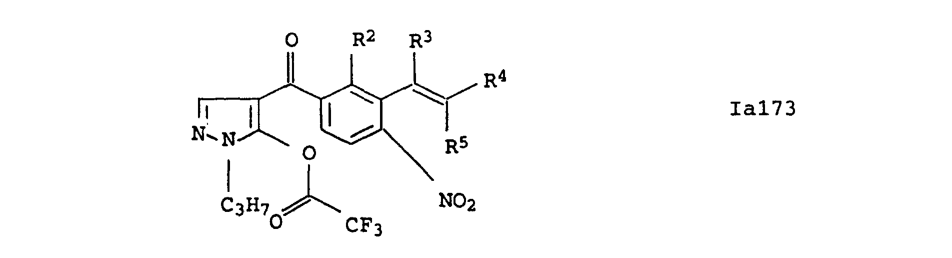
- Ebenso außergewöhnlich bevorzugt sind die Verbindungen Ia174, insbesondere die Verbindungen Ia174.001-Ia174.180, die sich von den entsprechenden Verbindungen Ia1.001-Ia1.180 dadurch unterscheiden, daß R¹ Nitro, R¹⁴ für n-Butyl und R¹⁵ für Trifluormethylcarbonyl stehen: 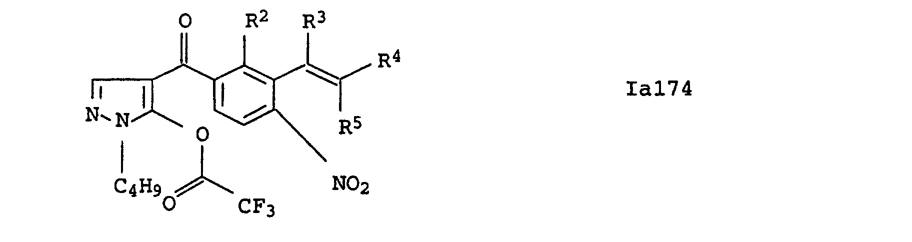
- Ebenso außergewöhnlich bevorzugt sind die Verbindungen Ia175, insbesondere die Verbindungen Ia175.001-Ia175.180, die sich von den entsprechenden Verbindungen Ia1.001-Ia1.180 dadurch unterscheiden, daß R¹ Nitro, R¹⁴ für iso-Butyl und R¹⁵ für Trifluormethylcarbonyl stehen: 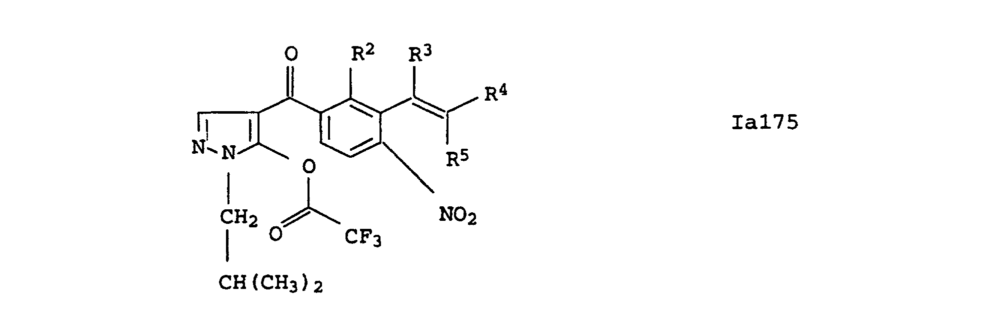
- Ebenso außergewöhnlich bevorzugt sind die Verbindungen Ia176, insbesondere die Verbindungen Ia176.001-Ia176.180, die sich von den entsprechenden Verbindungen Ia1.001-Ia1.180 dadurch unterscheiden, daß R¹ für Nitro und R¹⁵ für Methylsulfonyl stehen: 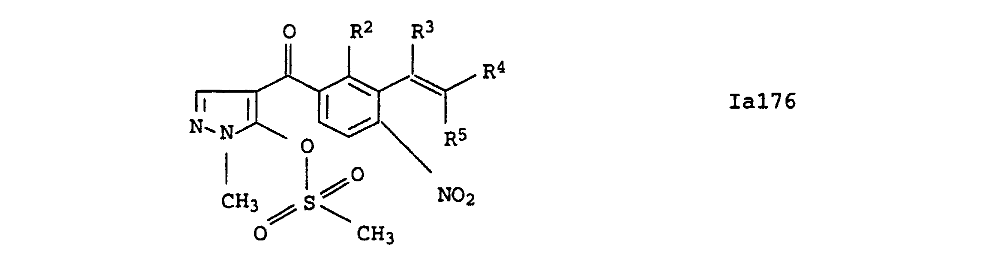
- Ebenso außergewöhnlich bevorzugt sind die Verbindungen Ia177, insbesondere die Verbindungen Ia177.001-Ia177.180, die sich von den entsprechenden Verbindungen Ia1.001-Ia1.180 dadurch unterscheiden, daß R¹ für Nitro, R¹⁴ für Ethyl und R¹⁵ für Methylsulfonyl stehen: 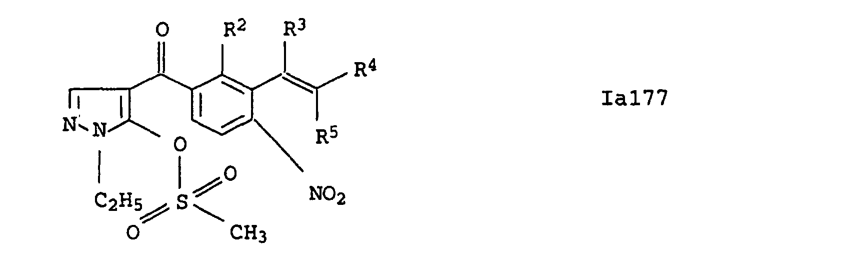
- Ebenso außergewöhnlich bevorzugt sind die Verbindungen Ia178, insbesondere die Verbindungen Ia178.001-Ia178.180, die sich von den entsprechenden Verbindungen Ia1.001-Ia1.180 dadurch unterscheiden, daß R¹ Nitro, R¹⁴ für n-Propyl und R¹⁵ für Methylsulfonyl stehen: 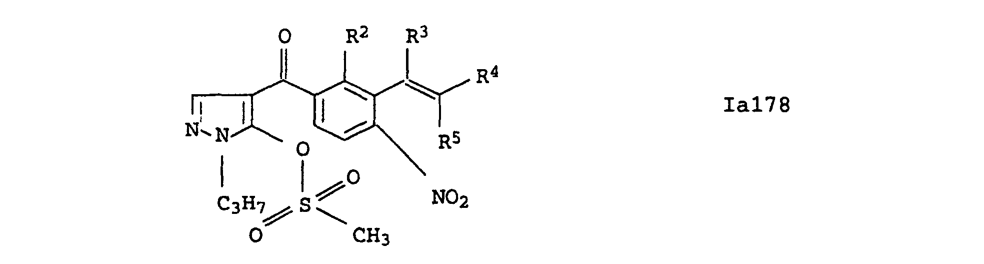
- Ebenso außergewöhnlich bevorzugt sind die Verbindungen Ia179, insbesondere die Verbindungen Ia179.001-Ia179.180, die sich von den entsprechenden Verbindungen Ia1.001-Ia1.180 dadurch unterscheiden, daß R¹ für Nitro, R¹⁴ für n-Butyl und R¹⁵ für Methylsulfonyl stehen: 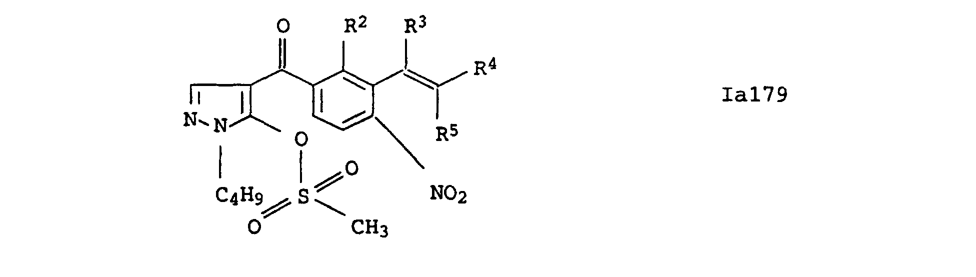
- Ebenso außergewöhnlich bevorzugt sind die Verbindungen Ia180, insbesondere die Verbindungen Ia180.001-Ia180.180, die sich von den entsprechenden Verbindungen Ia1.001-Ia1.180 dadurch unterscheiden, daß R¹ für Nitro, R¹⁴ für iso-Butyl und R¹⁵ für Methylsulfonyl stehen: 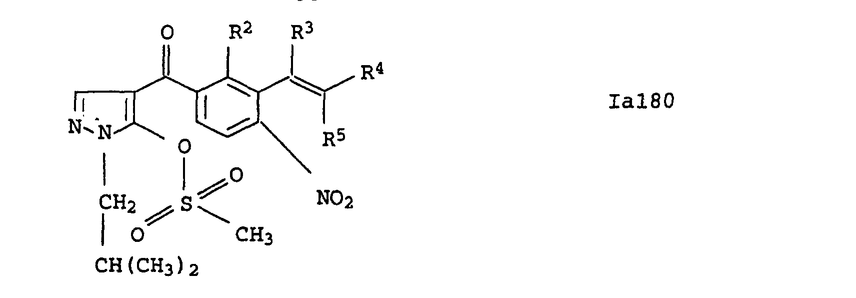
- Ebenso außergewöhnlich bevorzugt sind die Verbindungen Ia181, insbesondere die Verbindungen Ia181.001-Ia181.180, die sich von den entsprechenden Verbindungen Ia1.001-Ia1.180 dadurch unterscheiden, daß R¹ Nitro und R¹⁵ für Ethylsulfonyl stehen: 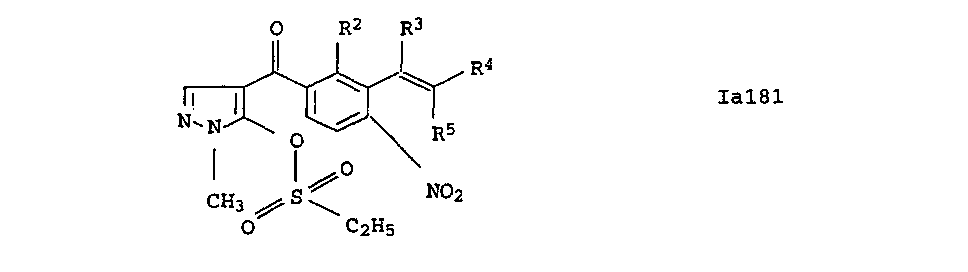
- Ebenso außergewöhnlich bevorzugt sind die Verbindungen Ia182, insbesondere die Verbindungen Ia182.001-Ia182.180, die sich von den entsprechenden Verbindungen Ia1.001-Ia1.180 dadurch unterscheiden, daß R¹ Nitro, R¹⁴ für Ethyl und R¹⁵ für Ethylsulfonyl stehen: 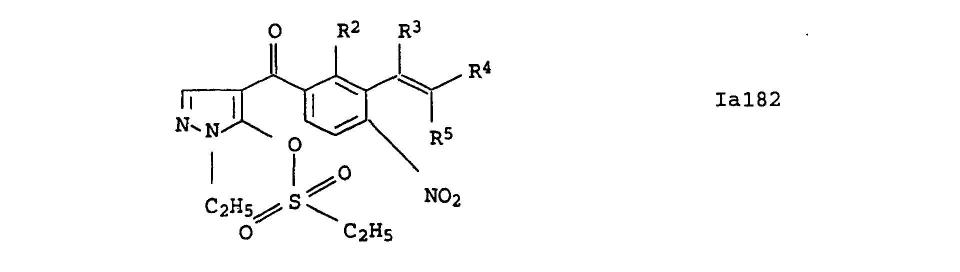
- Ebenso außergewöhnlich bevorzugt sind die Verbindungen Ia183, insbesondere die Verbindungen Ia183.001-Ia183.180, die sich von den entsprechenden Verbindungen Ia1.001-Ia1.180 dadurch unterscheiden, daß R¹ Nitro, R¹⁴ für n-Propyl und R¹⁵ für Ethylsulfonyl stehen: 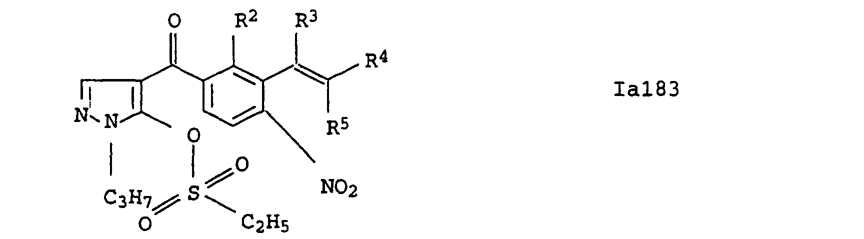
- Ebenso außergewöhnlich bevorzugt sind die Verbindungen Ia184, insbesondere die Verbindungen Ia184.001-Ia184.180, die sich von den entsprechenden Verbindungen Ia1.001-Ia1.180 dadurch unterscheiden, daß R¹ Nitro, R¹⁴ für n-Butyl und R¹⁵ für Ethylsulfonyl stehen: 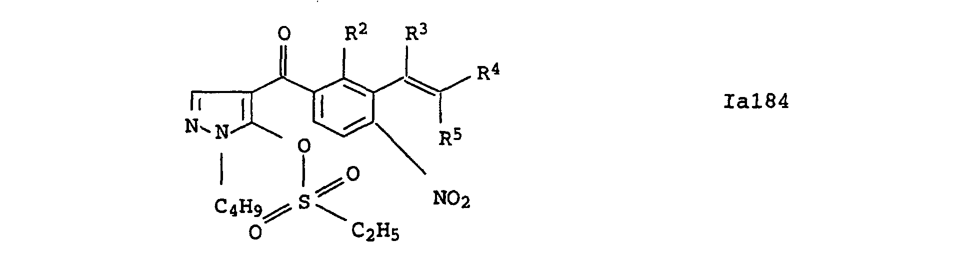
- Ebenso außergewöhnlich bevorzugt sind die Verbindungen Ia185, insbesondere die Verbindungen Ia185.001-Ia185.180, die sich von den entsprechenden Verbindungen Ia1.001-Ia1.180 dadurch unterscheiden, daß R¹ Nitro, R¹⁴ für iso-Butyl und R¹⁵ für Ethylsulfonyl stehen: 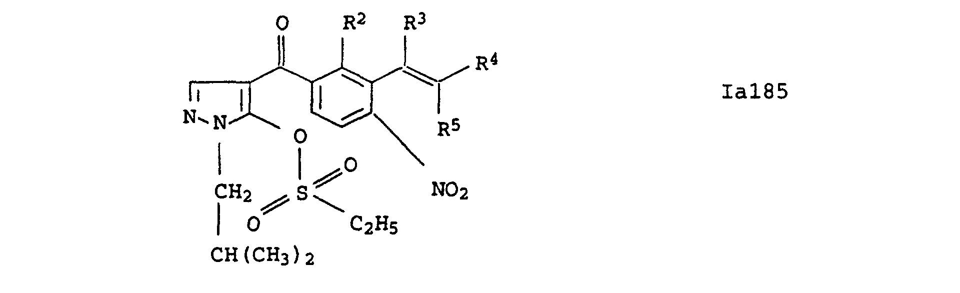
- Ebenso außergewöhnlich bevorzugt sind die Verbindungen Ia186, insbesondere die Verbindungen Ia186.001-Ia186.180, die sich von den entsprechenden Verbindungen Ia1.001-Ia1.180 dadurch unterscheiden, daß R¹ Nitro und R¹⁵ für n-Propylsulfonyl stehen: 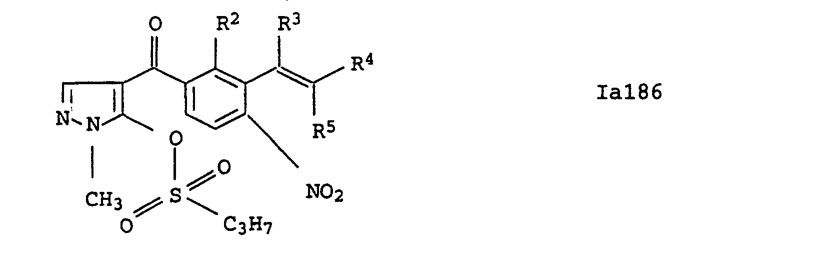
- Ebenso außergewöhnlich bevorzugt sind die Verbindungen Ia187, insbesondere die Verbindungen Ia187.001-Ia187.180, die sich von den entsprechenden Verbindungen Ia1.001-Ia1.180 dadurch unterscheiden, daß R¹ Nitro, R¹⁴ für Ethyl und R¹⁵ für n-Propylsulfonyl stehen: 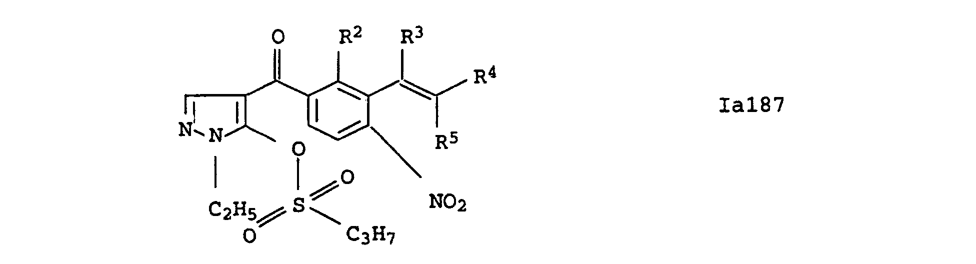
- Ebenso außergewöhnlich bevorzugt sind die Verbindungen Ia188, insbesondere die Verbindungen Ia188.001-Ia188.180, die sich von den entsprechenden Verbindungen Ia1.001-Ia1.180 dadurch unterscheiden, daß R¹ für Nitro, R¹⁴ für n-Propyl und R¹⁵ für n-Propylsulfonyl stehen: 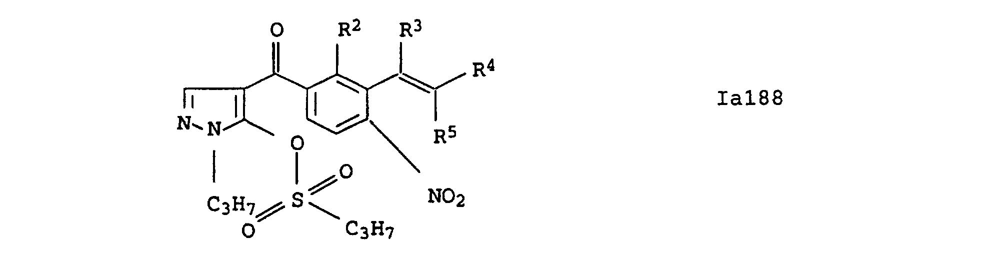
- Ebenso außergewöhnlich bevorzugt sind die Verbindungen Ia189, insbesondere die Verbindungen Ia189.001-Ia189.180, die sich von den entsprechenden Verbindungen Ia1.001-Ia1.180 dadurch unterscheiden, daß R¹ für Nitro, R¹⁴ für n-Butyl und R¹⁵ für n-Propylsulfonyl stehen: 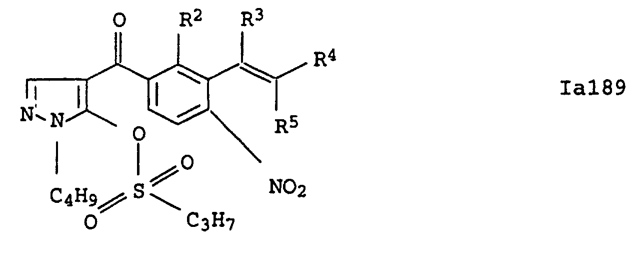
- Ebenso außergewöhnlich bevorzugt sind die Verbindungen Ia190, insbesondere die Verbindungen Ia190.001-Ia190.180, die sich von den entsprechenden Verbindungen Ia1.001-Ia1.180 dadurch unterscheiden, daß R¹ für Nitro, R¹⁴ für iso-Butyl und R¹⁵ für n-Propylsulfonyl stehen: 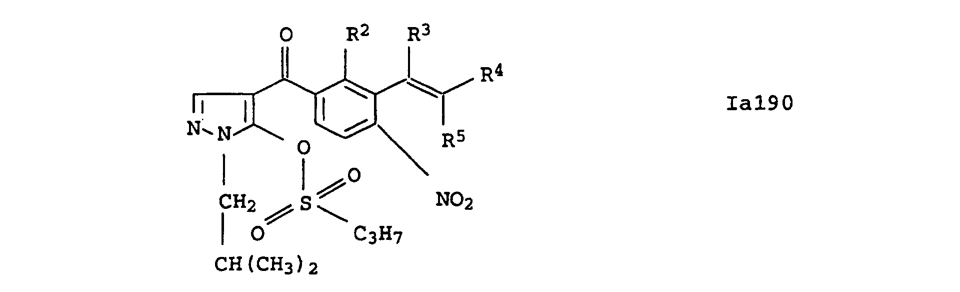
- Ebenso außergewöhnlich bevorzugt sind die Verbindungen Ia191, insbesondere die Verbindungen Ia191.001-Ia191.180, die sich von den entsprechenden Verbindungen Ia1.001-Ia1.180 dadurch unterscheiden, daß R¹ für Nitro und R¹⁵ für iso-Propylsulfonyl stehen: 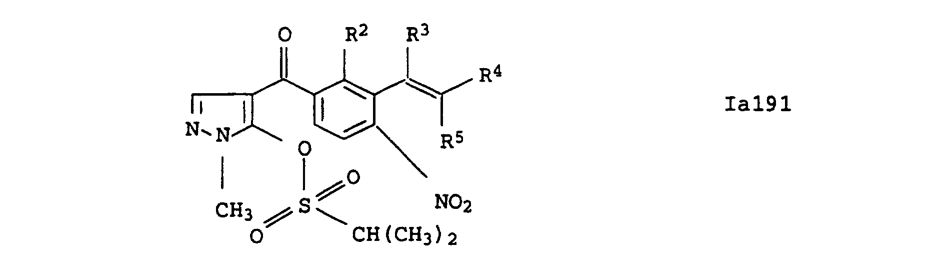
- Ebenso außergewöhnlich bevorzugt sind die Verbindungen Ia192, insbesondere die Verbindungen Ia192.001-Ia192.180, die sich von den entsprechenden Verbindungen Ia1.001-Ia1.180 dadurch unterscheiden, daß R¹ für Nitro, R¹⁴ für Ethyl und R¹⁵ für iso-Propylsulfonyl stehen: 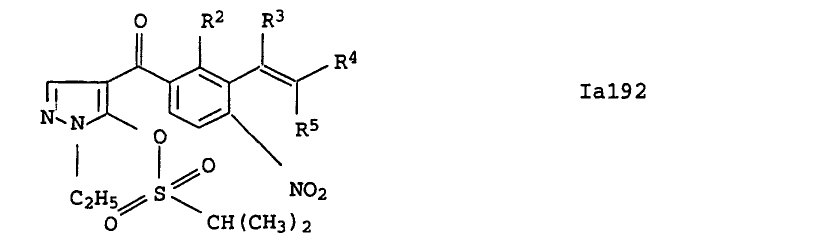
- Ebenso außergewöhnlich bevorzugt sind die Verbindungen Ia193, insbesondere die Verbindungen Ia193.001-Ia193.180, die sich von den entsprechenden Verbindungen Ia1.001-Ia1.180 dadurch unterscheiden, daß R¹ für Nitro, R¹⁴ für n-Propyl und R¹⁵ für iso-Propylsulfonyl stehen: 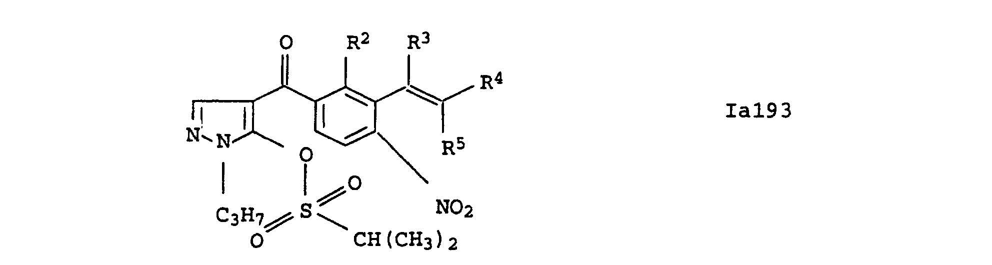
- Ebenso außergewöhnlich bevorzugt sind die Verbindungen Ia194, insbesondere die Verbindungen Ia194.001-Ia194.180, die sich von den entsprechenden Verbindungen Ia1.001-Ia1.180 dadurch unterscheiden, daß R¹ für Nitro, R¹⁴ für n-Butyl und R¹⁵ für iso-Propylsulfonyl stehen: 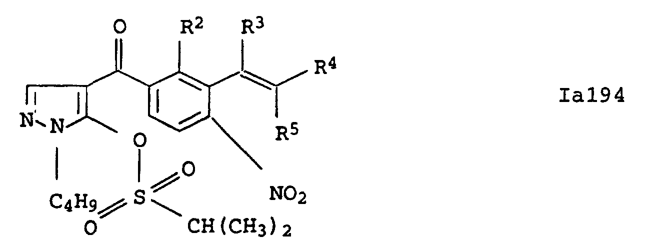
- Ebenso außergewöhnlich bevorzugt sind die Verbindungen Ia195, insbesondere die Verbindungen Ia195.001-Ia195.180, die sich von den entsprechenden Verbindungen Ia1.001-Ia1.180 dadurch unterscheiden, daß R¹ für Nitro, R¹⁴ für iso-Butyl und R¹⁵ für iso-Propylsulfonyl stehen: 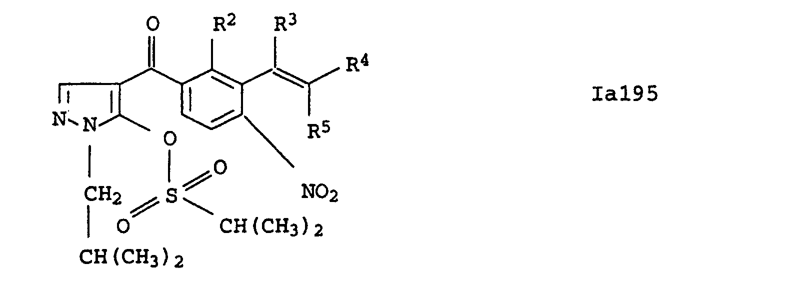
- Ebenso außergewöhnlich bevorzugt sind die Verbindungen Ia196, insbesondere die Verbindungen Ia196.001-Ia196.180, die sich von den entsprechenden Verbindungen Ia1.001-Ia1.180 dadurch unterscheiden, daß R¹ für Nitro und R¹⁵ für n-Butylsulfonyl stehen: 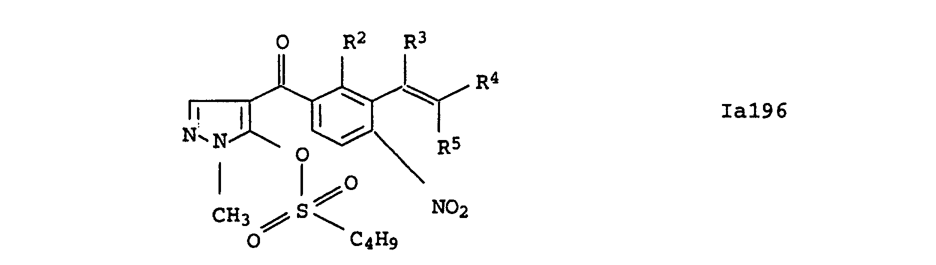
- Ebenso außergewöhnlich bevorzugt sind die Verbindungen Ia197, insbesondere die Verbindungen Ia197.001-Ia197.180, die sich von den entsprechenden Verbindungen Ia1.001-Ia1.180 dadurch unterscheiden, daß R¹ für Nitro, R¹⁴ für Ethyl und R¹⁵ für n-Butylsulfonyl stehen: 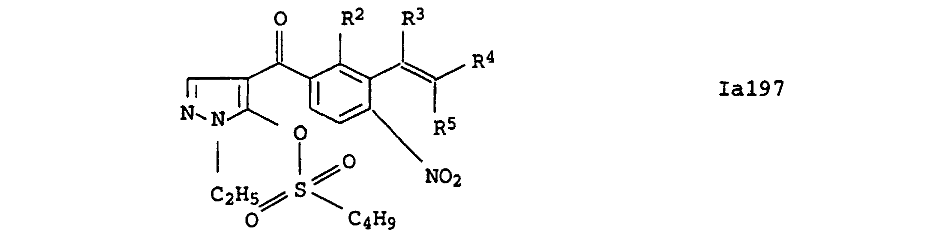
- Ebenso außergewöhnlich bevorzugt sind die Verbindungen Ia198, insbesondere die Verbindungen Ia198.001-Ia198.180, die sich von den entsprechenden Verbindungen Ia1.001-Ia1.180 dadurch unterscheiden, daß R¹ für Nitro, R¹⁴ für n-Propyl und R¹⁵ für n-Butylsulfonyl stehen: 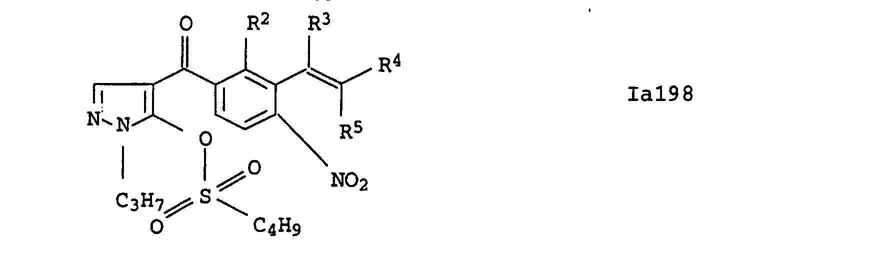
- Ebenso außergewöhnlich bevorzugt sind die Verbindungen Ia199, insbesondere die Verbindungen Ia199.001-Ia199.180, die sich von den entsprechenden Verbindungen Ia1.001-Ia1.180 dadurch unterscheiden, daß R¹ für Nitro, R¹⁴ für n-Butyl und R¹⁵ für n-Butylsulfonyl stehen: 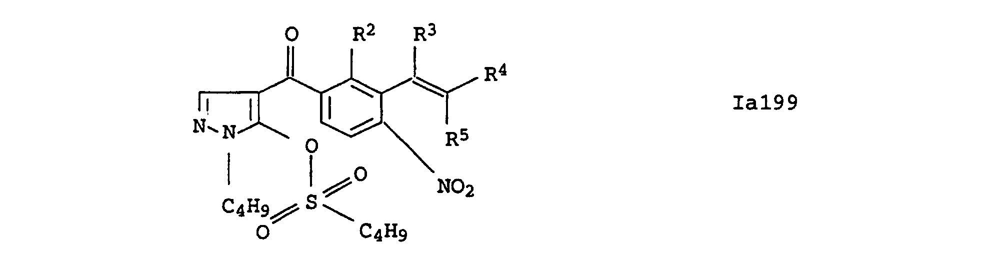
- Ebenso außergewöhnlich bevorzugt sind die Verbindungen Ia200, insbesondere die Verbindungen Ia200.001-Ia200.180, die sich von den entsprechenden Verbindungen Ia1.001-Ia1.180 dadurch unterscheiden, daß R¹ für Nitro, R¹⁴ für iso-Butyl und R¹⁵ für n-Butylsulfonyl stehen: 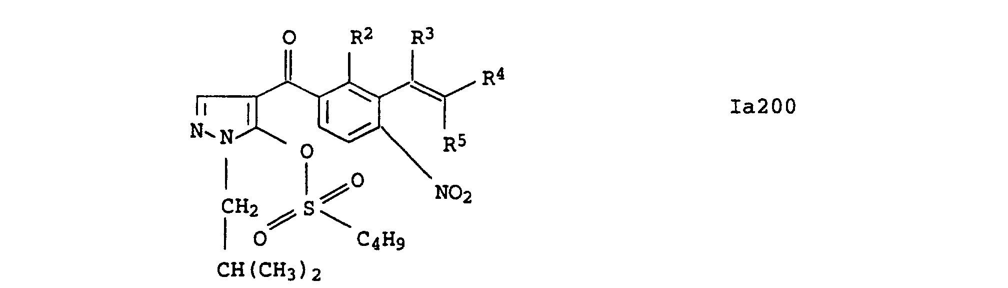
- Ebenso außergewöhnlich bevorzugt sind die Verbindungen Ia201, insbesondere die Verbindungen Ia201.001-Ia201.180, die sich von den entsprechenden Verbindungen Ia1.001-Ia1.180 dadurch unterscheiden, daß R¹ für Nitro und R¹⁵ für iso-Butylsulfonyl stehen: 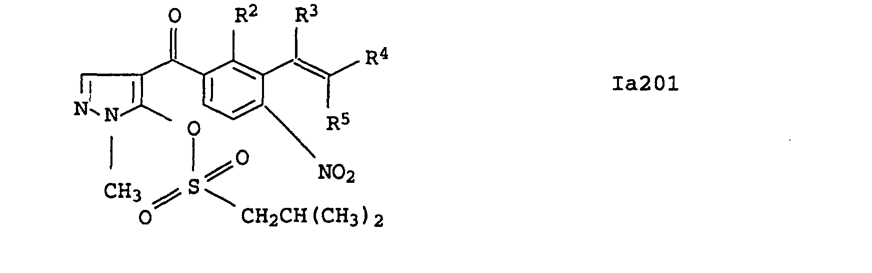
- Ebenso außergewöhnlich bevorzugt sind die Verbindungen Ia202, insbesondere die Verbindungen Ia202.001-Ia202.180, die sich von den entsprechenden Verbindungen Ia1.001-Ia1.180 dadurch unterscheiden, daß R¹ für Nitro, R¹⁴ für Ethyl und R¹⁵ für iso-Butylsulfonyl stehen: 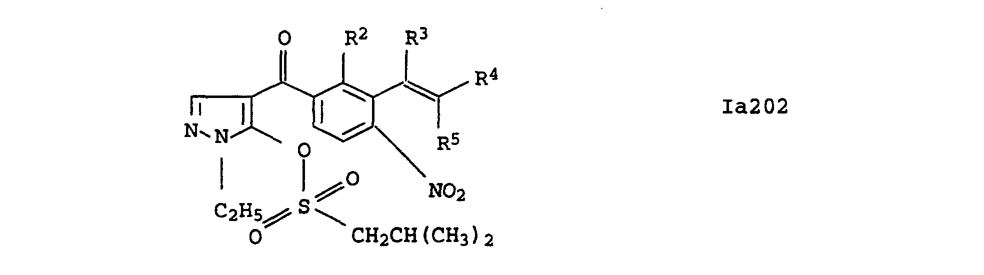
- Ebenso außergewöhnlich bevorzugt sind die Verbindungen Ia203, insbesondere die Verbindungen Ia203.001-Ia203.180, die sich von den entsprechenden Verbindungen Ia1.001-Ia1.180 dadurch unterscheiden, daß R¹ für Nitro, R¹⁴ für n-Propyl und R¹⁵ für iso-Butylsulfonyl stehen: 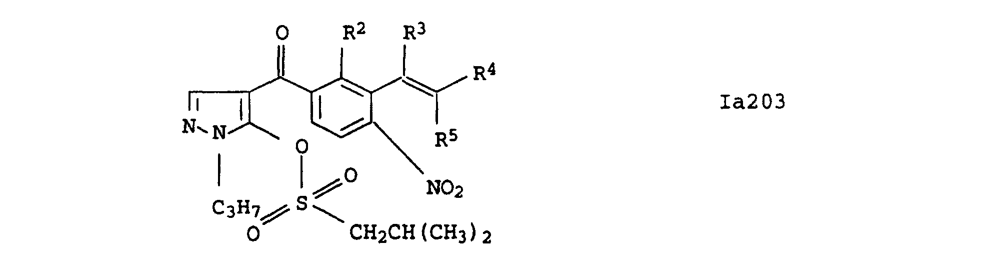
- Ebenso außergewöhnlich bevorzugt sind die Verbindungen Ia204, insbesondere die Verbindungen Ia204.001-Ia204.180, die sich von den entsprechenden Verbindungen Ia1.001-Ia1.180 dadurch unterscheiden, daß R¹ für Nitro, R¹⁴ für n-Butyl und R¹⁵ für iso-Butylsulfonyl stehen: 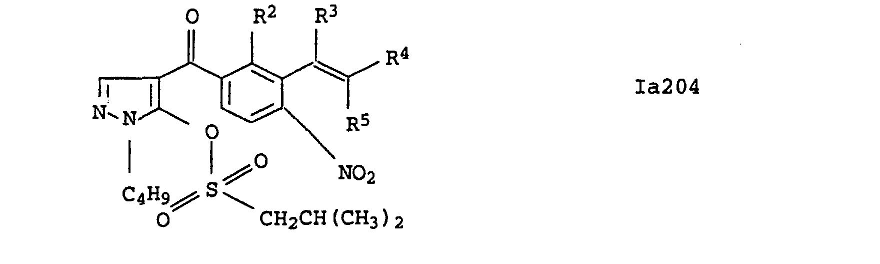
- Ebenso außergewöhnlich bevorzugt sind die Verbindungen Ia205, insbesondere die Verbindungen Ia205.001-Ia205.180, die sich von den entsprechenden Verbindungen Ia1.001-Ia1.180 dadurch unterscheiden, daß R¹ für Nitro, R¹⁴ für iso-Butyl und R¹⁵ für iso-Butylsulfonyl stehen: 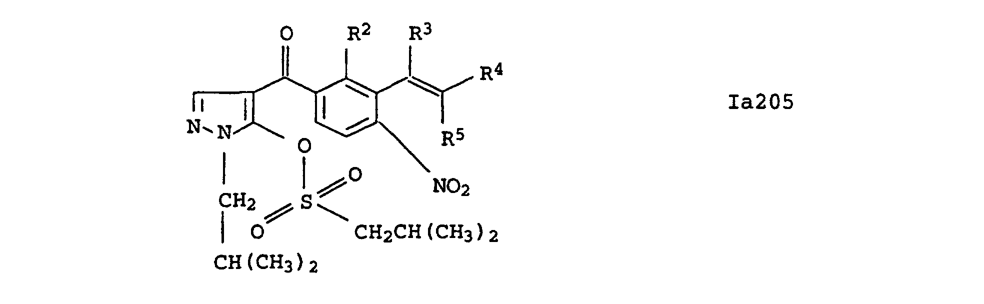
- Ebenso außergewöhnlich bevorzugt sind die Verbindungen Ia206, insbesondere die Verbindungen Ia206.001-Ia206.180, die sich von den entsprechenden Verbindungen Ia1.001-Ia1.180 dadurch unterscheiden, daß R¹ für Nitro und R¹⁵ für sec-Butylsulfonyl stehen: 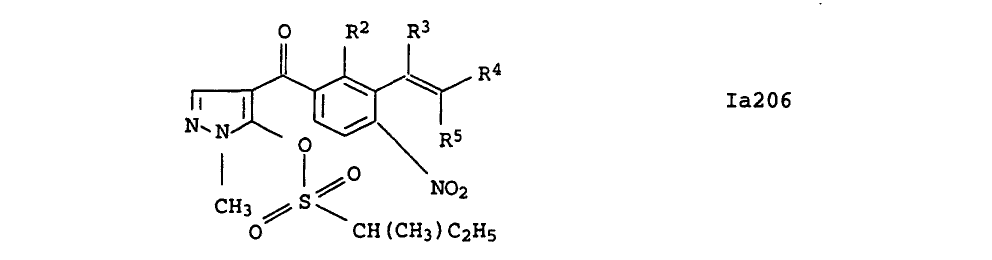
- Ebenso außergewöhnlich bevorzugt sind die Verbindungen Ia207, insbesondere die Verbindungen Ia207.001-Ia207.180, die sich von den entsprechenden Verbindungen Ia1.001-Ia1.180 dadurch unterscheiden, daß R¹ für Nitro, R¹⁴ für Ethyl und R¹⁵ für sec-Butylsulfonyl stehen: 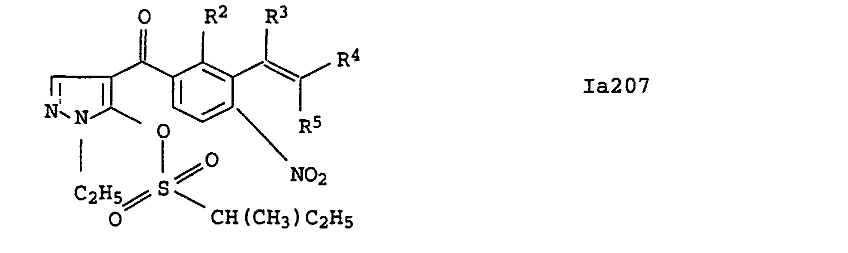
- Ebenso außergewöhnlich bevorzugt sind die Verbindungen Ia208, insbesondere die Verbindungen Ia208.001-Ia208.180, die sich von den entsprechenden Verbindungen Ia1.001-Ia1.180 dadurch unterscheiden, daß R¹ für Nitro, R¹⁴ für n-Propyl und R¹⁵ für sec-Butylsulfonyl stehen: 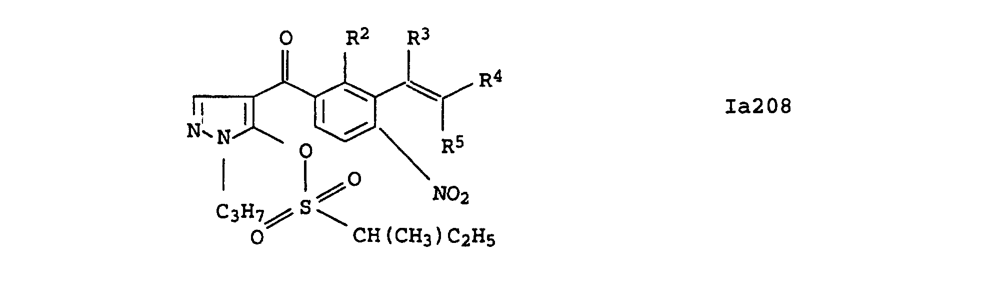
- Ebenso außergewöhnlich bevorzugt sind die Verbindungen Ia209, insbesondere die Verbindungen Ia209.001-Ia209.180, die sich von den entsprechenden Verbindungen Ia1.001-Ia1.180 dadurch unterscheiden, daß R¹ für Nitro, R¹⁴ für n-Butyl und R¹⁵ für sec-Butylsulfonyl stehen: 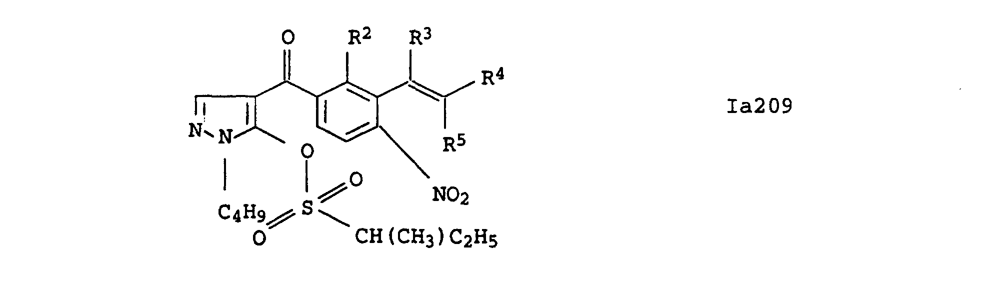
- Ebenso außergewöhnlich bevorzugt sind die Verbindungen Ia210, insbesondere die Verbindungen Ia210.001-Ia210.180, die sich von den entsprechenden Verbindungen Ia1.001-Ia1.180 dadurch unterscheiden, daß R¹ für Nitro, R¹⁴ für iso-Butyl und R¹⁵ für sec-Butylsulfonyl stehen: 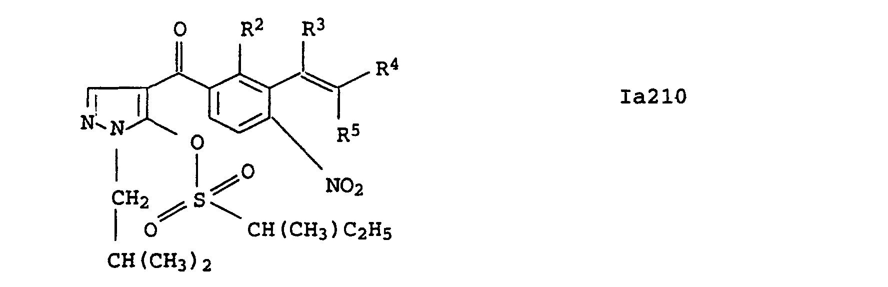
- Ebenso außergewöhnlich bevorzugt sind die Verbindungen Ia211, insbesondere die Verbindungen Ia211.001-Ia211.180, die sich von den entsprechenden Verbindungen Ia1.001-Ia1.180 dadurch unterscheiden, daß R¹ für Nitro und R¹⁵ für Trifluormethylsulfonyl stehen: 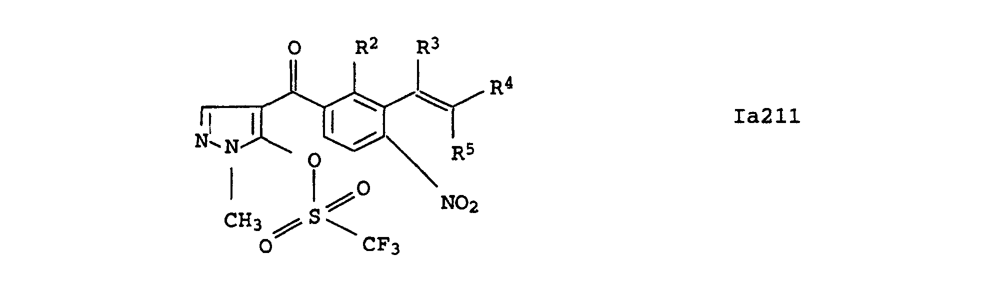
- Ebenso außergewöhnlich bevorzugt sind die Verbindungen Ia212, insbesondere die Verbindungen Ia212.001-Ia212.180, die sich von den entsprechenden Verbindungen Ia1.001-Ia1.180 dadurch unterscheiden, daß R¹ für Nitro, R¹⁴ für Ethyl und R¹⁵ für Trifluormethylsulfonyl stehen: 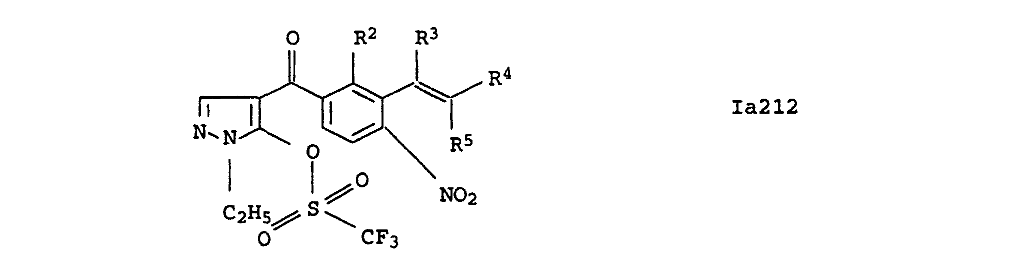
- Ebenso außergewöhnlich bevorzugt sind die Verbindungen Ia213, insbesondere die Verbindungen Ia213.001-Ia213.180, die sich von den entsprechenden Verbindungen Ia1.001-Ia1.180 dadurch unterscheiden, daß R¹ für Nitro, R¹⁴ für n-Propyl und R¹⁵ für Trifluormethylsulfonyl stehen: 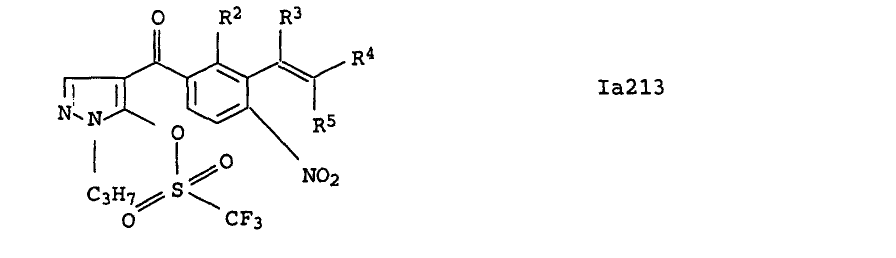
- Ebenso außergewöhnlich bevorzugt sind die Verbindungen Ia214, insbesondere die Verbindungen Ia214.001-Ia214.180, die sich von den entsprechenden Verbindungen Ia1.001-Ia1.180 dadurch unterscheiden, daß R¹ für Nitro, R¹⁴ für n-Butyl und R¹⁵ für Trifluormethylsulfonyl stehen: 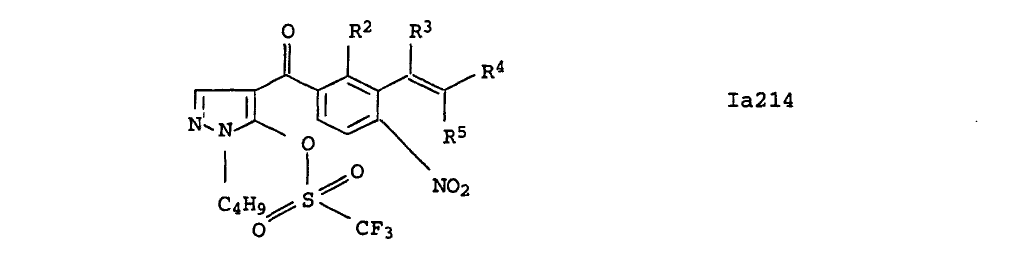
- Ebenso außergewöhnlich bevorzugt sind die Verbindungen Ia215, insbesondere die Verbindungen Ia215.001-Ia125.180, die sich von den entsprechenden Verbindungen Ia1.001-Ia1.180 dadurch unterscheiden, daß R¹ für Nitro, R¹⁴ für iso-Butyl und R¹⁵ für Trifluormethylsulfonyl stehen: 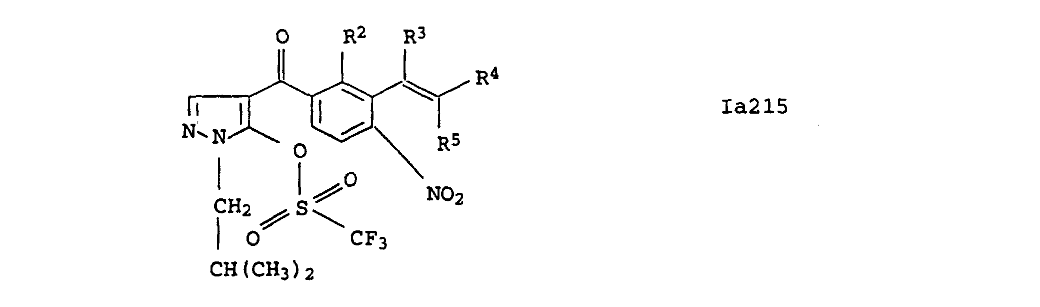
- Ebenso außergewöhnlich bevorzugt sind die Verbindungen Ia216, insbesondere die Verbindungen Ia216.001-Ia216.180, die sich von den entsprechenden Verbindungen Ia1.001-Ia1.180 dadurch unterscheiden, daß R¹ für Nitro und R¹⁵ für Phenylcarbonylmethyl stehen: 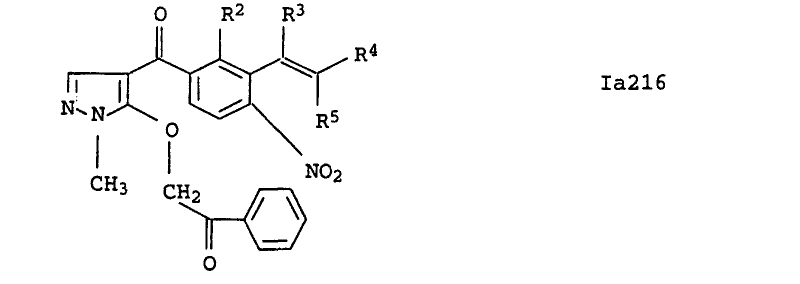
- Ebenso außergewöhnlich bevorzugt sind die Verbindungen Ia217, insbesondere die Verbindungen Ia217.001-Ia217.180, die sich von den entsprechenden Verbindungen Ia1.001-Ia1.180 dadurch unterscheiden, daß R¹ für Nitro, R¹⁴ für Ethyl und R¹⁵ für Phenylcarbonylmethyl stehen: 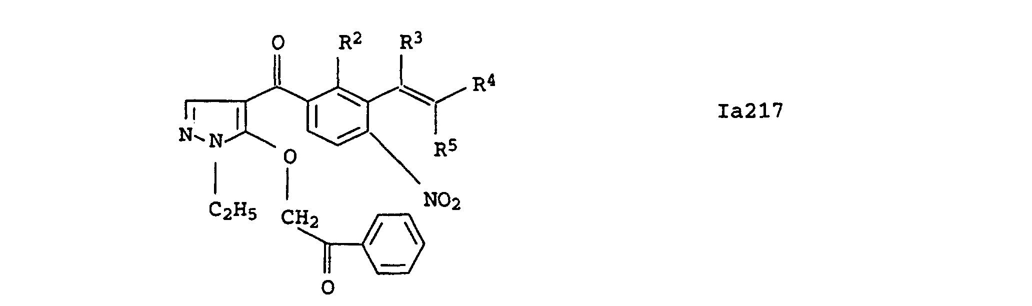
- Ebenso außergewöhnlich bevorzugt sind die Verbindungen Ia218, insbesondere die Verbindungen Ia218.001-Ia218.180, die sich von den entsprechenden Verbindungen Ia1.001-Ia1.180 dadurch unterscheiden, daß R¹ für Nitro, R¹⁴ für n-Propyl und R¹⁵ für Phenylcarbonylmethyl stehen: 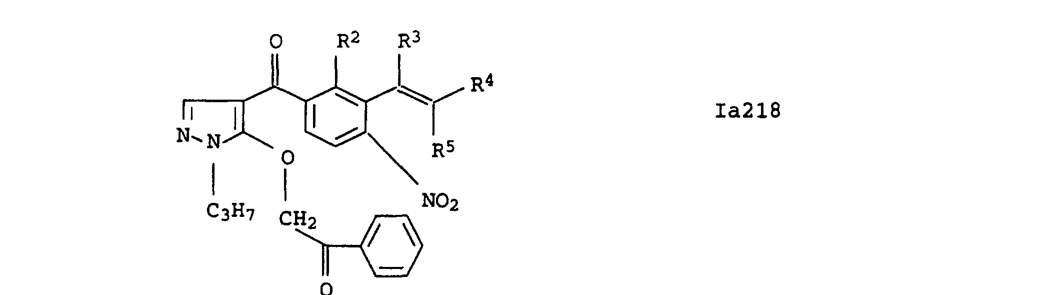
- Ebenso außergewöhnlich bevorzugt sind die Verbindungen Ia219, insbesondere die Verbindungen Ia219.001-Ia219.180, die sich von den entsprechenden Verbindungen Ia1.001-Ia1.180 dadurch unterscheiden, daß R¹ für Nitro, R¹⁴ für n-Butyl und R¹⁵ für Phenylcarbonylmethyl stehen: 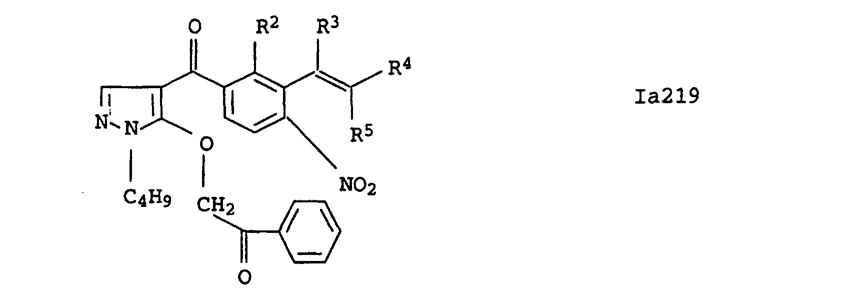
- Ebenso außergewöhnlich bevorzugt sind die Verbindungen Ia220, insbesondere die Verbindungen Ia220.001-Ia220.180, die sich von den entsprechenden Verbindungen Ia1.001-Ia1.180 dadurch unterscheiden, daß R¹ für Nitro, R¹⁴ für iso-Butyl und R¹⁵ für Phenylcarbonylmethyl stehen: 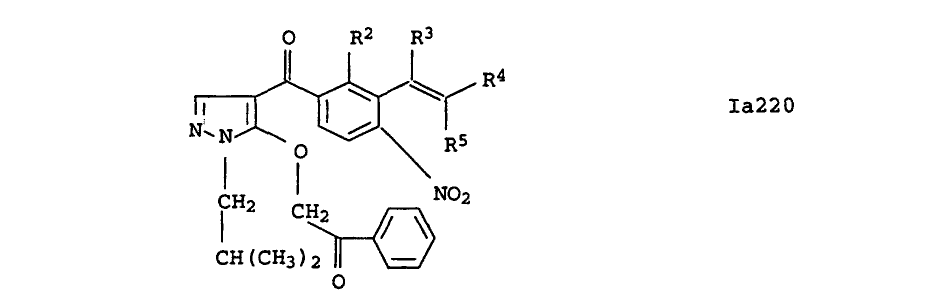
- Ebenso außergewöhnlich bevorzugt sind die Verbindungen Ia221, insbesondere die Verbindungen Ia221.001-Ia221.180, die sich von den entsprechenden Verbindungen Ia1.001-Ia1.180 dadurch unterscheiden, daß R¹ für Nitro und R¹⁵ für Phenylsulfonyl stehen: 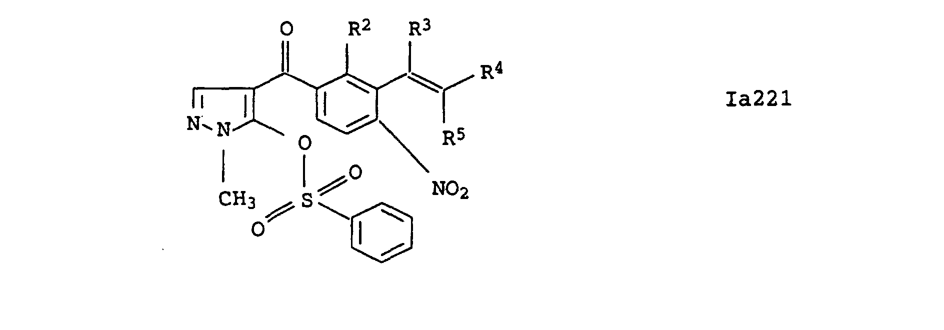
- Ebenso außergewöhnlich bevorzugt sind die Verbindungen Ia222, insbesondere die Verbindungen Ia222.001-Ia222.180, die sich von den entsprechenden Verbindungen Ia1.001-Ia1.180 dadurch unterscheiden, daß R¹ für Nitro, R¹⁴ für Ethyl und R¹⁵ für Phenylsulfonyl stehen: 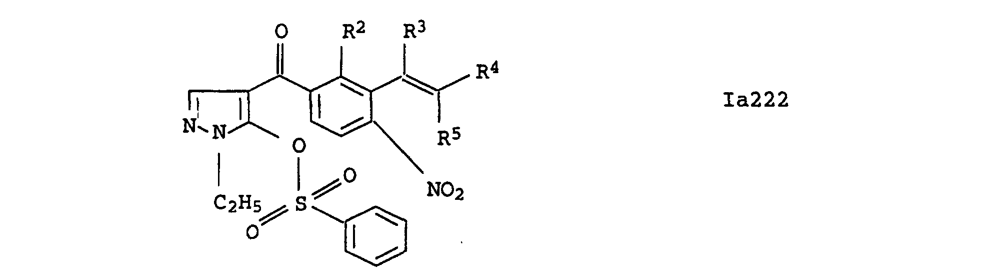
- Ebenso außergewöhnlich bevorzugt sind die Verbindungen Ia223, insbesondere die Verbindungen Ia223.001-Ia223.180, die sich von den entsprechenden Verbindungen Ia1.001-Ia1.180 dadurch unterscheiden, daß R¹ für Nitro, R¹⁴ für n-Propyl und R¹⁵ für Phenylsulfonyl stehen: 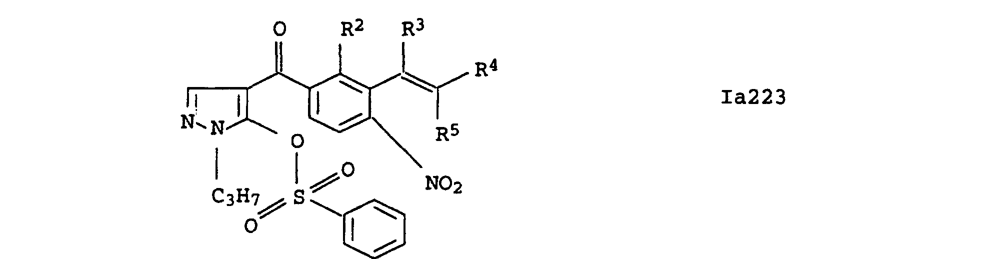
- Ebenso außergewöhnlich bevorzugt sind die Verbindungen Ia224, insbesondere die Verbindungen Ia224.001-Ia224.180, die sich von den entsprechenden Verbindungen Ia1.001-Ia1.180 dadurch unterscheiden, daß R¹ für Nitro, R¹⁴ für n-Butyl und R¹⁵ für Phenylsulfonyl stehen: 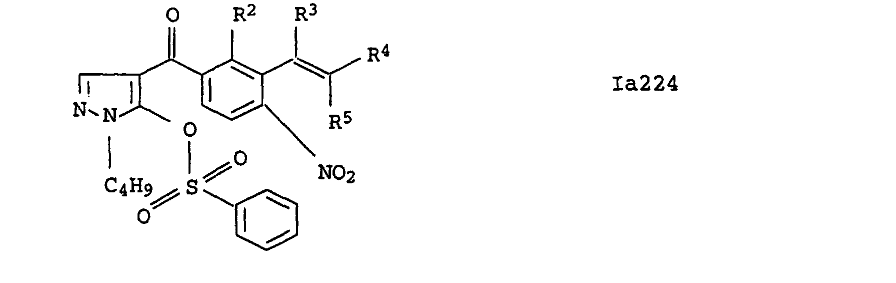
- Ebenso außergewöhnlich bevorzugt sind die Verbindungen Ia225, insbesondere die Verbindungen Ia225.441-Ia225.180, die sich von den entsprechenden Verbindungen Ia1.001-Ia1.180 dadurch unterscheiden, daß R¹ für Nitro, R¹⁴ für iso-Butyl und R¹⁵ für Phenylsulfonyl stehen: 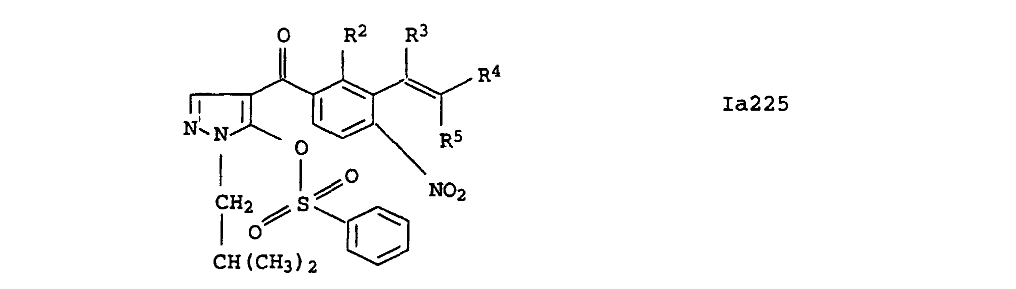
- Ebenso außergewöhnlich bevorzugt sind die Verbindungen Ia226, insbesondere die Verbindungen Ia226.001-Ia226.180, die sich von den entsprechenden Verbindungen Ia1.001-Ia1.180 dadurch unterscheiden, daß R¹ für Nitro und R¹⁵ für 4-Methylphenylsulfonyl stehen: 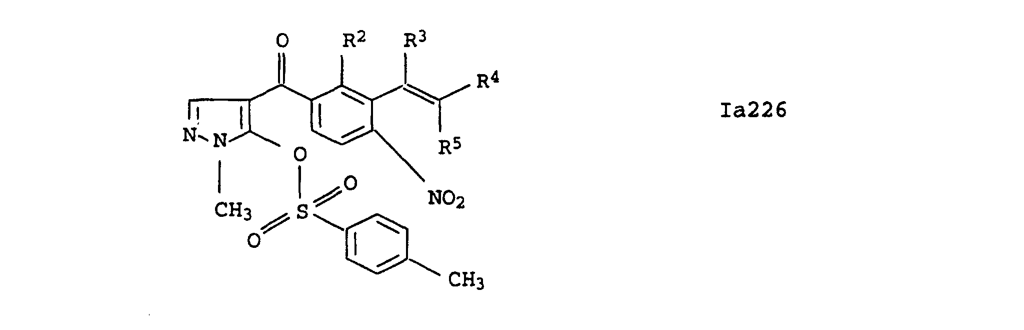
- Ebenso außergewöhnlich bevorzugt sind die Verbindungen Ia227, insbesondere die Verbindungen Ia227.001-Ia227.180, die sich von den entsprechenden Verbindungen Ia1.001-Ia1.180 dadurch unterscheiden, daß R¹ für Nitro, R¹⁴ für Ethyl und R¹⁵ für 4-Methylphenylsulfonyl stehen: 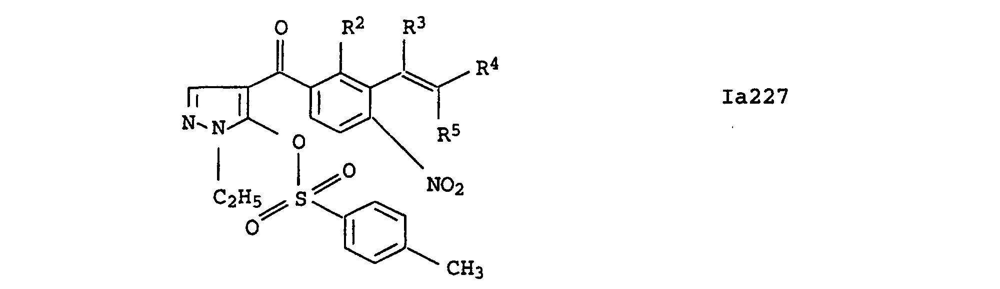
- Ebenso außergewöhnlich bevorzugt sind die Verbindungen Ia228, insbesondere die Verbindungen Ia228.001-Ia228.180, die sich von den entsprechenden Verbindungen Ia1.001-Ia1.180 dadurch unterscheiden, daß R¹ für Nitro, R¹⁴ für n-Propyl und R¹⁵ für 4-Methylphenylsulfonyl stehen: 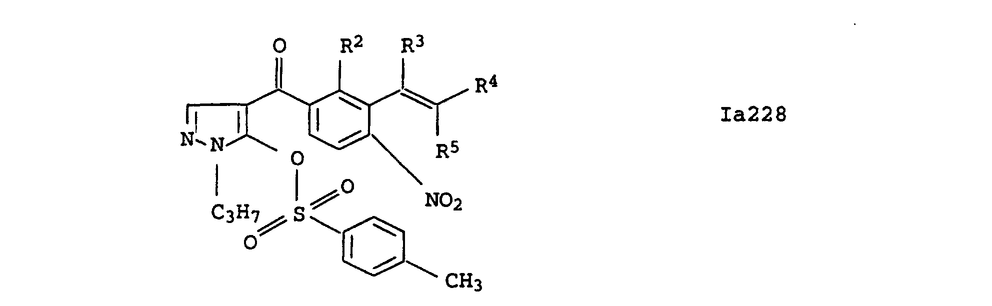
- Ebenso außergewöhnlich bevorzugt sind die Verbindungen Ia229, insbesondere die Verbindungen Ia229.001-Ia229.180, die sich von den entsprechenden Verbindungen Ia1.001-Ia1.180 dadurch unterscheiden, daß R¹ für Nitro, R¹⁴ für n-Butyl und R¹⁵ für 4-Methylphenylsulfonyl stehen: 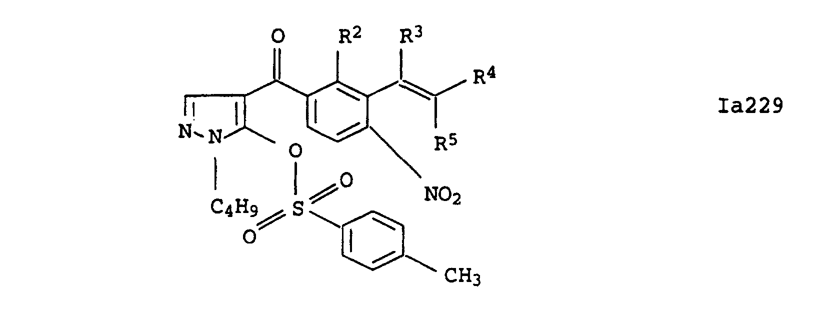
- Ebenso außergewöhnlich bevorzugt sind die Verbindungen Ia230, insbesondere die Verbindungen Ia230.001-Ia230.180, die sich von den entsprechenden Verbindungen Ia1.001-Ia1.180 dadurch unterscheiden, daß R¹ für Nitro, R¹⁴ für iso-Butyl und R¹⁵ für 4-Methylphenylsulfonyl stehen: 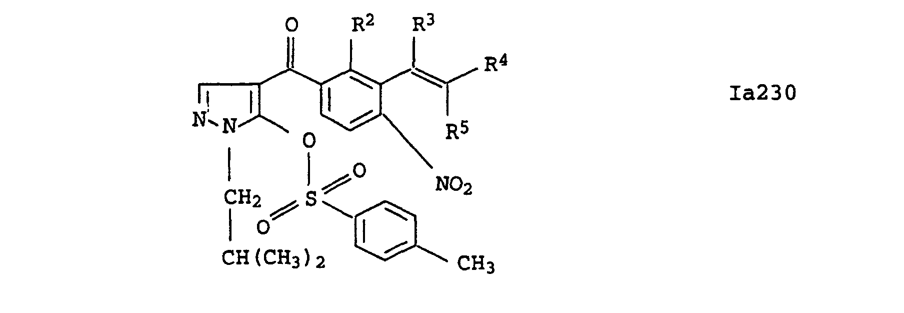
- Ebenso außergewöhnlich bevorzugt sind die Verbindungen Ia231, insbesondere die Verbindungen Ia231.001-Ia231.180, die sich von den entsprechenden Verbindungen Ia1.001-Ia1.180 dadurch unterscheiden, daß R¹ für Methylsulfonyl steht: 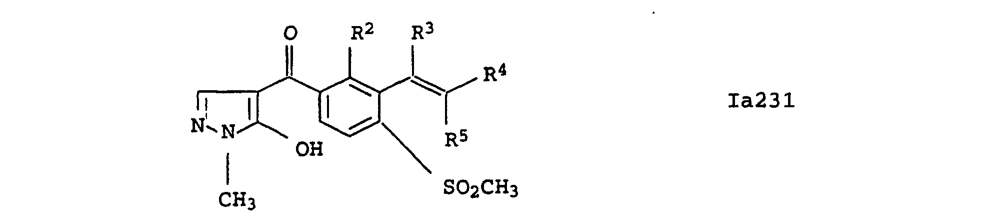
- Ebenso außergewöhnlich bevorzugt sind die Verbindungen Ia232, insbesondere die Verbindungen Ia232.001-Ia232.180, die sich von den entsprechenden Verbindungen Ia1.001-Ia1.180 dadurch unterscheiden, daß R¹ für Methylsulfonyl und R¹⁴ für Ethyl stehen: 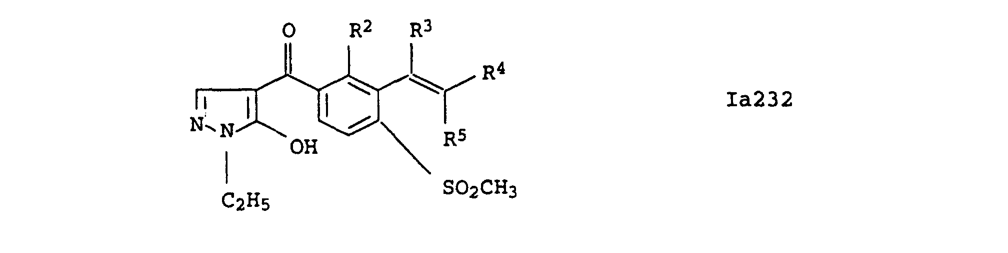
- Ebenso außergewöhnlich bevorzugt sind die Verbindungen Ia233, insbesondere die Verbindungen Ia233.001-Ia233.180, die sich von den entsprechenden Verbindungen Ia1.001-Ia1.180 dadurch unterscheiden, daß R¹ für Methylsulfonyl und R¹⁴ für n-Propyl stehen: 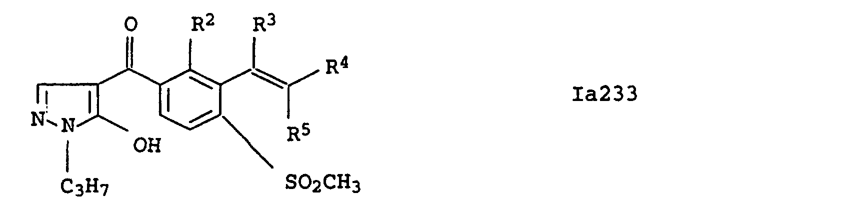
- Ebenso außergewöhnlich bevorzugt sind die Verbindungen Ia234, insbesondere die Verbindungen Ia234.001-Ia234.180, die sich von den entsprechenden Verbindungen Ia1.001-Ia1.180 dadurch unterscheiden, daß R¹ für Methylsulfonyl und R¹⁴ für n-Butyl stehen: 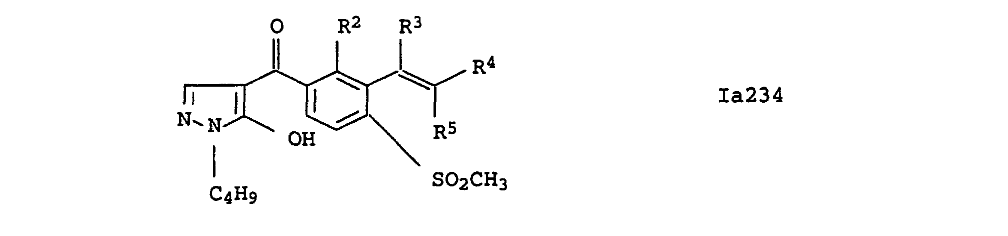
- Ebenso außergewöhnlich bevorzugt sind die Verbindungen Ia235, insbesondere die Verbindungen Ia235.001-Ia235.180, die sich von den entsprechenden Verbindungen Ia1.001-Ia1.180 dadurch unterscheiden, daß R¹ für Methylsulfonyl und R¹⁴ für iso-Butyl stehen: 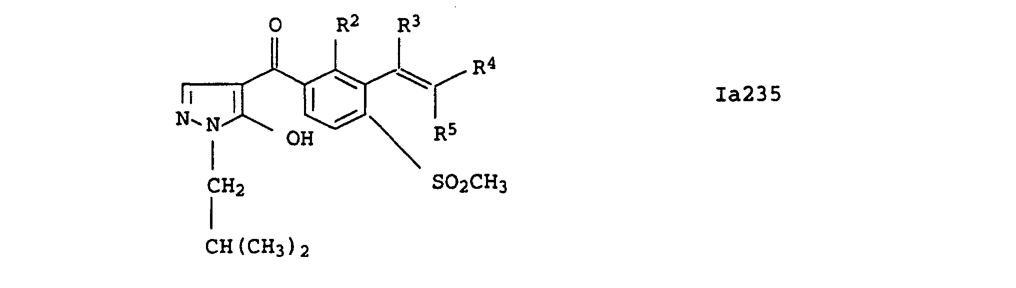
- Ebenso außergewöhnlich bevorzugt sind die Verbindungen Ia236, insbesondere die Verbindungen Ia236.001-Ia236.180, die sich von den entsprechenden Verbindungen Ia1.001-Ia1.180 dadurch unterscheiden, daß R¹ für Methylsulfonyl und R¹⁵ für Methyl stehen: 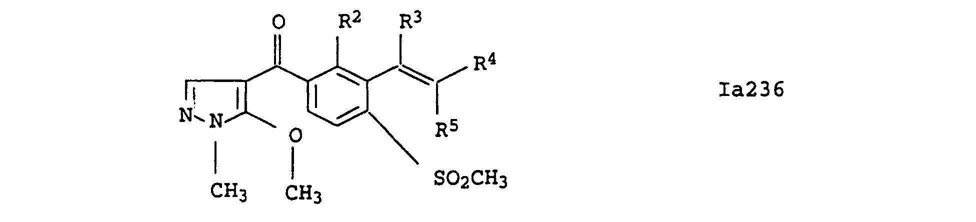
- Ebenso außergewöhnlich bevorzugt sind die Verbindungen Ia237, insbesondere die Verbindungen Ia237.001-Ia237.180, die sich von den entsprechenden Verbindungen Ia1.001-Ia1.180 dadurch unterscheiden, daß R¹ für Methylsulfonyl, R¹⁴ für Ethyl und R¹⁵ für Methyl stehen: 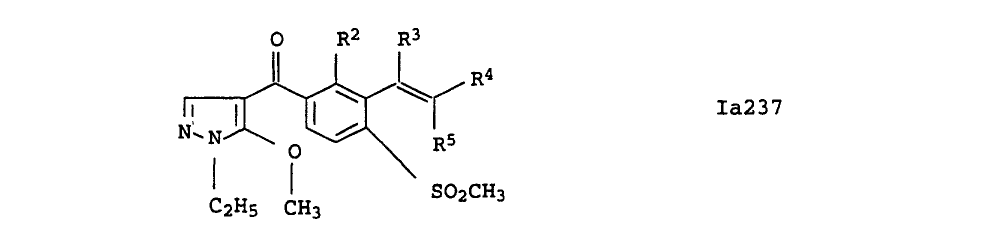
- Ebenso außergewöhnlich bevorzugt sind die Verbindungen Ia238, insbesondere die Verbindungen Ia238.001-Ia238.180, die sich von den entsprechenden Verbindungen Ia1.001-Ia1.180 dadurch unterscheiden, daß R¹ für Methylsulfonyl, R¹⁴ für n-Propyl und R¹⁵ für Methyl stehen: 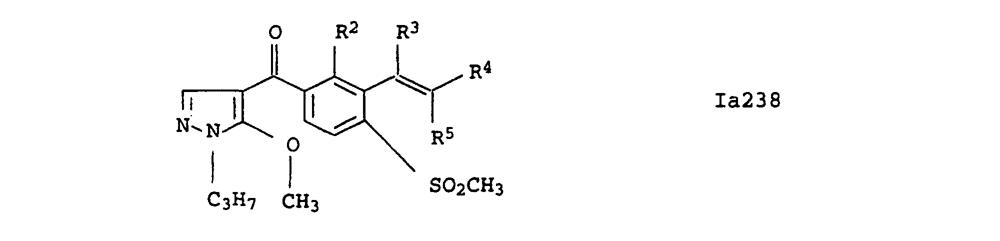
- Ebenso außergewöhnlich bevorzugt sind die Verbindungen Ia239, insbesondere die Verbindungen Ia239.001-Ia239.180, die sich von den entsprechenden Verbindungen Ia1.001-Ia1.180 dadurch unterscheiden, daß R¹ für Methylsulfonyl, R¹⁴ für n-Butyl und R¹⁵ für Methyl stehen: 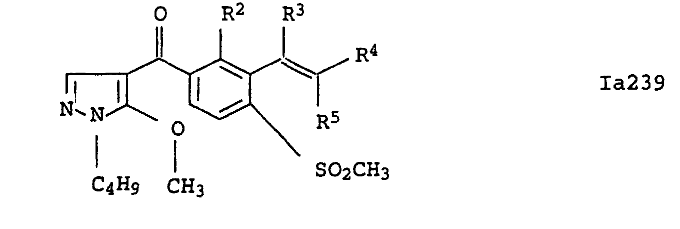
- Ebenso außergewöhnlich bevorzugt sind die Verbindungen Ia240, insbesondere die Verbindungen Ia240.001-Ia240.180, die sich von den entsprechenden Verbindungen Ia1.001-Ia1.180 dadurch unterscheiden, daß R¹ für Methylsulfonyl, R¹⁴ für iso-Butyl und R¹⁵ für Methyl stehen: 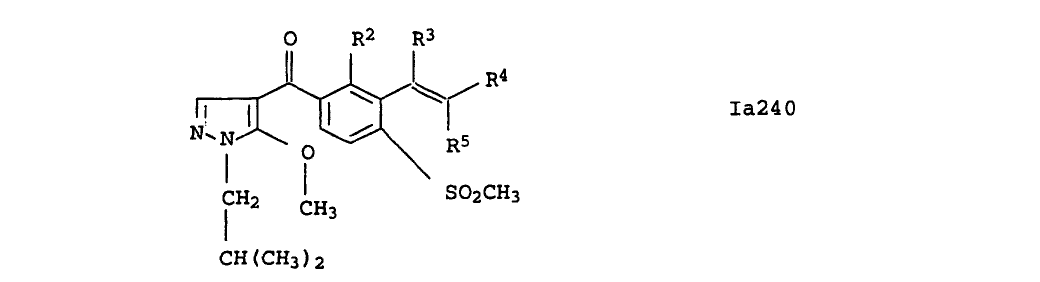
- Ebenso außergewöhnlich bevorzugt sind die Verbindungen Ia241, insbesondere die Verbindungen Ia241.001-Ia241.180, die sich von den entsprechenden Verbindungen Ia1.001-Ia1.180 dadurch unterscheiden, daß R¹ für Methylsulfonyl und R¹⁵ für Ethyl stehen: 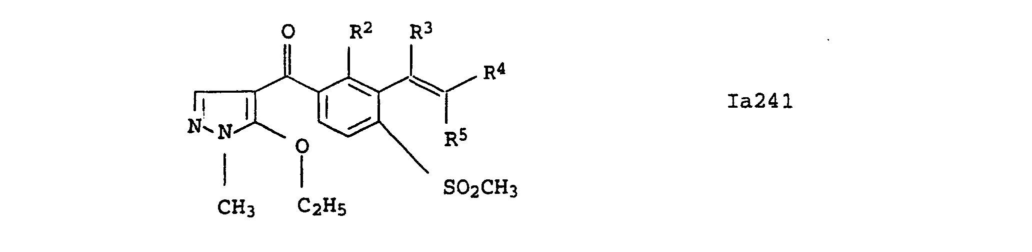
- Ebenso außergewöhnlich bevorzugt sind die Verbindungen Ia242, insbesondere die Verbindungen Ia242.001-Ia242.180, die sich von den entsprechenden Verbindungen Ia1.001-Ia1.180 dadurch unterscheiden, daß R¹ für Methylsulfonyl, R¹⁴ und R¹⁵ für Ethyl stehen: 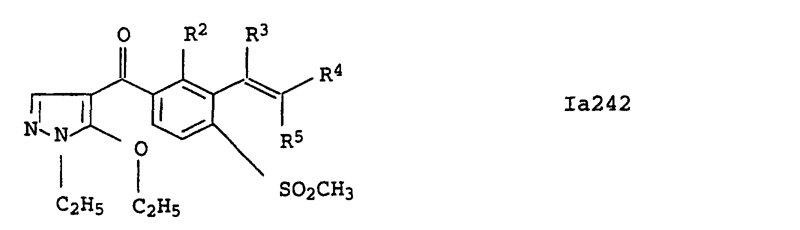
- Ebenso außergewöhnlich bevorzugt sind die Verbindungen Ia243, insbesondere die Verbindungen Ia243.001-Ia243.180, die sich von den entsprechenden Verbindungen Ia1.001-Ia1.180 dadurch unterscheiden, daß R¹ für Methylsulfonyl, R¹⁴ für n-Propyl und R¹⁵ für Ethyl stehen: 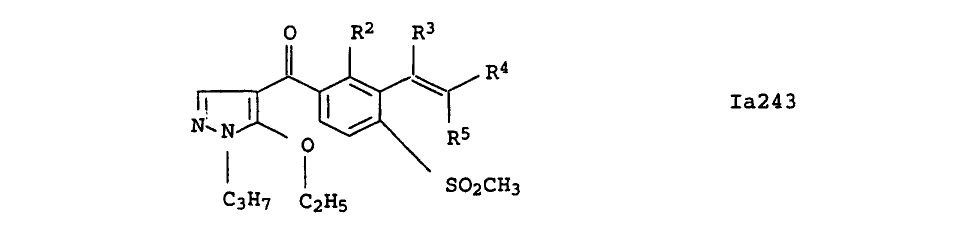
- Ebenso außergewöhnlich bevorzugt sind die Verbindungen Ia244, insbesondere die Verbindungen Ia244.001-Ia244.180, die sich von den entsprechenden Verbindungen Ia1.001-Ia1.180 dadurch unterscheiden, daß R¹ für Methylsulfonyl, R¹⁴ für n-Butyl und R¹⁵ für Ethyl stehen: 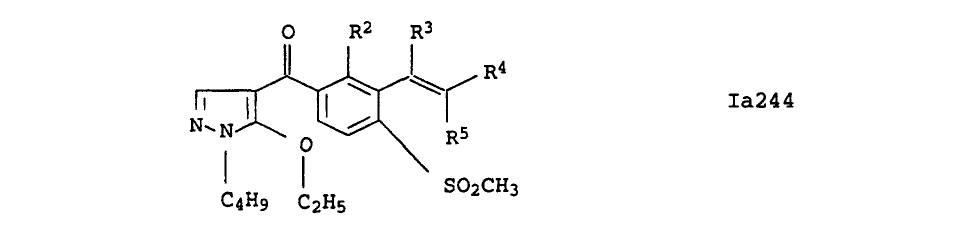
- Ebenso außergewöhnlich bevorzugt sind die Verbindungen Ia245, insbesondere die Verbindungen Ia245.001-Ia245.180, die sich von den entsprechenden Verbindungen Ia1.001-Ia1.180 dadurch unterscheiden, daß R¹ für Methylsulfonyl, R¹⁴ für iso-Butyl und R¹⁵ für Ethyl stehen: 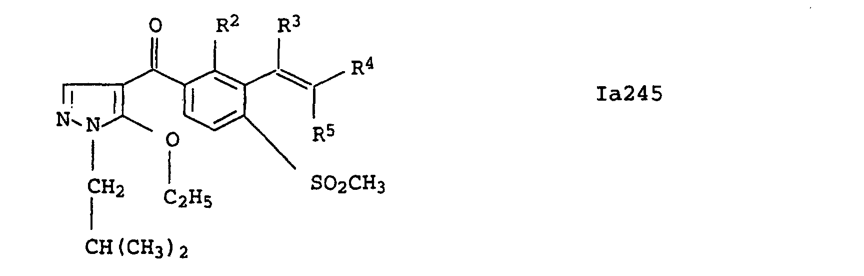
- Ebenso außergewöhnlich bevorzugt sind die Verbindungen Ia246, insbesondere die Verbindungen Ia246.001-Ia246.180, die sich von den entsprechenden Verbindungen Ia1.001-Ia1.180 dadurch unterscheiden, daß R¹ für Methylsulfonyl und R¹⁵ für n-Propyl stehen: 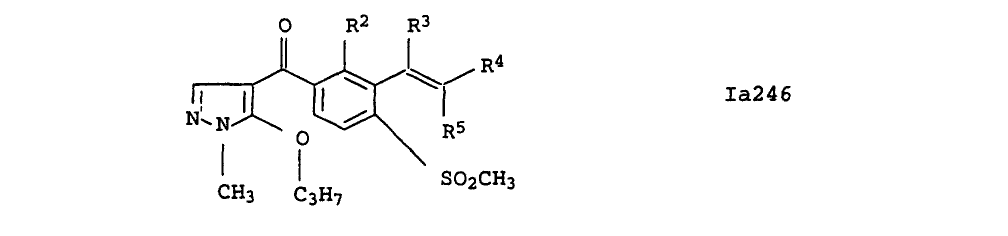
- Ebenso außergewöhnlich bevorzugt sind die Verbindungen Ia247, insbesondere die Verbindungen Ia247.001-Ia247.180, die sich von den entsprechenden Verbindungen Ia1.001-Ia1.180 dadurch unterscheiden, daß R¹ für Methylsulfonyl, R¹⁴ für Ethyl und R¹⁵ für n-Propyl stehen: 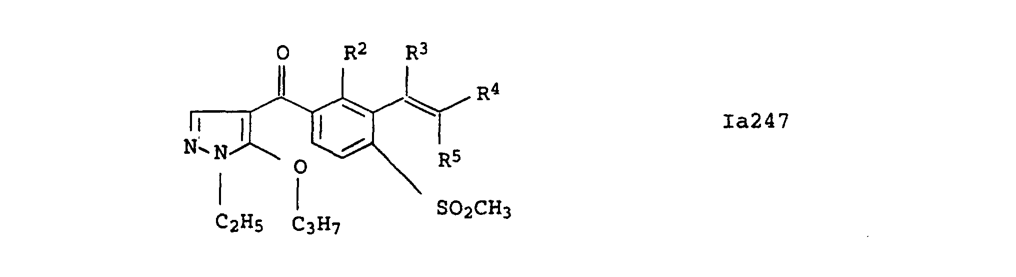
- Ebenso außergewöhnlich bevorzugt sind die Verbindungen Ia248, insbesondere die Verbindungen Ia248.001-Ia248.180, die sich von den entsprechenden Verbindungen Ia1.001-Ia1.180 dadurch unterscheiden, daß R¹ für Methylsulfonyl, R¹⁴ und R¹⁵ für n-Propyl stehen: 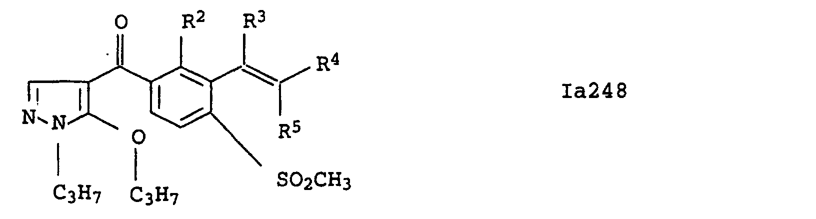
- Ebenso außergewöhnlich bevorzugt sind die Verbindungen Ia249, insbesondere die Verbindungen Ia249.001-Ia249.180, die sich von den entsprechenden Verbindungen Ia1.001-Ia1.180 dadurch unterscheiden, daß R¹ für Methylsulfonyl, R¹⁴ für n-Butyl und R¹⁵ für n-Propyl stehen: 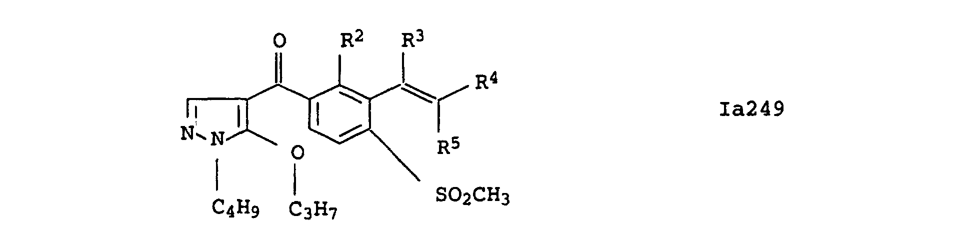
- Ebenso außergewöhnlich bevorzugt sind die Verbindungen Ia250, insbesondere die Verbindungen Ia250.001-Ia250.180, die sich von den entsprechenden Verbindungen Ia1.001-Ia1.180 dadurch unterscheiden, daß R¹ für Methylsulfonyl, R¹⁴ für iso-Butyl und R¹⁵ für n-Propyl stehen: 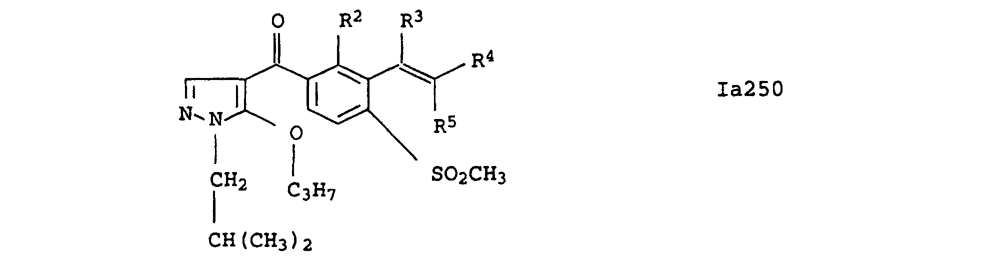
- Ebenso außergewöhnlich bevorzugt sind die Verbindungen Ia251, insbesondere die Verbindungen Ia251.001-Ia251.180, die sich von den entsprechenden Verbindungen Ia1.001-Ia1.180 dadurch unterscheiden, daß R¹ für Methylsulfonyl und R¹⁵ für iso-Propyl stehen: 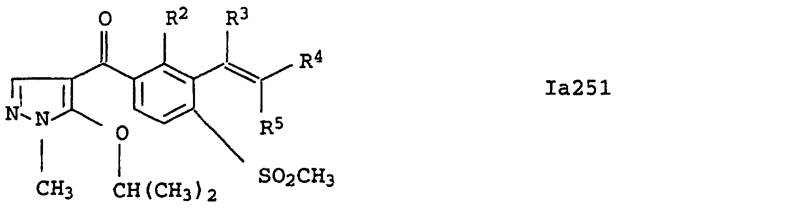
- Ebenso außergewöhnlich bevorzugt sind die Verbindungen Ia252, insbesondere die Verbindungen Ia252.001-Ia252.180, die sich von den entsprechenden Verbindungen Ia1.001-Ia1.180 dadurch unterscheiden, daß R¹ für Methylsulfonyl, R¹⁴ für Ethyl und R¹⁵ für iso-Propyl stehen: 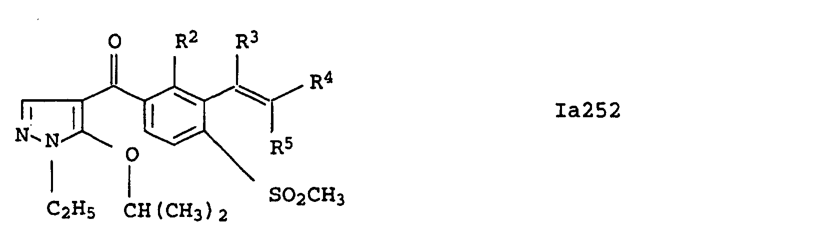
- Ebenso außergewöhnlich bevorzugt sind die Verbindungen Ia253, insbesondere die Verbindungen Ia253.001-Ia253.180, die sich von den entsprechenden Verbindungen Ia1.001-Ia1.180 dadurch unterscheiden, daß R¹ für Methylsulfonyl, R¹⁴ für n-Propyl und R¹⁵ für iso-Propyl stehen: 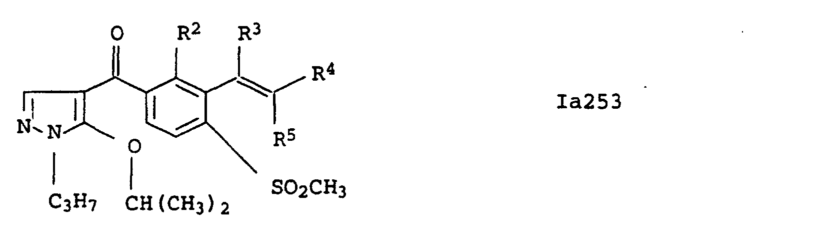
- Ebenso außergewöhnlich bevorzugt sind die Verbindungen Ia254, insbesondere die Verbindungen Ia254.001-Ia254.180, die sich von den entsprechenden Verbindungen Ia1.001-Ia1.180 dadurch unterscheiden, daß R¹ für Methylsulfonyl, R¹⁴ für n-Butyl und R¹⁵ für iso-Propyl stehen: 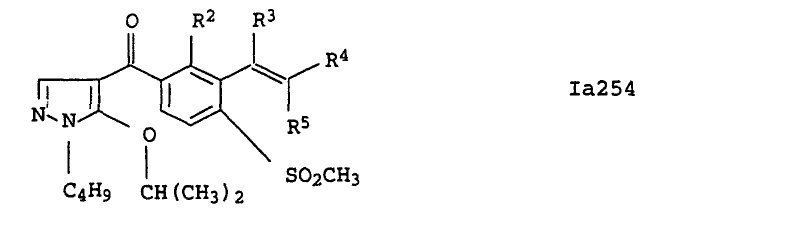
- Ebenso außergewöhnlich bevorzugt sind die Verbindungen Ia255, insbesondere die Verbindungen Ia255.001-Ia255.180, die sich von den entsprechenden Verbindungen Ia1.001-Ia1.180 dadurch unterscheiden, daß R¹ für Methylsulfonyl, R¹⁴ für iso-Butyl und R¹⁵ für iso-Propyl stehen: 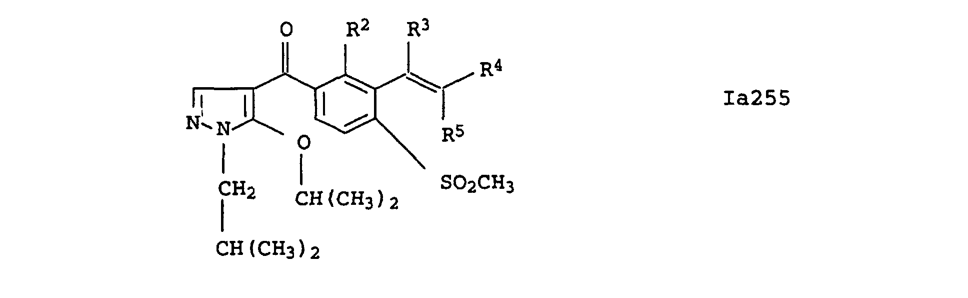
- Ebenso außergewöhnlich bevorzugt sind die Verbindungen Ia256, insbesondere die Verbindungen Ia256.001-Ia256.180, die sich von den entsprechenden Verbindungen Ia1.001-Ia1.180 dadurch unterscheiden, daß R¹ für Methylsulfonyl und R¹⁵ für n-Butyl stehen: 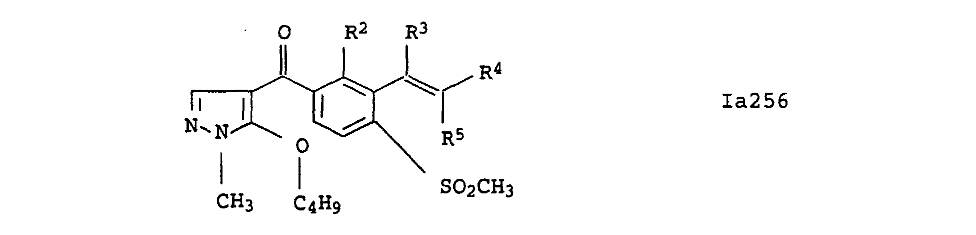
- Ebenso außergewöhnlich bevorzugt sind die Verbindungen Ia257, insbesondere die Verbindungen Ia257.001-Ia257.180, die sich von den entsprechenden Verbindungen Ia1.001-Ia1.180 dadurch unterscheiden, daß R¹ für Methylsulfonyl, R¹⁴ für Ethyl und R¹⁵ für n-Butyl stehen: 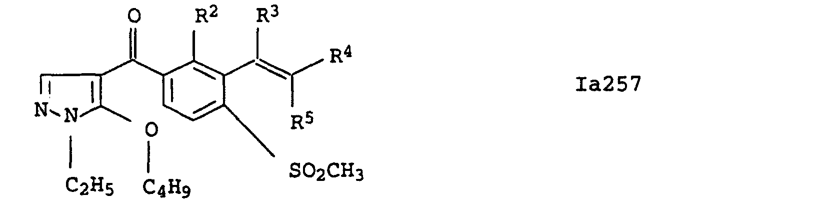
- Ebenso außergewöhnlich bevorzugt sind die Verbindungen Ia258, insbesondere die Verbindungen Ia258.001-Ia258.180, die sich von den entsprechenden Verbindungen Ia1.001-Ia1.180 dadurch unterscheiden, daß R¹ für Methylsulfonyl, R¹⁴ für n-Propyl und R¹⁵ für n-Butyl stehen: 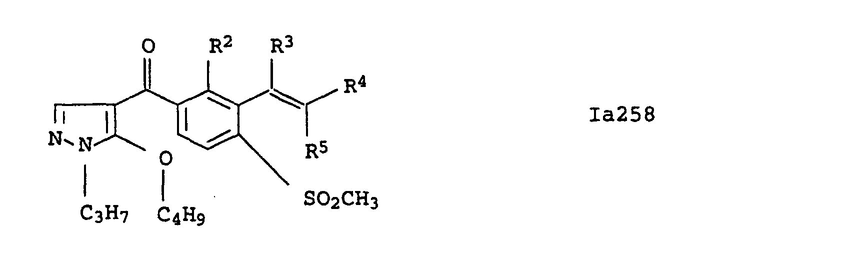
- Ebenso außergewöhnlich bevorzugt sind die Verbindungen Ia259, insbesondere die Verbindungen Ia259.001-Ia259.180, die sich von den entsprechenden Verbindungen Ia1.001-Ia1.180 dadurch unterscheiden, daß R¹ für Methylsulfonyl, R¹⁴ und R¹⁵ für n-Butyl stehen: 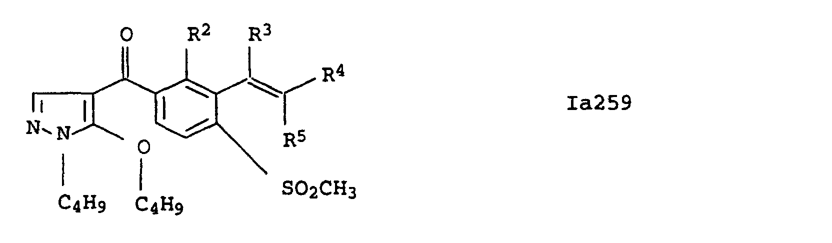
- Ebenso außergewöhnlich bevorzugt sind die Verbindungen Ia260, insbesondere die Verbindungen Ia260.001-Ia260.180, die sich von den entsprechenden Verbindungen Ia1.001-Ia1.180 dadurch unterscheiden, daß R¹ für Methylsulfonyl, R¹⁴ für iso-Butyl und R¹⁵ für n-Butyl stehen: 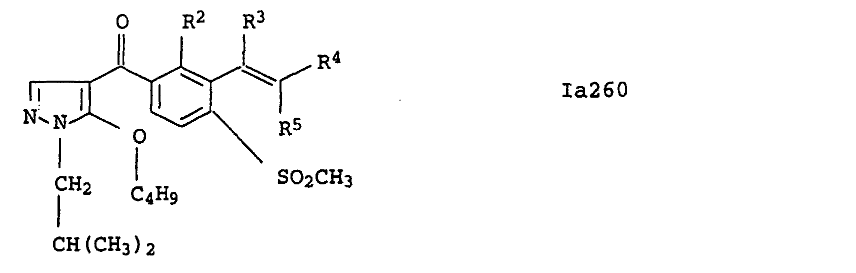
- Ebenso außergewöhnlich bevorzugt sind die Verbindungen Ia261, insbesondere die Verbindungen Ia261.001-Ia261.180, die sich von den entsprechenden Verbindungen Ia1.001-Ia1.180 dadurch unterscheiden, daß R¹ für Methylsulfonyl und R¹⁵ für sec-Butyl stehen: 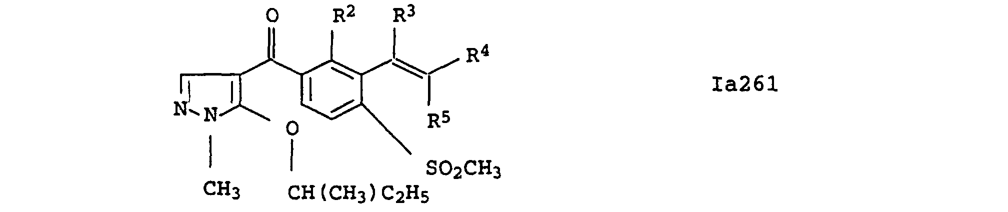 Ebenso außergewöhnlich bevorzugt sind die Verbindungen Ia262, insbesondere die Verbindungen Ia262.001-Ia262.180, die sich von den entsprechenden Verbindungen Ia1.001-Ia1.180 dadurch unterscheiden, daß R¹ für Methylsulfonyl, R¹⁴ für Ethyl und R¹⁵ für sec-Butyl stehen: 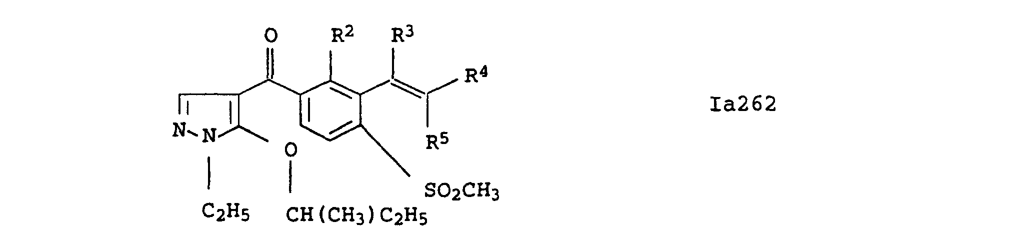
- Ebenso außergewöhnlich bevorzugt sind die Verbindungen Ia263, insbesondere die Verbindungen Ia263.001-Ia263.180, die sich von den entsprechenden Verbindungen Ia1.001-Ia1.180 dadurch unterscheiden, daß R¹ für Methylsulfonyl, R¹⁴ für n-Propyl und R¹⁵ für sec-Butyl stehen: 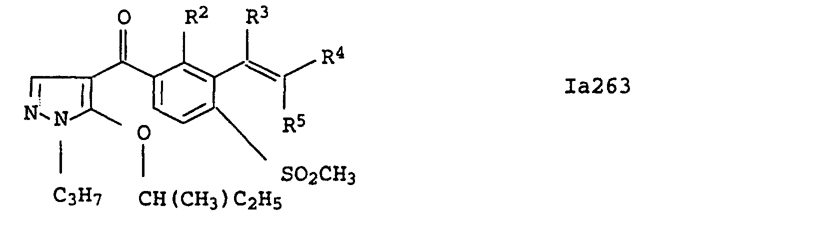
- Ebenso außergewöhnlich bevorzugt sind die Verbindungen Ia264, insbesondere die Verbindungen Ia264.001-Ia264.180, die sich von den entsprechenden Verbindungen Ia1.001-Ia1.180 dadurch unterscheiden, daß R¹ für Methylsulfony, R¹⁴ für n-Butyl und R¹⁵ für sec-Butyl stehen: 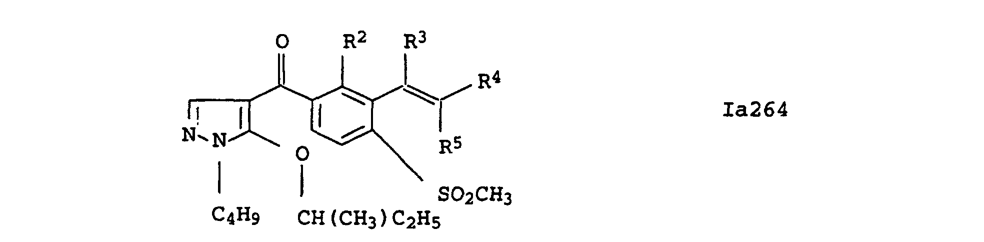
- Ebenso außergewöhnlich bevorzugt sind die Verbindungen Ia265, insbesondere die Verbindungen Ia265.001-Ia265.180, die sich von den entsprechenden Verbindungen Ia1.001-Ia1.180 dadurch unterscheiden, daß R¹ für Methylsulfonyl, R¹⁴ für iso-Butyl und R¹⁵ für sec-Butyl stehen: 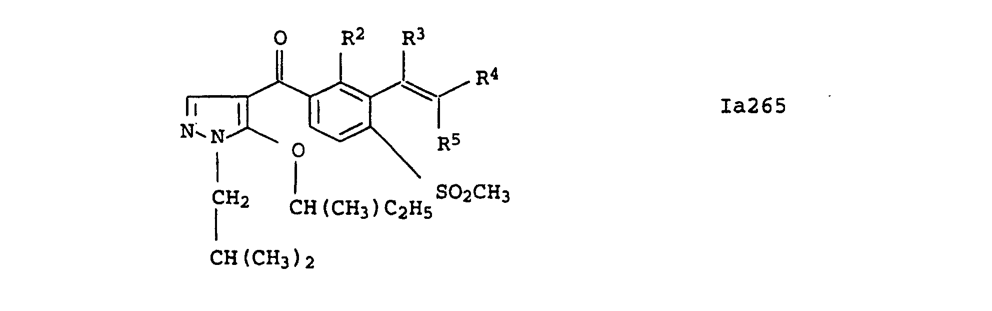
- Ebenso außergewöhnlich bevorzugt sind die Verbindungen Ia266, insbesondere die Verbindungen Ia266.001-Ia266.180, die sich von den entsprechenden Verbindungen Ia1.001-Ia1.180 dadurch unterscheiden, daß R¹ für Methylsulfonyl und R¹⁵ für iso-Butyl stehen: 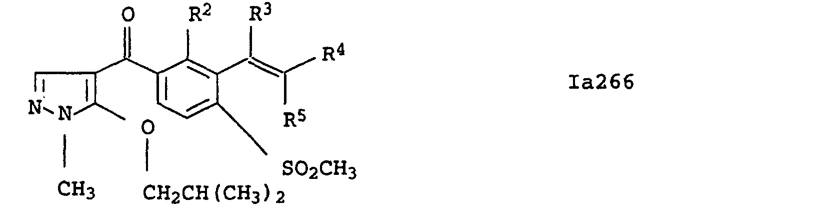
- Ebenso außergewöhnlich bevorzugt sind die Verbindungen Ia267, insbesondere die Verbindungen Ia267.001-Ia267.180, die sich von den entsprechenden Verbindungen Ia1.001-Ia1.180 dadurch unterscheiden, daß R¹ für Methylsulfonyl, R¹⁴ für Ethyl und R¹⁵ für iso-Butyl stehen: 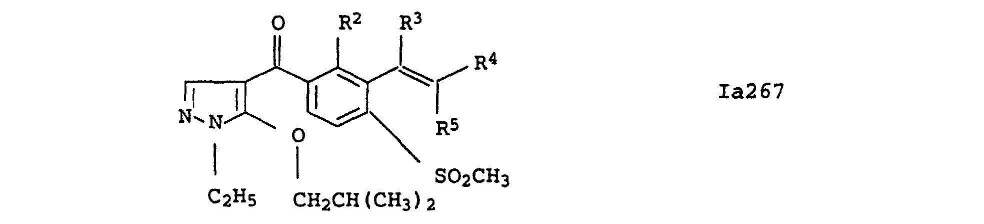
- Ebenso außergewöhnlich bevorzugt sind die Verbindungen Ia268, insbesondere die Verbindungen Ia268.001-Ia268.180, die sich von den entsprechenden Verbindungen Ia1.001-Ia1.180 dadurch unterscheiden, daß R¹ für Methylsulfonyl, R¹⁴ für n-Propyl und R¹⁵ für iso-Butyl stehen: 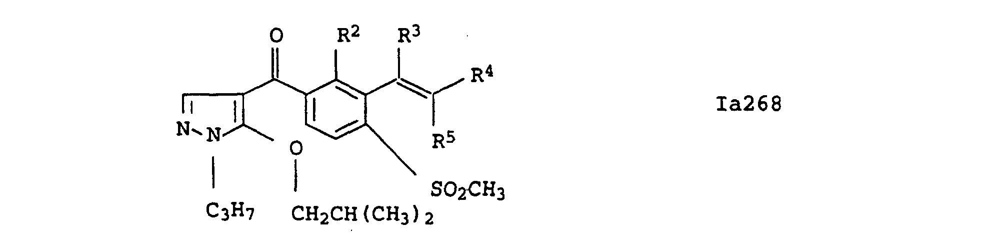
- Ebenso außergewöhnlich bevorzugt sind die Verbindungen Ia269, insbesondere die Verbindungen Ia269.001-Ia269.180, die sich von den entsprechenden Verbindungen Ia1.001-Ia1.180 dadurch unterscheiden, daß R¹ für Methylsulfonyl, R¹⁴ für n-Butyl und R¹⁵ für iso-Butyl stehen: 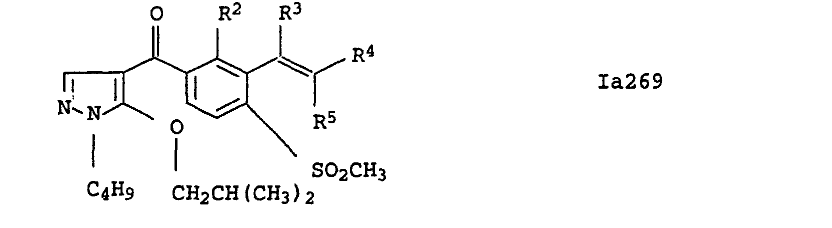
- Ebenso außergewöhnlich bevorzugt sind die Verbindungen Ia270, insbesondere die Verbindungen Ia270.001-Ia270.180, die sich von den entsprechenden Verbindungen Ia1.001-Ia1.180 dadurch unterscheiden, daß R¹ für Methylsulfonyl, R¹⁴ und R¹⁵ für iso-Butyl stehen: 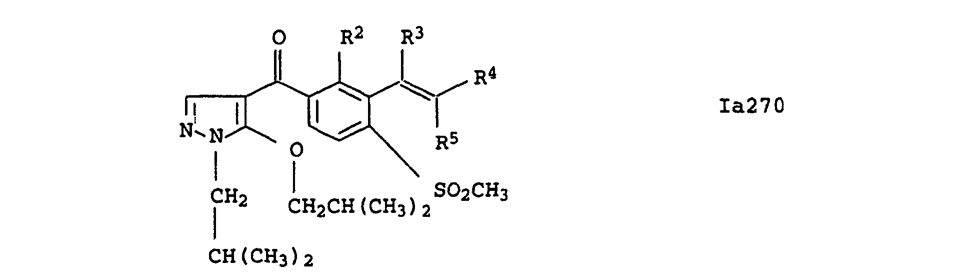
- Ebenso außergewöhnlich bevorzugt sind die Verbindungen Ia271, insbesondere die Verbindungen Ia271.001-Ia271.180, die sich von den entsprechenden Verbindungen Ia1.001-Ia1.180 dadurch unterscheiden, daß R¹ für Methylsulfonyl und R¹⁵ für Methylcarbonyl stehen: 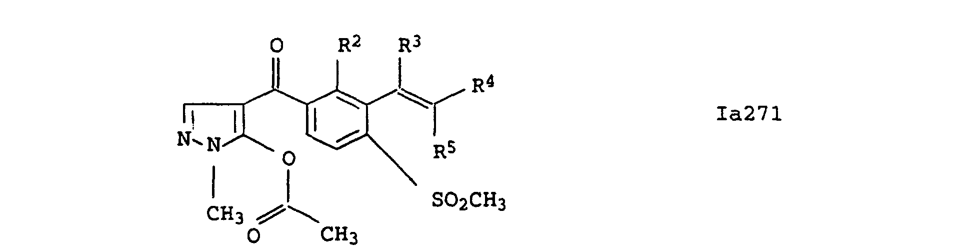
- Ebenso außergewöhnlich bevorzugt sind die Verbindungen Ia272, insbesondere die Verbindungen Ia272.001-Ia272.180, die sich von den entsprechenden Verbindungen Ia1.001-Ia1.180 dadurch unterscheiden, daß R¹ für Methylsulfonyl, R¹⁴ für Ethyl und R¹⁵ für Methylcarbonyl stehen: 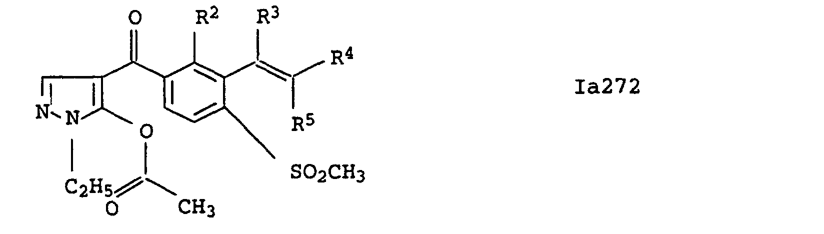
- Ebenso außergewöhnlich bevorzugt sind die Verbindungen Ia273, insbesondere die Verbindungen Ia273.001-Ia273.180, die sich von den entsprechenden Verbindungen Ia1.001-Ia1.180 dadurch unterscheiden, daß R¹ für Methylsulfonyl, R¹⁴ für n-Propyl und R¹⁵ für Methylcarbonyl stehen: 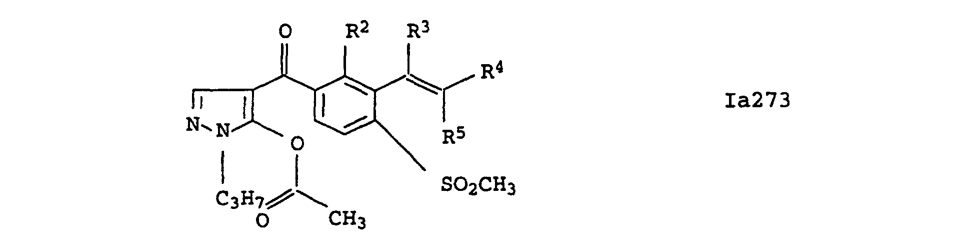
- Ebenso außergewöhnlich bevorzugt sind die Verbindungen Ia274, insbesondere die Verbindungen Ia274.001-Ia274.180, die sich von den entsprechenden Verbindungen Ia1.001-Ia1.180 dadurch unterscheiden, daß R¹ für Methylsulfonyl, R¹⁴ für n-Butyl und R¹⁵ für Methylcarbonyl stehen: 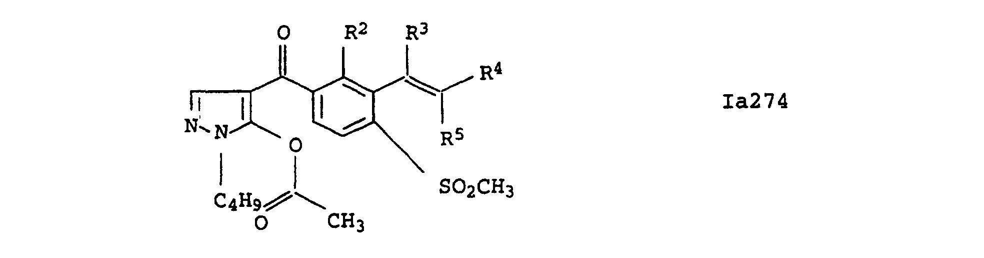
- Ebenso außergewöhnlich bevorzugt sind die Verbindungen Ia275, insbesondere die Verbindungen Ia275.001-Ia275.180, die sich von den entsprechenden Verbindungen Ia1.001-Ia1.180 dadurch unterscheiden, daß R¹ für Methylsulfonyl, R¹⁴ für iso-Butyl und R¹⁵ für Methylcarbonyl stehen: 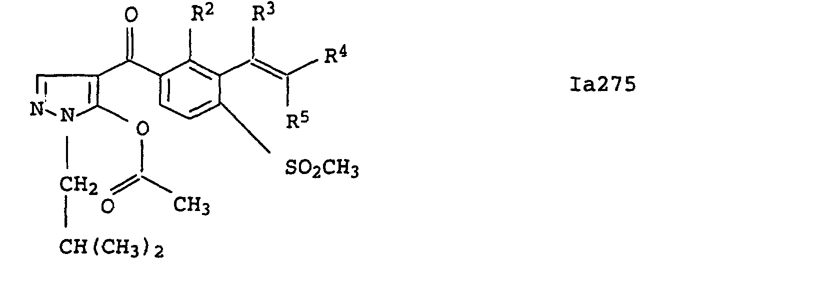
- Ebenso außergewöhnlich bevorzugt sind die Verbindungen Ia276, insbesondere die Verbindungen Ia276.001-Ia276.180, die sich von den entsprechenden Verbindungen Ia1.001-Ia1.180 dadurch unterscheiden, daß R¹ für Methylsulfonyl und R¹⁵ für Ethylcarbonyl stehen: 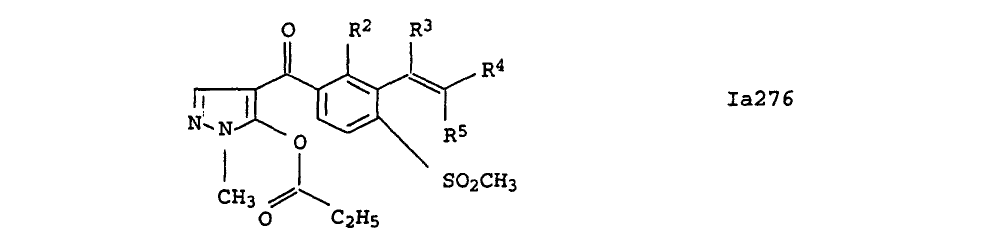
- Ebenso außergewöhnlich bevorzugt sind die Verbindungen Ia277, insbesondere die Verbindungen Ia277.001-Ia277.180, die sich von den entsprechenden Verbindungen Ia1.001-Ia1.180 dadurch unterscheiden, daß R¹ für Methylsulfonyl, R¹⁴ für Ethyl und R¹⁵ für Ethylcarbonyl stehen: 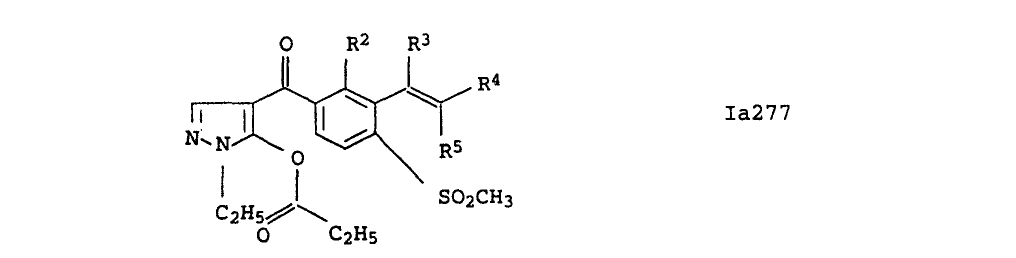
- Ebenso außergewöhnlich bevorzugt sind die Verbindungen Ia278, insbesondere die Verbindungen Ia278.001-Ia278.180, die sich von den entsprechenden Verbindungen Ia1.001-Ia1.180 dadurch unterscheiden, daß R¹ für Methylsulfonyl, R¹⁴ für n-Propyl und R¹⁵ für Ethylcarbonyl stehen: 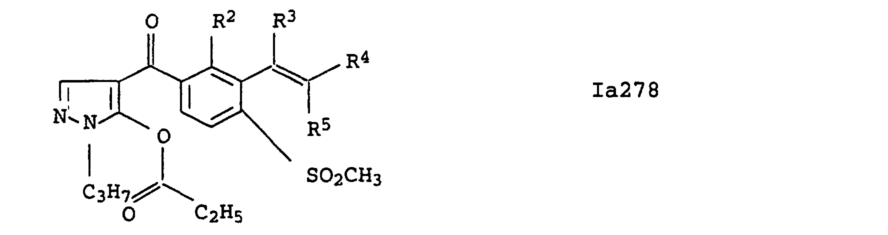
- Ebenso außergewöhnlich bevorzugt sind die Verbindungen Ia279, insbesondere die Verbindungen Ia279.001-Ia279.180, die sich von den entsprechenden Verbindungen Ia1.001-Ia1.180 dadurch unterscheiden, daß R¹ für Methylsulfonyl, R¹⁴ für n-Butyl und R¹⁵ für Ethylcarbonyl stehen: 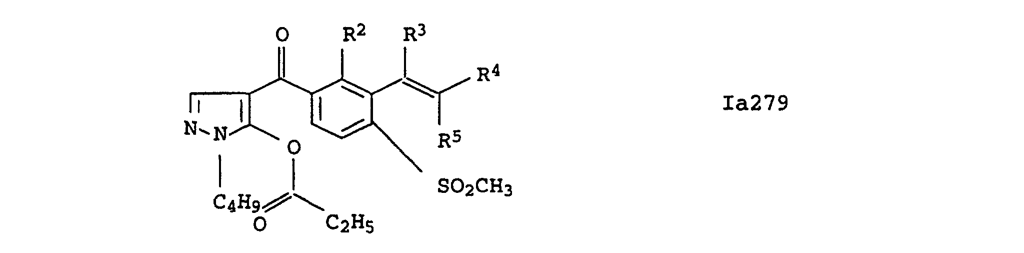
- Ebenso außergewöhnlich bevorzugt sind die Verbindungen Ia280, insbesondere die Verbindungen Ia280.001-Ia280.180, die sich von den entsprechenden Verbindungen Ia1.001-Ia1.180 dadurch unterscheiden, daß R¹ für Methylsulfonyl, R¹⁴ für iso-Butyl und R¹⁵ für Ethylcarbonyl stehen: 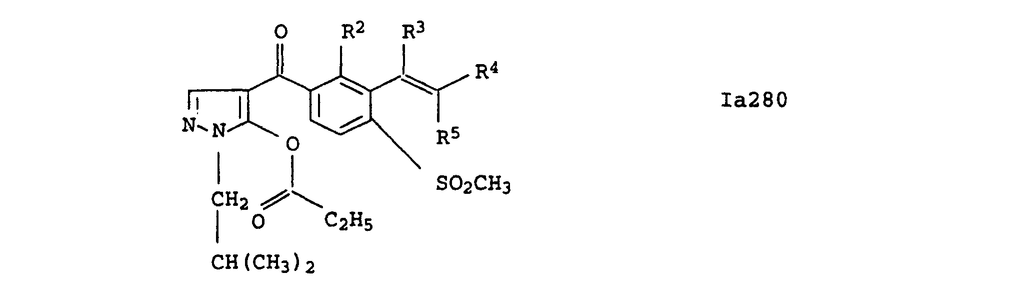
- Ebenso außergewöhnlich bevorzugt sind die Verbindungen Ia281, insbesondere die Verbindungen Ia281.001-Ia281.180, die sich von den entsprechenden Verbindungen Ia1.001-Ia1.180 dadurch unterscheiden, daß R¹ für Methylsulfonyl und R¹⁵ für n-Propylcarbonyl stehen: 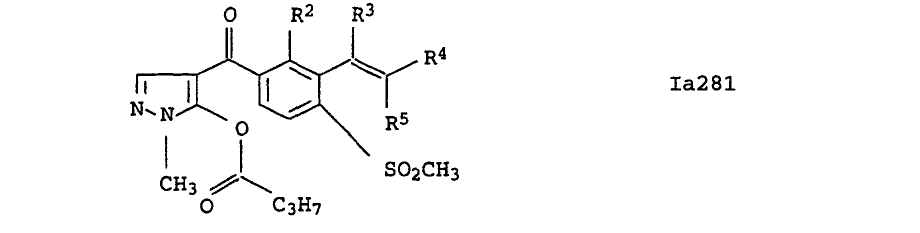
- Ebenso außergewöhnlich bevorzugt sind die Verbindungen Ia282, insbesondere die Verbindungen Ia282.001-Ia282.180, die sich von den entsprechenden Verbindungen Ia1.001-Ia1.180 dadurch unterscheiden, daß R¹ für Methylsulfonyl, R¹⁴ für Ethyl und R¹⁵ für n-Propylcarbonyl stehen: 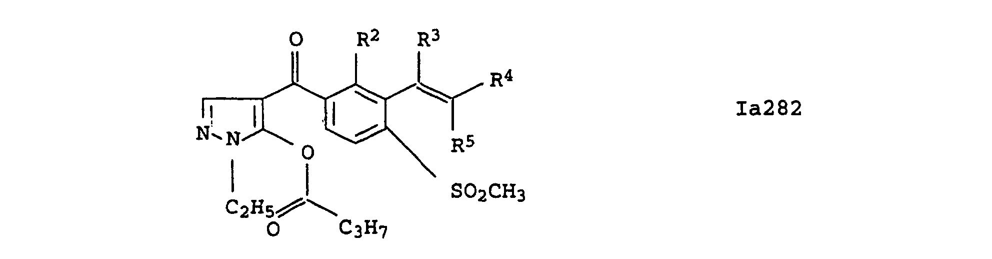
- Ebenso außergewöhnlich bevorzugt sind die Verbindungen Ia283, insbesondere die Verbindungen Ia283.001-Ia283.180, die sich von den entsprechenden Verbindungen Ia1.001-Ia1.180 dadurch unterscheiden, daß R¹ für Methylsulfonyl, R¹⁴ für n-Propyl und R¹⁵ für n-Propylcarbonyl stehen: 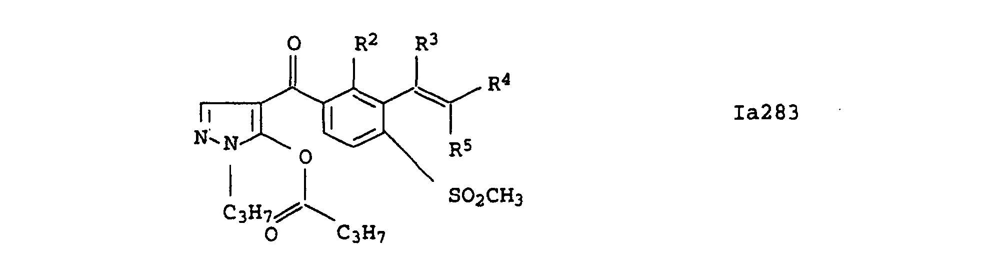
- Ebenso außergewöhnlich bevorzugt sind die Verbindungen Ia284, insbesondere die Verbindungen Ia284.001-Ia284.180, die sich von den entsprechenden Verbindungen Ia1.001-Ia1.I80 dadurch unterscheiden, daß R¹ für Methylsulfonyl, R¹⁴ für n-Butyl und R¹⁵ für n-Propylcarbonyl stehen: 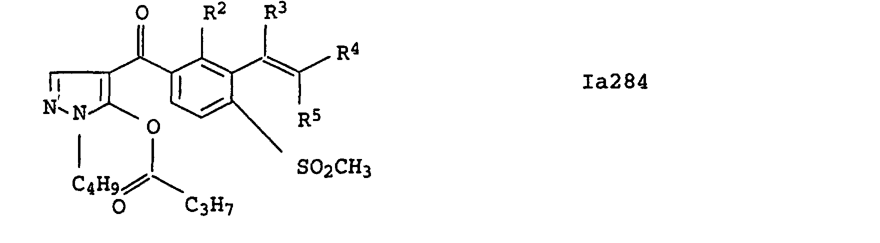
- Ebenso außergewöhnlich bevorzugt sind die Verbindungen Ia285, insbesondere die Verbindungen Ia285.001-Ia285.180, die sich von den entsprechenden Verbindungen Ia1.001-Ia1.180 dadurch unterscheiden, daß R¹ für Methylsulfonyl, R¹⁴ für iso-Butyl und R¹⁵ für n-Propylcarbonyl stehen: 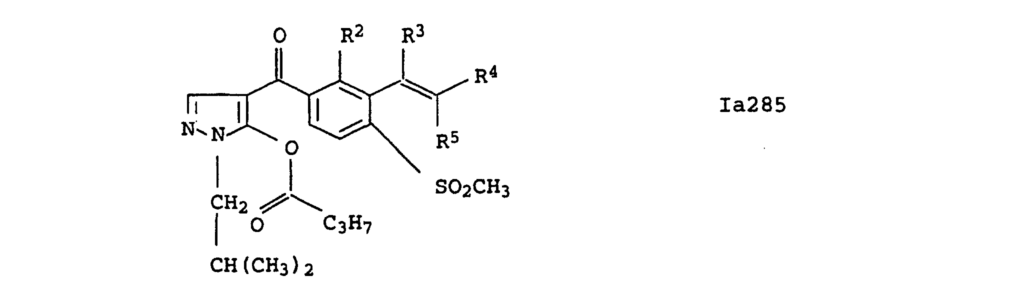
- Ebenso außergewöhnlich bevorzugt sind die Verbindungen Ia286, insbesondere die Verbindungen Ia286.001-Ia286.180, die sich von den entsprechenden Verbindungen Ia1.001-Ia1.180 dadurch unterscheiden, daß R¹ für Methylsulfonyl und R¹⁴ für Trifluormethylcarbonyl stehen: 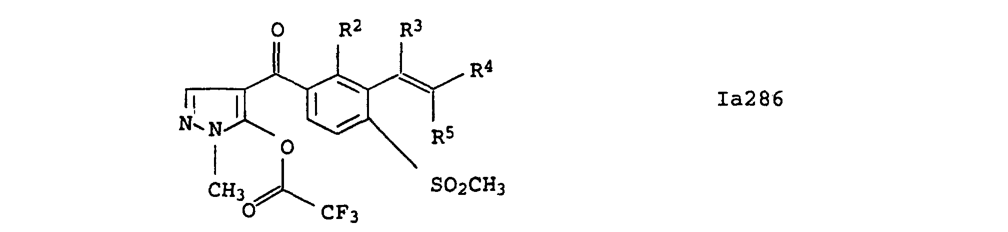
- Ebenso außergewöhnlich bevorzugt sind die Verbindungen Ia287, insbesondere die Verbindungen Ia287.001-Ia287.180, die sich von den entsprechenden Verbindungen Ia1.001-Ia1.180 dadurch unterscheiden, daß R¹ für Methylsulfonyl, R¹⁴ für Ethyl und R¹⁵ für Trifluormethylcarbonyl stehen: 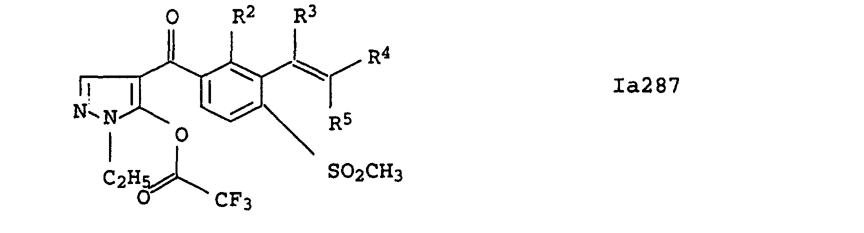
- Ebenso außergewöhnlich bevorzugt sind die Verbindungen Ia288, insbesondere die Verbindungen Ia288.001-Ia288.180, die sich von den entsprechenden Verbindungen Ia1.001-Ia1.180 dadurch unterscheiden, daß R¹ für Methylsulfonyl, R¹⁴ für n-Propyl und R¹⁵ für Trifluormethylcarbonyl stehen: 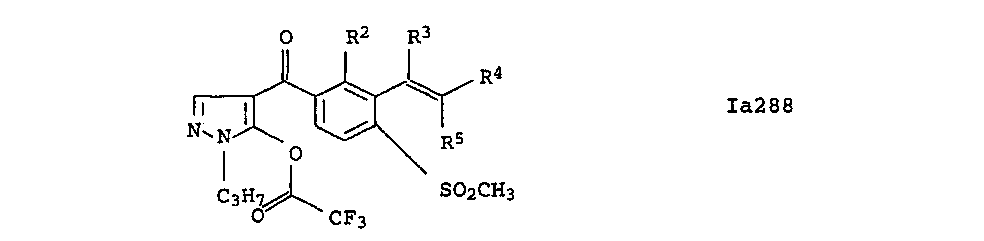
- Ebenso außergewöhnlich bevorzugt sind die Verbindungen Ia289, insbesondere die Verbindungen Ia289.001-Ia289.180, die sich von den entsprechenden Verbindungen Ia1.001-Ia1.180 dadurch unterscheiden, daß R¹ für Methylsulfonyl, R¹⁴ für n-Butyl und R¹⁵ für Trifluormethylcarbonyl stehen: 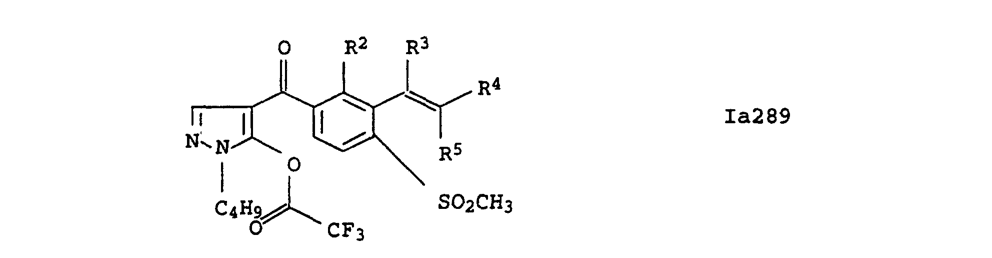
- Ebenso außergewöhnlich bevorzugt sind die Verbindungen Ia290, insbesondere die Verbindungen Ia290.001-Ia290.180, die sich von den entsprechenden Verbindungen Ia1.001-Ia1.180 dadurch unterscheiden, daß R¹ für Methylsulfonyl, R¹⁴ für iso-Butyl und R¹⁵ für Trifluormethylcarbonyl stehen: 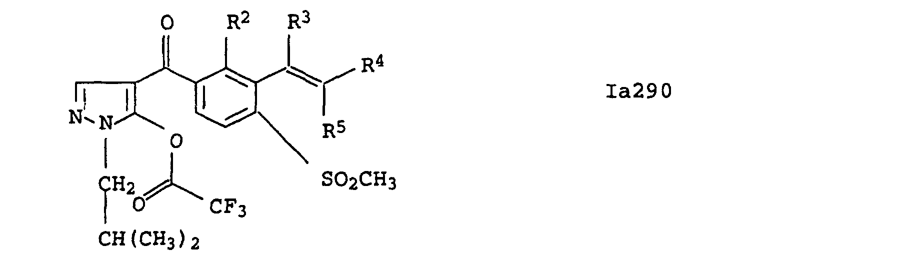
- Ebenso außergewöhnlich bevorzugt sind die Verbindungen Ia291, insbesondere die Verbindungen Ia291.001-Ia291.180, die sich von den entsprechenden Verbindungen Ia1.001-Ia1.180 dadurch unterscheiden, daß R¹ und R¹⁵ für Methylsulfonyl stehen: 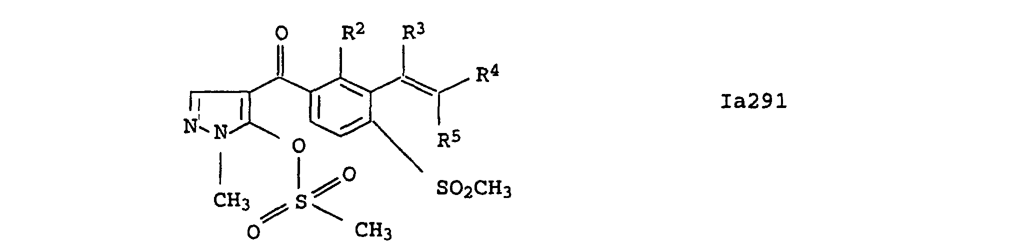
- Ebenso außergewöhnlich bevorzugt sind die Verbindungen Ia292, insbesondere die Verbindungen Ia292.001-Ia292.180, die sich von den entsprechenden Verbindungen Ia1.001-Ia1.180 dadurch unterscheiden, daß R¹ und R¹⁵ für Methylsulfonyl und R¹⁴ für Ethyl stehen: 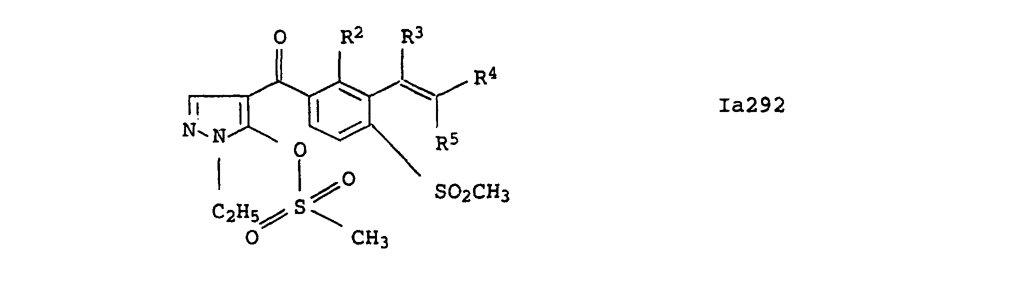
- Ebenso außergewöhnlich bevorzugt sind die Verbindungen Ia293, insbesondere die Verbindungen Ia293.001-Ia293.180, die sich von den entsprechenden Verbindungen Ia1.001-Ia1.180 dadurch unterscheiden, daß R¹ und R¹⁵ für Methylsulfonyl und R¹⁴ für n-Propyl stehen stehen: 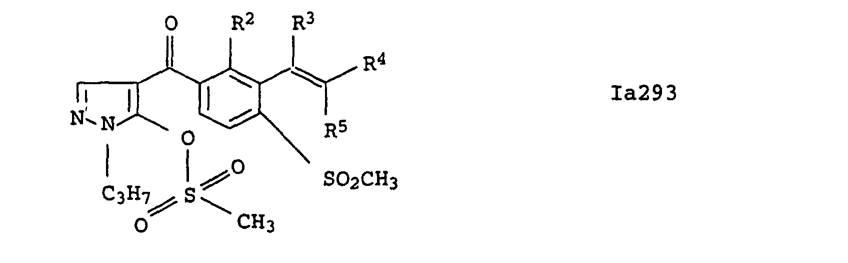
- Ebenso außergewöhnlich bevorzugt sind die Verbindungen Ia294, insbesondere die Verbindungen Ia294.001-Ia294.180, die sich von den entsprechenden Verbindungen Ia1.001-Ia1.180 dadurch unterscheiden, daß R¹ und R¹⁵ für Methylsulfonyl und R¹⁴ für n-Butyl stehen: 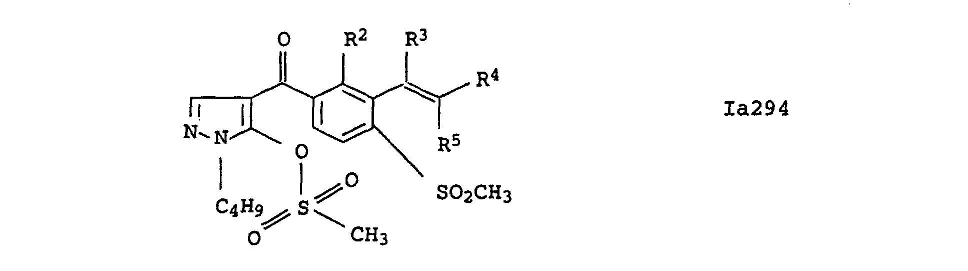 Ebenso außergewöhnlich bevorzugt sind die Verbindungen Ia295, insbesondere die Verbindungen Ia295.001-Ia295.180, die sich von den entsprechenden Verbindungen Ia1.001-Ia1.180 dadurch unterscheiden, daß R¹ und R¹⁵ für Methylsulfonyl und R¹⁴ für iso-Butyl stehen: 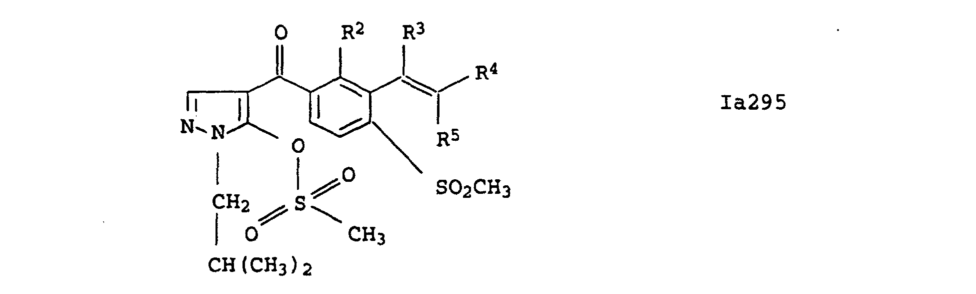
- Ebenso außergewöhnlich bevorzugt sind die Verbindungen Ia296, insbesondere die Verbindungen Ia296.001-Ia296.180, die sich von den entsprechenden Verbindungen Ia1.001-Ia1.180 dadurch unterscheiden, daß R¹ für Methylsulfonyl und R¹⁵ für Ethylsulfonyl stehen: 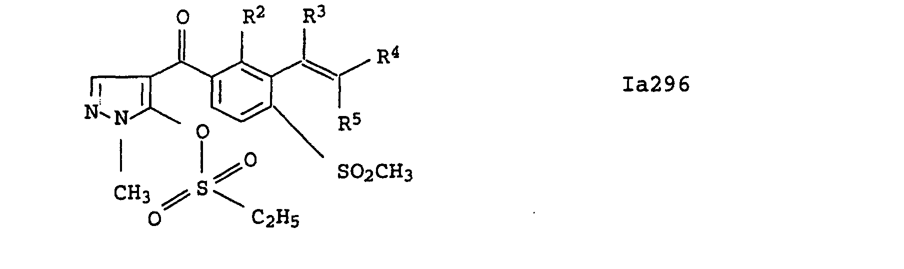
- Ebenso außergewöhnlich bevorzugt sind die Verbindungen Ia297, insbesondere die Verbindungen Ia297.001-Ia297.180, die sich von den entsprechenden Verbindungen Ia1.001-Ia1.180 dadurch unterscheiden, daß R¹ für Methylsulfonyl, R¹⁴ für Ethyl und R¹⁵ für Ethylsulfonyl stehen: 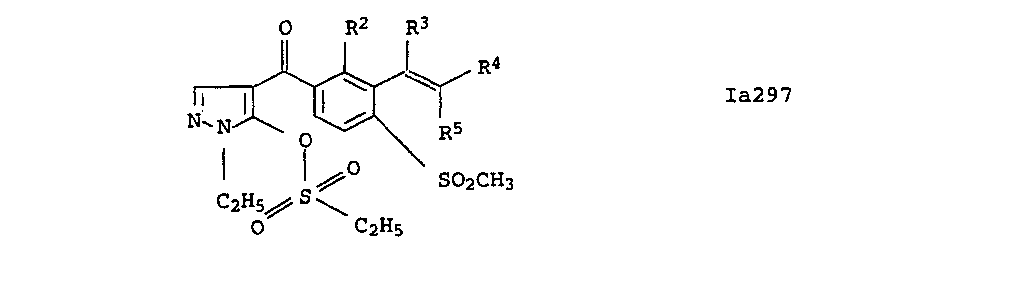
- Ebenso außergewöhnlich bevorzugt sind die Verbindungen Ia298, insbesondere die Verbindungen Ia298.001-Ia298.180, die sich von den entsprechenden Verbindungen Ia1.001-Ia1.180 dadurch unterscheiden, daß R¹ für Methylsulfonyl, R¹⁴ für n-Propyl und R¹⁵ für Ethylsulfonyl stehen: 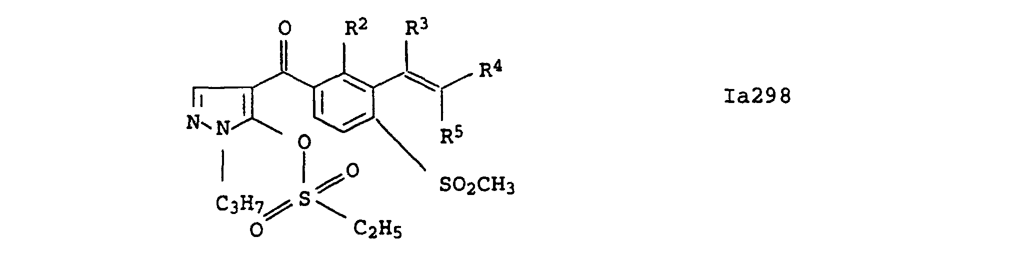
- Ebenso außergewöhnlich bevorzugt sind die Verbindungen Ia299, insbesondere die Verbindungen Ia299.001-Ia299.180, die sich von den entsprechenden Verbindungen Ia1.001-Ia1.180 dadurch unterscheiden, daß R¹ für Methylsulfonyl, R¹⁴ für n-Butyl und R¹⁵ für Ethylsulfonyl stehen: 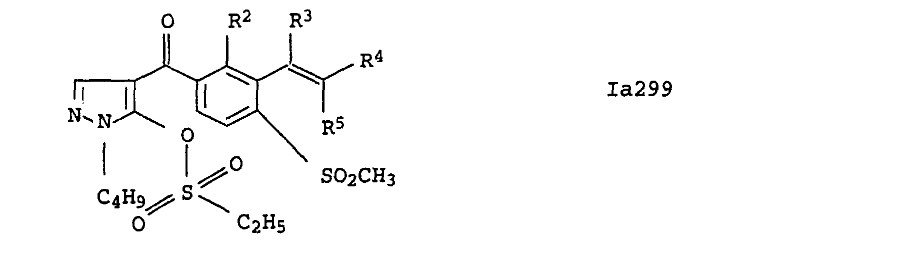
- Ebenso außergewöhnlich bevorzugt sind die Verbindungen Ia300, insbesondere die Verbindungen Ia300.001-Ia300.180, die sich von den entsprechenden Verbindungen Ia1.001-Ia1.180 dadurch unterscheiden, daß R¹ für Methylsulfonyl, R¹⁴ für iso-Butyl und R¹⁵ für Ethylsulfonyl stehen: 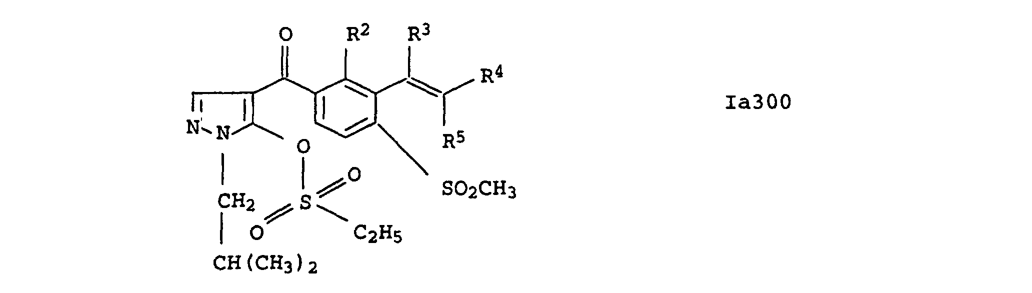
- Ebenso außergewöhnlich bevorzugt sind die Verbindungen Ia301, insbesondere die Verbindungen Ia301.001-Ia301.180, die sich von den entsprechenden Verbindungen Ia1.001-Ia1.180 dadurch unterscheiden, daß R¹ für Methylsulfonyl und R¹⁵ für n-Propylsulfonyl stehen: 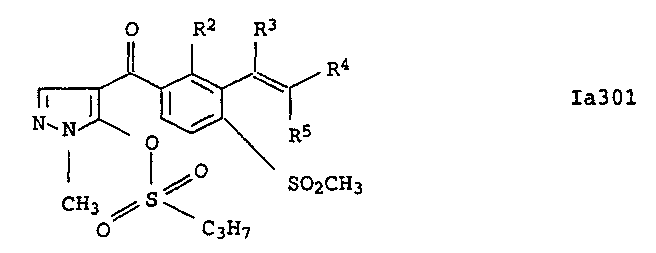
- Ebenso außergewöhnlich bevorzugt sind die Verbindungen Ia302, insbesondere die Verbindungen Ia302.001-Ia302.180, die sich von den entsprechenden Verbindungen Ia1.001-Ia1.180 dadurch unterscheiden, daß R¹ für Methylsulfonyl, R¹⁴ für Ethyl und R¹⁵ für n-Propylsulfonyl stehen: 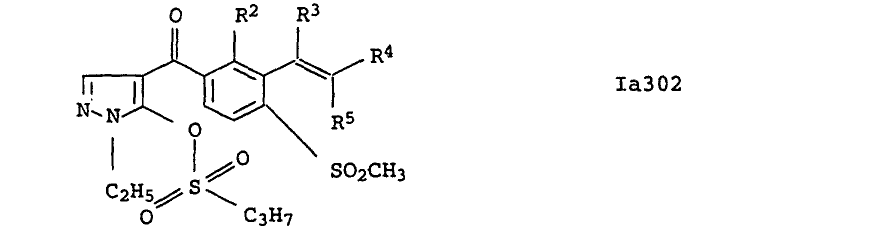
- Ebenso außergewöhnlich bevorzugt sind die Verbindungen Ia303, insbesondere die Verbindungen Ia303.001-Ia303.180, die sich von den entsprechenden Verbindungen Ia1.001-Ia1.180 dadurch unterscheiden, daß R¹ für Methylsulfonyl, R¹⁴ für n-Propyl und R¹⁵ für n-Propylsulfonyl stehen: 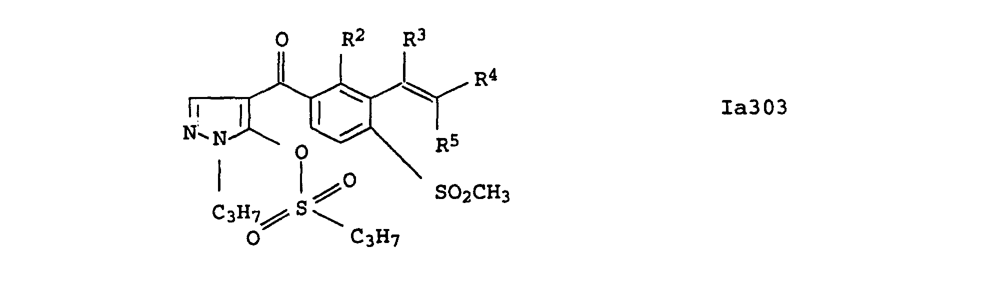
- Ebenso außergewöhnlich bevorzugt sind die Verbindungen Ia304, insbesondere die Verbindungen Ia304.001-Ia304.180, die sich von den entsprechenden Verbindungen Ia1.001-Ia1.180 dadurch unterscheiden, daß R¹ für Methylsulfonyl, R¹⁴ für n-Butyl und R¹⁵ für n-Propylsulfonyl stehen: 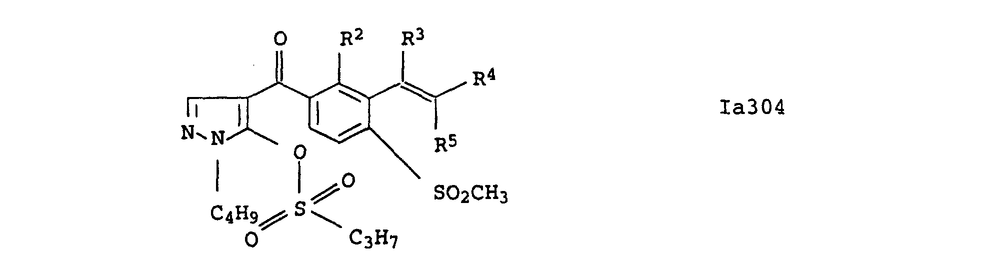
- Ebenso außergewöhnlich bevorzugt sind die Verbindungen Ia305, insbesondere die Verbindungen Ia305.001-Ia305.180, die sich von den entsprechenden Verbindungen Ia1.001-Ia1.180 dadurch unterscheiden, daß R¹ für Methylsulfonyl, R¹⁴ für iso-Butyl und R¹⁵ für n-Propylsulfonyl stehen: 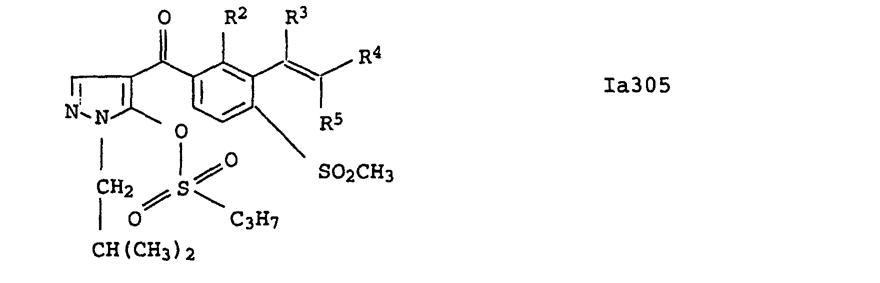
- Ebenso außergewöhnlich bevorzugt sind die Verbindungen Ia306, insbesondere die Verbindungen Ia306.001-Ia306.180, die sich von den entsprechenden Verbindungen Ia1.001-Ia1.180 dadurch unterscheiden, daß R¹ für Methylsulfonyl und R¹⁵ für iso-Propylsulfonyl stehen: 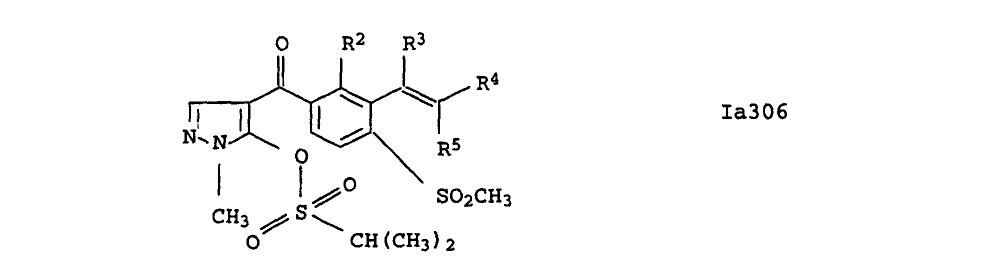
- Ebenso außergewöhnlich bevorzugt sind die Verbindungen Ia307, insbesondere die Verbindungen Ia307.001-Ia307.180, die sich von den entsprechenden Verbindungen Ia1.001-Ia1.180 dadurch unterscheiden, daß R¹ für Methylsulfonyl, R¹⁴ für Ethyl und R¹⁵ für iso-Propylsulfonyl stehen: 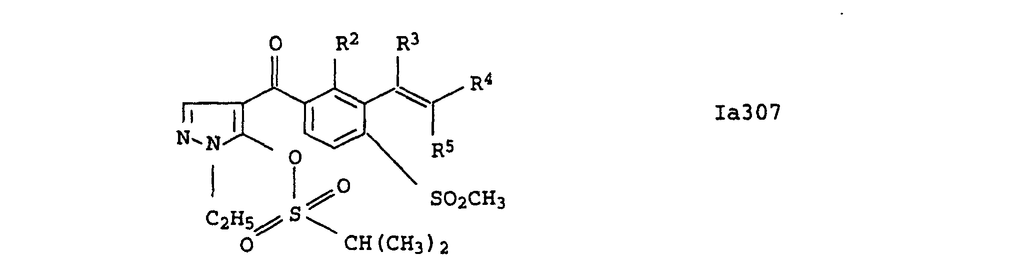
- Ebenso außergewöhnlich bevorzugt sind die Verbindungen Ia308, insbesondere die Verbindungen Ia308.001-Ia308.180, die sich von den entsprechenden Verbindungen Ia1.001-Ia1.180 dadurch unterscheiden, daß R¹ für Methylsulfonyl, R¹⁴ für n-Propyl und R¹⁵ für iso-Propylsulfonyl stehen: 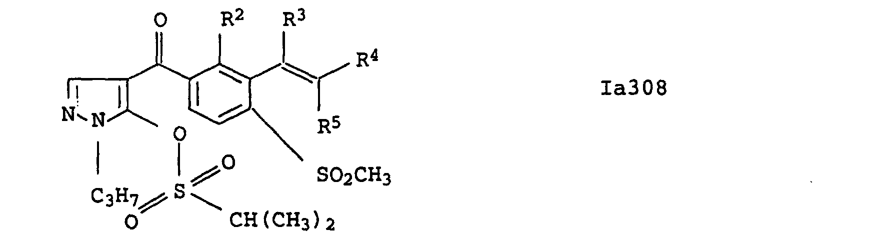
- Ebenso außergewöhnlich bevorzugt sind die Verbindungen Ia309, insbesondere die Verbindungen Ia309.001-Ia309.180, die sich von den entsprechenden Verbindungen Ia1.001-Ia1.180 dadurch unterscheiden, daß R¹ für Methylsulfonyl, R¹⁴ für n-Butyl und R¹⁵ für iso-Propylsulfonyl stehen: 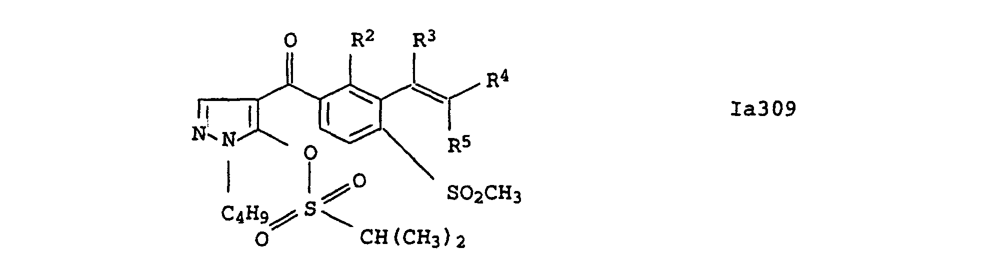
- Ebenso außergewöhnlich bevorzugt sind die Verbindungen Ia310, insbesondere die Verbindungen Ia310.001-Ia310.180, die sich von den entsprechenden Verbindungen Ia1.001-Ia1.180 dadurch unterscheiden, daß R¹ für Methylsulfonyl, R¹⁴ für iso-Butyl und R¹⁵ für iso-Propylsulfonyl stehen: 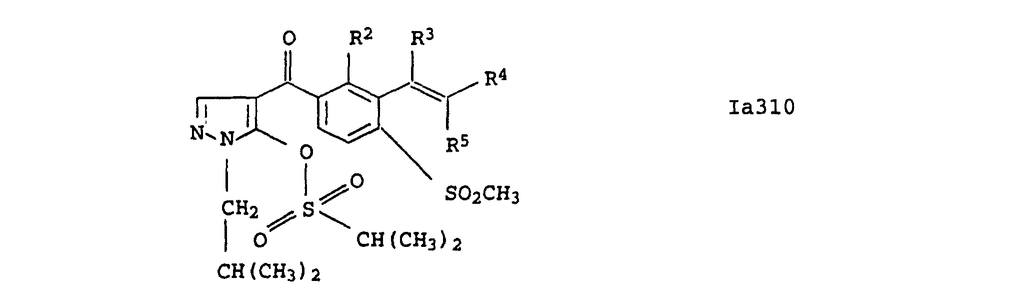
- Ebenso außergewöhnlich bevorzugt sind die Verbindungen Ia311, insbesondere die Verbindungen Ia311.001-Ia311.180, die sich von den entsprechenden Verbindungen Ia1.001-Ia1.180 dadurch unterscheiden, daß R¹ für Methylsulfonyl und R¹⁵ für n-Butylsulfonyl stehen: 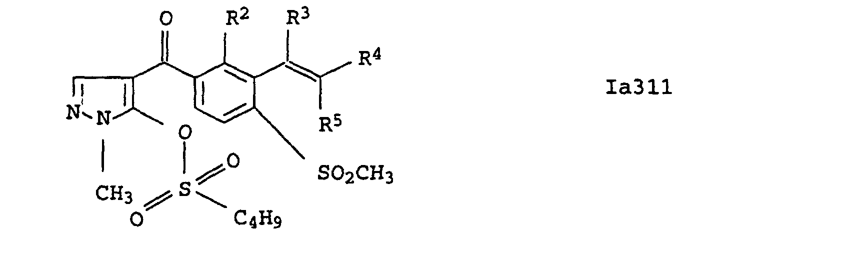
- Ebenso außergewöhnlich bevorzugt sind die Verbindungen Ia312, insbesondere die Verbindungen Ia312.001-Ia312.180, die sich von den entsprechenden Verbindungen Ia1.001-Ia1.180 dadurch unterscheiden, daß R¹ für Methylsulfonyl, R¹⁴ für Ethyl und R¹⁵ für n-Butylsulfonyl stehen: 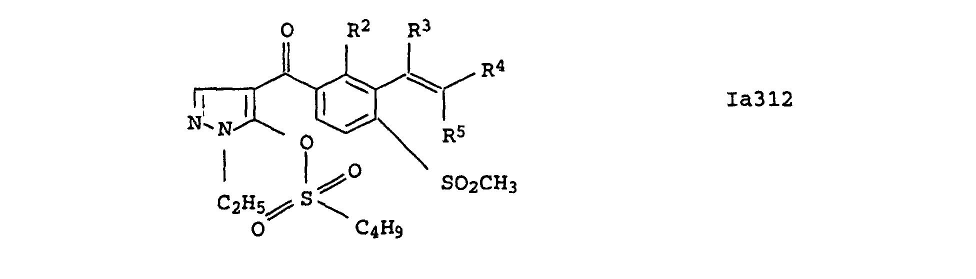
- Ebenso außergewöhnlich bevorzugt sind die Verbindungen Ia313, insbesondere die Verbindungen Ia313.001-Ia313.180, die sich von den entsprechenden Verbindungen Ia1.001-Ia1.180 dadurch unterscheiden, daß R¹ für Methylsulfonyl, R¹⁴ für n-Propyl und R¹⁵ für n-Butylsulfonyl stehen: 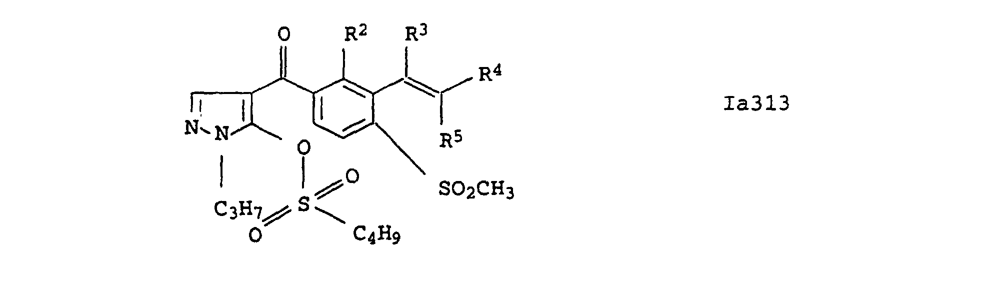
- Ebenso außergewöhnlich bevorzugt sind die Verbindungen Ia314, insbesondere die Verbindungen Ia314.001-Ia314.180, die sich von den entsprechenden Verbindungen Ia1.001-Ia1.180 dadurch unterscheiden, daß R¹ für Methylsulfonyl, R¹⁴ für n-Butyl und R¹⁵ für n-Butylsulfonyl stehen: 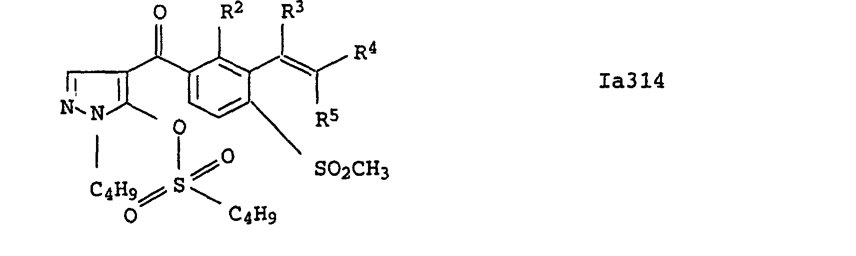
- Ebenso außergewöhnlich bevorzugt sind die Verbindungen Ia315, insbesondere die Verbindungen Ia315.001-Ia315.180, die sich von den entsprechenden Verbindungen Ia1.001-Ia1.180 dadurch unterscheiden, daß R¹ für Methylsulfonyl, R¹⁴ für iso-Butyl und R¹⁵ für n-Butylsulfonyl stehen: 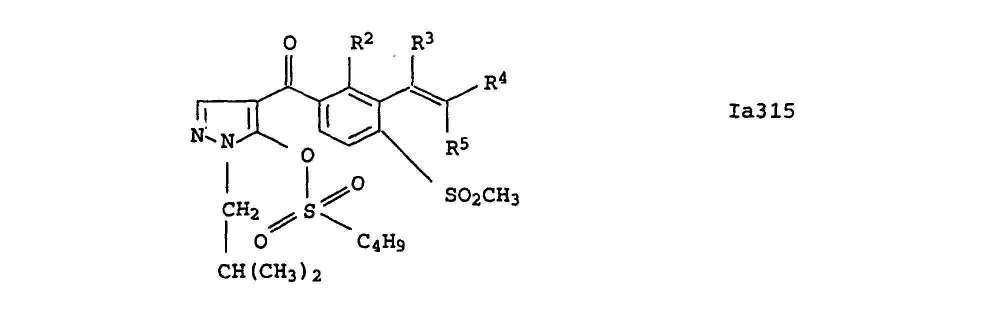
- Ebenso außergewöhnlich bevorzugt sind die Verbindungen Ia316, insbesondere die Verbindungen Ia316.001-Ia316.180, die sich von den entsprechenden Verbindungen Ia1.001-Ia1.180 dadurch unterscheiden, daß R¹ für Methylsulfonyl und R¹⁵ für iso-Butylsulfonyl stehen: 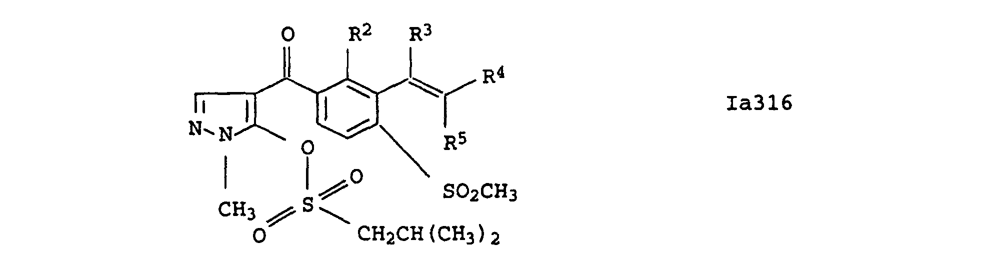
- Ebenso außergewöhnlich bevorzugt sind die Verbindungen Ia317, insbesondere die Verbindungen Ia317.001-Ia317.180, die sich von den entsprechenden Verbindungen Ia1.001-Ia1.180 dadurch unterscheiden, daß R¹ für Methylsulfonyl, R¹⁴ für Ethyl und R¹⁵ für iso-Butylsulfonyl stehen: 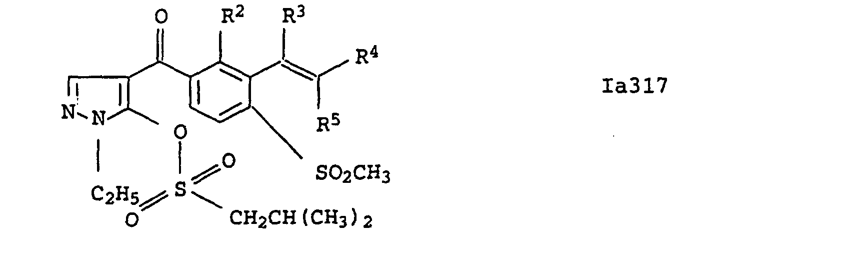
- Ebenso außergewöhnlich bevorzugt sind die Verbindungen Ia318, insbesondere die Verbindungen Ia318.001-Ia318.180, die sich von den entsprechenden Verbindungen Ia1.001-Ia1.180 dadurch unterscheiden, daß R¹ für Methylsulfonyl, R¹⁴ für n-Propyl und R¹⁵ für iso-Butylsulfonyl stehen: 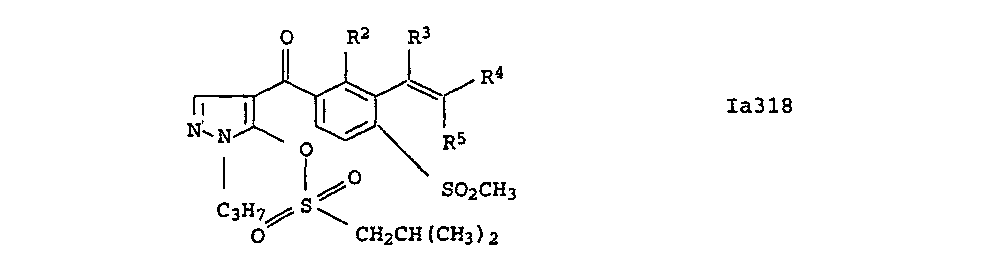
- Ebenso außergewöhnlich bevorzugt sind die Verbindungen Ia319, insbesondere die Verbindungen Ia319.001-Ia319.180, die sich von den entsprechenden Verbindungen Ia1.001-Ia1.180 dadurch unterscheiden, daß R¹ für Methylsulfonyl, R¹⁴ für n-Butyl und R¹⁵ für iso-Butylsulfonyl stehen: 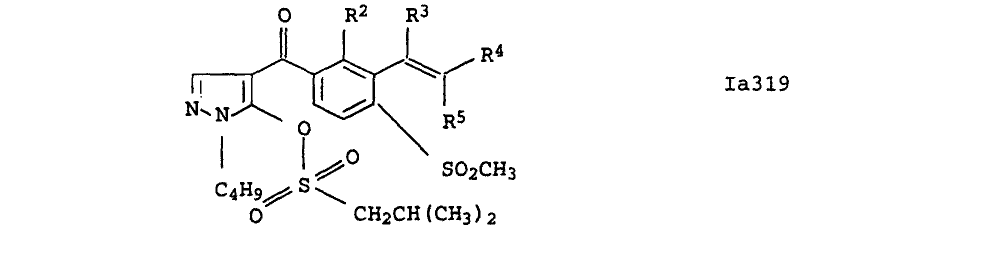
- Ebenso außergewöhnlich bevorzugt sind die Verbindungen Ia320, insbesondere die Verbindungen Ia320.001-Ia320.180, die sich von den entsprechenden Verbindungen Ia1.001-Ia1.180 dadurch unterscheiden, daß R¹ für Methylsulfonyl, R¹⁴ für iso-Butyl und R¹⁵ für iso-Butylsulfonyl stehen: 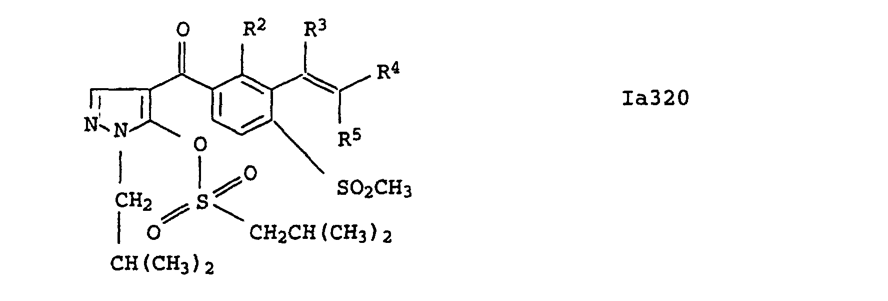
- Ebenso außergewöhnlich bevorzugt sind die Verbindungen Ia321, insbesondere die Verbindungen Ia321.001-Ia321.180, die sich von den entsprechenden Verbindungen Ia1.001-Ia1.180 dadurch unterscheiden, daß R¹ für Methylsulfonyl und R¹⁵ für sec-Butylsulfonyl stehen: 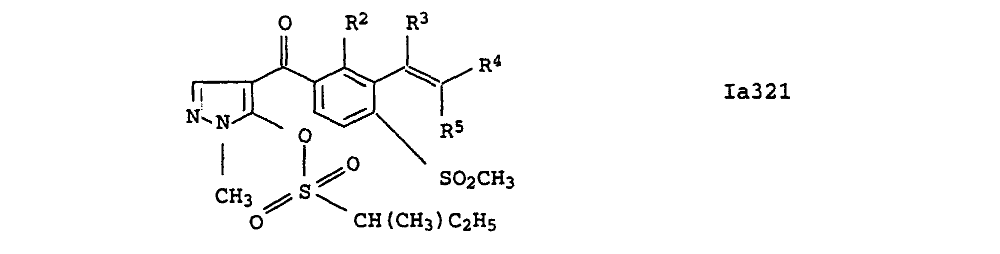
- Ebenso außergewöhnlich bevorzugt sind die Verbindungen Ia322, insbesondere die Verbindungen Ia322.001-Ia322.180, die sich von den entsprechenden Verbindungen Ia1.001-Ia1.180 dadurch unterscheiden, daß R¹ für Methylsulfonyl, R¹⁴ für Ethyl und R¹⁵ für sec-Butylsulfonyl stehen: 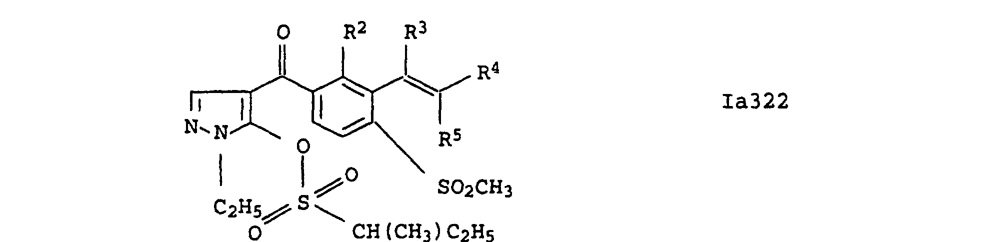
- Ebenso außergewöhnlich bevorzugt sind die Verbindungen Ia323, insbesondere die Verbindungen Ia323.001-Ia323.180, die sich von den entsprechenden Verbindungen Ia1.001-Ia1.180 dadurch unterscheiden, daß R¹ für Methylsulfonyl, R¹⁴ für für n-Propyl und R¹⁵ für sec-Butylsulfonyl stehen: 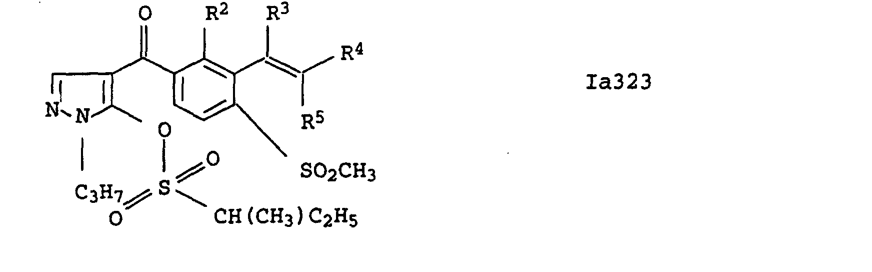
- Ebenso außergewöhnlich bevorzugt sind die Verbindungen Ia324, insbesondere die Verbindungen Ia324.001-Ia324.180, die sich von den entsprechenden Verbindungen Ia1.001-IaI.180 dadurch unterscheiden, daß R¹ für Methylsulfonyl, R¹⁴ für n-Butyl und R¹⁵ für sec-Butylsulfonyl stehen: 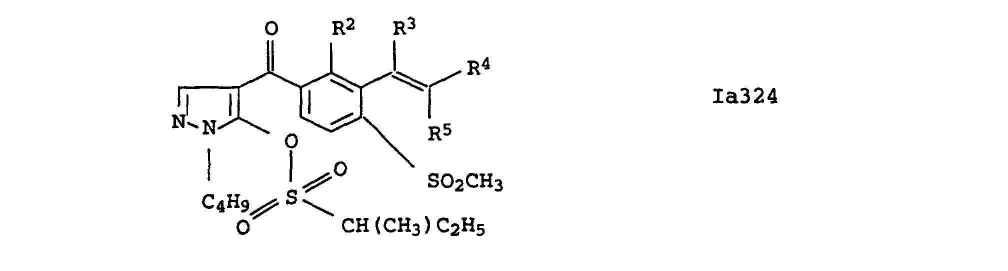
- Ebenso außergewöhnlich bevorzugt sind die Verbindungen Ia325, insbesondere die Verbindungen Ia325.001-Ia325.180, die sich von den entsprechenden Verbindungen Ia1.001-Ia1.180 dadurch unterscheiden, daß R¹ für Methylsulfonyl, R¹⁴ für iso-Butyl und R¹⁵ für sec-Butylsulfonyl stehen: 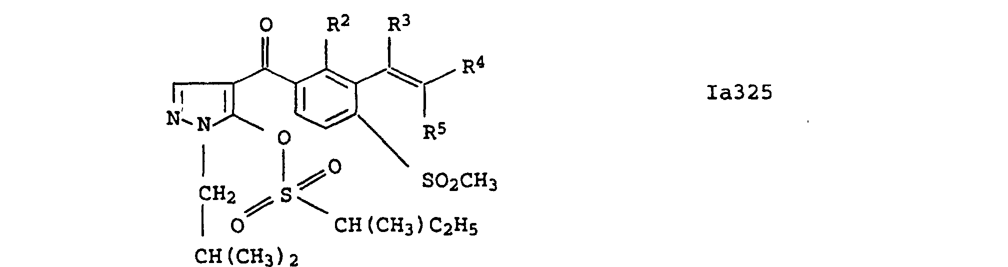
- Ebenso außergewöhnlich bevorzugt sind die Verbindungen Ia326, insbesondere die Verbindungen Ia326.001-Ia326.180, die sich von den entsprechenden Verbindungen Ia1.001-Ia1.180 dadurch unterscheiden, daß R¹ für Methylsulfonyl und R¹⁵ für Trifluormethylsulfonyl stehen: 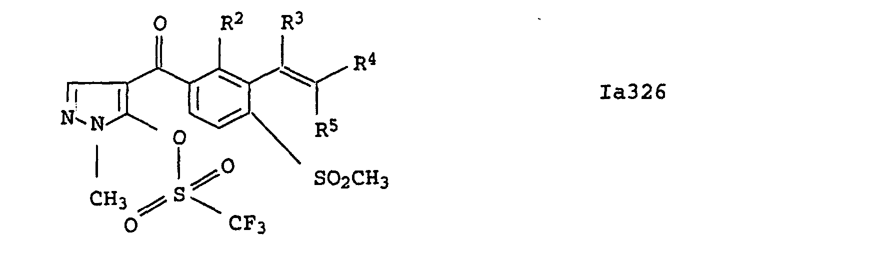
- Ebenso außergewöhnlich bevorzugt sind die Verbindungen Ia327, insbesondere die Verbindungen Ia327.001-Ia327.180, die sich von den entsprechenden Verbindungen Ia1.001-Ia1.180 dadurch unterscheiden, daß R¹ für Methylsulfonyl, R¹⁴ für Ethyl und R¹⁵ für Trifluormethylsulfonyl stehen: 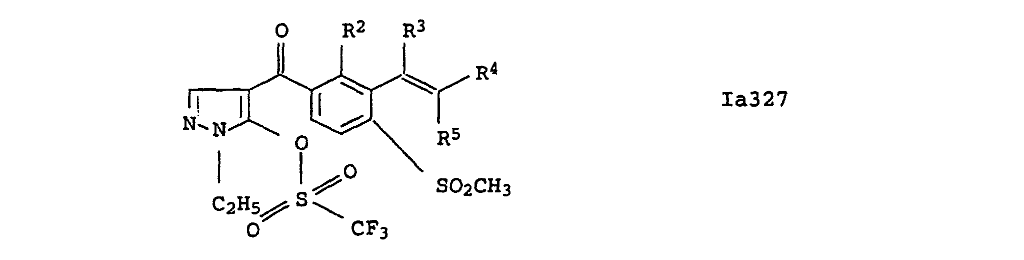
- Ebenso außergewöhnlich bevorzugt sind die Verbindungen Ia328, insbesondere die Verbindungen Ia328.001-Ia328.180, die sich von den entsprechenden Verbindungen Ia1.001-Ia1.180 dadurch unterscheiden, daß R¹ für Methylsulfonyl, R¹⁴ für n-Propyl und R¹⁵ für Trifluormethylsulfonyl stehen: 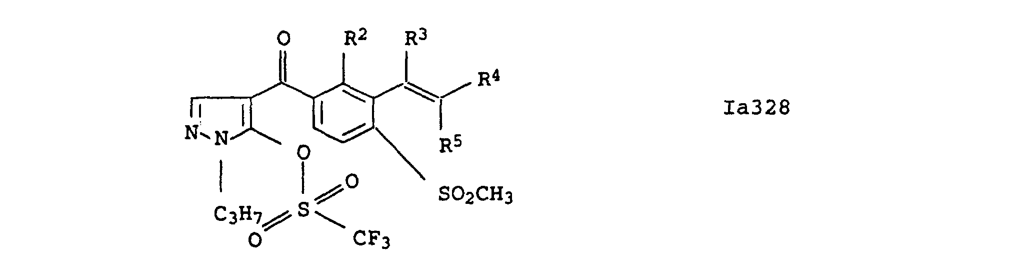
- Ebenso außergewöhnlich bevorzugt sind die Verbindungen Ia329, insbesondere die Verbindungen Ia329.001-Ia329.180, die sich von den entsprechenden Verbindungen Ia1.001-Ia1.180 dadurch unterscheiden, daß R¹ für Methylsulfonyl, R¹⁴ für n-Butyl und R¹⁵ für Trifluormethylsulfonyl stehen: 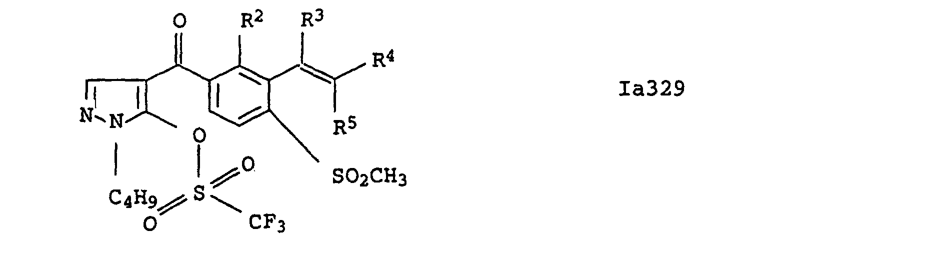
- Ebenso außergewöhnlich bevorzugt sind die Verbindungen Ia330, insbesondere die Verbindungen Ia330.001-Ia330.180, die sich von den entsprechenden Verbindungen Ia1.001-Ia1.180 dadurch unterscheiden, daß R¹ für Methylsulfonyl, R¹⁴ für iso-Butyl und R¹⁵ für Trifluormethylsulfonyl stehen: 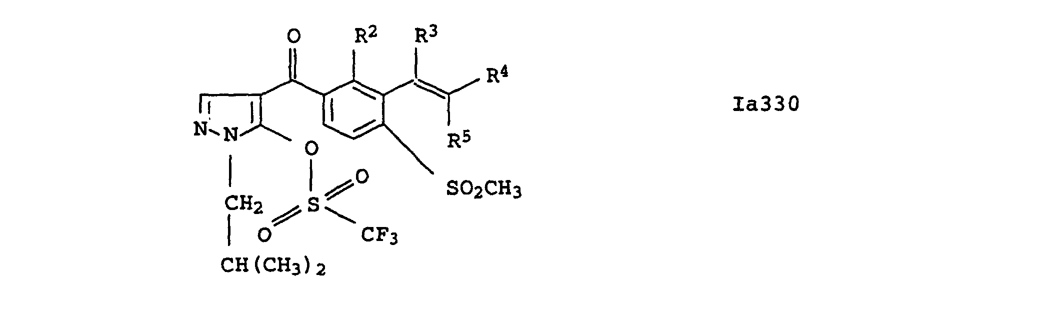
- Ebenso außergewöhnlich bevorzugt sind die Verbindungen Ia331, insbesondere die Verbindungen Ia331.001-Ia331.180, die sich von den entsprechenden Verbindungen Ia1.001-Ia1.180 dadurch unterscheiden, daß R¹ für Methylsulfonyl und R¹⁵ für Phenylcarbonylmethyl stehen: 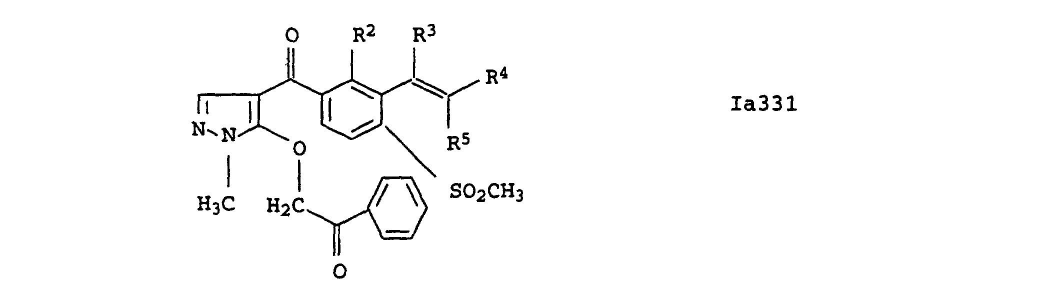
- Ebenso außergewöhnlich bevorzugt sind die Verbindungen Ia332, insbesondere die Verbindungen Ia332.001-Ia332.180, die sich von den entsprechenden Verbindungen Ia1.001-Ia1.180 dadurch unterscheiden, daß R¹ für Methylsulfonyl, R¹⁴ für Ethyl und R¹⁵ für Phenylcarbonylmethyl stehen: 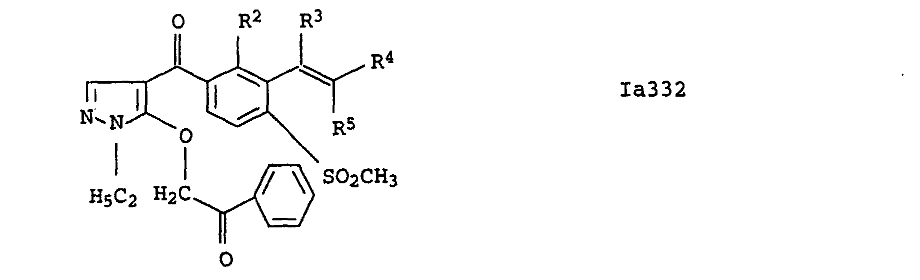
- Ebenso außergewöhnlich bevorzugt sind die Verbindungen Ia333, insbesondere die Verbindungen Ia333.001-Ia333.180, die sich von den entsprechenden Verbindungen Ia1.001-Ia1.180 dadurch unterscheiden, daß R¹ für Methylsulfonyl, R¹⁴ für n-Propyl und R¹⁵ für Phenylcarbonylmethyl stehen: 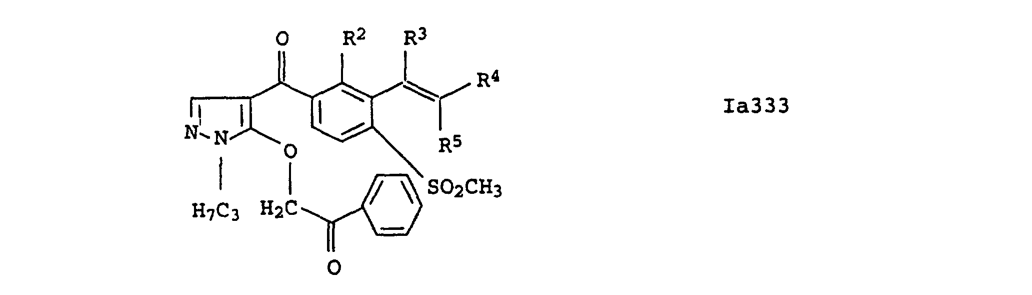
- Ebenso außergewöhnlich bevorzugt sind die Verbindungen Ia334, insbesondere die Verbindungen Ia334.001-Ia334.180, die sich von den entsprechenden Verbindungen Ia1.001-Ia1.180 dadurch unterscheiden, daß R¹ für Methylsulfonyl, R¹⁴ für n-Butyl und R¹⁵ für Phenylcarbonylmethyl stehen: 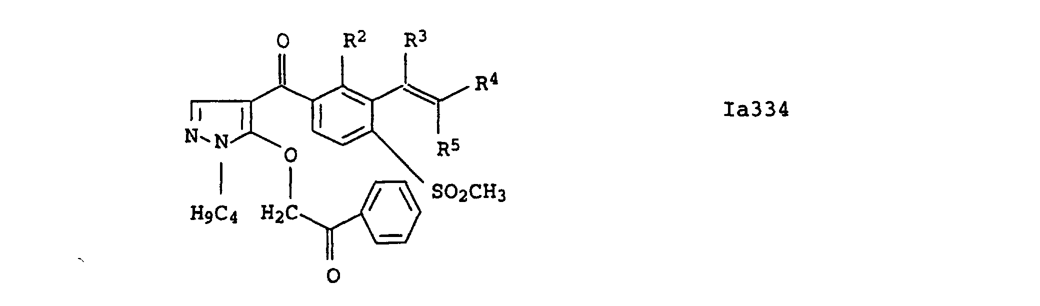
- Ebenso außergewöhnlich bevorzugt sind die Verbindungen Ia335, insbesondere die Verbindungen Ia335.001-Ia335.180, die sich von den entsprechenden Verbindungen Ia1.001-Ia1.180 dadurch unterscheiden, daß R¹ für Methylsulfonyl, R¹⁴ für iso-Butyl und R¹⁵ für Phenylcarbonylmethyl stehen: 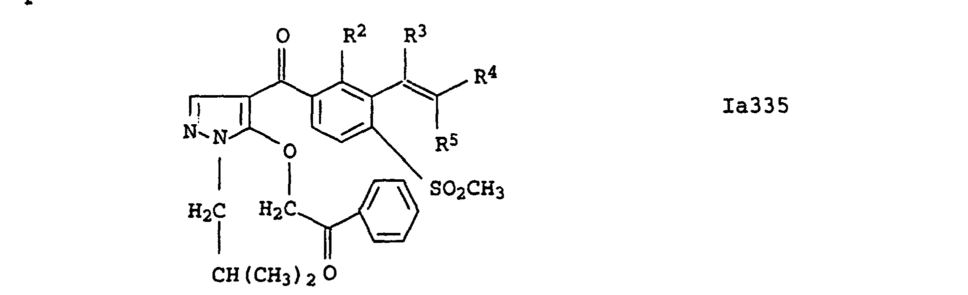
- Ebenso außergewöhnlich bevorzugt sind die Verbindungen Ia336, insbesondere die Verbindungen Ia336.001-Ia336.180, die sich von den entsprechenden Verbindungen Ia1.001-Ia1.180 dadurch unterscheiden, daß R¹ für Methylsulfonyl und R¹⁵ für Phenylsulfonyl stehen: 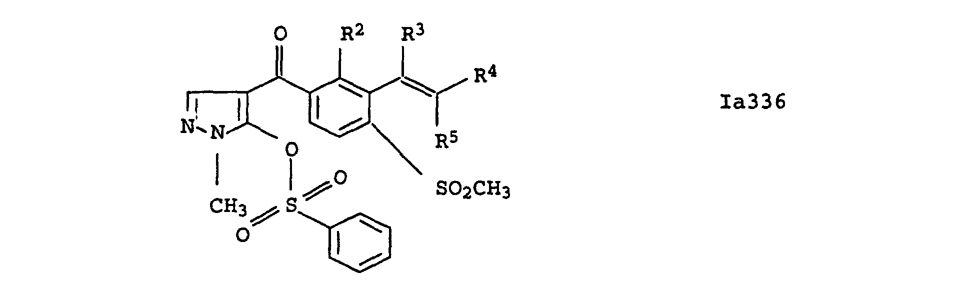
- Ebenso außergewöhnlich bevorzugt sind die Verbindungen Ia337, insbesondere die Verbindungen Ia337.001-Ia337.180, die sich von den entsprechenden Verbindungen Ia1.001-Ia1.180 dadurch unterscheiden, daß R¹ für Methylsulfonyl, R¹⁴ für Ethyl und R¹⁵ für Phenylsulfonyl stehen: 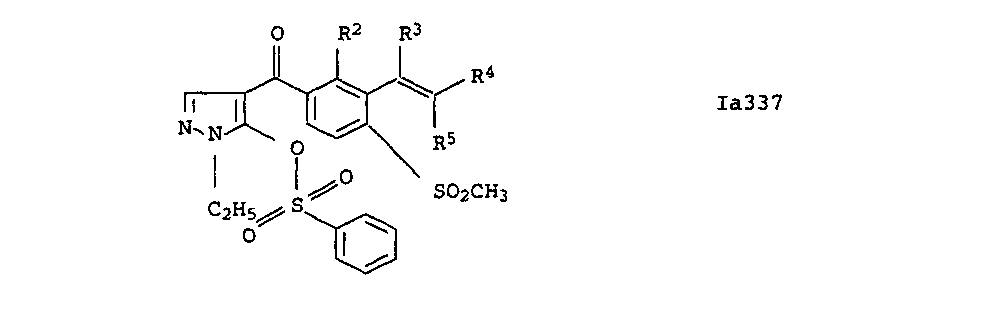
- Ebenso außergewöhnlich bevorzugt sind die Verbindungen Ia338, insbesondere die Verbindungen Ia338.001-Ia338.180, die sich von den entsprechenden Verbindungen Ia1.001-Ia1.180 dadurch unterscheiden, daß R¹ für Methylsulfonyl, R¹⁴ für n-Propyl und R¹⁵ für Phenylsulfonyl stehen: 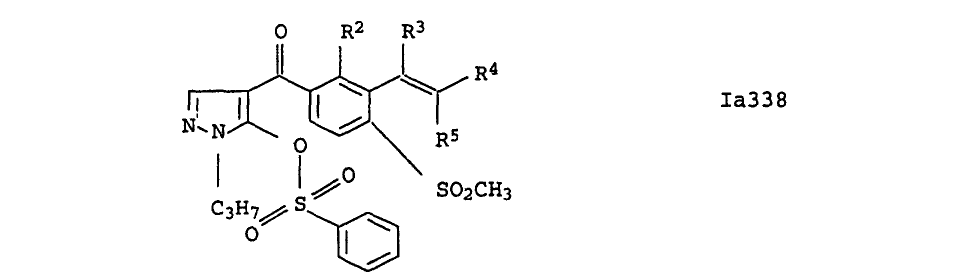
- Ebenso außergewöhnlich bevorzugt sind die Verbindungen Ia339, insbesondere die Verbindungen Ia339.001-Ia339.180, die sich von den entsprechenden Verbindungen Ia1.001-Ia1.180 dadurch unterscheiden, daß R¹ für Methylsulfonyl, R¹⁴ für n-Butyl und R¹⁵ für Phenylsulfonyl stehen: 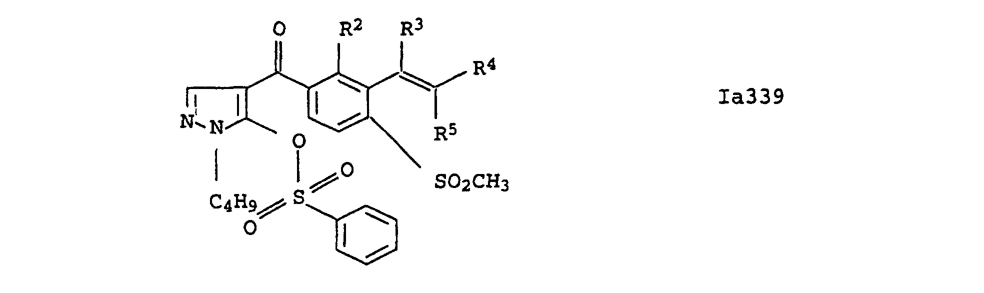
- Ebenso außergewöhnlich bevorzugt sind die Verbindungen Ia340, insbesondere die Verbindungen Ia340.001-Ia340.180, die sich von den entsprechenden Verbindungen Ia1.001-Ia1.180 dadurch unterscheiden, daß R¹ für Methylsulfonyl, R¹⁴ für iso-Butyl und R¹⁵ für Phenylsulfonyl stehen: 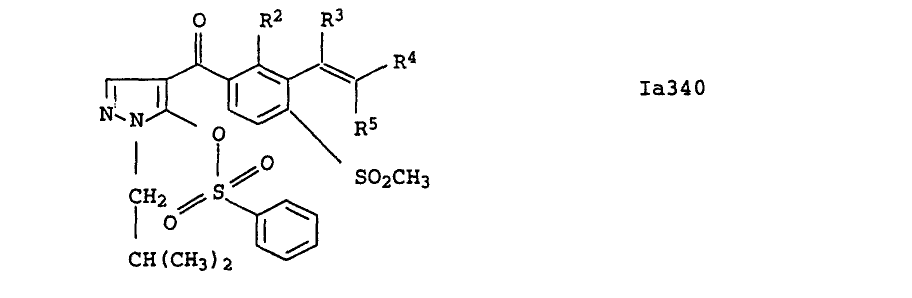
- Ebenso außergewöhnlich bevorzugt sind die Verbindungen Ia341, insbesondere die Verbindungen Ia341.001-Ia341.180, die sich von den entsprechenden Verbindungen Ia1.001-Ia1.180 dadurch unterscheiden, daß R¹ für Methylsulfonyl und R¹⁵ für 4-Methylphenylsulfonyl stehen: 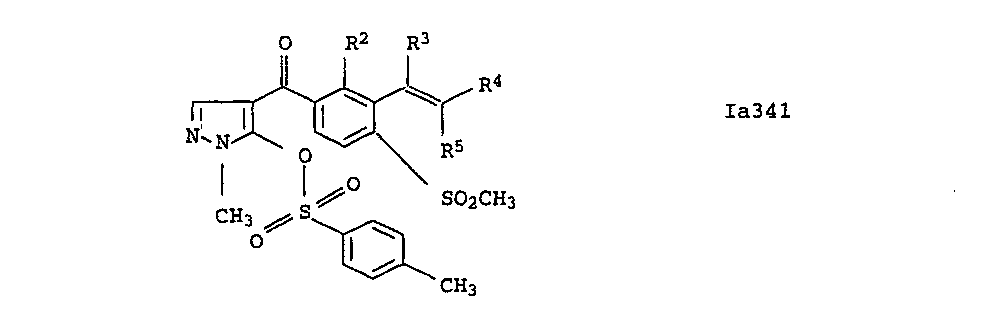
- Ebenso außergewöhnlich bevorzugt sind die Verbindungen Ia342, insbesondere die Verbindungen Ia342.001-Ia342.180, die sich von den entsprechenden Verbindungen Ia1.001-Ia1.180 dadurch unterscheiden, daß R¹ für Methylsulfonyl, R¹⁴ für Ethyl und R¹⁵ für 4-Methylphenylsulfonyl stehen: 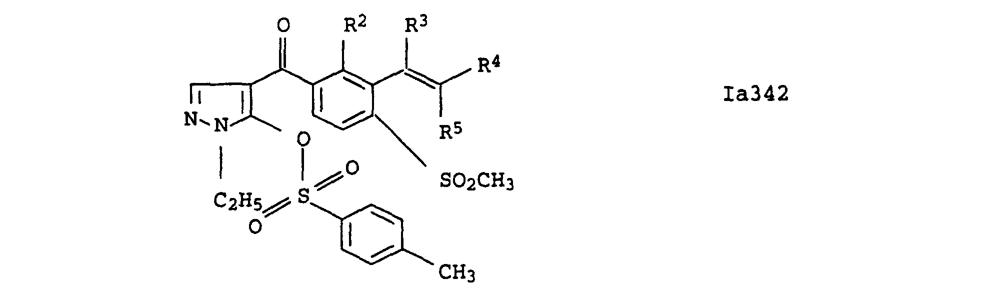
- Ebenso außergewöhnlich bevorzugt sind die Verbindungen Ia343, insbesondere die Verbindungen Ia343.001-Ia343.180, die sich von den entsprechenden Verbindungen Ia1.001-Ia1.180 dadurch unterscheiden, daß R¹ für Methylsulfonyl, R¹⁴ für n-Propyl und R¹⁵ für 4-Methylphenylsulfonyl stehen: 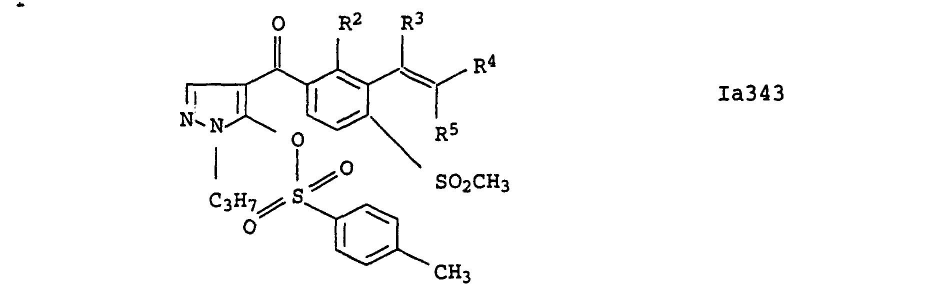
- Ebenso außergewöhnlich bevorzugt sind die Verbindungen Ia344, insbesondere die Verbindungen Ia344.001-Ia344.180, die sich von den entsprechenden Verbindungen Ia1.001-Ia1.180 dadurch unterscheiden, daß R¹ für Methylsulfonyl, R¹⁴ für n-Butyl und R¹⁵ für 4-Methylphenylsulfonyl stehen: 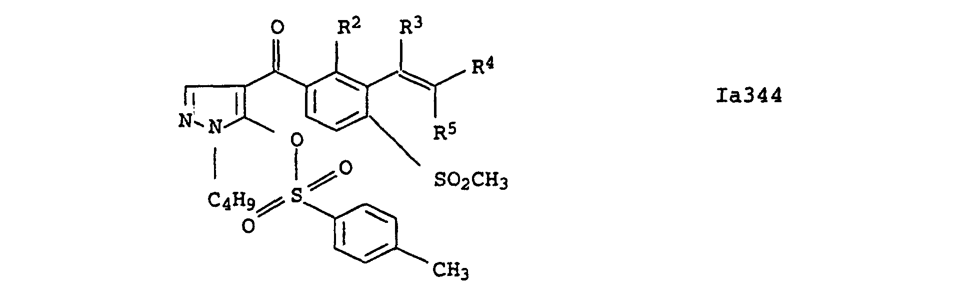
- Ebenso außergewöhnlich bevorzugt sind die Verbindungen Ia345, insbesondere die Verbindungen Ia345.001-Ia345.180, die sich von den entsprechenden Verbindungen Ia1.001-Ia1.180 dadurch unterscheiden, daß R¹ für Methylsulfonyl, R¹⁴ für iso-Butyl und R¹⁵ für 4-Methylphenylsulfonyl stehen: 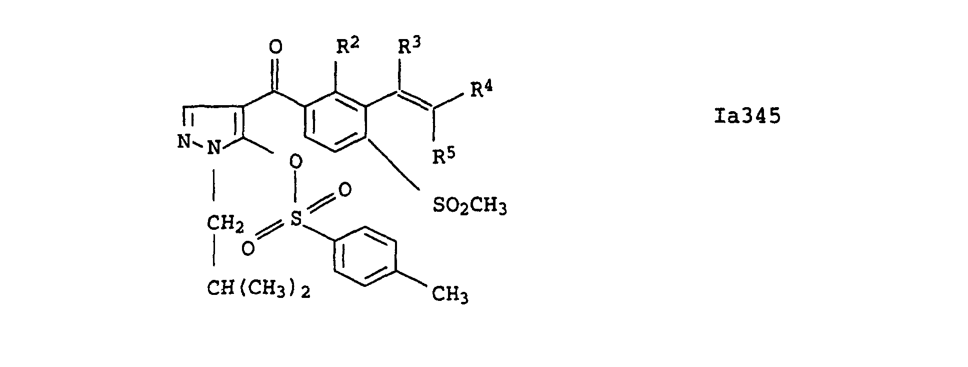
- Ebenso außergewöhnlich bevorzugt sind die Verbindungen Ia346, insbesondere die Verbindungen Ia346.001-Ia346.180, die sich von den entsprechenden Verbindungen Ia1.001-Ia1.180 dadurch unterscheiden, daß R¹⁶ für Methyl steht: 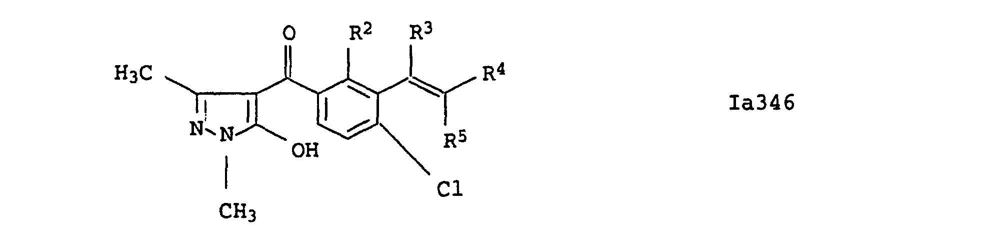
- Ebenso außergewöhnlich bevorzugt sind die Verbindungen Ia347, insbesondere die Verbindungen Ia347.001-Ia347.180, die sich von den entsprechenden Verbindungen Ia1.001-Ia1.180 dadurch unterscheiden, daß R¹⁴ für Ethyl und R¹⁶ für Methyl stehen: 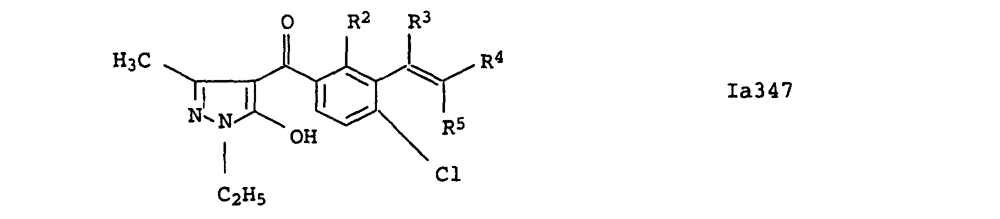
- Ebenso außergewöhnlich bevorzugt sind die Verbindungen Ia348, insbesondere die Verbindungen Ia348.001-Ia348.180, die sich von den entsprechenden Verbindungen Ia1.001-Ia1.180 dadurch unterscheiden, daß R¹⁴ für n-Propyl und R¹⁶ für Methyl stehen: 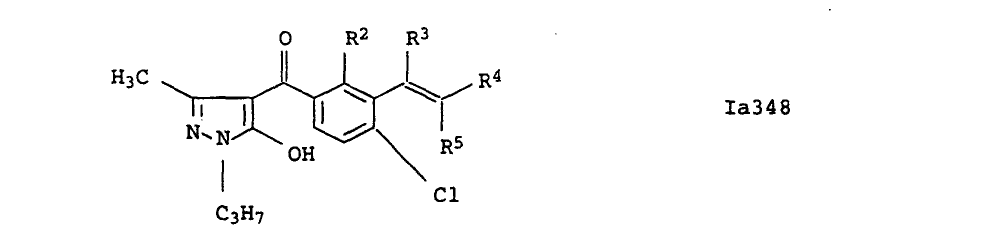
- Ebenso außergewöhnlich bevorzugt sind die Verbindungen Ia349, insbesondere die Verbindungen Ia349.001-Ia349.180, die sich von den entsprechenden Verbindungen Ia1.001-Ia1.180 dadurch unterscheiden, daß R¹⁴ für n-Butyl und R¹⁶ für Methyl stehen: 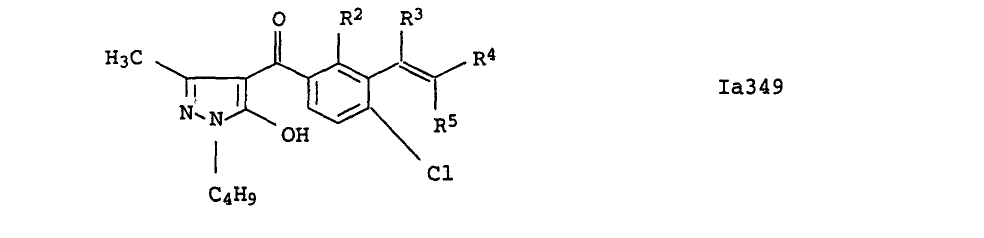
- Ebenso außergewöhnlich bevorzugt sind die Verbindungen Ia350, insbesondere die Verbindungen Ia350.001-Ia350.180, die sich von den entsprechenden Verbindungen Ia1.001-Ia1.180 dadurch unterscheiden, daß R¹⁴ für iso-Butyl und R¹⁶ für Methyl stehen: 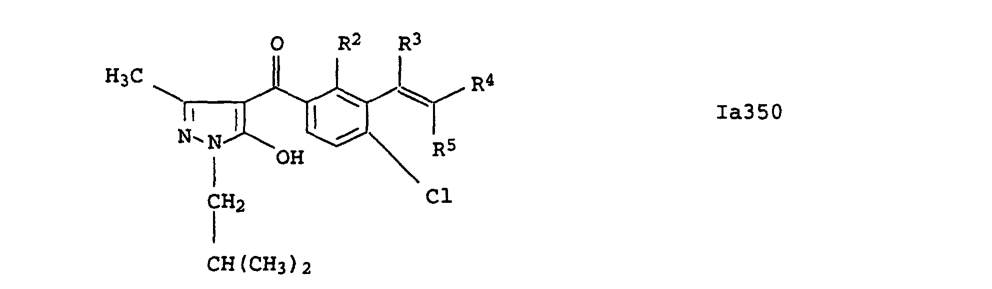
- Ebenso außergewöhnlich bevorzugt sind die Verbindungen Ia351, insbesondere die Verbindungen Ia351.001-Ia351.180, die sich von den entsprechenden Verbindungen Ia1.001-Ia1.180 dadurch unterscheiden, daß R¹⁵ und R¹⁶ für Methyl stehen: 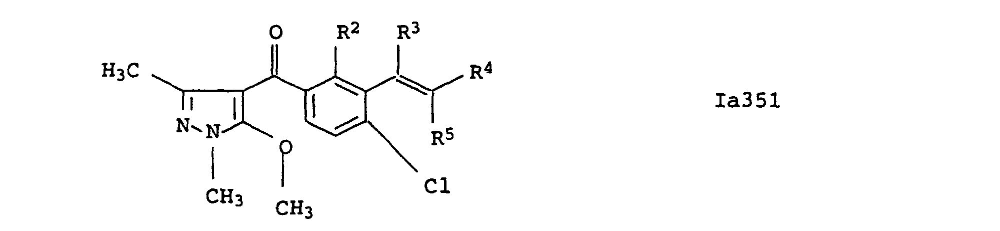
- Ebenso außergewöhnlich bevorzugt sind die Verbindungen Ia352, insbesondere die Verbindungen Ia352.001-Ia352.180, die sich von den entsprechenden Verbindungen Ia1.001-Ia1.180 dadurch unterscheiden, daß R¹⁴ für Ethyl, R¹⁵ und R¹⁶ für Methyl stehen: 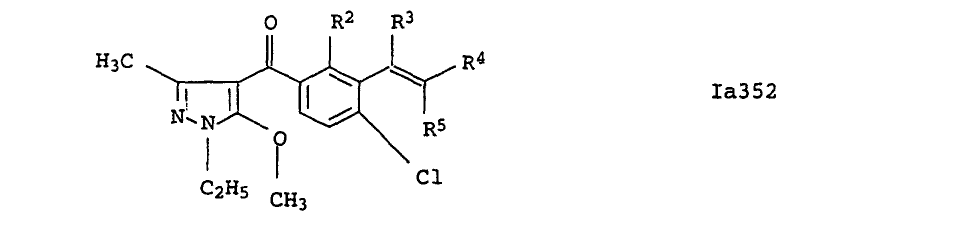
- Ebenso außergewöhnlich bevorzugt sind die Verbindungen Ia353, insbesondere die Verbindungen Ia353.001-Ia353.180, die sich von den entsprechenden Verbindungen Ia1.001-Ia1.180 dadurch unterscheiden, daß R¹⁴ für n-Propyl, R¹⁵ und R¹⁶ für Methyl stehen: 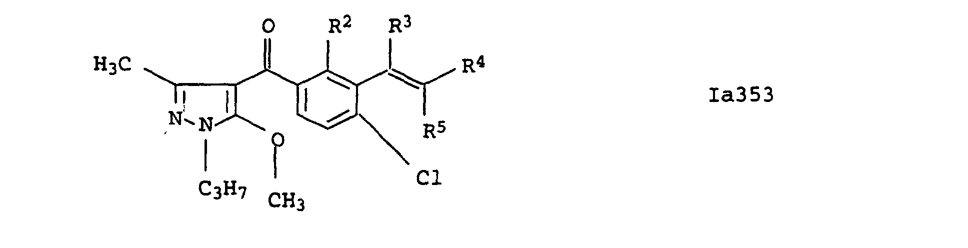
- Ebenso außergewöhnlich bevorzugt sind die Verbindungen Ia354, insbesondere die Verbindungen Ia354.001-Ia354.180, die sich von den entsprechenden Verbindungen Ia1.001-Ia1.180 dadurch unterscheiden, daß R¹⁴ für n-Butyl, R¹⁵ und R¹⁶ für Methyl stehen: 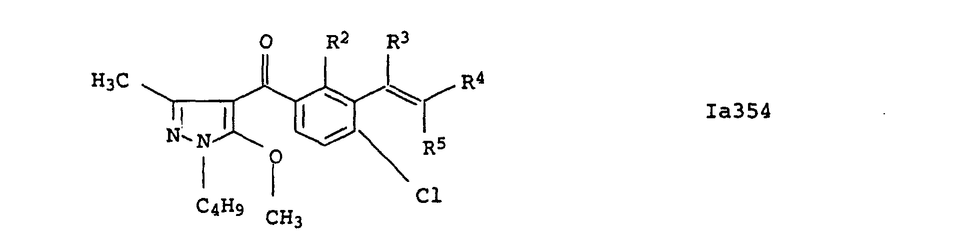
- Ebenso außergewöhnlich bevorzugt sind die Verbindungen Ia355, insbesondere die Verbindungen Ia355.001-Ia355.180, die sich von den entsprechenden Verbindungen Ia1.001-Ia1.180 dadurch unterscheiden, daß R¹⁴ für iso-Butyl, R¹⁵ und R¹⁶ für Methyl stehen: 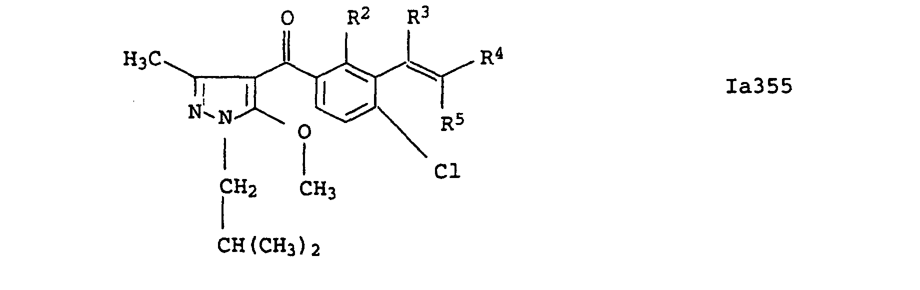
- Ebenso außergewöhnlich bevorzugt sind die Verbindungen Ia356, insbesondere die Verbindungen Ia356.001-Ia356.180, die sich von den entsprechenden Verbindungen Ia1.001-Ia1.180 dadurch unterscheiden, daß R¹⁵ für Ethyl und R¹⁶ für Methyl stehen: 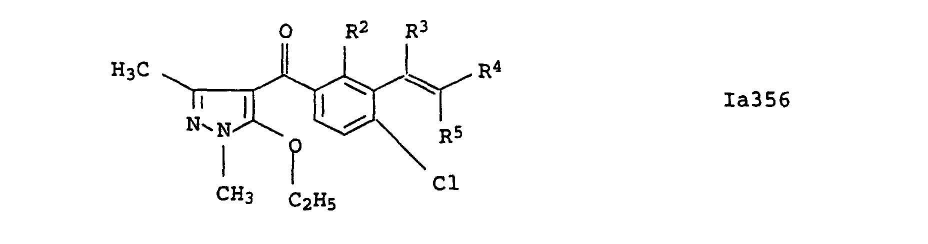
- Ebenso außergewöhnlich bevorzugt sind die Verbindungen Ia357, insbesondere die Verbindungen Ia357.001-Ia357.180, die sich von den entsprechenden Verbindungen Ia1.001-Ia1.180 dadurch unterscheiden, daß R¹⁴ und R¹⁵ für Ethyl und R¹⁶ für Methyl stehen: 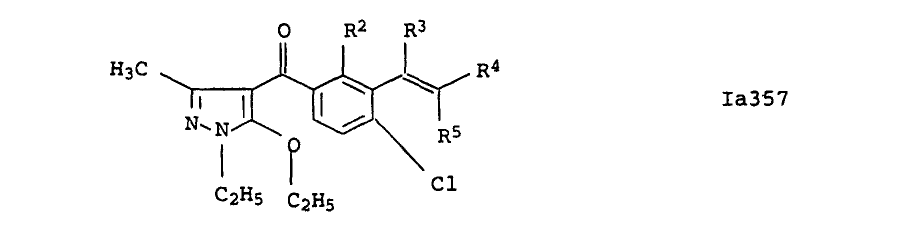
- Ebenso außergewöhnlich bevorzugt sind die Verbindungen Ia358, insbesondere die Verbindungen Ia358.001-Ia358.180, die sich von den entsprechenden Verbindungen Ia1.001-Ia1.180 dadurch unterscheiden, daß R¹⁴ für n-Propyl, R¹⁵ für Ethyl und R¹⁶ für Methyl stehen: 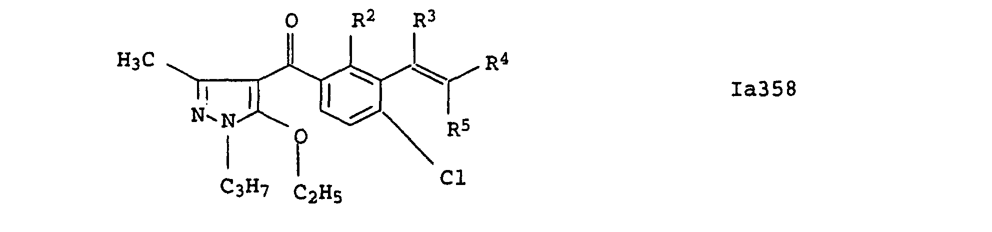
- Ebenso außergewöhnlich bevorzugt sind die Verbindungen Ia359, insbesondere die Verbindungen Ia359.001-Ia359.180, die sich von den entsprechenden Verbindungen Ia1.001-Ia1.180 dadurch unterscheiden, daß R¹⁴ für n-Butyl, R¹⁵ für Ethyl und R¹⁶ für Methyl stehen: 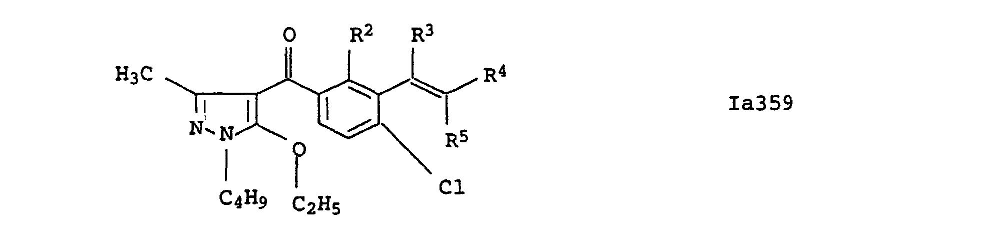
- Ebenso außergewöhnlich bevorzugt sind die Verbindungen Ia360, insbesondere die Verbindungen Ia360.001-Ia360.180, die sich von den entsprechenden Verbindungen Ia1.001-Ia1.180 dadurch unterscheiden, daß R¹⁴ für iso-Butyl, R¹⁵ für Ethyl und R¹⁶ für Methyl stehen: 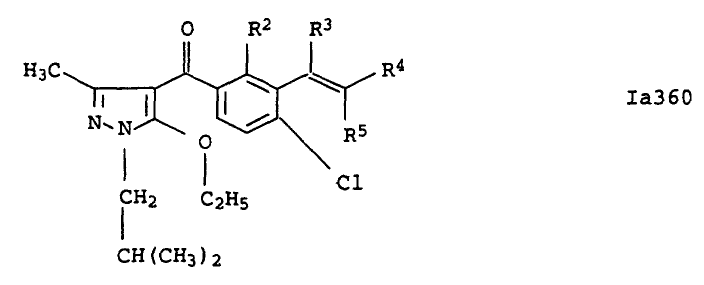
- Ebenso außergewöhnlich bevorzugt sind die Verbindungen Ia361, insbesondere die Verbindungen Ia361.001-Ia361.180, die sich von den entsprechenden Verbindungen Ia1.001-Ia1.180 dadurch unterscheiden, daß R¹⁴ für n-Propyl und R¹⁶ für Methyl stehen: 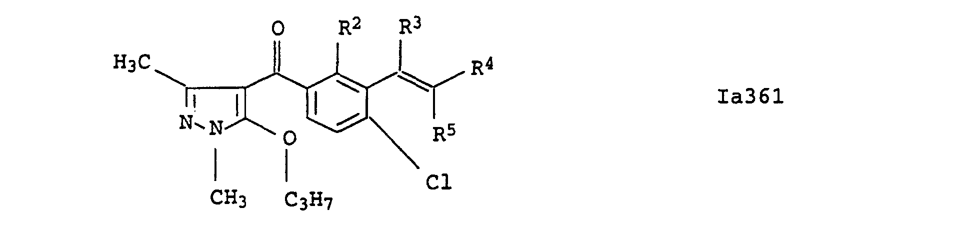
- Ebenso außergewöhnlich bevorzugt sind die Verbindungen Ia362, insbesondere die Verbindungen Ia362.001-Ia362.180, die sich von den entsprechenden Verbindungen Ia1.001-Ia1.180 dadurch unterscheiden, daß R¹⁴ für Ethyl, R¹⁵ für n-Propyl und R¹⁶ für Methyl stehen: 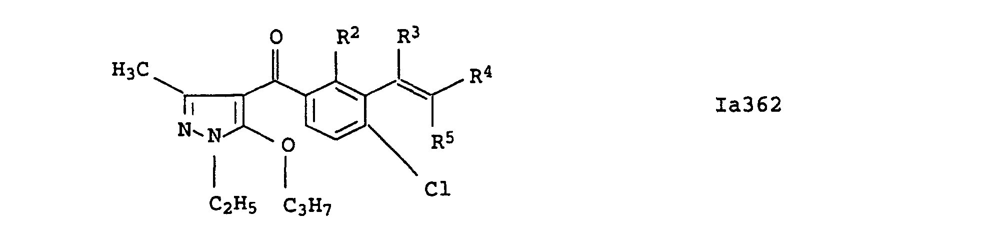
- Ebenso außergewöhnlich bevorzugt sind die Verbindungen Ia363, insbesondere die Verbindungen Ia363.001-Ia363.180, die sich von den entsprechenden Verbindungen Ia1.001-Ia1.180 dadurch unterscheiden, daß R¹⁴ und R¹⁵ für n-Propyl und R¹⁶ für Methyl stehen: 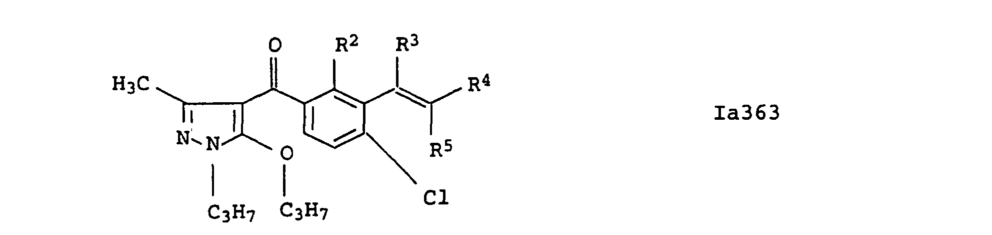
- Ebenso außergewöhnlich bevorzugt sind die Verbindungen Ia364, insbesondere die Verbindungen Ia364.001-Ia364.180, die sich von den entsprechenden Verbindungen Ia1.001-Ia1.180 dadurch unterscheiden, daß R¹⁴ für n-Butyl, R¹⁵ für n-Propyl und R¹⁶ für Methyl stehen: 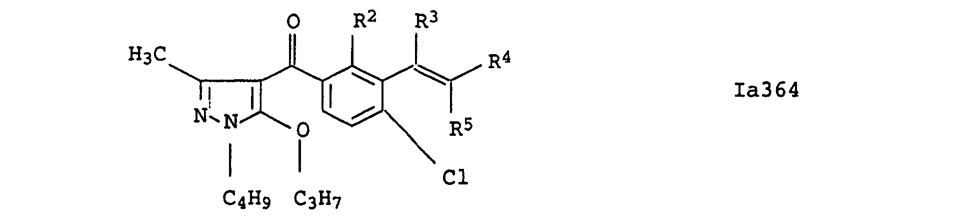
- Ebenso außergewöhnlich bevorzugt sind die Verbindungen Ia365, insbesondere die Verbindungen Ia365.001-Ia365.180, die sich von den entsprechenden Verbindungen Ia1.001-Ia1.180 dadurch unterscheiden, daß R¹⁴ für iso-Butyl, R¹⁵ für n-Propyl und R¹⁶ für Methyl stehen: 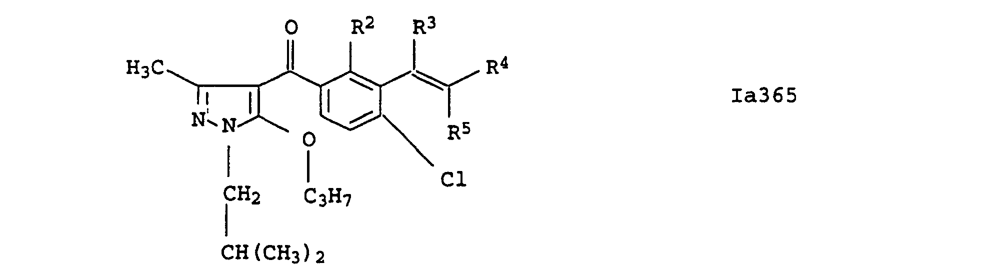
- Ebenso außergewöhnlich bevorzugt sind die Verbindungen Ia366, insbesondere die Verbindungen Ia366.001-Ia366.180, die sich von den entsprechenden Verbindungen Ia1.001-Ia1.180 dadurch unterscheiden, daß R¹⁵ für iso-Propyl und R¹⁶ für Methyl stehen: 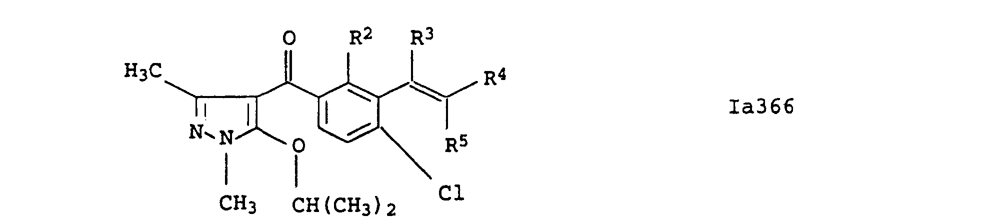
- Ebenso außergewöhnlich bevorzugt sind die Verbindungen Ia367, insbesondere die Verbindungen Ia367.001-Ia367.180, die sich von den entsprechenden Verbindungen Ia1.001-Ia1.180 dadurch unterscheiden, daß R¹⁴ für Ethyl, R¹⁵ für iso-Propyl und R¹⁶ für Methyl stehen: 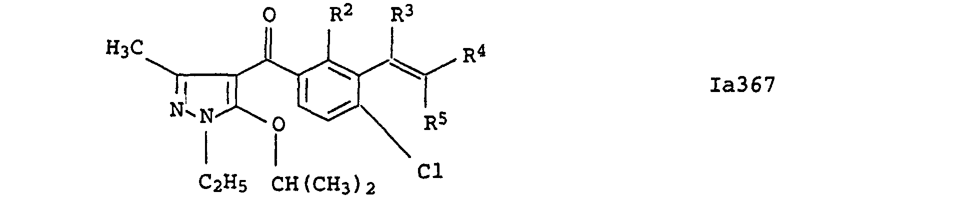
- Ebenso außergewöhnlich bevorzugt sind die Verbindungen Ia368, insbesondere die Verbindungen Ia368.001-Ia368.180, die sich von den entsprechenden Verbindungen Ia1.001-Ia1.180 dadurch unterscheiden, daß R¹⁴ für n-Propyl, R¹⁵ für iso-Propyl und R¹⁶ für Methyl stehen: 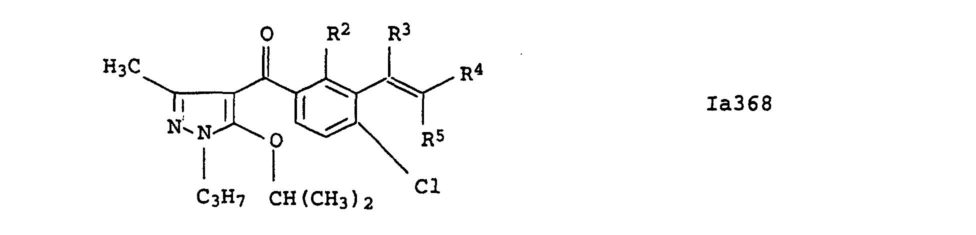
- Ebenso außergewöhnlich bevorzugt sind die Verbindungen Ia369, insbesondere die Verbindungen Ia369.001-Ia369.180, die sich von den entsprechenden Verbindungen Ia1.001-Ia1.180 dadurch unterscheiden, daß R¹⁴ für n-Butyl, R¹⁵ für iso-Propyl und R¹⁶ für Methyl stehen: 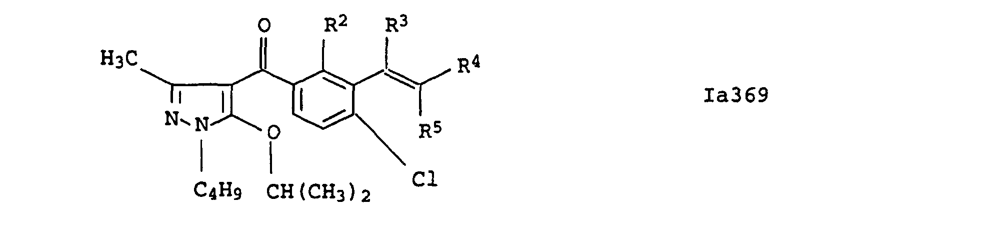
- Ebenso außergewöhnlich bevorzugt sind die Verbindungen Ia370, insbesondere die Verbindungen Ia370.001-Ia370.180, die sich von den entsprechenden Verbindungen Ia1.001-Ia1.180 dadurch unterscheiden, daß R¹⁴ für iso-Butyl, R¹⁵ für iso-Propyl und R¹⁶ für Methyl stehen: 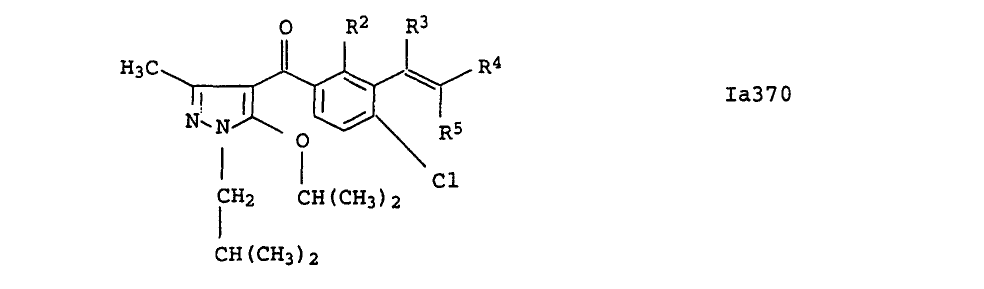
- Ebenso außergewöhnlich bevorzugt sind die Verbindungen Ia371, insbesondere die Verbindungen Ia371.001-Ia371.180, die sich von den entsprechenden Verbindungen Ia1.001-Ia1.180 dadurch unterscheiden, daß R¹⁵ für n-Butyl und R¹⁶ für Methyl stehen: 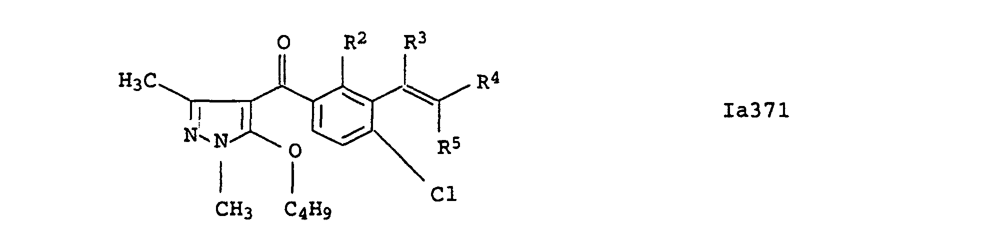
- Ebenso außergewöhnlich bevorzugt sind die Verbindungen Ia372, insbesondere die Verbindungen Ia372.001-Ia372.180, die sich von den entsprechenden Verbindungen Ia1.001-Ia1.180 dadurch unterscheiden, daß R¹⁴ für Ethyl, R¹⁵ für n-Butyl und R¹⁶ für Methyl stehen: 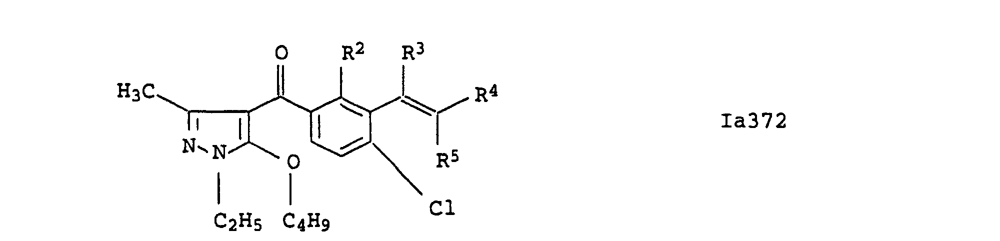
- Ebenso außergewöhnlich bevorzugt sind die Verbindungen Ia373, insbesondere die Verbindungen Ia373.001-Ia373.180, die sich von den entsprechenden Verbindungen Ia1.001-Ia1.180 dadurch unterscheiden, daß R¹⁴ für n-Propyl, R¹⁵ für n-Butyl und R¹⁶ für Methyl stehen: 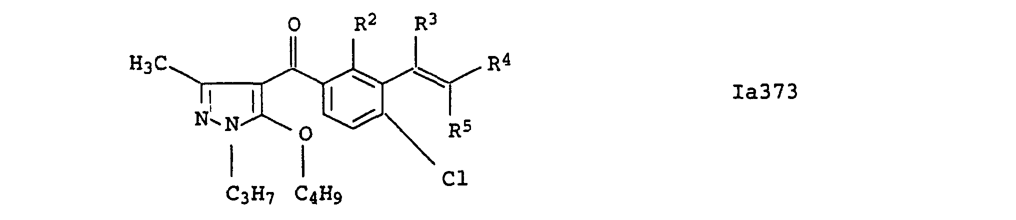
- Ebenso außergewöhnlich bevorzugt sind die Verbindungen Ia374, insbesondere die Verbindungen Ia374.001-Ia374.180, die sich von den entsprechenden Verbindungen Ia1.001-Ia1.180 dadurch unterscheiden, daß R¹⁴ und R¹⁵ für n-Butyl und R¹⁶ für Methyl stehen: 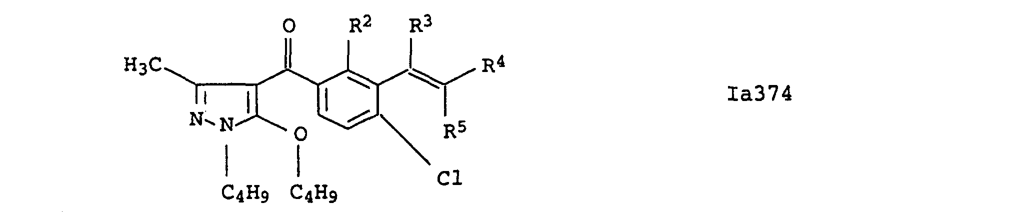
- Ebenso außergewöhnlich bevorzugt sind die Verbindungen Ia375, insbesondere die Verbindungen Ia375.001-Ia375.180, die sich von den entsprechenden Verbindungen Ia1.001-Ia1.180 dadurch unterscheiden, daß R¹⁴ für iso-Butyl, R¹⁵ für n-Butyl und R¹⁶ für Methyl stehen: 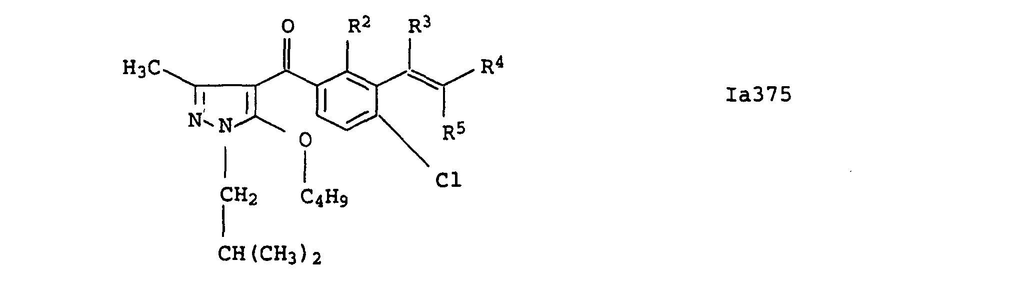
- Ebenso außergewöhnlich bevorzugt sind die Verbindungen Ia376, insbesondere die Verbindungen Ia376.001-Ia376.180, die sich von den entsprechenden Verbindungen Ia1.001-Ia1.180 dadurch unterscheiden, daß R¹⁵ für sec-Butyl und R¹⁶ für Methyl stehen: 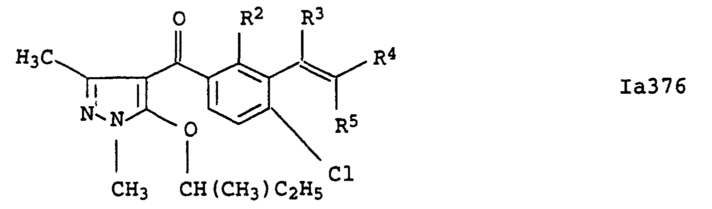
- Ebenso außergewöhnlich bevorzugt sind die Verbindungen Ia377, insbesondere die Verbindungen Ia377.001-Ia377.180, die sich von den entsprechenden Verbindungen Ia1.001-Ia1.180 dadurch unterscheiden, daß R¹⁴ für Ethyl, R¹⁵ für sec-Butyl und R¹⁶ für Methyl stehen: 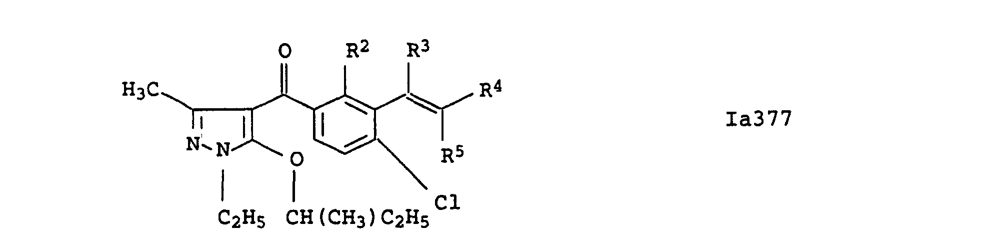
- Ebenso außergewöhnlich bevorzugt sind die Verbindungen Ia378, insbesondere die Verbindungen Ia378.001-Ia378.180, die sich von den entsprechenden Verbindungen Ia1.001-Ia1.180 dadurch unterscheiden, daß R¹⁴ für n-Propyl, R¹⁵ für sec-Butyl und R¹⁶ für Methyl stehen: 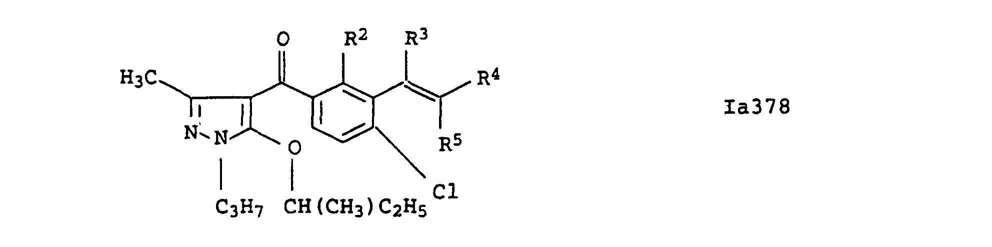
- Ebenso außergewöhnlich bevorzugt sind die Verbindungen Ia379, insbesondere die Verbindungen Ia379.001-Ia379.180, die sich von den entsprechenden Verbindungen Ia1.001-Ia1.180 dadurch unterscheiden, daß R¹⁴ für n-Butyl, R¹⁵ für sec-Butyl und R¹⁶ für Methyl stehen: 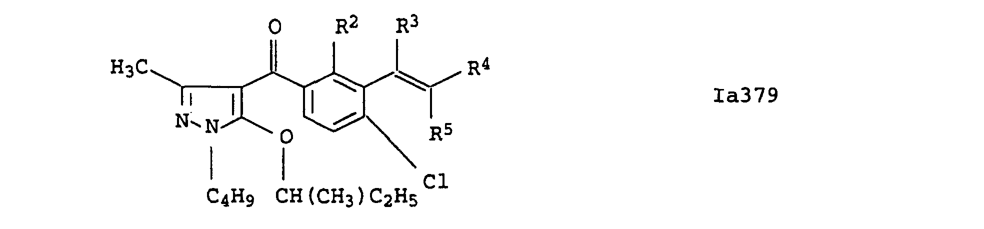
- Ebenso außergewöhnlich bevorzugt sind die Verbindungen Ia380, insbesondere die Verbindungen Ia380.001-Ia380.180, die sich von den entsprechenden Verbindungen Ia1.001-Ia1.180 dadurch unterscheiden, daß R¹⁴ für iso-Butyl, R¹⁵ für sec-Butyl und R¹⁶ für Methyl stehen: 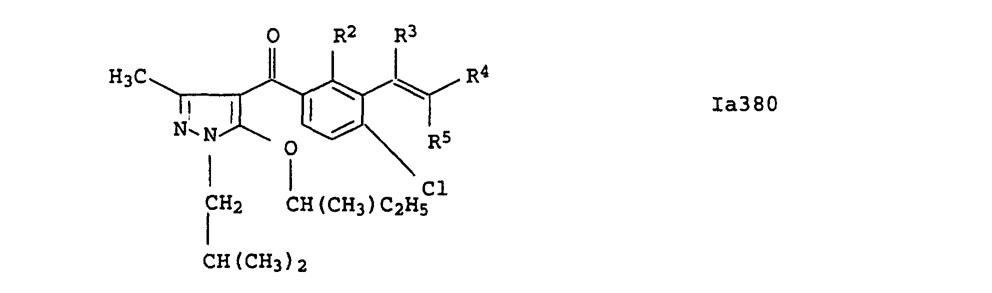
- Ebenso außergewöhnlich bevorzugt sind die Verbindungen Ia381, insbesondere die Verbindungen Ia381.001-Ia381.180, die sich von den entsprechenden Verbindungen Ia1.001-Ia1.180 dadurch unterscheiden, daß R¹⁵ für iso-Butyl und R¹⁶ für Methyl stehen: 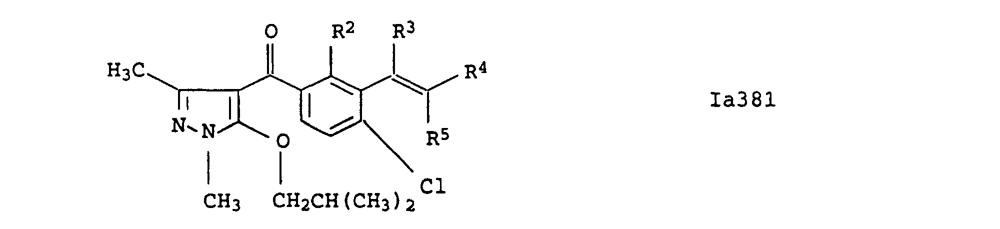
- Ebenso außergewöhnlich bevorzugt sind die Verbindungen Ia382, insbesondere die Verbindungen Ia382.001-Ia382.180, die sich von den entsprechenden Verbindungen Ia1.001-Ia1.180 dadurch unterscheiden, daß R¹⁴ für Ethyl, R¹⁵ für iso-Butyl und R¹⁶ für Methyl stehen: 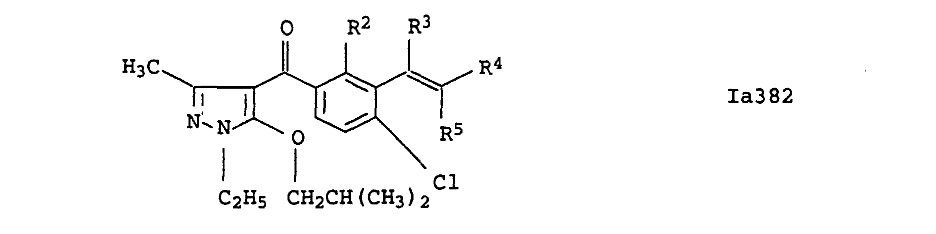
- Ebenso außergewöhnlich bevorzugt sind die Verbindungen Ia383, insbesondere die Verbindungen Ia383.001-Ia383.180, die sich von den entsprechenden Verbindungen Ia1.001-Ia1.180 dadurch unterscheiden, daß R¹⁴ für n-Propyl, R¹⁵ für iso-Butyl und R¹⁶ für Methyl stehen: 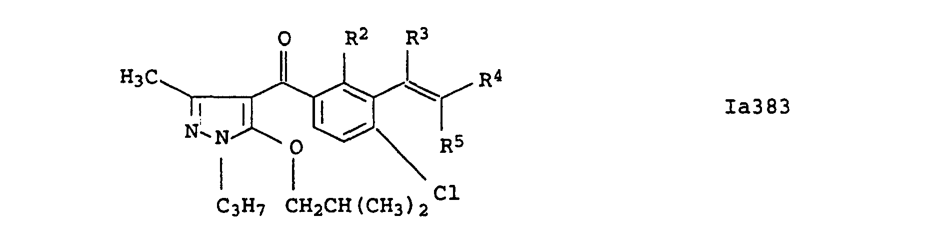
- Ebenso außergewöhnlich bevorzugt sind die Verbindungen Ia384, insbesondere die Verbindungen Ia384.001-Ia384.180, die sich von den entsprechenden Verbindungen Ia1.001-Ia1.180 dadurch unterscheiden, daß R¹⁴ für n-Butyl, R¹⁵ für iso-Butyl und R¹⁶ für Methyl stehen: 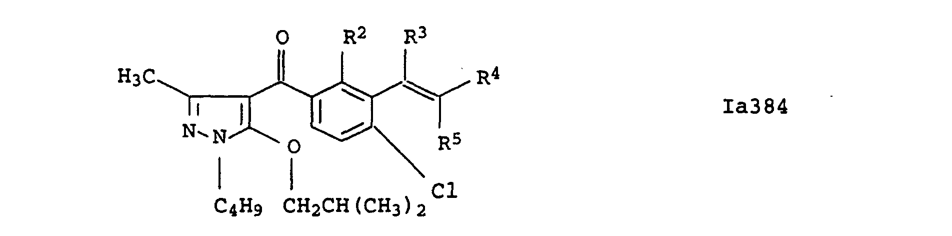
- Ebenso außergewöhnlich bevorzugt sind die Verbindungen Ia385, insbesondere die Verbindungen Ia385.001-Ia385.180, die sich von den entsprechenden Verbindungen Ia1.001-Ia1.180 dadurch unterscheiden, daß R¹⁴ und R¹⁵ für iso-Butyl und R¹⁶ für Methyl stehen: 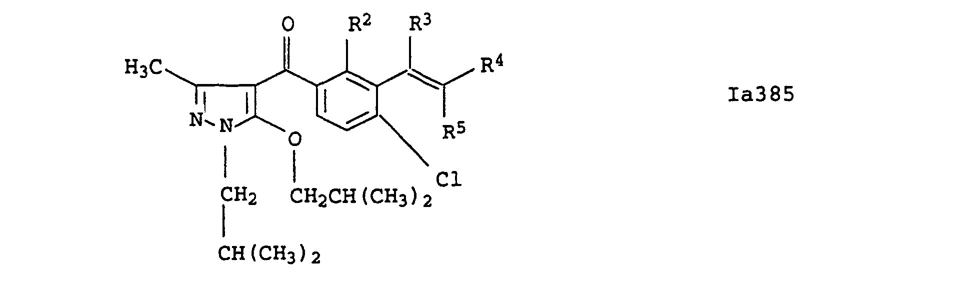
- Ebenso außergewöhnlich bevorzugt sind die Verbindungen Ia386, insbesondere die Verbindungen Ia386.001-Ia386.180, die sich von den entsprechenden Verbindungen Ia1.001-Ia1.180 dadurch unterscheiden, daß R¹⁵ für Methylcarbonyl und R¹⁶ für Methyl stehen: 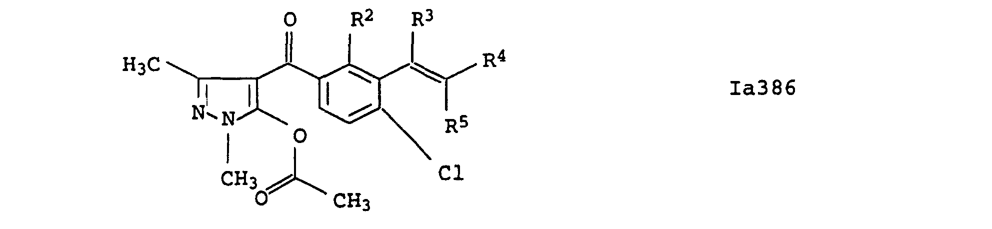
- Ebenso außergewöhnlich bevorzugt sind die Verbindungen Ia387, insbesondere die Verbindungen Ia387.001-Ia387.180, die sich von den entsprechenden Verbindungen Ia1.001-Ia1.180 dadurch unterscheiden, daß R¹⁴ für Ethyl, R¹⁵ für Methylcarbonyl und R¹⁶ für Methyl stehen: 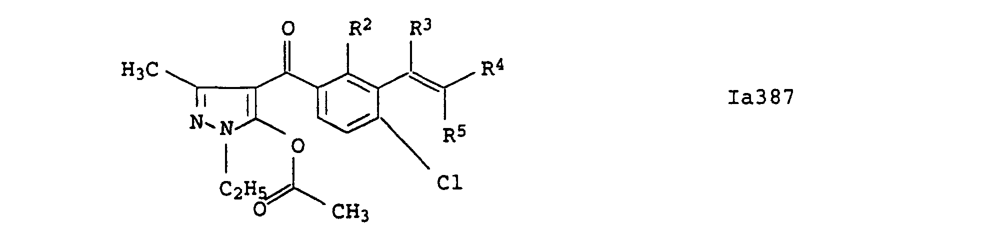
- Ebenso außergewöhnlich bevorzugt sind die Verbindungen Ia388, insbesondere die Verbindungen Ia388.001-Ia388.180, die sich von den entsprechenden Verbindungen Ia1.001-Ia1.180 dadurch unterscheiden, daß R¹⁴ für n-Propyl, R¹⁵ für Methylcarbonyl und R¹⁶ für Methyl stehen: 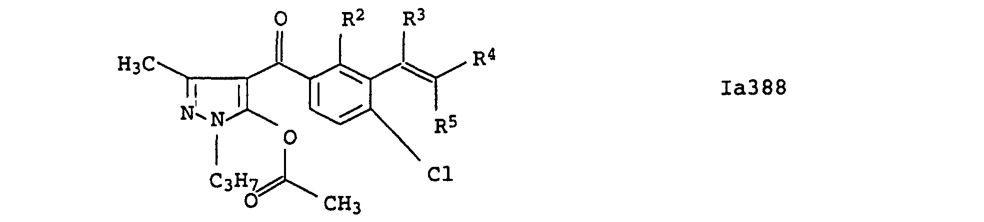
- Ebenso außergewöhnlich bevorzugt sind die Verbindungen Ia389, insbesondere die Verbindungen Ia389.001-Ia389.180, die sich von den entsprechenden Verbindungen Ia1.001-Ia1.180 dadurch unterscheiden, daß R¹⁴ für n-Butyl, R¹⁵ für Methylcarbonyl und R¹⁶ für Methyl stehen: 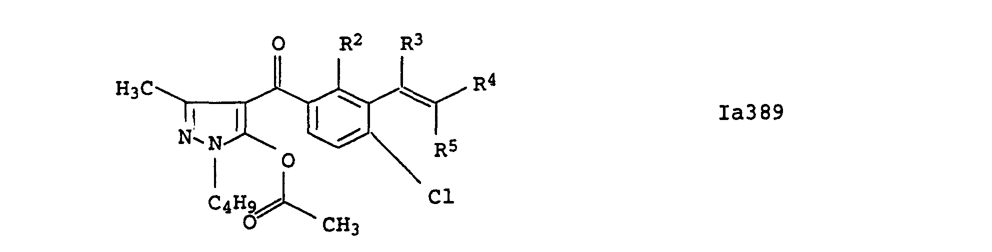
- Ebenso außergewöhnlich bevorzugt sind die Verbindungen Ia390, insbesondere die Verbindungen Ia390.001-Ia390.180, die sich von den entsprechenden Verbindungen Ia1.001-Ia1.180 dadurch unterscheiden, daß R¹⁴ für iso-Butyl, R¹⁵ für Methylcarbonyl und R¹⁶ für Methyl stehen: 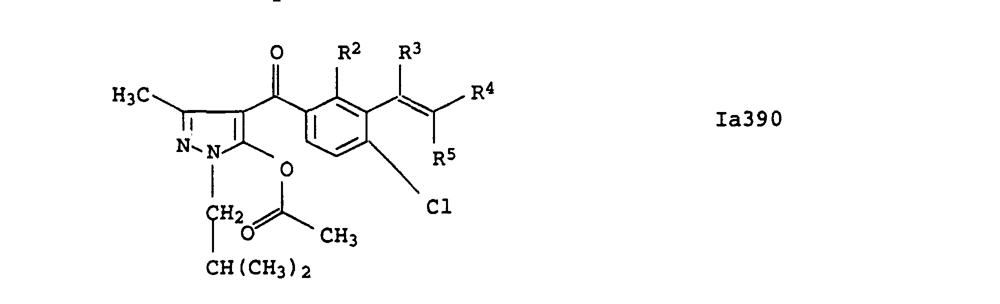
- Ebenso außergewöhnlich bevorzugt sind die Verbindungen Ia391, insbesondere die Verbindungen Ia391.001-Ia391.180, die sich von den entsprechenden Verbindungen Ia1.001-Ia1.180 dadurch unterscheiden, daß R¹⁴ für Ethylcarbonyl und R¹⁶ für Methyl stehen: 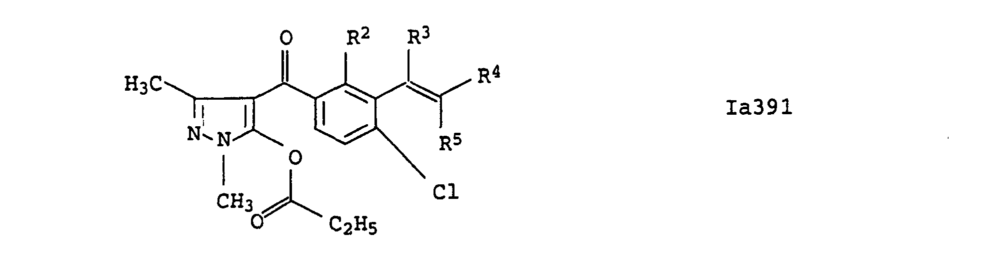
- Ebenso außergewöhnlich bevorzugt sind die Verbindungen Ia392, insbesondere die Verbindungen Ia392.001-Ia392.180, die sich von den entsprechenden Verbindungen Ia1.001-Ia1.180 dadurch unterscheiden, daß R¹⁴ für Ethyl, R¹⁵ für Ethylcarbonyl und R¹⁶ für Methyl stehen: 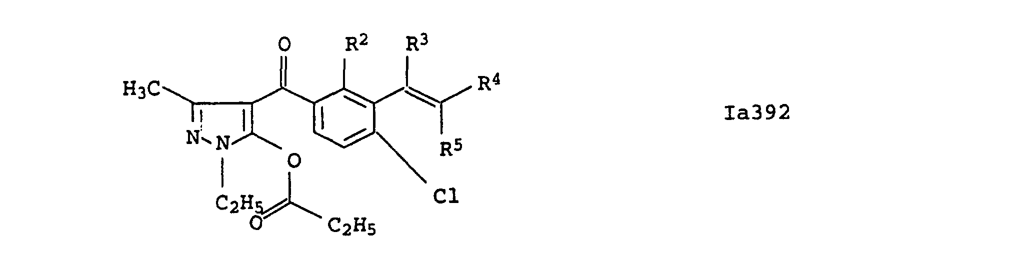
- Ebenso außergewöhnlich bevorzugt sind die Verbindungen Ia393, insbesondere die Verbindungen Ia393.001-Ia393.180, die sich von den entsprechenden Verbindungen Ia1.001-Ia1.180 dadurch unterscheiden, daß R¹⁴ für n-Propyl, R¹⁵ für Ethyl und R¹⁶ für Methyl stehen: 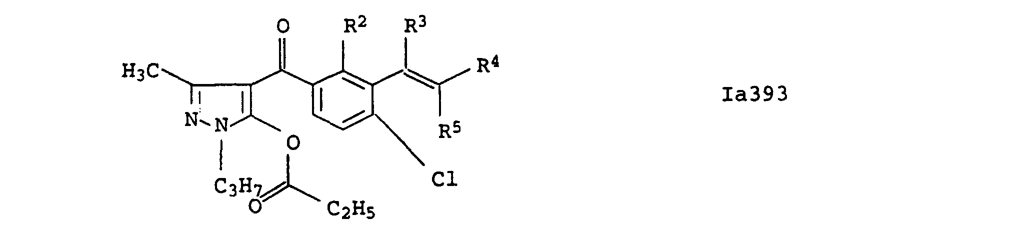
- Ebenso außergewöhnlich bevorzugt sind die Verbindungen Ia394, insbesondere die Verbindungen Ia394.001-Ia394.180, die sich von den entsprechenden Verbindungen Ia1.001-Ia1.180 dadurch unterscheiden, daß R¹⁴ für n-Butyl, R¹⁵ für Ethylcarbonyl und R¹⁶ für Methyl stehen: 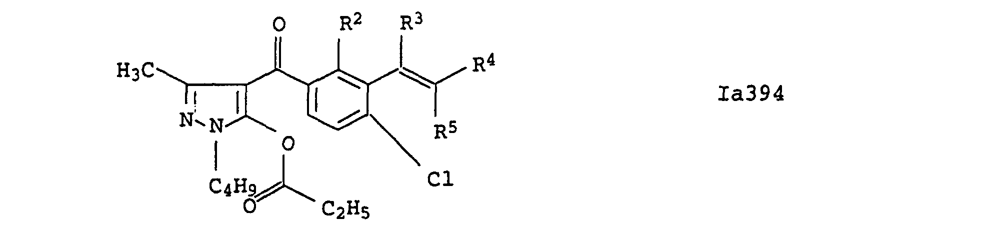
- Ebenso außergewöhnlich bevorzugt sind die Verbindungen Ia395, insbesondere die Verbindungen Ia395.001-Ia395.180, die sich von den entsprechenden Verbindungen Ia1.001-Ia1.180 dadurch unterscheiden, daß R¹⁴ für iso-Butyl, R¹⁵ für Ethylcarbonyl und R¹⁶ für Methyl stehen: 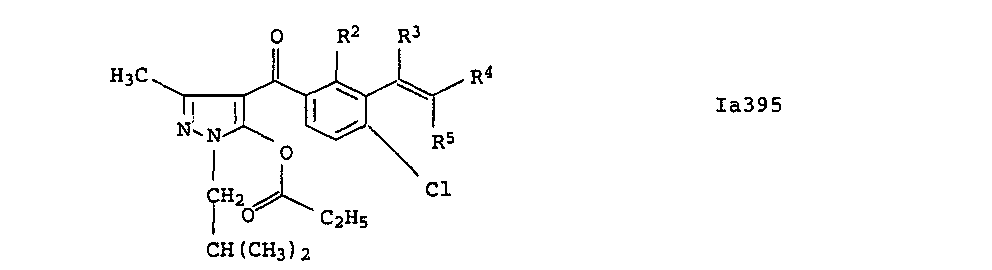
- Ebenso außergewöhnlich bevorzugt sind die Verbindungen Ia396, insbesondere die Verbindungen Ia396.001-Ia396.180, die sich von den entsprechenden Verbindungen Ia1.001-Ia1.180 dadurch unterscheiden, daß R¹⁵ für Phenylsulfonyl und R¹⁶ für Methyl stehen: 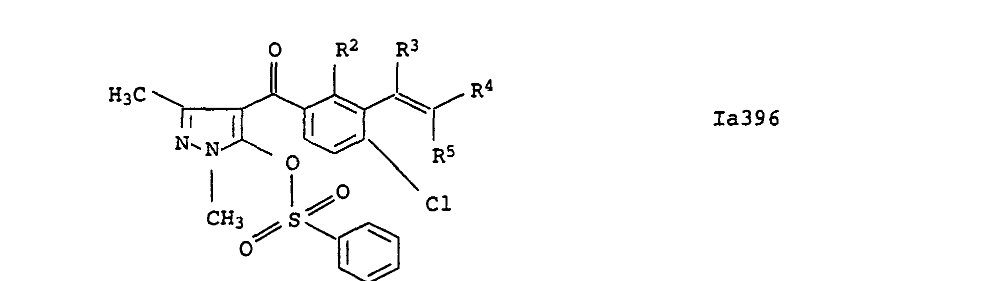
- Ebenso außergewöhnlich bevorzugt sind die Verbindungen Ia397, insbesondere die Verbindungen Ia397.001-Ia397.180, die sich von den entsprechenden Verbindungen Ia1.001-Ia1.180 dadurch unterscheiden, daß R¹⁴ für Ethyl, R¹⁵ für Phenylsulfonyl und R¹⁶ für Methyl stehen: 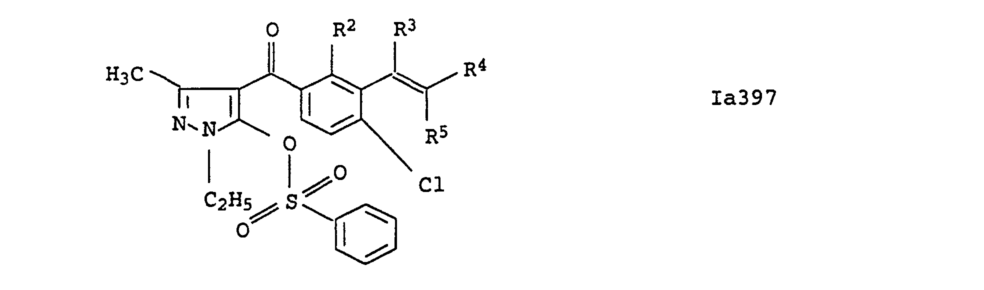
- Ebenso außergewöhnlich bevorzugt sind die Verbindungen Ia398, insbesondere die Verbindungen Ia398.001-Ia398.180, die sich von den entsprechenden Verbindungen Ia1.001-Ia1.180 dadurch unterscheiden, daß R¹⁴ für n-Propyl, R¹⁵ für Phenylsulfonyl und R¹⁶ für Methyl stehen: 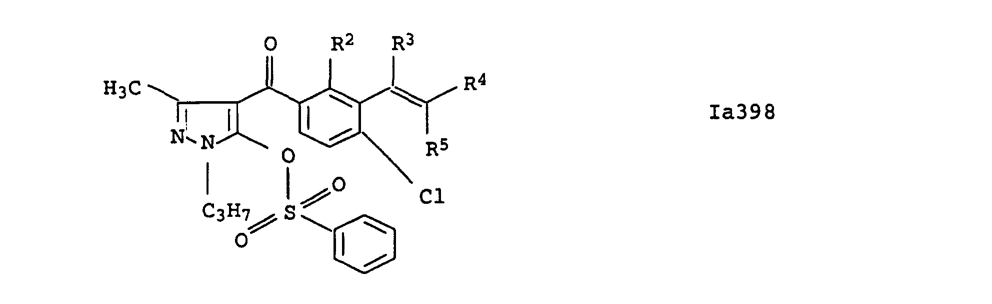
- Ebenso außergewöhnlich bevorzugt sind die Verbindungen Ia399, insbesondere die Verbindungen Ia399.001-Ia399.180, die sich von den entsprechenden Verbindungen Ia1.001-Ia1.180 dadurch unterscheiden, daß R¹⁴ für n-Butyl, R¹⁵ für Phenylsulfonyl und R¹⁶ für Methyl stehen: 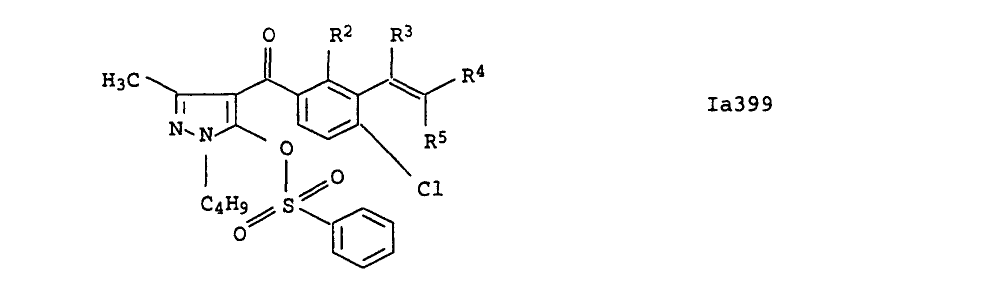
- Ebenso außergewöhnlich bevorzugt sind die Verbindungen Ia400, insbesondere die Verbindungen Ia400.001-Ia400.180, die sich von den entsprechenden Verbindungen Ia1.001-Ia1.180 dadurch unterscheiden, daß R¹⁴ für iso-Butyl, R¹⁵ für Phenylsulfonyl und R¹⁶ für Methyl stehen: 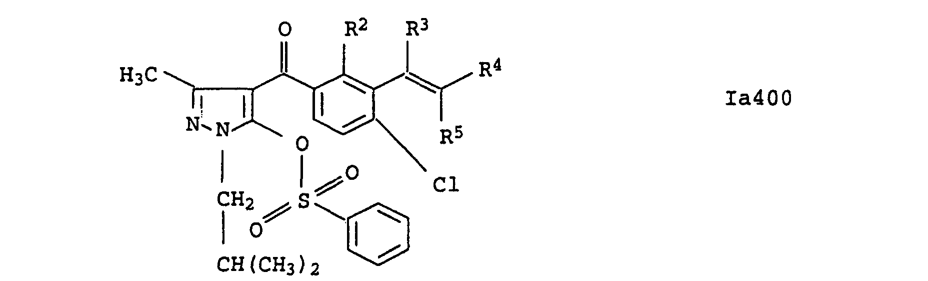
- Ebenso außergewöhnlich bevorzugt sind die Verbindungen Ia401, insbesondere die Verbindungen Ia401.001-Ia401.180, die sich von den entsprechenden Verbindungen Ia1.001-Ia1.180 dadurch unterscheiden, daß R¹⁵ für 4-Methylphenylsulfonyl und R¹⁶ für Methyl stehen: 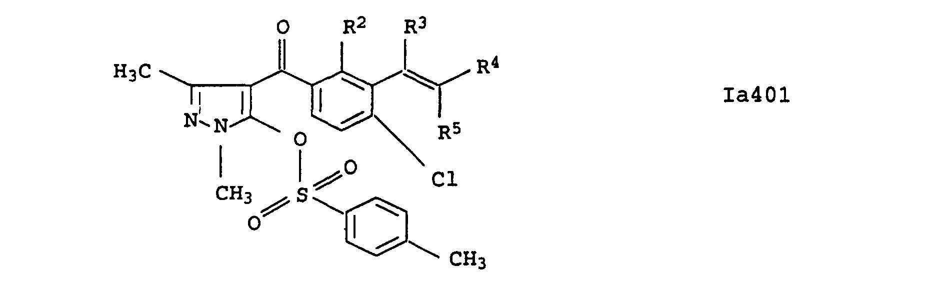
- Ebenso außergewöhnlich bevorzugt sind die Verbindungen Ia402, insbesondere die Verbindungen Ia402.001-Ia402.180, die sich von den entsprechenden Verbindungen Ia1.001-Ia1.180 dadurch unterscheiden, daß R¹⁴ für Ethyl, R¹⁵ für 4-Methylphenylsulfonyl und R¹⁶ für Methyl stehen: 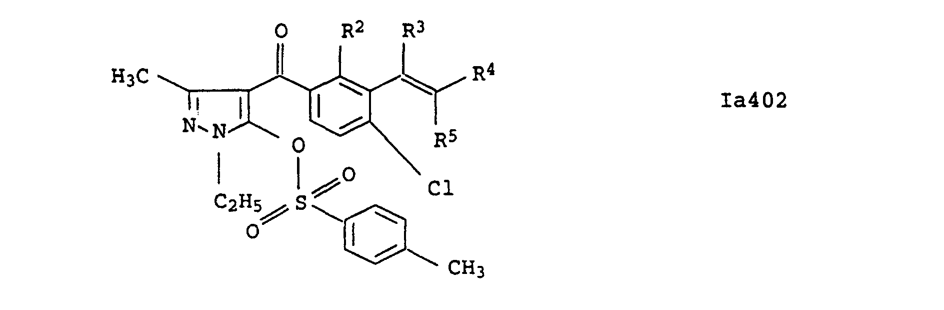
- Ebenso außergewöhnlich bevorzugt sind die Verbindungen Ia403, insbesondere die Verbindungen Ia403.001-Ia403.180, die sich von den entsprechenden Verbindungen Ia1.001-Ia1.180 dadurch unterscheiden, daß R¹⁴ für n-Propyl, R¹⁵ für 4-Methylphenylsulfonyl und R¹⁶ für Methyl stehen: 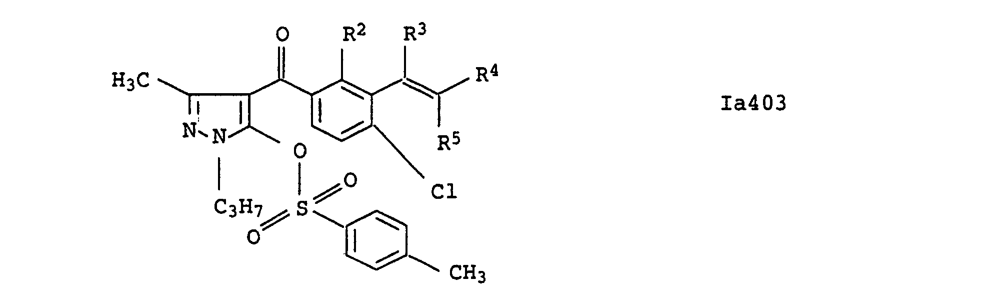
- Ebenso außergewöhnlich bevorzugt sind die Verbindungen Ia404, insbesondere die Verbindungen Ia404.001-Ia404.180, die sich von den entsprechenden Verbindungen Ia1.001-Ia1.180 dadurch unterscheiden, daß R¹⁴ für n-Butyl, R¹⁵ für 4-Methylphenylsulfonyl und R¹⁶ für Methyl stehen: 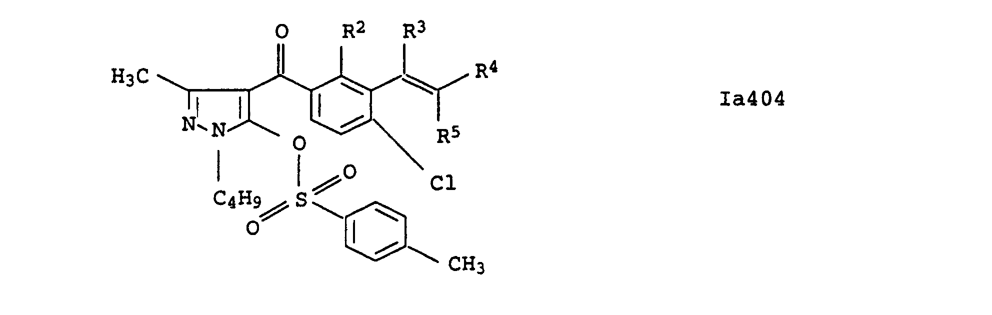
- Ebenso außergewöhnlich bevorzugt sind die Verbindungen Ia405, insbesondere die Verbindungen Ia405.001-Ia405.180, die sich von den entsprechenden Verbindungen Ia1.001-Ia1.180 dadurch unterscheiden, daß R¹⁴ für iso-Butyl, R¹⁵ für 4-Methylphenylsulfonyl und R¹⁶ für Methyl stehen: 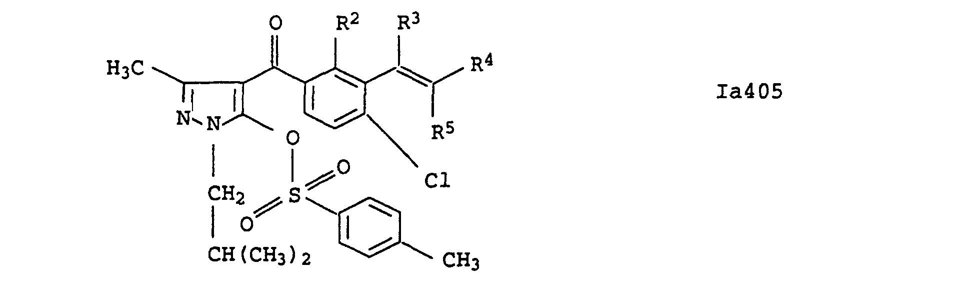
- Ebenso außergewöhnlich bevorzugt sind die Verbindungen Ia406, insbesondere die Verbindungen Ia406.001-Ia406.180, die sich von den entsprechenden Verbindungen Ia1.001-Ia1.180 dadurch unterscheiden, daß R¹⁵ für n-Propylcarbonyl und R¹⁶ für Methyl stehen: 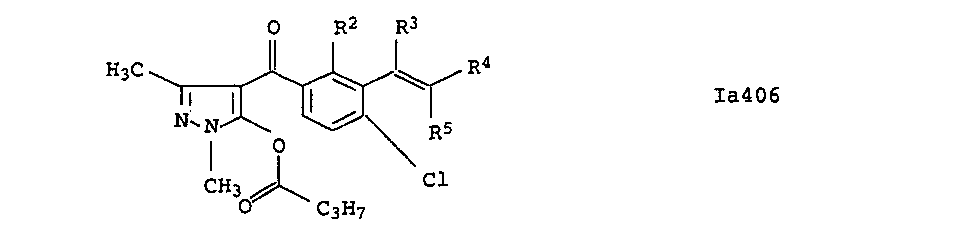
- Ebenso außergewöhnlich bevorzugt sind die Verbindungen Ia407, insbesondere die Verbindungen Ia407.001-Ia407.180, die sich von den entsprechenden Verbindungen Ia1.001-Ia1.180 dadurch unterscheiden, daß R¹⁴ für Ethyl, R¹⁵ für n-Propylcarbonyl und R¹⁶ für Methyl stehen: 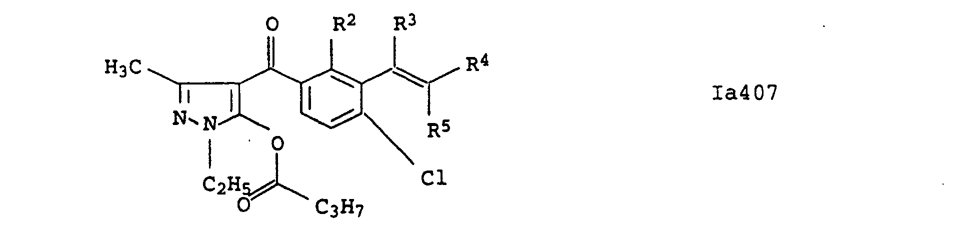
- Ebenso außergewöhnlich bevorzugt sind die Verbindungen Ia408, insbesondere die Verbindungen Ia408.001-Ia408.180, die sich von den entsprechenden Verbindungen Ia1.001-Ia1.180 dadurch unterscheiden, daß R¹⁴ für n-Propyl, R¹⁵ für n-Propylcarbonyl und R¹⁶ für Methyl stehen: 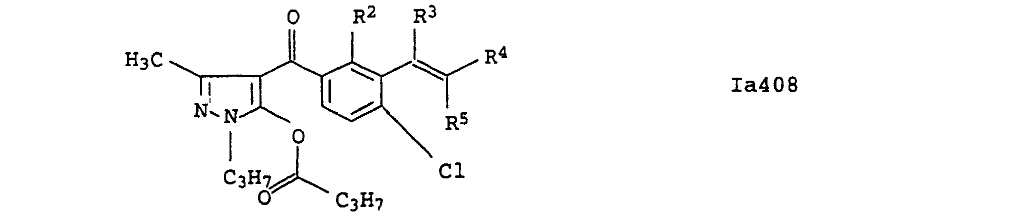
- Ebenso außergewöhnlich bevorzugt sind die Verbindungen Ia409, insbesondere die Verbindungen Ia409.001-Ia409.180, die sich von den entsprechenden Verbindungen Ia1.001-Ia1.180 dadurch unterscheiden, daß R¹⁴ für n-Butyl, R¹⁵ für n-Propylcarbonyl und R¹⁶ für Methyl stehen: 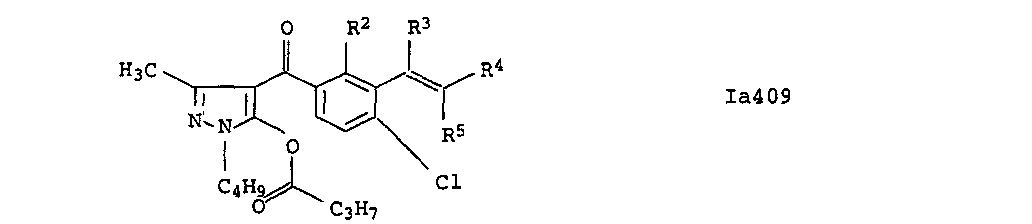
- Ebenso außergewöhnlich bevorzugt sind die Verbindungen Ia410, insbesondere die Verbindungen Ia410.001-Ia410.180, die sich von den entsprechenden Verbindungen Ia1.001-Ia1.180 dadurch unterscheiden, daß R¹⁴ für iso-Butyl, R¹⁵ für n-Propylcarbonyl und R¹⁶ für Methyl stehen: 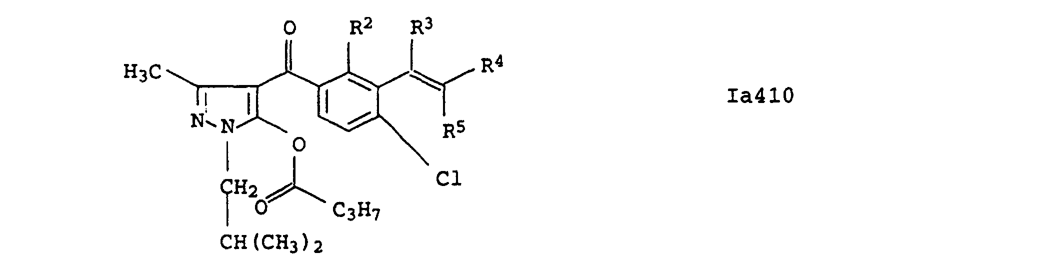
- Ebenso außergewöhnlich bevorzugt sind die Verbindungen Ia411, insbesondere die Verbindungen Ia411.001-Ia411.180, die sich von den entsprechenden Verbindungen Ia1.001-Ia1.180 dadurch unterscheiden, daß R¹⁵ für Trifluormethylcarbonyl und R¹⁶ für Methyl stehen: 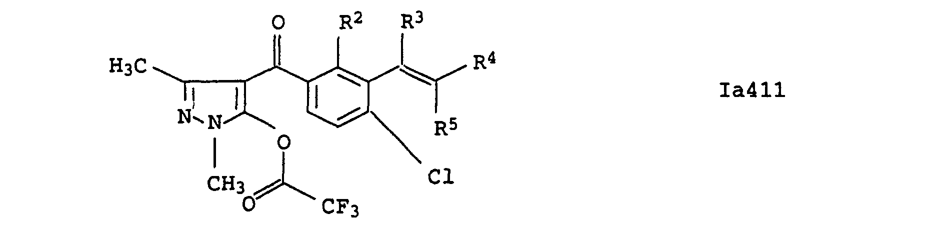
- Ebenso außergewöhnlich bevorzugt sind die Verbindungen Ia412, insbesondere die Verbindungen Ia412.001-Ia412.180, die sich von den entsprechenden Verbindungen Ia1.001-Ia1.180 dadurch unterscheiden, daß R¹⁴ für Ethyl, R¹⁵ für Trifluormethylcarbonyl und R¹⁶ für Methyl stehen: 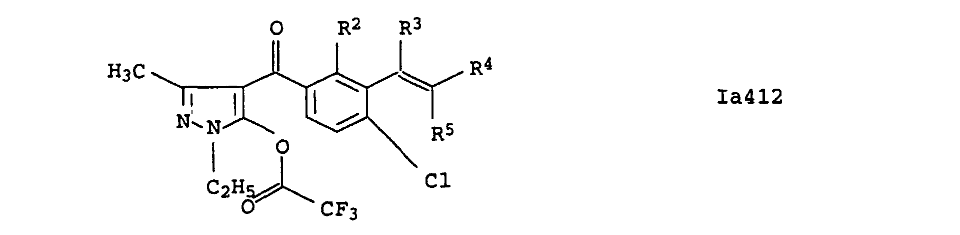
- Ebenso außergewöhnlich bevorzugt sind die Verbindungen Ia413, insbesondere die Verbindungen Ia413.001-Ia413.180, die sich von den entsprechenden Verbindungen Ia1.001-Ia1.180 dadurch unterscheiden, daß R¹⁴ für n-Propyl, R¹⁵ für Trifluormethylcarbonyl und R¹⁶ für Methyl stehen: 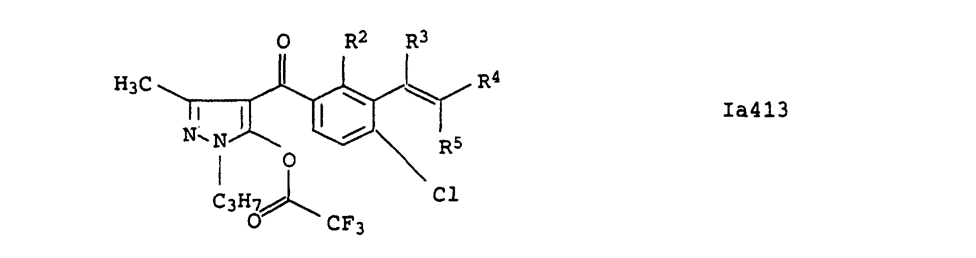
- Ebenso außergewöhnlich bevorzugt sind die Verbindungen Ia414, insbesondere die Verbindungen Ia419.001-Ia414.180, die sich von den entsprechenden Verbindungen Ia1.001-Ia1.180 dadurch unterscheiden, daß R¹⁴ für n-Butyl, R¹⁵ für Trifluormethylcarbonyl und R¹⁶ für Methyl stehen: 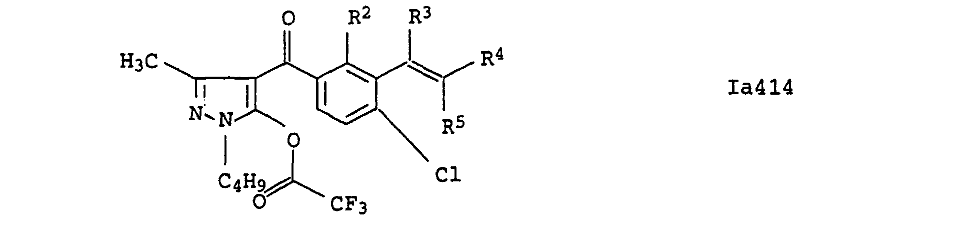
- Ebenso außergewöhnlich bevorzugt sind die Verbindungen Ia415, insbesondere die Verbindungen Ia415.001-Ia415.180, die sich von den entsprechenden Verbindungen Ia1.001-Ia1.180 dadurch unterscheiden, daß R¹⁴ für iso-Butyl, R¹⁵ für Trifluormethylcarbonyl und R¹⁶ für Methyl stehen: 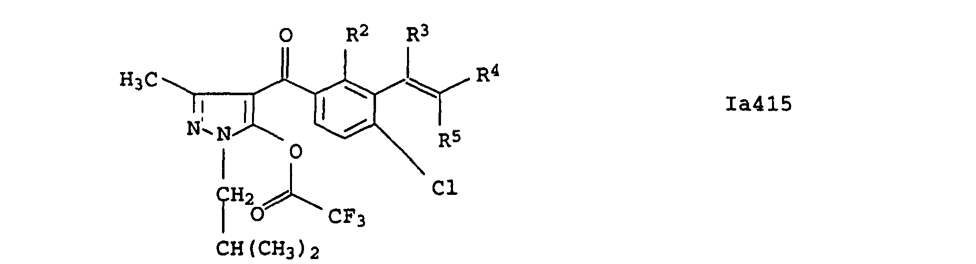
- Ebenso außergewöhnlich bevorzugt sind die Verbindungen Ia416, insbesondere die Verbindungen Ia416.001-Ia416.180, die sich von den entsprechenden Verbindungen Ia1.001-Ia1.180 dadurch unterscheiden, daß R¹⁵ für Methylsulfonyl und R¹⁶ für Methyl stehen: 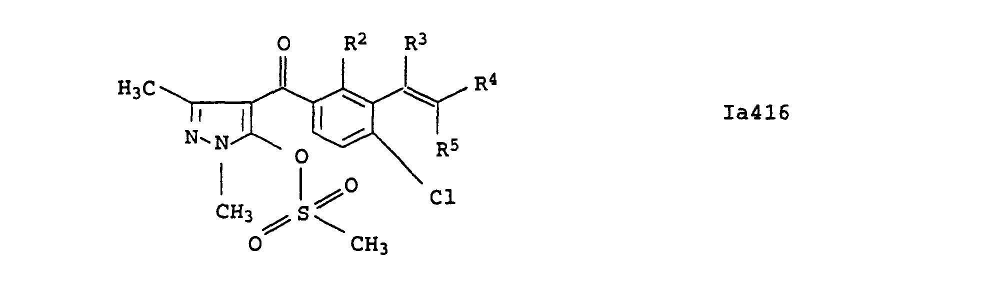
- Ebenso außergewöhnlich bevorzugt sind die Verbindungen Ia417, insbesondere die Verbindungen Ia417.001-Ia417.180, die sich von den entsprechenden Verbindungen Ia1.001-Ia1.180 dadurch unterscheiden, daß R¹⁴ für Ethyl, R¹⁵ für Methylsulfonyl und R¹⁶ für Methyl stehen: 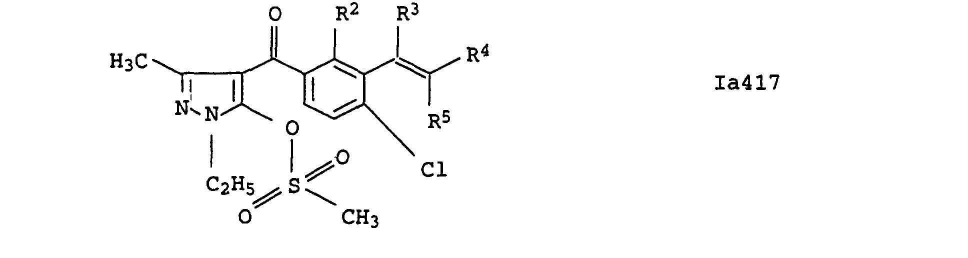
- Ebenso außergewöhnlich bevorzugt sind die Verbindungen Ia418, insbesondere die Verbindungen Ia418.001-Ia418.180, die sich von den entsprechenden Verbindungen Ia1.001-Ia1.180 dadurch unterscheiden, daß R¹⁴ für n-Propyl, R¹⁵ für Methylsulfonyl und R¹⁶ für Methyl stehen: 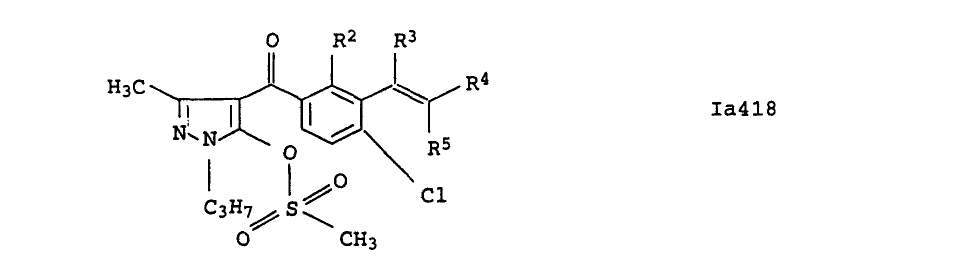
- Ebenso außergewöhnlich bevorzugt sind die Verbindungen Ia419, insbesondere die Verbindungen Ia419.001-Ia419.180, die sich von den entsprechenden Verbindungen Ia1.001-Ia1.180 dadurch unterscheiden, daß R¹⁴ für n-Butyl, R¹⁵ für Methylsulfonyl und R¹⁶ für Methyl stehen: 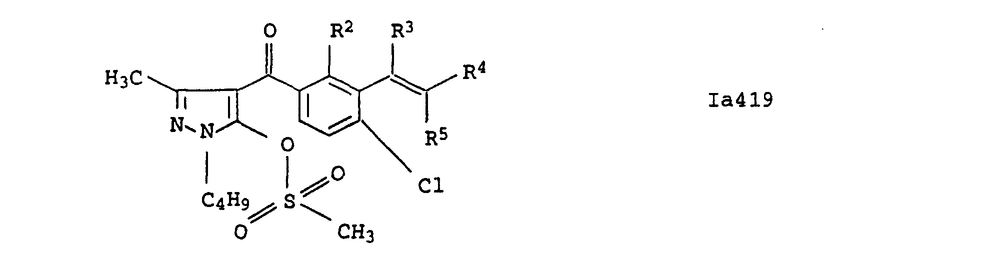
- Ebenso außergewöhnlich bevorzugt sind die Verbindungen Ia420, insbesondere die Verbindungen Ia420.001-Ia420.180, die sich von den entsprechenden Verbindungen Ia1.001-Ia1.180 dadurch unterscheiden, daß R¹⁴ für iso-Butyl, R¹⁵ für Methylsulfonyl und R¹⁶ für Methyl stehen: 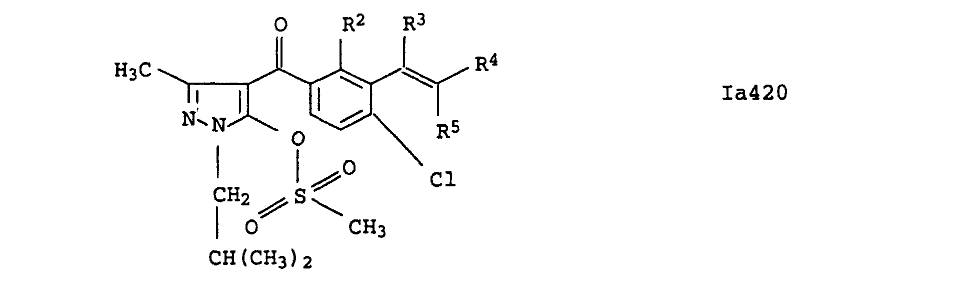
- Ebenso außergewöhnlich bevorzugt sind die Verbindungen Ia421, insbesondere die Verbindungen Ia421.001-Ia421.180, die sich von den entsprechenden Verbindungen Ia1.001-Ia1.180 dadurch unterscheiden, daß R¹⁵ für Ethylsulfonyl und R¹⁶ für Methyl stehen: 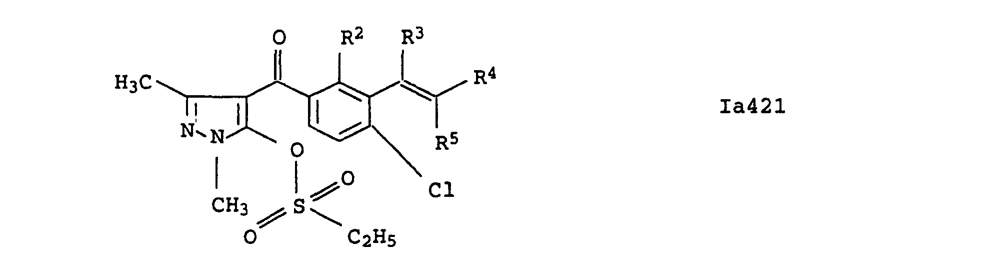
- Ebenso außergewöhnlich bevorzugt sind die Verbindungen Ia422, insbesondere die Verbindungen Ia422.001-Ia422.180, die sich von den entsprechenden Verbindungen Ia1.001-Ia1.180 dadurch unterscheiden, daß R¹⁴ für Ethyl, R¹⁵ für Ethylsulfonyl und R¹⁶ für Methyl stehen: 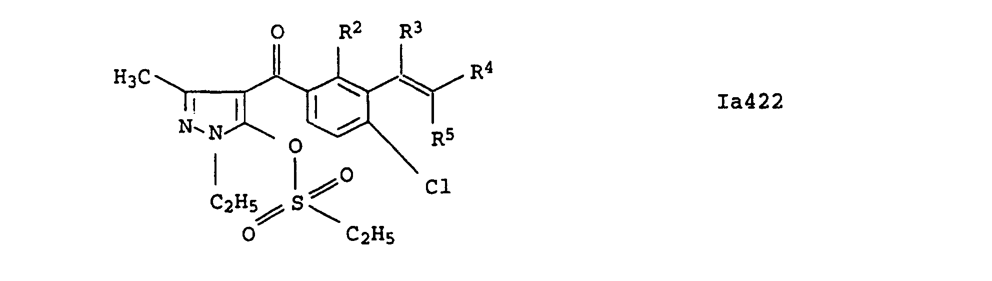
- Ebenso außergewöhnlich bevorzugt sind die Verbindungen Ia423, insbesondere die Verbindungen Ia423.001-Ia423.180, die sich von den entsprechenden Verbindungen Ia1.001-Ia1.180 dadurch unterscheiden, daß R¹⁴ für Ethyl, R¹⁵ für Ethylsulfonyl und R¹⁶ für Methyl stehen: 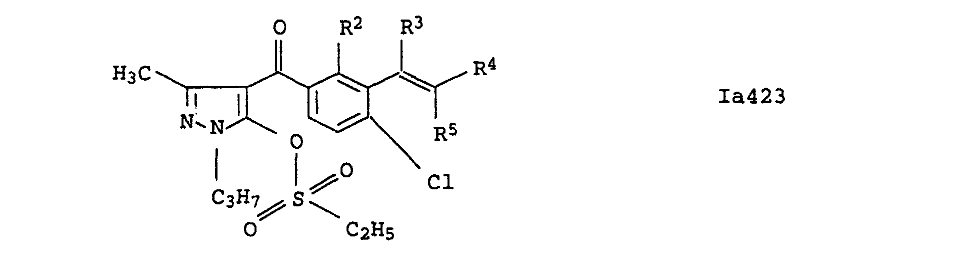
- Ebenso außergewöhnlich bevorzugt sind die Verbindungen Ia424, insbesondere die Verbindungen Ia424.001-Ia424.180, die sich von den entsprechenden Verbindungen Ia1.001-Ia1.180 dadurch unterscheiden, daß R¹⁴ für n-Butyl, R¹⁵ für Ethylsulfonyl und R¹⁶ für Methyl stehen: 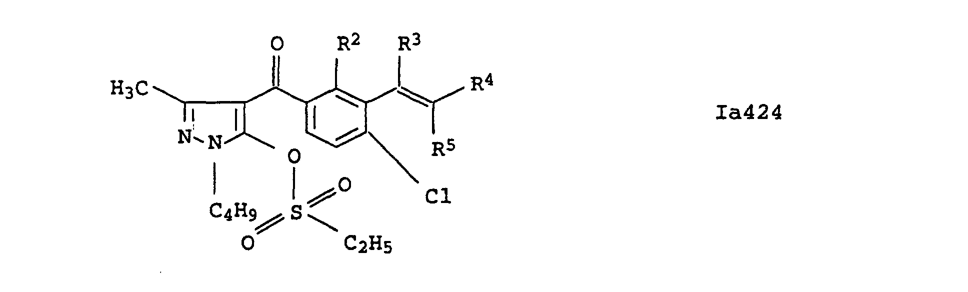
- Ebenso außergewöhnlich bevorzugt sind die Verbindungen Ia425, insbesondere die Verbindungen Ia425.001-Ia425.180, die sich von den entsprechenden Verbindungen Ia1.001-Ia1.180 dadurch unterscheiden, daß R¹⁴ für iso-Butyl, R¹⁵ für Ethylsulfonyl und R¹⁶ für Methyl stehen: 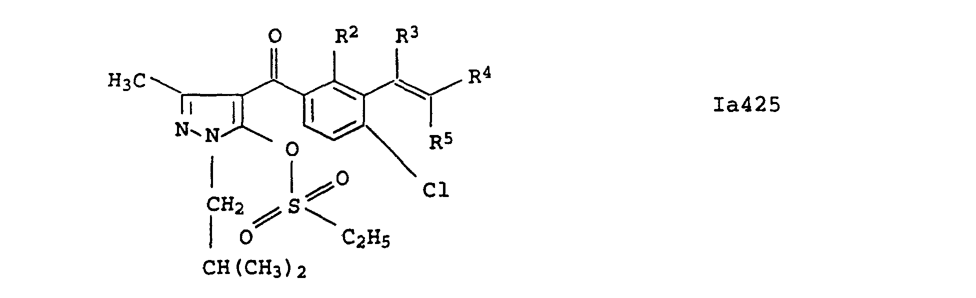
- Ebenso außergewöhnlich bevorzugt sind die Verbindungen Ia426, insbesondere die Verbindungen Ia426.001-Ia426.180, die sich von den entsprechenden Verbindungen Ia1.001-Ia1.180 dadurch unterscheiden, daß R¹⁵ für n-Propylsulfonyl und R¹⁶ für Methyl stehen: 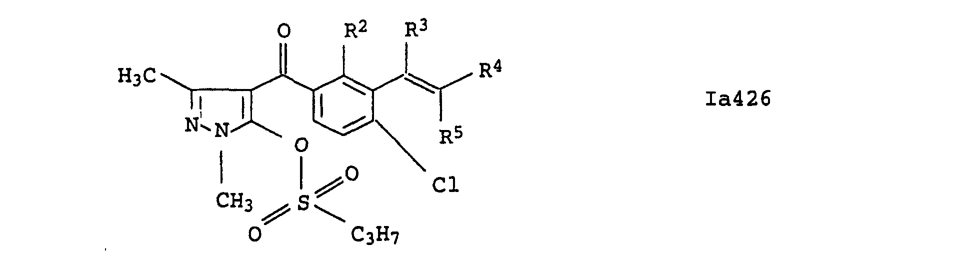
- Ebenso außergewöhnlich bevorzugt sind die Verbindungen Ia427, insbesondere die Verbindungen Ia427.001-Ia427.180, die sich von den entsprechenden Verbindungen Ia1.001-Ia1.180 dadurch unterscheiden, daß R¹⁴ für Ethyl, R¹⁵ für n-Propylsulfonyl und R¹⁶ für Methyl stehen: 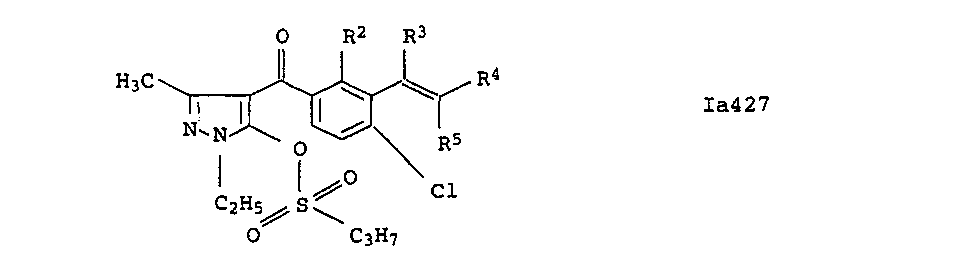
- Ebenso außergewöhnlich bevorzugt sind die Verbindungen Ia428, insbesondere die Verbindungen Ia428.001-Ia428.180, die sich von den entsprechenden Verbindungen Ia1.001-Ia1.180 dadurch unterscheiden, daß R¹⁴ für n-Propyl, R¹⁵ für n-Propylsulfonyl und R¹⁶ für Methyl stehen: 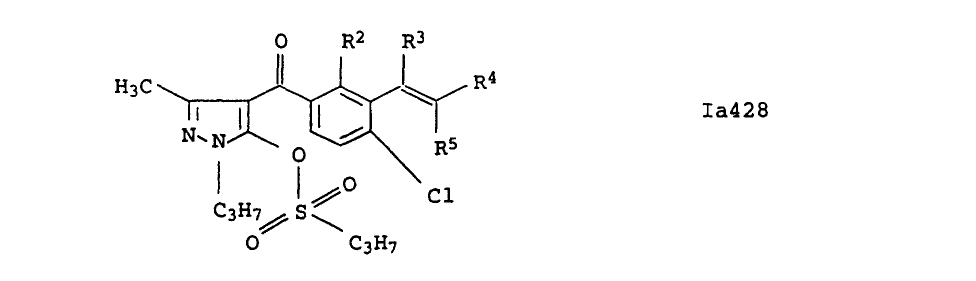
- Ebenso außergewöhnlich bevorzugt sind die Verbindungen Ia429, insbesondere die Verbindungen Ia429.001-Ia429.180, die sich von den entsprechenden Verbindungen Ia1.001-Ia1.180 dadurch unterscheiden, daß R¹⁴ für n-Butyl, R¹⁵ für n-Propylsulfonyl und R¹⁶ für Methyl stehen: 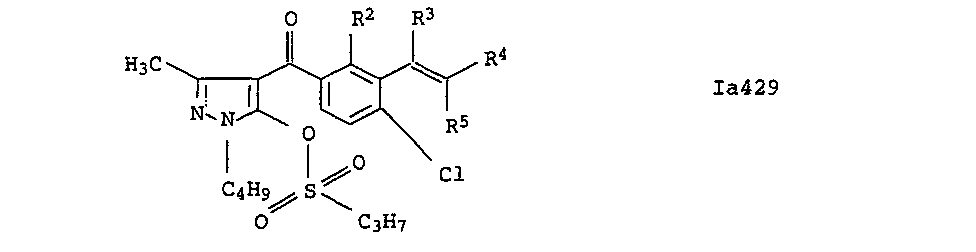
- Ebenso außergewöhnlich bevorzugt sind die Verbindungen Ia430, insbesondere die Verbindungen Ia430.001-Ia430.180, die sich von den entsprechenden Verbindungen Ia1.001-Ia1.180 dadurch unterscheiden, daß R¹⁴ für iso-Butyl, R¹⁵ für Propylsulfonyl und R¹⁶ für Methyl stehen: 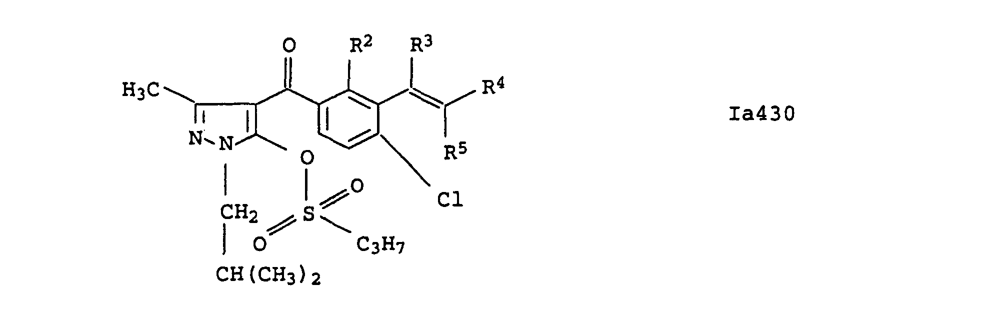
- Ebenso außergewöhnlich bevorzugt sind die Verbindungen Ia431, insbesondere die Verbindungen Ia431.001-Ia431.180, die sich von den entsprechenden Verbindungen Ia1.001-Ia1.180 dadurch unterscheiden, daß R¹⁵ für iso-Propylsulfonyl und R¹⁶ für Methyl stehen: 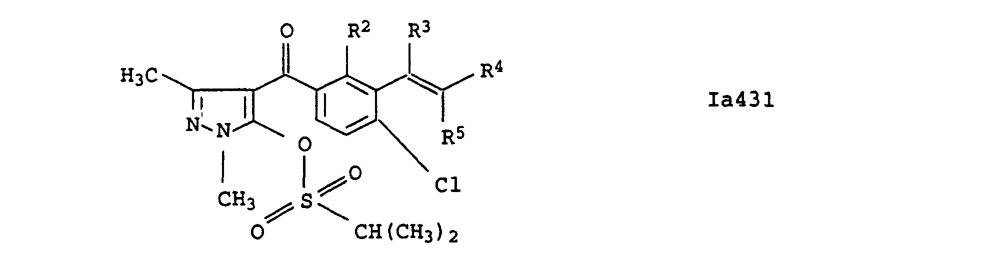
- Ebenso außergewöhnlich bevorzugt sind die Verbindungen Ia432, insbesondere die Verbindungen Ia432.001-Ia432.180, die sich von den entsprechenden Verbindungen Ia1.001-Ia1.180 dadurch unterscheiden, daß R¹⁴ für Ethyl, R¹⁵ für iso-Propylsulfonyl und R¹⁶ für Methyl stehen: 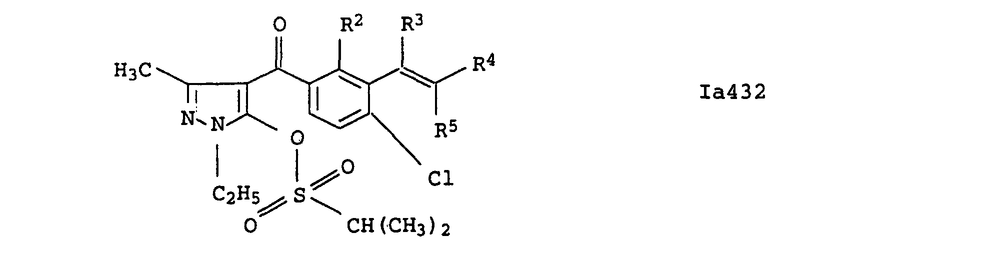
- Ebenso außergewöhnlich bevorzugt sind die Verbindungen Ia433, insbesondere die Verbindungen Ia433.001-Ia433.180, die sich von den entsprechenden Verbindungen Ia1.001-Ia1.180 dadurch unterscheiden, daß R¹⁴ für n-Propyl, R¹⁵ für iso-Propylsulfonyl und R¹⁶ für Methyl stehen: 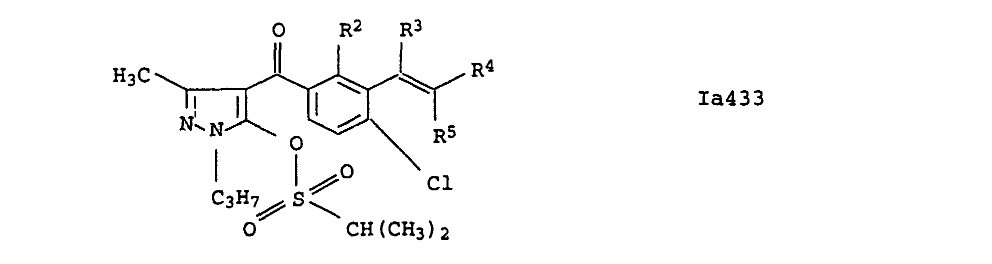
- Ebenso außergewöhnlich bevorzugt sind die Verbindungen Ia434, insbesondere die Verbindungen Ia434.001-Ia434.180, die sich von den entsprechenden Verbindungen Ia1.001-Ia1.180 dadurch unterscheiden, daß R¹⁴ für n-Butyl, R¹⁵ für iso-Propylsulfonyl und R¹⁶ für Methyl stehen: 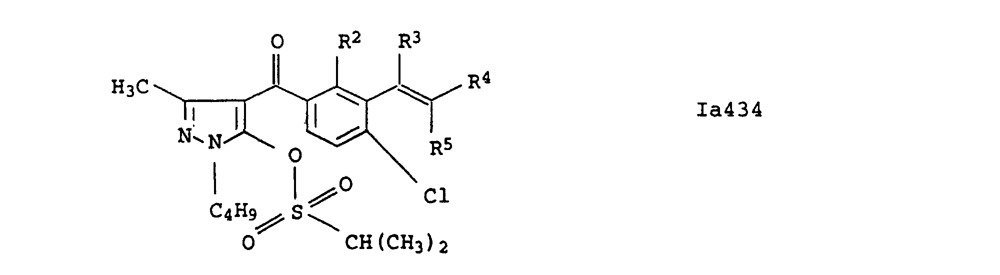
- Ebenso außergewöhnlich bevorzugt sind die Verbindungen Ia435, insbesondere die Verbindungen Ia435.001-Ia435.180, die sich von den entsprechenden Verbindungen Ia1.001-Ia1.180 dadurch unterscheiden, daß R¹⁴ für iso-Butyl, R¹⁵ für iso-Propylsulfonyl und R¹⁶ für Methyl stehen: 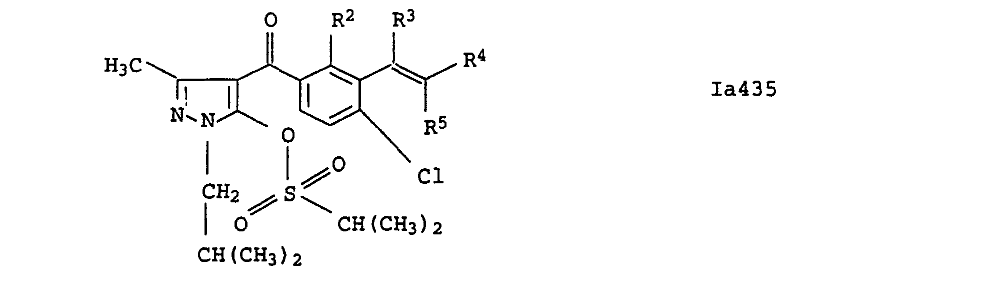
- Ebenso außergewöhnlich bevorzugt sind die Verbindungen Ia436, insbesondere die Verbindungen Ia436.001-Ia436.180, die sich von den entsprechenden Verbindungen Ia1.001-Ia1.180 dadurch unterscheiden, daß R¹⁵ für n-Butylsulfonyl und R¹⁶ für Methyl stehen: 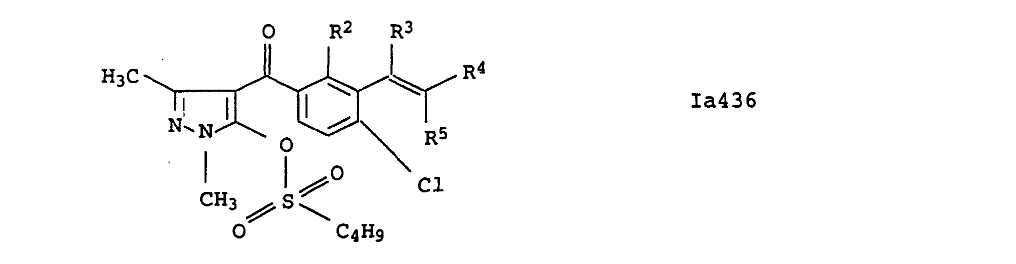
- Ebenso außergewöhnlich bevorzugt sind die Verbindungen Ia437, insbesondere die Verbindungen Ia437.001-Ia437.180, die sich von den entsprechenden Verbindungen Ia1.001-Ia1.180 dadurch unterscheiden, daß R¹⁴ für Ethyl, R¹⁵ für n-Butylsulfonyl und R¹⁶ für Methyl stehen: 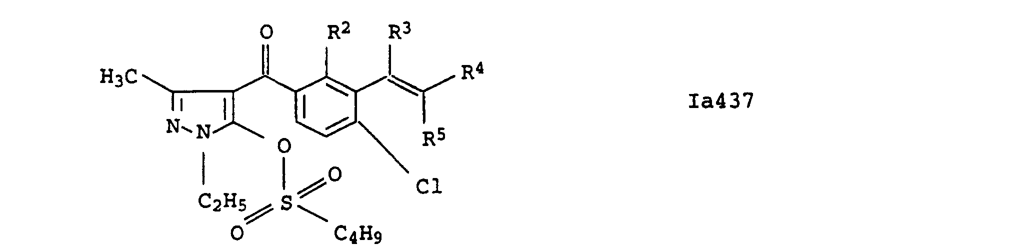
- Ebenso außergewöhnlich bevorzugt sind die Verbindungen Ia438, insbesondere die Verbindungen Ia438.001-Ia438.180, die sich von den entsprechenden Verbindungen Ia1.001-Ia1.180 dadurch unterscheiden, daß R¹⁴ für n-Propyl, R¹⁵ für n-Butylsulfonyl und R¹⁶ für Methyl stehen: 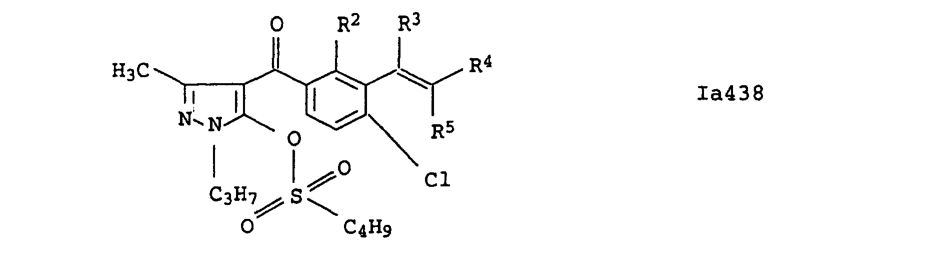
- Ebenso außergewöhnlich bevorzugt sind die Verbindungen Ia439, insbesondere die Verbindungen Ia439.001-Ia439.180, die sich von den entsprechenden Verbindungen Ia1.001-Ia1.180 dadurch unterscheiden, daß R¹⁴ für n-Butyl, R¹⁵ für n-Butylsulfonyl und R¹⁶ für Methyl stehen: 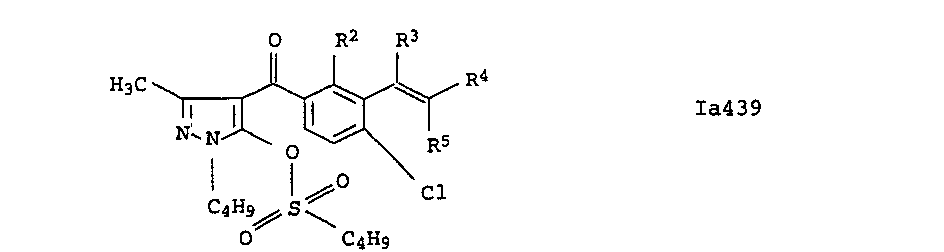
- Ebenso außergewöhnlich bevorzugt sind die Verbindungen Ia440, insbesondere die Verbindungen Ia440.001-Ia440.180, die sich von den entsprechenden Verbindungen Ia1.001-Ia1.180 dadurch unterscheiden, daß R¹⁴ für iso-Butyl, R¹⁵ für n-Butylsulfonyl und R¹⁶ für Methyl stehen: 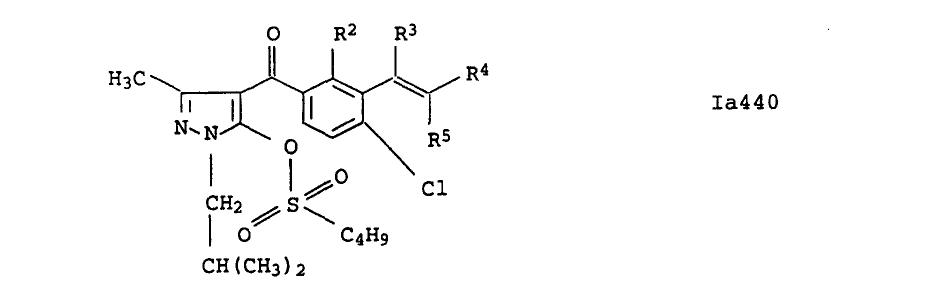
- Ebenso außergewöhnlich bevorzugt sind die Verbindungen Ia441, insbesondere die Verbindungen Ia441.001-Ia441.180, die sich von den entsprechenden Verbindungen Ia1.001-Ia1.180 dadurch unterscheiden, daß R¹⁵ für iso-Butylsulfonyl und R¹⁶ für Methyl stehen: 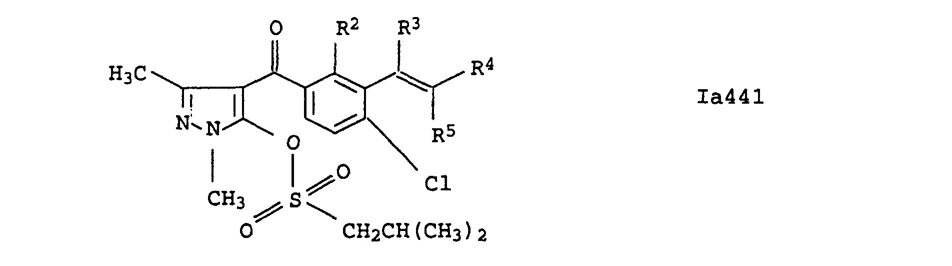
- Ebenso außergewöhnlich bevorzugt sind die Verbindungen Ia442, insbesondere die Verbindungen Ia442.001-Ia442.180, die sich von den entsprechenden Verbindungen Ia1.001-Ia1.180 dadurch unterscheiden, daß R¹⁴ für Ethyl, R¹⁵ für iso-Butylsulfonyl und R¹⁶ für Methyl stehen: 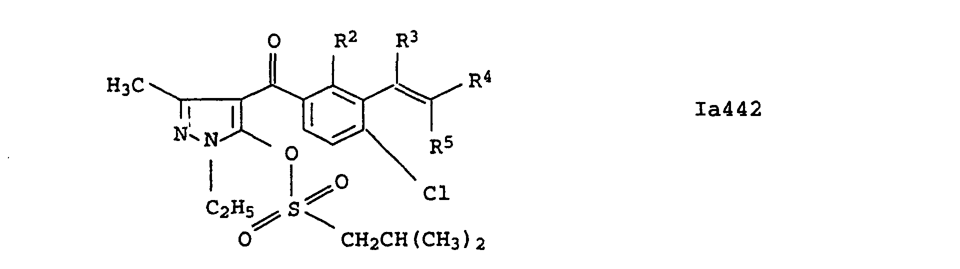
- Ebenso außergewöhnlich bevorzugt sind die Verbindungen Ia443, insbesondere die Verbindungen Ia443.001-Ia443.180, die sich von den entsprechenden Verbindungen Ia1.001-Ia1.180 dadurch unterscheiden, daß R¹⁴ für n-Propyl, R¹⁵ für iso-Butylsulfonyl und R¹⁶ für Methyl stehen: 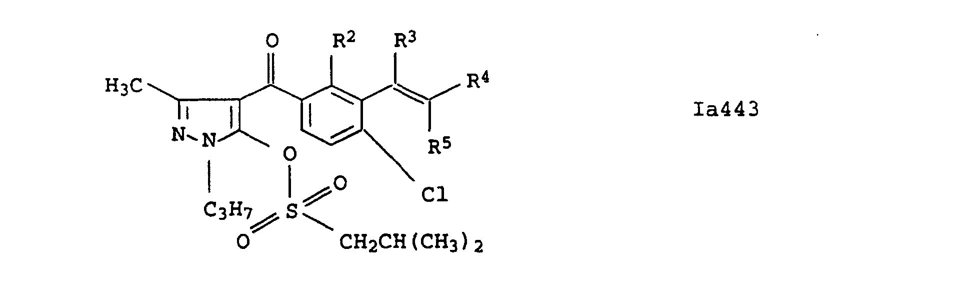
- Ebenso außergewöhnlich bevorzugt sind die Verbindungen Ia444, insbesondere die Verbindungen Ia444.001-Ia444.180, die sich von den entsprechenden Verbindungen Ia1.001-Ia1.180 dadurch unterscheiden, daß R¹⁴ für n-Butyl, R¹⁵ für iso-Butylsulfonyl und R¹⁶ für Methyl stehen: 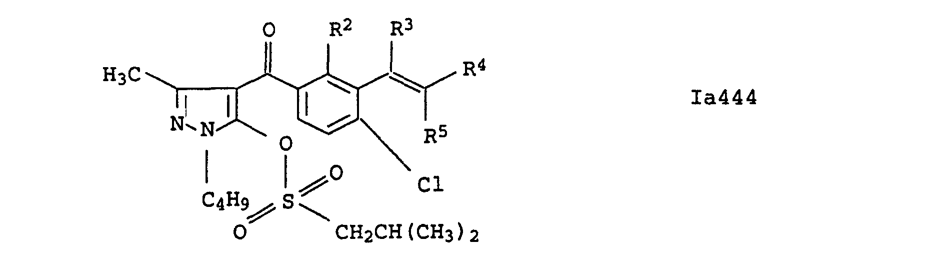
- Ebenso außergewöhnlich bevorzugt sind die Verbindungen Ia445, insbesondere die Verbindungen Ia445.001-Ia445.180, die sich von den entsprechenden Verbindungen Ia1.001-Ia1.180 dadurch unterscheiden, daß R¹⁴ für iso-Butyl, R¹⁵ für iso-Butylsulfonyl und R¹⁶ für Methyl stehen: 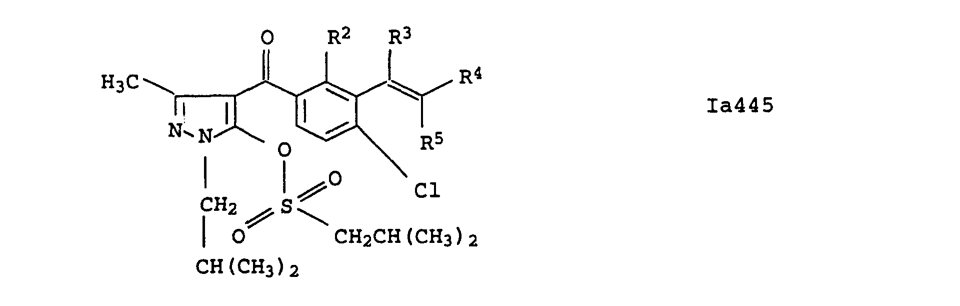
- Ebenso außergewöhnlich bevorzugt sind die Verbindungen Ia446, insbesondere die Verbindungen Ia446.001-Ia446.180, die sich von den entsprechenden Verbindungen Ia1.001-Ia1.180 dadurch unterscheiden, daß R¹⁵ für sec-Butylsulfonyl und R¹⁶ für Methyl stehen: 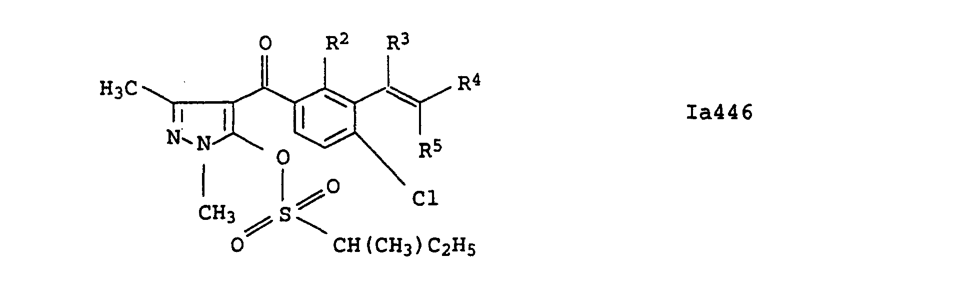
- Ebenso außergewöhnlich bevorzugt sind die Verbindungen Ia447, insbesondere die Verbindungen Ia447.001-Ia447.180, die sich von den entsprechenden Verbindungen Ia1.001-Ia1.180 dadurch unterscheiden, daß R¹⁴ für Ethyl, R¹⁵ für sec-Butylsulfonyl und R¹⁶ für Methyl stehen: 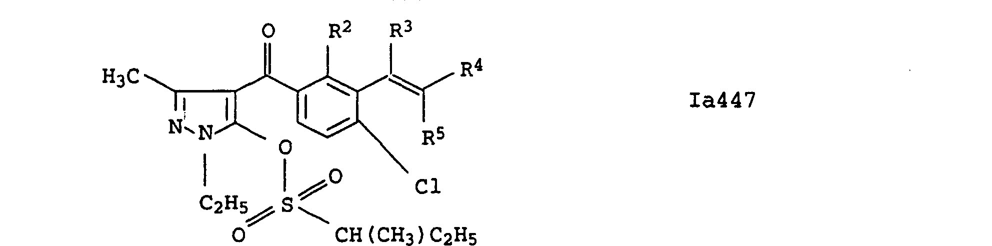
- Ebenso außergewöhnlich bevorzugt sind die Verbindungen Ia448, insbesondere die Verbindungen Ia448.001-Ia448.180, die sich von den entsprechenden Verbindungen Ia1.001-Ia1.180 dadurch unterscheiden, daß R¹⁴ für n-Propyl, R¹⁵ für sec-Butylsulfonyl und R¹⁶ für Methyl stehen: 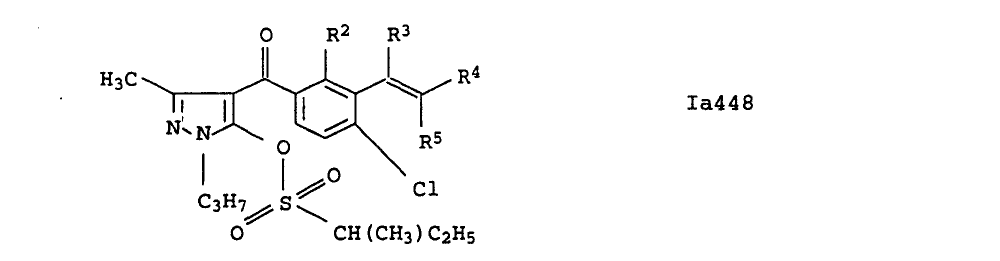
- Ebenso außergewöhnlich bevorzugt sind die Verbindungen Ia449, insbesondere die Verbindungen Ia449.001-Ia449.180, die sich von den entsprechenden Verbindungen Ia1.001-Ia1.180 dadurch unterscheiden, daß R¹⁴ für n-Butyl, R¹⁵ für sec-Butylsulfonyl und R¹⁶ für Methyl stehen: 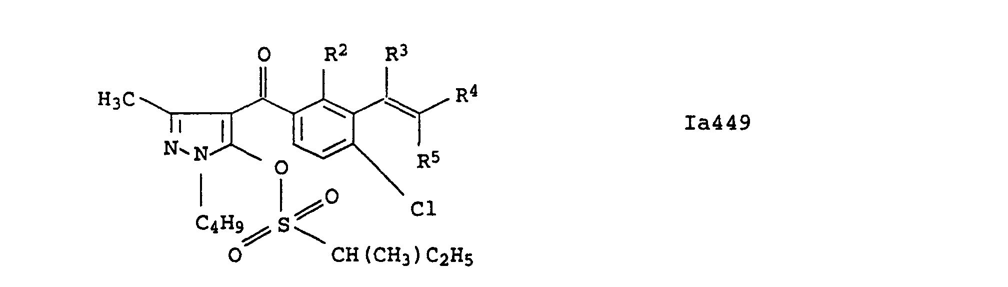
- Ebenso außergewöhnlich bevorzugt sind die Verbindungen Ia450, insbesondere die Verbindungen Ia450.001-Ia450.180, die sich von den entsprechenden Verbindungen Ta1.001-Ia1.180 dadurch unterscheiden, daß R¹⁴ für iso-Butyl, R¹⁵ für sec-Butylsulfonyl und R¹⁶ für Methyl stehen: 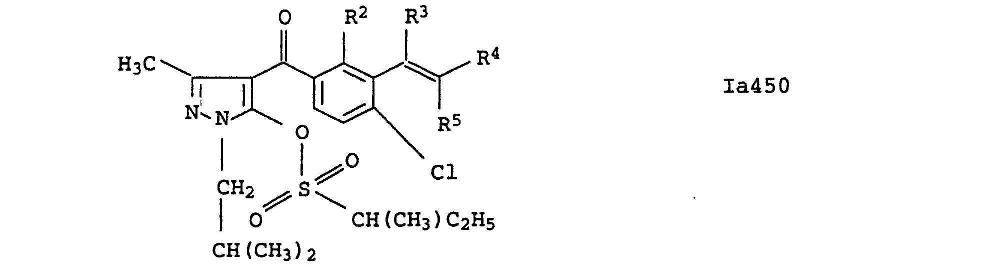
- Ebenso außergewöhnlich bevorzugt sind die Verbindungen Ia451, insbesondere die Verbindungen Ia451.001-Ia451.180, die sich von den entsprechenden Verbindungen Ia1.001-Ia1.180 dadurch unterscheiden, daß R¹⁵ für Trifluormethylsulfonyl und R¹⁶ für Methyl stehen: 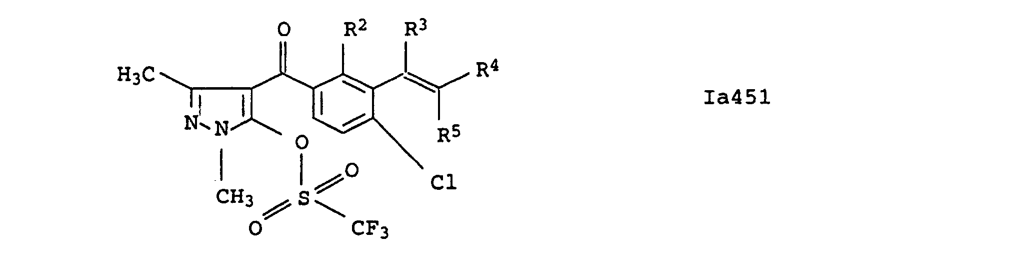
- Ebenso außergewöhnlich bevorzugt sind die Verbindungen Ia452, insbesondere die Verbindungen Ia452.001-Ia452.180, die sich von den entsprechenden Verbindungen Ia1.001-Ia1.180 dadurch unterscheiden, daß R¹⁴ für Ethyl, R¹⁵ für Trifluormethylsulfonyl und R¹⁶ für Methyl stehen: 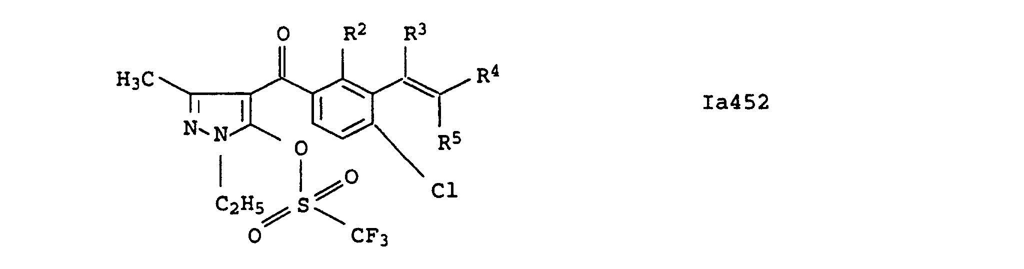
- Ebenso außergewöhnlich bevorzugt sind die Verbindungen Ia453, insbesondere die Verbindungen Ia453.001-Ia453.180, die sich von den entsprechenden Verbindungen Ia1.001-Ia1.180 dadurch unterscheiden, daß R¹⁴ für n-Propyl, R¹⁵ für Trifluormethylsulfonyl und R¹⁶ für Methyl stehen: 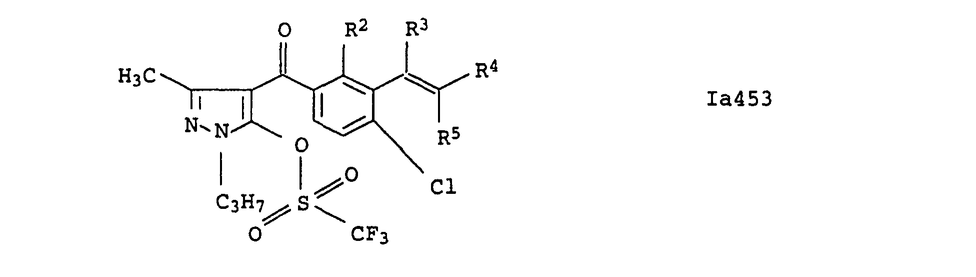
- Ebenso außergewöhnlich bevorzugt sind die Verbindungen Ia454, insbesondere die Verbindungen Ia454.001-Ia454.180, die sich von den entsprechenden Verbindungen Ia1.001-Ia1.180 dadurch unterscheiden, daß R¹⁴ für n-Butyl, R¹⁵ für Trifluormethylsulfonyl und R¹⁶ für Methyl stehen: 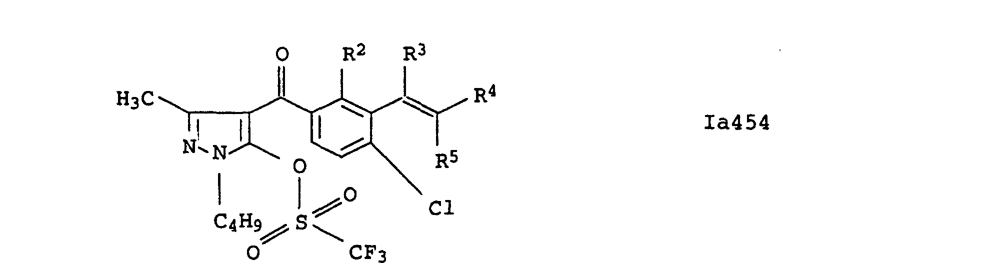
- Ebenso außergewöhnlich bevorzugt sind die Verbindungen Ia455, insbesondere die Verbindungen Ia455.001-Ia455.180, die sich von den entsprechenden Verbindungen Ia1.001-Ia1.180 dadurch unterscheiden, daß R¹⁴ für iso-Butyl, R¹⁵ für Trifluormethylsulfonyl und R¹⁶ für Methyl stehen: 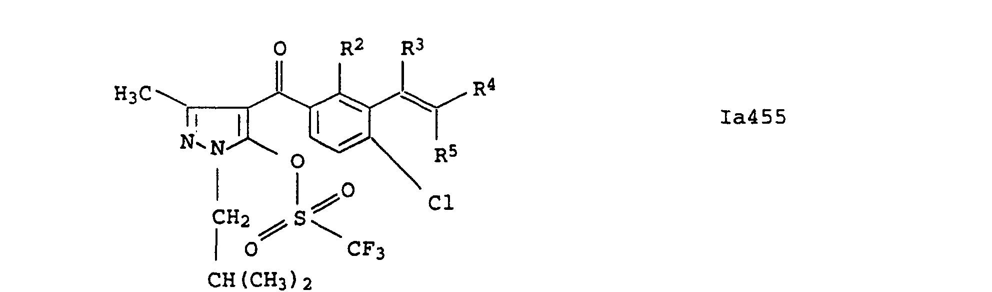
- Ebenso außergewöhnlich bevorzugt sind die Verbindungen Ia456, insbesondere die Verbindungen Ia456.001-Ia456.180, die sich von den entsprechenden Verbindungen Ia1.001-Ia1.180 dadurch unterscheiden, daß R¹⁵ für Phenylcarbonylmethyl und R¹⁶ für Methyl stehen: 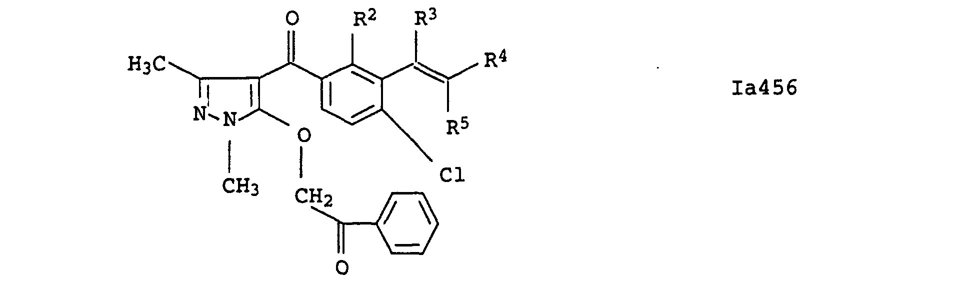
- Ebenso außergewöhnlich bevorzugt sind die Verbindungen Ia457, insbesondere die Verbindungen Ia457.001-Ia457.180, die sich von den entsprechenden Verbindungen Ia1.001-Ia1.180 dadurch unterscheiden, daß R¹⁴ für Ethyl, R¹⁵ für Phenylcarbonylmethyl und R¹⁶ für Methyl stehen: 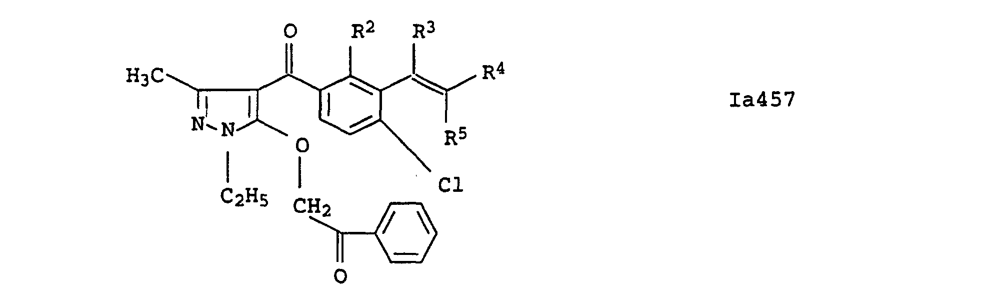
- Ebenso außergewöhnlich bevorzugt sind die Verbindungen Ia458, insbesondere die Verbindungen Ia458.001-Ia458.180, die sich von den entsprechenden Verbindungen Ia1.001-Ia1.180 dadurch unterscheiden, daß R¹⁴ für n-Propyl, R¹⁵ für Phenylcarbonylmethyl und R¹⁶ für Methyl stehen: 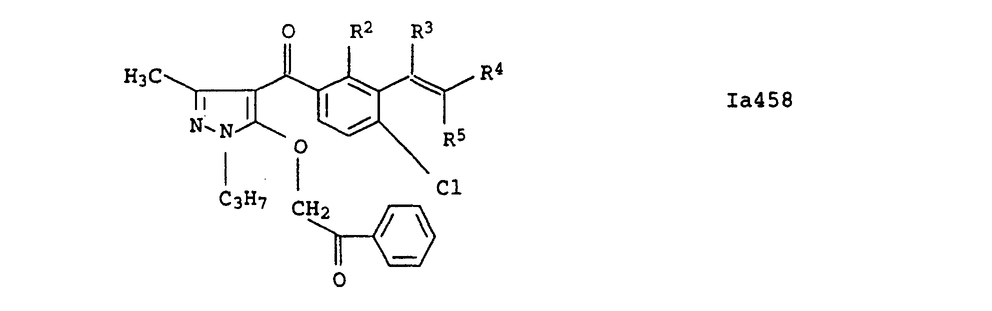
- Ebenso außergewöhnlich bevorzugt sind die Verbindungen Ia459, insbesondere die Verbindungen Ia459.001-Ia459.180, die sich von den entsprechenden Verbindungen Ia1.001-Ia1.180 dadurch unterscheiden, daß R¹⁴ für n-Butyl, R¹⁵ für Phenylcarbonylmethyl und R¹⁶ für Methyl stehen: 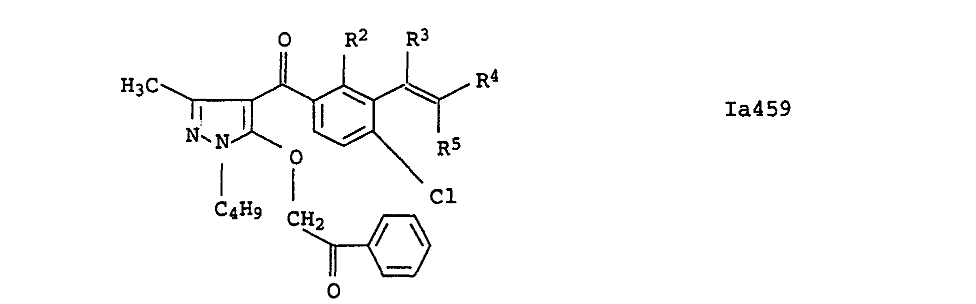
- Ebenso außergewöhnlich bevorzugt sind die Verbindungen Ia460, insbesondere die Verbindungen Ia460.001-Ia460.180, die sich von den entsprechenden Verbindungen Ia1.001-Ia1.180 dadurch unterscheiden, daß R¹⁴ für iso-Butyl, R¹⁵ für Phenylcarbonylmethyl und R¹⁶ für Methyl stehen: 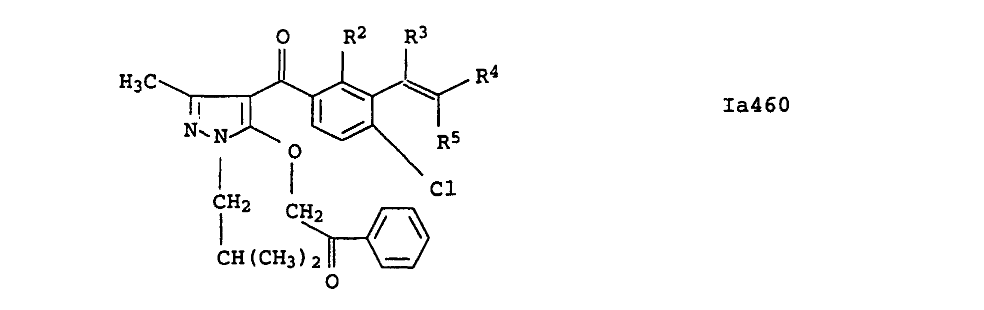
- Ebenso außergewöhnlich bevorzugt sind die Verbindungen Ia461, insbesondere die Verbindungen Ia461.001-Ia461.180, die sich von den entsprechenden Verbindungen Ia1.001-Ia1.180 dadurch unterscheiden, daß R¹ für Nitro und R¹⁶ für Methyl stehen: 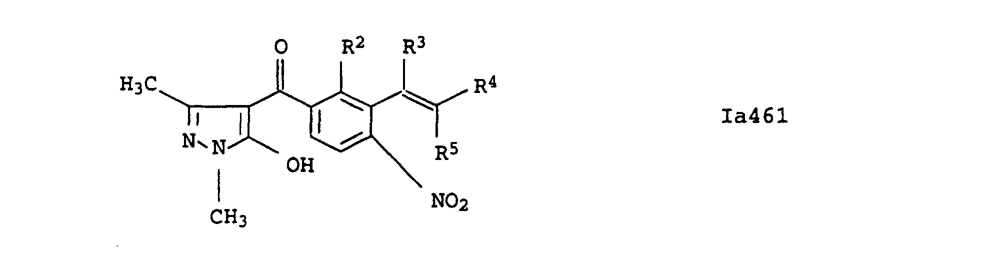
- Ebenso außergewöhnlich bevorzugt sind die Verbindungen Ia462, insbesondere die Verbindungen Ia462.001-Ia462.180, die sich von den entsprechenden Verbindungen Ia1.001-Ia1.180 dadurch unterscheiden, daß R¹ für Nitro, R¹⁴ für Ethyl und R¹⁶ für Methyl stehen: 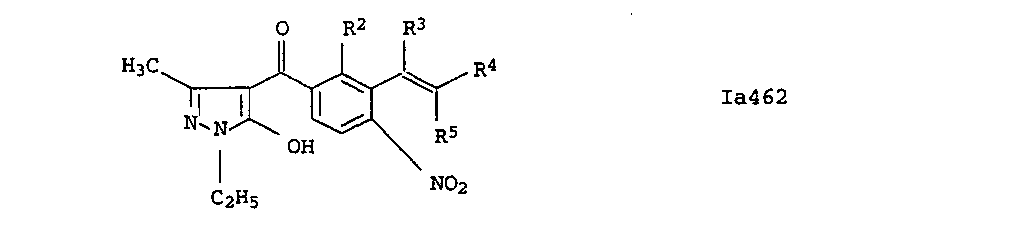
- Ebenso außergewöhnlich bevorzugt sind die Verbindungen Ia463, insbesondere die Verbindungen Ia463.001-Ia463.180, die sich von den entsprechenden Verbindungen Ia1.001-Ia1.180 dadurch unterscheiden, daß R¹ für Nitro, R¹⁴ für n-Propyl und R¹⁶ für Methyl stehen: 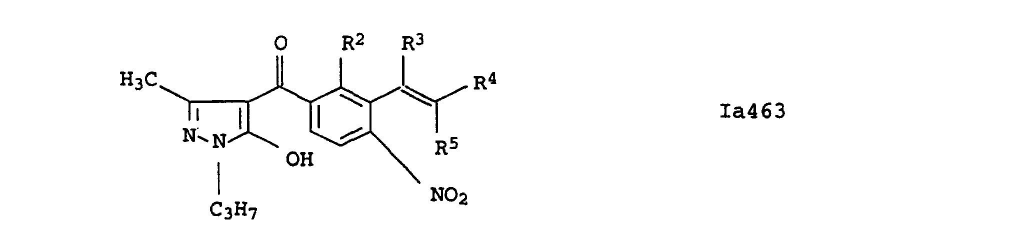
- Ebenso außergewöhnlich bevorzugt sind die Verbindungen Ia464, insbesondere die Verbindungen Ia464.001-Ia464.180, die sich von den entsprechenden Verbindungen Ia1.001-Ia1.180 dadurch unterscheiden, daß R¹ für Nitro, R¹⁴ für n-Butyl und R¹⁶ für Methyl stehen: 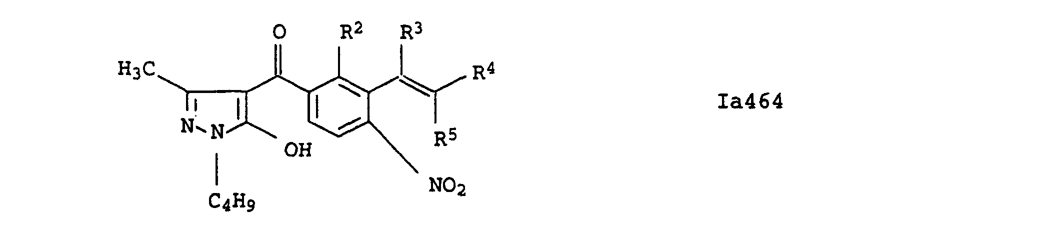
- Ebenso außergewöhnlich bevorzugt sind die Verbindungen Ia465, insbesondere die Verbindungen Ia465.001-Ia465.180, die sich von den entsprechenden Verbindungen Ia1.001-Ia1.180 dadurch unterscheiden, daß R¹ für Nitro, R¹⁴ für iso-Butyl und R¹⁶ für Methyl stehen: 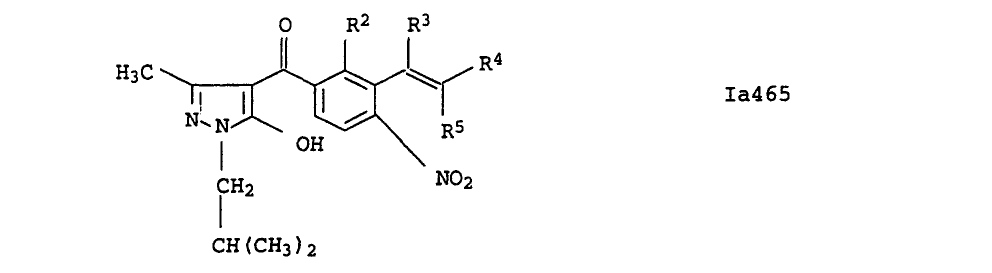
- Ebenso außergewöhnlich bevorzugt sind die Verbindungen Ia466, insbesondere die Verbindungen Ia466.001-Ia466.180, die sich von den entsprechenden Verbindungen Ia1.001-Ia1.180 dadurch unterscheiden, daß R¹ für Nitro und R¹⁵ und R¹⁶ für Methyl stehen: 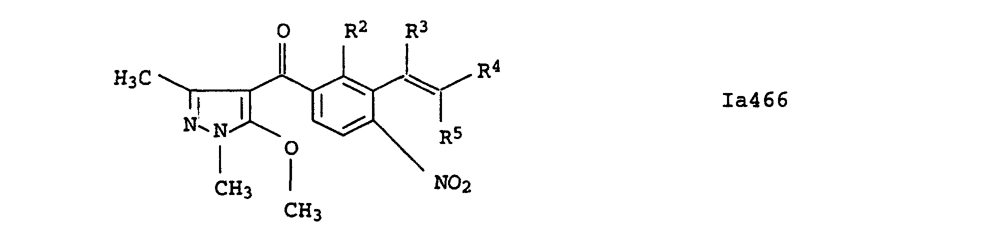
- Ebenso außergewöhnlich bevorzugt sind die Verbindungen Ia467, insbesondere die Verbindungen Ia467.001-Ia467.180, die sich von den entsprechenden Verbindungen Ia1.001-Ia1.180 dadurch unterscheiden, daß R¹ für Nitro, R¹⁴ für Ethyl, R¹⁵ und R¹⁶ für Methyl stehen: 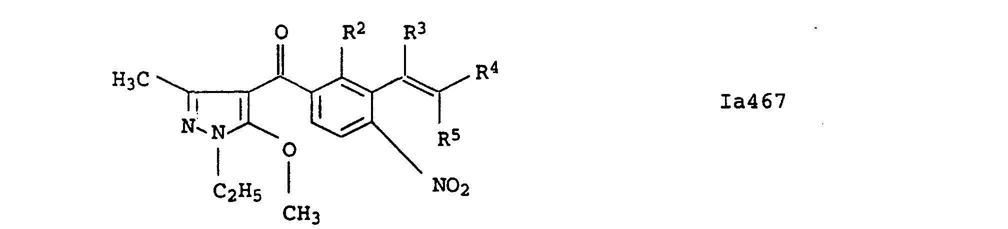
- Ebenso außergewöhnlich bevorzugt sind die Verbindungen Ia468, insbesondere die Verbindungen Ia468.001-Ia468.180, die sich von den entsprechenden Verbindungen Ia1.001-Ia1.180 dadurch unterscheiden, daß R¹ für Nitro, R¹⁴ für n-Propyl, R¹⁵ und R¹⁶ für Methyl stehen: 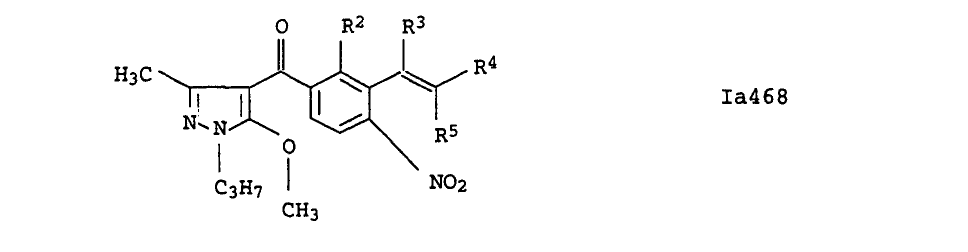
- Ebenso außergewöhnlich bevorzugt sind die Verbindungen Ia469, insbesondere die Verbindungen Ia469.001-Ia469.180, die sich von den entsprechenden Verbindungen Ia1.001-Ia1.180 dadurch unterscheiden, daß R¹ für Nitro, R¹⁴ für n-Butyl, R¹⁵ und R¹⁶ für Methyl stehen: 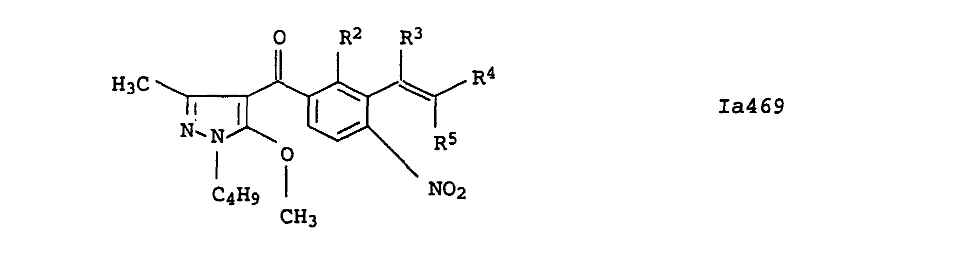
- Ebenso außergewöhnlich bevorzugt sind die Verbindungen Ia470, insbesondere die Verbindungen Ia470.001-Ia470.180, die sich von den entsprechenden Verbindungen Ia1.001-Ia1.180 dadurch unterscheiden, daß R¹ für Nitro, R¹⁴ für iso-Butyl, R¹⁵ und R¹⁶ für Methyl stehen: 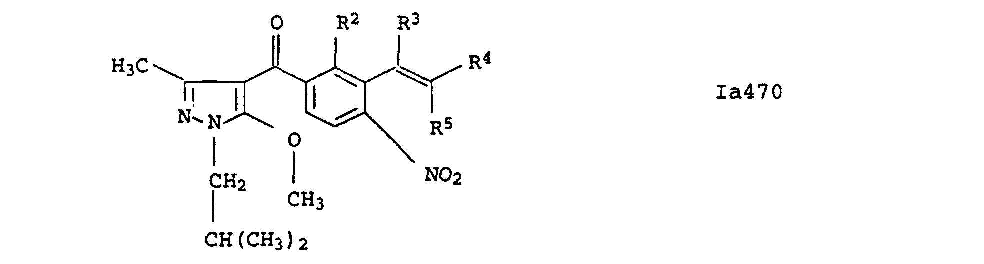
- Ebenso außergewöhnlich bevorzugt sind die Verbindungen Ia471, insbesondere die Verbindungen Ia471.001-Ia471.180, die sich von den entsprechenden Verbindungen Ia1.001-Ia1.180 dadurch unterscheiden, daß R¹ für Nitro, R¹⁵ für Ethyl und R¹⁶ für Methyl stehen: 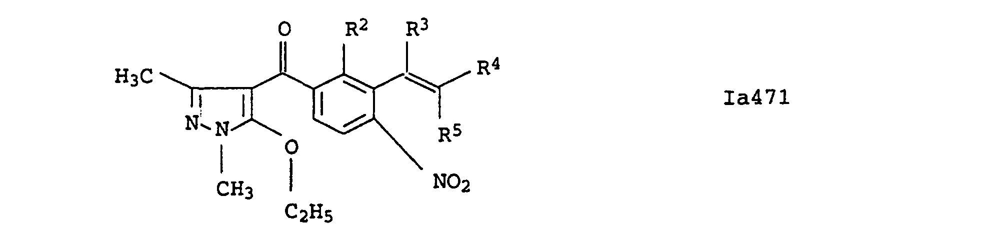
- Ebenso außergewöhnlich bevorzugt sind die Verbindungen Ia472, insbesondere die Verbindungen Ia472.001-Ia472.180, die sich von den entsprechenden Verbindungen Ia1.001-Ia1.180 dadurch unterscheiden, daß R¹ für Nitro, R¹⁴ und R¹⁵ für Ethyl und R¹⁶ für Methyl stehen: 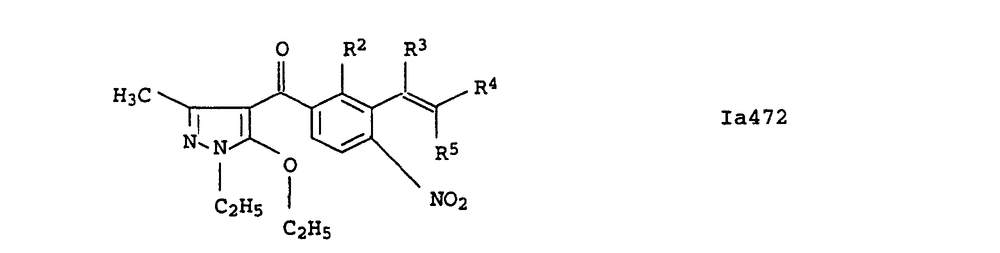
- Ebenso außergewöhnlich bevorzugt sind die Verbindungen Ia473, insbesondere die Verbindungen Ia473.001-Ia473.180, die sich von den entsprechenden Verbindungen Ia1.001-Ia1.180 dadurch unterscheiden, daß R¹ für Nitro, R¹⁴ für n-Propyl, R¹⁵ für Ethyl und R¹⁶ für Methyl stehen: 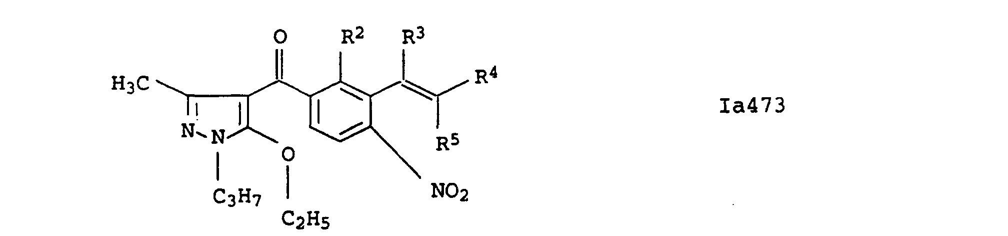
- Ebenso außergewöhnlich bevorzugt sind die Verbindungen Ia474, insbesondere die Verbindungen Ia474.001-Ia474.180, die sich von den entsprechenden Verbindungen Ia1.001-Ia1.180 dadurch unterscheiden, daß R¹ für Nitro, R¹⁴ für n-Butyl, R¹⁵ für Ethyl und R¹⁶ für Methyl stehen: 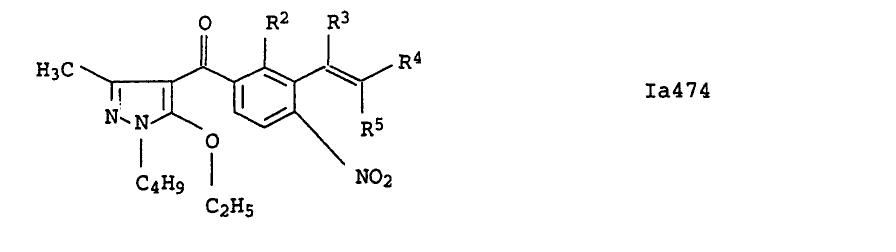
- Ebenso außergewöhnlich bevorzugt sind die Verbindungen Ia475, insbesondere die Verbindungen Ia475.001-Ia475.180, die sich von den entsprechenden Verbindungen Ia1.001-Ia1.180 dadurch unterscheiden, daß R¹ für Nitro, R¹⁴ für iso-Butyl, R¹⁵ für Ethyl und R¹⁶ für Methyl stehen: 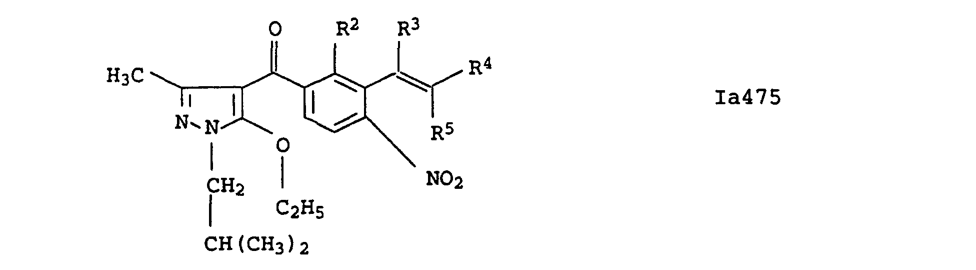
- Ebenso außergewöhnlich bevorzugt sind die Verbindungen Ia476, insbesondere die Verbindungen Ia476.001-Ia476.180, die sich von den entsprechenden Verbindungen Ia1.001-Ia1.180 dadurch unterscheiden, daß R¹ für Nitro, R¹⁵ für n-Propyl und R¹⁶ für Methyl stehen: 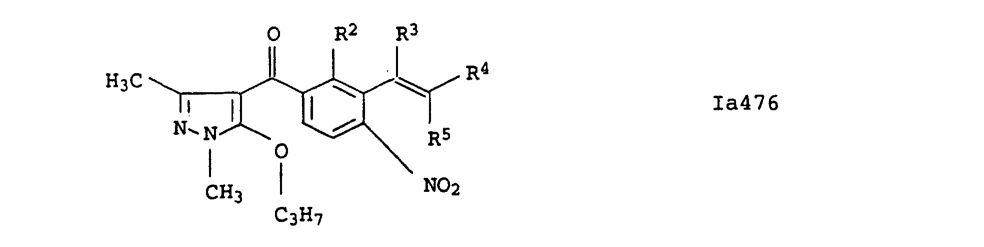
- Ebenso außergewöhnlich bevorzugt sind die Verbindungen Ia477, insbesondere die Verbindungen Ia477.001-Ia477.180, die sich von den entsprechenden Verbindungen Ia1.001-Ia1.180 dadurch unterscheiden, daß R¹ für Nitro, R¹⁴ für Ethyl, R¹⁵ für n-Propyl und R¹⁶ für Methyl stehen: 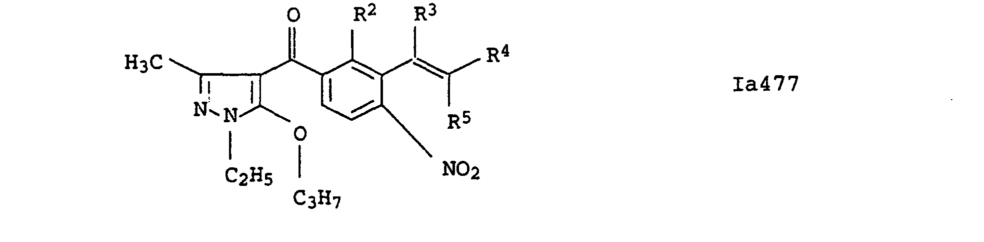
- Ebenso außergewöhnlich bevorzugt sind die Verbindungen Ia478, insbesondere die Verbindungen Ia478.001-Ia478.180, die sich von den entsprechenden Verbindungen Ia1.001-Ia1.180 dadurch unterscheiden, daß R¹ für Nitro, R¹⁴ und R¹⁵ für n-Propyl und R¹⁶ für Methyl stehen: 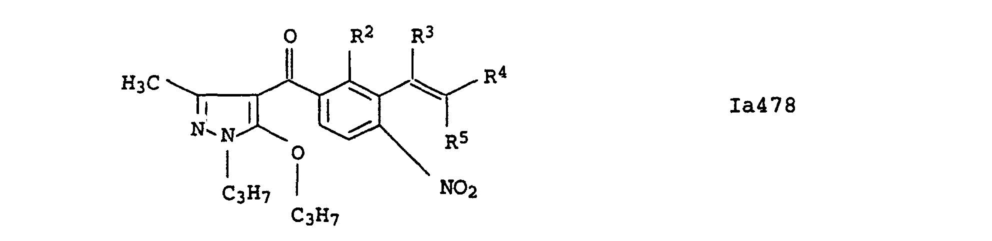
- Ebenso außergewöhnlich bevorzugt sind die Verbindungen Ia479, insbesondere die Verbindungen Ia479.001-Ia479.180, die sich von den entsprechenden Verbindungen Ia1.001-Ia1.180 dadurch unterscheiden, daß R¹ für Nitro, R¹⁴ für n-Butyl, R¹⁵ für n-Propyl und R¹⁶ für Methyl stehen: 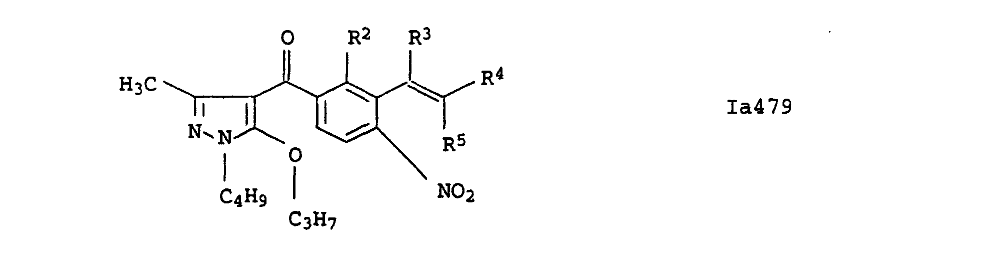
- Ebenso außergewöhnlich bevorzugt sind die Verbindungen Ia480, insbesondere die Verbindungen Ia480.001-Ia480.180, die sich von den entsprechenden Verbindungen Ia1.001-Ia1.180 dadurch unterscheiden, daß R¹ für Nitro, R¹⁴ für iso-Butyl, R¹⁵ für n-Propyl und R¹⁶ für Methyl stehen: 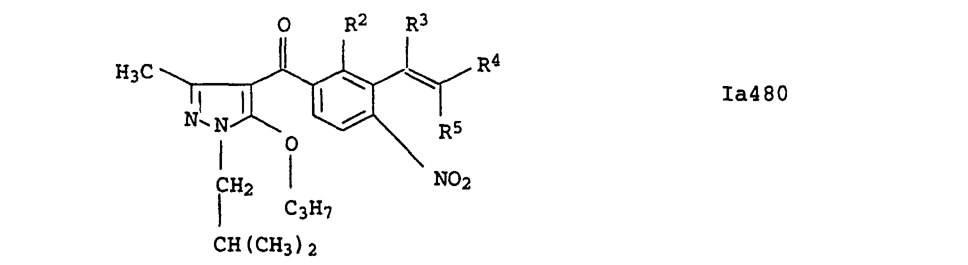
- Ebenso außergewöhnlich bevorzugt sind die Verbindungen Ia481, insbesondere die Verbindungen Ia481.001-Ia481.180, die sich von den entsprechenden Verbindungen Ia1.001-Ia1.180 dadurch unterscheiden, daß R¹ für Nitro, R¹⁵ für iso-Propyl und R¹⁶ für Methyl stehen: 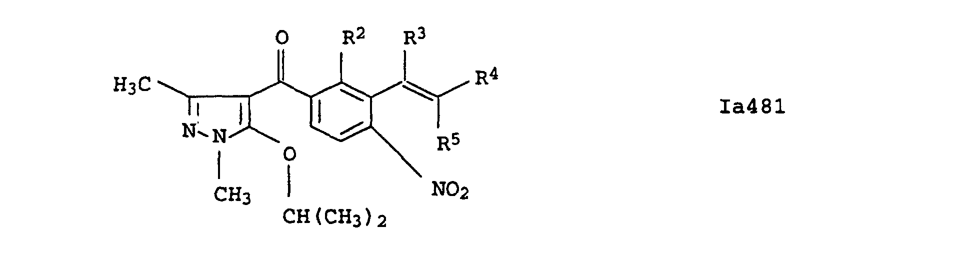
- Ebenso außergewöhnlich bevorzugt sind die Verbindungen Ia482, insbesondere die Verbindungen Ia482.001-Ia482.180, die sich von den entsprechenden Verbindungen Ia1.001-Ia1.180 dadurch unterscheiden, daß R¹ für Nitro, R¹⁴ für Ethyl, R¹⁵ für iso-Propyl und R¹⁶ für Methyl stehen: 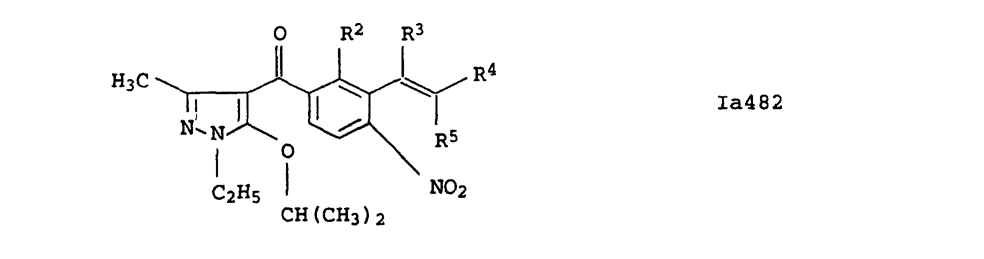
- Ebenso außergewöhnlich bevorzugt sind die Verbindungen Ia483, insbesondere die Verbindungen Ia483.001-Ia483.180, die sich von den entsprechenden Verbindungen Ia1.001-Ia1.180 dadurch unterscheiden, daß R¹ für Nitro, R¹⁴ für n-Propyl, R¹⁵ für iso-Propyl und R¹⁶ für Methyl stehen: 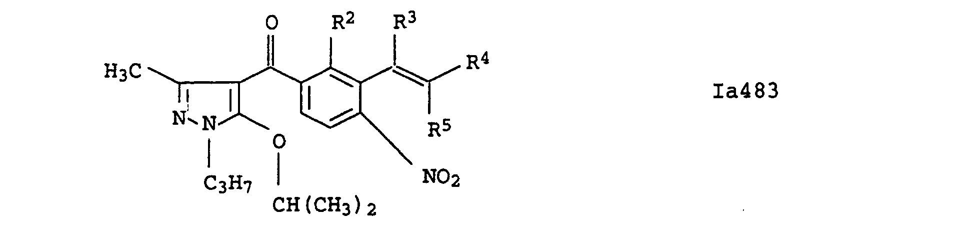
- Ebenso außergewöhnlich bevorzugt sind die Verbindungen Ia484, insbesondere die Verbindungen Ia484.001-Ia484.180, die sich von den entsprechenden Verbindungen Ia1.001-Ia1.180 dadurch unterscheiden, daß R¹ für Nitro, R¹⁴ für n-Butyl, R¹⁵ für iso-Propyl und R¹⁶ für Methyl stehen: 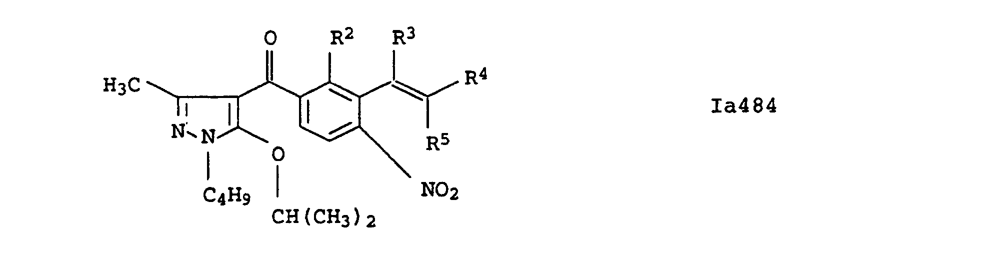
- Ebenso außergewöhnlich bevorzugt sind die Verbindungen Ia485, insbesondere die Verbindungen Ia485.001-Ia485.180, die sich von den entsprechenden Verbindungen Ia1.001-Ia1.180 dadurch unterscheiden, daß R¹ für Nitro, R¹⁴ für iso-Butyl, R¹⁵ für iso-Propyl und R¹⁶ für Methyl stehen: 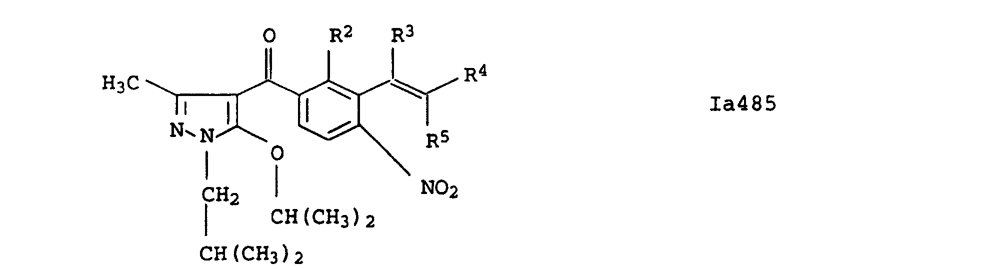
- Ebenso außergewöhnlich bevorzugt sind die Verbindungen Ia486, insbesondere die Verbindungen Ia486.001-Ia486.180, die sich von den entsprechenden Verbindungen Ia1.001-Ia1.180 dadurch unterscheiden, daß R¹ für Nitro, R¹⁵ für n-Butyl und R¹⁶ für Methyl stehen: 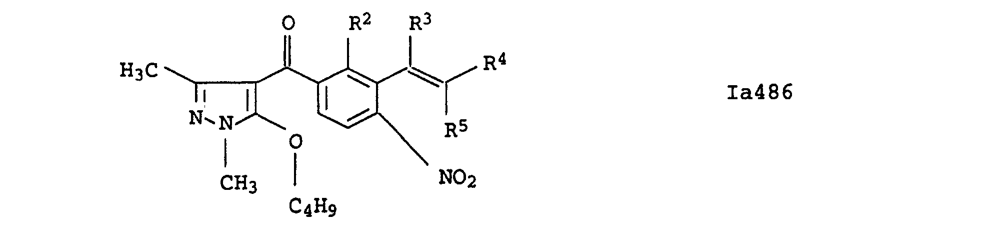
- Ebenso außergewöhnlich bevorzugt sind die Verbindungen Ia487, insbesondere die Verbindungen Ia487.001-Ia487.180, die sich von den entsprechenden Verbindungen Ia1.001-Ia1.180 dadurch unterscheiden, daß R¹ für Nitro, R¹⁴ für Ethyl, R¹⁵ für n-Butyl und R¹⁶ für Methyl stehen: 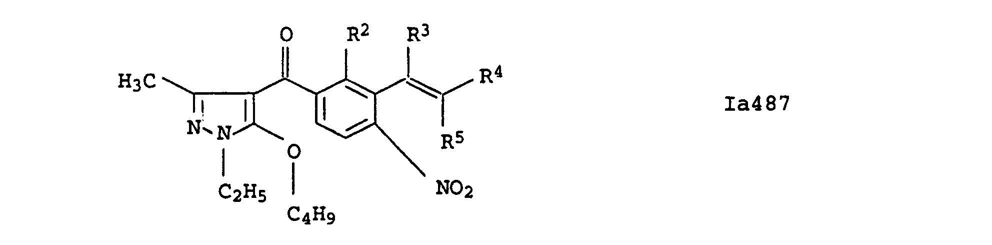
- Ebenso außergewöhnlich bevorzugt sind die Verbindungen Ia488, insbesondere die Verbindungen Ia488.001-Ia488.180, die sich von den entsprechenden Verbindungen Ia1.001-Ia1.180 dadurch unterscheiden, daß R¹ für Nitro, R¹⁴ für n-Propyl, R¹⁵ für n-Butyl und R¹⁶ für Methyl stehen: 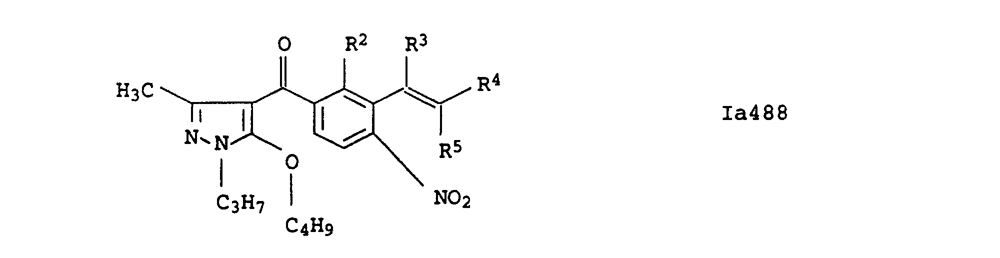
- Ebenso außergewöhnlich bevorzugt sind die Verbindungen Ia489, insbesondere die Verbindungen Ia489.001-Ia489.180, die sich von den entsprechenden Verbindungen Ia1.001-Ia1.180 dadurch unterscheiden, daß R¹ für Nitro, R¹⁴ und R¹⁵ für n-Butyl und R¹⁶ für Methyl stehen: 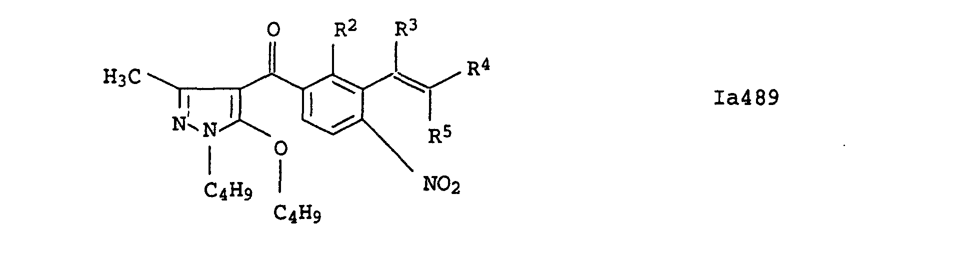
- Ebenso außergewöhnlich bevorzugt sind die Verbindungen Ia490, insbesondere die Verbindungen Ia490.001-Ia490.180, die sich von den entsprechenden Verbindungen Ia1.001-Ia1.180 dadurch unterscheiden, daß R¹ für Nitro, R¹⁴ für iso-Butyl, R¹⁵ für n-Butyl und R¹⁶ für Methyl stehen: 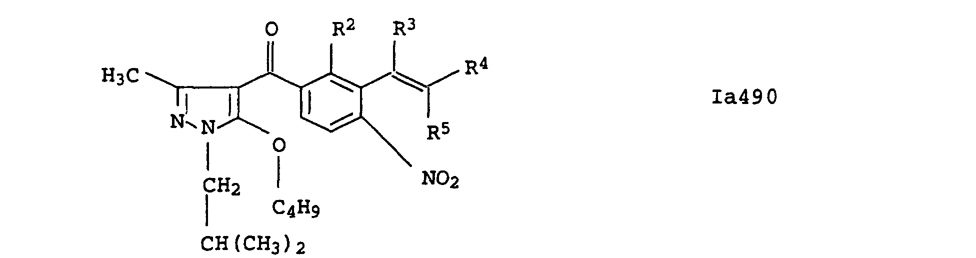
- Ebenso außergewöhnlich bevorzugt sind die Verbindungen Ia491, insbesondere die Verbindungen Ia491.001-Ia491.180, die sich von den entsprechenden Verbindungen Ia1.001-Ia1.180 dadurch unterscheiden, daß R¹ für Nitro, R¹⁵ für sec-Butyl und R¹⁶ für Methyl stehen: 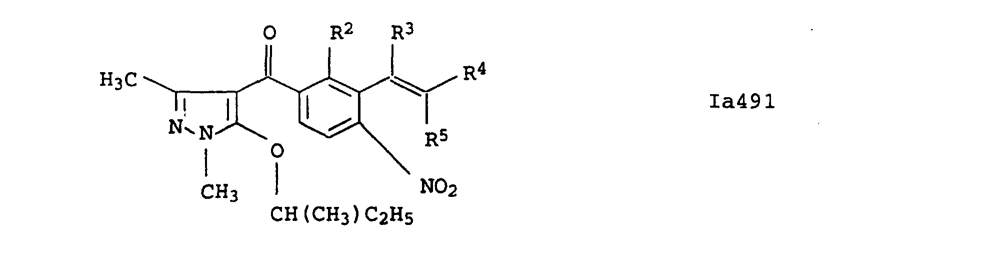
- Ebenso außergewöhnlich bevorzugt sind die Verbindungen Ia492, insbesondere die Verbindungen Ia492.001-Ia492.180, die sich von den entsprechenden Verbindungen Ia1.001-Ia1.180 dadurch unterscheiden, daß R¹ für Nitro, R¹⁴ für Ethyl, R¹⁵ für sec-Butyl und R¹⁶ für Methyl stehen: 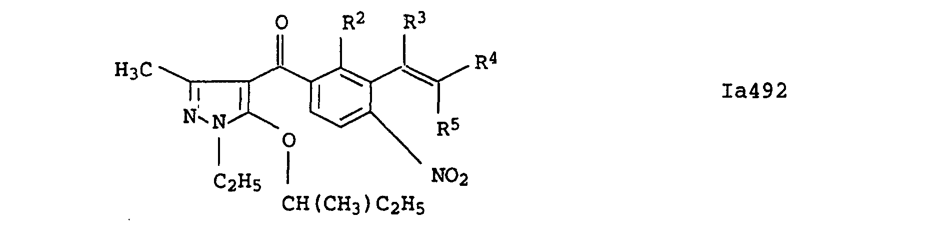
- Ebenso außergewöhnlich bevorzugt sind die Verbindungen Ia493, insbesondere die Verbindungen Ia493.001-Ia493.180, die sich von den entsprechenden Verbindungen Ia1.001-Ia1.180 dadurch unterscheiden, daß R¹ für Nitro, R¹⁴ für n-Propyl, R¹⁵ für sec-Butyl und R¹⁶ für Methyl stehen: 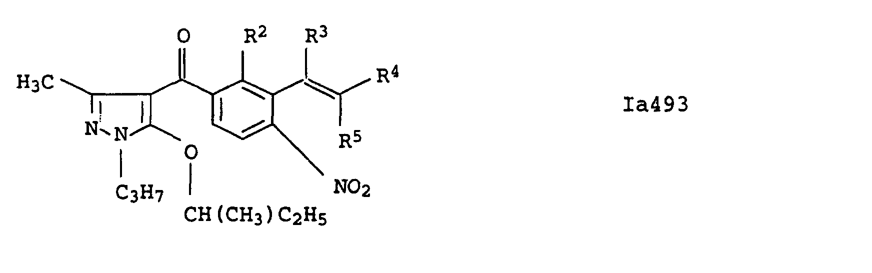
- Ebenso außergewöhnlich bevorzugt sind die Verbindungen Ia494, insbesondere die Verbindungen Ia494.001-Ia494.180, die sich von den entsprechenden Verbindungen Ia1.001-Ia1.180 dadurch unterscheiden, daß R¹ für Nitro, R¹⁴ für n-Butyl, R¹⁵ für sec-Butyl und R¹⁶ für Methyl stehen: 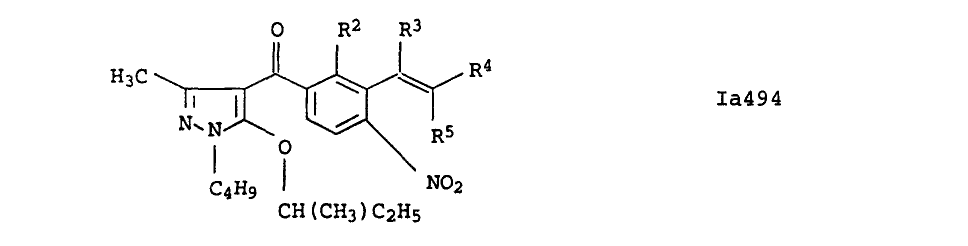
- Ebenso außergewöhnlich bevorzugt sind die Verbindungen Ia495, insbesondere die Verbindungen Ia495.001-Ia495.180, die sich von den entsprechenden Verbindungen Ia1.001-Ia1.180 dadurch unterscheiden, daß R¹ für Nitro, R¹⁴ für iso-Butyl, R¹⁵ für sec-Butyl und R¹⁶ für Methyl stehen: 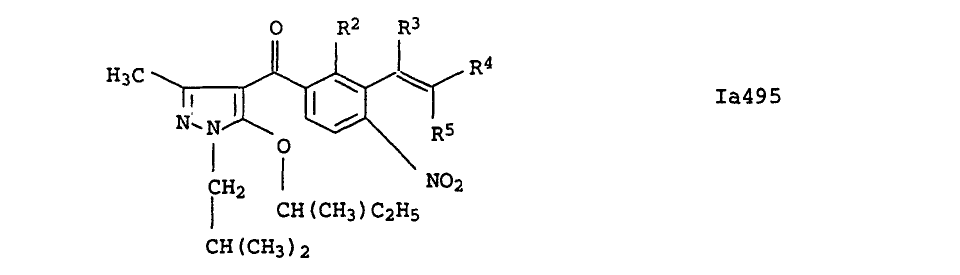
- Ebenso außergewöhnlich bevorzugt sind die Verbindungen Ia496, insbesondere die Verbindungen Ia496.001-Ia496.180, die sich von den entsprechenden Verbindungen Ia1.001-Ia1.180 dadurch unterscheiden, daß R¹ für Nitro, R¹⁵ für iso-Butyl und R¹⁶ für Methyl stehen: 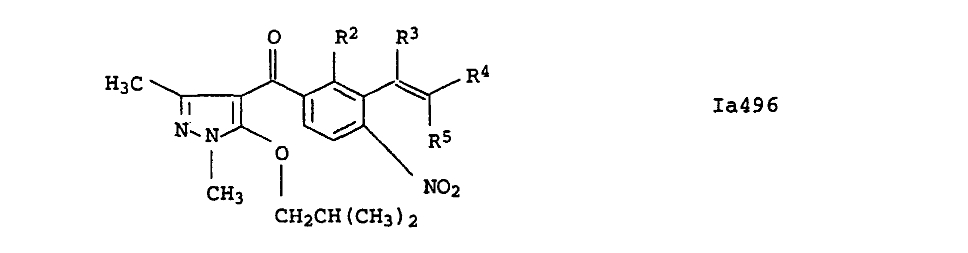
- Ebenso außergewöhnlich bevorzugt sind die Verbindungen Ia497, insbesondere die Verbindungen Ia497.001-Ia497.180, die sich von den entsprechenden Verbindungen Ia1.001-Ia1.180 dadurch unterscheiden, daß R¹ für Nitro, R¹⁴ für Ethyl, R¹⁵ für iso-Butyl und R¹⁶ für Methyl stehen: 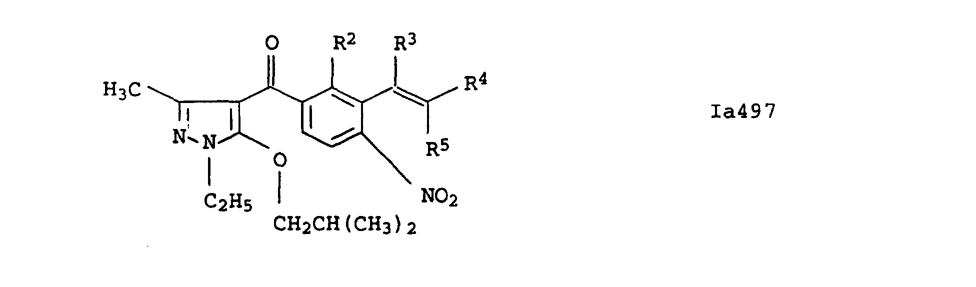
- Ebenso außergewöhnlich bevorzugt sind die Verbindungen Ia498, insbesondere die Verbindungen Ia498.001-Ia498.180, die sich von den entsprechenden Verbindungen Ia1.001-Ia1.180 dadurch unterscheiden, daß R¹ für Nitro, R¹⁴ für n-Propyl, R¹⁵ für iso-Butyl und R¹⁶ für Methyl stehen: 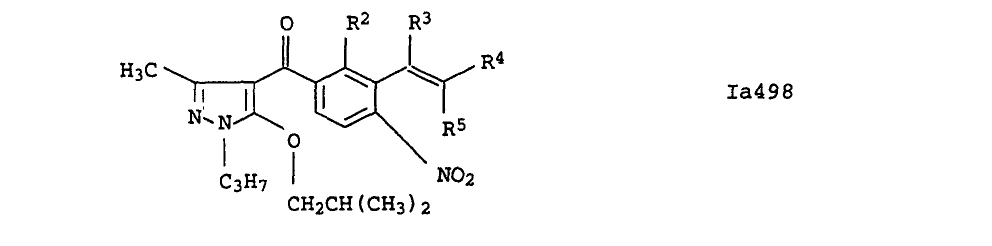
- Ebenso außergewöhnlich bevorzugt sind die Verbindungen Ia499, insbesondere die Verbindungen Ia499.001-Ia499.180, die sich von den entsprechenden Verbindungen Ia1.001-Ia1.180 dadurch unterscheiden, daß R¹ für Nitro, R¹⁴ für n-Butyl, R¹⁵ für iso-Butyl und R¹⁶ für Methyl stehen: 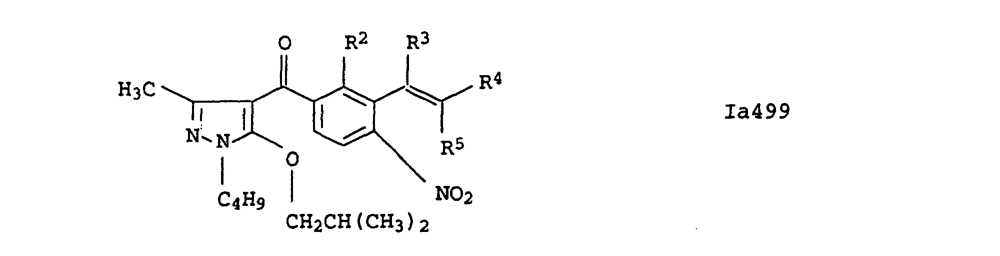
- Ebenso außergewöhnlich bevorzugt sind die Verbindungen Ia500, insbesondere die Verbindungen Ia500.001-Ia500.180, die sich von den entsprechenden Verbindungen Ia1.001-Ia1.180 dadurch unterscheiden, daß R¹ für Nitro, R¹⁴ und R¹⁵ für iso-Butyl und R¹⁶ für Methyl stehen: 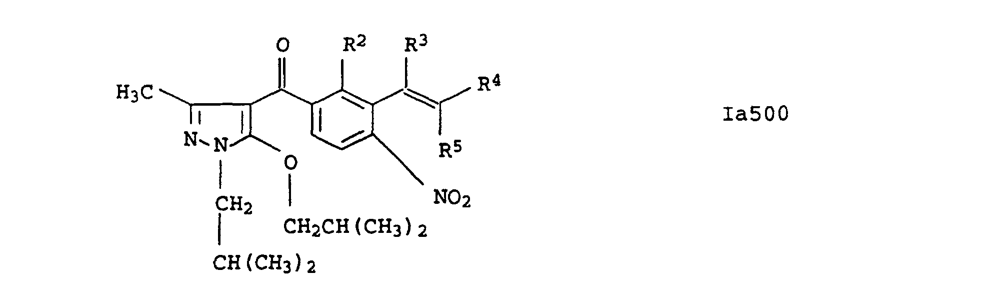
- Ebenso außergewöhnlich bevorzugt sind die Verbindungen Ia501, insbesondere die Verbindungen Ia501.001-Ia501.180, die sich von den entsprechenden Verbindungen Ia1.001-Ia1.180 dadurch unterscheiden, daß R¹ für Nitro, R¹⁵ für Methylcarbonyl und R¹⁶ für Methyl stehen: 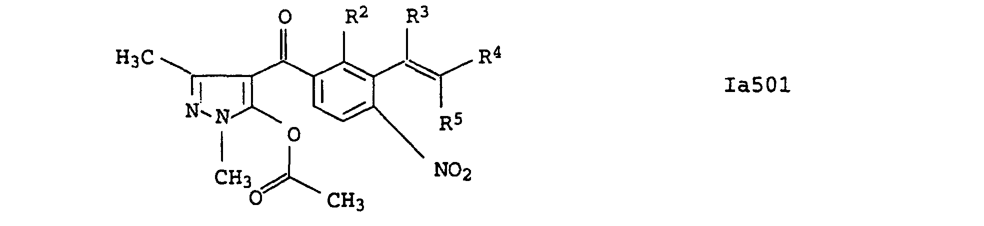
- Ebenso außergewöhnlich bevorzugt sind die Verbindungen Ia502, insbesondere die Verbindungen Ia502.001-Ia502.180, die sich von den entsprechenden Verbindungen Ia1.001-Ia1.180 dadurch unterscheiden, daß R¹ für Nitro, R¹⁴ für Ethyl, R¹⁵ für Methylcarbonyl und R¹⁶ für Methyl stehen: 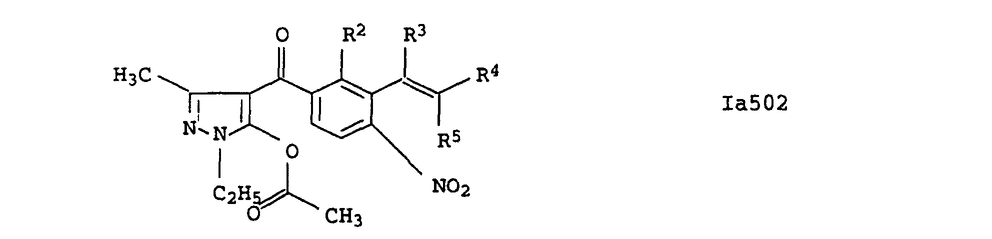
- Ebenso außergewöhnlich bevorzugt sind die Verbindungen Ia503, insbesondere die Verbindungen Ia503.001-Ia503.180, die sich von den entsprechenden Verbindungen Ia1.001-Ia1.180 dadurch unterscheiden, daß R¹ für Nitro, R¹⁴ für n-Propyl, R¹⁵ für Methylcarbonyl und R¹⁶ für Methyl stehen: 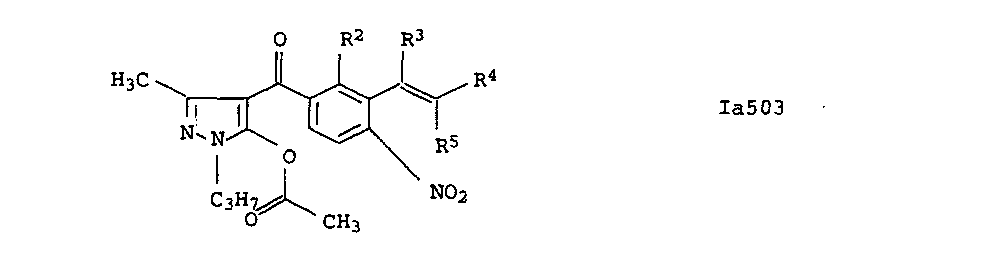
- Ebenso außergewöhnlich bevorzugt sind die Verbindungen Ia504, insbesondere die Verbindungen Ia504.001-Ia504.180, die sich von den entsprechenden Verbindungen Ia1.001-Ia1.180 dadurch unterscheiden, daß R¹ für Nitro, R¹⁴ für n-Butyl, R¹⁵ für Methylcarbonyl und R¹⁶ für Methyl stehen: 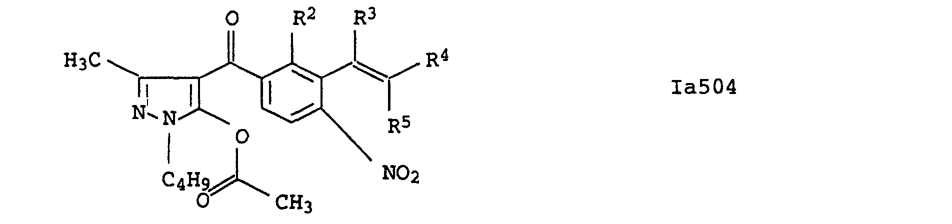
- Ebenso außergewöhnlich bevorzugt sind die Verbindungen Ia505, insbesondere die Verbindungen Ia505.001-Ia505.180, die sich von den entsprechenden Verbindungen Ia1.001-Ia1.180 dadurch unterscheiden, daß R¹ für Nitro, R¹⁴ für iso-Butyl, R¹⁵ für Methylcarbonyl und R¹⁶ für Methyl stehen: 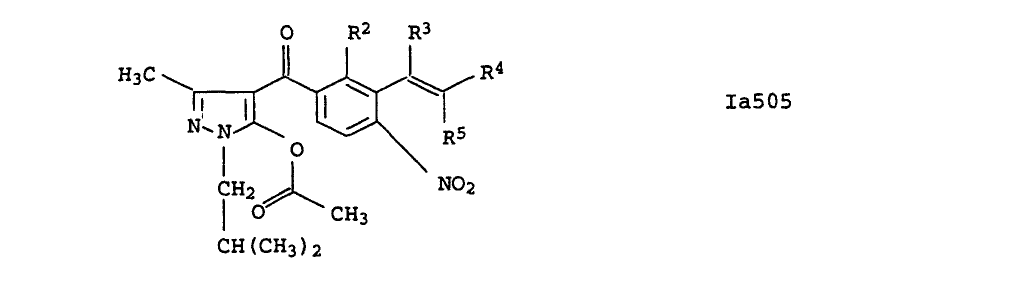
- Ebenso außergewöhnlich bevorzugt sind die Verbindungen Ia506, insbesondere die Verbindungen Ia506.001-Ia506.180, die sich von den entsprechenden Verbindungen Ia1.001-Ia1.180 dadurch unterscheiden, daß R¹ für Nitro, R¹⁵ für Ethylcarbonyl und R¹⁶ für Methyl stehen: 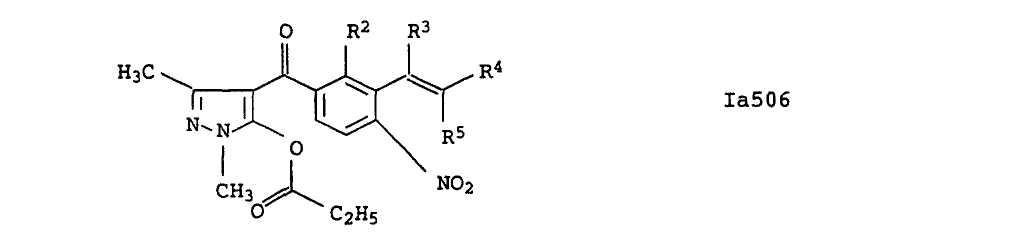
- Ebenso außergewöhnlich bevorzugt sind die Verbindungen Ia507, insbesondere die Verbindungen Ia507.001-Ia507.180, die sich von den entsprechenden Verbindungen Ia1.001-Ia1.180 dadurch unterscheiden, daß R¹ für Nitro, R¹⁴ für Ethyl, R¹⁵ für Ethylcarbonyl und R¹⁶ für Methyl stehen: 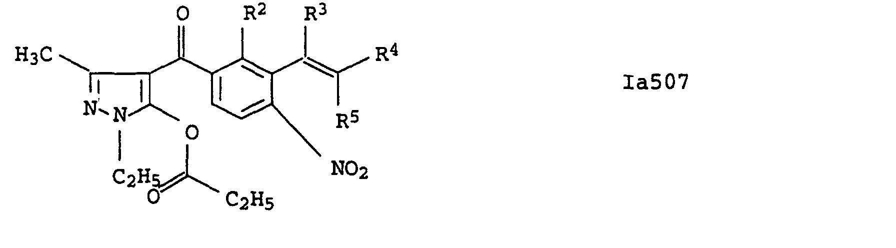
- Ebenso außergewöhnlich bevorzugt sind die Verbindungen Ia508, insbesondere die Verbindungen Ia508.001-Ia508.180, die sich von den entsprechenden Verbindungen Ia1.001-Ia1.180 dadurch unterscheiden, daß R¹ für Nitro, R¹⁴ für n-Propyl, R¹⁵ für Ethylcarbonyl und R¹⁶ für Methyl stehen: 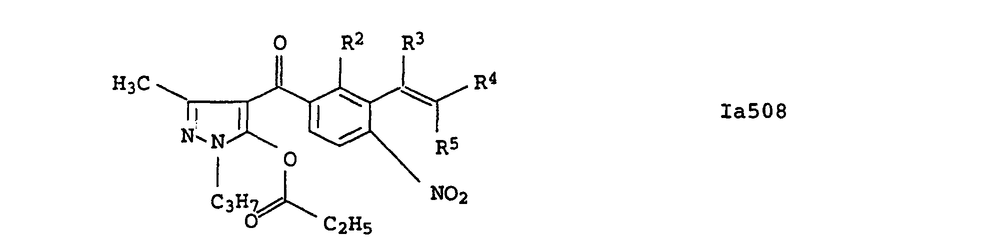
- Ebenso außergewöhnlich bevorzugt sind die Verbindungen Ia509, insbesondere die Verbindungen Ia509.001-Ia509.180, die sich von den entsprechenden Verbindungen Ia1.001-Ia1.180 dadurch unterscheiden, daß R¹ für Nitro, R¹⁴ für n-Butyl, R¹⁵ für Ethylcarbonyl und R¹⁶ für Methyl stehen: 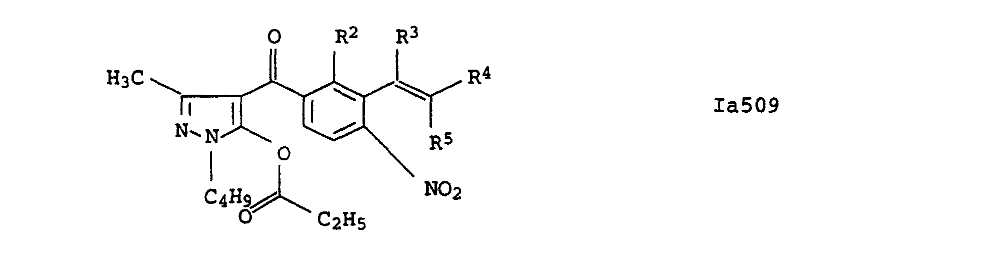
- Ebenso außergewöhnlich bevorzugt sind die Verbindungen Ia510, insbesondere die Verbindungen Ia510.001-Ia510.180, die sich von den entsprechenden Verbindungen Ia1.001-Ia1.180 dadurch unterscheiden, daß R¹ für Nitro, R¹⁴ für iso-Butyl, R¹⁵ für Ethylcarbonyl und R¹⁶ für Methyl stehen: 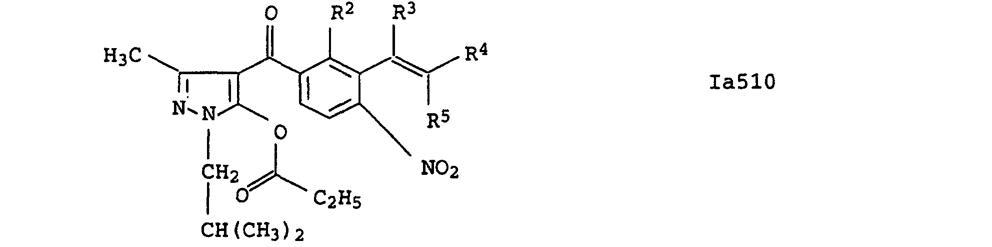
- Ebenso außergewöhnlich bevorzugt sind die Verbindungen Ia511, insbesondere die Verbindungen Ia511.001-Ia511.180, die sich von den entsprechenden Verbindungen Ia1.001-Ia1.180 dadurch unterscheiden, daß R¹ für Nitro, R¹⁵ für n-Propylcarbonyl und R¹⁶ für Methyl stehen: 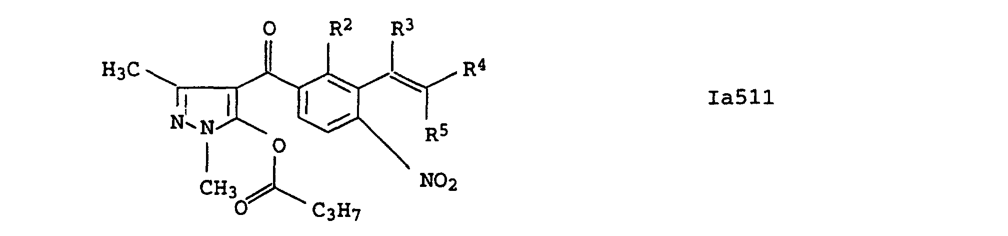
- Ebenso außergewöhnlich bevorzugt sind die Verbindungen Ia512, insbesondere die Verbindungen Ia512.001-Ia512.180, die sich von den entsprechenden Verbindungen Ia1.001-Ia1.180 dadurch unterscheiden, daß R¹ für Nitro, R¹⁴ für Ethyl, R¹⁵ für n-Propylcarbonyl und R¹⁶ für Methyl stehen: 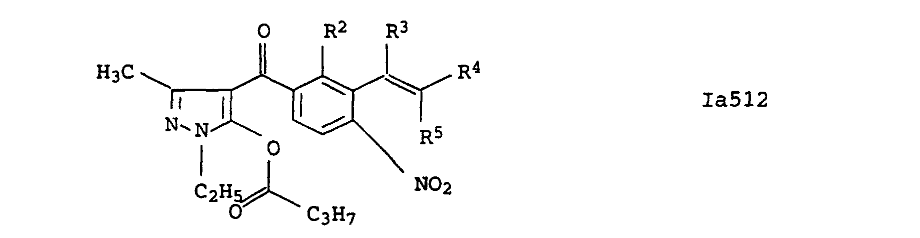
- Ebenso außergewöhnlich bevorzugt sind die Verbindungen Ia513, insbesondere die Verbindungen Ia513.001-Ia513.180, die sich von den entsprechenden Verbindungen Ia1.001-Ia1.180 dadurch unterscheiden, daß R¹ für Nitro, R¹⁴ für n-Propyl, R¹⁵ für n-Propylcarbonyl und R¹⁶ für Methyl stehen: 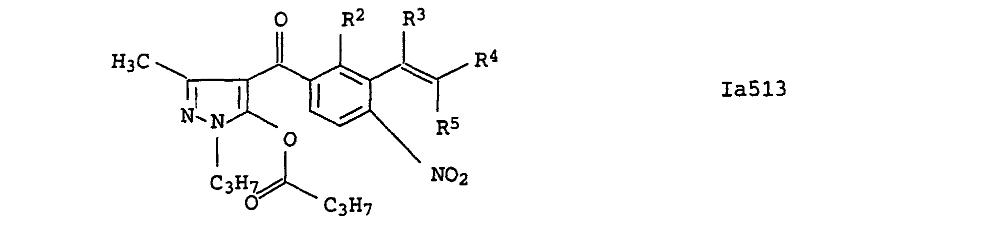
- Ebenso außergewöhnlich bevorzugt sind die Verbindungen Ia514, insbesondere die Verbindungen Ia514.001-Ia514.180, die sich von den entsprechenden Verbindungen Ia1.001-Ia1.180 dadurch unterscheiden, daß R¹ für Nitro, R¹⁴ für n-Butyl, R¹⁵ für n-Propylcarbonyl und R¹⁶ für Methyl stehen: 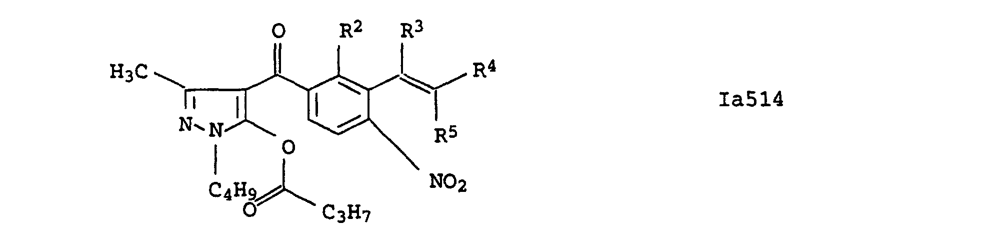
- Ebenso außergewöhnlich bevorzugt sind die Verbindungen Ia515, insbesondere die Verbindungen Ia515.001-Ia515.180, die sich von den entsprechenden Verbindungen Ia1.001-Ia1.180 dadurch unterscheiden, daß R¹ für Nitro, R¹⁴ für iso-Butyl, R¹⁵ für n-Propylcarbonyl und R¹⁶ für Methyl stehen: 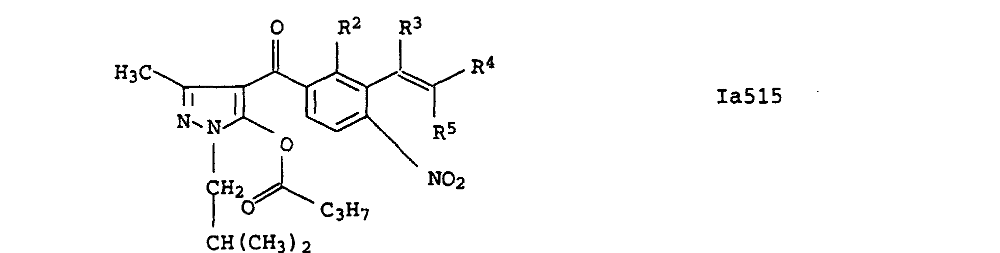
- Ebenso außergewöhnlich bevorzugt sind die Verbindungen Ia516, insbesondere die Verbindungen Ia516.001-Ia516.180, die sich von den entsprechenden Verbindungen Ia1.001-Ia1.180 dadurch unterscheiden, daß R¹ für Nitro, R¹⁵ für Trifluormethylcarbonyl und R¹⁶ für Methyl stehen: 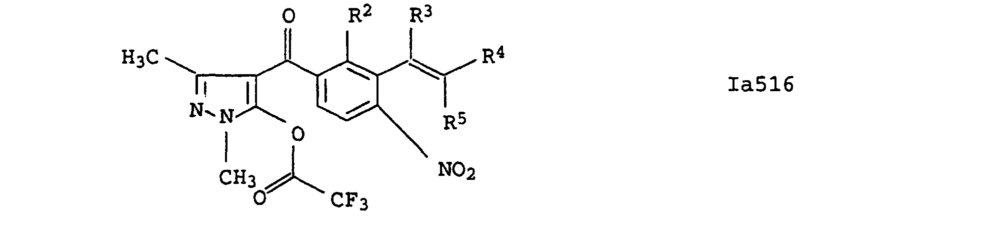
- Ebenso außergewöhnlich bevorzugt sind die Verbindungen Ia517, insbesondere die Verbindungen Ia517.001-Ia517.180, die sich von den entsprechenden Verbindungen Ia1.001-Ia1.180 dadurch unterscheiden, daß R¹ für Nitro, R¹⁴ für Ethyl, R¹⁵ für Trifluormethylcarbonyl und R¹⁶ für Methyl stehen: 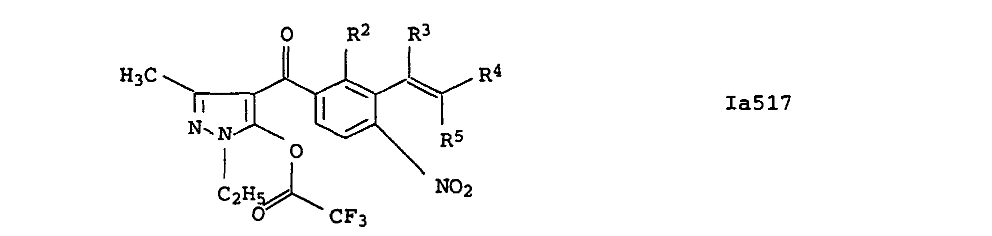
- Ebenso außergewöhnlich bevorzugt sind die Verbindungen Ia518, insbesondere die Verbindungen Ia518.001-Ia518.180, die sich von den entsprechenden Verbindungen Ia1.001-Ia1.180 dadurch unterscheiden, daß R¹ für Nitro, R¹⁴ für n-Propyl, R¹⁵ für Trifluormethylcarbonyl und R¹⁶ für Methyl stehen: 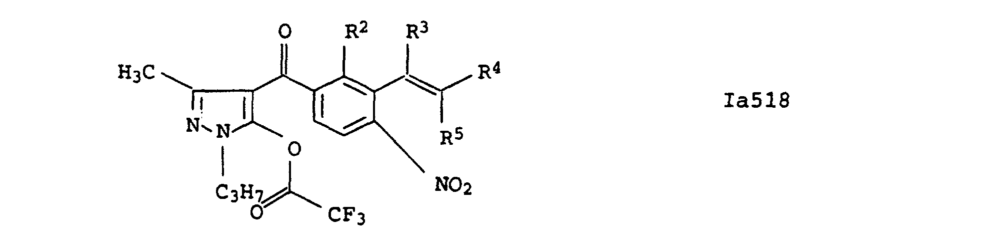
- Ebenso außergewöhnlich bevorzugt sind die Verbindungen Ia519, insbesondere die Verbindungen Ia519.001-Ia519.180, die sich von den entsprechenden Verbindungen Ia1.001-Ia1.180 dadurch unterscheiden, daß R¹ für Nitro, R¹⁴ für n-Butyl, R¹⁵ für Trifluormethylcarbonyl und R¹⁶ für Methyl stehen: 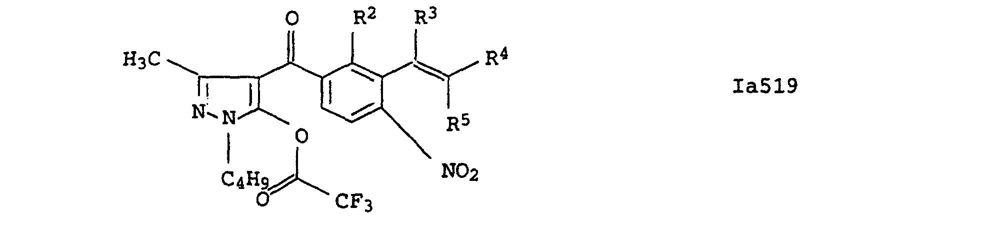
- Ebenso außergewöhnlich bevorzugt sind die Verbindungen Ia520, insbesondere die Verbindungen Ia520.001-Ia520.180, die sich von den entsprechenden Verbindungen Ia1.001-Ia1.180 dadurch unterscheiden, daß R¹ für Nitro, R¹⁴ für iso-Butyl, R¹⁵ für Trifluormethylcarbonyl und R¹⁶ für Methyl stehen: 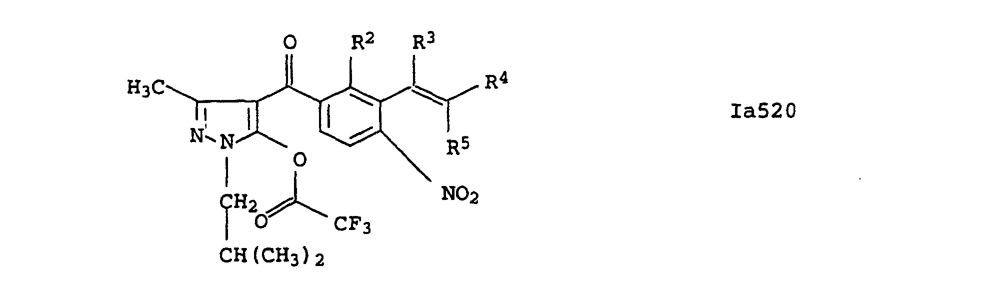
- Ebenso außergewöhnlich bevorzugt sind die Verbindungen Ia521, insbesondere die Verbindungen Ia521.001-Ia521.180, die sich von den entsprechenden Verbindungen Ia1.001-Ia1.180 dadurch unterscheiden, daß R¹ für Nitro, R¹⁵ für Methylsulfonyl und R¹⁶ für Methyl stehen: 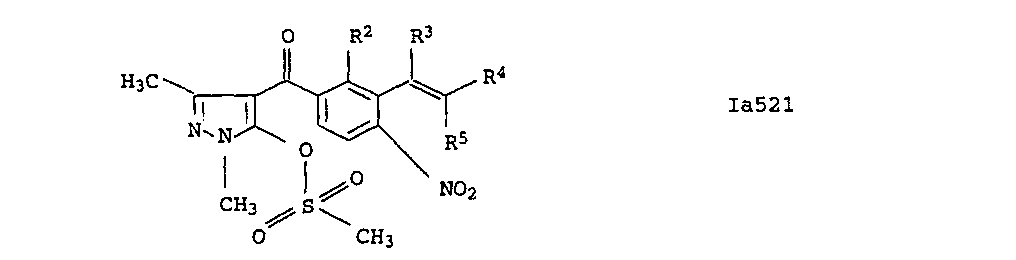
- Ebenso außergewöhnlich bevorzugt sind die Verbindungen Ia522, insbesondere die Verbindungen Ia522.001-Ia522.180, die sich von den entsprechenden Verbindungen Ia1.001-Ia1.180 dadurch unterscheiden, daß R¹ für Nitro, R¹⁴ für Ethyl, R¹⁵ für Methylsulfonyl und R¹⁶ für Methyl stehen: 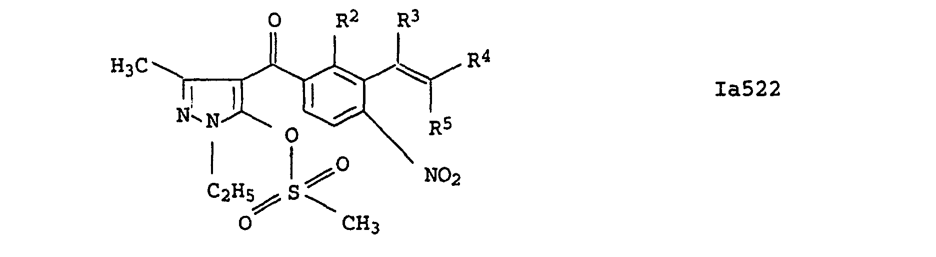
- Ebenso außergewöhnlich bevorzugt sind die Verbindungen Ia523, insbesondere die Verbindungen Ia523.001-Ia523.180, die sich von den entsprechenden Verbindungen Ia1.001-Ia1.180 dadurch unterscheiden, daß R¹ für Nitro, R¹⁴ für n-Propyl, R¹⁵ für Methylsulfonyl und R¹⁶ für Methyl stehen: 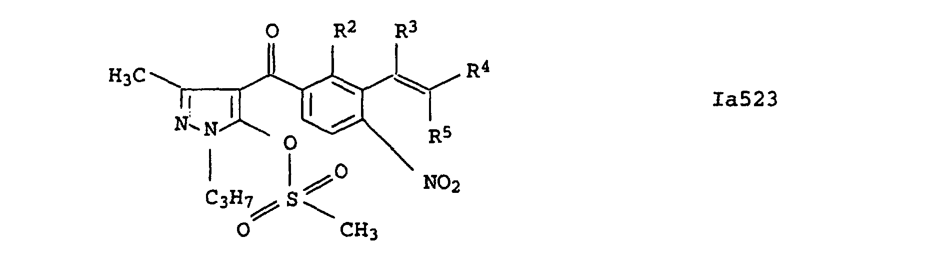
- Ebenso außergewöhnlich bevorzugt sind die Verbindungen Ia524, insbesondere die Verbindungen Ia524.001-Ia524.180, die sich von den entsprechenden Verbindungen Ia1.001-Ia1.180 dadurch unterscheiden, daß R¹ für Nitro, R¹⁴ für n-Butyl, R¹⁵ für Methylsulfonyl und R¹⁶ für Methyl stehen: 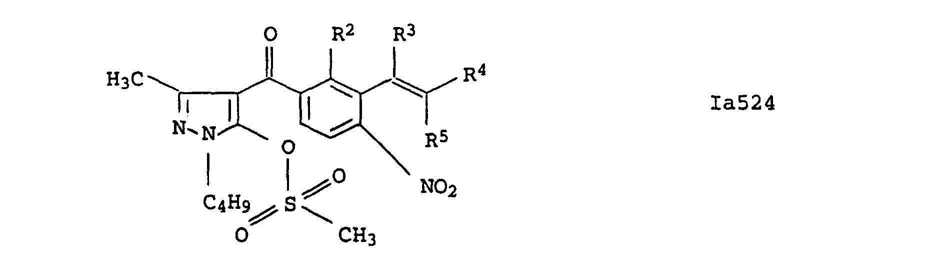
- Ebenso außergewöhnlich bevorzugt sind die Verbindungen Ia525, insbesondere die Verbindungen Ia525.001-Ia525.180, die sich von den entsprechenden Verbindungen Ia1.001-Ia1.180 dadurch unterscheiden, daß R¹ für Nitro, R¹⁴ für iso-Butyl, R¹⁵ für Methylsulfonyl und R¹⁶ für Methyl stehen: 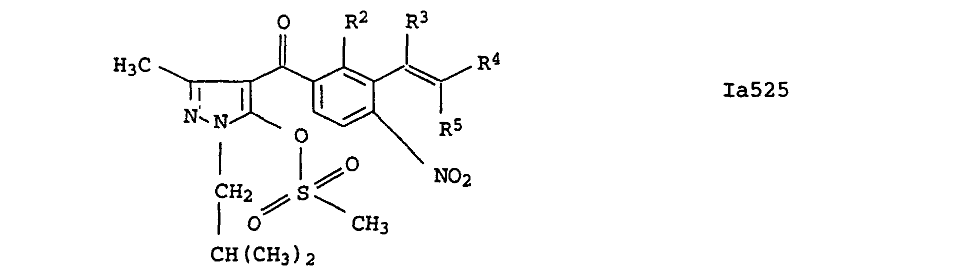
- Ebenso außergewöhnlich bevorzugt sind die Verbindungen Ia526, insbesondere die Verbindungen Ia526.001-Ia526.180, die sich von den entsprechenden Verbindungen Ia1.001-Ia1.180 dadurch unterscheiden, daß R¹ für Nitro, R¹⁵ für Ethylsulfonyl und R¹⁶ für Methyl stehen: 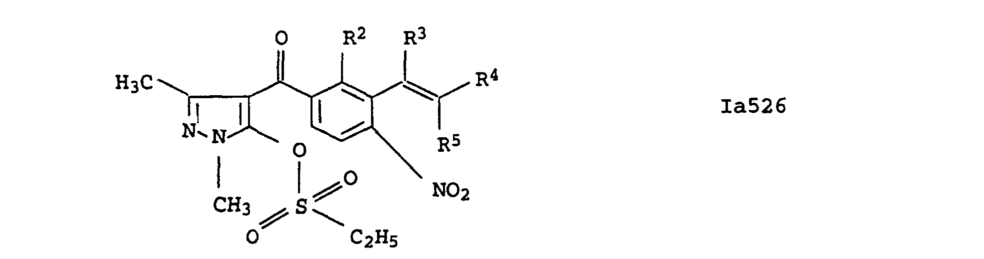
- Ebenso außergewöhnlich bevorzugt sind die Verbindungen Ia527, insbesondere die Verbindungen Ia527.001-Ia527.180, die sich von den entsprechenden Verbindungen Ia1.001-Ia1.180 dadurch unterscheiden, daß R¹ für Nitro, R¹⁴ für Ethyl, R¹⁵ für Ethylsulfonyl und R¹⁶ für Methyl stehen: 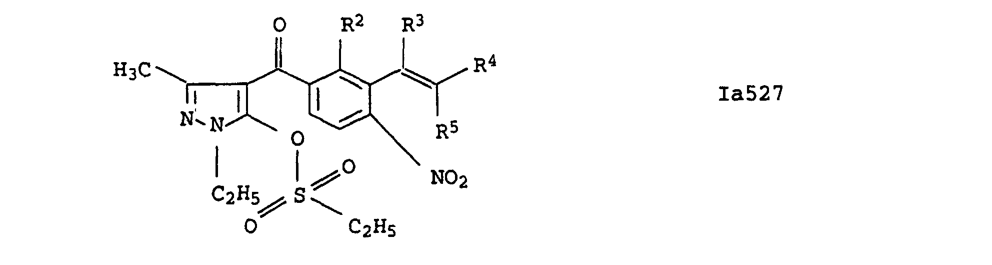
- Ebenso außergewöhnlich bevorzugt sind die Verbindungen Ia528, insbesondere die Verbindungen Ia528.001-Ia528.180, die sich von den entsprechenden Verbindungen Ia1.001-Ia1.180 dadurch unterscheiden, daß R¹ für Nitro, R¹⁴ für n-Propyl, R¹⁵ für Ethylsulfonyl und R¹⁶ für Methyl stehen: 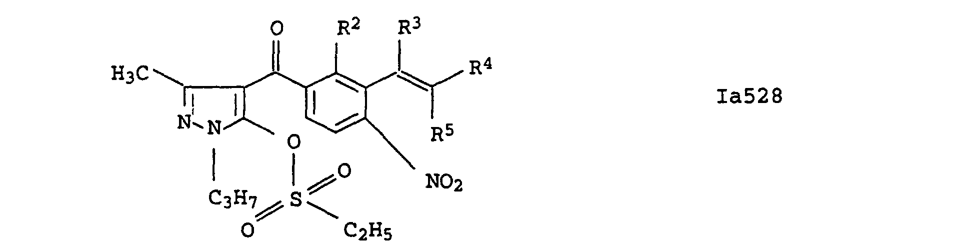
- Ebenso außergewöhnlich bevorzugt sind die Verbindungen Ia529, insbesondere die Verbindungen Ia529.001-Ia529.180, die sich von den entsprechenden Verbindungen Ia1.001-Ia1.180 dadurch unterscheiden, daß R¹ für Nitro, R¹⁴ für n-Butyl, R¹⁵ für Ethylsulfonyl und R¹⁶ für Methyl stehen: 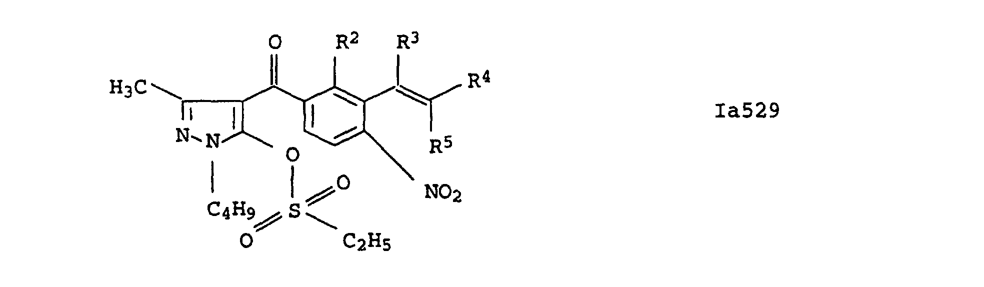
- Ebenso außergewöhnlich bevorzugt sind die Verbindungen Ia530, insbesondere die Verbindungen Ia530.001-Ia530.180, die sich von den entsprechenden Verbindungen Ia1.001-Ia1.180 dadurch unterscheiden, daß R¹ für Nitro, R¹⁴ für iso-Butyl, R¹⁵ für Ethylsulfonyl und R¹⁶ für Methyl stehen: 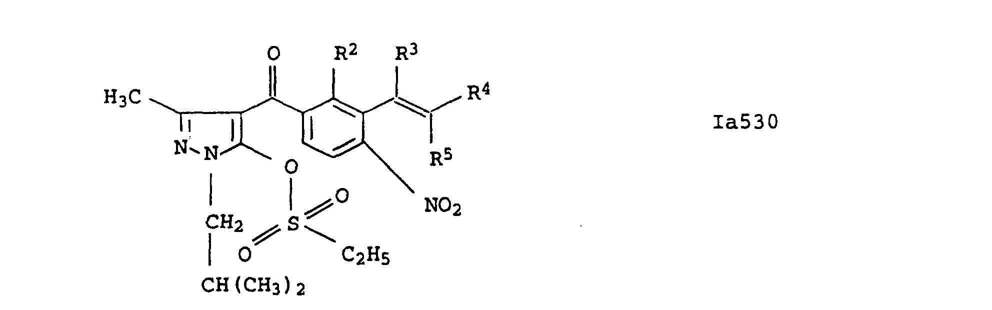
- Ebenso außergewöhnlich bevorzugt sind die Verbindungen Ia531, insbesondere die Verbindungen Ia531.001-Ia531.180, die sich von den entsprechenden Verbindungen Ia1.001-Ia1.180 dadurch unterscheiden, daß R¹ für Nitro, R¹⁵ für n-Propylsulfonyl und R¹⁶ für Methyl stehen: 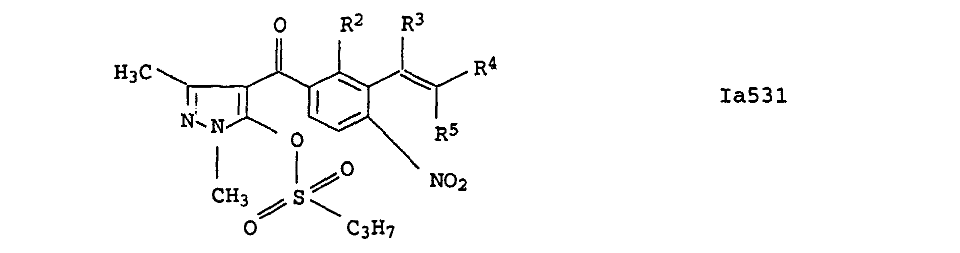
- Ebenso außergewöhnlich bevorzugt sind die Verbindungen Ia532, insbesondere die Verbindungen Ia532.001-Ia532.180, die sich von den entsprechenden Verbindungen Ia1.001-Ia1.180 dadurch unterscheiden, daß R¹ für Nitro, R¹⁴ für Ethyl, R¹⁵ für n-Propylsulfonyl und R¹⁶ für Methyl stehen: 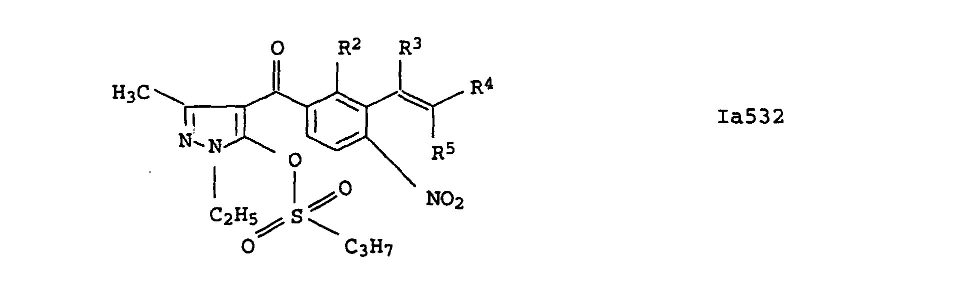
- Ebenso außergewöhnlich bevorzugt sind die Verbindungen Ia533, insbesondere die Verbindungen Ia533.001-Ia533.180, die sich von den entsprechenden Verbindungen Ia1.001-Ia1.180 dadurch unterscheiden, daß R¹ für Nitro, R¹⁴ für n-Propyl, R¹⁵ für n-Propylsulfonyl und R¹⁶ für Methyl stehen: 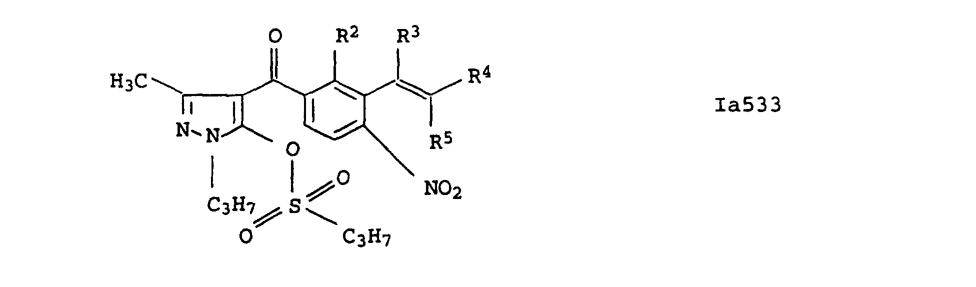
- Ebenso außergewöhnlich bevorzugt sind die Verbindungen Ia534, insbesondere die Verbindungen Ia534.001-Ia534.180, die sich von den entsprechenden Verbindungen Ia1.001-Ia1.180 dadurch unterscheiden, daß R¹ für Nitro, R¹⁴ für n-Butyl, R¹⁵ für n-Propylsulfonyl und R¹⁶ für Methyl stehen: 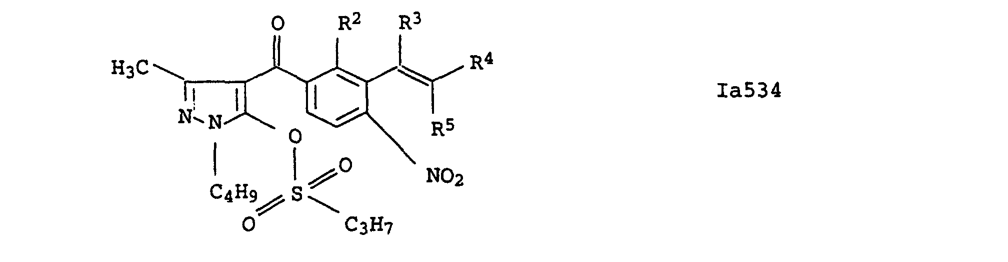
- Ebenso außergewöhnlich bevorzugt sind die Verbindungen Ia535, insbesondere die Verbindungen Ia535.001-Ia535.180, die sich von den entsprechenden Verbindungen Ia1.001-Ia1.180 dadurch unterscheiden, daß R¹ für Nitro, R¹⁴ für iso-Butyl, R¹⁵ für n-Propylsulfonyl und R¹⁶ für Methyl stehen: 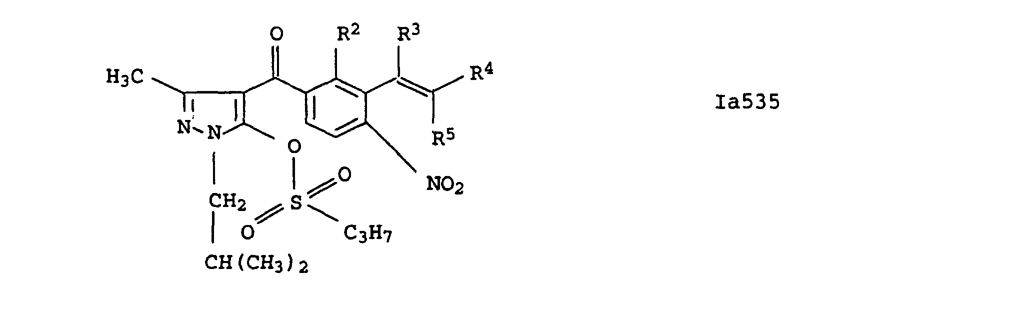
- Ebenso außergewöhnlich bevorzugt sind die Verbindungen Ia536, insbesondere die Verbindungen Ia536.001-Ia536.180, die sich von den entsprechenden Verbindungen Ia1.001-Ia1.180 dadurch unterscheiden, daß R¹ für Nitro, R¹⁵ für iso-Propylsulfonyl und R¹⁶ für Methyl stehen: 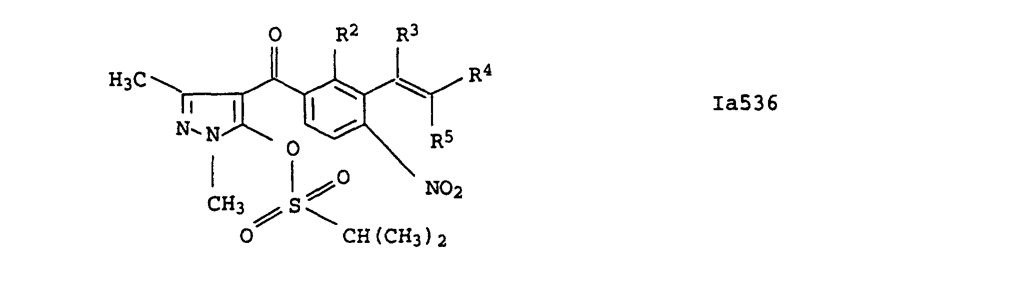
- Ebenso außergewöhnlich bevorzugt sind die Verbindungen Ia537, insbesondere die Verbindungen Ia537.001-Ia537.180, die sich von den entsprechenden Verbindungen Ia1.001-Ia1.180 dadurch unterscheiden, daß R¹ für Nitro, R¹⁴ für Ethyl, R¹⁵ für iso-Propylsulfonyl und R¹⁶ für Methyl stehen: 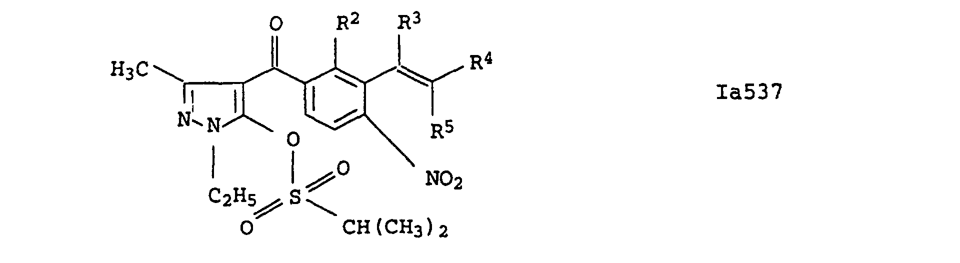
- Ebenso außergewöhnlich bevorzugt sind die Verbindungen Ia538, insbesondere die Verbindungen Ia538.001-Ia538.180, die sich von den entsprechenden Verbindungen Ia1.001-Ia1.180 dadurch unterscheiden, daß R¹ für Nitro, R¹⁴ für n-Propyl, R¹⁵ für iso-Propylsulfonyl und R¹⁶ für Methyl stehen: 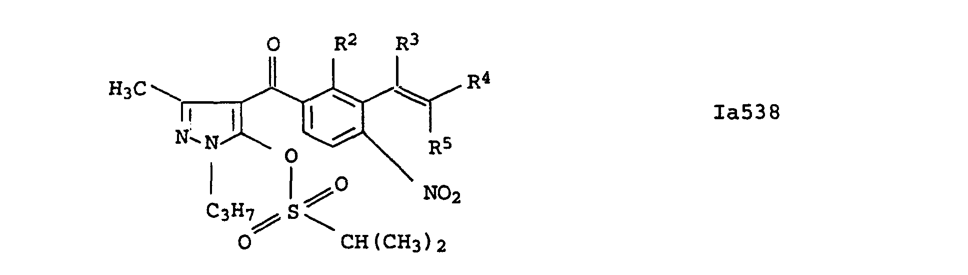
- Ebenso außergewöhnlich bevorzugt sind die Verbindungen Ia539, insbesondere die Verbindungen Ia539.001-Ia539.180, die sich von den entsprechenden Verbindungen Ia1.001-Ia1.180 dadurch unterscheiden, daß R¹ für Nitro, R¹⁴ für n-Butyl, R¹⁵ für iso-Propylsulfonyl und R¹⁶ für Methyl stehen: 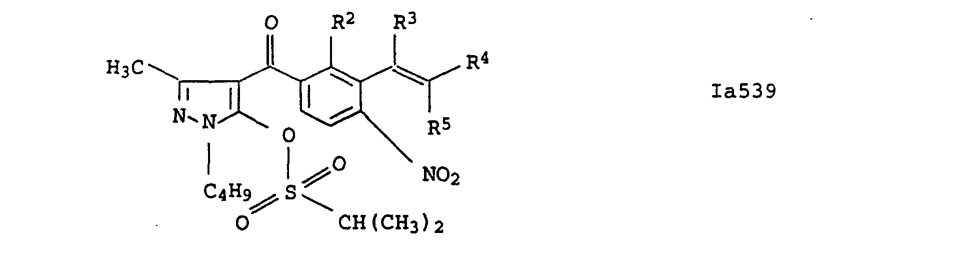
- Ebenso außergewöhnlich bevorzugt sind die Verbindungen Ia540, insbesondere die Verbindungen Ia540.001-Ia540.180, die sich von den entsprechenden Verbindungen Ia1.001-Ia1.180 dadurch unterscheiden, daß R¹ für Nitro, R¹⁴ für iso-Butyl, R¹⁵ für iso-Propylsulfonyl und R¹⁶ für Methyl stehen: 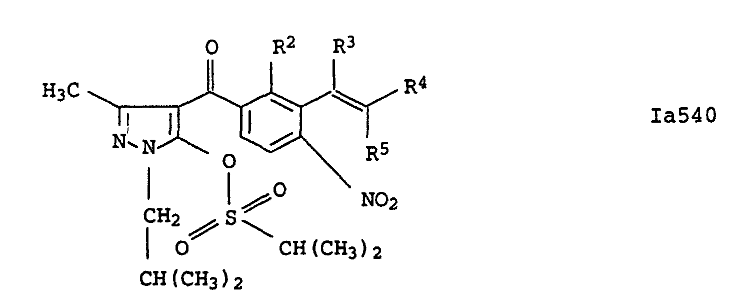
- Ebenso außergewöhnlich bevorzugt sind die Verbindungen Ia541, insbesondere die Verbindungen Ia541.001-Ia541.180, die sich von den entsprechenden Verbindungen Ia1.001-Ia1.180 dadurch unterscheiden, daß R¹ für Nitro, R¹⁵ für n-Butylsulfonyl und R¹⁶ für Methyl stehen: 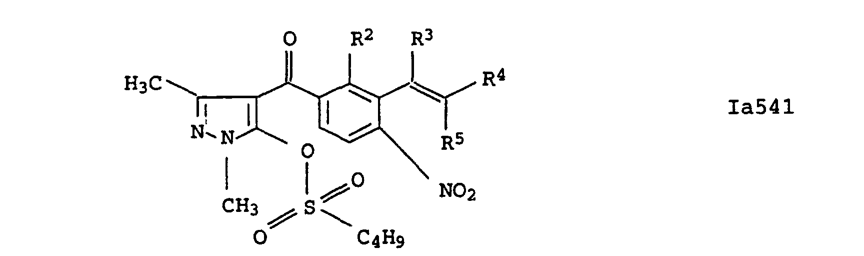
- Ebenso außergewöhnlich bevorzugt sind die Verbindungen Ia542, insbesondere die Verbindungen Ia542.001-Ia542.180, die sich von den entsprechenden Verbindungen Ia1.001-Ia1.180 dadurch unterscheiden, daß R¹ für Nitro, R¹⁴ für Ethyl, R¹⁵ für n-Butylsulfonyl und R¹⁶ für Methyl stehen: 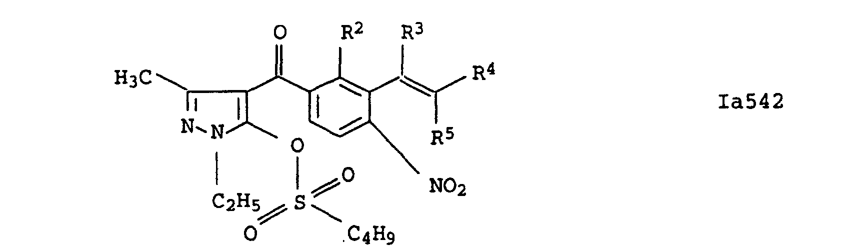
- Ebenso außergewöhnlich bevorzugt sind die Verbindungen Ia543, insbesondere die Verbindungen Ia543.001-Ia543.180, die sich von den entsprechenden Verbindungen Ia1.001-Ia1.180 dadurch unterscheiden, daß R¹ für Nitro, R¹⁴ für n-Propyl, R¹⁵ für n-Butylsulfonyl und R¹⁶ für Methyl stehen: 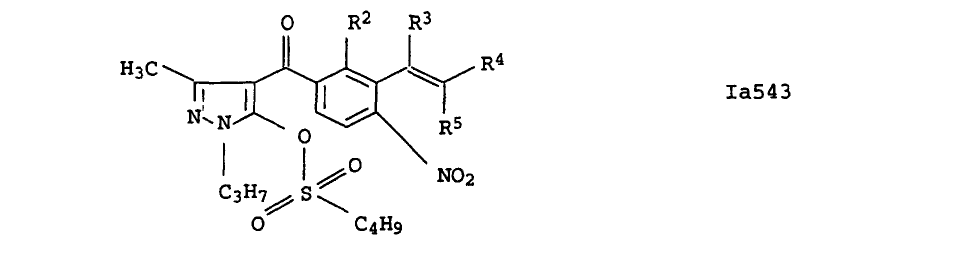
- Ebenso außergewöhnlich bevorzugt sind die Verbindungen Ia544, insbesondere die Verbindungen Ia544.001-Ia544.180, die sich von den entsprechenden Verbindungen Ia1.001-Ia1.180 dadurch unterscheiden, daß R¹ für Nitro, R¹⁴ für n-Butyl, R¹⁵ für n-Butylsulfonyl und R¹⁶ für Methyl stehen: 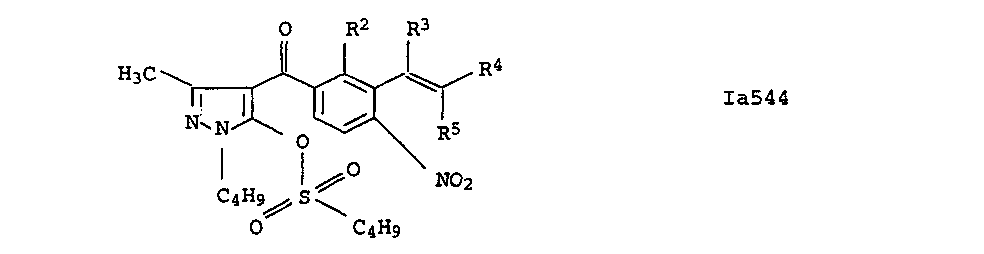
- Ebenso außergewöhnlich bevorzugt sind die Verbindungen Ia545, insbesondere die Verbindungen Ia545.001-Ia545.180, die sich von den entsprechenden Verbindungen Ia1.001-Ia1.180 dadurch unterscheiden, daß R¹ für Nitro, R¹⁴ für iso-Butyl, R¹⁵ für n-Butylsulfonyl und R¹⁶ für Methyl stehen: 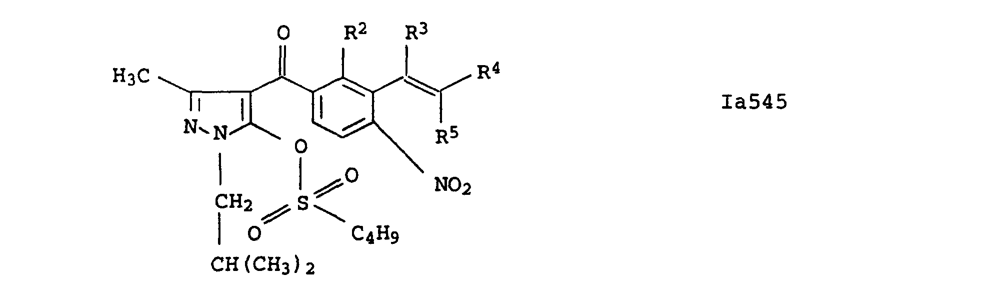
- Ebenso außergewöhnlich bevorzugt sind die Verbindungen Ia546, insbesondere die Verbindungen Ia546.001-Ia546.180, die sich von den entsprechenden Verbindungen Ia1.001-Ia1.180 dadurch unterscheiden, daß R¹ für Nitro, R¹⁵ für iso-Butylsulfonyl und R¹⁶ für Methyl stehen: 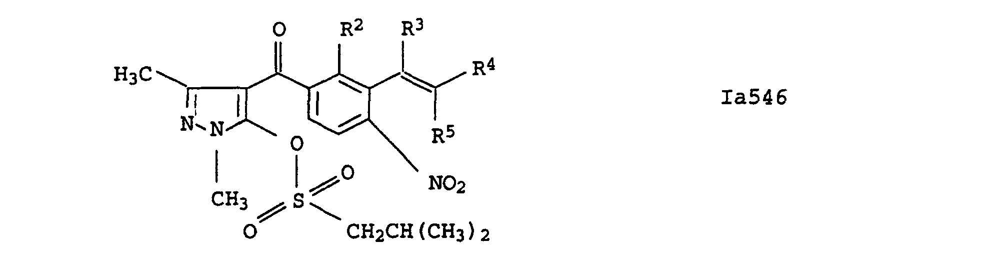
- Ebenso außergewöhnlich bevorzugt sind die Verbindungen Ia547, insbesondere die Verbindungen Ia547.001-Ia547.180, die sich von den entsprechenden Verbindungen Ia1.001-Ia1.180 dadurch unterscheiden, daß R¹ für Nitro, R¹⁴ für Ethyl, R¹⁵ für iso-Butylsulfonyl und R¹⁶ für Methyl stehen: 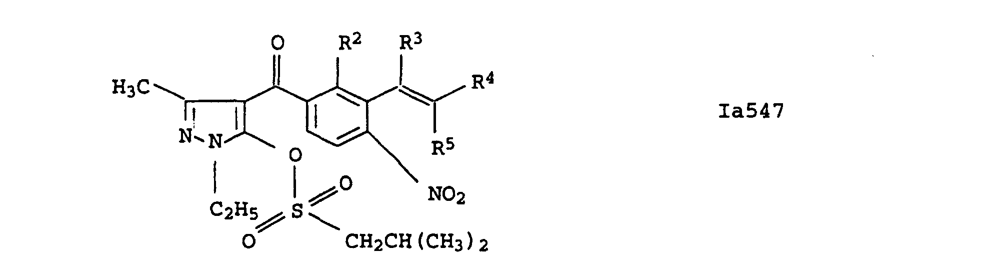
- Ebenso außergewöhnlich bevorzugt sind die Verbindungen Ia548, insbesondere die Verbindungen Ia548.001-Ia548.180, die sich von den entsprechenden Verbindungen Ia1.001-Ia1.180 dadurch unterscheiden, daß R¹ für Nitro, R¹⁴ für n-Propyl, R¹⁵ für iso-Butylsulfonyl und R¹⁶ für Methyl stehen: 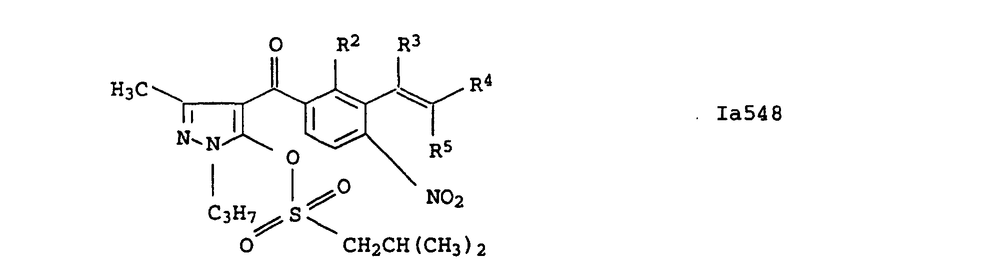
- Ebenso außergewöhnlich bevorzugt sind die Verbindungen Ia549, insbesondere die Verbindungen Ia549.001-Ia549.180, die sich von den entsprechenden Verbindungen Ia1.001-Ia1.180 dadurch unterscheiden, daß R¹ für Nitro, R¹⁴ für n-Butyl, R¹⁵ für iso-Butylsulfonyl und R¹⁶ für Methyl stehen: 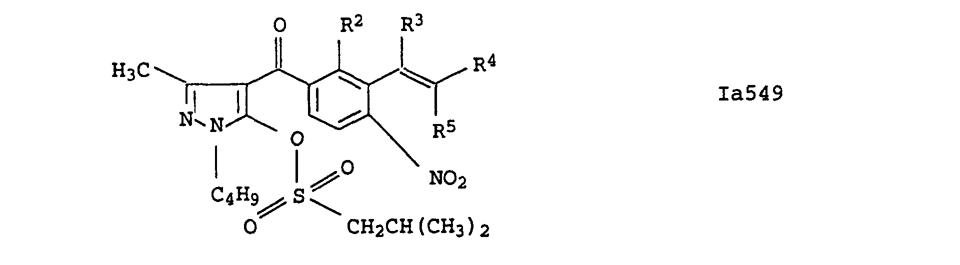
- Ebenso außergewöhnlich bevorzugt sind die Verbindungen Ia550, insbesondere die Verbindungen Ia550.001-Ia550.180, die sich von den entsprechenden Verbindungen Ia1.001-Ia1.180 dadurch unterscheiden, daß R¹ für Nitro, R¹⁴ für iso-Butyl, R¹⁵ für iso-Butylsulfonyl und R¹⁶ für Methyl stehen: 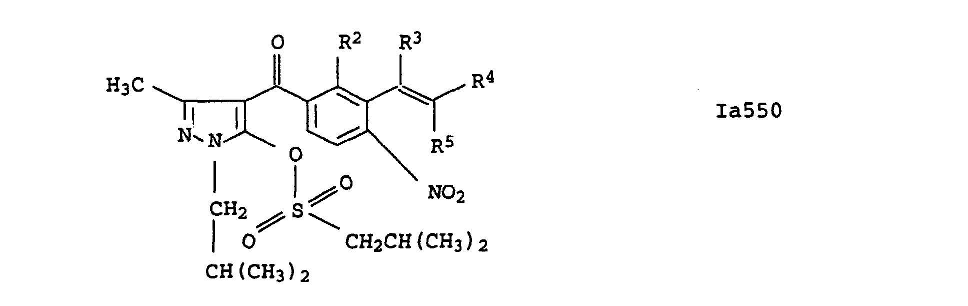
- Ebenso außergewöhnlich bevorzugt sind die Verbindungen Ia551, insbesondere die Verbindungen Ia551.001-Ia551.180, die sich von den entsprechenden Verbindungen Ia1.001-Ia1.180 dadurch unterscheiden, daß R¹ für Nitro, R¹⁵ für sec-Butylsulfonyl und R¹⁶ für Methyl stehen: 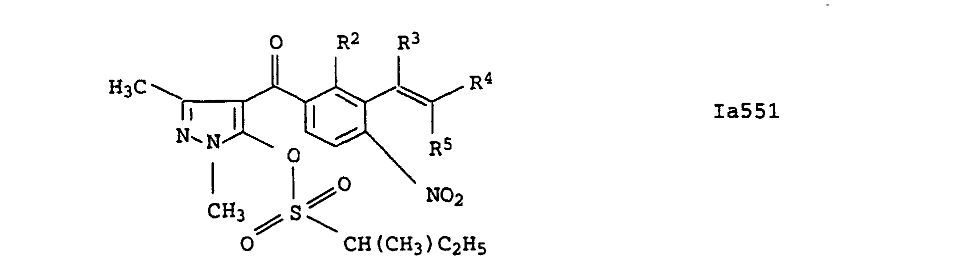
- Ebenso außergewöhnlich bevorzugt sind die Verbindungen Ia552, insbesondere die Verbindungen Ia552.001-Ia552.180, die sich von den entsprechenden Verbindungen Ia1.001-Ia1.180 dadurch unterscheiden, daß R¹ für Nitro, R¹⁴ für Ethyl, R¹⁵ für sec-Butylsulfonyl und R¹⁶ für Methyl stehen: 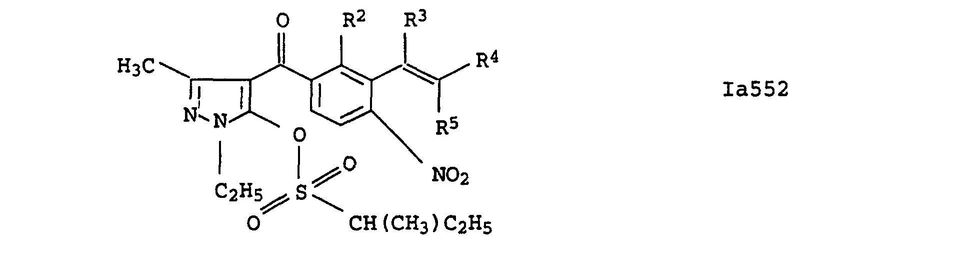
- Ebenso außergewöhnlich bevorzugt sind die Verbindungen Ia553, insbesondere die Verbindungen Ia553.001-Ia553.180, die sich von den entsprechenden Verbindungen Ia1.001-Ia1.180 dadurch unterscheiden, daß R¹ für Nitro, R¹⁴ für n-Propyl, R¹⁵ für sec-Butylsulfonyl und R¹⁶ für Methyl stehen: 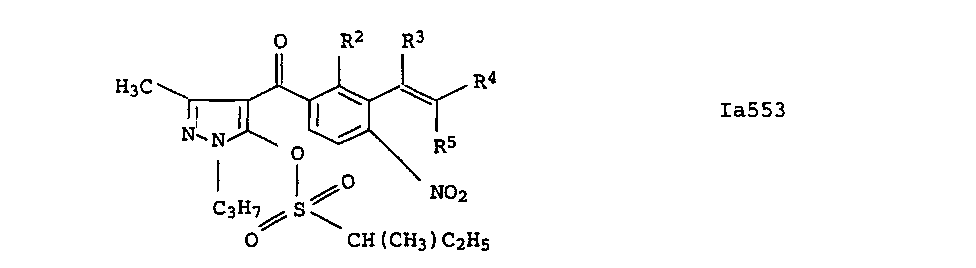
- Ebenso außergewöhnlich bevorzugt sind die Verbindungen Ia554, insbesondere die Verbindungen Ia554.001-Ia554.180, die sich von den entsprechenden Verbindungen Ia1.001-Ia1.180 dadurch unterscheiden, daß R¹ für Nitro, R¹⁴ für n-Butyl, R¹⁵ für sec-Butylsulfonyl und R¹⁶ für Methyl stehen: 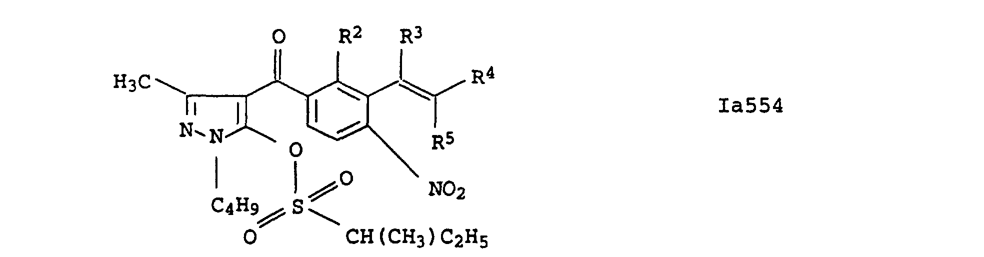
- Ebenso außergewöhnlich bevorzugt sind die Verbindungen Ia555, insbesondere die Verbindungen Ia555.001-Ia555.180, die sich von den entsprechenden Verbindungen Ia1.001-Ia1.180 dadurch unterscheiden, daß R¹ für Nitro, R¹⁴ für iso-Butyl, R¹⁵ für sec-Butylsulfonyl und R¹⁶ für Methyl stehen: 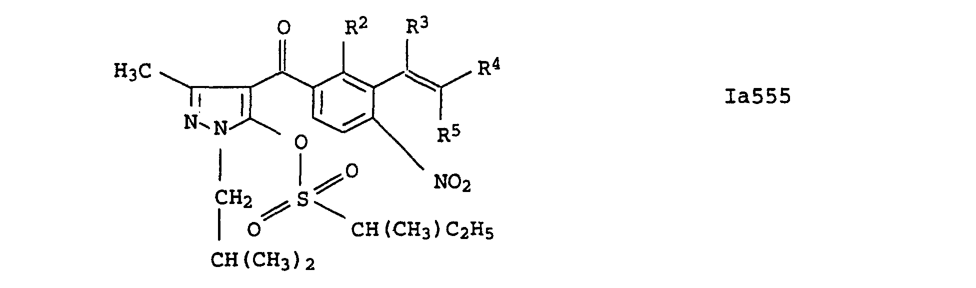
- Ebenso außergewöhnlich bevorzugt sind die Verbindungen Ia556, insbesondere die Verbindungen Ia556.001-Ia556.180, die sich von den entsprechenden Verbindungen Ia1.001-Ia1.180 dadurch unterscheiden, daß R¹ für Nitro, R¹⁵ für Trifluormethylsulfonyl und R¹⁶ für Methyl stehen: 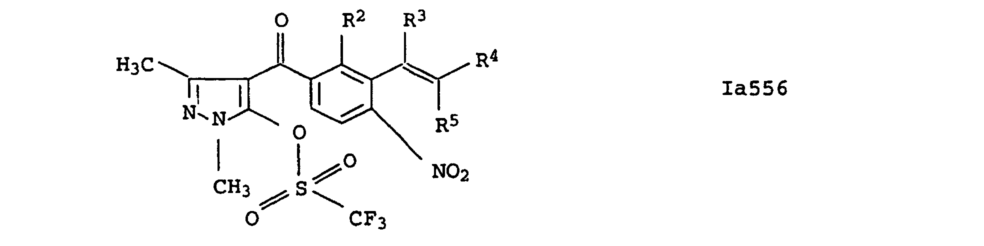
- Ebenso außergewöhnlich bevorzugt sind die Verbindungen Ia557, insbesondere die Verbindungen Ia557.001-Ia557.180, die sich von den entsprechenden Verbindungen Ia1.001-Ia1.180 dadurch unterscheiden, daß R¹ für Nitro, R¹⁴ für Ethyl, R¹⁵ für Trifluormethylsulfonyl und R¹⁶ für Methyl stehen: 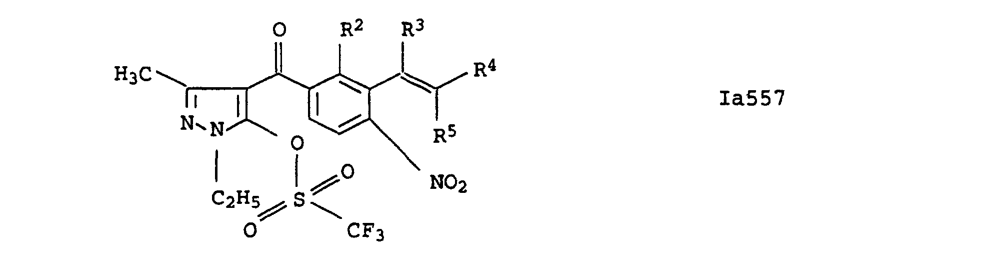
- Ebenso außergewöhnlich bevorzugt sind die Verbindungen Ia558, insbesondere die Verbindungen Ia558.001-Ia558.180, die sich von den entsprechenden Verbindungen Ia1.001-Ia1.180 dadurch unterscheiden, daß R¹ für Nitro, R¹⁴ für n-Propyl, R¹⁵ für Trifluormethylsulfonyl und R¹⁶ für Methyl stehen: 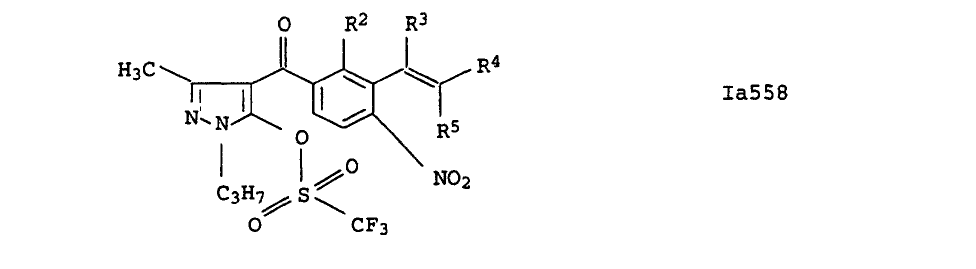
- Ebenso außergewöhnlich bevorzugt sind die Verbindungen Ia559, insbesondere die Verbindungen Ia559.001-Ia559.180, die sich von den entsprechenden Verbindungen Ia1.001-Ia2.180 dadurch unterscheiden, daß R¹ für Nitro, R¹⁴ für n-Butyl, R¹⁵ für Trifluormethylsulfonyl und R¹⁶ für Methyl stehen: 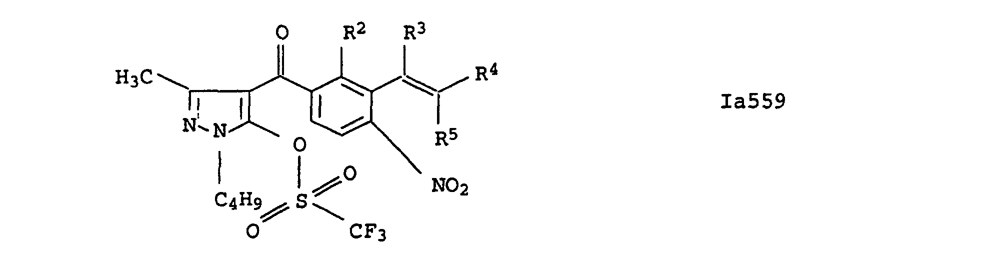
- Ebenso außergewöhnlich bevorzugt sind die Verbindungen Ia560, insbesondere die Verbindungen Ia560.001-Ia560.180, die sich von den entsprechenden Verbindungen Ia1.001-Ia1.180 dadurch unterscheiden, daß R¹ für Nitro, R¹⁴ für iso-Butyl, R¹⁵ für Trifluormethylsulfonyl und R¹⁶ für Methyl stehen: 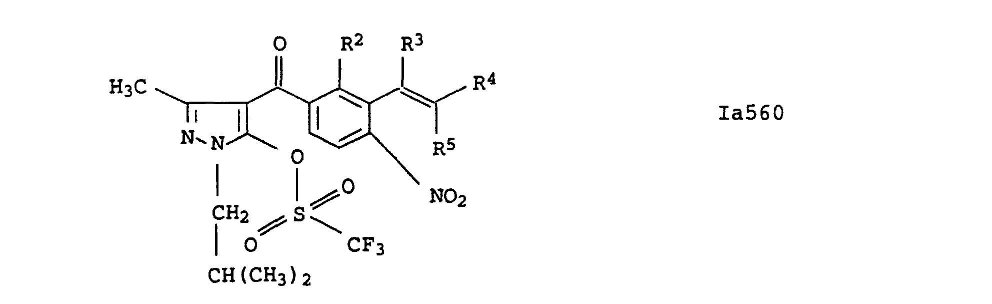
- Ebenso außergewöhnlich bevorzugt sind die Verbindungen Ia561, insbesondere die Verbindungen Ia561.001-Ia561.180, die sich von den entsprechenden Verbindungen Ia1.001-Ia1.180 dadurch unterscheiden, daß R¹ für Nitro, R¹⁵ für Phenylcarbonylmethyl und R¹⁶ für Methyl stehen: 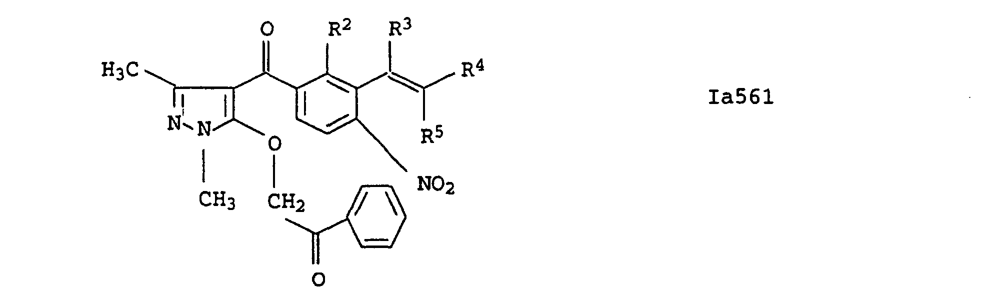
- Ebenso außergewöhnlich bevorzugt sind die Verbindungen Ia562, insbesondere die Verbindungen Ia562.001-Ia562.180, die sich von den entsprechenden Verbindungen Ia1.001-Ia1.180 dadurch unterscheiden, daß R¹ für Nitro, R¹⁴ für Ethyl, R¹⁵ für Phenylcarbonylmethyl und R¹⁶ für Methyl stehen: 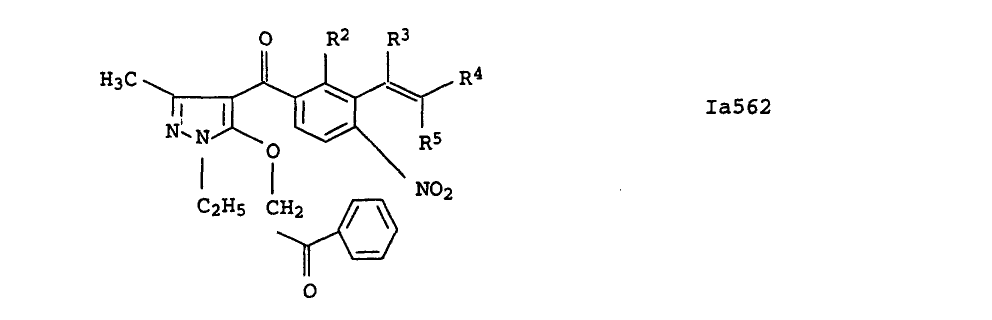
- Ebenso außergewöhnlich bevorzugt sind die Verbindungen Ia563, insbesondere die Verbindungen Ia563.001-Ia563.180, die sich von den entsprechenden Verbindungen Ia1.001-Ia1.180 dadurch unterscheiden, daß R¹ für Nitro, R¹⁴ für n-Propyl, R¹⁵ für Phenylcarbonylmethyl und R¹⁶ für Methyl stehen: 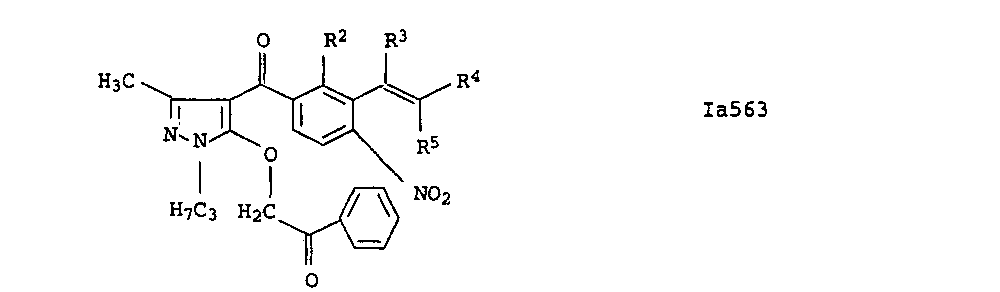
- Ebenso außergewöhnlich bevorzugt sind die Verbindungen Ia564, insbesondere die Verbindungen Ia564.001-Ia564.180, die sich von den entsprechenden Verbindungen Ia1.001-Ia1.180 dadurch unterscheiden, daß R¹ für Nitro, R¹⁴ für n-Butyl, R¹⁵ für Phenylcarbonylmethyl und R¹⁶ für Methyl stehen: 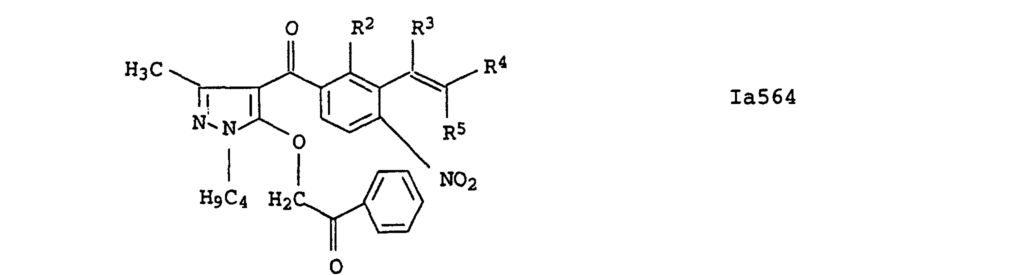
- Ebenso außergewöhnlich bevorzugt sind die Verbindungen Ia565, insbesondere die Verbindungen Ia565.001-Ia565.180, die sich von den entsprechenden Verbindungen Ia1.001-Ia1.180 dadurch unterscheiden, daß R¹ für Nitro, R¹⁴ für iso-Butyl, R¹⁵ für Phenylcarbonylmethyl und R¹⁶ für Methyl stehen: 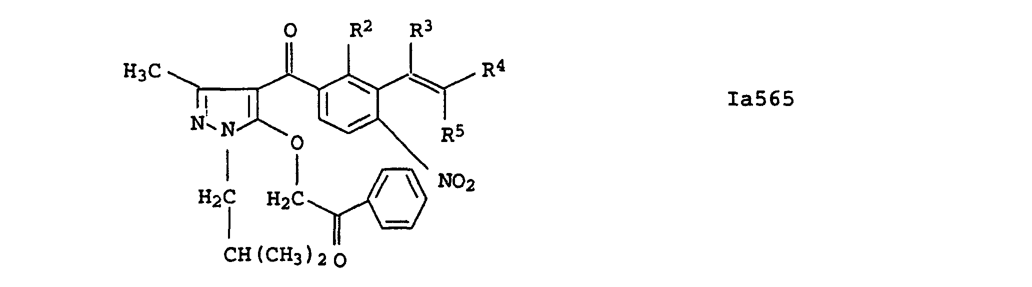
- Ebenso außergewöhnlich bevorzugt sind die Verbindungen Ia566, insbesondere die Verbindungen Ia566.001-Ia566.180, die sich von den entsprechenden Verbindungen Ia1.001-Ia1.180 dadurch unterscheiden, daß R¹ für Nitro, R¹⁵ für Phenylsulfonyl und R¹⁶ für Methyl stehen: 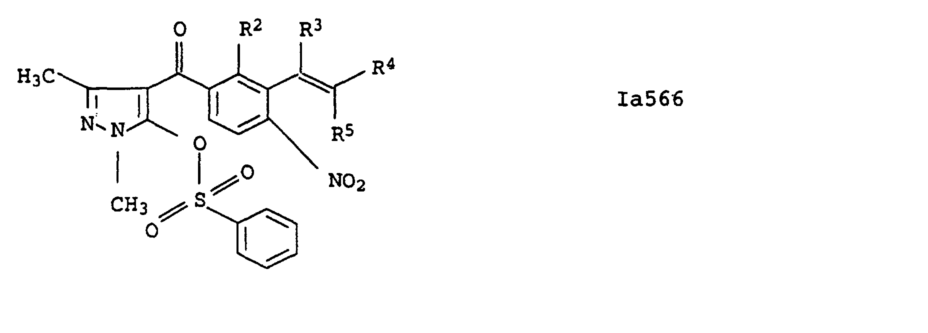
- Ebenso außergewöhnlich bevorzugt sind die Verbindungen Ia567, insbesondere die Verbindungen Ia567.001-Ia567.180, die sich von den entsprechenden Verbindungen Ia1.001-Ia1.180 dadurch unterscheiden, daß R¹ für Nitro, R¹⁴ für Ethyl, R¹⁵ für Phenylsulfonyl und R¹⁶ für Methyl stehen: 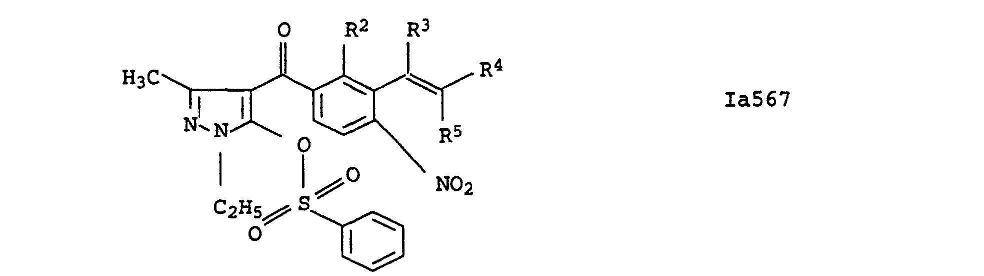
- Ebenso außergewöhnlich bevorzugt sind die Verbindungen Ia568, insbesondere die Verbindungen Ia568.001-Ia568.180, die sich von den entsprechenden Verbindungen Ia1.001-Ia1.180 dadurch unterscheiden, daß R¹ für Nitro, R¹⁴ für Ethyl, R¹⁵ für Phenylsulfonyl und R¹⁶ für Methyl stehen: 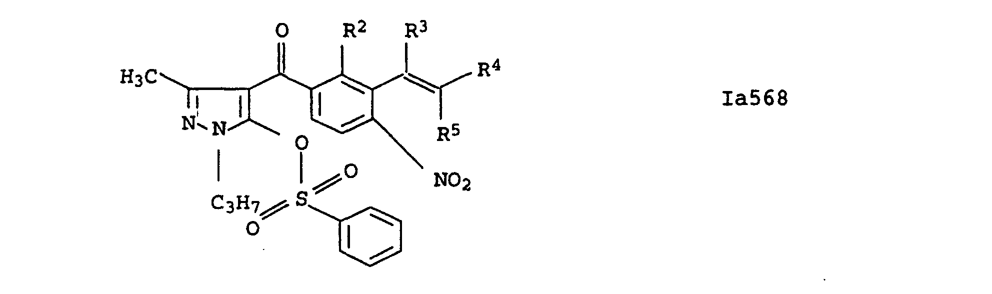
- Ebenso außergewöhnlich bevorzugt sind die Verbindungen Ia569, insbesondere die Verbindungen Ia569.001-Ia569.180, die sich von den entsprechenden Verbindungen Ia1.001-Ia1.180 dadurch unterscheiden, daß R¹ für Nitro, R¹⁴ für n-Butyl, R¹⁵ für Phenylsulfonyl und R¹⁶ für Methyl stehen: 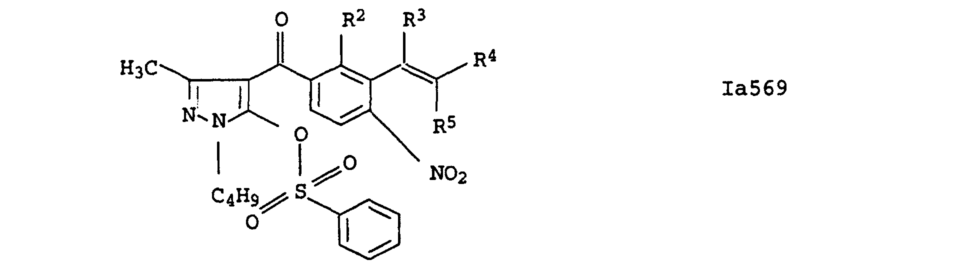
- Ebenso außergewöhnlich bevorzugt sind die Verbindungen Ia570, insbesondere die Verbindungen Ia570.001-Ia570.180, die sich von den entsprechenden Verbindungen Ia1.001-Ia1.180 dadurch unterscheiden, daß R¹ für Nitro, R¹⁴ für iso-Butyl, R¹⁵ für Phenylsulfonyl und R¹⁶ für Methyl stehen: 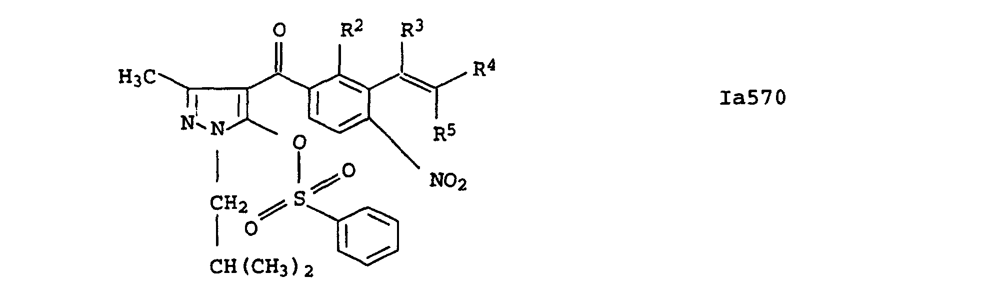
- Ebenso außergewöhnlich bevorzugt sind die Verbindungen Ia571, insbesondere die Verbindungen Ia571.001-Ia571.180, die sich von den entsprechenden Verbindungen Ia1.001-Ia1.180 dadurch unterscheiden, daß R¹ für Nitro, R¹⁵ für 4-Methylphenylsulfonyl und R¹⁶ für Methyl stehen: 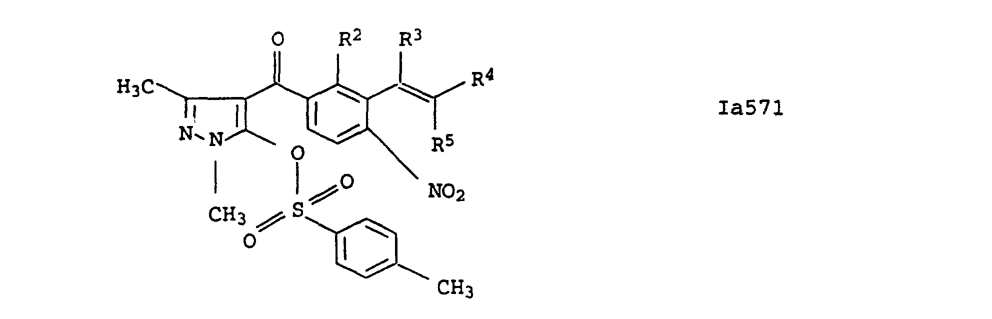
- Ebenso außergewöhnlich bevorzugt sind die Verbindungen Ia572, insbesondere die Verbindungen Ia572.001-Ia572.180, die sich von den entsprechenden Verbindungen Ia1.001-Ia1.180 dadurch unterscheiden, daß R¹ für Nitro, R¹⁴ für Ethyl, R¹⁵ für 4-Methylphenylsulfonyl und R¹⁶ für Methyl stehen: 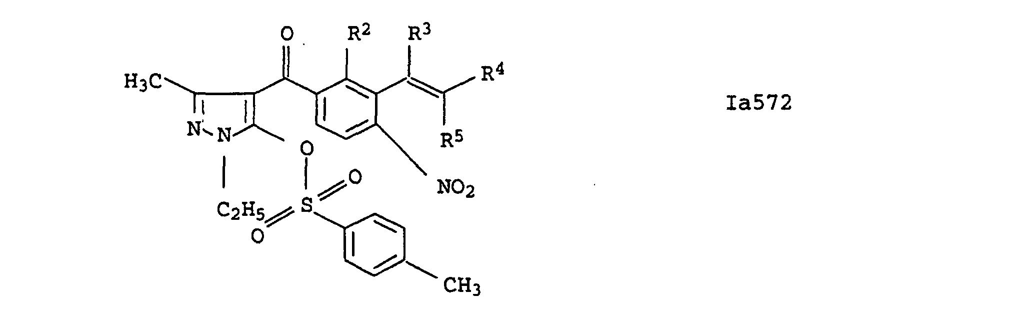
- Ebenso außergewöhnlich bevorzugt sind die Verbindungen Ia573, insbesondere die Verbindungen Ia573.001-Ia573.180, die sich von den entsprechenden Verbindungen Ia1.001-Ia1.180 dadurch unterscheiden, daß R¹ für Nitro, R¹⁴ für n-Propyl, R¹⁵ für 4-Methylphenylsulfonyl und R¹⁶ für Methyl stehen: 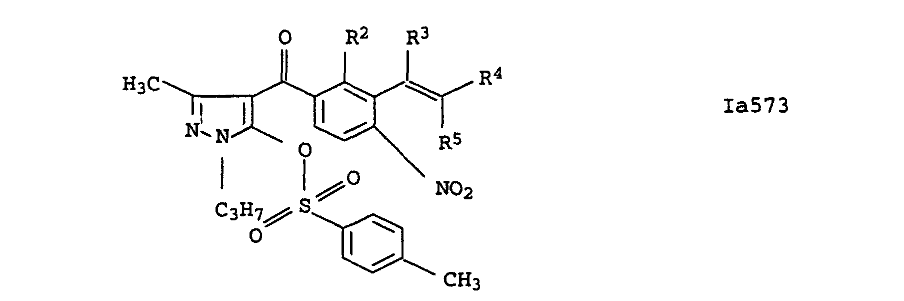
- Ebenso außergewöhnlich bevorzugt sind die Verbindungen Ia574, insbesondere die Verbindungen Ia574.001-Ia574.180, die sich von den entsprechenden Verbindungen Ia1.001-Ia1.180 dadurch unterscheiden, daß R¹ für Nitro, R¹⁴ für n-Butyl, R¹⁵ für 4-Methylphenylsulfonyl und R¹⁶ für Methyl stehen: 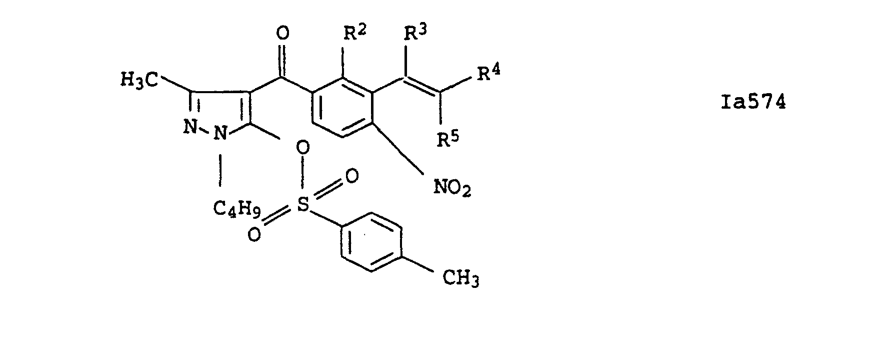
- Ebenso außergewöhnlich bevorzugt sind die Verbindungen Ia575, insbesondere die Verbindungen Ia575.001-Ia575.180, die sich von den entsprechenden Verbindungen Ia1.001-Ia1.180 dadurch unterscheiden, daß R¹ für Nitro, R¹⁴ für iso-Butyl, R¹⁵ für 4-Methylphenylsulfonyl und R¹⁶ für Methyl stehen: 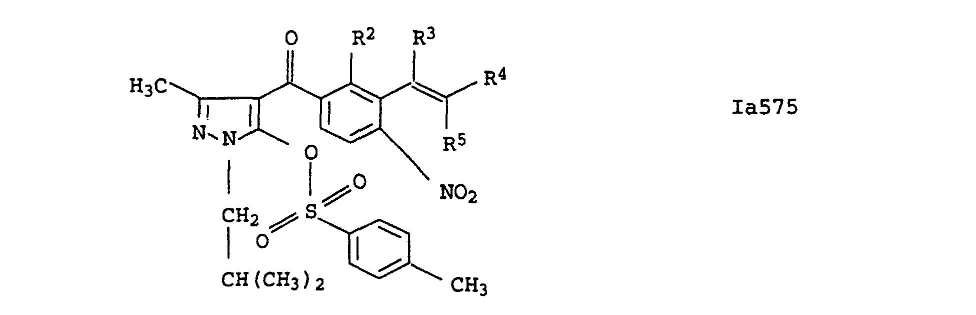
- Ebenso außergewöhnlich bevorzugt sind die Verbindungen Ia576, insbesondere die Verbindungen Ia576.001-Ia576.180, die sich von den entsprechenden Verbindungen Ia1.001-Ia1.180 dadurch unterscheiden, daß R¹ für Methylsulfonyl und R¹⁶ für Methyl stehen: 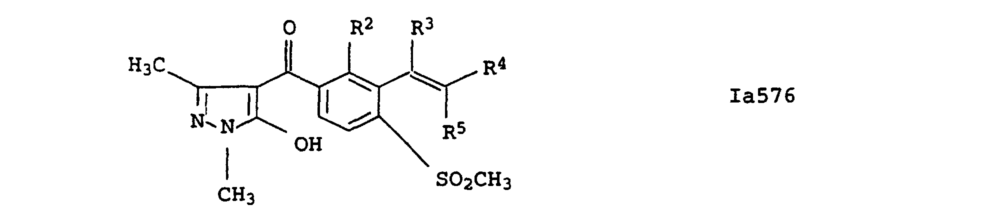
- Ebenso außergewöhnlich bevorzugt sind die Verbindungen Ia577, insbesondere die Verbindungen Ia577.001-Ia577.180, die sich von den entsprechenden Verbindungen Ia1.001-Ia1.180 dadurch unterscheiden, daß R¹ für Methylsulfonyl, R¹⁴ für Ethyl und R¹⁵ für Methyl stehen: 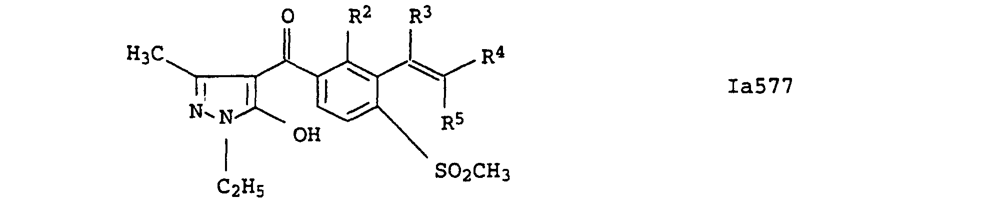
- Ebenso außergewöhnlich bevorzugt sind die Verbindungen Ia578, insbesondere die Verbindungen Ia578.001-Ia578.180, die sich von den entsprechenden Verbindungen Ia1.001-Ia1.180 dadurch unterscheiden, daß R¹ für Methylsulfonyl, R¹⁴ für n-Propyl und R¹⁶ für Methyl stehen: 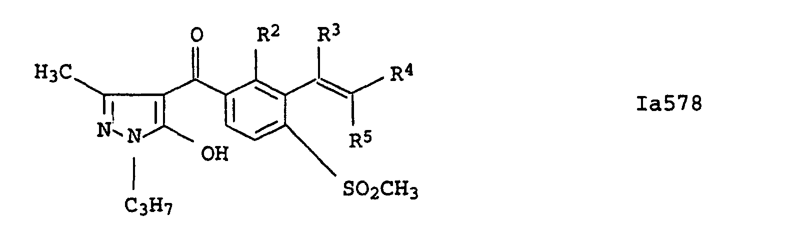
- Ebenso außergewöhnlich bevorzugt sind die Verbindungen Ia579, insbesondere die Verbindungen Ia579.001-Ia579.180, die sich von den entsprechenden Verbindungen Ia1.001-Ia1.180 dadurch unterscheiden, daß R¹ für Methylsulfonyl, R¹⁴ für n-Butyl und R¹⁶ für Methyl stehen: 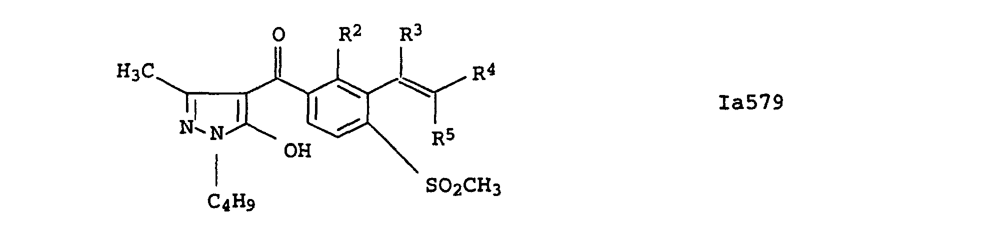
- Ebenso außergewöhnlich bevorzugt sind die Verbindungen Ia580, insbesondere die Verbindungen Ia580.001-Ia580.180, die sich von den entsprechenden Verbindungen Ia1.001-Ia1.180 dadurch unterscheiden, daß R¹ für Methylsulfonyl, R¹⁴ für iso-Butyl und R¹⁶ für Methyl stehen: 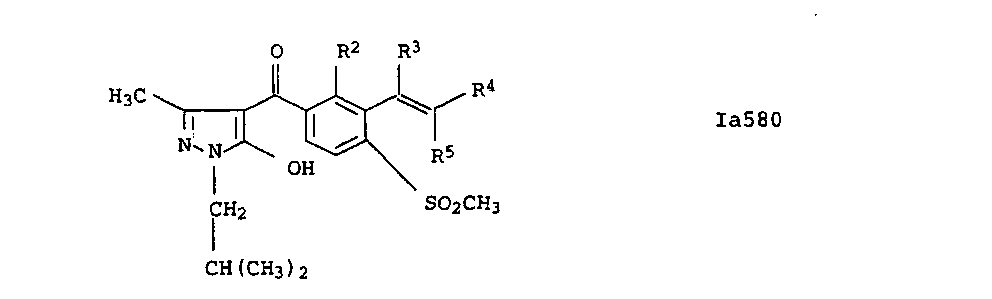
- Ebenso außergewöhnlich bevorzugt sind die Verbindungen Ia581, insbesondere die Verbindungen Ia581.001-Ia581.180, die sich von den entsprechenden Verbindungen Ia1.001-Ia1.180 dadurch unterscheiden, daß R¹ für Methylsulfonyl und R¹⁵ und R¹⁶ für Methyl stehen: 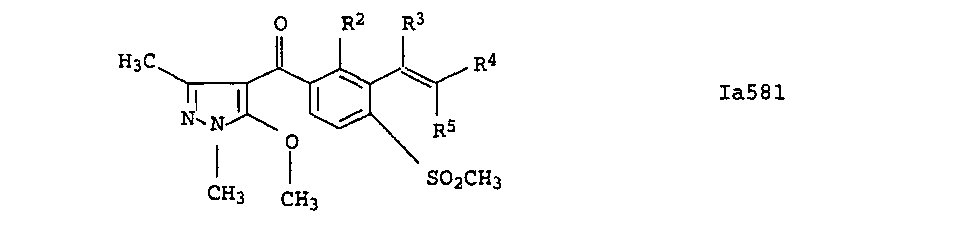
- Ebenso außergewöhnlich bevorzugt sind die Verbindungen Ia582, insbesondere die Verbindungen Ia582.001-Ia582.180, die sich von den entsprechenden Verbindungen Ia1.001-Ia1.180 dadurch unterscheiden, daß R¹ für Methylsulfonyl, R¹⁴ für Ethyl und R¹⁵ und R¹⁶ für Methyl stehen: 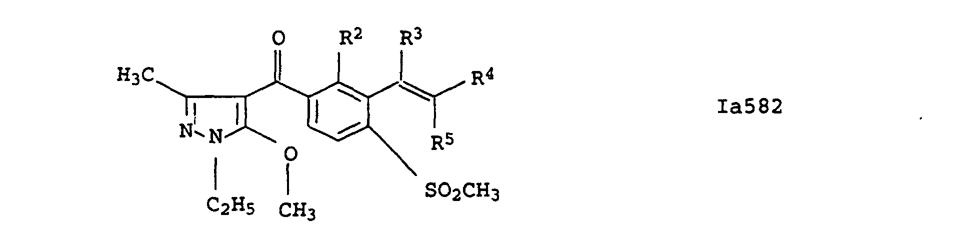
- Ebenso außergewöhnlich bevorzugt sind die Verbindungen Ia583, insbesondere die Verbindungen Ia583.001-Ia583.180, die sich von den entsprechenden Verbindungen Ia1.001-Ia1.180 dadurch unterscheiden, daß R¹ für Methylsulfonyl, R¹⁴ für n-Propyl und R¹⁵ und R¹⁶ für Methyl stehen: 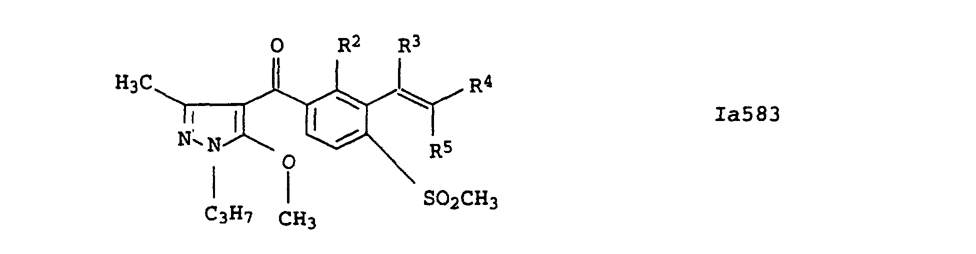
- Ebenso außergewöhnlich bevorzugt sind die Verbindungen Ia584, insbesondere die Verbindungen Ia584.001-Ia584.180, die sich von den entsprechenden Verbindungen Ia1.001-Ia1.180 dadurch unterscheiden, daß R¹ für Methylsulfonyl, R¹⁴ für n-Butyl und R¹⁵ und R¹⁶ für Methyl stehen: 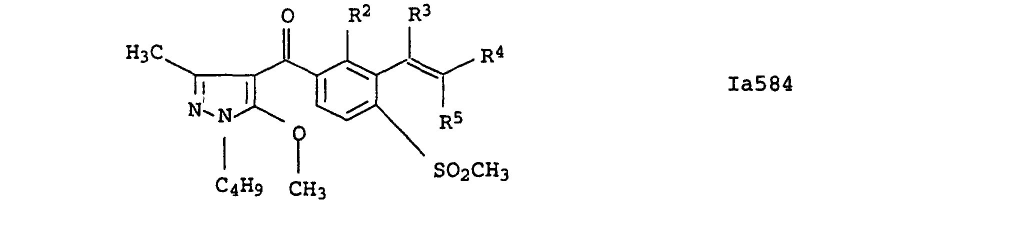
- Ebenso außergewöhnlich bevorzugt sind die Verbindungen Ia585, insbesondere die Verbindungen Ia585.001-Ia585.180, die sich von den entsprechenden Verbindungen Ta1.001-Ia1.180 dadurch unterscheiden, daß R¹ für Methylsulfonyl, R¹⁴ für iso-Butyl und R¹⁵ und R¹⁶ für Methyl stehen: 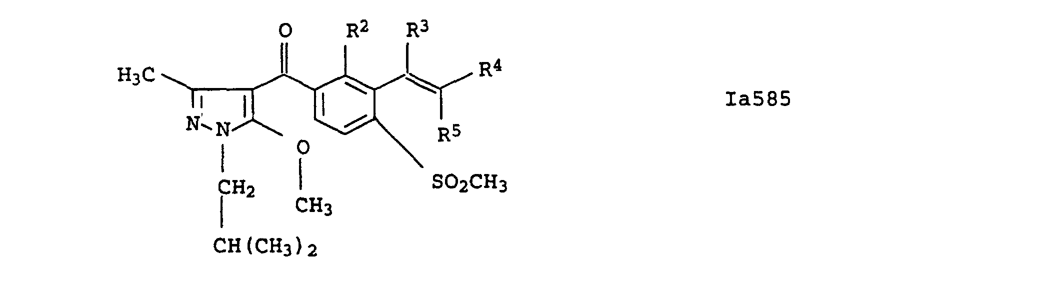
- Ebenso außergewöhnlich bevorzugt sind die Verbindungen Ia586, insbesondere die Verbindungen Ia586.001-Ia586.180, die sich von den entsprechenden Verbindungen Ia1.001-Ia1.180 dadurch unterscheiden, daß R¹ für Methylsulfonyl, R¹⁵ für Ethyl und R¹⁶ für Methyl stehen: 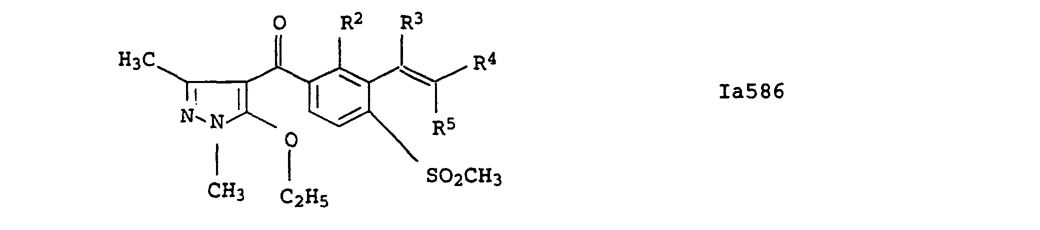
- Ebenso außergewöhnlich bevorzugt sind die Verbindungen Ia587, insbesondere die Verbindungen Ia587.001-Ia587.180, die sich von den entsprechenden Verbindungen Ia1.001-Ia1.180 dadurch unterscheiden, daß R¹ für Methylsulfonyl, R¹⁴ und R¹⁵ für Ethyl und R¹⁶ für Methyl stehen: 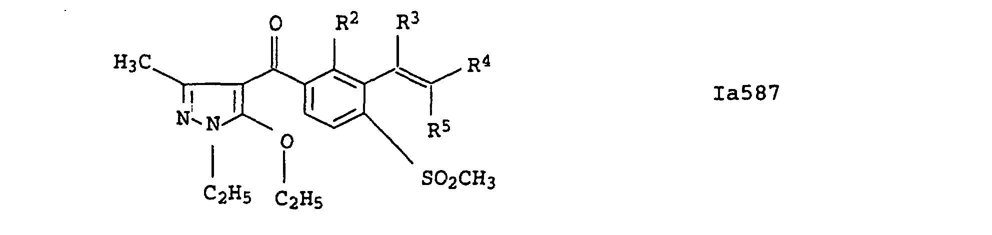
- Ebenso außergewöhnlich bevorzugt sind die Verbindungen Ia588, insbesondere die Verbindungen Ia588.001-Ia588.180, die sich von den entsprechenden Verbindungen Ia1.001-Ia1.180 dadurch unterscheiden, daß R¹ für Methylsulfonyl, R¹⁴ für n-Propyl, R¹⁵ für Ethyl und R¹⁶ für Methyl stehen: 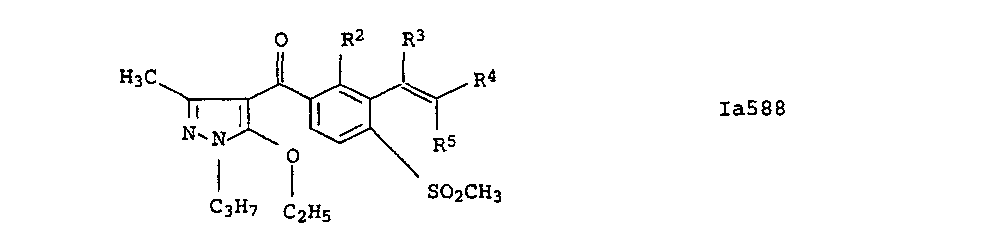
- Ebenso außergewöhnlich bevorzugt sind die Verbindungen Ia589, insbesondere die Verbindungen Ia589.001-Ia589.180, die sich von den entsprechenden Verbindungen Ia1.001-Ia1.180 dadurch unterscheiden, daß R¹ für Methylsulfonyl, R¹⁴ für n-Butyl, R¹⁵ für Ethyl und R¹⁶ für Methyl stehen: 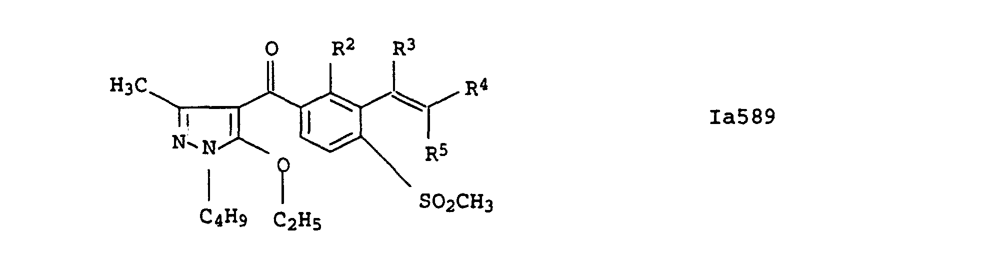
- Ebenso außergewöhnlich bevorzugt sind die Verbindungen Ia590, insbesondere die Verbindungen Ia590.001-Ia590.180, die sich von den entsprechenden Verbindungen Ia1.001-Ia1.180 dadurch unterscheiden, daß R¹ für Methylsulfonyl, R¹⁴ für iso-Butyl, R¹⁵ für Ethyl und R¹⁶ für Methyl stehen: 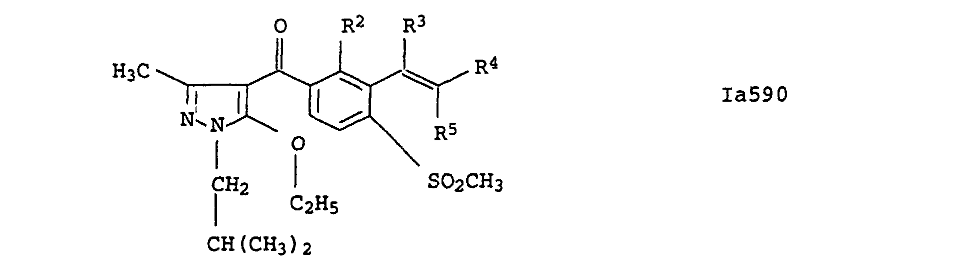
- Ebenso außergewöhnlich bevorzugt sind die Verbindungen Ia591, insbesondere die Verbindungen Ia591.001-Ia591.180, die sich von den entsprechenden Verbindungen Ia1.001-Ia1.180 dadurch unterscheiden, daß R¹ für Methylsulfonyl, R¹⁵ für n-Propyl und R¹⁶ für Methyl stehen: 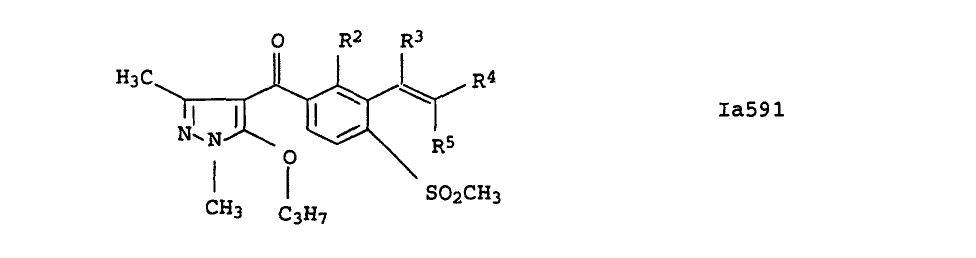
- Ebenso außergewöhnlich bevorzugt sind die Verbindungen Ia592, insbesondere die Verbindungen Ia592.001-Ia592.180, die sich von den entsprechenden Verbindungen Ia1.001-Ia1.180 dadurch unterscheiden, daß R¹ für Methylsulfonyl, R¹⁴ für Ethyl, R¹⁵ für n-Propyl und R¹⁶ für Methyl stehen: 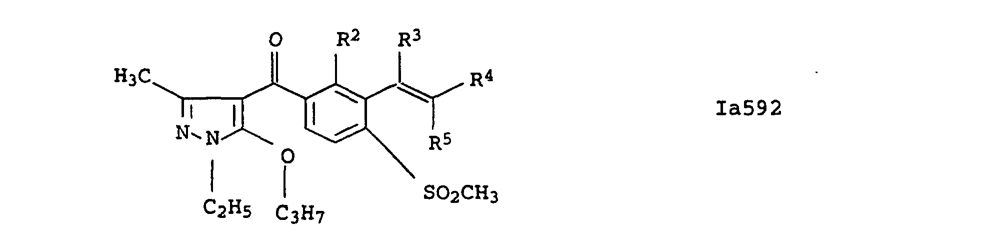
- Ebenso außergewöhnlich bevorzugt sind die Verbindungen Ia593, insbesondere die Verbindungen Ia593.001-Ia593.180, die sich von den entsprechenden Verbindungen Ia1.001-Ia1.180 dadurch unterscheiden, daß R¹ für Methylsulfonyl, R¹⁴ und R¹⁵ für n-Propyl und R¹⁶ für Methyl stehen: 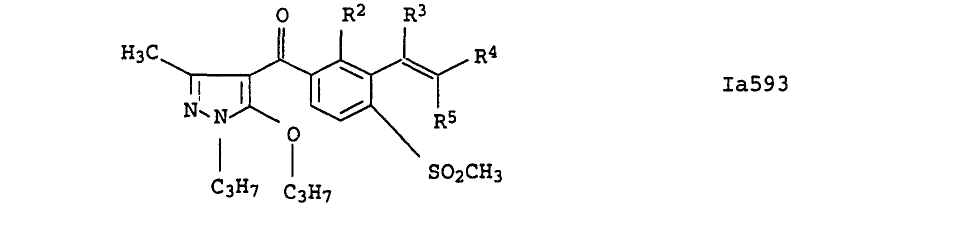
- Ebenso außergewöhnlich bevorzugt sind die Verbindungen Ia594, insbesondere die Verbindungen Ia594.001-Ia594.180, die sich von den entsprechenden Verbindungen Ia1.001-Ia1.180 dadurch unterscheiden, daß R¹ für Methylsulfonyl, R¹⁴ für n-Butyl, R¹⁵ für n-Propyl und R¹⁶ für Methyl stehen: 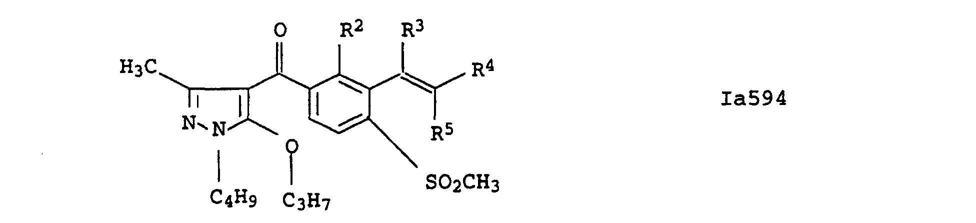
- Ebenso außergewöhnlich bevorzugt sind die Verbindungen Ia595, insbesondere die Verbindungen Ia595.001-Ia595.180, die sich von den entsprechenden Verbindungen Ia1.001-Ia1.180 dadurch unterscheiden, daß R¹ für Methylsulfonyl, R¹⁴ für iso-Butyl, R¹⁵ für n-Propyl und R¹⁶ für Methyl stehen: 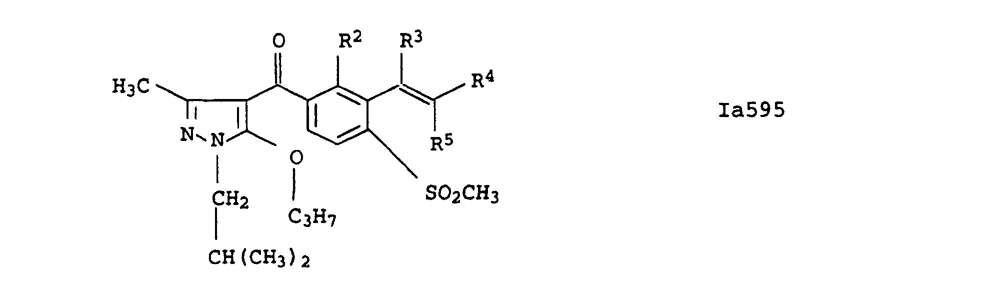
- Ebenso außergewöhnlich bevorzugt sind die Verbindungen Ia596, insbesondere die Verbindungen Ia596.001-Ia596.180, die sich von den entsprechenden Verbindungen Ia1.001-Ia1.180 dadurch unterscheiden, daß R¹ für Methylsulfonyl, R¹⁵ für iso-Propyl und R¹⁶ für Methyl stehen: 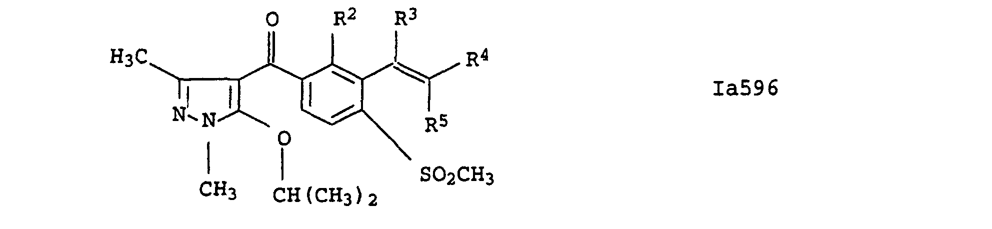
- Ebenso außergewöhnlich bevorzugt sind die Verbindungen Ia597, insbesondere die Verbindungen Ia597.001-Ia597.180, die sich von den entsprechenden Verbindungen Ia1.001-Ia1.180 dadurch unterscheiden, daß R¹ für Methylsulfonyl, R¹⁴ für Ethyl, R¹⁵ für iso-Propyl und R¹⁶ für Methyl stehen: 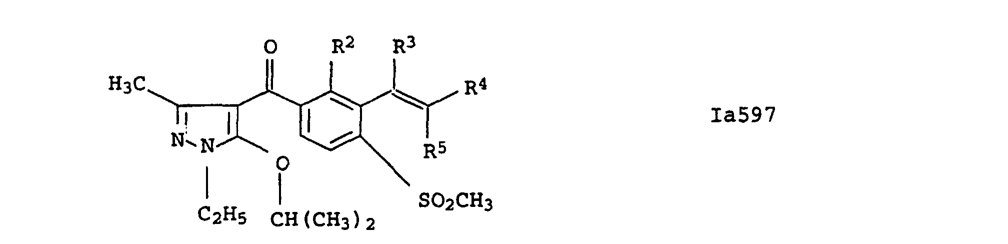
- Ebenso außergewöhnlich bevorzugt sind die Verbindungen Ia598, insbesondere die Verbindungen Ia598.001-Ia598.180, die sich von den entsprechenden Verbindungen Ia1.001-Ia1.180 dadurch unterscheiden, daß R¹ für Methylsulfonyl, R¹⁴ für n-Propyl, R¹⁵ für iso-Propyl und R¹⁶ für Methyl stehen: 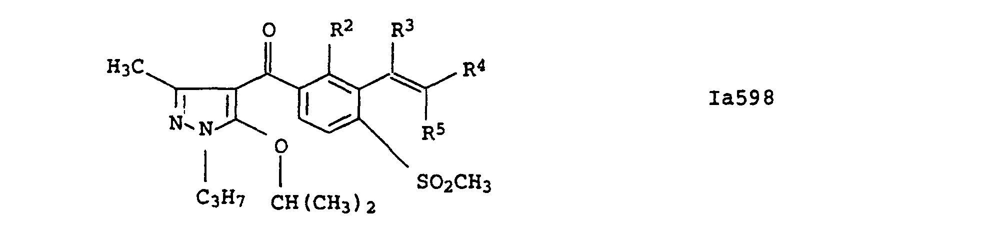
- Ebenso außergewöhnlich bevorzugt sind die Verbindungen Ia599, insbesondere die Verbindungen Ia599.001-Ia599.180, die sich von den entsprechenden Verbindungen Ia1.001-Ia1.180 dadurch unterscheiden, daß R¹ für Methylsulfonyl, R¹⁴ für n-Butyl, R¹⁵ für iso-Propyl und R¹⁶ für Methyl stehen: 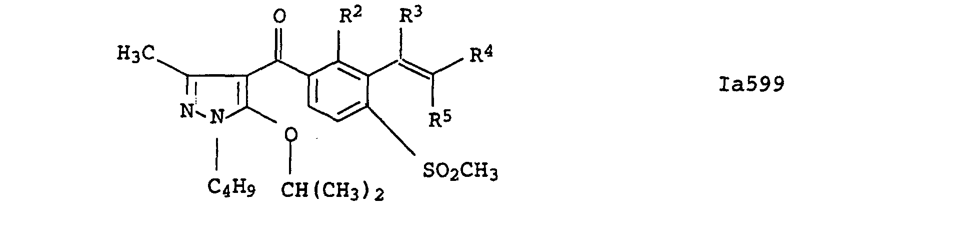
- Ebenso außergewöhnlich bevorzugt sind die Verbindungen Ia600, insbesondere die Verbindungen Ia600.001-Ia600.180, die sich von den entsprechenden Verbindungen Ia1.001-Ia1.180 dadurch unterscheiden, daß R¹ für Methylsulfonyl, R¹⁴ für iso-Butyl, R¹⁵ für iso-Propyl und R¹⁶ für Methyl stehen: 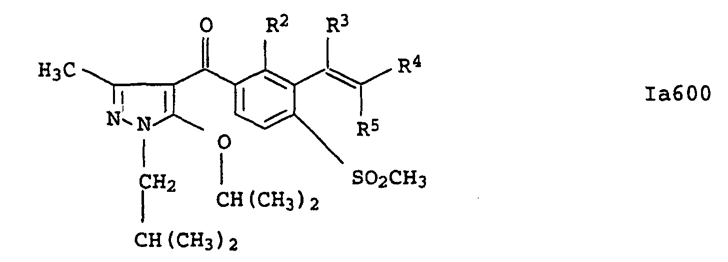
- Ebenso außergewöhnlich bevorzugt sind die Verbindungen Ia601, insbesondere die Verbindungen Ia601.001-Ia601.180, die sich von den entsprechenden Verbindungen Ia1.001-Ia1.180 dadurch unterscheiden, daß R¹ für Methylsulfonyl, R¹⁵ für n-Butyl und R¹⁶ für Methyl stehen: 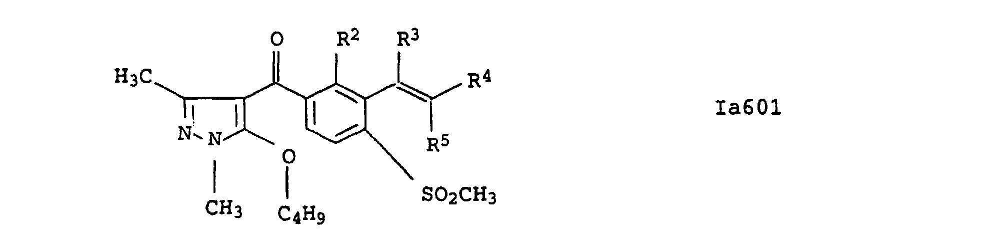
- Ebenso außergewöhnlich bevorzugt sind die Verbindungen Ia602, insbesondere die Verbindungen Ia602.001-Ia602.180, die sich von den entsprechenden Verbindungen Ia1.001-Ia1.180 dadurch unterscheiden, daß R¹ für Methylsulfonyl, R¹⁴ für Ethyl, R¹⁵ für n-Butyl und R¹⁶ für Methyl stehen: 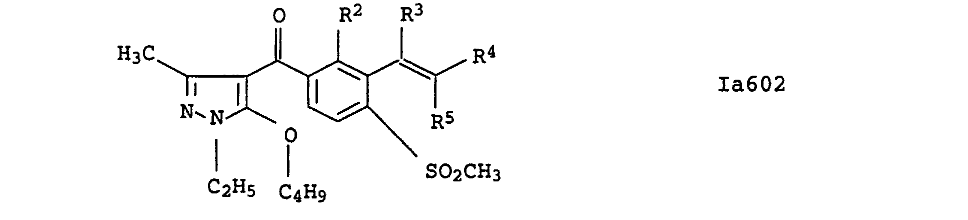
- Ebenso außergewöhnlich bevorzugt sind die Verbindungen Ia603, insbesondere die Verbindungen Ia603.001-Ia603.180, die sich von den entsprechenden Verbindungen Ia1.001-Ia1.180 dadurch unterscheiden, daß R¹ für Methylsulfonyl, R¹⁴ für n-Propyl, R¹⁵ für n-Butyl und R¹⁶ für Methyl stehen: 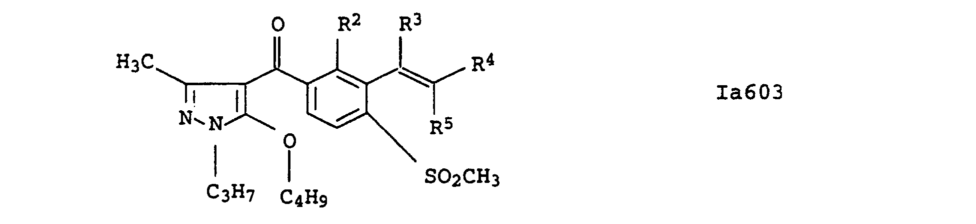
- Ebenso außergewöhnlich bevorzugt sind die Verbindungen Ia604, insbesondere die Verbindungen Ia604.001-Ia604.180, die sich von den entsprechenden Verbindungen Ia1.001-Ia1.180 dadurch unterscheiden, daß R¹ für Methylsulfonyl, R¹⁴ und R¹⁵ für n-Butyl und R¹⁶ für Methyl stehen: 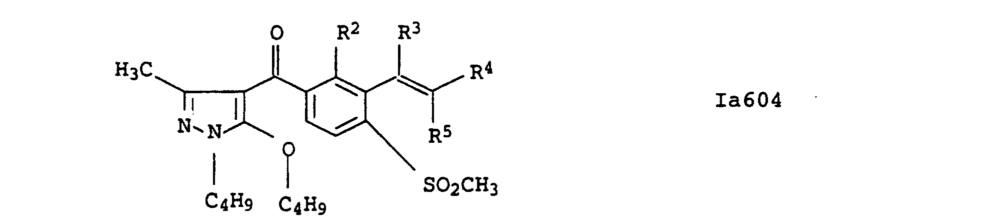
- Ebenso außergewöhnlich bevorzugt sind die Verbindungen Ia605, insbesondere die Verbindungen Ia605.001-Ia605.180, die sich von den entsprechenden Verbindungen Ia1.001-Ia1.180 dadurch unterscheiden, daß R¹ für Methylsulfonyl, R¹⁴ für iso-Butyl, R¹⁵ für n-Butyl und R¹⁶ für Methyl stehen: 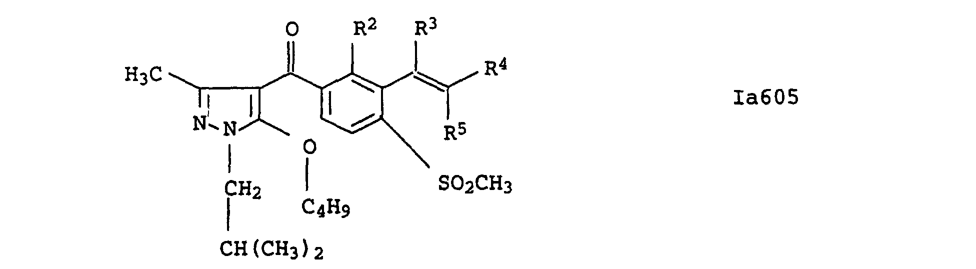
- Ebenso außergewöhnlich bevorzugt sind die Verbindungen Ia606, insbesondere die Verbindungen Ia606.001-Ia606.180, die sich von den entsprechenden Verbindungen Ia1.001-Ia1.180 dadurch unterscheiden, daß R¹ für Methylsulfonyl, R¹⁵ für sec-Butyl und R¹⁶ für Methyl stehen: 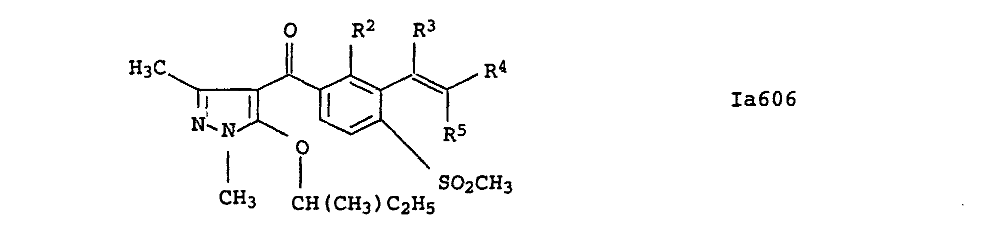
- Ebenso außergewöhnlich bevorzugt sind die Verbindungen Ia607, insbesondere die Verbindungen Ia607.001-Ia607.180, die sich von den entsprechenden Verbindungen Ia1.001-Ia1.180 dadurch unterscheiden, daß R¹ für Methylsulfonyl, R¹⁴ für Ethyl, R¹⁵ für sec-Butyl und R¹⁶ für Methyl stehen: 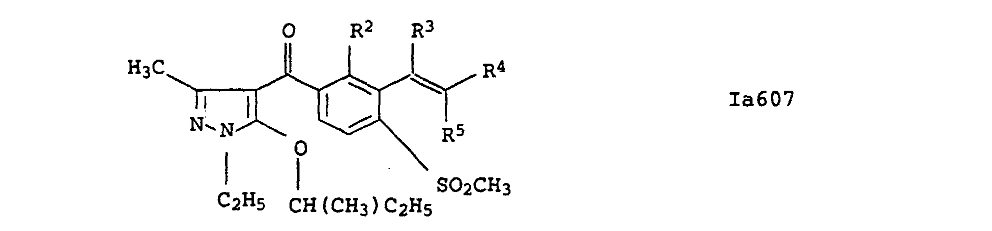
- Ebenso außergewöhnlich bevorzugt sind die Verbindungen Ia608, insbesondere die Verbindungen Ia608.001-Ia608.180, die sich von den entsprechenden Verbindungen Ia1.001-Ia1.180 dadurch unterscheiden, daß R¹ für Methylsulfonyl, R¹⁴ für n-Propyl, R¹⁵ für sec-Butyl und R¹⁶ für Methyl stehen: 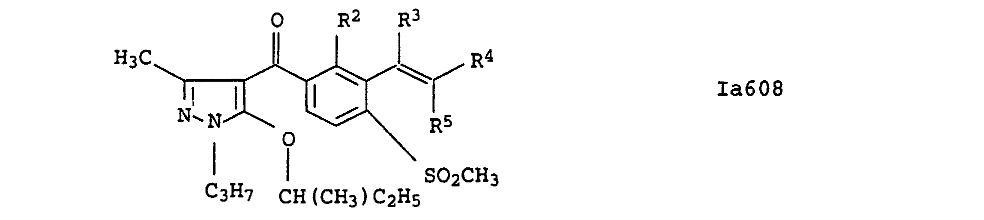
- Ebenso außergewöhnlich bevorzugt sind die Verbindungen Ia609, insbesondere die Verbindungen Ia609.001-Ia609.180, die sich von den entsprechenden Verbindungen Ia1.001-Ia1.180 dadurch unterscheiden, daß R¹ für Methylsulfonyl, R¹⁴ für n-Butyl, R¹⁵ für sec-Butyl und R¹⁶ für Methyl stehen: 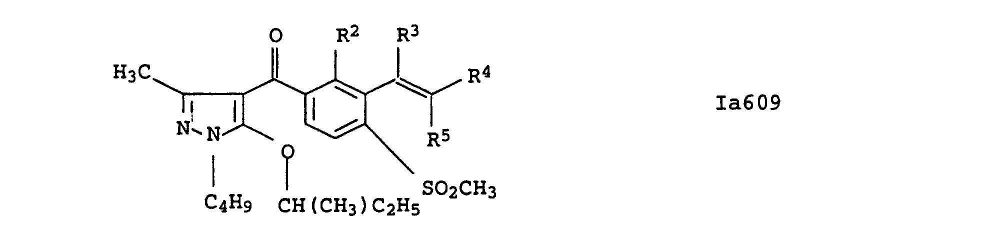
- Ebenso außergewöhnlich bevorzugt sind die Verbindungen Ia610, insbesondere die Verbindungen Ia610.001-Ia610.180, die sich von den entsprechenden Verbindungen Ia1.001-Ia1.180 dadurch unterscheiden, daß R¹ für Methylsulfonyl, R¹⁴ für iso-Butyl, R¹⁵ für sec-Butyl und R¹⁶ für Methyl stehen: 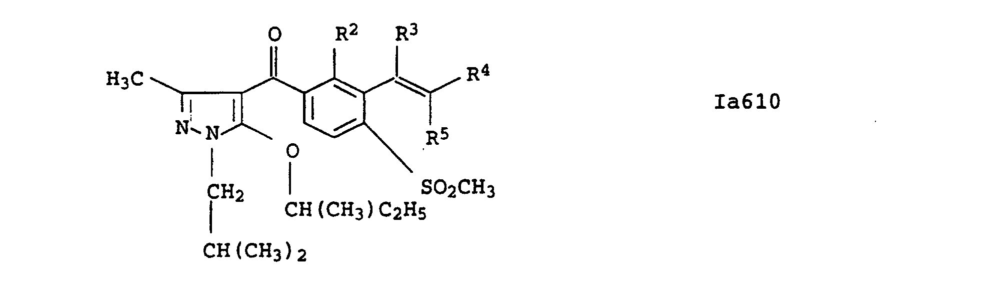
- Ebenso außergewöhnlich bevorzugt sind die Verbindungen Ia611, insbesondere die Verbindungen Ia611.001-Ia611.180, die sich von den entsprechenden Verbindungen Ia1.001-Ia1.180 dadurch unterscheiden, daß R¹ für Methylsulfonyl, R¹⁵ für iso-Butyl und R¹⁶ für Methyl stehen: 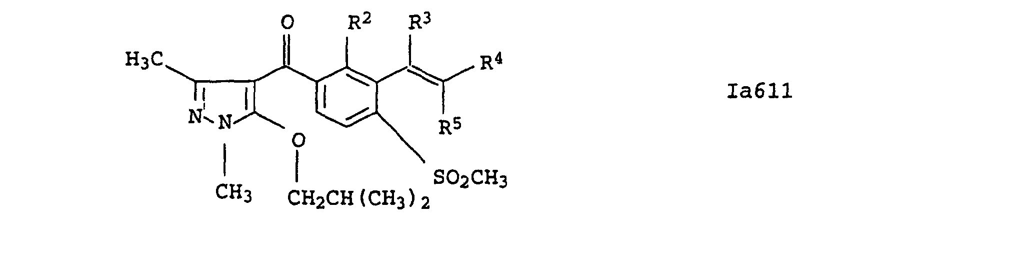
- Ebenso außergewöhnlich bevorzugt sind die Verbindungen Ia612, insbesondere die Verbindungen Ia612.001-Ia612.180, die sich von den entsprechenden Verbindungen Ia1.001-Ia1.180 dadurch unterscheiden, daß R¹ für Methylsulfonyl, R¹⁴ für Ethyl, R¹⁵ für iso-Butyl und R¹⁶ für Methyl stehen: 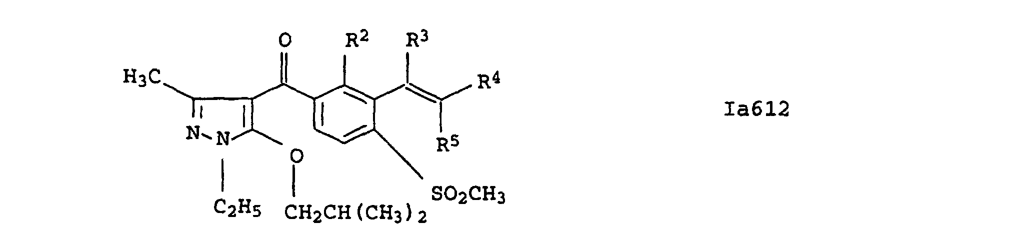
- Ebenso außergewöhnlich bevorzugt sind die Verbindungen Ia613, insbesondere die Verbindungen Ia613.001-Ia613.180, die sich von den entsprechenden Verbindungen Ia1.001-Ia1.280 dadurch unterscheiden, daß R¹ für Methylsulfonyl, R¹⁴ für n-Propyl, R¹⁵ für iso-Butyl und R¹⁶ für Methyl stehen: 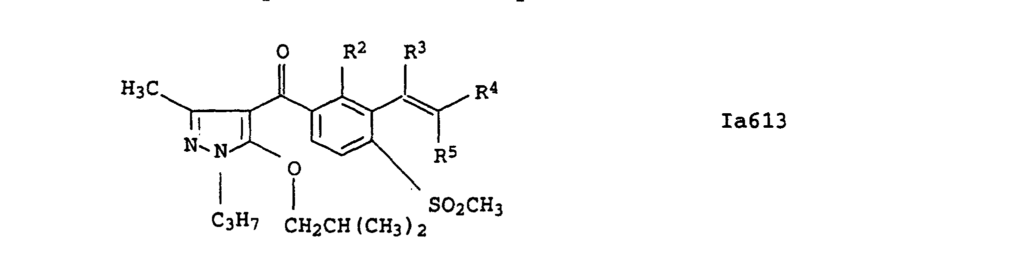
- Ebenso außergewöhnlich bevorzugt sind die Verbindungen Ia614, insbesondere die Verbindungen Ia614.001-Ia614.180, die sich von den entsprechenden Verbindungen Ia1.001-Ia1.180 dadurch unterscheiden, daß R¹ für Methylsulfonyl, R¹⁴ für n-Butyl, R¹⁵ für iso-Butyl und R¹⁶ für Methyl stehen: 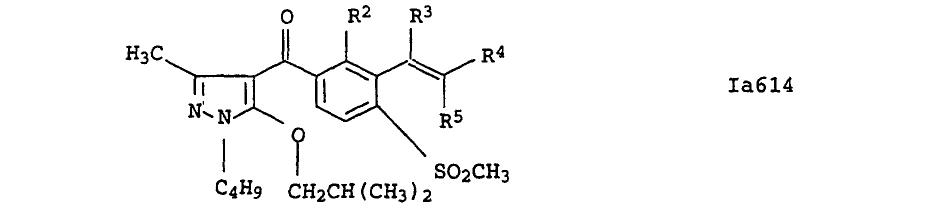
- Ebenso außergewöhnlich bevorzugt sind die Verbindungen Ia615, insbesondere die Verbindungen Ia615.001-Ia615.180, die sich von den entsprechenden Verbindungen Ia1.001-Ia1.180 dadurch unterscheiden, daß R¹ für Methylsulfonyl, R¹⁴ und R¹⁵ für iso-Butyl und R¹⁶ für Methyl stehen: 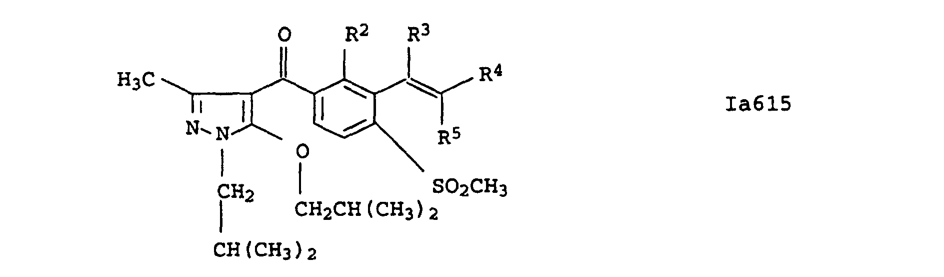
- Ebenso außergewöhnlich bevorzugt sind die Verbindungen Ia616, insbesondere die Verbindungen Ia616.001-Ia616.180, die sich von den entsprechenden Verbindungen Ia1.001-Ia1.180 dadurch unterscheiden, daß R¹ für Methylsulfonyl, R¹⁵ für Methylcarbonyl und R¹⁶ für Methyl stehen: 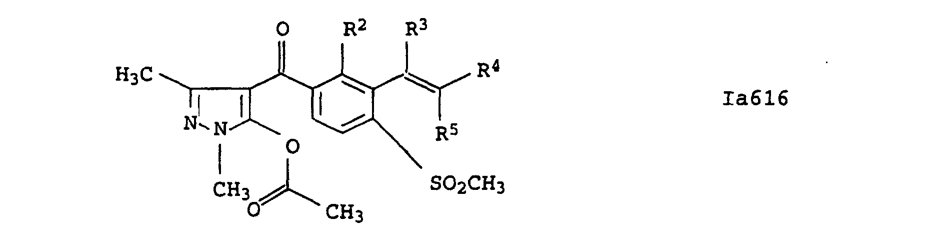
- Ebenso außergewöhnlich bevorzugt sind die Verbindungen Ia617, insbesondere die Verbindungen Ia617.001-Ia617.180, die sich von den entsprechenden Verbindungen Ia1.001-Ia1.180 dadurch unterscheiden, daß R¹ für Methylsulfonyl, R¹⁴ für Ethyl, R¹⁵ für Methylcarbonyl und R¹⁶ für Methyl stehen: 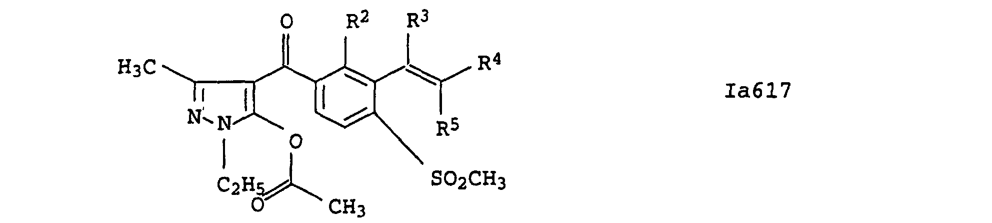
- Ebenso außergewöhnlich bevorzugt sind die Verbindungen Ia618, insbesondere die Verbindungen Ia618.001-Ia618.180, die sich von den entsprechenden Verbindungen Ia1.001-Ia1.180 dadurch unterscheiden, daß R¹ für Methylsulfonyl, R¹⁴ für n-Propyl, R¹⁵ für Methylcarbonyl und R¹⁶ für Methyl stehen: 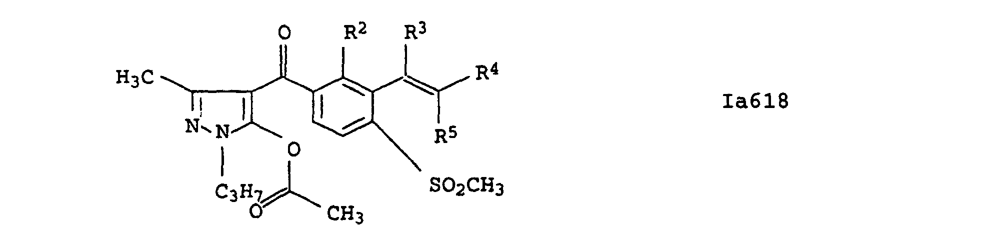
- Ebenso außergewöhnlich bevorzugt sind die Verbindungen Ia619, insbesondere die Verbindungen Ia619.001-Ia619.180, die sich von den entsprechenden Verbindungen Ia1.001-Ia1.180 dadurch unterscheiden, daß R¹ für Methylsulfonyl, R¹⁴ für n-Butyl, R¹⁵ für Methylcarbonyl und R¹⁶ für Methyl stehen: 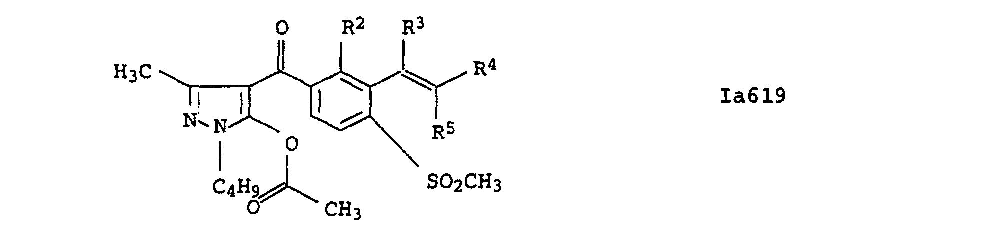
- Ebenso außergewöhnlich bevorzugt sind die Verbindungen Ia620, insbesondere die Verbindungen Ia620.001-Ia620.180, die sich von den entsprechenden Verbindungen Ia1.001-Ia1.180 dadurch unterscheiden, daß R¹ für Methylsulfonyl, R¹⁴ für iso-Butyl, R¹⁵ für Methylcarbonyl und R¹⁶ für Methyl stehen: 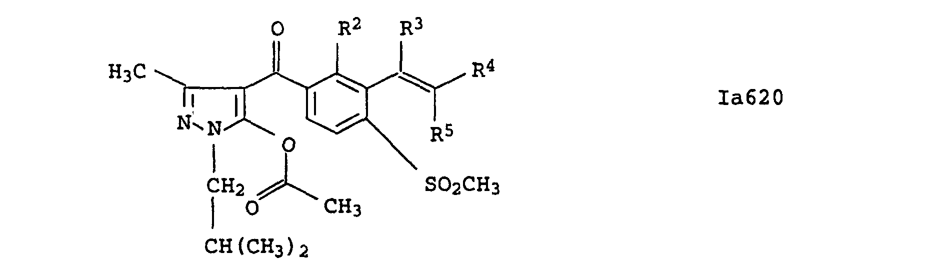
- Ebenso außergewöhnlich bevorzugt sind die Verbindungen Ia621, insbesondere die Verbindungen Ia621.001-Ia621.180, die sich von den entsprechenden Verbindungen Ia1.001-Ia1.180 dadurch unterscheiden, daß R¹ für Methylsulfonyl, R¹⁵ für Ethylcarbonyl und R¹⁶ für Methyl stehen: 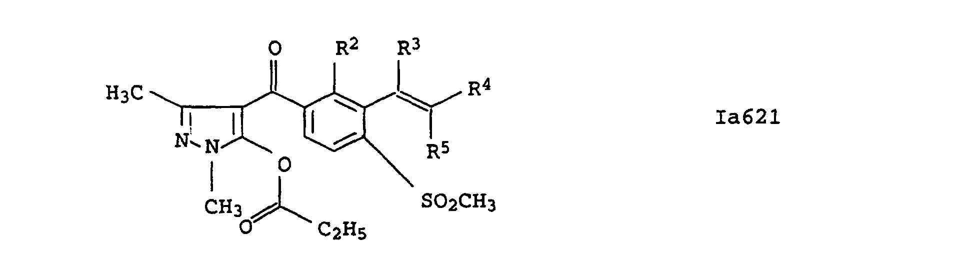
- Ebenso außergewöhnlich bevorzugt sind die Verbindungen Ia622, insbesondere die Verbindungen Ia622.001-Ia622.180, die sich von den entsprechenden Verbindungen Ia1.001-Ia1.180 dadurch unterscheiden, daß R¹ für Methylsulfonyl, R¹⁴ für Ethyl, R¹⁵ für Ethylcarbonyl und R¹⁶ für Methyl stehen: 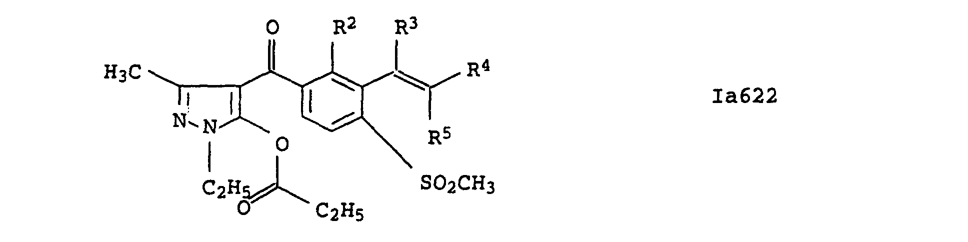
- Ebenso außergewöhnlich bevorzugt sind die Verbindungen Ia623, insbesondere die Verbindungen Ia623.001-Ia623.180, die sich von den entsprechenden Verbindungen Ia1.001-Ia1.180 dadurch unterscheiden, daß R¹ für Methylsulfonyl, R¹⁴ für n-Propyl, R¹⁵ für Ethylcarbonyl und R¹⁶ für Methyl stehen: 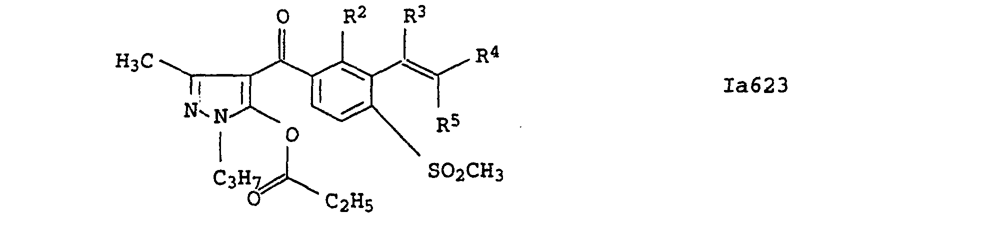
- Ebenso außergewöhnlich bevorzugt sind die Verbindungen Ia624, insbesondere die Verbindungen Ia624.001-Ia624.180, die sich von den entsprechenden Verbindungen Ia1.001-Ia1.180 dadurch unterscheiden, daß R¹ für Methylsulfonyl, R¹⁴ für n-Butyl, R¹⁵ für Ethylcarbonyl und R¹⁶ für Methyl stehen: 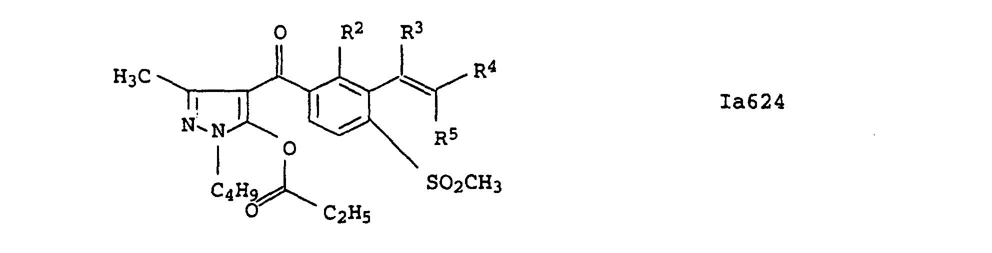
- Ebenso außergewöhnlich bevorzugt sind die Verbindungen Ia625, insbesondere die Verbindungen Ia625.001-Ia625.180, die sich von den entsprechenden Verbindungen Ia1.001-Ia1.180 dadurch unterscheiden, daß R¹ für Methylsulfonyl, R¹⁴ für iso-Butyl, R¹⁵ für Ethylcarbonyl und R¹⁶ für Methyl stehen: 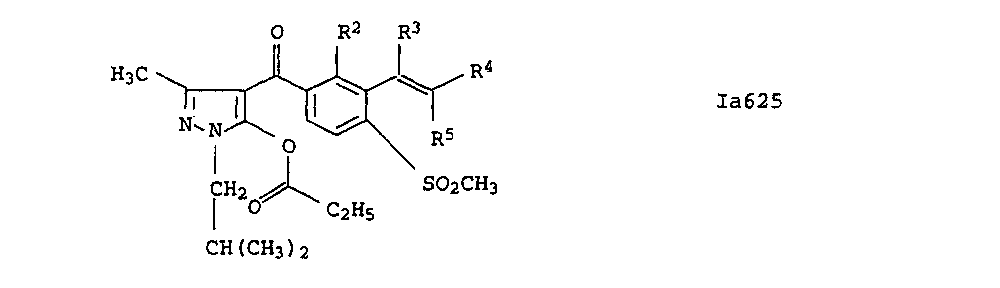
- Ebenso außergewöhnlich bevorzugt sind die Verbindungen Ia626, insbesondere die Verbindungen Ia626.001-Ia626.180, die sich von den entsprechenden Verbindungen Ia1.001-Ia1,180 dadurch unterscheiden, daß R¹ für Methylsulfonyl, R¹⁵ für n-Propylcarbonyl und R¹⁶ für Methyl stehen: 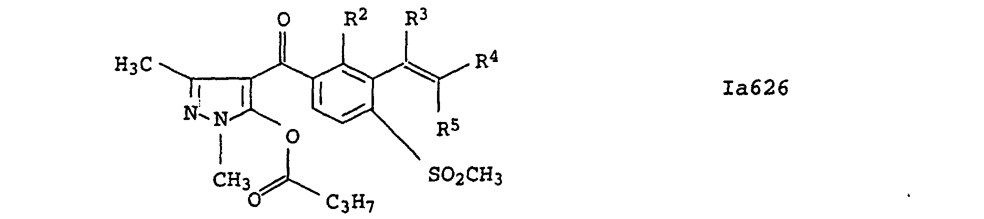
- Ebenso außergewöhnlich bevorzugt sind die Verbindungen Ia627, insbesondere die Verbindungen Ia627.001-Ia627.180, die sich von den entsprechenden Verbindungen Ia1.001-Ia1.180 dadurch unterscheiden, daß R¹ für Methylsulfonyl, R¹⁴ für Ethyl, R¹⁵ für n-Propylcarbonyl und R¹⁶ für Methyl stehen: 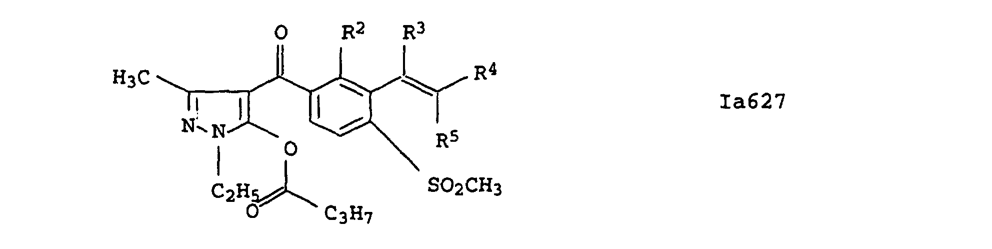
- Ebenso außergewöhnlich bevorzugt sind die Verbindungen Ia628, insbesondere die Verbindungen Ia628.001-Ia628.180, die sich von den entsprechenden Verbindungen Ia1.001-Ia1.180 dadurch unterscheiden, daß R¹ für Methylsulfonyl, R¹⁴ für n-Propyl, R¹⁵ für n-Propylcarbonyl und R¹⁶ für Methyl stehen: 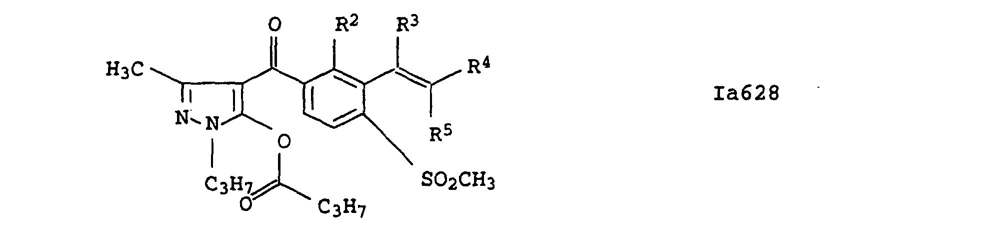
- Ebenso außergewöhnlich bevorzugt sind die Verbindungen Ia629, insbesondere die Verbindungen Ia629.001-Ia629.180, die sich von den entsprechenden Verbindungen Ia1.001-Ia1.180 dadurch unterscheiden, daß R¹ für Methylsulfonyl, R¹⁴ für n-Butyl, R¹⁵ für n-Propylcarbonyl und R¹⁶ für Methyl stehen: 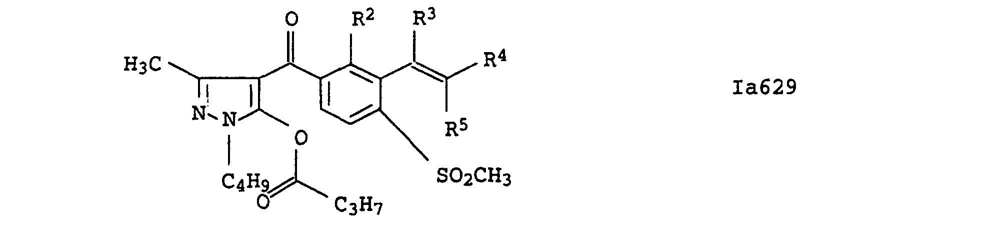
- Ebenso außergewöhnlich bevorzugt sind die Verbindungen Ia630, insbesondere die Verbindungen Ia630.001-Ia630.180, die sich von den entsprechenden Verbindungen Ia1.001-Ia1.180 dadurch unterscheiden, daß R¹ für Methylsulfonyl, R¹⁴ für iso-Butyl, R¹⁵ für n-Propylcarbonyl und R¹⁶ für Methyl stehen: 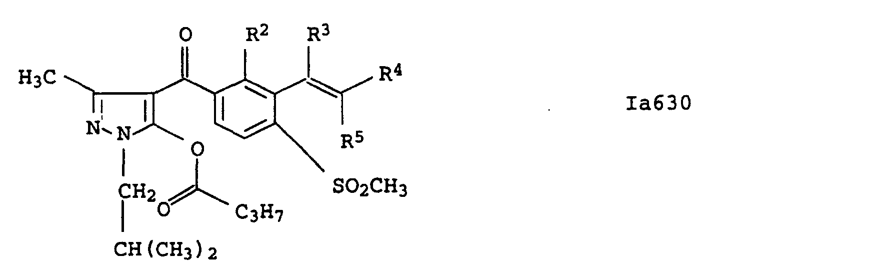
- Ebenso außergewöhnlich bevorzugt sind die Verbindungen Ia631, insbesondere die Verbindungen Ia631.001-Ia631.180, die sich von den entsprechenden Verbindungen Ia1.001-Ia1.180 dadurch unterscheiden, daß R¹ für Methylsulfonyl, R¹⁵ für Trifluormethylcarbonyl und R¹⁶ für Methyl stehen: 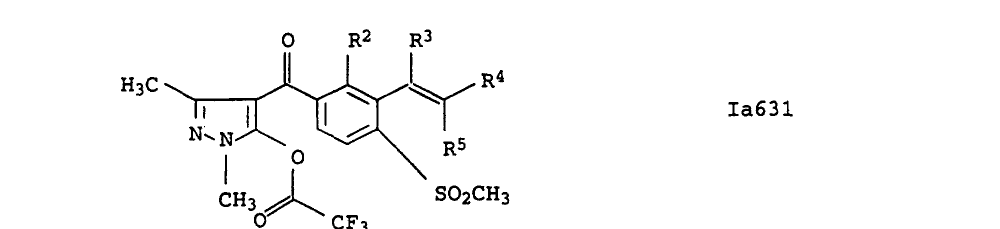
- Ebenso außergewöhnlich bevorzugt sind die Verbindungen Ia632, insbesondere die Verbindungen Ia632.001-Ia632.180, die sich von den entsprechenden Verbindungen Ia1.001-Ia1.180 dadurch unterscheiden, daß R¹ für Methylsulfonyl, R¹⁴ für Ethyl, R¹⁵ für Trifluormethylcarbonyl und R¹⁶ für Methyl stehen: 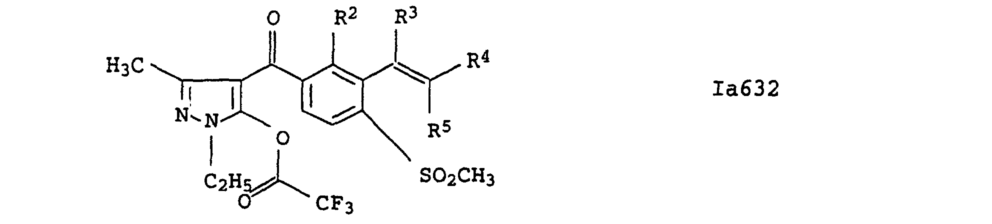
- Ebenso außergewöhnlich bevorzugt sind die Verbindungen Ia633, insbesondere die Verbindungen Ia633.001-Ia633.180, die sich von den entsprechenden Verbindungen Ia1.001-Ia1.180 dadurch unterscheiden, daß R¹ für Methylsulfonyl, R¹⁴ für n-Propyl, R¹⁵ für Trifluormethylcarbonyl und R¹⁶ für Methyl stehen: 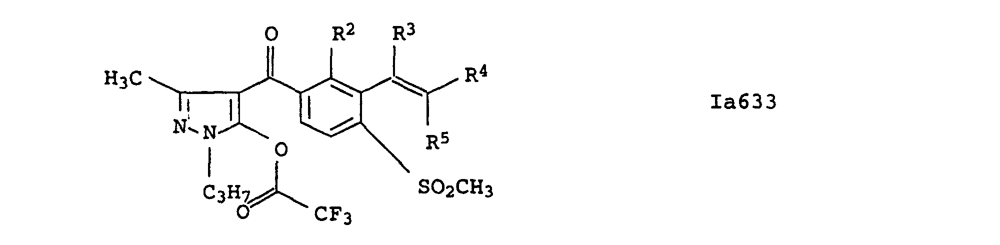
- Ebenso außergewöhnlich bevorzugt sind die Verbindungen Ia634, insbesondere die Verbindungen Ia634.001-Ia634.180, die sich von den entsprechenden Verbindungen Ia1.001-Ia1.180 dadurch unterscheiden, daß R¹ für Methylsulfonyl, R¹⁴ für n-Butyl, R¹⁵ für Trifluormethylcarbonyl und R¹⁶ für Methyl stehen: 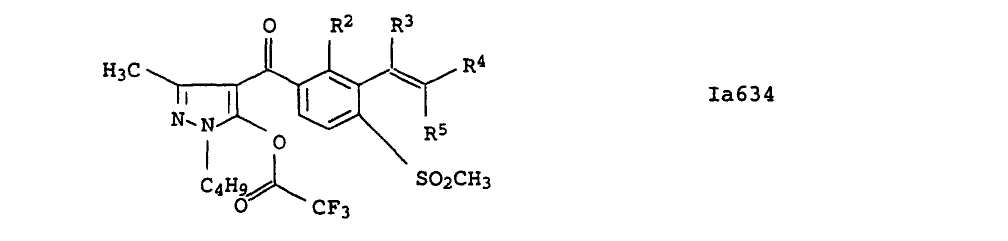
- Ebenso außergewöhnlich bevorzugt sind die Verbindungen Ia635, insbesondere die Verbindungen Ia635.001-Ia635.180, die sich von den entsprechenden Verbindungen Ia1.001-Ia1.180 dadurch unterscheiden, daß R¹ für Methylsulfonyl, R¹⁴ für iso-Butyl, R¹⁵ für Trifluormethylcarbonyl und R¹⁶ für Methyl stehen: 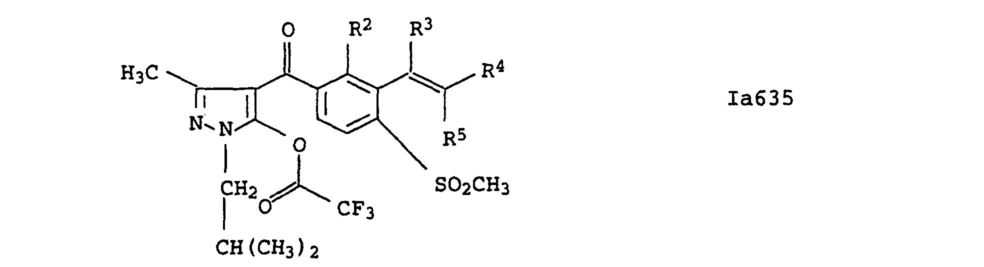
- Ebenso außergewöhnlich bevorzugt sind die Verbindungen Ia636, insbesondere die Verbindungen Ia636.001-Ia636.180, die sich von den entsprechenden Verbindungen Ia1.001-Ia1.180 dadurch unterscheiden, daß R¹ und R¹⁵ für Methylsulfonyl und R¹⁶ für Methyl stehen: 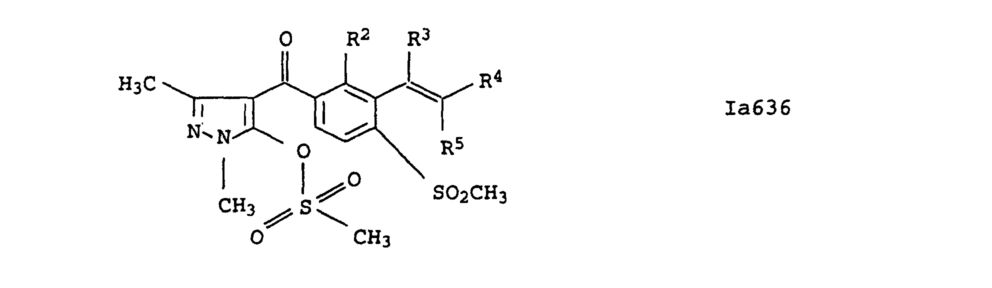
- Ebenso außergewöhnlich bevorzugt sind die Verbindungen Ia637, insbesondere die Verbindungen Ia637.001-Ia637.180, die sich von den entsprechenden Verbindungen Ia1.001-Ia1.180 dadurch unterscheiden, daß R¹ und R¹⁵ für Methylsulfonyl, R¹⁴ für Ethyl und R¹⁶ für Methyl stehen: 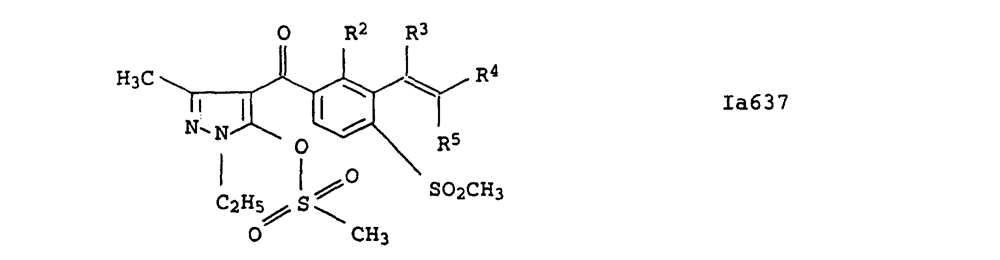
- Ebenso außergewöhnlich bevorzugt sind die Verbindungen Ia638, insbesondere die Verbindungen Ia638.001-Ia638.180, die sich von den entsprechenden Verbindungen Ia1.001-Ia1.180 dadurch unterscheiden, daß R¹ und R¹⁵ für Methylsulfonyl, R¹⁴ für n-Propyl und R¹⁶ für Methyl stehen: 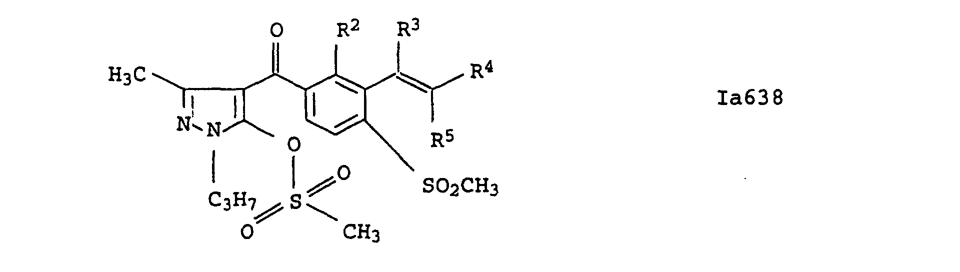
- Ebenso außergewöhnlich bevorzugt sind die Verbindungen Ia639, insbesondere die Verbindungen Ia639.001-Ia639.180, die sich von den entsprechenden Verbindungen Ia1.001-Ia1.180 dadurch unterscheiden, daß R¹ und R¹⁵ für Methylsulfonyl, R¹⁴ für n-Butyl und R¹⁶ für Methyl stehen: 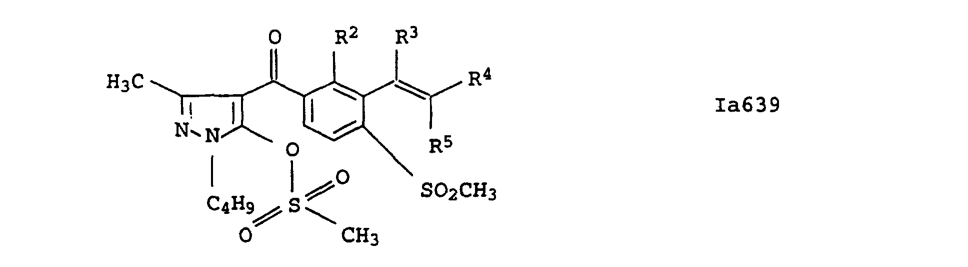
- Ebenso außergewöhnlich bevorzugt sind die Verbindungen Ia640, insbesondere die Verbindungen Ia640.001-Ia640.180, die sich von den entsprechenden Verbindungen Ia1.001-Ia1.180 dadurch unterscheiden, daß R¹ und R¹⁵ für Methylsulfonyl, R¹⁴ für iso-Butyl und R¹⁶ für Methyl stehen: 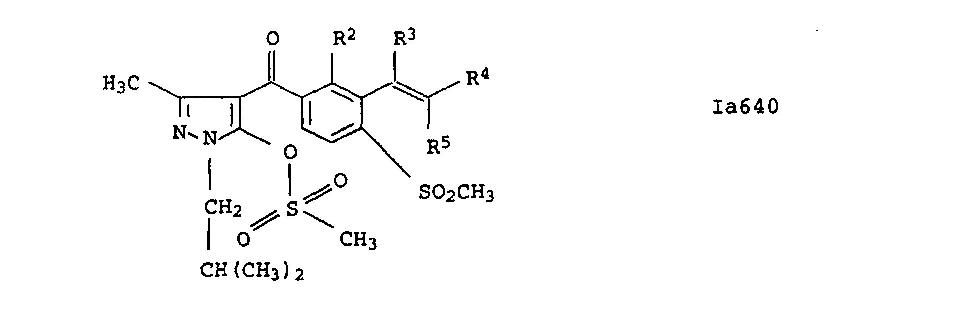
- Ebenso außergewöhnlich bevorzugt sind die Verbindungen Ia641, insbesondere die Verbindungen Ia641.001-Ia641.180, die sich von den entsprechenden Verbindungen Ia1.001-Ia1.180 dadurch unterscheiden, daß R¹ für Methylsulfonyl, R¹⁵ für Ethylsulfonyl und R¹⁶ für Methyl stehen: 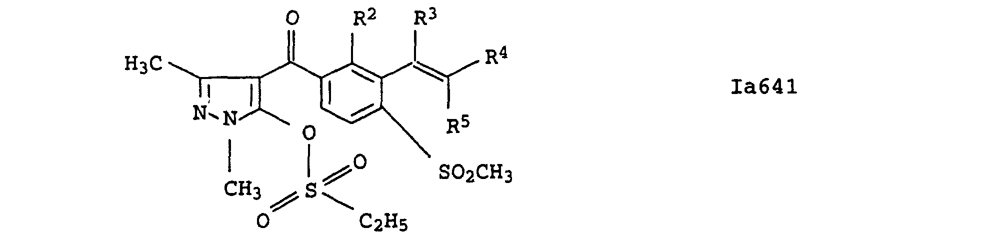
- Ebenso außergewöhnlich bevorzugt sind die Verbindungen Ia642, insbesondere die Verbindungen Ia642.001-Ia642.180, die sich von den entsprechenden Verbindungen Ia1.001-Ia1.180 dadurch unterscheiden, daß R¹ für Methylsulfonyl, R¹⁴ für Ethyl, R¹⁵ für Ethylsulfonyl und R¹⁶ für Methyl stehen: 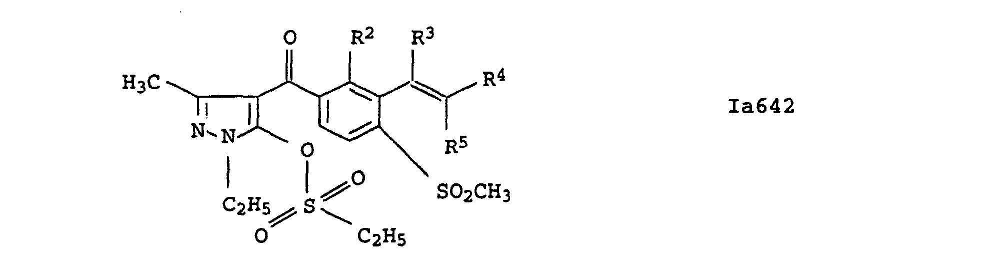
- Ebenso außergewöhnlich bevorzugt sind die Verbindungen Ia643, insbesondere die Verbindungen Ia643.001-Ia643.180, die sich von den entsprechenden Verbindungen Ia1.001-Ia1.180 dadurch unterscheiden, daß R¹ für Methylsulfonyl, R¹⁴ für n-Propyl, R¹⁵ für Ethylsulfonyl und R¹⁶ für Methyl stehen: 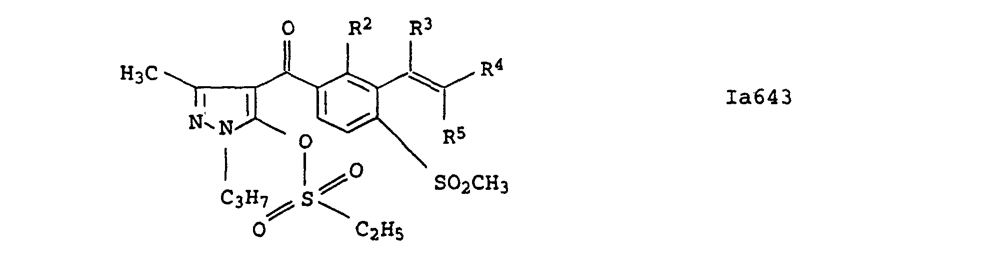
- Ebenso außergewöhnlich bevorzugt sind die Verbindungen Ia644, insbesondere die Verbindungen Ia644.001-Ia644.180, die sich von den entsprechenden Verbindungen Ia1.001-Ia1.180 dadurch unterscheiden, daß R¹ für Methylsulfonyl, R¹⁴ für n-Butyl, R¹⁵ für Ethylsulfonyl und R¹⁶ für Methyl stehen: 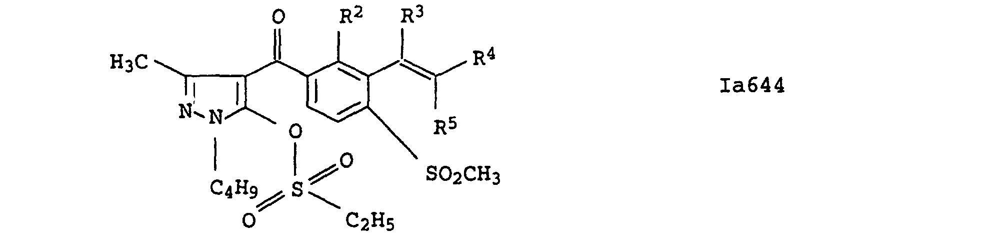
- Ebenso außergewöhnlich bevorzugt sind die Verbindungen Ia645, insbesondere die Verbindungen Ia645.001-Ia645.180, die sich von den entsprechenden Verbindungen Ia1.001-Ia1.180 dadurch unterscheiden, daß R¹ und R¹⁵ für Methylsulfonyl, R¹⁴ für iso-Butyl, R¹⁵ für Ethylsulfonyl und R¹⁶ für Methyl stehen: 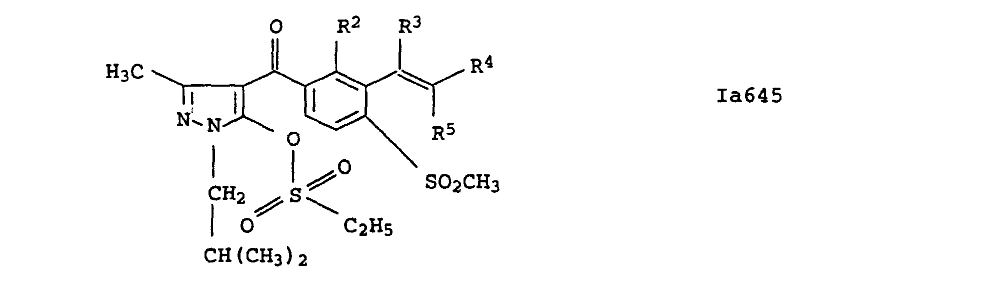
- Ebenso außergewöhnlich bevorzugt sind die Verbindungen Ia646, insbesondere die Verbindungen Ia646.001-Ia646.180, die sich von den entsprechenden Verbindungen Ia1.001-Ia1.180 dadurch unterscheiden, daß R¹ für Methylsulfonyl, R¹⁵ für n-Propylsulfonyl und R¹⁶ für Methyl stehen: 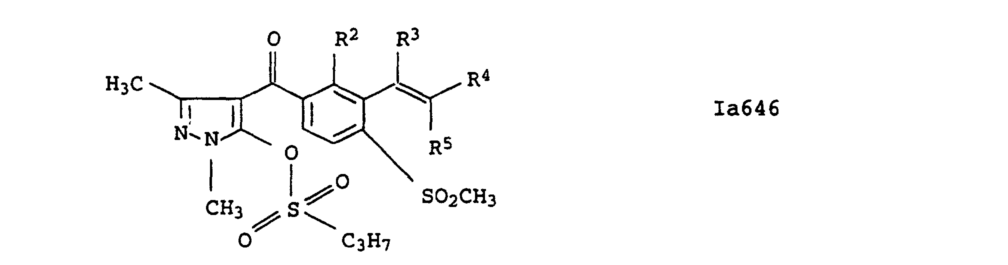 Ebenso außergewöhnlich bevorzugt sind die Verbindungen Ia647, insbesondere die Verbindungen Ia647.001-Ia647.180, die sich von den entsprechenden Verbindungen Ia1.001-Ia1.180 dadurch unterscheiden, daß R¹ für Methylsulfonyl, R¹⁴ für Ethyl, R¹⁵ für n-Propylsulfonyl und R¹⁶ für Methyl stehen: 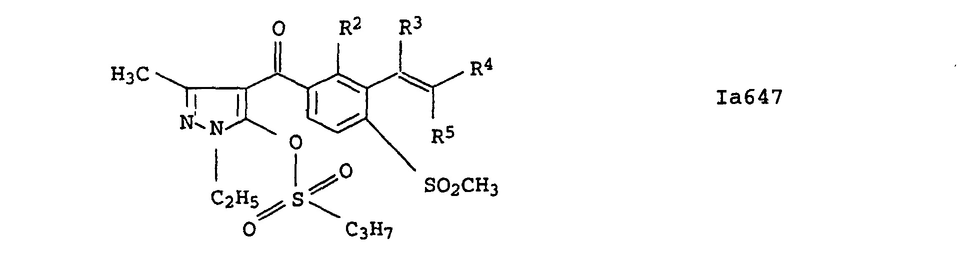
- Ebenso außergewöhnlich bevorzugt sind die Verbindungen Ia648, insbesondere die Verbindungen Ia648.001-Ia648.180, die sich von den entsprechenden Verbindungen Ia1.001-Ia1.180 dadurch unterscheiden, daß R¹ für Methylsulfonyl, R¹⁴ für n-Propyl, R¹⁵ für n-Propylsulfonyl und R¹⁶ für Methyl stehen: 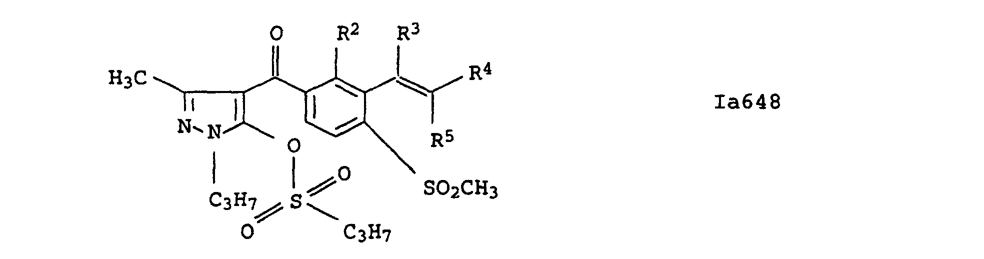
- Ebenso außergewöhnlich bevorzugt sind die Verbindungen Ia649, insbesondere die Verbindungen Ia649.001-Ia649.180, die sich von den entsprechenden Verbindungen Ia1.001-Ia1.180 dadurch unterscheiden, daß R¹ für Methylsulfonyl, R¹⁴ für n-Butyl, R¹⁵ für n-Propylsulfonyl und R¹⁶ für Methyl stehen: 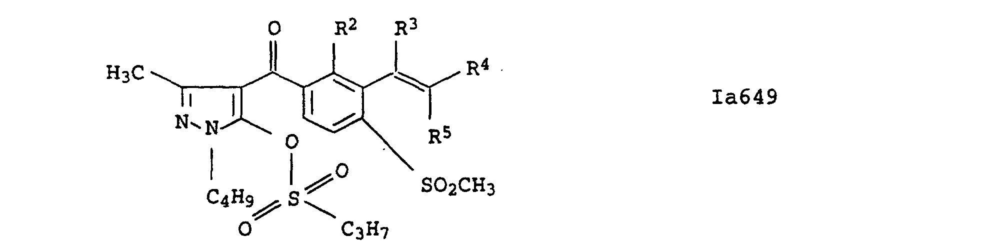 Ebenso außergewöhnlich bevorzugt sind die Verbindungen Ia650, insbesondere die Verbindungen Ia650.001-Ia650.180, die sich von den entsprechenden Verbindungen Ia1.001-Ia1.180 dadurch unterscheiden, daß R¹ und R¹⁵ für Methylsulfonyl, R¹⁴ für iso-Butyl, R¹⁵ für n-Propylsulfonyl und R¹⁶ für Methyl stehen: 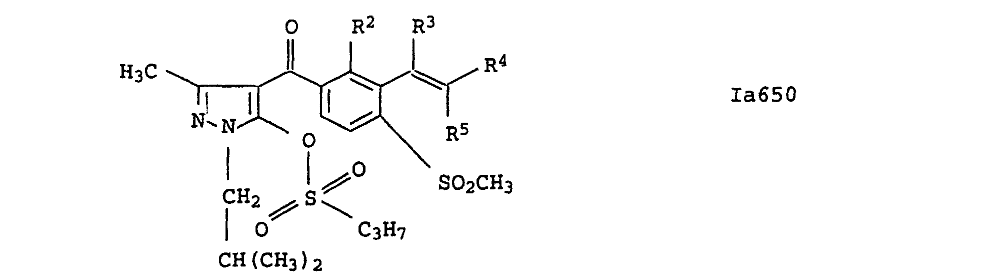
- Ebenso außergewöhnlich bevorzugt sind die Verbindungen Ia651, insbesondere die Verbindungen Ia651.001-Ia651.180, die sich von den entsprechenden Verbindungen Ia1.001-Ia1.180 dadurch unterscheiden, daß R¹ für Methylsulfonyl, R¹⁵ für iso-Propylsulfonyl und R¹⁶ für Methyl stehen: 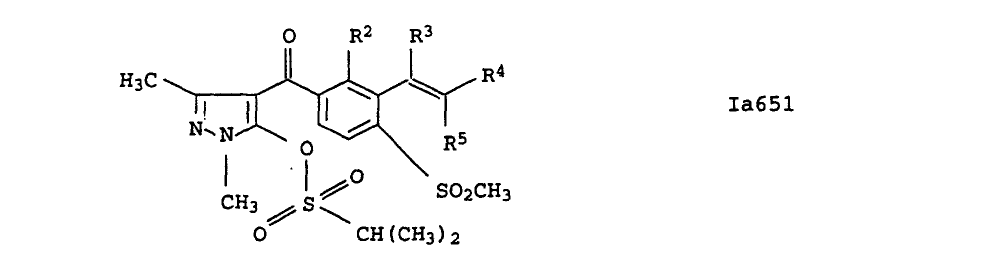
- Ebenso außergewöhnlich bevorzugt sind die Verbindungen Ia652, insbesondere die Verbindungen Ia652.001-Ia652.180, die sich von den entsprechenden Verbindungen Ia1.001-Ia1.180 dadurch unterscheiden, daß R¹ für Methylsulfonyl, R¹⁴ für Ethyl, R¹⁵ für iso-Propylsulfonyl und R¹⁶ für Methyl stehen: 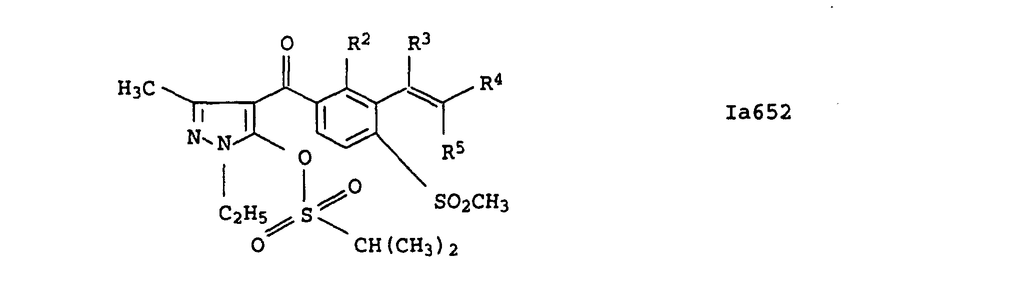
- Ebenso außergewöhnlich bevorzugt sind die Verbindungen Ia653, insbesondere die Verbindungen Ia653.001-Ia653.180, die sich von den entsprechenden Verbindungen Ia1.001-Ia1.180 dadurch unterscheiden, daß R¹ für Methylsulfonyl, R¹⁴ für n-Propyl, R¹⁵ für iso-Propylsulfonyl und R¹⁶ für Methyl stehen: 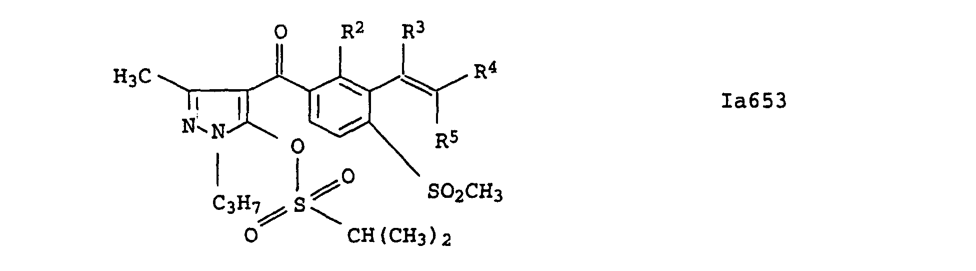
- Ebenso außergewöhnlich bevorzugt sind die Verbindungen Ia654, insbesondere die Verbindungen Ia654.001-Ia654.180, die sich von den entsprechenden Verbindungen Ia1.001-Ia1.180 dadurch unterscheiden, daß R¹ für Methylsulfonyl, R¹⁴ für n-Butyl, R¹⁵ für iso-Propylsulfonyl und R¹⁶ für Methyl stehen: 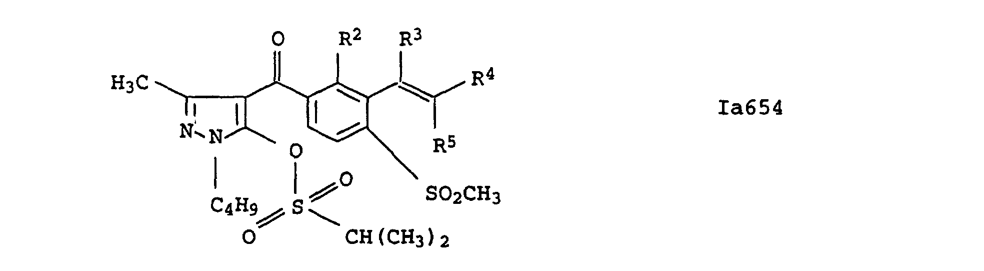
- Ebenso außergewöhnlich bevorzugt sind die Verbindungen Ia655, insbesondere die Verbindungen Ia655.001-Ia655.180, die sich von den entsprechenden Verbindungen Ia1.001-Ia1.180 dadurch unterscheiden, daß R¹ für Methylsulfonyl, R¹⁴ für iso-Butyl, R¹⁵ für iso-Propylsulfonyl und R¹⁶ für Methyl stehen: 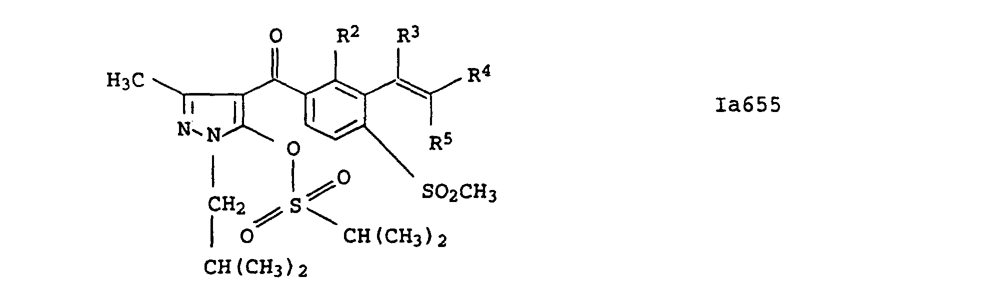
- Ebenso außergewöhnlich bevorzugt sind die Verbindungen Ia656, insbesondere die Verbindungen Ia656.001-Ia656.180, die sich von den entsprechenden Verbindungen Ia1.001-Ia1.180 dadurch unterscheiden, daß R¹ für Methylsulfonyl, R¹⁵ für n-Butylsulfonyl und R¹⁶ für Methyl stehen: 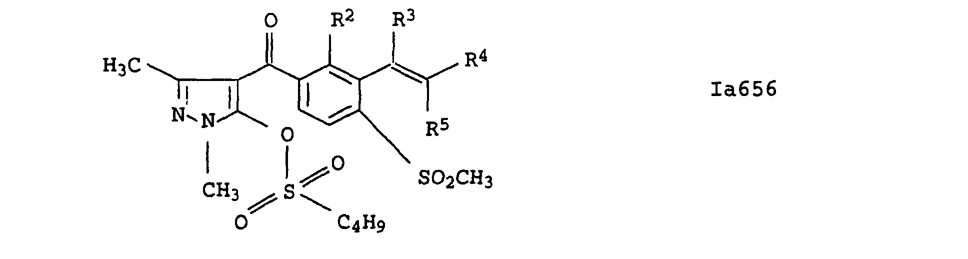
- Ebenso außergewöhnlich bevorzugt sind die Verbindungen Ia657, insbesondere die Verbindungen Ia657.001-Ia657.180, die sich von den entsprechenden Verbindungen Ia1.001-Ia1.180 dadurch unterscheiden, daß R¹ für Methylsulfonyl, R¹⁴ für Ethyl, R¹⁵ für n-Butylsulfonyl und R¹⁶ für Methyl stehen: 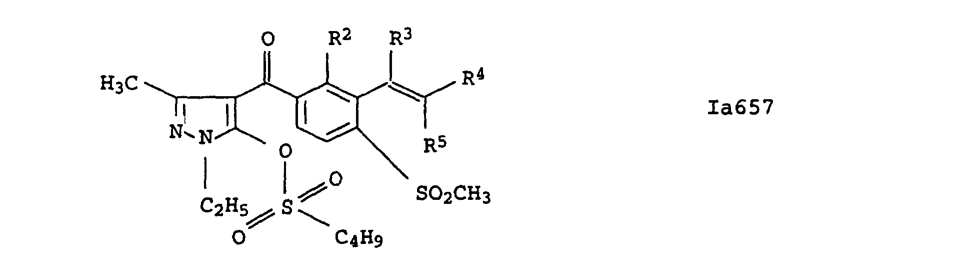
- Ebenso außergewöhnlich bevorzugt sind die Verbindungen Ia658, insbesondere die Verbindungen Ia658.001-Ia658.180, die sich von den entsprechenden Verbindungen Ia1.001-Ia1.180 dadurch unterscheiden, daß R¹ für Methylsulfonyl, R¹⁴ für n-Propyl, R¹⁵ für n-Butylsulfonyl und R¹⁶ für Methyl stehen: 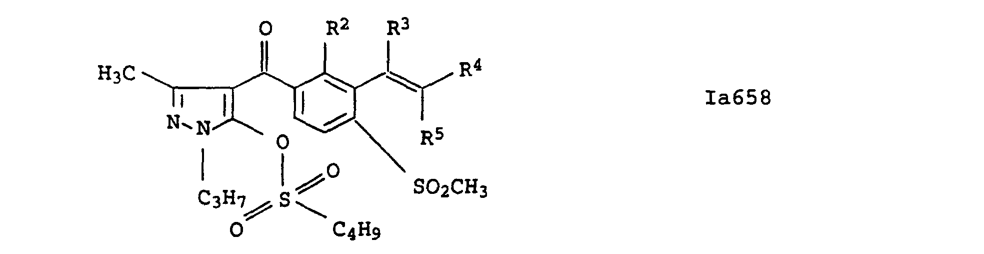
- Ebenso außergewöhnlich bevorzugt sind die Verbindungen Ia659, insbesondere die Verbindungen Ia659.001-Ia659.180, die sich von den entsprechenden Verbindungen Ia1.001-Ia1.180 dadurch unterscheiden, daß R¹ für Methylsulfonyl, R¹⁴ für n-Butyl, R¹⁵ für n-Butylsulfonyl und R¹⁶ für Methyl stehen: 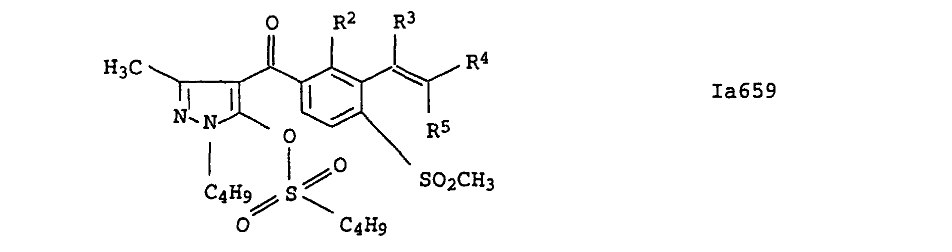
- Ebenso außergewöhnlich bevorzugt sind die Verbindungen Ia660, insbesondere die Verbindungen Ia660.001-Ia660.180, die sich von den entsprechenden Verbindungen Ia1.001-Ia1.180 dadurch unterscheiden, daß R¹ und R¹⁵ für Methylsulfonyl, R¹⁴ für iso-Butyl, R¹⁵ für n-Butylsulfonyl und R¹⁶ für Methyl stehen: 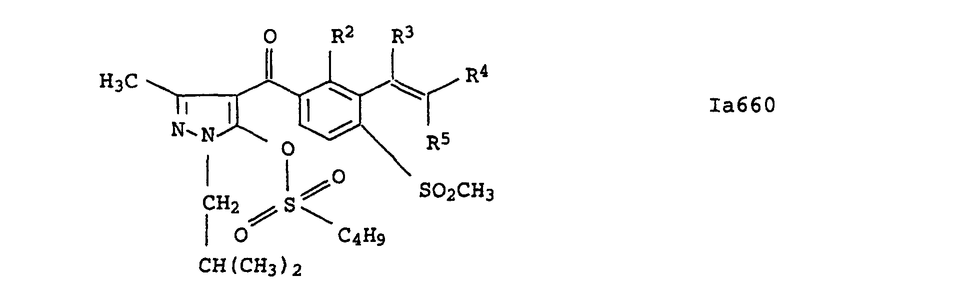
- Ebenso außergewöhnlich bevorzugt sind die Verbindungen Ia661, insbesondere die Verbindungen Ia661.001-Ia661.180, die sich von den entsprechenden Verbindungen Ia1.001-Ia1.180 dadurch unterscheiden, daß R¹ für Methylsulfonyl, R¹⁵ für iso-Butylsulfonyl und R¹⁶ für Methyl stehen: 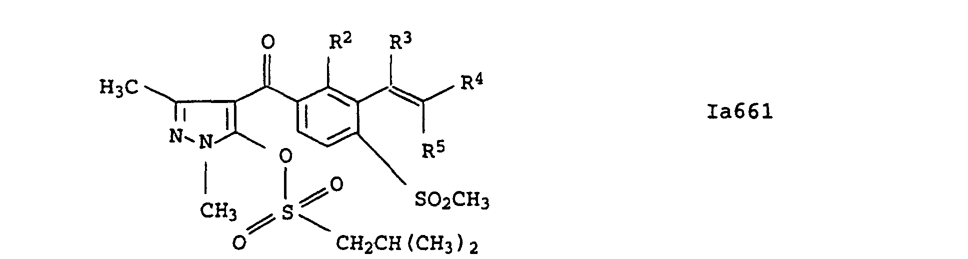
- Ebenso außergewöhnlich bevorzugt sind die Verbindungen Ia662, insbesondere die Verbindungen Ia662.001-Ia662.180, die sich von den entsprechenden Verbindungen Ia1.001-Ia1.180 dadurch unterscheiden, daß R¹ für Methylsulfonyl, R¹⁴ für Ethyl, R¹⁵ für iso-Butylsulfonyl und R¹⁶ für Methyl stehen: 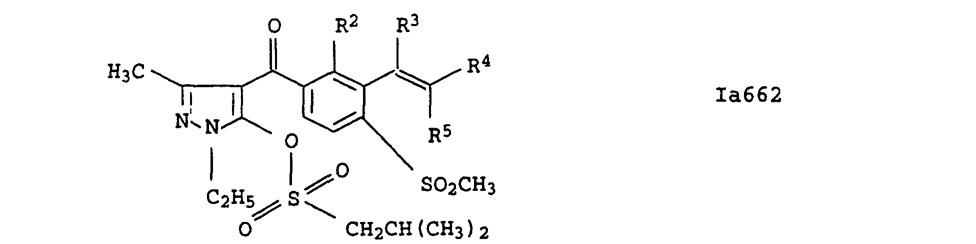
- Ebenso außergewöhnlich bevorzugt sind die Verbindungen Ia663, insbesondere die Verbindungen Ia663.001-Ia663.180, die sich von den entsprechenden Verbindungen Ia1.001-Ia1.180 dadurch unterscheiden, daß R¹ für Methylsulfonyl, R¹⁴ für n-Propyl, R¹⁵ für iso-Butylsulfonyl und R¹⁶ für Methyl stehen: 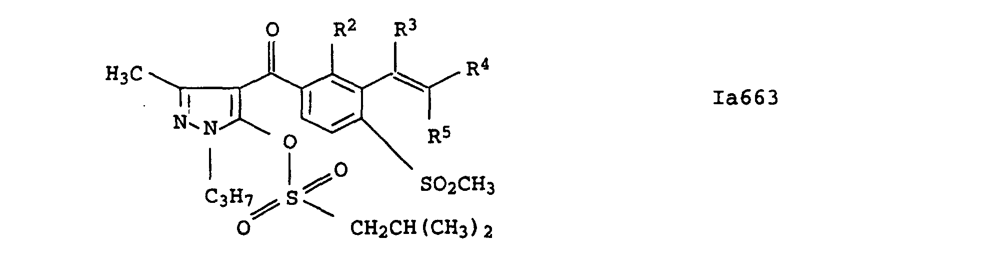
- Ebenso außergewöhnlich bevorzugt sind die Verbindungen Ia664, insbesondere die Verbindungen Ia664.001-Ia664.180, die sich von den entsprechenden Verbindungen Ia1.001-Ia1.180 dadurch unterscheiden, daß R¹ für Methylsulfonyl, R¹⁴ für n-Butyl, R¹⁵ für iso-Butylsulfonyl und R¹⁶ für Methyl stehen: 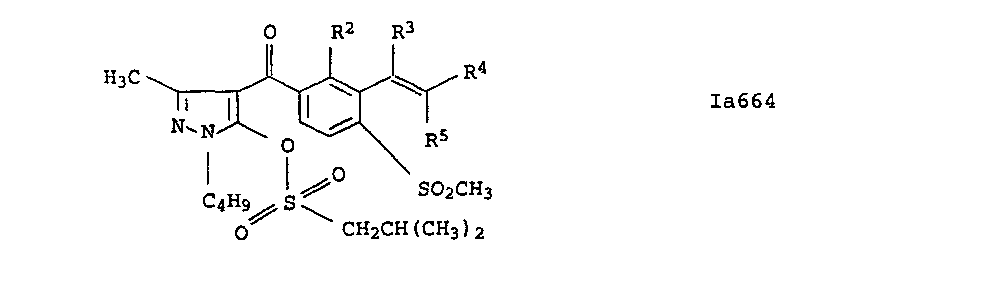
- Ebenso außergewöhnlich bevorzugt sind die Verbindungen Ia665, insbesondere die Verbindungen Ia665.001-Ia665.180, die sich von den entsprechenden Verbindungen Ia1.001-Ia1.180 dadurch unterscheiden, daß R¹ für Methylsulfonyl, R¹⁴ für iso-Butyl, R¹⁵ für iso-Butylsulfonyl und R¹⁶ für Methyl stehen: 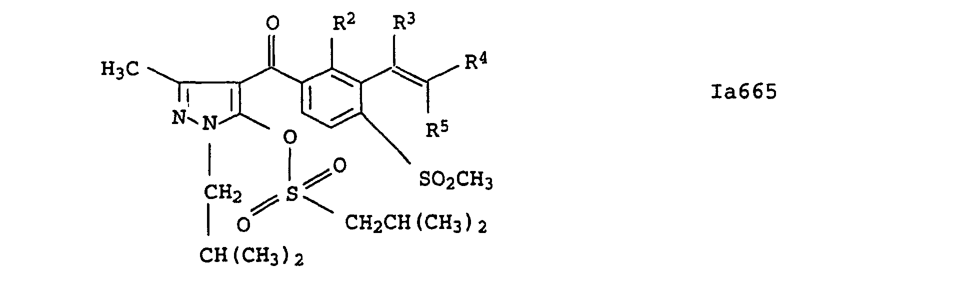
- Ebenso außergewöhnlich bevorzugt sind die Verbindungen Ia666, insbesondere die Verbindungen Ia666.001-Ia666.180, die sich von den entsprechenden Verbindungen Ia1.001-Ia1.180 dadurch unterscheiden, daß R¹ für Methylsulfonyl, R¹⁵ für sec-Butylsulfonyl und R¹⁶ für Methyl stehen: 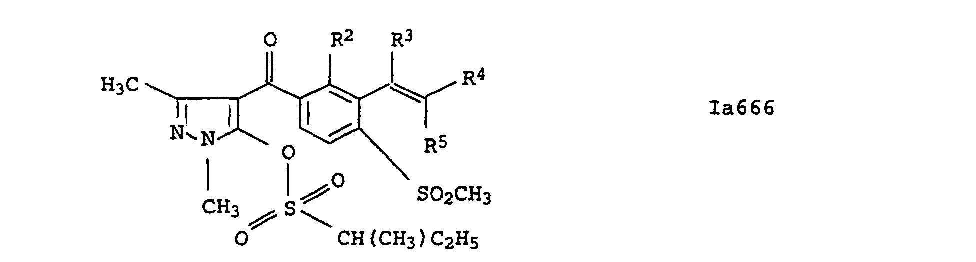
- Ebenso außergewöhnlich bevorzugt sind die Verbindungen Ia667, insbesondere die Verbindungen Ia667.001-Ia667.180, die sich von den entsprechenden Verbindungen Ia1.001-Ia1.180 dadurch unterscheiden, daß R¹ für Methylsulfonyl, R¹⁴ für Ethyl, R¹⁵ für sec-Butylsulfonyl und R¹⁶ für Methyl stehen: 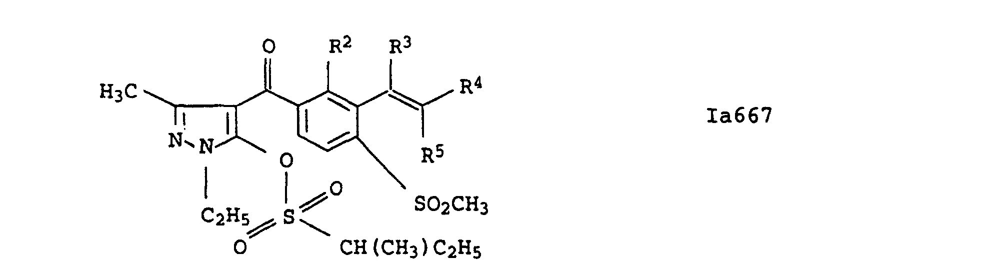
- Ebenso außergewöhnlich bevorzugt sind die Verbindungen Ia668, insbesondere die Verbindungen Ia668.001-Ia668.180, die sich von den entsprechenden Verbindungen Ia1.001-Ia1.180 dadurch unterscheiden, daß R¹ für Methylsulfonyl, R¹⁴ für n-Propyl, R¹⁵ für sec-Butylsulfonyl und R¹⁶ für Methyl stehen: 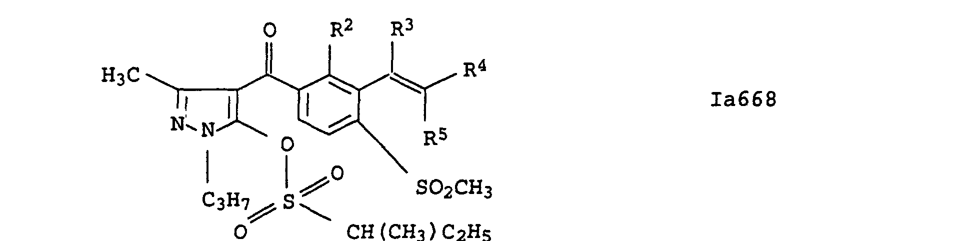
- Ebenso außergewöhnlich bevorzugt sind die Verbindungen Ia669, insbesondere die Verbindungen Ia669.001-Ia669.180, die sich von den entsprechenden Verbindungen Ia1.001-Ia1.180 dadurch unterscheiden, daß R¹ für Methylsulfonyl, R¹⁴ für n-Butyl, R¹⁵ für sec-Butylsulfonyl und R¹⁶ für Methyl stehen: 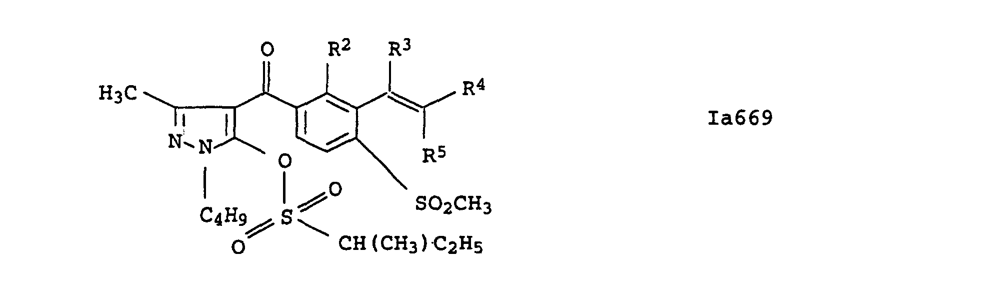
- Ebenso außergewöhnlich bevorzugt sind die Verbindungen Ia670, insbesondere die Verbindungen Ia670.001-Ia670.180, die sich von den entsprechenden Verbindungen Ia1.001-Ia1.180 dadurch unterscheiden, daß R¹ für Methylsulfonyl, R¹⁴ für iso-Butyl, R¹⁵ für sec-Butylsulfonyl und R¹⁶ für Methyl stehen: 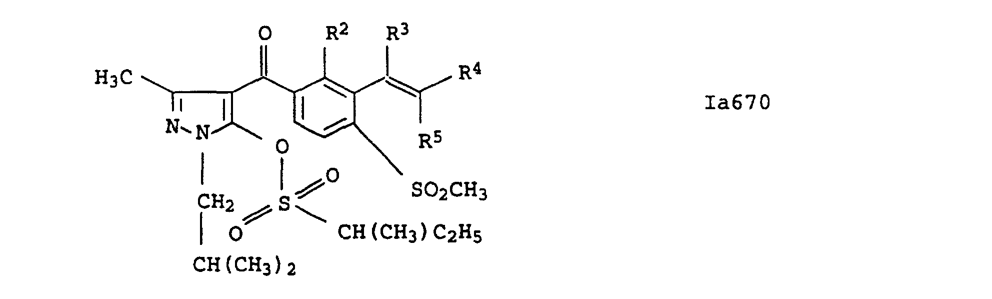
- Ebenso außergewöhnlich bevorzugt sind die Verbindungen Ia671, insbesondere die Verbindungen Ia671.001-Ia671.180, die sich von den entsprechenden Verbindungen Ia1.001-Ia1.180 dadurch unterscheiden, daß R¹ für Methylsulfonyl, R¹⁵ für Trifluormethylsulfonyl und R¹⁶ für Methyl stehen: 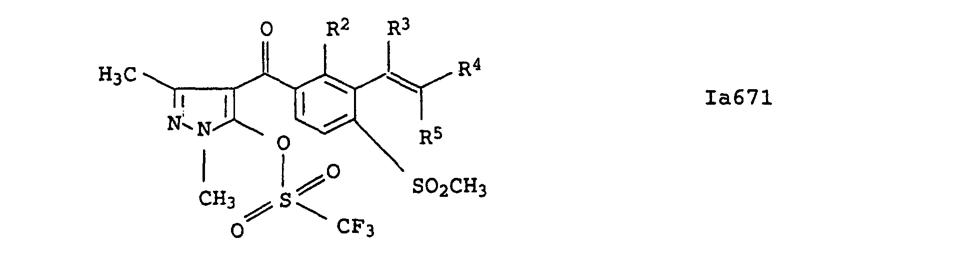
- Ebenso außergewöhnlich bevorzugt sind die Verbindungen Ia672, insbesondere die Verbindungen Ia672.001-Ia672.180, die sich von den entsprechenden Verbindungen Ia1.001-Ia1.180 dadurch unterscheiden, daß R¹ für Methylsulfonyl, R¹⁴ für Ethyl, R¹⁵ für Trifluormethylsulfonyl und R¹⁶ für Methyl stehen: 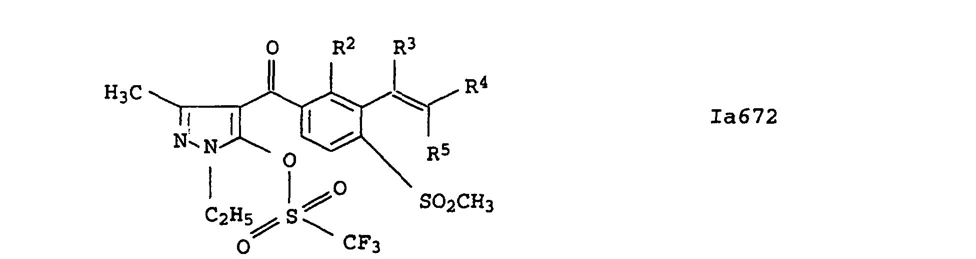
- Ebenso außergewöhnlich bevorzugt sind die Verbindungen Ia673, insbesondere die Verbindungen Ia673.001-Ia673.180, die sich von den entsprechenden Verbindungen Ia1.001-Ia1.180 dadurch unterscheiden, daß R¹ für Methylsulfonyl, R¹⁴ für n-Propyl, R¹⁵ für Trifluormethylsulfonyl und R¹⁶ für Methyl stehen: 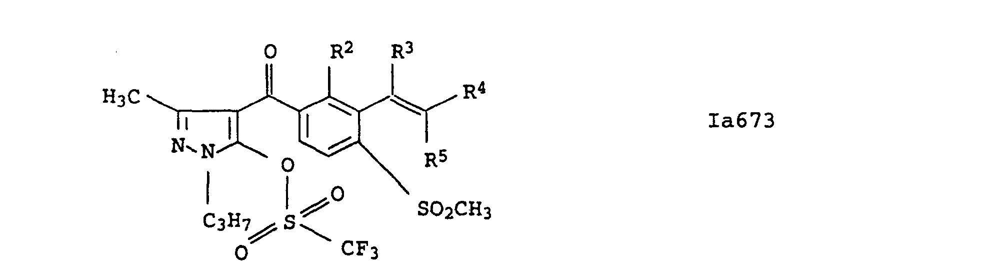
- Ebenso außergewöhnlich bevorzugt sind die Verbindungen Ia674, insbesondere die Verbindungen Ia674.001-Ia674.180, die sich von den entsprechenden Verbindungen Ia1.001-Ia1.180 dadurch unterscheiden, daß R¹ für Methylsulfonyl, R¹⁴ für n-Butyl, R¹⁵ für Trifluormethylsulfonyl und R¹⁶ für Methyl stehen: 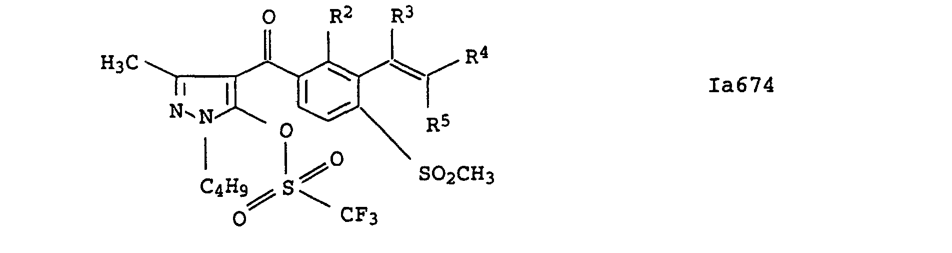
- Ebenso außergewöhnlich bevorzugt sind die Verbindungen Ia675, insbesondere die Verbindungen Ia675.001-Ia675.180, die sich von den entsprechenden Verbindungen Ia1.001-Ia1.180 dadurch unterscheiden, daß R¹ für Methylsulfonyl, R¹⁴ für iso-Butyl, R¹⁵ für Trifluormethylsulfonyl und R¹⁶ für Methyl stehen: 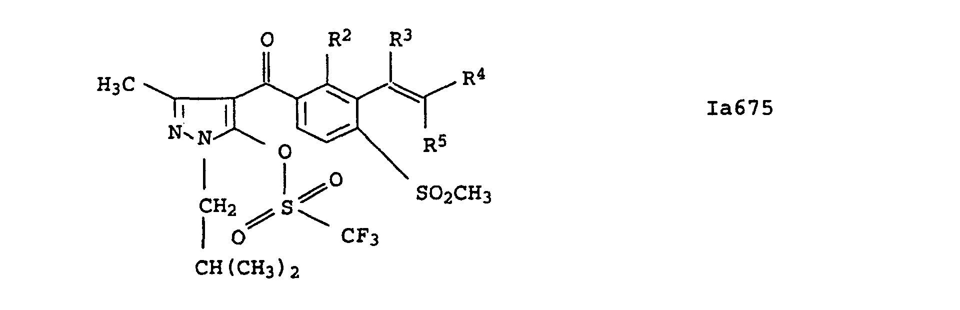
- Ebenso außergewöhnlich bevorzugt sind die Verbindungen Ia676, insbesondere die Verbindungen Ia676.001-Ia676.180, die sich von den entsprechenden Verbindungen Ia1.001-Ia1.180 dadurch unterscheiden, daß R¹ für Methylsulfonyl, R¹⁴ für Methyl, R¹⁵ für Phenylcarbonylmethyl und R¹⁶ für Methyl stehen: 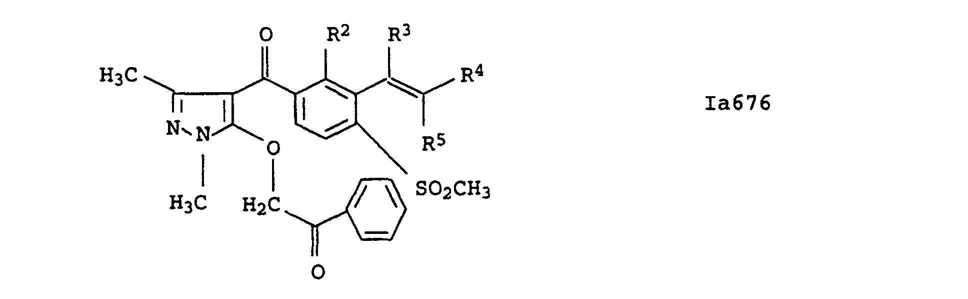
- Ebenso außergewöhnlich bevorzugt sind die Verbindungen Ia677, insbesondere die Verbindungen Ia677.001-Ia677.180, die sich von den entsprechenden Verbindungen Ia1.001-Ia1.180 dadurch unterscheiden, daß R¹ für Methylsulfonyl, R¹⁴ für Ethyl, R¹⁵ für Phenylcarbonylmethyl und R¹⁶ für Methyl stehen: 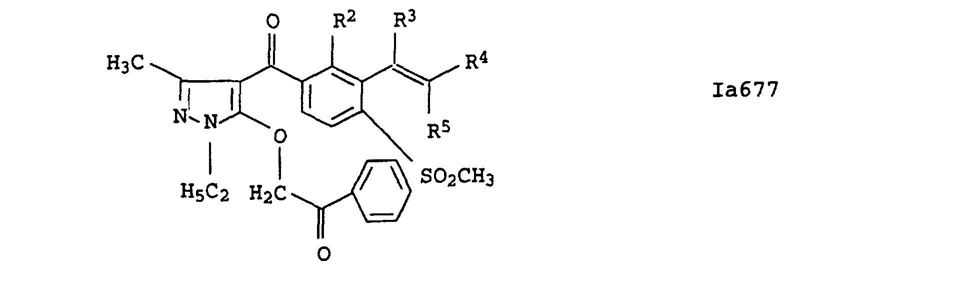
- Ebenso außergewöhnlich bevorzugt sind die Verbindungen Ia678, insbesondere die Verbindungen Ia678.001-Ia678.180, die sich von den entsprechenden Verbindungen Ia1.001-Ia1.180 dadurch unterscheiden, daß R¹ für Methylsulfonyl, R¹⁴ für n-Propyl, R¹⁵ für Phenylcarbonylmethyl und R¹⁶ für Methyl stehen: 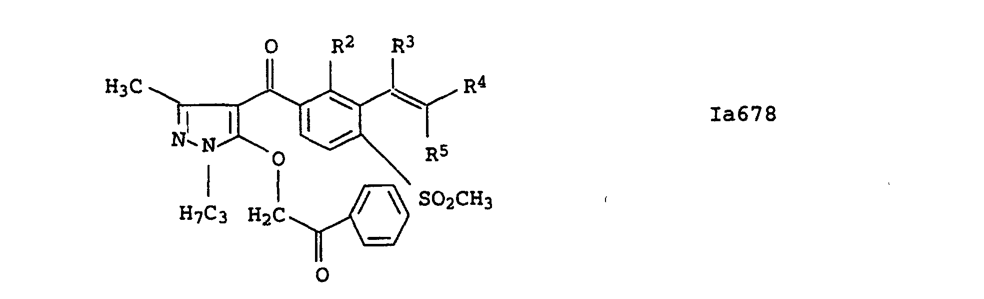
- Ebenso außergewöhnlich bevorzugt sind die Verbindungen Ia679, insbesondere die Verbindungen Ia679.001-Ia679.180, die sich von den entsprechenden Verbindungen Ia1.001-Ia1.180 dadurch unterscheiden, daß R¹ für Methylsulfonyl, R¹⁴ für n-Butyl, R¹⁵ für Phenylcarbonylmethyl und R¹⁶ für Methyl stehen: 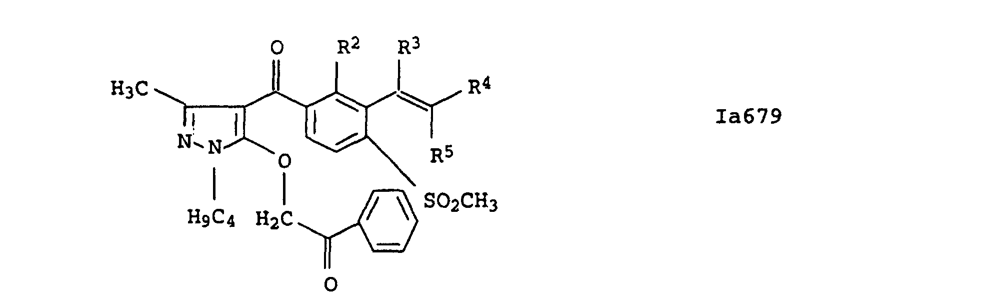
- Ebenso außergewöhnlich bevorzugt sind die Verbindungen Ia680, insbesondere die Verbindungen Ia680.001-Ia680.180, die sich von den entsprechenden Verbindungen Ia1.001-Ia1.180 dadurch unterscheiden, daß R¹ für Methylsulfonyl, R¹⁴ für iso-Butyl, R¹⁵ für Phenylcarbonylmethyl und R¹⁶ für Methyl stehen: 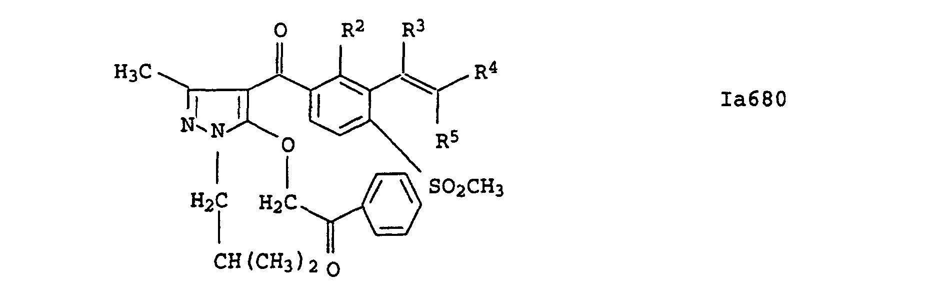
- Ebenso außergewöhnlich bevorzugt sind die Verbindungen Ia681, insbesondere die Verbindungen Ia681.001-Ia681.180, die sich von den entsprechenden Verbindungen Ia1.001-Ia1.180 dadurch unterscheiden, daß R¹ für Methylsulfonyl, R¹⁵ für Phenylsulfonyl und R¹⁶ für Methyl stehen: 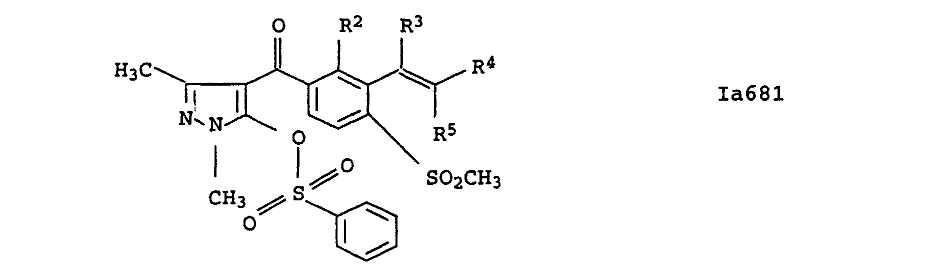
- Ebenso außergewöhnlich bevorzugt sind die Verbindungen Ia682, insbesondere die Verbindungen Ia682.001-Ia682.180, die sich von den entsprechenden Verbindungen Ia1.001-Ia1.180 dadurch unterscheiden, daß R¹ für Methylsulfonyl, R¹⁴ für n-Propyl, R¹⁵ für Phenylsulfonyl und R¹⁶ für Methyl stehen: 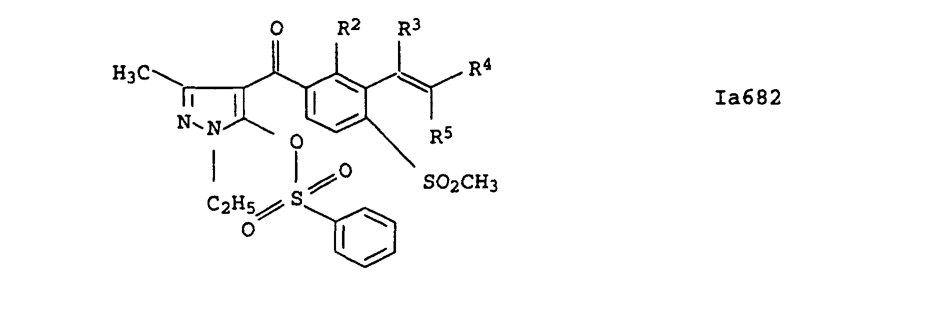
- Ebenso außergewöhnlich bevorzugt sind die Verbindungen Ia683, insbesondere die Verbindungen Ia683.001-Ia683.180, die sich von den entsprechenden Verbindungen Ia1.001-Ia1.180 dadurch unterscheiden, daß R¹ für Methylsulfonyl, R¹⁴ für Ethyl, R¹⁵ für Phenylsulfonyl und R¹⁶ für Methyl stehen: 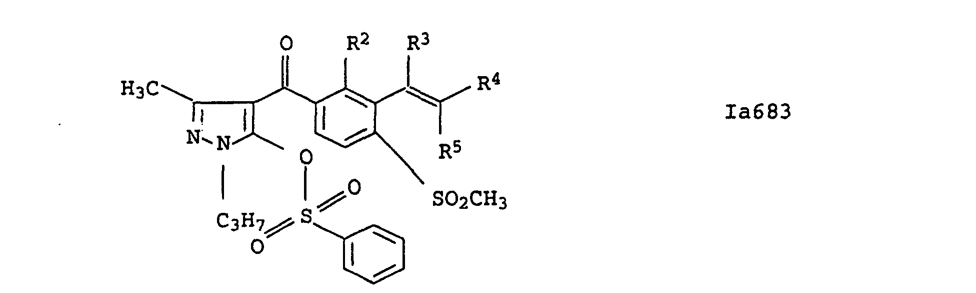
- Ebenso außergewöhnlich bevorzugt sind die Verbindungen Ia684, insbesondere die Verbindungen Ia684.001-Ia684.180, die sich von den entsprechenden Verbindungen Ia1.001-Ia1.180 dadurch unterscheiden, daß R¹ für Methylsulfonyl, R¹⁴ für n-Butyl, R¹⁵ für Phenylsulfonyl und R¹⁶ für Methyl stehen: 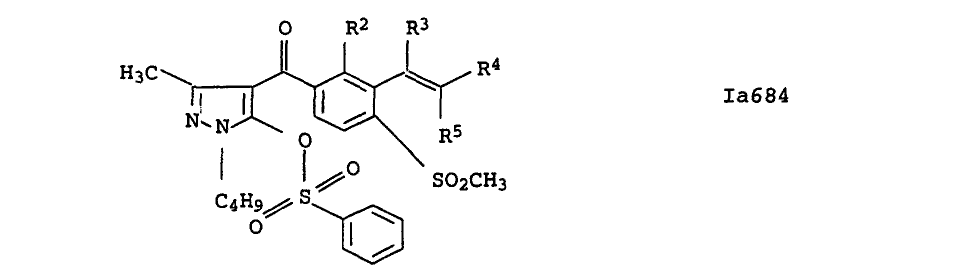
- Ebenso außergewöhnlich bevorzugt sind die Verbindungen Ia685, insbesondere die Verbindungen Ia685.001-Ia685.180, die sich von den entsprechenden Verbindungen Ia1.001-Ia1.180 dadurch unterscheiden, daß R¹ für Methylsulfonyl, R¹⁴ für iso-Butyl, R¹⁵ für Phenylsulfonyl und R¹⁶ für Methyl stehen: 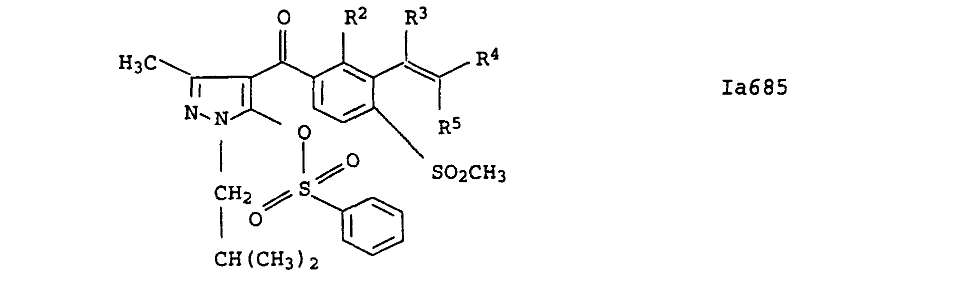
- Ebenso außergewöhnlich bevorzugt sind die Verbindungen Ia686, insbesondere die Verbindungen Ia686.001-Ia686.180, die sich von den entsprechenden Verbindungen Ia1.001-Ia1.180 dadurch unterscheiden, daß R¹ für Methylsulfonyl, R¹⁵ für 4-Methylphenylsulfonyl und R¹⁶ für Methyl stehen: 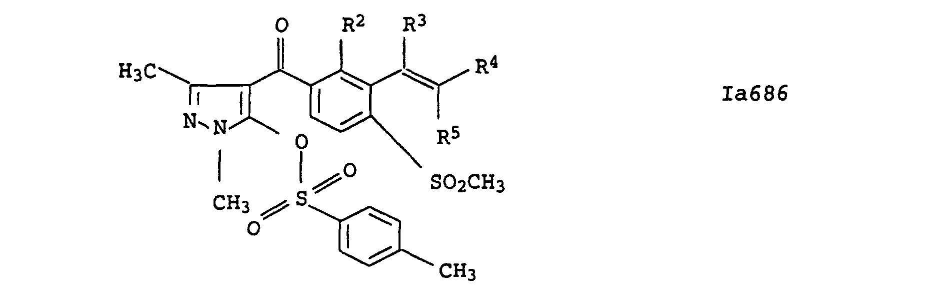
- Ebenso außergewöhnlich bevorzugt sind die Verbindungen Ia687, insbesondere die Verbindungen Ia687.001-Ia687.180, die sich von den entsprechenden Verbindungen Ia1.001-Ia1.180 dadurch unterscheiden, daß R¹ für Methylsulfonyl, R¹⁴ für Ethyl, R¹⁵ für 4-Methylphenylsulfonyl und R¹⁶ für Methyl stehen: 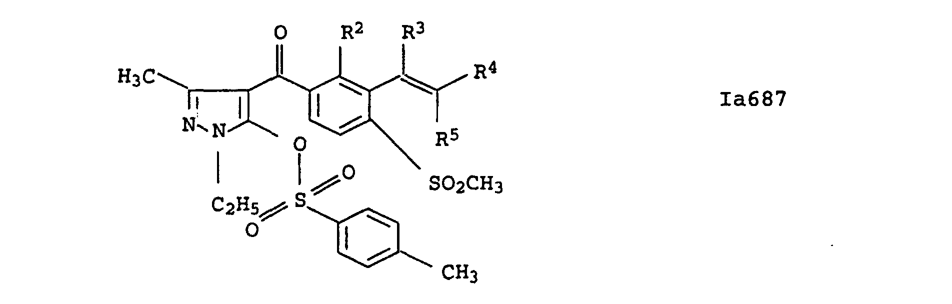
- Ebenso außergewöhnlich bevorzugt sind die Verbindungen Ia688, insbesondere die Verbindungen Ia688.001-Ia688.180, die sich von den entsprechenden Verbindungen Ia1.001-Ia1.180 dadurch unterscheiden, daß R¹ für Methylsulfonyl, R¹⁴ für n-Propyl, R¹⁵ für 4-Methylphenylsulfonyl und R¹⁶ für Methyl stehen: 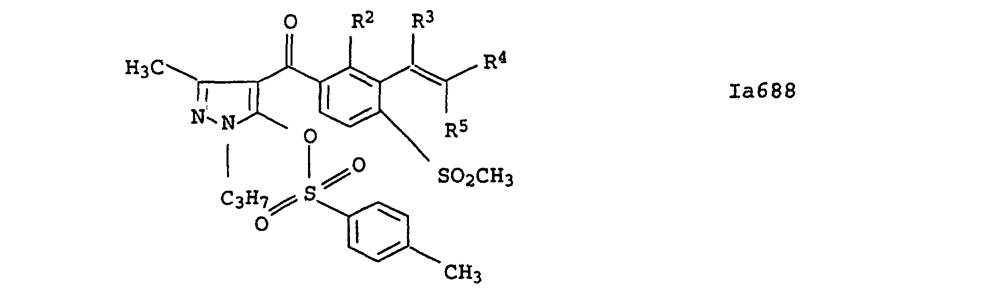
- Ebenso außergewöhnlich bevorzugt sind die Verbindungen Ia689, insbesondere die Verbindungen Ia689.001-Ia689.180, die sich von den entsprechenden Verbindungen Ia1.001-Ia1.180 dadurch unterscheiden, daß R¹ für Methylsulfonyl, R¹⁴ für n-Butyl, R¹⁵ für 4-Methylphenylsulfonyl und R¹⁶ für Methyl stehen: 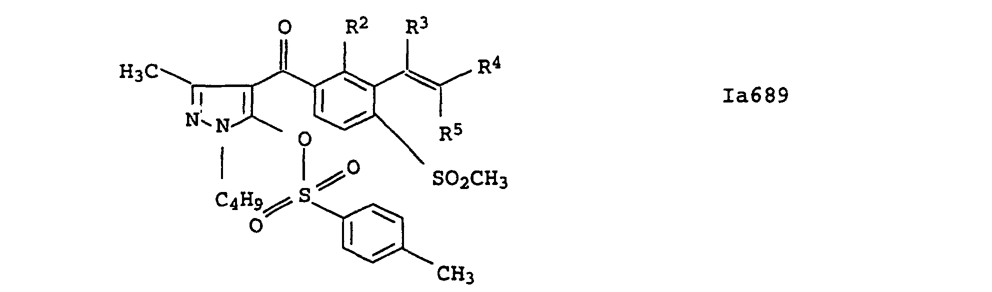
- Ebenso außergewöhnlich bevorzugt sind die Verbindungen Ia690, insbesondere die Verbindungen Ia690.001-Ia690.180, die sich von den entsprechenden Verbindungen Ia1.001-Ia1.180 dadurch unterscheiden, daß R¹ für Methylsulfonyl, R¹⁴ für iso-Butyl, R¹⁵ für 4-Methylphenylsulfonyl und R¹⁶ für Methyl stehen:

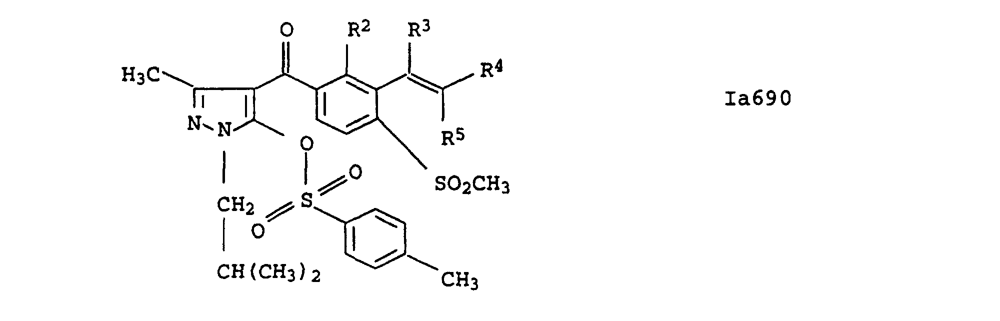

Ebenso außergewöhnlich bevorzugt sind die Verbindungen der Formel I, wobei
- R¹: Halogen wie Chlor oder Brom, C₁-C₆-Alkylsulfonyl wie Methylsulfonyl oder Ethylsulfonyl; besonders bevorzugt Chlor oder Methylsulfonyl;
- R²: Halogen wie Chlor oder Brom;
besonders bevorzugt Chlor;
- R³: Wasserstoff
- R⁴: Wasserstoff, Cyano, C₁-C₆-Alkyl wie Methyl, Ethyl, Isopropyl, Pentyl, Formyl, C₁-C₆-Alkylcarbonyl wie Methylcarbonyl oder Ethylcarbonyl, Hydroxycarbonyl, C₁-C₆-Alkoxycarbonyl wie Methoxcarbonyl oder Ethoxycarbonyl, -C(R¹²)=NR¹³; Heterocyclyl, Heterocyclyl-C₁-C₄-alkyl (wobei der Heterocyclyl-Rest der beiden letztgenannten Reste drei- bis siebengliedrig, gesättigt oder partiell ungesättigt, mono- oder polycyclisch ist, und ein- bis drei Heteroatome, ausgewählt aus der Gruppe bestehend aus Sauerstoff, Stickstoff und Schwefel, enthält), Phenyl, Hetaryl (wobei der Hetaryl-Rest aromatisch, mono- oder polycyclisch ist und neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten kann), wobei die vier letztgenannten Reste ihrerseits substituiert sein können durch ein bis drei Halogenatome und/oder einen bis drei Reste aus folgender Gruppe tragen können: Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl, C₁-C₄-Alkoxycarbonyl, insbesondere C₁-C₄-Alkyl;
besonders bevorzugt Wasserstoff, Cyano, Methyl, Isopropyl, Pentyl, Formyl, Methylcarbonyl, Hydroxycarbonyl, Ethoxycarbonyl, -C(R¹²)=NR¹³, 2-Methyl-1,3-dioxolan-4-yl, 2,2-Dimethyl-1,3-dioxolan-4-yl, 1,3-Dioxan-2-yl, 1,3-Dioxan-2-ylmethyl, Phenyl, 2-Furyl, 3-Furyl, 2-Thienyl, 3-Thienyl;
- R⁵: Wasserstoff, Halogen wie Chlor oder Brom, C₁-C₆-Alkyl wie Methyl oder Ethyl;
besonders bevorzugt Wasserstoff, Chlor oder Methyl;
- R¹²: Wasserstoff, C₁-C₆-Alkyl wie Methyl oder Ethyl, C₁-C₆-Alkoxycarbonyl wie Methoxycarbonyl oder Ethoxycarbonyl;
besonders bevorzugt Wasserstoff, Methyl oder Ethoxycarbonyl;
- R¹³: C₁-C₆-Alkoxy wie Methoxy oder Ethoxy, oder Phenyl-C₁-C₄-alkoxy, wobei der letztgenannte Rest partiell oder vollständig halogeniert sein kann und/oder einen bis drei Reste aus folgender Gruppe tragen kann: Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl, C₁-C₄-Alkoxycarbonyl;
- R¹⁴: C₁-C₆-Alkyl;
besonders bevorzugt Methyl oder Ethyl;
- R¹⁵: Wasserstoff, C₁-C₆-Alkylsulfonyl wie Methylsulfonyl oder Ethylsulfonyl, Phenyl-C₁-C₄-alkyl, wobei der Phenyl des letztgenannten Substituenten partiell oder vollständig halogeniert sein kann und/oder einen bis drei der folgenden Reste trägt: Nitro, Cyano, C₁-C₄-Alkyl, C₁-C₄-Halogenalkyl, C₁-C₄-Alkoxy, C₁-C₄-Halogenalkoxy;
besonders bevorzugt Wasserstoff, Methylsulfonyl oder Benzyl;
- R¹⁶: Wasserstoff oder C₁-C₆-Alkyl wie Methyl;
besonders bevorzugt Wasserstoff;
bedeuten.

Die 4-(3-Alkenyl-benzoyl)-pyrazole der Formel I sind auf verschiedene Art und Weise erhältlich, beispielsweise nach folgenden Verfahren:

### Verfahren A:

Umsetzung von Pyrazolen der Formel II mit R¹⁵ = H mit einer aktivierten Carbonsäure IIIα oder einer Carbonsäure IIIβ, die vorzugsweise in situ aktiviert wird, zu dem Acylierungsprodukt IV und anschließende Umlagerung.

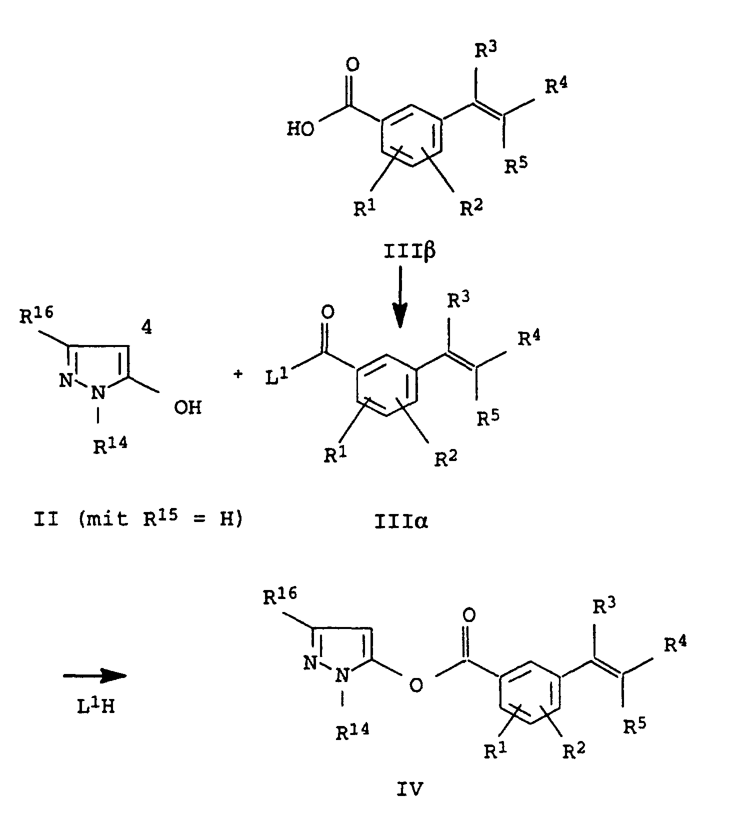

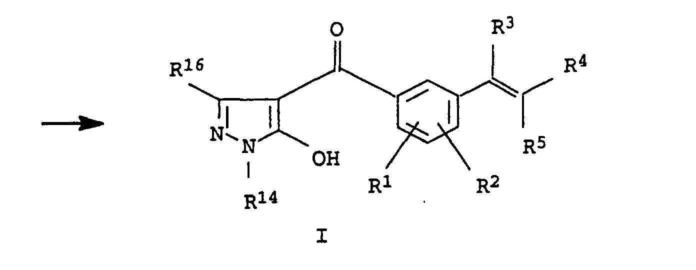

L¹ steht für eine nucleophil verdrängbare Abgangsgruppe, wie Halogen z.B. Brom, Chlor, Hetaryl, z.B. Imidazolyl, Pyridyl, Carboxylat, z.B. Acetat, Trifluoracetat etc.

Die aktivierte Carbonsäure kann direkt eingesetzt werden, wie im Fall der Carbonsäurehalogenide oder in situ erzeugt werden, z.B. mit Dicyclohexylcarbodiimid, Triphenylphosphin/Azodicarbonsäureester, 2-Pyridindisulfid/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Carbonsäurehalogenide als aktivierte Carbonsäurekomponente eingesetzt, so kann es zweckmäßig sein, bei Zugabe dieses Reaktionspartners die Reaktionsmischung auf 0-10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels wird der rohe Enolester der Formel IV vorzugsweise durch Chromatographie gereinigt. Es ist aber auch möglich, den rohen Enolester der Formel IV ohne weitere Reinigung zur Umlagerung einzusetzen.

Die Umlagerung der Enolester der Formel IV zu den Verbindungen der Formel I erfolgt zweckmäßigerweise bei Temperaturen von 20 bis 40°C in einem Lösungsmittel und in Gegenwart einer Hilfsbase sowie gegebenenfalls mit Hilfe einer Cyanoverbindung als Katalysator.

Als Lösungsmittel können z.B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonate verwendet.

Als Cyanoverbindungen kommen anorganische Cyanide, wie Natriumcyanid, Kaliumcyanid und organische Cyanoverbindungen, wie Acetoncyanhydrin, Trimethylsilylcyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise 10 Molprozent, bezogen auf den Ester, eingesetzt.

Besonders bevorzugt werden Alkalicarbonate, wie Kaliumcarbonat, in Acetonitril oder Dioxan eingesetzt.

Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid, Essigsäureethylester extrahiert. Der organische Extrakt kann mit 5-10%iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt. (Beispiele für die Darstellung von Estern von Hydroxypyrazolen und für die Umlagerung der Ester sind z.B. in EP-A 282 944 oder US 4 643 757 genannt).

### Verfahren B:

Umsetzung von 4-(3-Alkenyl-benzoyl)-pyrazolen der Formel I mit R¹⁵ = H mit einer Verbindung der Formel V (mit R¹⁵ ≠ H):

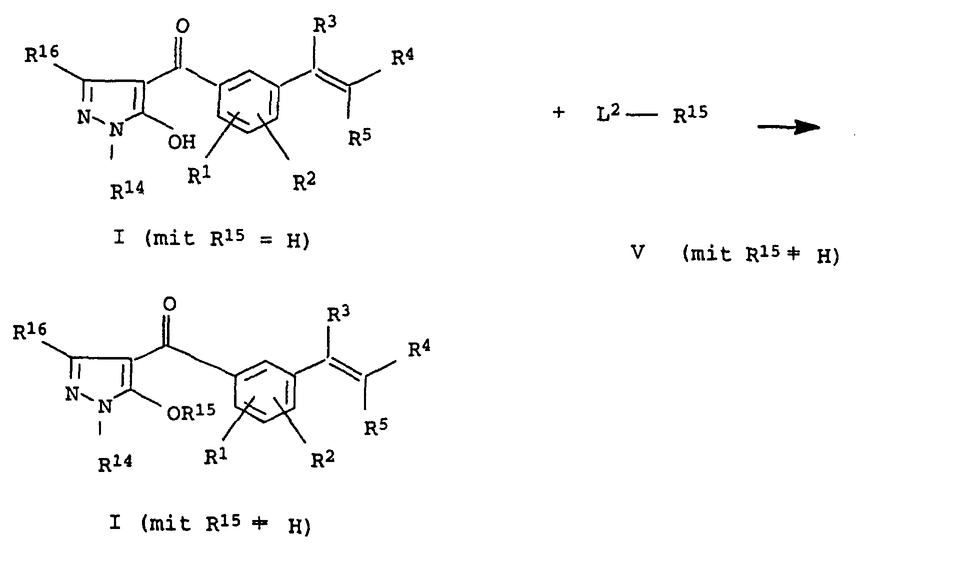

L² steht für eine nucleophil verdrängbare Abgangsgruppe, wie Halogen, z.B. Brom, Chlor, Hetaryl, z.B. Imidazolyl, Pyridyl, Carboxylat, z.B. Acetat, Trifluoracetat, Sulfonat, z.B. Mesylat, Triflat etc.

Die Verbindungen der Formel V können direkt eingesetzt werden, wie z.B. im Fall der Alkylhalogenide, Carbonsäurehalogenide, Sulfonsäurehalogenide, Carbonsäureanhydride und Sulfonsäureanhydride oder in situ erzeugt werden, z.B. aktivierte Carbonsäuren (mittels Carbonsäure und Dicyclohexylcarbodiimid, Carbonyldiimidazol etc.).

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuß der Hilfsbase z.B. 1,5 bis 3 Moläquivalente, bezogen auf II, kann unter Umständen vorteilhaft sein.

Als Hilfsbasen eignen sich tertiäre Alkylamine, wie Triethylamin, Pyridin, Alkalimetallcarbonate, z.B. Natriumcarbonat, Kaliumcarbonat und Alkalimetallhydride, z.B. Natriumhydrid. Bevorzugt verwendet werden Triethylamin, Pyridin oder Kaliumcarbonat.

Als Lösungsmittel kommen z.B. chlorierte Kohlenwasserstoffe, wie Methylenchlorid, 1,2-Dichlorethan, aromatische Kohlenwasserstoffe, z.B. Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0°C bis zur Höhe des Siedepunktes des Reaktionsgemisches.

Die Aufarbeitung kann in an sich bekannter Weise zum Produkt hin erfolgen.

Die als Ausgangsmaterialien verwendeten Pyrazole der Formel II (mit R¹⁵ = H) sind bekannt oder können nach an sich bekannten Verfahren hergestellt werden (z.B. EP-A 240 001, J. Prakt. Chem. 315, 383 (1973)).

Die aktivierten Carbonsäuren IIIα, die nicht in situ erzeugt werden, können auf an sich bekannte Art und Weise dargestellt werden. Beispielsweise können Carbonsäurehalogenide der Formel IIIα (mit L = Halogen) in Analogie zu literaturbekannten Methoden (vgl. L.G. Fieser, M. Fieser "Reagents for Organic Synthesis", Bd. I, S. 767 - 769 (1967)) durch Umsetzung von Benzoesäuren der Formel IIIβ mit Halogenierungsreagentien wie Thionylchlorid, Thionylbromid, Phosgen, Diphosgen, Triphosgen, Oxalylchlorid, Oxalylbromid synthetisiert werden.

Die 3-Alkenyl-benzoesäuren der Formel IIIβ sind literaturbekannt oder können in Analogie zu literaturbekannten Methoden, z.B. durch Verseifung der entsprechenden 3-Alkenyl-benzoesäureester der Formel IIIγ (mit M = C₁-C₆-Alkoxy) erhalten werden.

Die 3-Alkenyl-benzoesäureester (mit M = C₁-C₆-Alkoxy) der Formel IIIγ sind auf verschiedene Art und Weise erhältlich, beispielsweise nach folgenden Verfahren:

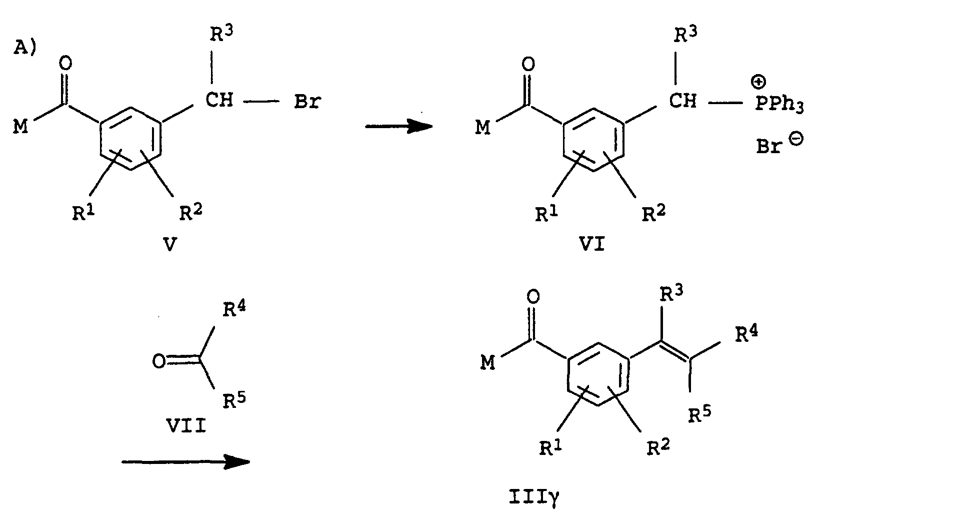

Durch Wittig-Reaktion von Phosphoniumsalzen der Formel VI mit Aldehyden oder Ketonen (VII) können auf an sich bekannte Art und Weise (J. March, "Advanced Organic Chemistry", 3. Auflage, S. 864 ff, Wiley-Interscience Publication, 1985) die Verbindungen der allgemeinen Formel IIIγ erhalten werden.

Die Phosphoniumsalze der Formel VI sind auf an sich bekannte Art und Weise (J. March, "Advanced Organic Chemistry", 3. Auflage, S. 377 ff, Wiley-Interscience Publication, 1985) aus den Bromverbindungen der Formel V zugänglich.

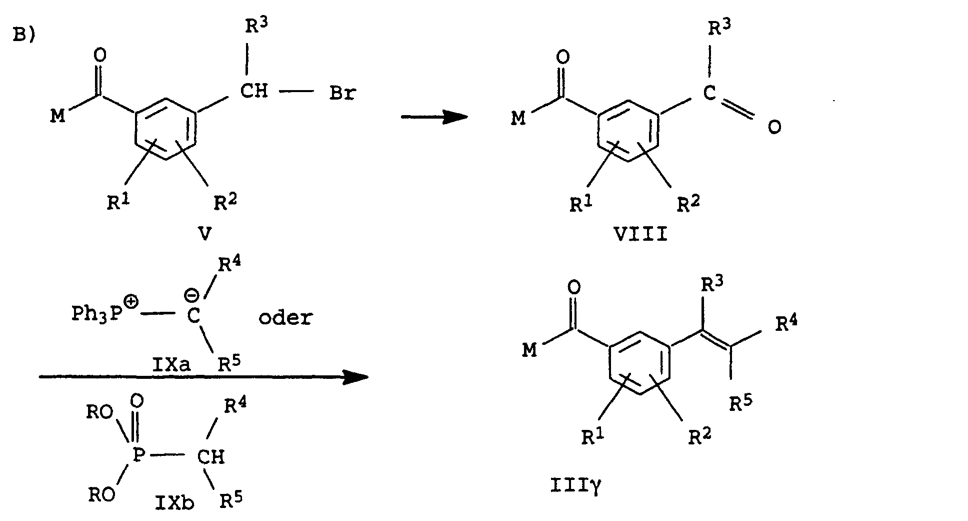

Die Verbindungen der allgemeinen Formel IIIγ können auch durch Wittig-Reaktion bzw. Horner-Emmons-Reaktion von Aldehyden oder Ketonen der Formel VIII mit Phosphoniumsalzen IXa (siehe A) bzw.

Phosphonaten IXb (J. March, "Advanced Organic Chemistry", 3. Auflage, S. 867 ff., Wiley-Interscience Publication, 1985) erhalten werden.

Die Verbindungen der Formel VIII sind auf an sich bekannte Art und Weise (J. March, "Advanced Organic Chemistry", 3. Auflage, S. 1105 ff, Wiley-Interscience Publication, 1985) durch Oxidation von Bromverbindungen der Formel V zugänglich.

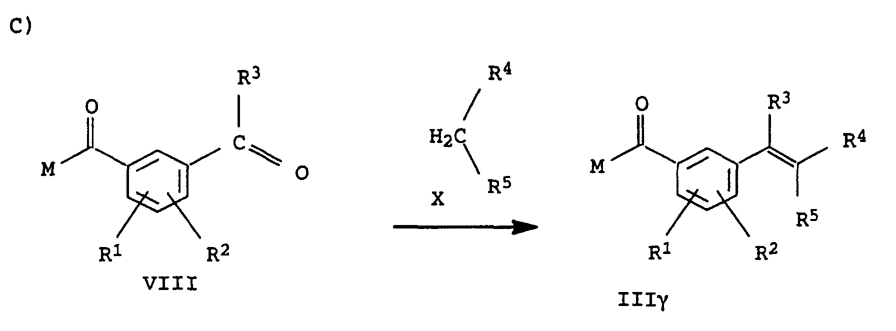

Durch Aldolkondensation und verwandten Reaktionen von Aldehyden oder Ketonen der Formel VIII können ebenfalls auf an sich bekannte Art und Weise (J. March, "Advanced Organic Chemistry", 3. Auflage, S. 849 ff, Wiley-Interscience Publications, 1985) die Verbindungen der allgemeinen Formel IIIγ erhalten werden.

### Herstellungsbeispiele

### 4-{2',4'-Dichlor-3'-[2"-(3"'-furyl)-ethen-1"-yl]benzoyl}-2-ethyl-3-hydroxypyrazol (Verbindung 2.1)

Zu einer Lösung von 3,0 g (10 mmol) 2,4-Dichlor-3-[2'-(3"-furyl)-ethen-1'-yl]benzoylchlorid in 30 ml Acetonitril wurden 1,2 g (10 mmol) 2-Ethyl-3-hydroxypyrazol und 1,5 ml (10 mmol) Triethylamin gegeben. Nach 12 Stunden Rühren bei Raumtemperatur wurde das Reaktionsgemisch in Wasser aufgenommen und dreimal mit Essigsäureethylester extrahiert. Nach Trocknen wurde das Lösungsmittel am Vakuum entfernt und der Rückstand an Kieselgel (Cyclohexan/Essigester = 8/2 bis 4/6) chromatographiert. Der so erhaltene Ester wurde in 100 ml Dioxan gelöst, mit 1,2 g (9 mmol) fein gepulvertem Kaliumcarbonat versetzt und 10 Stunden am Rückfluß erhitzt. Nach Abkühlen wurde der gebildete Niederschlag abgetrennt, mit Dioxan gewaschen und in Wasser eingerührt. Anschließend wurde mit 10 %iger Salzsäure ein pH-Wert von 1-2 eingestellt, 30 Minuten bei Raumtemperatur gerührt und der Niederschlag abgetrennt. Nach Trocknen verblieben 1,4 g (37 % d.Th.) eines weißen Feststoffes vom Fp. 115-120°C.
¹H-NMR(DMSO/δ in ppm): 7,75 (1H); 7,60 (1H); 7,50 (1H); 7,25 (2H); 7,00 (1H); 6,85 (1H); 6,80 (1H); 3,90 (2H); 1,30 (3H).

In der folgenden Tabelle 2 sind neben den voranstehend beschriebenen 4-(3-Alkenyl-benzoyl)-pyrazolen der Formel I noch weitere aufgeführt, die in analoger Weise hergestellt wurden:

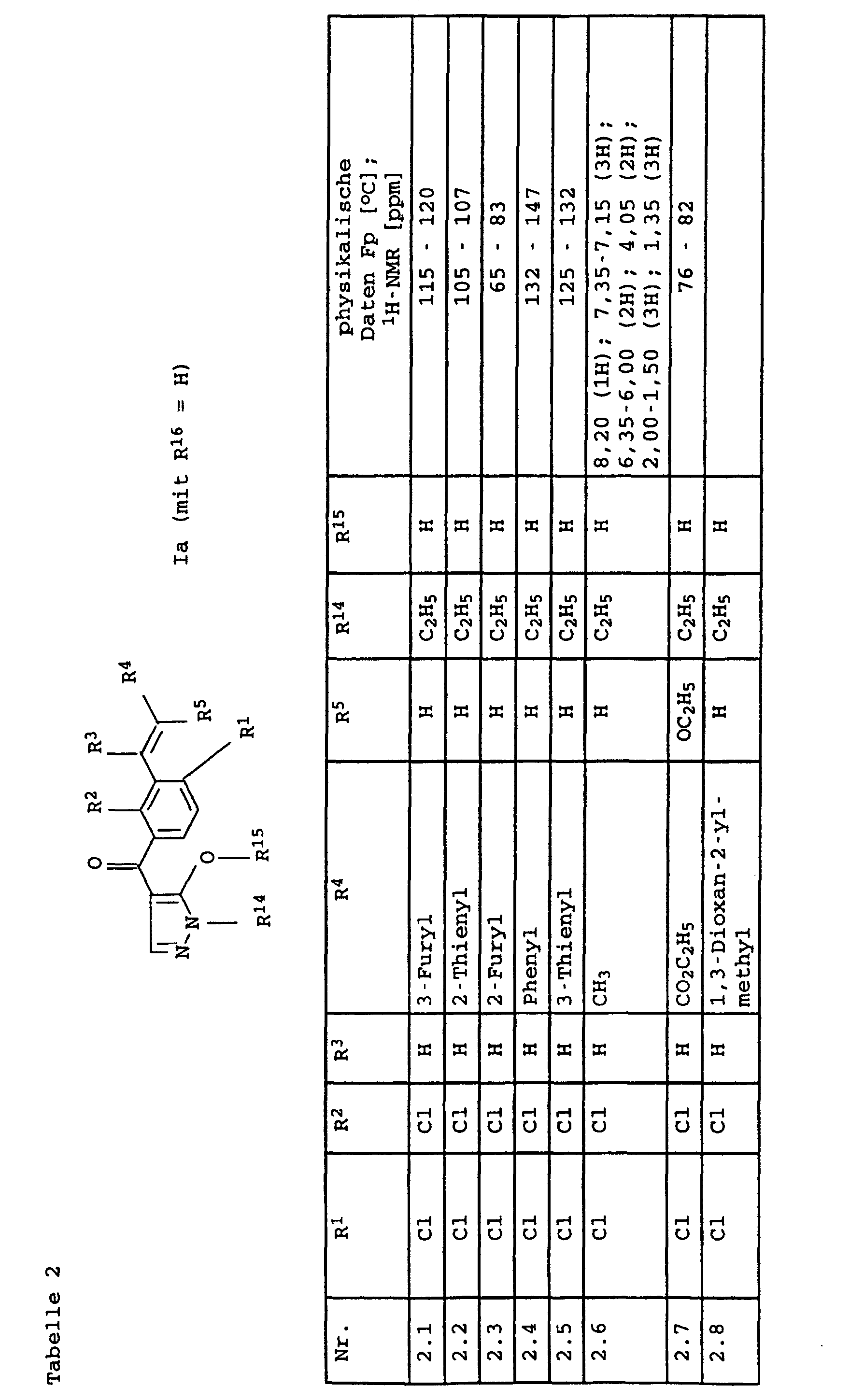

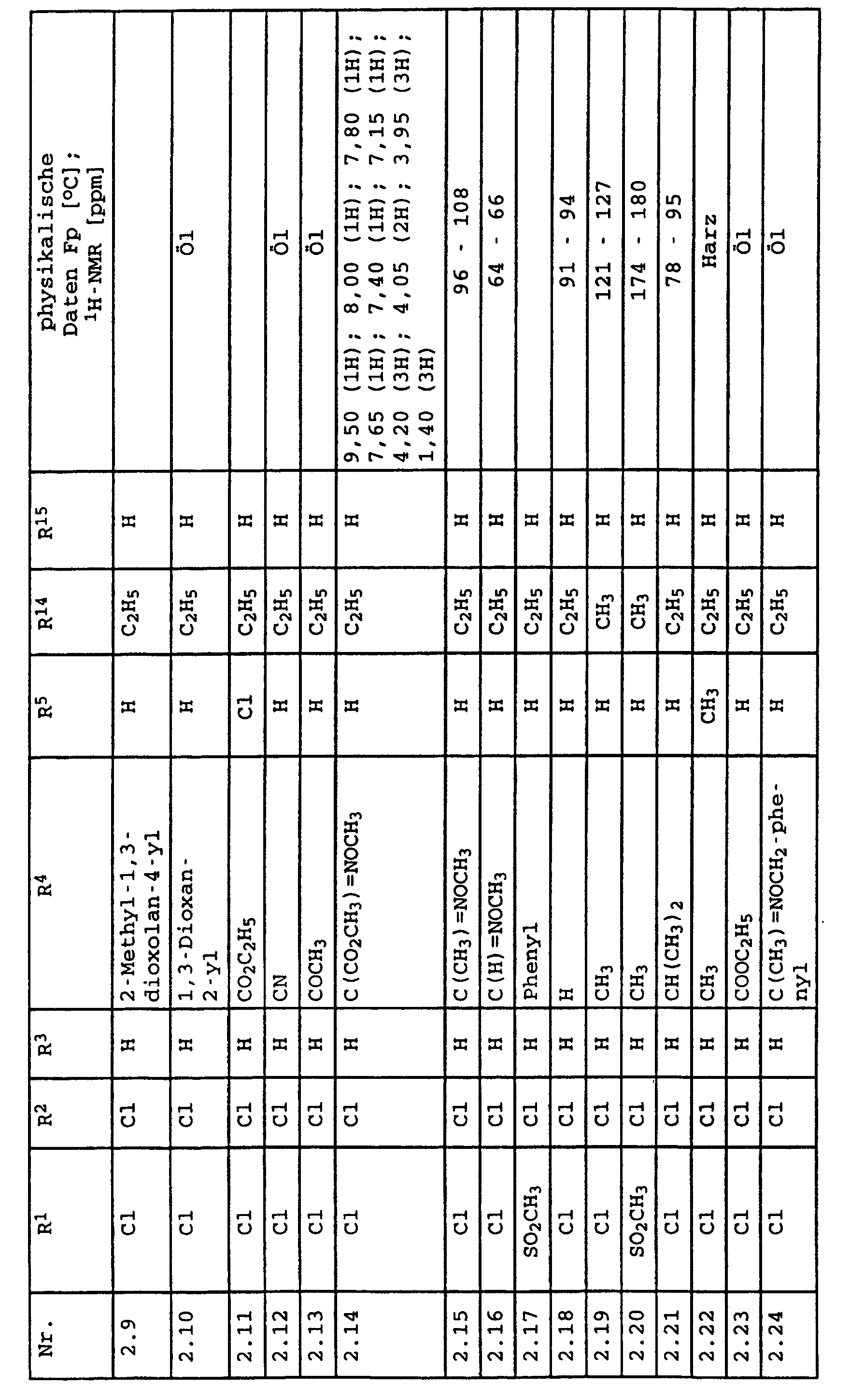

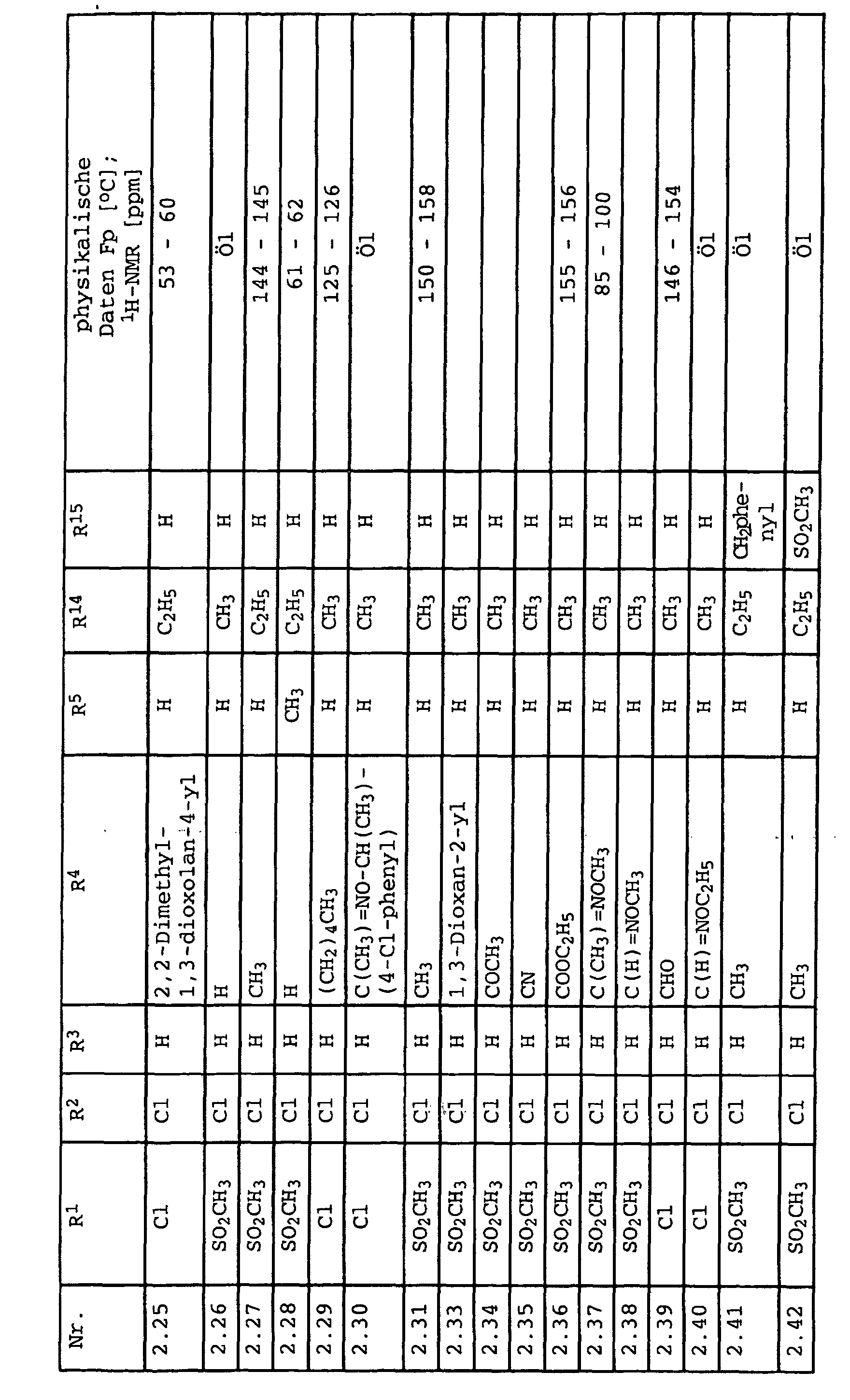

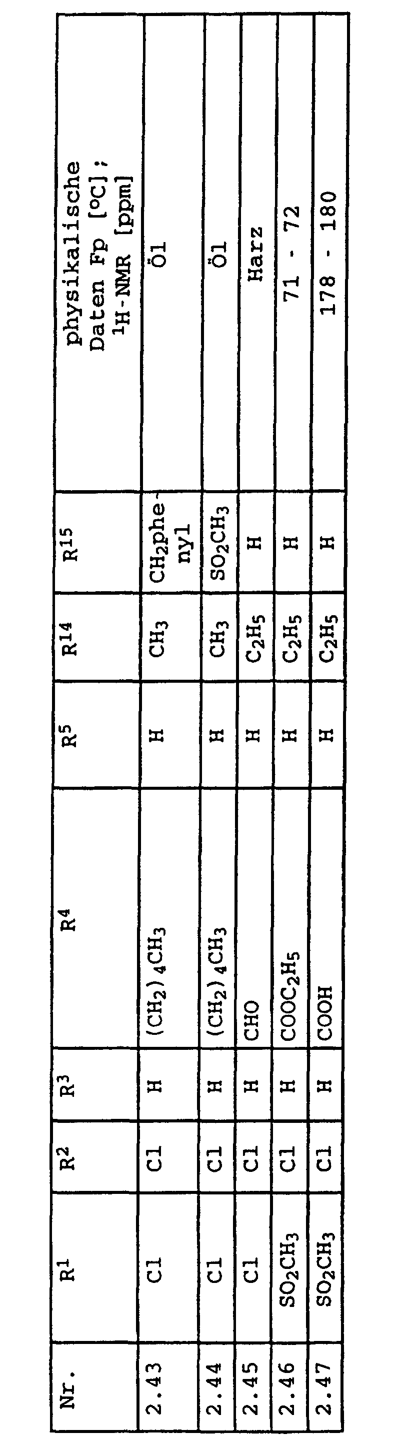

Nachfolgend sind die Synthesen einiger Ausgangsstoffe aufgeführt:

### 2-Chlor-4-methylsulfonyl-3-(2'-phenylethen-1'-yl)-benzoesäure (Verbindung 3.02)

Stufe a) 2-Chlor-4-methylsulfonyl-3-(2'-phenylethen-1'-yl)-benzoesäuremethylester (Verbindung 3.01)
   Zu einer Lösung von 52,0 g (120 mmol) Benzyltriphenylphosphoniumbromid in 400 ml Tetrahydrofuran wurden 10,1 g (90 mmol) Kalium-tert.-butylat gegeben. Nach 30 Minuten Rühren bei Raumtemperatur wurden 16,6 g (60 mmol) 2-Chlor-3-formyl-4-methylsulfonyl-benzoesäuremethylester in 100 ml Tetrahydrofuran zugetropft und weitere 3 Stunden bei Raumtemperatur gerührt. Anschließend wurde das Reaktionsgemisch in 500 ml Wasser eingerührt und mit Methyl-t-butylether extrahiert. Nach Entfernen des Lösungsmittels im Vakuum wurde der Rückstand mit Diethylether digeriert und das ausgefallene Triphenylphosphinoxid abgesaugt. Der Rückstand wurde mittels Chromatographie an Kieselgel (Cyclohexan/Essigester = 95/5 bis 1/1) gereinigt. Man erhielt 10,9 g (52 % d.Th.) eines leicht gelben Öls, das langsam erstarrte.
   ¹H-NMR (CDCl₃/δ in ppm): 8,15 (1H); 7,70 (1H); 7,60-7,30 (7H); 7,0 (1H); 4,0 (3H); 3,10 (3H).
Stufe b) 2-Chlor-4-methylsulfonyl-3-(2'-phenylethen-1'-yl)-benzoesäure
   10,0 g (28 mmol) 2-Chlor-4-methylsulfonyl-3-(2'-phenylethen-1'-yl)-benzoesäuremethylester wurden in 200 ml Tetrahydrofuran/Methanol (1/1) gelöst und mit 35,2 g 10 %iger Natronlauge versetzt. Danach wurde 12 Stunden bei Raumtemperatur gerührt und anschließend das Lösungsmittel im Vakuum entfernt. Der Rückstand wurde mit 400 ml Wasser versetzt und mit Essigsaureethylester gewaschen. Nun wurde mit 10 %iger Salzsäure ein pH-Wert von 1 eingestellt und der gebildete Niederschlag abgesaugt. Nach Trocknen verblieben 9,4 g (97 % d.Th.) eines weißen Pulvers vom Fp.: 232-233°C.
   ¹H-NMR (CDCl₃/δ in ppm): 8,20 (1H); 7,90 (1H); 7,55 (2H); 7,40 (4H); 7,00 (1H); 3,10 (3H).

### 2,4-Dichlor-3-[2'-(2"-furyl)ethen-1'-yl]-benzoesäuremethylester (Verbindung 3.05)

Stufe a) 2,4-Dichlor-3-methyl-acetophenon
   Zu einer Lösung von 502,0 g (3,12 mol) 2,6-Dichlortoluol und 408,0 g (3,06 mol) Aluminiumtrichlorid wurden bei 100°C unter Rühren 235,0 g (3,0 mol) Acetylchlorid über einen Zeitraum von 2 Stunden zugetropft. Nach 2 Stunden Rühren bei 100-105°C, kühlte man ab und goß das Reaktionsgemisch auf 3 l Eis und 1 l Wasser. Der dabei ausgefallene Feststoff wurde abgesaugt und mit Wasser neutral gewaschen. Nach dem Trocknen bei 40°C erhielt man 500,0 g 2,4-Dichlor-3-methyl-acetophenon als Rohprodukt, das anschließend im Hochvakuum destilliert wurde.
   (Sdp.: 121-128°C (4 mbar))
Stufe b) 2,4-Dichlor-3-methyl-benzoesäure
   In eine Lösung von 520,0 g (13 mol) Natriumhydroxid in 2600 ml Wasser wurden bei 0-10°C zunächst 655,2 g (4,1 mol) Brom und anschließend 203,0 g (1,0 mol) 2,4-Dichlor-3-methyl-acetophenon in 1300 ml 1,4-Dioxan getropft. Nach 12 Stunden Rühren, trennte man die organische Phase ab, versetzte die wäßrige Phase mit einer 30 %igen Lösung, dargestellt aus Natriumpyrosulfit und Wasser, und stellte mit Salzsäure einen pH-Wert von 1 ein. Der ausgefallene Niederschlag wurde abgesaugt, mit Wasser gewaschen und am Vakuum bei 60°C getrocknet. Man erhielt 197,0 g 2,4-Dichlor-3-methyl-benzoesäure.
   (Fp.: 173-175°C)
Stufe c) 2,4-Dichlor-3-methyl-benzoesäuremethylester
   In einer Lösung von 424,0 g (2 mol) 2,4-Dichlor-3-methylbenzoesäure und 1500 ml Methanol wurden 60 ml konz. Schwefelsäure getropft. Nach 5 Stunden Erhitzen unter Rückfluß wurde das Reaktionsgemisch abgekühlt, im Vakuum eingeengt und anschließend in 1000 ml Methylenchlorid aufgenommen. Die organische Phase wurde mit Wasser, anschließend mit 5 %iger Natriumhydrogencarbonat-Lösung und dann wiederum mit Wasser gewaschen, getrocknet und am Vakuum eingeengt. Man erhielt 401,0 g 2,4-Dichlor-3-methylbenzoesäuremethylester.
   (Sdp: 103-107°C (1-1,5 mbar))
Stufe d) 3-Brommethyl-2,4-dichlor-benzoesäuremethylester
   Zu einer Lösung von 84,0 g (0,38 mol) 2,4-Dichlor-3-methyl-benzoesäuremethylester und 67,6 g (0,38 mol) N-Bromsuccinimid in 380 ml Tetrachlorkohlenstoff wurde 1,0 g Azobisisobutyronitril gegeben. Nach 3,5 Stunden Erhitzen unter Rückfluß wurde das Reaktionsgemisch abgekühlt und der gebildete Niederschlag abgesaugt. Das Filtrat wurde am Vakuum eingeengt und der resultierende Rückstand aus Methyl-t-butylether ausgerührt. Man erhielt 108,0 g 3-Brommethyl-2,4-dichlor-benzoesäuremethylester.
   (Fp.: 51-54°C)
Stufe e) (2,6-Dichlor-3-methoxycarbonyl)-benzyl-triphenylphosphoniumbromid
   80,65 g (262 mmol) 3-Brommethyl-2,4-dichlor-benzoesäuremethylester wurden in 800 ml Toluol gelöst und mit 68,7 g (262 mmol) Triphenylphosphin versetzt. Nach 9 Stunden Rühren am Rückfluß kühlte man ab und saugte den gebildeten Niederschlag ab. Nach Trocknen verblieben 129,0 g (89 % d.Th.) eines hellbeigen Pulvers.
   (Fp.: 238-239°C)
Stufe f) 2,4-Dichlor-3-[2'-(2"-furyl)ethen-1'-yl]benzoesäuremethylester
   28,0 g (50 mmol) (2,6-Dichlor-3-methoxycarbonyl)-benzyltriphenylphosphoniumbromid wurden in 200 ml Tetrahydrofuran suspendiert und bei 0°C mit 5,6 g (50 mmol) Kalium-t-butylat versetzt. Danach wurde auf -20°C abgekühlt und eine Lösung von 6,2 g (65 mmol) Furfurol in 50 ml Tetrahydrofuran zugetropft. Man erwärmte auf Raumtemperatur und rührte weitere 12 Stunden. Das Reaktionsgemisch wurde in 200 ml Wasser eingerührt und mit Methyl-tert-butylether extrahiert. Nach Entfernen des Lösungsmittels im Vakuum wurde der Rückstand mit Diethylether digeriert und das ausgefallene Triphenylphosphinoxid abgetrennt. Der Rückstand wurde mittels Chromatographie an Kieselgel (Cyclohexan/Essigester = 98/2 bis 90/10) gereinigt. Man erhielt 8,2 g (55 % d.Th.) eines gelben Öls.
   ¹H-NMR (CDCl₃/δ in ppm): 7,55 (1H); 7,50 (1H); 7,40 (1H); 7,05 (1H); 6,95 (1H); 6,45 (2H); 3,95 (3H).

### 2,4-Dichlor-3-(3'-methoxycarbonyl-3'-methoxyimino-prop-1'-en-1'yl)benzoesäuremethylester (Verbindung 3.13)

Zu 17,9 g (75 mmol) (2-Methoxycarbonyl-2-methoxyimino-ethyl)dimethylphosphonat in 150 ml Tetrahydrofuran wurden 1,9 g (75 mmol) Natriumhydrid gegeben und 2 Stunden bei Raumtemperatur gerührt. Anschließend werden 11,7 g (50 mmol) 2,4-Dichlor-3-formylbenzoesäuremethylester in 50 ml Tetrahydrofuran zugetropft und weitere 12 Stunden bei Raumtemperatur gerührt. Nach Aufnehmen des Reaktionsansatzes in Wasser wurde mit Methyl-t-butylether extrahiert, getrocknet und das Lösungsmittel im Vakuum entfernt. Der Rückstand wurde mit Diethylether digeriert und der Niederschlag abgetrennt. Nach Trocknen verblieben 11,3 g (65 % d.Th.) eines weißen Pulvers.
(Fp.: 96 - 97°C)

In nachfolgender Tabelle 3 sind neben den voranstehend beschriebenen Verbindungen weitere 3-Alkenylbenzoesäurederivate der Formel IIIa aufgeführt, die in analoger Weise hergestellt wurden oder herstellbar sind.

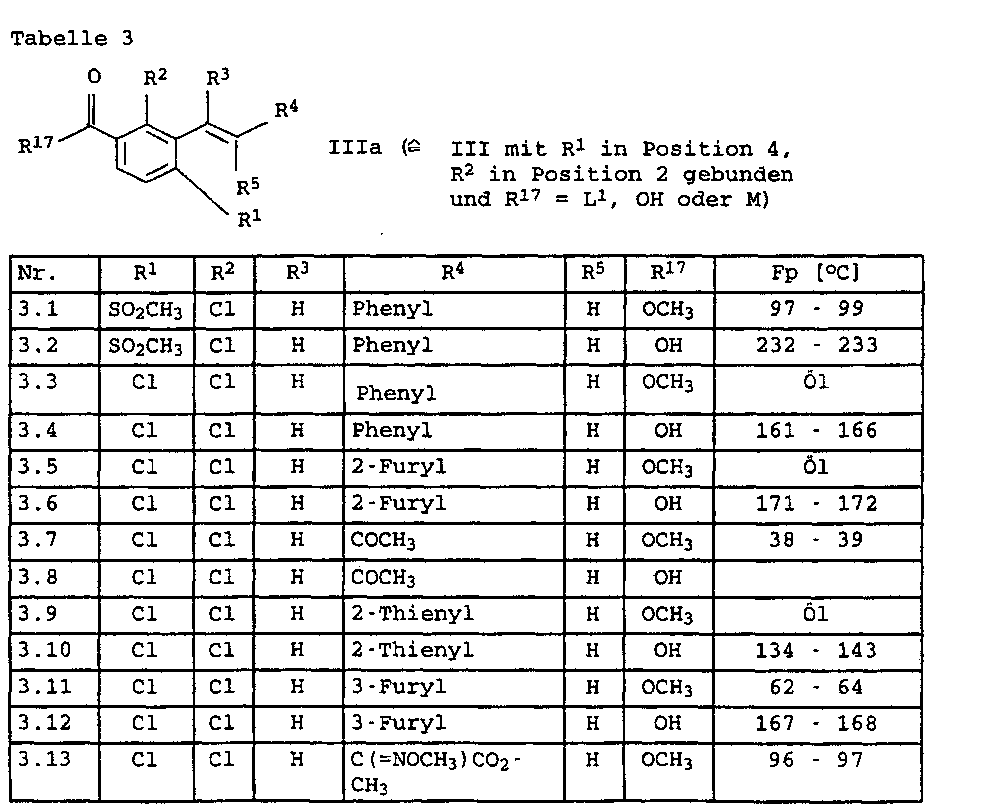

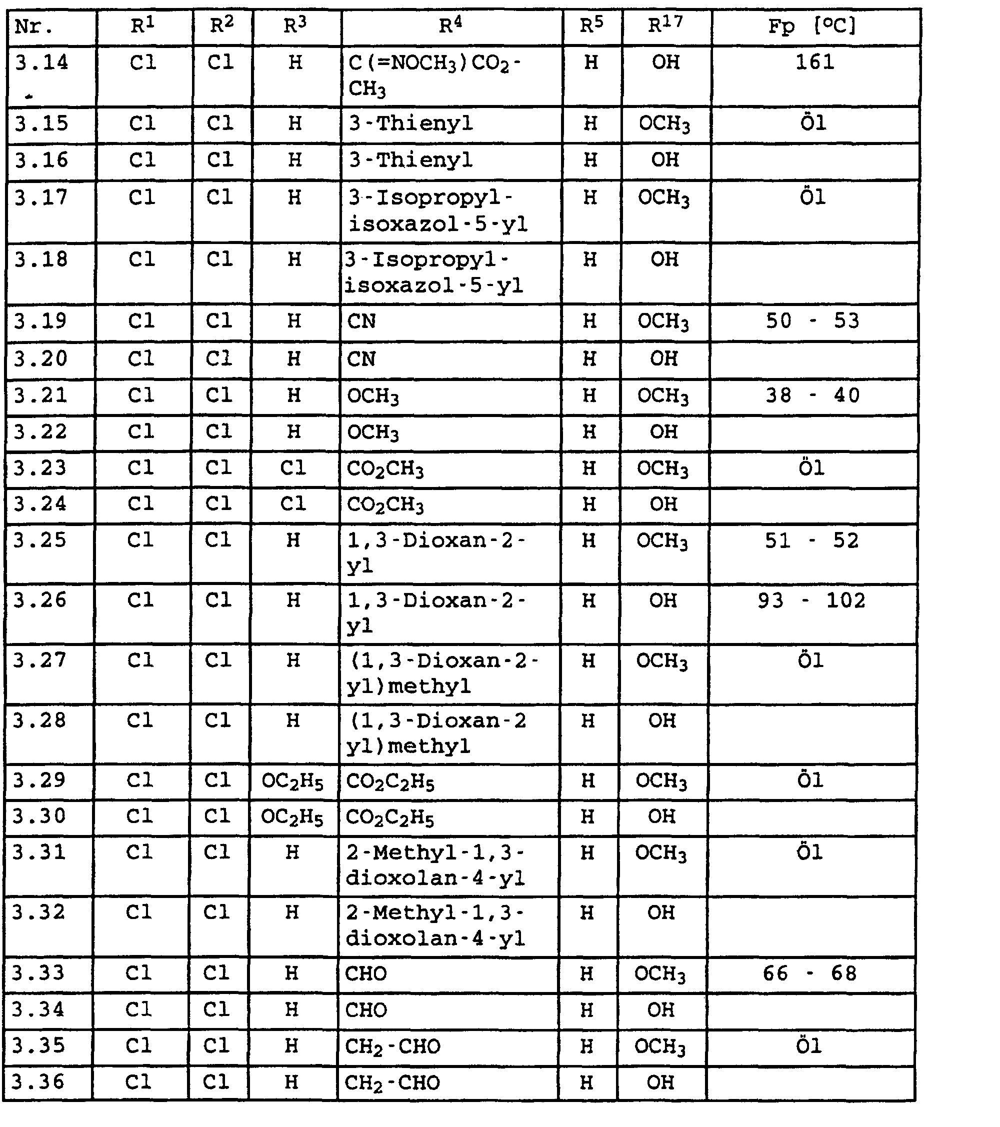

Die 4-(3-Alkenyl-benzoyl)-pyrazole der Formel I und deren landwirtschaftlich brauchbaren Salze eignen sich - sowohl als Stereoisomerengemische als auch in Form der reinen Stereoisomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die 4-(3-Alkenyl-benzoyl)-pyrazole der Formel I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlichen brauchbaren Salzes von I und für die Formulierung von Pflanzenschutzmitteln üblichen Hilfsmittel.

Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z.B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die 4-(3-Alkenyl-benzoyl)-pyrazole als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I. 20 Gewichtsteile der Verbindung Nr. 2.1 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.
II. 20 Gewichtsteile der Verbindung Nr. 2.2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
III. 20 Gewichtsteile des Wirkstoffs Nr. 2.3 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
IV. 20 Gewichtsteile des Wirkstoffs Nr. 2.4 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
V. 3 Gewichtsteile des Wirkstoffs Nr. 2.5 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 2.1 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil der Verbindung Nr. 2.2 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil der Verbindung Nr. 2.4 wird in einer Mischung gelöst, die aus 80 Gewichtsteilen Cyclohexanon und 20 Gewichtsteilen Wettol® EM 31 (nicht ionischer Emulgator auf der Basis von ethoxyliertem Ricinusöl) besteht. Man erhält ein stabiles Emulsionskonzentrat.

Die Applikation der Wirkstoffe der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Zur Verbreiterung des Wirkungsspektrums und zur Erzielung synergistischer Effekte können die Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy/Hetaryloxyalkansäure und deren Derivate, Benzoesäure und deren Derivate, Benzothiadiazinone, 2-(Aroyl/Hetaroyl)-1,3-cyclohexandione, Hetaryl-Aryl-Ketone, Benzylisoxazolidinone, Meta-CF₃-phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a. S.)

### Anwendungsbeispiele

Die herbizide Wirkung der 4-(3-Alkenyl-benzoyl)-pyrazole der Formel I ließ sich durch Gewächshausversuche zeigen:

Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,5 bzw. 0,25 kg/ha a. S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Englischer Name | Deutscher Name |
|---|---|---|
| Chenopodium album | lambsquarters (goosefoot) | Weißer Gänsefuß |
| Sinapis alba | white mustard | Weißer Senf |
| Solanum nigrum | black nightshade | Schwarzer Nachtschatten |
| Triticum aestivum | summer wheat | Sommerweizen |

Oben genannte Unkräuter werden von Verbindung 2.4 im Nachauflauf bei Aufwandmengen von 0,5 bzw. 0,25 kg/ha a. S. sehr gut bekämpft - die Kulturpflanze Sommerweizen jedoch nicht geschädigt.

## Patentansprüche

1. 4-(3-Alkenyl-benzoyl)-pyrazole der Formel I 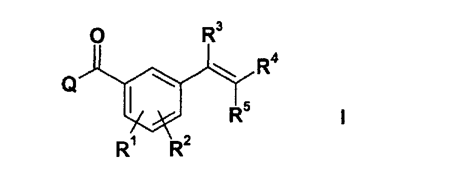 in der die Variablen folgende Bedeutung haben:
R¹, R² Wasserstoff, Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁶, -OCOR⁷, -OSO₂R⁷, -SH, -S(O)ₙR⁸,-SO₂OR⁶, -SO₂NR⁶R⁹, -NR⁹SO₂R⁷ oder -NR⁹COR⁷;
R³ Wasserstoff, Halogen, C₁-C₆-Alkyl, C₁₋C₆-Halogen-alkyl, C₁-C₆-Alkoxy, C₂-C₆-Alkenyl oder C₂-C₆-Alkinyl;
R⁴ Wasserstoff, Nitro, Halogen, Cyano, Rhodano, C₁-C₆-Alkyl, C₁-C₆-Halogenalkyl, C₃-C₆-Cycloalkyl, C₂-C₆-Alkenyl, C₄-C₆-Cycloalkenyl, C₂-C₆-Alkinyl, -COR¹⁰, -CO₂R¹⁰, -COSR¹⁰, -CONR¹⁰R¹¹, -C(R¹²)=NR¹³, -PO(OR¹⁰)(OR¹¹), C₁-C₄-Alkyl, das einen Rest aus folgender Gruppe trägt: -COR¹⁰, -CO₂R¹⁰, -COSR¹⁰, -CONR¹⁰R¹¹ oder -C(R¹²)=NR¹³; Heterocyclyl, Heterocyclyl-C₁-C₄-alkyl (wobei der Heterocyclyl-Rest der beiden letztgenannten Reste drei- bis siebengliedrig, gesättigt oder partiell ungesättigt, mono- oder polycyclisch ist, und ein bis drei Hetero-atome, ausgewählt aus der Gruppe bestehend aus Sauerstoff, Stickstoff und Schwefel, enthält), Phenyl, Phenyl-C₁-C₄-alkyl, Hetaryl oder Hetaryl-C₁-C₄-alkyl (wobei der Hetaryl-Rest der beiden letztgenannten Reste aromatisch, mono- oder polycyclisch ist und neben Kohlenstoffringgliedern zusätzlich ein bis 4 Stickstoffatome oder ein bis drei Stickstoffatome und ein Sauerstoff- oder ein Schwefelatom oder ein Sauerstoff- oder ein Schwefelatom enthalten kann), wobei die sechs letztgenannten Reste substituiert sein können;
R⁵ Wasserstoff, Halogen, Cyano, Rhodano, C₁-C₆-Alkyl, C₁-C₆-Halogenalkyl, -COR¹⁰, -CO₂R¹⁰, -COSR¹⁰, - CONR¹⁰R¹¹, oder -PO(OR¹⁰)(OR¹¹);
n 0, 1 oder 2;
R⁶ Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
R⁷ C₁-C₆-Alkyl oder C₁-C₆-Halogenalkyl;
R⁸ C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
R⁹ Wasserstoff oder C₁-C₆-Alkyl;
R¹⁰ Wasserstoff, C₁-C₆-Alkyl, C₃-C₆-Cycloalkyl, C₁-C₆-Halogenalkyl, C₃-C₆-Alkenyl, C₃-C₆-Alkinyl, Phenyl oder Benzyl, wobei die beiden letztgenannten Reste partiell oder vollständig halogeniert sein können und/oder einen bis drei Reste aus der folgenden Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl oder C₁-C₄-Alkoxycarbonyl;
R¹¹ Wasserstoff, C₁-C₆-Alkyl, C₃-C₆-Alkenyl oder C₃-C₆-Alkinyl;
oder
R¹⁰ und R¹¹ bilden gemeinsam eine C₂-C₆-Alkandiylkette, die ein- bis vierfach durch C₁-C₄-Alkyl substituiert sein kann und/oder durch Sauerstoff oder Schwefel oder einen gegebenenfalls C₁-C₄-Alkyl substituierten Stickstoff unterbrochen sein kann;
R¹² Wasserstoff, C₁-C₆-Alkyl, C₁-C₆-Halogenalkyl, C₁-C₆-Alkoxy, C₁-C₆-Alkoxycarbonyl, C₃-C₆-Cycloalkyl, C₂-C₆-Alkenyl, C₂-C₆-Alkinyl, Phenyl oder Benzyl, wobei die beiden letztgenannten Reste partiell oder vollständig halogeniert sein können und/oder einen bis drei Reste aus der folgenden Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl oder C₁-C₄-Alkoxycarbonyl;
R¹³ C₁-C₆-Alkyl, C₁-C₆-Halogenalkyl, C₃-C₆-Cycloalkyl, C₃-C₆-Alkenyl, C₃-C₆-Alkinyl, C₁-C₆-Alkoxy, C₁-C₆-Halogenalkoxy, C₃-C₆-Cycloalkoxy, C₃-C₆-Alkenyloxy, C₃-C₆-Alkinyloxy, Phenyl, Benzyl oder Phenyl-C₁-C₄-alkoxy, wobei die drei letztgenannten Reste partiell oder vollständig halogeniert sein können und/oder einen bis drei Reste aus der folgenden Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl oder C₁-C₄-Alkoxycarbonyl;
Q ein in 4-Stellung verknüpftes Pyrazol der Formel II 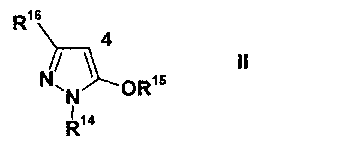 wobei
R¹⁴ für C₁-C₆-Alkyl, C₁-C₆-Halogenalkyl, Phenyl oder Phenyl das partiell oder vollständig halogeniert ist und/oder einen bis drei der folgenden Reste trägt:
Nitro, Cyano, C₁-C₄-Alkyl, C₁-C₄-Halogenalkyl, C₁-C₄-Alkoxy, C₁-C₄-Halogenalkoxy;
R¹⁵ für Wasserstoff, C₁-C₆-Alkyl, C₁-C₆-Halogenalkyl, C₁-C₆-Alkylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, Phenyl-C₁-C₄-alkyl, Phenylcarbonyl, Phenylcarbonylmethyl, Phenoxycarbonyl oder Phenylsulfonyl, wobei die fünf letztgenannten Substituenten unsubstituiert sind oder der Phenylring jeweils partiell oder vollständig halogeniert ist und/oder einen bis drei der folgenden Reste trägt:
Nitro, Cyano, C₁-C₄-Alkyl, C₁-C₄-Halogenalkyl, C₁-C₄-Alkoxy, C₁-C₄-Halogenalkoxy;
R¹⁶ für Wasserstoff, C₁-C₆-Alkyl oder C₁-C₆-Halogenalkyl;
stehen;
sowie deren landwirtschaftlich brauchbaren Salze.

2. 2-(3-Alkenyl-benzoyl)-pyrazole der Formel I nach Anspruch 1, in der
R¹³ C₁-C₆-Alkyl, C₁-C₆-Halogenalkyl, C₃-C₆-Cycloalkyl, C₃-C₆-Alkenyl, C₃-C₆-Alkinyl, C₁-C₆-Alkoxy, C₁-C₆-Halogenalkoxy, C₃-C₆-Cycloalkoxy, C₃-C₆-Alkenyloxy, C₃-C₆-Alkinyloxy, Phenyl, Benzyl oder Benzyloxy, wobei die drei letztgenannten Reste partiell oder vollständig halogeniert sein können und/oder einen bis drei Reste aus folgender Gruppe tragen können: Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl oder C₁-C₄-Alkoxycarbonyl;
R¹⁵ Wasserstoff, C₁-C₆-Alkyl, C₁-C₆-Halogenalkyl, C₁-C₆-Alkylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, Phenylcarbonyl, Phenylcarbonylmethyl, Phenoxycarbonyl oder Phenylsulfonyl, wobei die vier letztgenannten Substituenten unsubstituiert sind oder der Phenylring jeweils partiell oder vollständig halogeniert ist und/oder einen bis drei der folgenden Reste trägt:
Nitro, Cyano, C₁-C₄-Alkyl, C₁-C₄-Halogenalkyl, C₁-C₄-Alkoxy, C₁-C₄-Halogenalkoxy;
bedeutet.

3. 4-(3-Alkenyl-benzoyl)-pyrazole der Formel I nach den Ansprüchen 1 oder 2, in der
R¹ Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁶ oder -S(O)ₙR⁸ bedeutet;
R² für Wasserstoff oder einen wie voranstehend unter R¹ genannten Rest steht.

4. 4-(3-Alkenyl-benzoyl)-pyrazole der Formel Ia 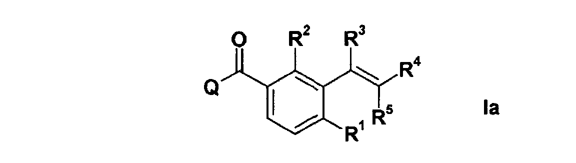 in der die Variablen R¹ bis R⁵ und Q die unter den Ansprüchen 1 bis 3 genannte Bedeutung haben.

5. Verfahren zur Herstellung von 4-(3-Alkenyl-benzoyl)-pyrazolen der Formel I gemäß den Ansprüchen 1 bis 4, **dadurch gekennzeichnet, daß** man ein Pyrazol der Formel II (mit R¹⁵ = H), in der die Variablen R¹⁴ und R¹⁶ die unter Anspruch 1 genannte Bedeutung haben, 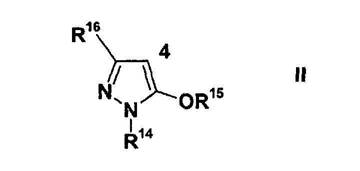 mit einer aktivierten Carbonsäure IIIα oder mit einer Carbonsäure IIIß, 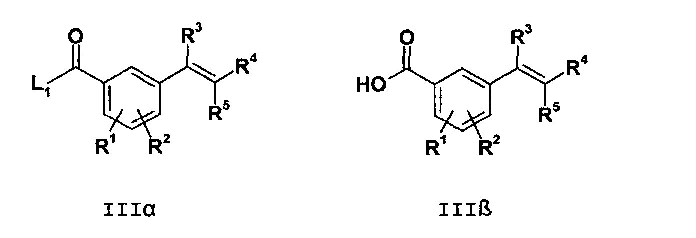 wobei die Variablen R¹ bis R⁵ die unter Anspruchl genannten Bedeutungen haben und L¹ für eine nucleophil verdrängbare Abgangsgruppe steht, acyliert und das Acylierungsprodukt gegebenenfalls in Gegenwart eines Katalysators zu den Verbindungen I (mit R¹⁵ = H) umlagert und gewünschtenfalls zur Herstellung von 4-(3-Alkenyl-benzoyl)-pyrazolen der Formel I mit R¹⁵ ≠ H mit einer Verbindung der Formel V,
L²-R¹⁵ V
(mit R¹⁵ ≠ H) in der R¹⁵ abgesehen von Wasserstoff die unter Anspruch 1 genannte Bedeutung hat und L² für eine nucleophil verdrängbare Abgangsgruppe steht, umsetzt.

6. Mittel, enthaltend eine herbizid wirksame Menge mindestens eines 4-(3-Alkenyl-benzoyl)-pyrazols der Formel I oder eines landwirtschaftlich brauchbaren Salzes von I gemäß den Ansprüchen 1 bis 4 und für die Formulierung von Pflanzenschutzmitteln übliche Hilfsmittel.

7. Verfahren zur Herstellung von herbizid wirksamen Mitteln gemäß Anspruch 6, **dadurch gekennzeichnet, daß** man eine herbizid wirksame Menge mindestens eines 4-(3-Alkenylbenzoyl)-pyrazols der Formel I oder eines landwirtschaftlich brauchbaren Salzes von I gemäß den Ansprüchen 1 bis 4 und für die Formulierung von Pflanzenschutzmitteln übliche Hilfsmittel mischt.

8. Verfahren zur Bekämpfung von unerwünschtem Pflanzenwuchs, **dadurch gekennzeichnet, daß** man eine herbizid wirksame Menge mindestens eines 4-(3-Alkenyl-benzoyl)-pyrazols der Formel I oder eines landwirtschaftlich brauchbaren Salzes von I gemäß den Ansprüchen 1 bis 5 auf Pflanzen, deren Lebensraum und/oder auf Samen einwirken läßt.

9. Verwendung der 4-(3-Alkenyl-benzoyl)-pyrazole der Formel I und deren landwirtschaftlich brauchbaren Salze gemäß den Ansprüchen 1 bis 4 als Herbizide.

## Claims

1. 4-(3-Alkenylbenzoyl)pyrazoles of the formula I 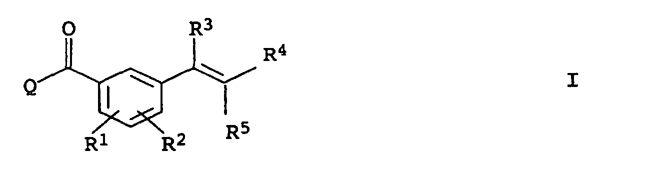 where:
R¹ and R² are each hydrogen, nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl, -OR⁶, -OCOR⁷, -OSO₂R⁷, -SH, -S(O)ₙR⁸, -SO₂OR⁶, -SO₂NR⁶R⁹, -NR⁹SO₂R⁷ or -NR⁹COR⁷;
R³ is hydrogen, halogen, C₁-C₆-alkyl, C₁-C₆-haloalkyl, C₁-C₆-alkoxy, C₂-C₆-alkenyl or C₂-C₆-alkynyl;
R⁴ is hydrogen, nitro, halogen, cyano, thiocyanato, C₁-C₆-alkyl, C₁-C₆-haloalkyl, C₃-C₆-cycloalkyl, C₂-C₆-alkenyl, C₄-C₆-cycloalkenyl, C₂-C₆-alkynyl, -COR¹⁰, -CO₂R¹⁰, -COSR¹⁰, -CONR¹⁰R¹¹, -C(R¹²)=NR¹³, -PO(OR¹⁰)(OR¹¹), C₁-C₄-alkyl which carries a radical from the following group: -COR¹⁰, -CO₂R¹⁰, -COSR¹⁰, -CONR¹⁰R¹¹ or -C(R¹²)=NR¹³; heterocyclyl, heterocyclyl-C₁-C₄-alkyl (wherein the heterocyclyl radical of the two last-mentioned radicals is 3 to 7 membered, saturated or partially unsaturated, mono or polycyclic, and comprises one to three heteroatoms selected from the group consisting of oxygen, nitrogen and sulfur), phenyl, phenyl-C₁-C₄-alkyl, hetaryl or hetaryl-C₁-C₄-alkyl (wherein the hetaryl radical of the two last-mentioned radicals is aromatic, mono or polycyclic and may comprise in addition to carbon ring members one to four nitrogen atoms or one to three nitrogen atoms and one oxygen or one sulfur atom or one oxygen or one sulfur atom), it being possible for the last six radicals to be substituted;
or
R⁵ is hydrogen, halogen, cyano, thiocyanato, C₁-C₆alkyl, C₁-C₆-haloalkyl, -COR¹⁰, -CO₂R¹⁰, -COSR¹⁰, -CONR¹⁰R¹¹, or -PO(OR¹⁰)(OR¹¹);
n is 0, 1 or 2;
R⁶ is hydrogen, C₁-C₆-alkyl, C₁-C₆-haloalkyl, C₁-C₆-alkoxy-C₂-C₆-alkyl, C₃-C₆-alkenyl or C₃-C₆-alkynyl;
R⁷ is C₁-C₆-alkyl or C₁-C₆-haloalkyl;
R⁸ is C₁-C₆-alkyl, C₁-C₆-haloalkyl, C₁-C₆-alkoxy-C₂-C₆-alkyl, C₃-C₆-alkenyl or C₃-C₆-alkynyl;
R⁹ is hydrogen or C₁-C₆-alkyl;
R¹⁰ is hydrogen, C₁-C₆-alkyl, C₃-C₆-cycloalkyl, C₁-C₆-haloalkyl, C₃-C₆-alkenyl, C₃-C₆-alkynyl, phenyl or benzyl, it being possible for the last two radicals to be partially or fully halogenated and/or to carry one to three radicals from the following group:
nitro, cyano, C₁-C₄-alkyl, C₁-C₄-haloalkyl, C₁-C₄-alkoxy, C₁-C₄-haloalkoxy, C₁-C₄-alkylcarbonyl or C₁-C₄-alkoxycarbonyl;
R¹¹ is hydrogen, C₁-C₆-alkyl, C₃-C₆-alkenyl or C₃-C₆-alkynyl;
or
R¹⁰ and R¹¹ together form a C₂-C₆-alkanediyl chain which may be mono- to tetrasubstituted by C₁-C₄-alkyl and/or interrupted by oxygen or sulfur or by nitrogen with or without substitution by C₁-C₄-alkyl;
R¹² is hydrogen, C₁-C₆-alkyl, C₁-C₆-haloalkyl, C₁-C₆-alkoxy, C₁-C₆-alkoxycarbonyl, C₃-C₆-cycloalkyl, C₂-C₆-alkenyl, C₂-C₆-alkynyl, phenyl or benzyl, it being possible for the last two radicals to be partially or fully halogenated and/or to carry one to three radicals of the following group:
nitro, cyano, C₁-C₄-alkyl, C₁-C₄-haloalkyl, C₁-C₄-alkoxy, C₁-C₄-haloalkoxy, C₁-C₄-alkylcarbonyl or C₁-C₄-alkoxycarbonyl;
R¹³ is C₁-C₆-alkyl, C₁-C₆-haloalkyl, C₃-C₆-cycloalkyl, C₃-C₆-alkenyl, C₃-C₆-alkynyl, C₁-C₆-alkoxy, C₁-C₆-haloalkoxy, C₃-C₆-cycloalkoxy, C₃-C₆-alkenyloxy, C₃-C₆-alkynyloxy, phenyl, benzyl or phenyl-C₁-C₄-alkoxy, it being possible for the last three radicals to be partially or fully halogenated and/or to carry one to three radicals from the following group:
nitro, cyano, C₁-C₄-alkyl, C₁-C₄-haloalkyl, C₁-C₄-alkoxy, C₁-C₄-haloalkoxy, C₁-C₄-alkylcarbonyl or C₁-C₄-alkoxycarbonyl;
Q is a pyrazole of the formula II 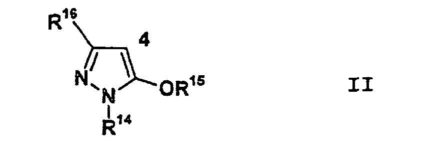 which is linked in position 4 and where
R¹⁴ is C₁-C₆-alkyl, C₁-C₆-haloalkyl, phenyl or phenyl which is partially or fully halogenated and/or carries one to three of the following radicals:
nitro, cyano, C₁-C₄-alkyl, C₁-C₄-haloalkyl, C₁-C₄-alkoxy, C₁-C₄-haloalkoxy;
R¹⁵ is hydrogen, C₁-C₆-alkyl, C₁-C₆-haloalkyl, C₁-C₆-alkylcarbonyl, C₁-C₆-haloalkylcarbonyl, C₁-C₆-alkoxycarbonyl, C₁-C₆-alkylsulfonyl, C₁-C₆-haloalkylsulfonyl, phenyl-C₁-C₄-alkyl, phenylcarbonyl, phenylcarbonylmethyl, phenoxycarbonyl or phenylsulfonyl, the last five substituents being unsubstituted or the phenyl ring in question being partially or fully halogenated and/or carrying one to three of the following radicals:
nitro, cyano, C₁-C₄-alkyl, C₁-C₄-haloalkyl, C₁-C₄-alkoxy, C₁-C₄-haloalkoxy;
R¹⁶ is hydrogen, C₁-C₆-alkyl or C₁-C₆-haloalkyl;
and agriculturally useful salts thereof.

2.4-(3-Alkenylbenzoyl)pyrazoles of formula I as claimed in claim 1 where
R¹³ is C₁-C₆-alkyl, C₁-C₆-haloalkyl, C₃-C₆-cycloalkyl, C₃-C₆-alkenyl, C₃-C₆-alkynyl, C₁-C₆-alkoxy, C₁-C₆-haloalkoxy, C₃-C₆-cycloalkoxy, C₃-C₆-alkenyloxy, C₃-C₆-alkynyloxy, phenyl, benzyl or benzyloxy, it being possible for the last three radicals to be partially or fully halogenated and/or to carry one to three radicals from the following group:
nitro, cyano, C₁-C₄-alkyl, C₁-C₄-haloalkyl, C₁-C₄-alkoxy, C₁-C₄-haloalkoxy, C₁-C₄-alkylcarbonyl or C₁-C₄-alkoxycarbonyl;
R¹⁵ is hydrogen, C₁-C₆-alkyl, C₁-C₆-haloalkyl, C₁-C₆-alkylcarbonyl, C₁-C₆-haloalkylcarbonyl, C₁-C₆-alkoxycarbonyl, C₁-C₆-alkylsulfonyl, C₁-C₆-haloalkylsulfonyl, phenylcarbonyl, phenylcarbonylmethyl, phenoxycarbonyl or phenylsulfonyl, the last four substituents being unsubstituted or the phenyl ring in question being partially or fully halogenated and/or carrying one to three of the following radicals:
nitro, cyano, C₁-C₄-alkyl, C₁-C₄-haloalkyl, C₁-C₄-alkoxy, C₁-C₄-haloalkoxy.

3. 4-(3-Alkenylbenzoyl)pyrazoles of the formula I as claimed in claim 1 where
R¹ is nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl, -OR⁶ or -S(O)ₙR⁸;
R² is hydrogen or a radical as mentioned above under R¹.

4. 4-(3-Alkenylbenzoyl)pyrazoles of the formula Ia 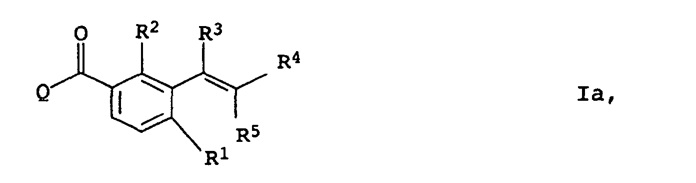 where the variables R¹ to R⁵ and Q are each as defined under claims 1 to 3.

5. A process for preparing 4-(3-alkenylbenzoyl)pyrazoles of the formula I as claimed in claims 1 to 4, which comprises acylating a pyrazole of the formula II (where R¹⁵ = H), where the variables R¹⁴ and R¹⁶ are each as defined under claim 1 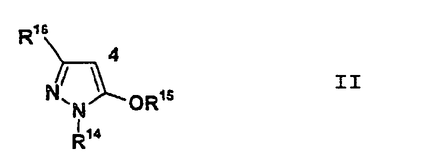 with an activated carboxylic acid IIIα or with a carboxylic acid IIIβ, 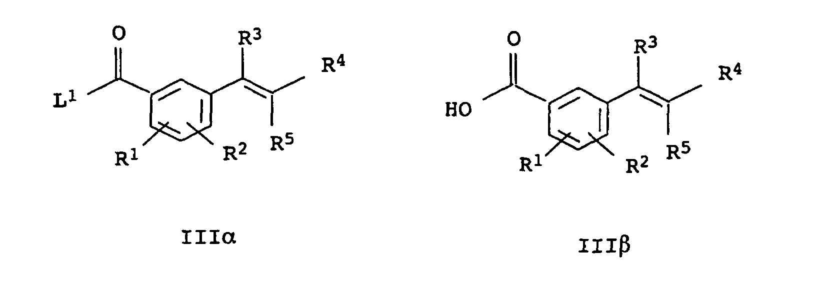 where the variables R¹ to R⁵ are each as defined under claim 1 and L¹ is a nucleophilically displaceable leaving group, subjecting the acylation product to a rearrangement reaction, if appropriate in the presence of a catalyst, to give the compounds I (where R¹⁵ = H) and, if desired, to prepare 4-(3-alkenylbenzoyl)pyrazoles of the formula I where R¹⁵ ≠ H reacting the product with a compound of the formula V,
L² ― R¹⁵ V
(where R¹⁵ ≠ H)
where R¹⁵ is as defined under claim 1 with the exception of hydrogen and L² is a nucleophilically displaceable leaving group.

6. A composition comprising a herbicidally effective amount of at least one 4-(3-alkenylbenzoyl)pyrazole of the formula I or of an agriculturally useful salt of I as claimed in claims 1 to 4 and auxiliaries conventionally used for formulation of crop protection products.

7. A process for preparing herbicides as claimed in claim 6, which comprises mixing a herbicidally effective amount of at least one 4-(3-alkenylbenzoyl)pyrazole of the formula I or of an agriculturally useful salt of I as claimed in claims 1 to 4 with auxiliaries conventionally used in the formulation of crop protection products.

8. A method of controlling undesirable vegetation, which comprises allowing a herbicidally effective amount of at least one 4-(3-alkenylbenzoyl)pyrazole of the formula I or of an agriculturally useful salt of I as claimed in claims 1 to 4 to act on plants, their habitat and/or on seeds.

9. The use of the 4-(3-alkenylbenzoyl)pyrazoles of the formula I and agriculturally useful salts thereof as claimed in claims 1 to 4 as herbicides.

## Revendications

1. 4-(3-alcényl-benzoyl)-pyrazoles répondant à la formule I 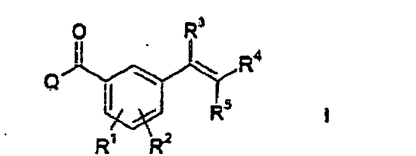 dans laquelle les variables possèdent la signification ci-après :
R¹, R² représentent un atome d'hydrogène, un groupe nitro, un atome d'halogène, un groupe cyano, un groupe rhodano, un groupe alkyle en C₁-C₆, un groupe halogénoalkyle en C₁-C₆, un groupe alcoxy(en C₁-C₆)alkyle en C₁-C₆, un groupe alcényle en C₂-C₆, un groupe alcynyle en C₂-C₆, un groupe -OR⁶, un groupe -OCOR⁷, un groupe -OSO₂R⁷, un groupe -SH, un groupe -S(O)ₙR⁸, un groupe -SO₂OR⁶, un groupe -SO₂NR⁶R⁹, un groupe -NR⁹SO₂R⁷ ou un groupe -NR⁹COR⁷ ;
R³ représente un atome d'hydrogène, un atome d'halogène, un groupe alkyle en C₁-C₆, un groupe halogénoalkyle en C₁-C₆, un groupe alcoxy en C₁-C₆, un groupe alcényle en C₂-C₆ ou un groupe alcynyle en C₂-C₆ ;
R⁴ représente un atome d'hydrogène, un groupe nitro, un atome d'halogène, un groupe cyano, un groupe rhodano, un groupe alkyle en C₁-C₆, un groupe halogénoalkyle en C₁-C₆, un groupe cycloalkyle en C₃-C₆, un groupe alcényle en C₂-C₆, un groupe cycloalcényle en C₄-C₆, un groupe alcynyle en C₂-C₆, un groupe -COR¹⁰, un groupe -CO₂R¹⁰, un groupe -COSR¹⁰, un groupe -CONR¹⁰R¹¹, un groupe -C(R¹²)=NR¹³, un groupe -PO(OR¹⁰)(OR¹¹), un groupe alkyle en C₁-C₄ qui porte un radical choisi parmi le groupe ci-après comprenant: un groupe -COR¹⁰, un groupe -CO₂R¹⁰, un groupe -COSR¹⁰, un groupe -CONR¹⁰R¹¹ ou un groupe -C(R¹²)=NR¹³; un groupe hétérocyclyle, un groupe hétérocyclylalkyle en C₁-C₄ (le radical hétérocyclyle des deux radicaux mentionnés en dernier lieu étant un radical de 3 à 7 membres, saturé ou partiellement insaturé, monocyclique ou polycyclique et contenant de 1 à 3 hétéroatomes choisis parmi le groupe constitué par un atome d'oxygène, un atome d'azote et un atome de soufre); un groupe phényle, un groupe phénylalkyle en C₁-C₄, un groupe hétaryle ou un groupe hétarylalkyle en C₁-C₄ (le radical hétaryle des deux radicaux mentionnés en dernier lieu étant un radical aromatique, mono- ou polycyclique et pouvant contenir, à côté de termes cycliques carbonés, en outre de 1 à 4 atomes d'azote ou bien de 1 à 3 atomes d'azote et un atome d'oxygène ou un atome de soufre ou un atome d'oxygène ou un atome de soufre), les six radicaux mentionnés en dernier lieu pouvant être substitués ;
R⁵ représente un atome d'hydrogène, un atome d'halogène, un groupe cyano, un groupe rhodano, un groupe alkyle en C₁-C₆, un groupe halogénoalkyle en C₁-C₆, un groupe -COR¹⁰, un groupe -CO₂R¹⁰, un groupe -COSR¹⁰, un groupe -CONR¹⁰R¹¹ ou un groupe -PO(OR¹⁰)(OR¹¹) ;
n représente 0, 1 ou 2 ;
R⁶ représente un atome d'hydrogène, un groupe alkyle en C₁-C₆, un groupe halogénoalkyle en C₁-C₆, un groupe alcoxy(en C₁-C₆)alkyle en C₂-C₆, un groupe alcényle en C₃-C₆ ou un groupe alcynyle en C₃-C₆ ;
R⁷ représente un groupe alkyle en C₁-C₆ ou un groupe halogéno-alkyle en C₁-C₆;
R⁸ représente un groupe alkyle en C₁-C₆, un groupe halogéno-alkyle en C₁-C₆, un groupe alcoxy(en C₁-C₆)alkyle en C₂-C₆, un groupe alcényle en C₃-C₆ ou un groupe alcynyle en C₃-C₆ ;
R⁹ représente un atome d'hydrogène ou un groupe alkyle en C₁-C₆ ;
R¹⁰ représente un atome d'hydrogène, un groupe alkyle en C₁-C₆, un groupe cycloalkyle en C₃-C₆, un groupe halogénoalkyle en C₁-C₆, un groupe alcényle en C₃-C₆, un groupe alcynyle en C₃-C₆, un groupe phényle ou un groupe benzyle, les deux radicaux mentionnés en dernier lieu pouvant être partiellement ou complètement halogénés et/ou pouvant porter de 1 à 3 radicaux choisis parmi le groupe ci-après comprenant :
un groupe nitro, un groupe cyano, un groupe alkyle en C₁-C₄, un groupe halogénoalkyle en C₁-C₄, un groupe alcoxy en C₁-C₄, un groupe halogénoalcoxy en C₁-C₄, un groupe alkyl(en C₁-C₄)-carbonyle ou un groupe alcoxy(en C₁-C₄)carbonyle ;
R¹¹ représente un atome d'hydrogène, un groupe alkyle en C₁-C₆, un groupe alcényle en C₃-C₆ ou un groupe alcynyle en C₃-C₆ ;
ou bien
R¹⁰ et R¹¹ forment ensemble une chaîne alcane(en C₂-C₆)diyle, qui peut porter, à titre de substituant, de 1 à 4 groupes alkyle en C₁-C₄ et/ou qui peut être interrompue par un atome d'oxygène ou par un atome de soufre ou encore par un atome d'azote le cas échéant substitué avec un groupe alkyle en C₁-C₄ ;
R¹² représente un atome d'hydrogène, un groupe alkyle en C₁-C₆, un groupe halogénoalkyle en C₁-C₆, un groupe alcoxy en C₁-C₆, un groupe alcoxy(en C₁-C₆)carbonyle, un groupe cycloalkyle en C₃-C₆, un groupe alcényle en C₂-C₆, un groupe alcynyle en C₂-C₆, un groupe phényle ou un groupe benzyle, les deux radicaux mentionnés en dernier lieu pouvant être partiellement ou complètement halogénés et/ou pouvant porter de 1 à 3 radicaux choisis parmi le groupe ci-après comprenant :
un groupe nitro, un groupe cyano, un groupe alkyle en C₁-C₄, un groupe halogénoalkyle en C₁-C₄, un groupe alcoxy en C₁-C₄, un groupe halogénoalcoxy en C₁-C₄, un groupe alkyl(en C₁-C₄)-carbonyle ou un groupe alcoxy(en C₁-C₄)carbonyle ;
R¹³ représente un groupe alkyle en C₁-C₆, un groupe halogénoalkyle en C₁-C₆, un groupe cycloalkyle en C₃-C₆, un groupe alcényle en C₃-C₆, un groupe alcynyle en C₃-C₆, un groupe alcoxy en C₁-C₆, un groupe halogénoalcoxy en C₁-C₆, un groupe cycloalcoxy en C₃-C₆, un groupe alcényl(en C₃-C₆)oxy, un groupe alcynyl(en C₃-C₆)oxy, un groupe phényle, un groupe benzyle ou un groupe phénylalcoxy en C₁-C₄, les trois radicaux mentionnés en dernier lieu pouvant être partiellement ou complètement halogénés et/ou pouvant porter de 1 à 3 radicaux choisis parmi le groupe ci-après comprenant :
un groupe nitro, un groupe cyano, un groupe alkyle en C₁-C₄, un groupe halogénoalkyle en C₁-C₄, un groupe alcoxy en C₁-C₄, un groupe halogénoalcoxy en C₁-C₄, un groupe alkyl(en C₁-C₄)-carbonyle ou un groupe alcoxy(en C₁-C₄)carbonyle ;
Q représente un groupe pyrazole fixé en position 4, répondant à la formule II 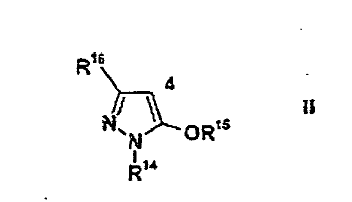
dans laquelle
R¹⁴ représente un groupe alkyle en C₁-C₆, un groupe halogénoalkyle en C₁-C₆, un groupe phényle ou un groupe phényle qui est partiellement ou complètement halogéné et/ou qui porte de 1 à 3 radicaux choisi parmi le groupe ci-après comprenant :
un groupe nitro, un groupe cyano, un groupe alkyle en C₁-C₄, un groupe halogénoalkyle en C₁-C₄, un groupe alcoxy en C₁-C₄, un groupe halogénoalcoxy en C₁-C₄ ;
R¹⁵ représente un atome d'hydrogène, un groupe alkyle en C₁-C₆, un groupe halogénoalkyle en C₁-C₆, un groupe alkyl(en C₁-C₆)carbonyle, un groupe halogénoalkyl(en C₁-C₆)carbonyle, un groupe alcoxy(en C₁-C₆)carbonyle, un groupe alkyl(en C₁-C₆)sulfonyle, un groupe halogéno-alkyl(en C₁-C₆)sulfonyle, un groupe phénylalkyle en C₁-C₄, un groupe phénylcarbonyle, un groupe phénylcarbonylméthyle, un groupe phénoxycarbonyle ou un groupe phénylsulfonyle, les cinq substituants mentionnés en dernier lieu étant non substitués ou bien le noyau phényle étant respectivement en partie ou complètement halogéné et/ou portant de 1 à 3 radicaux choisis parmi le groupe ci-après comprenant :
un groupe nitro, un groupe cyano, un groupe alkyle en C₁-C₄, un groupe halogénoalkyle en C₁-C₄, un groupe alcoxy en C₁-C₄, un groupe halogénoalcoxy en C₁-C₄ ;
R¹⁶ représente un atome d'hydrogène, un groupe alkyle en C₁-C₆ ou un groupe halogénoalkyle en C₁-C₆ ;
ainsi que leurs sels utiles en agriculture.

2. 2-(3-alcényl-benzoyl)-pyrazoles répondant à la formule I selon la revendication 1, dans laquelle
R¹³ représente un groupe alkyle en C₁-C₆, un groupe halogénoalkyle en C₁-C₆, un groupe cycloalkyle en C₃-C₆, un groupe alcényle en C₃-C₆, un groupe alcynyle en C₃-C₆, un groupe alcoxy en C₁-C₆, un groupe halogénoalcoxy en C₁-C₆, un groupe cycloalcoxy en C₃-C₆, un groupe alcényl(en C₃-C₆)oxy, un groupe alcynyl(en C₃-C₆)oxy, un groupe phényle, un groupe benzyle ou un groupe benzyloxy, les trois radicaux mentionnés en dernier lieu pouvant être partiellement ou complètement halogénés et/ou pouvant porter de 1 à 3 radicaux choisis parmi le groupe ci-après comprenant :
un groupe nitro, un groupe cyano, un groupe alkyle en C₁-C₄, un groupe halogénoalkyle en C₁-C₄, un groupe alcoxy en C₁-C₄, un groupe halogénoalcoxy en C₁-C₄, un groupe alkyl(en C₁-C₄)-carbonyle ou un groupe alcoxy(en C₁-C₄)carbonyle ;
R¹⁵ représente un atome d'hydrogène, un groupe alkyle en C₁-C₆, un groupe halogénoalkyle en C₁-C₆, un groupe alkyl(en C₁-C₆)carbonyle, un groupe halogénoalkyl(en C₁-C₆)carbonyle, un groupe alcoxy(en C₁-C₆)carbonyle, un groupe alkyl(en C₁-C₆)sulfonyle, un groupe halogénoalkyl(en C₁-C₆)sulfonyle, un groupe phénylcarbonyle, un groupe phénylcarbonylméthyle, un groupe phénoxycarbonyle ou un groupe phénylsulfonyle, les quatre substituants mentionnés en dernier lieu étant non substitués ou bien le noyau phényle étant respectivement en partie ou complètement halogéné et/ou portant de 1 à 3 radicaux choisis parmi le groupe ci-après comprenant :
un groupe nitro, un groupe cyano, un groupe alkyle en C₁-C₄, un groupe halogénoalkyle en C₁-C₄, un groupe alcoxy en C₁-C₄, un groupe halogénoalcoxy en C₁-C₄.

3. 4-(3-alcényl-benzoyl)-pyrazoles répondant à la formule I selon la revendication 1 ou 2, dans laquelle
R¹ représente un groupe nitro, un atome d'halogène, un groupe cyano, un groupe rhodano, un groupe alkyle en C₁-C₆, un groupe halogénoalkyle en C₁-C₆, un groupe alcoxy(en C₁-C₆)-alkyle en C₁-C₆, un groupe alcényle en C₂-C₆, un groupe alcynyle en C₂-C₆, un groupe -OR⁶ ou un groupe -S(O)ₙR⁸;
R² représente un atome d'hydrogène ou un des radicaux tels que mentionné ci-dessus sous R¹.

4. 4-(3-alcényl-benzoyl)-pyrazoles répondant à la formule la 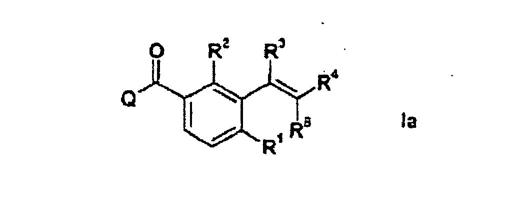 dans laquelle les variables R¹ à R⁵ et Q ont la signification mentionnée dans les revendications 1 à 3.

5. Procédé pour la préparation de 4-(3-alcényl-benzoyl)-pyrazoles répondant à la formule I selon les revendications 1 à 4, **caractérisé en ce qu'**on soumet à une acylation un pyrazole répondant à la formule II (dans laquelle R¹⁵ représente un atome d'hydrogène), les variables R¹⁴ et R¹⁶ possédant la signification indiquée à la revendication 1, 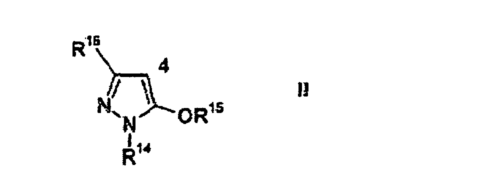 avec un acide carboxylique activé IIIα ou avec un acide carboxylique IIIβ 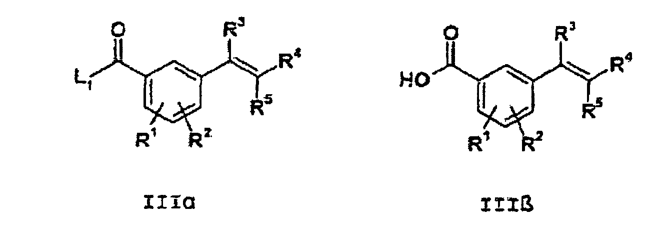 les variables R¹ à R⁵ possédant les significations indiquées à la revendication 1 et L¹ représentant un groupe de départ apte à un déplacement nucléophile, et on transpose le produit d'acylation, le cas échéant en présence d'un catalyseur, pour obtenir les composés I (dans lesquels R¹⁵ représente un atome d'hydrogène) et, le cas échéant, pour la préparation des 4-(3-alcényl-benzoyl)-pyrazoles répondant à la formule I dans lesquels R¹⁵ représente autre chose qu'un atome d'hydrogène, on fait réagir ledit produit avec un composé répondant à la formule V
L²-R¹⁵ V
(avec R¹⁵ ≠ H)
dans laquelle R¹⁵, en faisant abstraction d'un atome d'hydrogène, possède la signification indiquée à la revendication 1 et L² représente un groupe de départ apte à un déplacement nucléophile.

6. Agent contenant une quantité efficace du point de vue herbicide d'au moins un 4-(3-alcényl-benzoyl)-pyrazole répondant à la formule I ou d'un sel de I utile en agriculture, selon les revendications 1 à 4, ainsi que des adjuvants habituels pour la formulation d'agents de protection des plantes.

7. Procédé pour la préparation d'agents à activité herbicide selon la revendication 6, **caractérisé en ce qu'**on mélange une quantité efficace du point de vue herbicide d'au moins un 4-(3-alcényl-benzoyl)-pyrazole répondant à la formule I ou d'un sel de I utile en agriculture, selon les revendications 1 à 4 et des adjuvants habituels pour la formulation d'agents de protection des plantes.

8. Procédé pour lutter contre la croissance non désirée de plantes, **caractérisé en ce qu'**on laisse agir une quantité efficace du point de vue herbicide d'au moins un 4-(3-alcényl-benzoyl)-pyrazole répondant à la formule I ou d'un sel de I utile en agriculture, selon les revendications 1 à 5, sur des plantes, sur leur biotope et/ou sur des semences.

9. Utilisation des 4-(3-alcényl-benzoyl)-pyrazoles répondant à la formule I et de leurs sels utiles en agriculture, selon les revendications 1 à 4, à titre d'herbicides.
